(19) Europäisches Patentamt
European Patent Office
Office européen des brevets

(11) EP 3 010 889 B1

(12) # EUROPÄISCHE PATENTSCHRIFT

(45) Veröffentlichungstag und Bekanntmachung des Hinweises auf die Patenterteilung:
**03.10.2018 Patentblatt 2018/40**

(21) Anmeldenummer: **14729923.4**

(22) Anmeldetag: **16.06.2014**

(51) Int Cl.:
*C07D 233/34* (2006.01) *C07D 233/74* (2006.01)
*C07D 233/96* (2006.01) *C07D 249/12* (2006.01)
*C07D 207/22* (2006.01) *C07D 207/404* (2006.01)
*C07D 277/54* (2006.01) *A01N 43/50* (2006.01)
*A61K 31/402* (2006.01) *A01N 43/653* (2006.01)
*A01N 43/36* (2006.01) *A01N 43/78* (2006.01)
*A61K 31/4196* (2006.01) *A61K 31/4166* (2006.01)
*A61K 31/426* (2006.01)

(86) Internationale Anmeldenummer:
**PCT/EP2014/062510**

(87) Internationale Veröffentlichungsnummer:
**WO 2014/202505 (24.12.2014 Gazette 2014/52)**

(54) **ARYLSULFID- UND ARYLSULFOXID-DERIVATE ALS AKARIZIDE UND INSEKTIZIDE**

ARYLSULFIDE AND ARYLSULFOXIDE DERIVATIVES AS ACARICIDES AND INSECTICIDES

DÉRIVÉS D'ARYLSULFURE ET D'ARYLSULFOXYDE COMME ACARICIDES ET INSECTICIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.06.2013 EP 13172993**

(43) Veröffentlichungstag der Anmeldung:
**27.04.2016 Patentblatt 2016/17**

(73) Patentinhaber: **Bayer CropScience Aktiengesellschaft**
**40789 Monheim am Rhein (DE)**

(72) Erfinder:
- **ALIG, Bernd**
**53639 Königswinter (DE)**
- **CEREZO-GALVEZ, Silvia**
**40764 Langenfeld (DE)**
- **FISCHER, Reiner**
**40789 Monheim (DE)**
- **KÖHLER, Adeline**
**40764 Langenfeld (DE)**
- **HAHN, Julia, Johanna**
**40597 Düsseldorf (DE)**
- **BECKER, Angela**
**40235 Düsseldorf (DE)**
- **ILG, Kerstin**
**50670 Köln (DE)**
- **VOERSTE, Arnd**
**50674 Köln (DE)**
- **PORTZ, Daniela**
**52391 Vettweiss (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 1 803 712** **EP-A1- 2 202 226**
**WO-A1-97/26248** **WO-A1-2010/100189**
**JP-A- 2007 284 386**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 010 889 B1

## Beschreibung

Die vorliegende Erfindung betrifft Arylsulfid-, Arylsulfoxid- und Arylsulfon-Derivate, deren Verwendung als Akarizide und Insektizide zur Bekämpfung tierischer Schädlinge und Verfahren und Zwischenprodukte zu ihrer Herstellung.

Verschiedene Arylsulfide und Arylsulfoxide sowie deren insektizide und akarizide Wirkung sind bereits aus WO 2007/131680 A, WO 2010/100189 A und JP 2011/42611 bekannt. Strukturell teilweise ähnliche, insektizidwirksame 3-Alkoxy-1-Phenyl-Pyrazolderivate, 3-Pyrazolylphenyl-Sulfidderivate und 3-Triazolylphenylsulfid-Derivate sind aus EP 2202226 A1, JP 2007/284386 A und EP 1803712 A1 bekannt. Ferner werden in WO 97/26248 A1 substituierte N-Aryl-Stickstoffheterozyklen beschrieben, welche insbesondere herbizid wirksam sind.

Die gemäß den oben genannten Schriften bereits bekannten Wirkstoffe weisen bei ihrer Anwendung Nachteile auf, beispielsweise dahingehend, dass sie gegebenenfalls keine oder aber eine nur unzureichende insektizide und/oder akarizide Wirkung gegen tierische Schädlinge, insbesondere bei niedrigeren Aufwandmengen, besitzen.

Aufgabe der vorliegenden Erfindung ist es daher, Arylsulfid-, Arylsulfoxid- und Arylsulfon-Derivate bereitzustellen, welche als Insektizide und/oder Akarizide mit einer zufriedenstellenden insektiziden und/oder akariziden Wirkung gegen tierische Schädlinge, insbesondere bei niederigeren Aufwandmengen, mit einer hohen Selektivität und verbesserten Verträglichkeit in Nutzpflanzenkulturen eingesetzt werden können.

Es wurden nun neue Verbindungen der Formel (I)

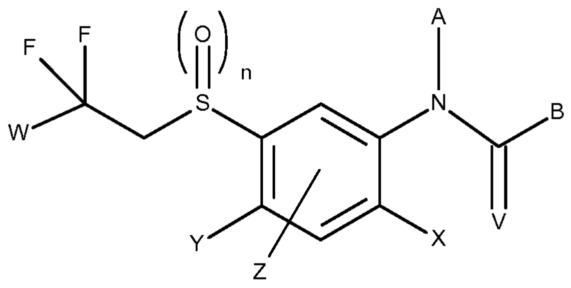

(I)

gefunden,
in welcher

A und B gemeinsam mit den Atomen, an die sie gebunden sind, für eine Substruktur stehen, die ausgewählt ist aus der Gruppe, bestehend aus

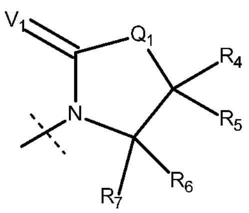

(I-A)

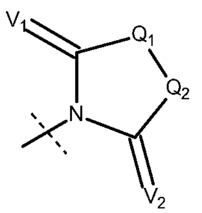

(I-B)

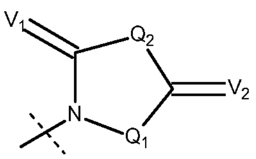

(I-C)

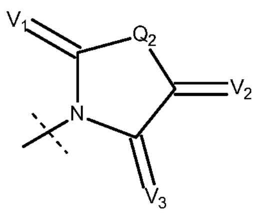

(I-D)

wobei
für den Fall, dass eine Substruktur gemäß Formel (I-A), (I-B) oder (I-D) vorliegt,

$V^1$, $V^2$ und $V^3$, jeweils unabhängig voneinander, für Sauerstoff; Schwefel, einen gegebenenfalls substituierten Stickstoff oder Salze eines gegebenfalls substituierten Stickstoffs stehen;

und für den Fall, dass eine Substruktur gemäß Formel (I-C) vorliegt,

$V^1$ und $V^2$, jeweils unabhängig voneinander, für Sauerstoff oder Schwefel stehen;

für den Fall, dass eine Substruktur gemäß Formel (I-A), (I-B) oder (I-D) vorliegt,

$Q^1$ und $Q^2$, jeweils unabhängig voneinander, für Sauerstoff, Schwefel oder einen gegebenenfalls substituierten Stickstoff stehen; oder für ein gegebenenfalls substituiertes Kohlenstoffatom stehen; mit der Maßgabe, dass $Q_1$ keinen Alkylcarbonylaminorest darstellt;

und für den Fall, dass eine Substruktur gemäß Formel (I-C) vorliegt,

$Q^1$ und $Q^2$, jeweils unabhängig voneinander, für Sauerstoff, Schwefel oder einen substituierten Stickstoff stehen; oder für ein gegebenenfalls substituiertes Kohlenstoffatom stehen; mit der Maßgabe, dass $Q_1$ keinen Alkylcarbonylaminorest darstellt;

$R^4$, $R^5$, $R^6$, und $R^7$, jeweils unabhängig voneinander, für Wasserstoff, Cyano, Halogen oder Nitro stehen; oder für Alkyl, Cycloalkylalkyl, Halogenalkyl, Cyanoalkyl, Hydroxyalkyl, Alkoxyalkyl, Aminoalkyl, Alkoxycarbonylalkyl, Alkylthioalkyl, Halogenalkylthioalkyl, Alkoxyalkylthioalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl oder Hetarylthioalkyl stehen, wobei die vorgenannten Reste jeweils gesättigt oder ungesättigt und/oder gegebenenfalls substituiert sein können; oder für gegebenenfalls substituiertes gesättigtes oder ungesättigtes Cycloalkyl, welches gegebenenfalls durch ein oder mehrere Heteroatome unterbrochen sein kann, stehen; oder für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder für gegebenenfalls substituiertes Phenyl oder gegebenenfalls substituiertes Hetaryl stehen; für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder $R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können; oder $R^6$ und $R^7$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können;

W für Wasserstoff oder Halogen steht;

X, Y und Z, jeweils unabhängig voneinander, für Wasserstoff, Halogen, Hydroxy, Amino, Nitro, OCN, SCN, $SF_5$, stehen;

oder
für Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl oder Dialkylaminothiocarbonyl stehen, wobei alle vorgenannten Reste gegebenenfalls substituiert sein können; oder
für Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy, Hetarylthio, Hetarylsulfinyl oder Hetarylsulfonyl stehen, wobei alle vorgenannten Reste gegebenenfalls substituiert sein können; oder
für Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl oder Cycloalkenyl stehen, wobei alle vorgenannten Reste jeweils gegebenenfalls substituiert sein können; oder
für NR'R" stehen,
wobei R' und R" unabhängig voneinander
für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl oder Alkoxycarbonyl stehen; oder
R' und R" gemeinsam mit dem N-Atom, an das sie gebunden sind, einen gegebenenfalls substituierten und gegebenenfalls durch ein oder mehrere Heteroatome, welche unabhängig ausgewählt werden aus der Gruppe, bestehend aus O, S und N, unterbrochenen gesättigten oder ungesättigten fünf- bis achtgliedrigen Ring bilden können; oder
für einen 3- bis 6-gliedrigen gesättigten, teilgesättigten oder aromatischen Ring, dergegebenenfalls ein bis drei Heteroatome, welche unabhängig ausgewählt werden aus der Gruppe, bestehend aus O, S und N, enthalten kann und der gegebenenfalls substituiert sein kann stehen;
und

n für die Zahl 0 oder 1 steht.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.
Die Verbindungen der Formel (I) umfassen gegebenenfalls Diastereomere oder Enantiomere.
Die erfindungsgemäßen Verbindungen sind durch die Formel (I) allgemein definiert.
In einer bevorzugten Ausführungsform der vorliegenden Erfindung weisen die Verbindungen eine Struktur der allgemeinen Formel (I) auf, in welcher

A und B gemeinsam mit den Atomen, an die sie gebunden sind, für eine Substruktur stehen, die ausgewählt ist aus der Gruppe, bestehend aus (I-A) bis (I-D),

wobei
für den Fall, dass eine Substruktur gemäß Formel (I-A), (I-B) oder (I-D) vorliegt,

$V^1$, $V^2$ und $V^3$, jeweils unabhängig voneinander, für Sauerstoff; Schwefel, $NR^{11}$ oder ein Salz von $NR^{11}$ stehen; und für den Fall, dass eine Substruktur gemäß Formel (I-C) vorliegt,

$V^1$ und $V^2$, jeweils unabhängig voneinander, für Sauerstoff oder Schwefel stehen;

$Q^1$ für Sauerstoff, Schwefel, $NR^1$ oder $CR^2R^3$ steht;

$Q^2$ $NR^{10}$ oder $CR^8R^9$ steht;

für den Fall, dass eine Substruktur gemäß Formel (I-A), (I-B) oder (I-D) vorliegt,

$R^1$ für Wasserstoff, Cyano oder Nitro stehen; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^{10}$ und $R^{11}$, jeweils unabhängig voneinander,
für Wasserstoff, Cyano oder Nitro stehen; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Alkylcarbonyl, Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht;

und für den Fall, dass eine Substruktur gemäß Formel (I-C) vorliegt,

$R^1$ für Cyano oder Nitro steht; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^{10}$ für Cyano oder Nitro steht; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Alkylcarbonyl, Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^2$, $R^3$, $R^4$, $R^5$, $R^6$, $R^7$, $R^8$ und $R^9$, jeweils unabhängig voneinander,

für Wasserstoff, Cyano, Halogen oder Nitro stehen; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylthioalkyl, Halogenalkylthioalkyl, Alkoxyalkylthioalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl oder Hetarylthioalkyl stehen, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, stehen; oder
für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder
für gegebenenfalls substituiertes Phenyl oder gegebenenfalls substituiertes Hetaryl stehen;
für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder
für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder
für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder
$R^2$ und $R^3$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können; oder
$R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können; oder
$R^6$ und $R^7$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können; oder
$R^8$ und $R^9$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können;

W für Wasserstoff oder Halogen steht;
X, Y und Z die zuvor genannten Bedeutungen haben; und
n für die Zahl 0 oder 1 steht.

Erfindungsgemäße Verbindungen, welche in tautomeren Formen vorliegen können, sind allesamt von der vorliegenden Erfindung umfasst.

In einer besonders bevorzugten Ausführungsform der vorliegenden Erfindung weisen die Verbindungen eine Struktur der allgemeinen Formel (I) auf, in welcher

A und B gemeinsam mit den Atomen, an die sie gebunden sind, für eine Substruktur stehen, die ausgewählt ist aus der Gruppe, bestehend aus (I-A) bis (I-D),

wobei
für den Fall, dass eine Substruktur gemäß Formel (I-A), (I-B) oder (I-D) vorliegt,

$V^1$, $V^2$ und $V^3$, jeweils unabhängig voneinander,
für Sauerstoff; Schwefel, $NR^{11}$ oder ein Salz von $NR^{11}$ stehen;

und für den Fall, dass eine Substruktur gemäß Formel (I-C) vorliegt,

$V^1$ und $V^2$, jeweils unabhängig voneinander,
für Sauerstoff oder Schwefel stehen;
$Q^1$ für Sauerstoff, Schwefel, $NR^1$ oder $CR^2R^3$ steht;
$Q^2$ $NR^{10}$ oder $CR^8R^9$ steht;

für den Fall, dass eine Substruktur gemäß Formel (I-A), (I-B) oder (I-D) vorliegt,

$R^1$ für Wasserstoff, Cyano oder Nitro steht; oder
für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, Hydroxy$(C_1\text{-}C_6)$alkyl, $(C_1\text{-}C_6)$Alkoxy$(C_1\text{-}C_6)$alkyl, Amino$(C_1\text{-}C_6)$alkyl, $(C_1\text{-}C_6)$Alkoxycarbonyl$(C_1\text{-}C_6)$alkyl, $(C_1\text{-}C_6)$Alkylsulfanyl$(C_1\text{-}C_6)$alkyl, $(C_1\text{-}C_6)$Alkylsulfinyl$(C_1\text{-}C_6)$alkyl, $(C_1\text{-}C_6)$Alkylsulfonyl$(C_1\text{-}C_6)$alkyl, Halogen$(C_1\text{-}C_6)$alkylsulfanyl$(C_1\text{-}C_6)$alkyl, Halogen$(C_1\text{-}C_6)$alkylsulfinyl$(C_1\text{-}C_6)$alkyl, Halogen$(C_1\text{-}C_6)$alkylsulfonyl$(C_1\text{-}C_6)$alkyl, $(C_1\text{-}C_6)$Alkoxy$(C_1\text{-}C_6)$alkylsulfanyl$(C_1\text{-}C_6)$alkyl, $(C_1\text{-}C_6)$Alkoxy$(C_1\text{-}C_6)$alkylsulfinyl$(C_1\text{-}C_6)$alkyl, $(C_1\text{-}C_6)$Alkoxy$(C_1\text{-}C_6)$alkylsulfonyl$(C_1\text{-}C_6)$alkyl, Phenyl$(C_1\text{-}C_6)$alkyl, Phenoxy$(C_1\text{-}C_6)$alkyl, Phenylsulfanyl$(C_1\text{-}C_6)$alkyl, Phenylsulfinyl$(C_1\text{-}C_6)$alkyl, Phenylsulfonyl$(C_1\text{-}C_6)$alkyl, Hetaryl$(C_1\text{-}C_6)$alkyl, Hetaryloxy$(C_1\text{-}C_6)$alkyl, Hetarylsulfanyl$(C_1\text{-}C_6)$alkyl, Hetarylsulfinyl$(C_1\text{-}C_6)$alkyl, Hetarylsulfonyl$(C_1\text{-}C_6)$alkyl, $(C_1\text{-}C_6)$Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{10}$ und $R^{11}$, jeweils unabhängig voneinander,
für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, ($C_1$-$C_6$)Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht;

und für den Fall, dass eine Substruktur gemäß Formel (I-C) vorliegt,

$R^1$ für Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl

steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{10}$ für Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, $R^5$, $R^6$, $R^7$, $R^8$ und $R^9$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylthio$(C_1-C_6)$alkyl, Halogen$(C_1-C_6)$alkylthio$(C_1-C_6)$alkyl, $(C_1-C_6)$Alkoxy$(C_1-C_6)$alkylthioalkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl oder Hetarylthio$(C_1-C_6)$alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;-oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können oder

$R^6$ und $R^7$ gemeinsam mit dem Atom, an das sie gebunden sind-einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;-oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Halogen steht;

X, Y und Z die zuvor genannten Bedeutungen haben; und

n für die Zahl 0 oder 1 steht.

Weitere bevorzugte isolierte Ausführungsformen werden im Folgenden näher beschreiben:

Erste Ausführungsform (I-A):

In einer ersten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-A) stehen

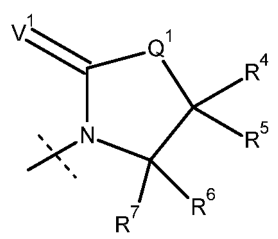

(I-A)

wobei
$V^1$ für Sauerstoff; Schwefel, $NR^{11}$ oder ein Salz von $NR^{11}$ steht;
$Q^1$ für Sauerstoff, Schwefel, $NR^1$ oder $CR^2R^3$ steht;
$R^1$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder

für $(C_1\text{-}C_6)$Alkoxy, Halogen$(C_1\text{-}C_6)$alkoxy, $(C_3\text{-}C_6)$Cycloalkyloxy, Aryloxy, Aryl$(C_1\text{-}C_6)$alkyloxy oder Carbonyloxy steht, wobei die vorgenannten Reste jeweils gegebenenfalls substiuiert sein können, oder für Hydroxy steht; oder

für $(C_1\text{-}C_6)$Alkylamino, Halogen$(C_1\text{-}C_6)$alkylamino, Dihalogen$(C_1\text{-}C_6)$alkylamino, Di$(C_1\text{-}C_6)$alkylamino, $(C_3\text{-}C_6)$Cycloalkylamino, Di$(C_3\text{-}C_6)$cycloalkylamino, $(C_3\text{-}C_6)$Cycloalkyl($(C_1\text{-}C_6)$alkyl)amino, Arylamino, Diarylamino, Hetarylamino, Dihetarylamino, $(C_1\text{-}C_6)$Alkyl(aryl)amino, $(C_3\text{-}C_6)$Cycloalkyl(aryl)amino, $(C_1\text{-}C_6)$Alkylcarbonylamino, Arylcarbonylamino, $(C_1\text{-}C_6)$Alkoxycarbonylamino, Aryloxycarbonylamino, $(C_1\text{-}C_6)$Alkylcarbamoylamino, Arylcarbamoylamino, $(C_1\text{-}C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1\text{-}C_6)$Alkylsulfanyl, $(C_1\text{-}C_6)$Alkylsulfinyl, $(C_1\text{-}C_6)$Alkylsulfonyl, Halogen$(C_1\text{-}C_6)$alkylsulfanyl, Halogen$(C_1\text{-}C_6)$alkylsulfinyl, Halogen$(C_1\text{-}C_6)$alkylsulfanyl, $(C_3\text{-}C_6)$Cycloalkylsulfanyl, $(C_3\text{-}C_6)$Cycloalkylsulfinyl, $(C_3\text{-}C_6)$Cycloalkylsulfonyl, $(C_3\text{-}C_6)$Cycloalkyl$(C_1\text{-}C_6)$alkylsulfanyl, $(C_3\text{-}C_6)$Cycloalkyl$(C_1\text{-}C_6)$alkylsulfinyl, $(C_3\text{-}C_6)$Cycloalkyl$(C_1\text{-}C_6)$alkylsulfonyl, Arylsulfanyl, Arylsulfinyl, Arylsulfonyl, Aryl$(C_1\text{-}C_6)$alkylsulfanyl, Aryl$(C_1\text{-}C_6)$alkylsulfinyl, Aryl$(C_1\text{-}C_6)$alkylsulfonyl, Aminosulfonyl, $(C_1\text{-}C_6)$Alkylaminosulfonyl, Di$(C_1\text{-}C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{11}$ für Wasserstoff, Cyano oder Nitro steht; oder

für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, Hydroxy$(C_1\text{-}C_6)$alkyl, $(C_1\text{-}C_6)$Alkoxy$(C_1\text{-}C_6)$alkyl, Amino$(C_1\text{-}C_6)$alkyl, $(C_1\text{-}C_6)$Alkoxycarbonyl$(C_1\text{-}C_6)$alkyl, $(C_1\text{-}C_6)$Alkylsulfanyl$(C_1\text{-}C_6)$alkyl, $(C_1\text{-}C_6)$Alkylsulfinyl$(C_1\text{-}C_6)$alkyl, $(C_1\text{-}C_6)$Alkylsulfonyl$(C_1\text{-}C_6)$alkyl, Halogen$(C_1\text{-}C_6)$alkylsulfanyl$(C_1\text{-}C_6)$alkyl, Halogen$(C_1\text{-}C_6)$alkylsulfinyl$(C_1\text{-}C_6)$alkyl, Halogen$(C_1\text{-}C_6)$alkylsulfonyl$(C_1\text{-}C_6)$alkyl, $(C_1\text{-}C_6)$Alkoxy$(C_1\text{-}C_6)$alkylsulfanyl$(C_1\text{-}C_6)$alkyl, $(C_1\text{-}C_6)$Alkoxy$(C_1\text{-}C_6)$alkylsulfinyl$(C_1\text{-}C_6)$alkyl, $(C_1\text{-}C_6)$Alkoxy$(C_1\text{-}C_6)$alkylsulfonyl$(C_1\text{-}C_6)$alkyl, Phenyl$(C_1\text{-}C_6)$alkyl, Phenoxy$(C_1\text{-}C_6)$alkyl, Phenylsulfanyl$(C_1\text{-}C_6)$alkyl, Phenylsulfinyl$(C_1\text{-}C_6)$alkyl, Phenylsulfonyl$(C_1\text{-}C_6)$alkyl, Hetaryl$(C_1\text{-}C_6)$alkyl, Hetaryloxy$(C_1\text{-}C_6)$alkyl, Hetarylsulfanyl$(C_1\text{-}C_6)$alkyl, Hetarylsulfinyl$(C_1\text{-}C_6)$alkyl, Hetarylsulfonyl$(C_1\text{-}C_6)$alkyl, $(C_1\text{-}C_6)$Alkoxycarbonyl, Aryloxycarbonyl, $(C_1\text{-}C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3\text{-}C_6)$Cycloalkyl, welches gegebenenfalls durch ein oder mehrere Heteroatome unterbrochen sein kann, steht; oder

für Halogen$(C_1\text{-}C_6)$alkylcarbonyl, Hydroxy$(C_1\text{-}C_6)$alkylcarbonyl, $(C_1\text{-}C_6)$Alkoxy$(C_1\text{-}C_6)$alkylcarbonyl, Phenylcarbonyl, Hetarylcarbonyl, Halogen$(C_1\text{-}C_6)$alkoxycarbonyl, Aryloxycarbonyl, Aminocarbonyl, $(C_1\text{-}C_6)$Alkylaminocarbonyl, Di$(C_1\text{-}C_6)$alkylaminocarbonyl, $(C_3\text{-}C_6)$Cycloalkylaminocarbonyl, Di$(C_3\text{-}C_6)$cycloalkylaminocarbonyl, $(C_3\text{-}C_6)$Cycloalkyl($(C_1\text{-}C_6)$alkyl)aminocarbonyl, Arylaminocarbonyl, Diarylaminocarbonyl, $(C_1\text{-}C_6)$Alkyl(aryl)aminocarbonyl, $(C_3\text{-}C_6)$Cycloalkyl(aryl)aminocarbonyl, $(C_1\text{-}C_6)$Alkylaminothiocarbonyl, Di$(C_1\text{-}C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1\text{-}C_6)$Alkoxy, Halogen$(C_1\text{-}C_6)$alkoxy, $(C_3\text{-}C_6)$Cycloalkyloxy, Aryloxy, Aryl$(C_1\text{-}C_6)$alkyloxy oder Carbonyloxy steht, wobei die vorgenannten Reste jeweils gegebenenfalls substiuiert sein können, oder für Hydroxy steht; oder

für $(C_1\text{-}C_6)$Alkylamino, Halogen$(C_1\text{-}C_6)$alkylamino, Dihalogen$(C_1\text{-}C_6)$alkylamino, Di$(C_1\text{-}C_6)$alkylamino, $(C_3\text{-}C_6)$Cycloalkylamino, Di$(C_3\text{-}C_6)$cycloalkylamino, $(C_3\text{-}C_6)$Cycloalkyl($(C_1\text{-}C_6)$alkyl)amino, Arylamino, Diarylamino, Hetarylamino, Dihetarylamino, $(C_1\text{-}C_6)$Alkyl(aryl)amino, $(C_3\text{-}C_6)$Cycloalkyl(aryl)amino, $(C_1\text{-}C_6)$Alkylcarbonylamino, Arylcarbonylamino, $(C_1\text{-}C_6)$Alkoxycarbonylamino, Aryloxycarbonylamino, $(C_1\text{-}C_6)$Alkylcarbamoylamino, Arylcarbamoylamino, $(C_1\text{-}C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1\text{-}C_6)$Alkylsulfanyl, $(C_1\text{-}C_6)$Alkylsulfinyl, $(C_1\text{-}C_6)$Alkylsulfonyl, Halogen$(C_1\text{-}C_6)$alkylsulfanyl, Halogen$(C_1\text{-}C_6)$alkylsulfinyl, Halogen$(C_1\text{-}C_6)$alkylsulfanyl, $(C_3\text{-}C_6)$Cycloalkylsulfanyl, $(C_3\text{-}C_6)$Cycloalkylsulfinyl,

$(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, $R^5$, $R^6$ und $R^7$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylthio$(C_1-C_6)$alkyl, Halogen$(C_1-C_6)$alkylthio$(C_1-C_6)$alkyl, $(C_1-C_6)$Alkoxy$(C_1-C_6)$alkylthioalkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl oder Hetarylthio$(C_1-C_6)$alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome

aus der Reihe O, S oder N unterbrochenen gesättigten oder ungesättigten drei- bis sechsgliedrigen Ring bilden können; oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^6$ und $R^7$ gemeinsam mit dem Atom, an das sie gebunden sind-einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder

W für Wasserstoff, Fluor oder Chlor steht;

X, Y und Z unabhängig voneinander für Wasserstoff, Fluor, Chlor, Brom, Nitro, Amino, Hydroxy, $(C_1\text{-}C_4)$Alkyl, $(C_1\text{-}C_4)$Halogenalkyl, $(C_2\text{-}C_4)$Alkenyl, $(C_2\text{-}C_4)$Alkinyl, $(C_1\text{-}C_4)$Alkoxy, $(C_1\text{-}C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

In einer bevorzugten Ausgestaltung dieser ersten Ausführungsform steht die Substruktur der Formel (I-A) für eine Substruktur, welche ausgewählt wird aus der Gruppe, bestehend aus

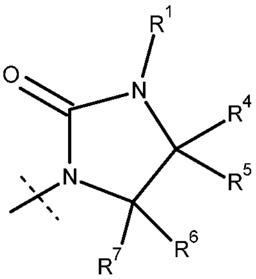

(I-A-1)

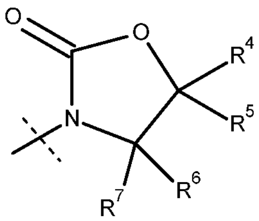

(I-A-2)

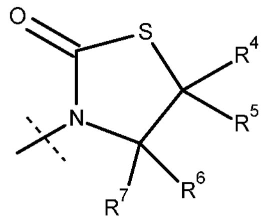

(I-A-3)

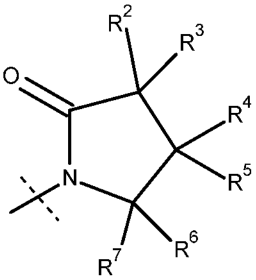

(I-A-4)

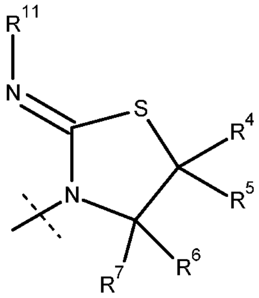

(I-A-5)

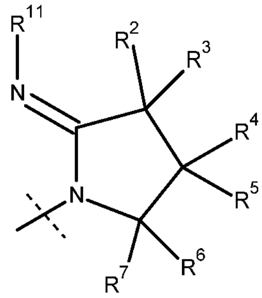

(I-A-6)

wobei

$R^1$ für Wasserstoff steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^{11}$ für Wasserstoff steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino,

Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, , ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; und

$R^3$, $R^4$, $R^2$, $R^5$, $R^6$ und $R^7$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Phenyl($C_1$-$C_6$)alkyl, , Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können oder für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy stehen; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, , ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano sub-

stituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder
$R^6$ und $R^7$ gemeinsam mit dem Atom, an das sie gebunden sindeinen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

W für Wasserstoff oder Fluor steht;

X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Hierbei sind insbesondere bei den Substrukturen (I-A-5) und (I-A-6) E- und Z-Isomere der exocyclischen Doppelbindung zum Stickstoff umfasst. Ebenso sind die Salze des an der exocyclischen Doppelbindung befindlichen Stickstoffs der Substrukturen (I-A-5) und (I-A-6) umfasst. Bevorzugt handelt es sich hier um die Salze durch Addition von Halogensäuren. Ganz besonders bevorzugt handelt es sich hier um Hydrochloride.

Dabei ist es insbesondere bevorzugt, dass

$R^1$ und $R^{11}$, jeweils unabhängig voneinander,
für Wasserstoff stehen; oder
($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_2$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl oder gegebenenfalls substituiertes Phenyl steht;

$R^2$, $R^3$, $R^4$, $R^5$, $R^6$ und $R^7$, jeweils unabhängig voneinander,
für Wasserstoff stehen;
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl, oder gegebenenfalls substituiertes Phenyl steht; oder

W für Wasserstoff oder Fluor steht;

X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Bevorzugt steht die Substruktur der Formel (I-A) für eine Substruktur, welche ausgewählt wird aus der Gruppe, bestehend aus

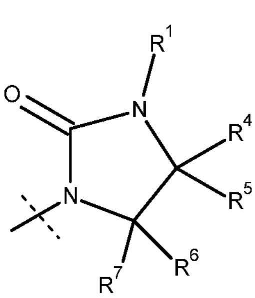
(I-A-1)

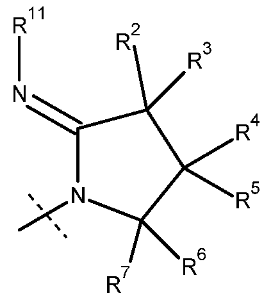
(I-A-6)

wobei

| | |
|---|---|
| $R^1$ und $R^{11}$, | jeweils unabhängig voneinander, für Wasserstoff, Methyl, Ethyl, Propyl, Isopropyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ stehen; oder |
| $R^2$, $R^3$, $R^4$, $R^5$, $R^6$ und $R^7$, | jeweils unabhängig voneinander für Wasserstoff, Methyl, Trifluoromethyl oder Phenyl stehen; |
| W | Wasserstoff oder Fluor steht; |
| X | für Wasserstoff, Chlor, Fluor oder Methyl steht; |
| Y | für Chlor, Brom, Methyl, Trifluoromethyl, Fluor oder Methoxy steht; |
| X und Y | insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H); |
| Z | für Wasserstoff steht; und |
| n | für die Zahl 0 oder 1 steht. |

Die Salze des an der exocyclischen Doppelbindung befindlichen Stickstoffs der Substruktur (I-A-6) sind ebenfalls umfasst. Bevorzugt handelt es sich hier um die Salze durch Addition von Halogensäuren. Ganz besonders bevorzugt handelt es sich hier um Hydrochloride.

Ebenfalls bevorzugt steht die Substruktur der Formel (I-A) für eine Substruktur, welche ausgewählt wird aus der Gruppe, bestehend aus

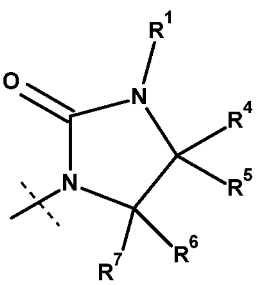
(I-A-1)

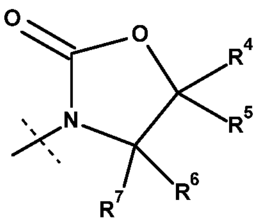
(I-A-2)

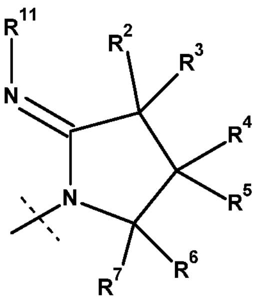
(I-A-6)

wobei

| | |
|---|---|
| $R^1$ und $R^{11}$, | jeweils unabhängig voneinander, für Wasserstoff, Methyl, Ethyl, Propyl, Isopropyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ stehen; oder |
| $R^2$, $R^3$, $R^4$, $R^5$, $R^6$ und $R^7$, | jeweils unabhängig voneinander für Wasserstoff, Methyl, Trifluoromethyl oder Phenyl stehen; |
| W | für Wasserstoff oder Fluor steht; |
| X | für Wasserstoff, Chlor, Fluor oder Methyl steht; |
| Y | für Chlor, Brom, Methyl, Trifluoromethyl oder Fluor steht; |
| X und Y | insbesondere für folgende Kombinationen (Y,X) stehen: (Me, F), (Me,H), (Me,Cl), (Me,Me), |

(Cl,Cl), (Cl,F), (Cl,H), (Br,H), (Br,F), (F,F), ($CF_3$,H);

Z für Wasserstoff steht; und

n für die Zahl 0 oder 1 steht.

Handelt es sich bei der Substruktur der Formel (I-A) um die Substrukturformel (I-A-1), so ist es bevorzugt, dass

$R^1$ für Wasserstoff, Methyl, Ethyl, $CH_2CF_3$, Cyclopropyl oder Cyclopropylmethyl steht;

$R^4$, $R^5$, $R^6$ und $R^7$, jeweils unabhängig voneinander für Wasserstoff, Methyl, Trifluoromethyl oder Phenyl stehen;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Wasserstoff steht;

$R^4$, $R^5$, $R^6$ und $R^7$ für Wasserstoff stehen;

W für Fluor steht;

X für Methyl oder Fluor steht;

Y für Methyl steht;

X und Y stehen insbesondere für folgende Kombinationen (Y, X): (Me, F), (Me, Me)

Z für Wasserstoff steht; und

n für die Zahl 0 oder 1 steht.

Handelt es sich bei der Substruktur der Formel (I-A) um die Substrukturformel (I-A-1), so ist es ebenfalls bevorzugt, dass

$R^1$ für Wasserstoff, Methyl, Ethyl, $CH_2CF_3$, Cyclopropyl oder Cyclopropylmethyl steht;

$R^4$, $R^5$, $R^6$ und $R^7$, jeweils unabhängig voneinander für Wasserstoff, Methyl, Trifluoromethyl oder Phenyl stehen;

W Wasserstoff oder Fluor steht;

X für Wasserstoff, Chlor, Fluor oder Methyl steht;

Y für Chlor, Brom, Methyl, Trifluoromethyl oder Fluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Wasserstoff oder Methyl steht;

$R^4$, $R^5$, $R^6$ und $R^7$ für Wasserstoff stehen;

W für Fluor steht;

X für Methyl oder Fluor steht;

Y für Methyl steht;

X und Y stehen insbesondere für folgende Kombinationen (Y, X): (Me, F), (Me, Me)

Z für Wasserstoff steht; und

n für die Zahl 0 oder 1 steht.

Handelt es sich bei der Substruktur der Formel (I-A) um die Substrukturformel (I-A-2), so ist es bevorzugt, dass

$R^4$, $R^5$, $R^6$ und $R^7$, jeweils unabhängig voneinander für Wasserstoff, Methyl, Trifluoromethyl oder Phenyl stehen;

| | |
|---|---|
| W | Wasserstoff oder Fluor steht; |
| X | für Wasserstoff, Chlor, Fluor oder Methyl steht; |
| Y | für Chlor, Brom, Methyl, Trifluoromethyl oder Fluor steht; |
| X und Y | insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H); |
| Z | für Wasserstoff steht; und |
| n | für die Zahl 0 oder 1 steht. |

Hierbei ist es besonders bevorzugt, dass

$R^4$ für Wasserstoff oder Methyl steht;

$R^5$ für Wasserstoff steht;

$R^6$ für Wasserstoff oder Methyl steht;

$R^7$ für Wasserstoff steht;

W für Fluor steht;

X für Fluor steht;

Y für Methyl steht;

Z für Wasserstoff steht; und

n für die Zahl 0 oder 1 steht.

Handelt es sich bei der Substruktur der Formel (I-A) um die Substrukturformel (I-A-6), so ist es bevorzugt, dass

| | |
|---|---|
| $R^{11}$ | für Wasserstoff, Methyl, Ethyl, $CH_2CF_3$, $CH_2CHF_2$, $CH(CH_3)CF_3$, $CH_2CH_2CF_3$ oder $CH_2CH_2CHF_2$, Cyclopropyl oder Cyclopropylmethyl steht; |
| $R^2$, $R^3$, $R^4$, $R^5$, $R^6$ und $R^7$, | jeweils unabhängig voneinander für Wasserstoff, Methyl, Trifluoromethyl oder Phenyl stehen; |
| W | Wasserstoff oder Fluor steht; |
| X | für Wasserstoff, Chlor, Fluor oder Methyl steht; |
| Y | für Chlor, Brom, Methyl, Trifluoromethyl, Fluor oder Methoxy steht; |
| X und Y | insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H); |
| Z | für Wasserstoff steht; und |
| n | für die Zahl 0 oder 1 steht, |

wobei der an der exocyclischen Doppelbindung befindliche Stickstoff auch als Hydrohalogenid vorliegen kann.

Hierbei ist es besonders bevorzugt, dass

| | |
|---|---|
| $R^{11}$ | für $CH_2CF_3$ oder $CH(CH_3)CF_3$ steht; |
| $R^2$, $R^3$, $R^4$, $R^5$, $R^6$ und $R^7$ | für Wasserstoff stehen; |
| W | für Fluor steht; |
| X | für Methyl oder Fluor steht; |
| Y | für Methyl steht; |

X und Y stehen insbesondere für folgende Kombinationen (Y, X): (Me, F), (Me, Me)

Z für Wasserstoff steht; und

n für die Zahl 0 oder 1 steht,

wobei der an der exocyclischen Doppelbindung befindliche Stickstoff auch als Hydrochlorid vorliegen kann.

Zweite Ausführungsform (I-B):

In einer zweiten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-B) stehen

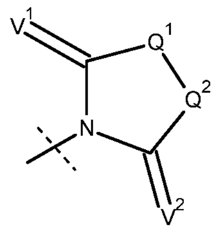

(I-B)

wobei

$V^1$ und $V^2$, jeweils unabhängig voneinander, für Sauerstoff; Schwefel, $NR^{11}$ oder ein Salz von $NR^{11}$ stehen;

$Q^1$ für Sauerstoff, Schwefel, $NR^1$ oder $CR^2R^3$ steht;

$Q^2$ $NR^{10}$ oder $CR^8R^9$ steht;

$R^1$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkyl-

amino, $(C_3-C_6)$Cycloalkylamino, $Di(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, $Di(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{10}$ und $R^{11}$, jeweils unabhängig voneinander,

für Wasserstoff, Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, $Di(C_1-C_6)$alkylaminocarbonyl, $(C_3-C_6)$Cycloalkylaminocarbonyl, $Di(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, $Di(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, $Di(C_1-C_6)$alkylamino, $(C_3-C_6)$Cycloalkylamino, $Di(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^8$ und $R^9$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylthio$(C_1-C_6)$alkyl, Halogen$(C_1-C_6)$alkylthio$(C_1-C_6)$alkyl, $(C_1-C_6)$Alkoxy$(C_1-C_6)$alkylthioalkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl oder Hetarylthio$(C_1-C_6)$alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder $R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z unabhängig voneinander für Wasserstoff, Fluor, Chlor, Brom, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

In einer bevorzugten Ausgestaltung dieser zweiten Ausführungsform steht die Substruktur der Formel (I-B) für eine Substruktur, welche ausgewählt wird aus der Gruppe, bestehend aus

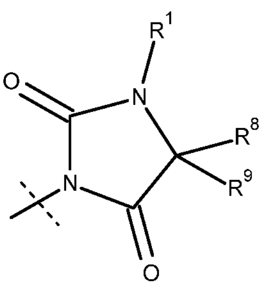

(I-B-1)

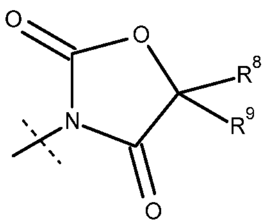

(I-B-2)

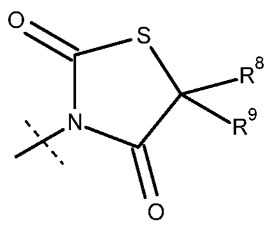

(I-B-3)

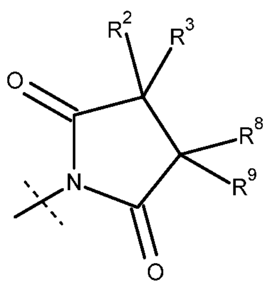

(I-B-4)

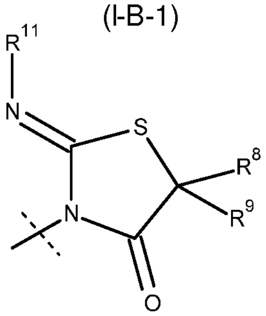

(I-B-5)

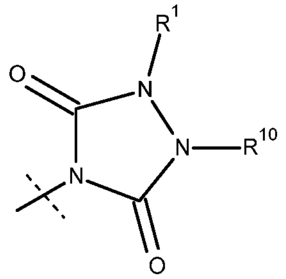

(I-B-6)

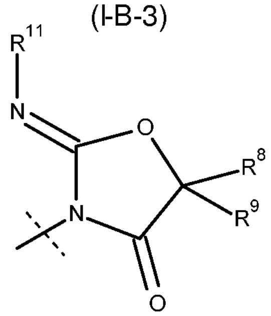

(I-B-7)

wobei

R[1] für Wasserstoff steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

R[10] und R[11], jeweils unabhängig voneinander,
für Wasserstoff steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl,

($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; und

$R^2$, $R^3$, $R^8$ und $R^9$, jeweils unabhängig voneinander,
für Wasserstoff, Cyano, Halogen oder Nitro steht; oder
für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können ; oder
für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder
für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy stehen; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, , ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; oder
$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;oder
$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Fluor steht;

X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Hierbei ist es bevorzugt, dass

$R^1$, $R^{10}$ und $R^{11}$, jeweils unabhängig voneinander,
für Wasserstoff stehen; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halo-

gen($C_2$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl oder gegebenenfalls substituiertes Phenyl steht;

$R^2$, $R^3$, $R^8$ und $R^9$, jeweils unabhängig voneinander,
für Wasserstoff stehen; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl, oder gegebenenfalls substituiertes Phenyl steht; oder

W für Wasserstoff oder Fluor steht;

X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Bevorzugt steht die Substruktur der Formel (I-B) für eine Substruktur, welche ausgewählt wird aus der Gruppe, bestehend aus

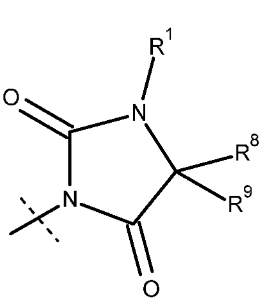

(I-B-1)

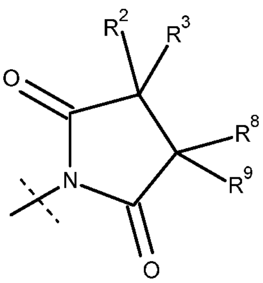

(I-B-4)

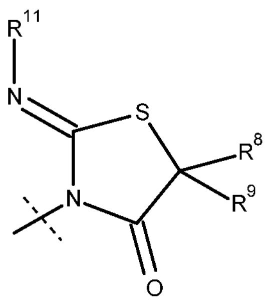

(I-B-5)

wobei

$R^1$ und $R^{11}$, jeweils unabhängig voneinander,
für Wasserstoff, Methyl, Ethyl, Propyl, Isopropyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ stehen; oder

$R^2$, $R^3$, $R^8$ und $R^9$, jeweils unabhängig voneinander,
für Wasserstoff, Methyl, Trifluoromethyl oder Phenyl stehen;

W für Wasserstoff oder Halogen steht;

X, Y und Z, jeweils unabhängig voneinander,
für Wasserstoff; Halogen oder Nitro stehen; oder
für ($C_1$-$C_6$)Alkyl; ($C_1$-$C_6$)Alkoxy; Halogen($C_1$-$C_6$)alkyl; Halogen($C_1$-$C_6$)alkoxy, wobei die vorgenannten Reste gegebenenfalls substituiert sein können, stehen; und

n für die Zahl 0 oder 1 steht.

Ebenfalls bevorzugt steht die Substruktur der Formel (I-B) für eine Substruktur, welche ausgewählt wird aus der Gruppe, bestehend aus

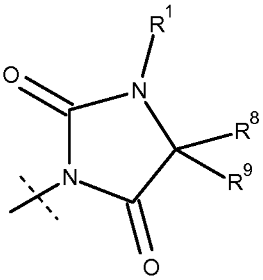

(I-B-1)

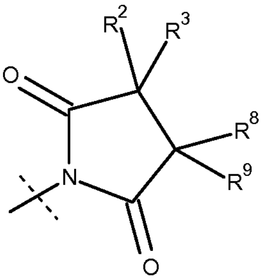

(I-B-4)

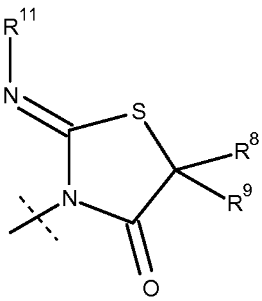

(I-B-5)

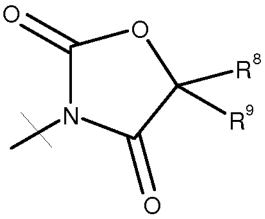

(I-B-2)

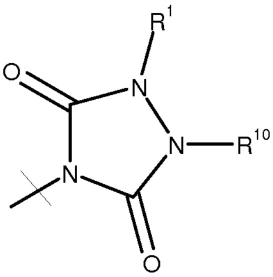

(I-B-6)

wobei

| | |
|---|---|
| $R^1$, $R^{10}$ und $R^{11}$, | jeweils unabhängig voneinander, für Wasserstoff, Methyl, Ethyl, Propyl, Isopropyl, n-Butyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$, $CH_2C(CH_3)_3$, $CH_2CH(CH_3)_2$, $CH_2CH=CH_2$ (= Allyl), $CH_2CCH$ oder Benzyl stehen; |
| $R^2$, $R^3$, $R^8$ und $R^9$, | jeweils unabhängig voneinander, für Wasserstoff, Methyl, Trifluoromethyl oder Phenyl stehen; |
| W | für Wasserstoff oder Fluor steht; |
| X, Y und Z, | jeweils unabhängig voneinander, für Wasserstoff; Fluor, Brom, Chlor oder Nitro stehen; oder für $(C_1\text{-}C_6)$Alkyl; $(C_1\text{-}C_6)$Alkoxy; Halogen$(C_1\text{-}C_6)$alkyl; Halogen$(C_1\text{-}C_6)$alkoxy, wobei die vorgenannten Reste gegebenenfalls substituiert sein können, stehen; und |
| n | für die Zahl 0 oder 1 steht. |

Ebenfalls bevorzugt steht die Substruktur der Formel (I-B) für eine Substruktur, welche ausgewählt wird aus der Gruppe, bestehend aus

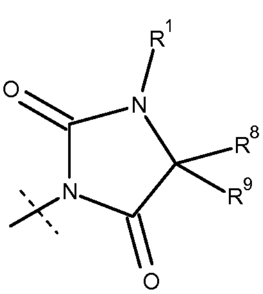

(I-B-1)

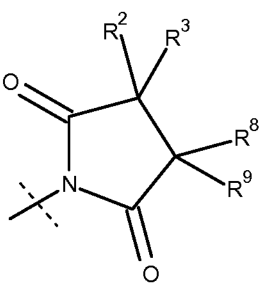

(I-B-4)

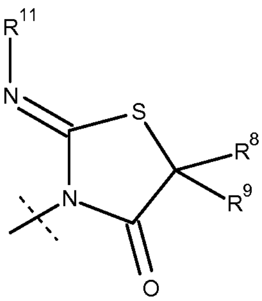

(I-B-5)

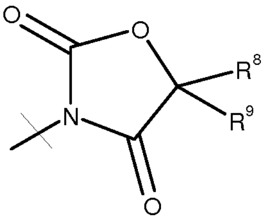
(I-B-2)

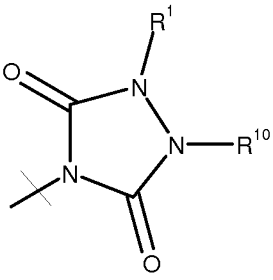
(I-B-6)

wobei

$R^1$, $R^{10}$ und $R^{11}$, jeweils unabhängig voneinander, für Wasserstoff, Methyl, Ethyl, Propyl, Isopropyl, n-Butyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$, $CH_2C(CH_3)_3$, $CH_2CH(CH_3)_2$, $CH_2CH{=}CH_2$ (= Allyl), $CH_2CCH$ oder Benzyl stehen;
$R^2$, $R^3$, $R^8$ und $R^9$, jeweils unabhängig voneinander, für Wasserstoff, Methyl, Trifluoromethyl oder Phenyl stehen;
W für Wasserstoff oder Fluor steht;
X für Wasserstoff, Chlor, Fluor oder Methyl steht;
Y für Chlor, Brom, Methyl, Trifluoromethyl oder Fluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht; und
n für die Zahl 0 oder 1 steht.

Handelt es sich bei der Substruktur der Formel (I-B) um die Substrukturformel (I-B-1), so ist es bevorzugt, dass

$R^1$ für Wasserstoff, Methyl, Ethyl, Propyl, Isopropyl, $CH_2CF_3$, Cyclopropyl oder Cyclopropylmethyl steht;

$R^8$ und $R^9$ unabhängig voneinander für Wasserstoff, Methyl, Trifluoromethyl oder Phenyl stehen;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Methyl steht;
$R^8$ und $R^9$ jeweils für Methyl stehen;
W für Fluor steht;
X für Wasserstoff oder Fluor steht;
Y für Methyl steht;
Z für Wasserstoff steht; und
n für die Zahl 0 oder 1 steht.

Handelt es sich bei der Substruktur der Formel (I-B) um die Substrukturformel (I-B-1), so ist es ebenfalls

bevorzugt, dass

| | |
|---|---|
| $R^1$ | für Wasserstoff, Methyl, Ethyl, Propyl, Isopropyl, n-Butyl, $CH_2CF_3$, $CH_2CH=CH_2$, Cyclopropyl, Cyclopropylmethyl oder Benzyl steht; |
| $R^8$ und $R^9$ | unabhängig voneinander für Wasserstoff, Methyl, Trifluoromethyl oder Phenyl stehen; |
| W | Wasserstoff oder Fluor steht; |
| X | für Wasserstoff, Chlor, Fluor oder Methyl steht; |
| Y | für Chlor, Brom, Methyl, Trifluoromethyl oder Fluor steht; |
| X und Y | insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H); |
| Z | für Wasserstoff steht; und |
| n | für die Zahl 0 oder 1 steht. |

Hierbei ist es besonders bevorzugt, dass

| | |
|---|---|
| $R^1$ | für Methyl, n-Butyl, $CH_2CH=CH_2$ oder Benzyl steht; |
| $R^8$ und $R^9$ | jeweils für Wasserstoff oder Methyl stehen; |
| W | für Fluor steht; |
| X | für Wasserstoff oder Fluor steht; |
| Y | für Methyl steht; |
| Z | für Wasserstoff steht; und |
| n | für die Zahl 0 oder 1 steht. |

Handelt es sich bei der Substruktur der Formel (I-B) um die Substrukturformel (I-B-2), so ist es bevorzugt, dass

| | |
|---|---|
| $R^8$ und $R^9$ | unabhängig voneinander für Wasserstoff, Methyl, Trifluoromethyl oder Phenyl stehen; |
| W | Wasserstoff oder Fluor steht; |
| X | für Wasserstoff, Chlor, Fluor oder Methyl steht; |
| Y | für Chlor, Brom, Methyl, Trifluoromethyl oder Fluor steht; |
| X und Y | insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H); |
| Z | für Wasserstoff steht; und |
| n | für die Zahl 0 oder 1 steht. |

Hierbei ist es besonders bevorzugt, dass

| | |
|---|---|
| $R^8$ und $R^9$ | unabhängig voneinander für Wasserstoff oder Methyl stehen; |
| W | für Fluor steht; |
| X | für Fluor steht; |
| Y | für Methyl steht; |
| Z | für Wasserstoff steht; und |
| n | für die Zahl 0 oder 1 steht. |

Handelt es sich bei der Substruktur der Formel (I-B) um die Substrukturformel (I-B-4), so ist es bevorzugt, dass

| | |
|---|---|
| $R^2$, $R^3$, $R^8$ und $R^9$, | jeweils unabhängig voneinander unabhängig voneinander für Wasserstoff, Methyl, Trifluoromethyl oder Phenyl stehen; |
| W | Wasserstoff oder Fluor steht; |
| X | für Wasserstoff, Chlor, Fluor oder Methyl steht; |
| Y | für Chlor, Brom, Methyl, Trifluoromethyl, Fluor oder Methoxy steht; |
| X und Y | insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H); |
| Z | für Wasserstoff steht; und |
| n | für die Zahl 0 oder 1 steht. |

Hierbei ist es besonders bevorzugt, dass

| | |
|---|---|
| $R^2$, $R^3$, $R^8$ und $R^9$ | für Wasserstoff stehen; |

W für Fluor steht;

X für Fluor steht;

Y für Methyl steht;

Z für Wasserstoff steht; und

n für die Zahl 0 oder 1 steht.

Handelt es sich bei der Substruktur der Formel (I-B) um die Substrukturformel (I-B-5), so ist es bevorzugt, dass

$R^{11}$ für Wasserstoff, Methyl, Ethyl, Propyl, Isopropyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ steht;

$R^8$ und $R^9$, jeweils unabhängig voneinander, für Wasserstoff, Methyl, Trifluoromethyl oder Phenyl stehen;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);

Z für Wasserstoff steht; und

n für die Zahl 0 oder 1 steht.

Hierbei ist es besonders bevorzugt, dass

$R^{11}$ für $CH_2CF_3$ oder Cyclopropyl steht;
$R^8$ und $R^9$ jeweils für Methyl stehen;
W für Fluor steht;
X für Methyl oder Fluor steht;
Y für für Methyl steht;
Z für Wasserstoff steht; und
n für die Zahl 0 oder 1 steht.

Handelt es sich bei der Substruktur der Formel (I-B) um die Substrukturformel (I-B-5), so ist es ebenfalls bevorzugt, dass

$R^{11}$ für Wasserstoff, Methyl, Ethyl, Propyl, Isopropyl, $CH_2C(CH_3)_3$, $CH_2CH(CH_3)_2$, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ steht;

$R^8$ und $R^9$, jeweils unabhängig voneinander, für Wasserstoff, Methyl, Trifluoromethyl oder Phenyl stehen;

W Wasserstoff oder Fluor steht;

X für Wasserstoff, Chlor, Fluor oder Methyl steht;

Y für Chlor, Brom, Methyl, Trifluoromethyl oderFluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es ebenfalls besonders bevorzugt, dass

$R^{11}$ für $CH_2C(CH_3)_3$, $CH_2CH(CH_3)_2$, $CH_2CF_3$ oder Cyclopropyl steht;

$R^8$ und $R^9$ jeweils für Methyl stehen;

W für Fluor steht;

X für Methyl oder Fluor steht;

Y für Methyl steht;

Z für Wasserstoff steht; und

n für die Zahl 0 oder 1 steht.

Handelt es sich bei der Substruktur der Formel (I-B) um die Substrukturformel (I-B-6), so ist es bevorzugt, dass

$R^1$ und $R^{10}$ jeweils unabhängig voneinander, für Wasserstoff, Methyl, Ethyl, Propyl, Isopropyl, $CH_2CH=CH_2$, $CH_2CCH$, $CH_2CF_3$, Cyclopropyl oder Cyclopropylmethyl stehen;
W Wasserstoff oder Fluor steht;
X für Wasserstoff, Chlor, Fluor oder Methyl steht;
Y für Chlor, Brom, Methyl, Trifluoromethyl oderFluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht; und
n für die Zahl 0 oder 1 steht.

Hierbei ist es besonders bevorzugt, dass

$R^1$ für Methyl, Ethyl oder $CH_2CH=CH_2$ steht;
$R^{10}$ für Methyl, Ethyl, Isopropyl, $CH_2CH=CH_2$, $CH_2CCH$ oder Cyclopropylmethyl stehen;
W für Fluor steht;
X für Fluor steht;
Y für Methyl steht;
Z für Wasserstoff steht; und
n für die Zahl 0 oder 1 steht.

Dritte Ausführungsform (I-C):

In einer dritten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-C) stehen

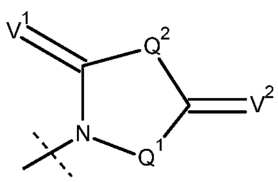

(I-C)

wobei

$V^1$ und $V^2$, jeweils unabhängig voneinander, für Sauerstoff oder Schwefel stehen;

$Q^1$ für Sauerstoff, Schwefel, $NR_1$ oder $CR_2R_3$ steht;

$Q^2$ $NR_{10}$ oder $CR_8R_9$ steht;

$R^1$ für Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{10}$ für Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^8$ und $R^9$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylthio$(C_1-C_6)$alkyl, Halogen$(C_1-C_6)$alkylthio$(C_1-C_6)$alkyl, $(C_1-C_6)$Alkoxy$(C_1-C_6)$alkylthioalkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$al-

kyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z, jeweils unabhängig voneinander,
für Wasserstoff, Fluor, Chlor, Brom, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

In einer bevorzugten Ausgestaltung dieser dritten Ausführungsform steht die Substruktur der Formel (I-C) für eine Substruktur, welche ausgewählt wird aus der Gruppe, bestehend aus

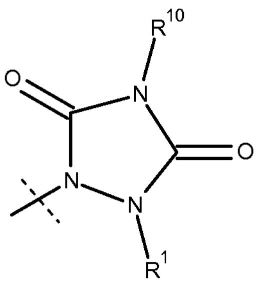
(I-C-1)

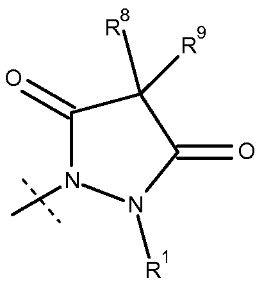
(I-C-2)

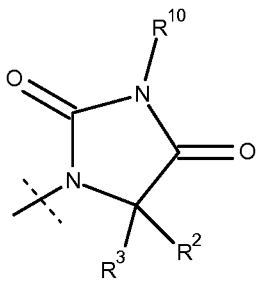
(I-C-3)

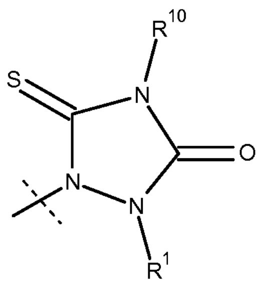
(I-C-4)

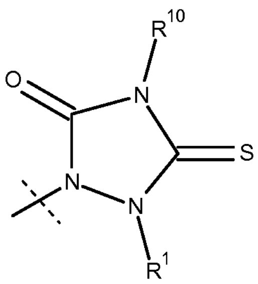
(I-C-5)

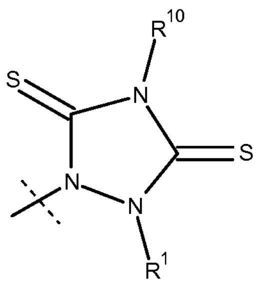
(I-C-6)

wobei

$R^1$ für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, oder Carbonyloxy steht, wobei

die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; und

$R^{10}$ für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können steht; oder

für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; und

$R^2$, $R^3$, $R^8$ und $R^9$, jeweils unabhängig voneinander,

für Wasserstoff, Cyano, Halogen oder Nitro steht; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können; oder

für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; oder $R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder $R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Fluor steht;

X und Y, jeweils unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Hierbei ist es bevorzugt, dass

$R^1$ und $R^{10}$, jeweils unabhängig voneinander, für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, $(C_1-C_6)$Alkoxy$(C_1-C_6)$alkyl oder Phenyl$(C_1-C_3)$alkyl stehen, wobei die vorgenannten Reste jeweils gegebenenfalls substituiert sein können; oder für gegebenenfalls substituiertes gesättigtes oder ungesättigtes $(C_3-C_6)$Cycloalkyl oder gegebenenfalls substituiertes Phenyl steht;

$R^2$, $R^3$, $R^8$ und $R^9$, jeweils unabhängig voneinander, für Wasserstoff stehen; oder für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls substituiert sein können; oder für gegebenenfalls substituiertes gesättigtes oder ungesättigtes $(C_3-C_6)$Cycloalkyl, oder gegebenenfalls substituiertes Phenyl steht; oder

W für Wasserstoff oder Fluor steht;

X und Y, jeweils unabhängig voneinander,

für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Bevorzugt steht die Substruktur der Formel (I-C) für die Substruktur (I-C-1)

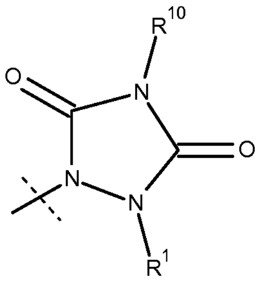

(I-C-1)

wobei

$R^1$ und $R^{10}$, jeweils unabhängig voneinander, für $(C_1\text{-}C_4)$Alkyl, $(C_1\text{-}C_4)$Halogenalkyl oder $(C_2\text{-}C_4)$Alkenyl stehen; oder für gesättigtes oder ungesättigtes $(C_3\text{-}C_6)$Cycloalkyl, $(C_3\text{-}C_6)$Cycloalkyl$(C_1\text{-}C_2)$alkyl, Phenyl oder Phenyl$(C_1\text{-}C_2)$alkyl stehen, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), $(CF_3,H)$;

Z für Wasserstoff steht; und

n für die Zahl 0 oder 1 steht.

Ebenfalls bevorzugt steht die Substruktur der Formel (I-C) für eine Substruktur, welche ausgewählt wird aus der Gruppe, bestehend aus

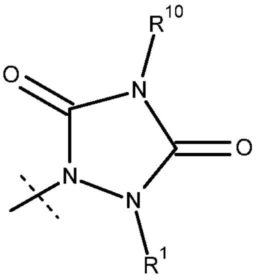

(I-C-1)

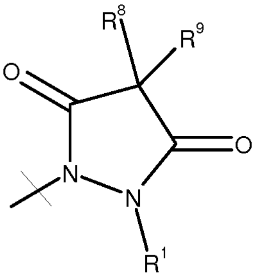

(I-C-2)

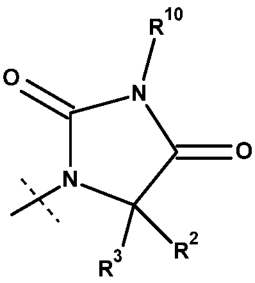

(I-C-3)

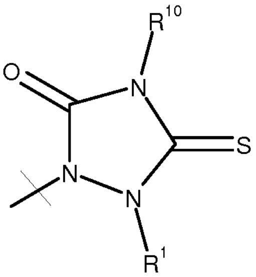

(I-C-5)

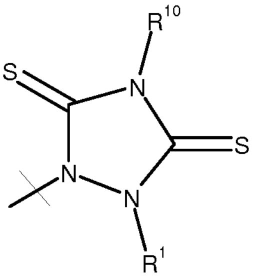

(I-C-6)

wobei

$R^1$ und $R^{10}$, jeweils unabhängig voneinander,
für $(C_1\text{-}C_4)$Alkyl, $(C_2\text{-}C_4)$Halogenalkyl, $(C_1\text{-}C_3)$Alkoxy$(C_1\text{-}C_4)$alkyl oder $(C_2\text{-}C_4)$Alkenyl stehen; oder
für gesättigtes oder ungesättigtes $(C_3\text{-}C_6)$Cycloalkyl, $(C_3\text{-}C_6)$Cycloalkyl$(C_1\text{-}C_2)$alkyl, Phenyl oder Phenyl$(C_1\text{-}C_2)$alkyl stehen, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können;

$R^2$, $R^3$, $R^8$ und $R^9$, jeweils unabhängig voneinander,
für Wasserstoff stehen; oder
für $(C_1\text{-}C_6)$Alkyl, $(C_2\text{-}C_6)$Alkenyl, $(C_2\text{-}C_6)$Alkinyl, Halogen$(C_1\text{-}C_6)$alkyl oder Phenyl$(C_1\text{-}C_3)$alkyl steht, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können; oder
für $(C_3\text{-}C_6)$Cycloalkyl oder Phenyl steht, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können; oder
$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituiertes Cyclopropyl-, Cyclobutyl-, Cyclopentyl- oder Cyclohexylring bilden können; oder
$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy

oder gegebenenfalls durch Methyl, Fluor, Chlor, Cyano substituiertes $(C_3-C_6)$Cycloalkyl, substituiertes Cyclopropyl-, Cyclobutyl-, Cyclopentyl- oder Cyclohexylring bilden können;

W Wasserstoff oder Fluor steht;

X für Wasserstoff, Chlor, Fluor oder Methyl steht;

Y für Chlor, Brom, Methyl, Trifluoromethyl oder Fluor steht;
Insbesondere wobei X und Y für folgende Kombinationen (Y,X) stehen: (Me, F), (Me,H), (Me,Cl), (Me,Me), (Cl,Cl), (Cl,F), (Cl,H), (Me,H), (Br,H), (Br,F), (F,F), ($CF_3$,H);

Z für Wasserstoff steht; und

n für die Zahl 0 oder 1 steht.

Handelt es sich bei der Substruktur der Formel (I-C) um die Substrukturformel (I-C-1), so ist es bevorzugt, dass

$R^1$ für Methyl, Ethyl, Propyl, Isopropyl, Butyl, Tertbutyl, Isobutyl, 2-Butyl, Allyl, $CH_2CF_3$ steht; oder, für gesättigtes oder ungesättigtes Cyclopropyl, Cyclobutyl, Cyclopentyl, Cyclohexyl, Cyclopentenyl, Cyclohexenyl, Cyclopropylmethyl, Phenyl, Benzyl stehen, wobei die vorgenannten Reste jeweils gegebenenfalls einfach oder mehrfach durch Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können;

$R^{10}$ für Methyl, Ethyl, Propyl, Isopropyl, Butyl, Tertbutyl, Isobutyl, 2-Butyl, Allyl, Cyclopropyl, Cyclopropylmethyl oder ein gegebenenfalls einfach oder mehrfach durch Halogen, Methyl, Trifluoromethyl, Cyclopropyl, Cyano substituiertes Phenyl steht;
steht;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Cyclopentyl, Cyclohex-2-en-1-yl, Methyl, Ethyl, $CH_2CH(CH_3)_2$, $CH(CH_3)CH_2CH_3$, $CH(CH_3)_2$, $CH_2$-Cyclopropyl, $CH_2CH=CH_2$, Benzyl, 4-Cyano-2,5-difluoro-phenyl, 3-(2,2,2-Trifluoroethylsulfinyl)-4-methyl-phenyl, 3-(2,2,2-Trifluoroethylsulfanyl)-4-methyl-phenyl oder (2-chlorophenyl)methyl steht;

$R^{10}$ für Methyl, Ethyl, $CH_2CH=CH_2$, $CH(CH_3)_2$, $C(CH_3)_3$, Cyclopropyl, Phenyl oder 3-(Trifluoromethyl)phenyl steht;

W für Fluor steht;

X für Wasserstoff, Fluor oder Methyl steht;

Y für Brom, Chlor, Fluor, Methyl, Methoxy oder $CF_3$ steht;

Z für Wasserstoff steht; und

n für die Zahl 0 oder 1 steht.

Handelt es sich bei der Substruktur der Formel (I-C) um die Substrukturformel (I-C-1), so ist es ebenfalls bevorzugt, dass

$R^1$ für Methyl, Ethyl, Propyl, Isopropyl, Butyl, Tertbutyl, Isobutyl, 2-Butyl, Allyl, $CH_2CF_3$ steht; oder, für gesättigtes oder ungesättigtes Cyclopropyl, Cyclobutyl, Cyclopentyl, Cyclohexyl, Cyclopentenyl, Cyclohexenyl, Cyclopropylmethyl, Phenyl oder Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl, Trifluoroethylsulfinyl oder gegebenenfalls durch Methyl, Fluor, Chlor oder Cyano substituiertes Cyclopropyl substituiert sein können;

$R^{10}$ für Methyl, Ethyl, Propyl, Isopropyl, Butyl, Tertbutyl, Isobutyl, 2-Butyl, $CH_2CHF_2$, $CH_2CH_2CH_2OCH_3$, Allyl, Cyclopropyl, Cyclohexyl, Cyclopropylmethyl oder ein gegebenenfalls einfach oder mehrfach durch Halogen, Methyl, Trifluoromethyl, Cyclopropyl, Cyano substituiertes Phenyl steht;

W Wasserstoff oder Fluor steht;

X für Wasserstoff, Chlor, Fluor oder Methyl steht;

Y für Chlor, Brom, Methyl, Trifluoromethyloder Fluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es ebenfalls besonders bevorzugt, dass

$R^1$ für Cyclopentyl, Cyclohex-2-en-1-yl, Methyl, Ethyl, Propyl, $CH_2CH(CH_3)_2$, $CH(CH_3)CH_2CH_3$, $CH(CH_3)_2$, $C(CH_3)_3$, Cyclopropylmethyl, $CH_2CH=CH_2$, $CH_2CF_3$, Benzyl, 4-Cyano-2,5-difluoro-phenyl, 3-(2,2,2-Trifluoroethylsulfinyl)-4-methyl-phenyl, 3-(2,2,2-Trifluoroethylsulfanyl)-4-methyl-phenyl oder (2-Chlorophenyl)methyl steht;

$R^{10}$ für Methyl, Ethyl, Propyl, $CH_2CH=CH_2$, $CH(CH_3)_2$, $C(CH_3)_3$, $CH_2CHF_2$, $CH_2CH_2CH_2OCH_3$, Cyclopropyl, Cyclohexyl, Phenyl, 3-Chlorphenyl oder 3-(Trifluoromethyl)phenyl steht;

W für Fluor steht;

X für Wasserstoff, Fluor oder Methyl steht;

Y für Brom, Chlor, Fluor, Methyl oder $CF_3$ steht;

X und Y insbesondere für folgende Kombinationen (Y,X) stehen: (Me, F), (Me,H), (Me,Me), (Cl,F), (Cl,H), (Br,H), (Br,F), (F,F), ($CF_3$,H);

Z für Wasserstoff steht; und

n für die Zahl 0 oder 1 steht.

Handelt es sich bei der Substruktur der Formel (I-C) um die Substrukturformel (I-C-2), so ist es bevorzugt, dass

$R^1$ für Methyl, Ethyl, Propyl, Isopropyl, Butyl, Tertbutyl, Isobutyl, 2-Butyl, Allyl, $CH_2CF_3$ steht; oder, für gesättigtes oder ungesättigtes Cyclopropyl, Cyclobutyl, Cyclopentyl, Cyclohexyl, Cyclopentenyl, Cyclohexenyl, Cyclopropylmethyl, Phenyl, Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können;

$R^8$ und $R^9$ unabhängig voneinander für Wasserstoff, Methyl, Ethyl, Trifluoromethyl oder Phenyl stehen; oder bilden einen Cyclobutylring;

W Wasserstoff oder Fluor steht;

X für Wasserstoff, Chlor, Fluor oder Methyl steht;

Y für Chlor, Brom, Methyl, Trifluoromethyl oderFluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Methyl, Benzyl oder Phenyl steht;

$R^8$ und $R^9$ jeweils für Methyl oder Ethyl stehen; oder bilden einen Cyclobutylring;

W für Fluor steht;

X für Wasserstoff, Fluor oder Methyl steht;

Y für Brom, Chlor, Fluor, Methyl oder $CF_3$ steht;

Z für Wasserstoff steht; und

n für die Zahl 0 oder 1 steht.

Handelt es sich bei der Substruktur der Formel (I-C) um die Substrukturformel (I-C-3), so ist es bevorzugt, dass

$R^{10}$ für Methyl, Ethyl, Propyl, Isopropyl, Butyl, Tertbutyl, Isobutyl, 2-Butyl, $CH_2CHF_2$, $CH_2CH_2CH_2OCH_3$, Allyl, Cyclopropyl, Cyclohexyl, Cyclopropylmethyl oder ein gegebenenfalls einfach oder mehrfach durch Halogen, Methyl, Trifluoromethyl, Cyclopropyl, Cyano substituiertes Phenyl steht;
$R^2$ und $R^3$ unabhängig voneinander für Wasserstoff, Methyl, Ethyl, Trifluoromethyl oder Phenyl stehen; oder bilden einen Cyclopropyl- oder einen Cyclobutylring;
W Wasserstoff oder Fluor steht;
X für Wasserstoff, Chlor, Fluor oder Methyl steht;
Y für Chlor, Brom, Methyl, Trifluoromethyl oder Fluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht; und
n für die Zahl 0 oder 1 steht.

Hierbei ist es besonders bevorzugt, dass

$R^{10}$ für Methyl oder Cyclopropyl steht;
$R^2$ und $R^3$ unabhängig voneinander für Wasserstoff oder Methyl stehen;
W für Fluor steht;
X für Fluor steht;

Y für Methyl steht;
Z für Wasserstoff steht; und
n für die Zahl 0 oder 1 steht.

Handelt es sich bei der Substruktur der Formel (I-C) um die Substrukturformel (I-C-5), so ist es bevorzugt, dass

$R^1$ für Methyl, Ethyl, Isopropyl, Tertbutyl, Isobutyl oder Trifluoromethyl steht; oder, für Cyclopropyl, Cyclobutyl, Cyclopropylmethyl, Phenyl oder Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch gegebenenfalls einfach durch Methyl, Fluor, Chlor oder Cyano substituiertes Cyclopropyl substituiert sein können;

$R^{10}$ für Methyl, Ethyl, Propyl, Isopropyl, Tertbutyl, Isobutyl, Trifluoromethyl oder Allyl steht; oder, für gesättigtes oder ungesättigtes Cyclopropyl, Cyclobutyl, Cyclopropylmethyl, Phenyl oder Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor oder Cyano substituiertes Cyclopropyl substituiert sein können;

W Wasserstoff oder Fluor steht;

X für Wasserstoff, Chlor, Fluor oder Methyl steht;

Y für Chlor, Brom, Methyl, Trifluoromethyl oder Fluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Methyl steht;

$R^{10}$ für Cyclopropyl steht;

W für Fluor steht;

X für Fluor steht;

Y für Methyl steht;

Z für Wasserstoff steht; und

n für die Zahl 0 oder 1 steht.

Handelt es sich bei der Substruktur der Formel (I-C) um die Substrukturformel (I-C-6), so ist es bevorzugt, dass

$R^1$ für Methyl, Ethyl, Isopropyl, Tertbutyl, Isobutyl oder Trifluoromethyl steht; oder, für Cyclopropyl, Cyclobutyl, Cyclopropylmethyl, Phenyl oder Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch gegebenenfalls einfach durch Methyl, Fluor, Chlor oder Cyano substituiertes Cyclopropyl substituiert sein können;

$R^{10}$ für Methyl, Ethyl, Propyl, Isopropyl, Tertbutyl, Isobutyl, Trifluoromethyl oder Allyl steht; oder, für gesättigtes oder ungesättigtes Cyclopropyl, Cyclobutyl, Cyclopropylmethyl, Phenyl oder Benzyl stehen, wobei die vorgenannten Reste jeweils gegebenenfalls einfach oder mehrfach durch Cyano, Halogen, Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor oder Cyano substituiertes Cyclopropyl substituiert sein können;

W Wasserstoff oder Fluor steht;

X für Wasserstoff, Chlor, Fluor oder Methyl steht;

Y für Chlor, Brom, Methyl, Trifluoromethyl oder Fluor steht;

X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);

Z für Wasserstoff steht; und

n für die Zahl 0 oder 1 steht.

Hierbei ist es besonders bevorzugt, dass

$R^1$ für Methyl steht;
$R^{10}$ für Cyclopropyl steht;
W für Fluor steht;
X für Fluor steht;
Y für Methyl steht;
Z für Wasserstoff steht; und
n für die Zahl 0 oder 1 steht.

Vierte Ausführungsform (I-D):

In einer vierten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-D) stehen

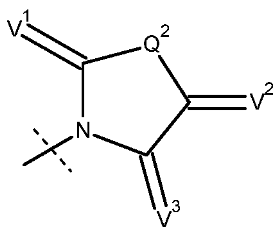

(I-D)

wobei

$V^1$, $V^2$ und $V^3$, jeweils unabhängig voneinander, für Sauerstoff; Schwefel, $NR_{11}$ oder ein Salz von $NR_{11}$ stehen;
$Q^2$ $NR^{10}$ oder $CR^8R^9$ steht;
$R^{10}$ für Wasserstoff, Cyano oder Nitro steht; oder für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)al-

kylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, Arylcarbonyl, ($C_1$-$C_6$)Alkoxycarbonyl, Aryloxycarbonyl, ($C_1$-$C_6$)Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht;

$R^{11}$ für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, ($C_1$-$C_6$)Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylami-

nocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^8$ und $R^9$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylthio($C_1$-$C_6$)alkyl, Halogen($C_1$-$C_6$)alkylthio($C_1$-$C_6$)alkyl, ($C_1$-$C_6$)Alkoxy($C_1$-$C_6$)alkylthioalkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass

im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z, jeweils unabhängig voneinander,

für Wasserstoff, Fluor, Chlor, Brom, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen; oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

In einer bevorzugten Ausgestaltung dieser vierten Ausführungsform steht die Substruktur der Formel (I-D) für eine Substruktur, welche ausgewählt wird aus der Gruppe, bestehend aus

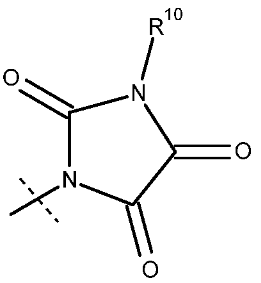
(I-D-1)

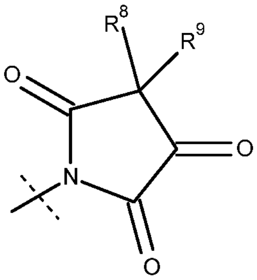
(I-D-2)

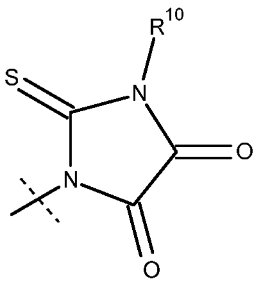
(I-D-3)

wobei

$R^{10}$ für Wasserstoff steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^8$ und $R^9$, jeweils unabhängig voneinander,

für Wasserstoff, Cyano, Halogen oder Nitro steht; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können ; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; oder

$R^8$ und $R^9$ gemeinsam mit dem Atom, an das sie gebunden sind, einen gegebenenfalls gleich oder

verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Fluor steht;

X und Y, jeweils unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Hierbei ist es bevorzugt, dass

$R^{10}$ für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_2$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl oder gegebenenfalls substituiertes Phenyl steht;

$R^8$ und $R^9$, jeweils unabhängig voneinander,
für Wasserstoff stehen; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls substituiert sein können; oder
für gegebenenfalls substituiertes gesättigtes oder ungesättigtes ($C_3$-$C_6$)Cycloalkyl, oder gegebenenfalls substituiertes Phenyl steht; oder

W für Wasserstoff oder Fluor steht;

X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

Bevorzugt steht die Substruktur der Formel (I-D) für die Substruktur (I-D-1)

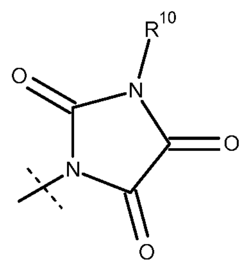

(I-D-1)

wobei

$R^{10}$ für Wasserstoff, Methyl, Ethyl, Propyl, Isopropyl, Cyclopropylmethyl, $CH_2CF_3$, $CH_2CHF_2$, $CH(CH_3)CF_3$,

$CH_2CH_2CF_3$, $CH_2CH_2CHF_2$ oder gegebenenfalls durch Methyl, Fluor, Chlor, Cyano substituiertes Cyclopropyl steht;

W für Wasserstoff oder Fluor steht;

X für Wasserstoff, Chlor, Fluor oder Methyl steht;

Y für Chlor, Brom, Methyl, Trifluoromethyl, Fluor oder Methoxy steht;

X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);

Z für Wasserstoff steht; und

n für die Zahl 0 oder 1 steht.

Ebenfalls bevorzugt steht die Substruktur der Formel (I-D) für eine Substruktur, welche ausgewählt wird aus der Gruppe, bestehend aus

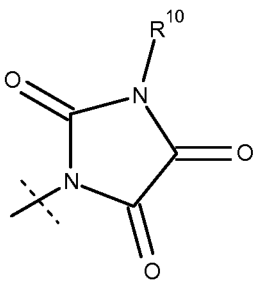

(I-D-1)

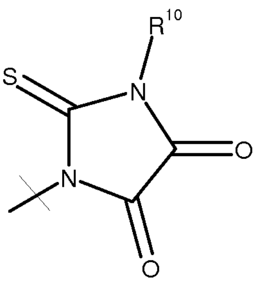

(I-D-3)

wobei

$R^{10}$ für Wasserstoff, Methyl, Ethyl, Propyl, Isopropyl, tert-Butyl, Cyclopropylmethyl, $CH_2CH(CH_3)_2$, $CH_2C(CH_3)_3$, $CH_2CF_3$, $CH_2CHF_2$, $CH(CH_3)CF_3$, $CH_2CH_2CF_3$, $CH_2CH_2CHF_2$ oder gegebenenfalls durch Methyl, Fluor, Chlor, Cyano substituiertes Cyclopropyl oder gegebenenfalls durch Methyl, Fluor, Chlor, Cyano substituiertes Cyclohexyl oder gegebenenfalls durch Methyl, Fluor, Chlor, Cyano substituiertes Cyclopropylmethyl steht;

W für Wasserstoff oder Fluor steht;

X für Wasserstoff, Chlor, Fluor oder Methyl steht;

Y für Chlor, Brom, Methyl, Trifluoromethyl oderFluor steht;

X und Y insbesondere für folgende Kombinationen (Y,X) stehen: (Me, F), (Me,H), (Me,Cl), (Me,Me), (Cl,Cl), (Cl,F), (Cl,H), (Me,H), (Br,H), (Br,F), (F,F), ($CF_3$,H);

Z für Wasserstoff steht; und

n für die Zahl 0 oder 1 steht.

Handelt es sich bei der Substruktur der Formel (I-D) um die Substrukturformel (I-D-1), so ist es bevorzugt, dass

$R^{10}$ für Wasserstoff, Methyl, Ethyl, $CH_2CF_3$, $CH_2CHF_2$, $CH(CH_3)CF_3$, $CH_2CH_2CF_3$, $CH_2CH_2CHF_2$, Cyclopropyl oder Cyclopropylmethyl steht;

W Wasserstoff oder Fluor steht;

X für Wasserstoff, Chlor, Fluor oder Methyl steht;

Y für Chlor, Brom, Methyl, Trifluoromethyl, Fluor oder Methoxy steht;
X und Y insbesondere für folgende Kombinationen (Y,X) stehen: (Me, F), (Me,H), (Me,Cl), (Me,Me), (Cl,Cl), (Cl,F), (MeO,F), (MeO,H), (Cl,H), (Me,H), (Br,H), (Br,F), (F,F), ($CF_3$,H);
Z für Wasserstoff steht;
n für die Zahl 0 oder 1 steht.

Hierbei ist es besonders bevorzugt, dass

$R^{10}$ für $CH_2CHF_2$ oder Cyclopropyl steht;

W für Fluor steht;

X für Wasserstoff oder Fluor steht;

Y für Methyl steht;

Z für Wasserstoff steht; und

n für die Zahl 0 oder 1 steht.

Handelt es sich bei der Substruktur der Formel (I-D) um die Substrukturformel (I-D-1), so ist es ebenfalls bevorzugt, dass

$R^{10}$ für Wasserstoff, Methyl, Ethyl, $CH_2CF_3$, $CH_2CHF_2$, $CH(CH_3)CF_3$, $CH_2CH_2CF_3$, $CH_2CH_2CHF_2$, Cyclopropyl, Cyclohexyl oder Cyclopropylmethyl steht;
W Wasserstoff oder Fluor steht;
X für Wasserstoff, Chlor, Fluor oder Methyl steht;
Y für Chlor, Brom, Methyl, Trifluoromethyl oder Fluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht;
n für die Zahl 0 oder 1 steht.

Hierbei ist es ebenfalls besonders bevorzugt, dass

$R^{10}$ für $CH_2CHF_2$, Cyclopropyl oder Cyclohexyl steht;
W für Fluor steht;
X für Wasserstoff, Methyl oder Fluor steht;
Y für Methyl steht;
X und Y insbesondere für folgende Kombinationen (Y,X) stehen: (Me, F), (Me,H), (Me,Me);
Z für Wasserstoff steht; und
n für die Zahl 0 oder 1 steht.

Handelt es sich bei der Substruktur der Formel (I-D) um die Substrukturformel (I-D-3), so ist es bevorzugt, dass

$R^{10}$ für Wasserstoff, Methyl, Ethyl, tert-Butyl, $CH_2CH(CH_3)_2$, $CH_2C(CH_3)_3$, $CH_2CF_3$, $CH_2CHF_2$, $CH(CH_3)CF_3$, $CH_2CH_2CF_3$, $CH_2CH_2CHF_2$, Cyclopropyl oder Cyclopropylmethyl steht;
W Wasserstoff oder Fluor steht;
X für Wasserstoff, Chlor, Fluor oder Methyl steht;
Y für Chlor, Brom, Methyl, Trifluoromethyl oder Fluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht;
n für die Zahl 0 oder 1 steht.

Hierbei ist es besonders bevorzugt, dass

$R^{10}$ für tert-Butyl, $CH_2CH(CH_3)_2$, $CH_2C(CH_3)_3$ oder $CH_2CF_3$ steht;

W für Fluor steht;

X für Methyl oder Fluor steht;

Y für Methyl steht;

X und Y insbesondere für folgende Kombinationen (Y,X) stehen: (Me, F), (Me,Me);

Z für Wasserstoff steht; und

n für die Zahl 0 oder 1 steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.
Erfindungsgemäß bevorzugt werden die Verbindungen der Formel (I), in welchen eine Kombination der vorstehend als bevorzugt aufgeführten Bedeutungen vorliegt.
Erfindungsgemäß besonders bevorzugt werden die Verbindungen der Formel (I), in welchen eine Kombination der vorstehend als besonders bevorzugt aufgeführten Bedeutungen vorliegt.
Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.
Gegebenenfalls substituierte Reste können, sofern nichts anderes angegeben ist, einfach oder mehrfach substituiert sein, wobei bei Mehrfachsubstitutionen die Substituenten gleich oder verschieden sein können.
Im Rahmen der vorliegenden Erfindung steht Halogen für Fluor, Chlor, Brom und Jod, besonders bevorzugt für Fluor, Chlor und Brom, und ganz besonders bevorzugt für Fluor und Chlor.
Ferner steht Alkyl für geradkettiges oder verzweigtes $C_1$- bis $C_8$-Alkyl, bevorzugt geradkettiges oder verzweigtes $C_1$- bis $C_6$-Alkyl, weiter bevorzugt geradkettiges oder verzweigtes $C_1$- bis $C_4$-Alkyl, insbesondere Methyl und Ethyl.
Alkoxy steht für geradkettiges oder verzweigtes $C_1$- bis $C_8$-Alkoxy, bevorzugt geradkettiges oder verzweigtes $C_1$- bis $C_6$-Alkoxy, weiter bevorzugt geradkettiges oder verzweigtes $C_1$- bis $C_4$-Alkoxy, insbesondere Methoxy.
Halogenalkyl und Halogenalkoxy ergeben sich aus substituierten Alkyl- bzw. Alkoxyresten gemäß der vorstehenden Definition.
Alkylreste in Cycloalkyl, Alkoxycarbonyl, Alkylthioalkyl, Alkylsulfinylalkyl, Phenylalkyl, Hetarylalkyl und Alkylsulfonylalkyl ergeben sich ebenfalls aus der vorstehenden Definition von Alkyl.

## Herstellverfahren

Die Verbindungen der allgemeinen Formel (I) lassen sich in Verbindungen mit n=0 (Ia) und n=1 (Ib) unterteilen und können nach folgenden Schemata hergestellt werden.
Die Verbindungen der allgemeinen Formel (I) lassen sich in Verbindungen mit n=0 (Ia) und n=1 (Ib) unterteilen und können prinzipiell nach den allgemeinen Verfahren V1, V2, V3 und V3' hergestellt werden.

## Verfahren V1

Unter Verfahren V1 lassen sich alle Methoden zusammenfassen, die -meistens in einem mehrstufigen Prozess-einen Aufbau des 5-gliedrigen Ringes ermöglichen, insbesondere ausgehend aus den Anilinen der allgemeinen Formel (IVa), nach folgendem Schema,

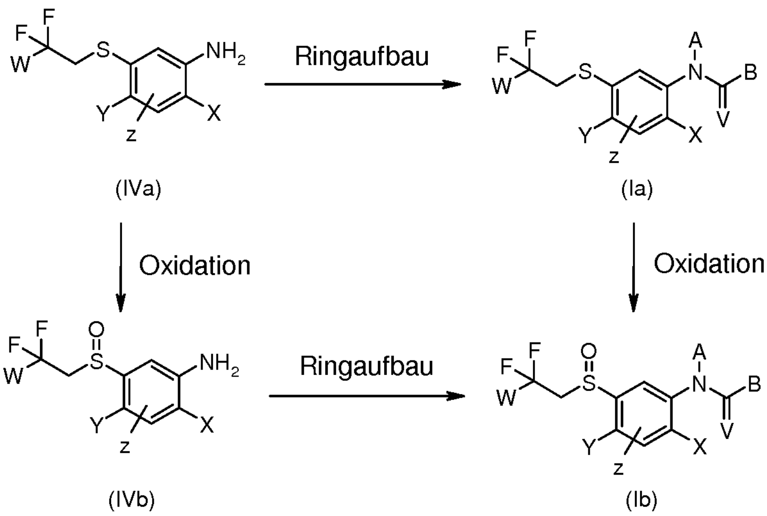


(IVa) (Ia)

(IVb) (Ib)

wobei X, Y, Z, A, B und V die oben angegebene Bedeutung haben.

Durch Oxidation der Thioether der allgemeinen Formel (Ia) nach literaturbekannten Methoden können die Sulfoxide der allgemeinen Formel (Ib) erhalten werden.

Alternativ können einige der in Verfahren V1 beschriebenen Methoden auch ausgehend von Sulfoxiden der allgemeinen Formel (IVb) zu den Sulfoxiden der allgemeinen Formel (Ib) angewandt werden. Die Sulfoxide der Formel (IVb) können aus den Sulfiden (IVa) nach literaturbekannten Methoden hergestellt werden.

Verfahren V1 eignet sich insbesondere zur Darstellung der Ausführungsformen I-A bis I-D.

**Verfahren V2**

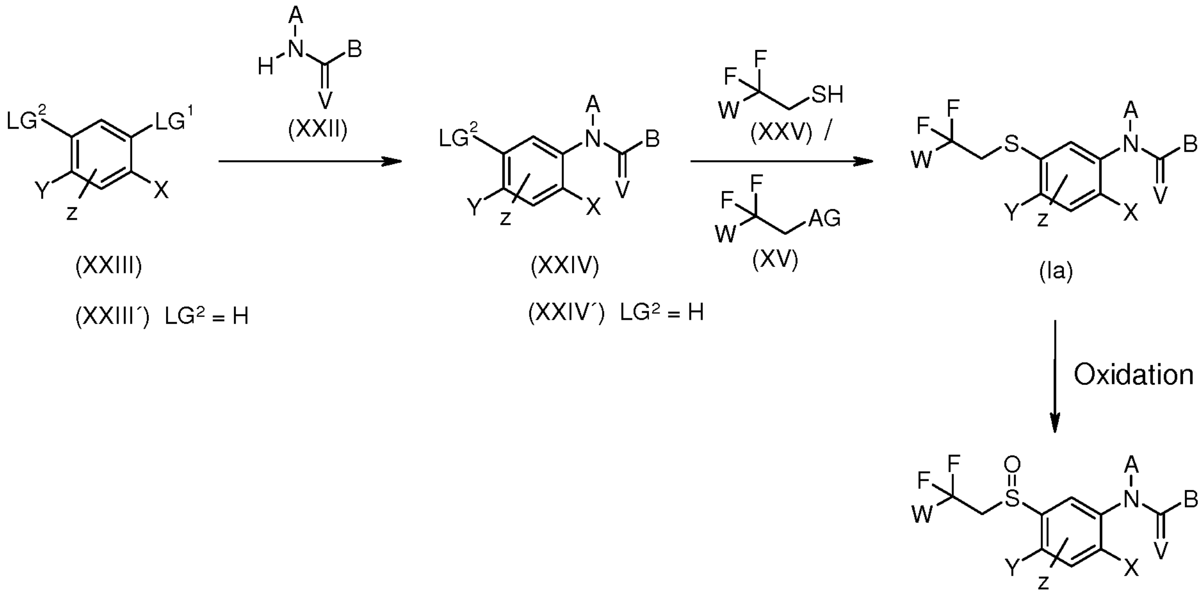


(XXII) (XXV) / (XV)

(XXIII) (XXIV) (Ia)

(XXIII´) $LG^2 = H$ (XXIV´) $LG^2 = H$

(Ib)

wobei Z, A, B, V und W die oben angegebene Bedeutung haben, X steht für Wasserstoff oder eine elektronenziehende Gruppe (insbesondere Nitro, Chlor, Fluor, Cyano), Y stellt elektronenziehende Substituenten (insbesondere Nitro, Chlor, Fluor, Cyano) dar, $LG^1$ steht für typische Abgangsgruppen in nucleophilen Substitutionsreaktionen (insbesondere Fluorid, Chlorid) und $LG^2$ kann für Wasserstoff oder für typische Abgangsgruppen in nucleophilen Substitutionsreaktionen (insbesondere Fluorid, Chlorid) stehen.

Die Umsetzung von Verbindungen der allgemeinen Formel (XXIII) mit heterocyclischen Verbindungen der

allgemeinen Formel (XXII), meistens unter basischen Reaktionsbedingungen wie zum Beispiel in US 6906006 und DE 19500439 für Triazolinone, WO 2010/0119194 für Hydantoine, DE 4431218 für Pyrimidin(thi)one, WO 2009/012275 und WO 2008/155034 für Pyridone oder DE 19528305 für Uracile beschrieben, liefert die Verbindungen der allgemeinen Formel (XXIV). Durch erneute nukleophile aromatische Substitution mit Thiolen der allgemeinen Formel (XXV) können die Thioether der Formel (Ia) hergestellt werden. Geeignete Reaktionsbedingungen für solche Reaktionen werden in WO 2007/131680 und WO 2008/086226 beschrieben.

Durch Oxidation der Thioether der allgemeinen Formel (Ia) nach literaturbekannten Methoden können die Sulfoxide der allgemeinen Formel (Ib) erhalten werden.

Verbindungen der Formel (XXIII) in denen $LG^2$ für Wasserstoff steht werden als (XXIII') genannt und können auf ähnlicher Weise wie oben dargestellt mit Verbindungen der Formel (XXII) umgesetzt werden, in diesem Fall zu Verbindungen der Formel (XXIV').

Verbindungen der Formel (XXIV') sind zum Teil kommerziell erhältlich.

Die Verbindungen der Formel (XXIV') können in einem mehrstufigen Prozess zu den erfindungsgemäßen Verbindungen (Ia) umgesetzt werden. Zu den benötigten Schritten gehören Chlorsulfonierung, Reduktion und Alkylierung mit Haloalkylelektrophilen der Formel (XV), alle möglich nach literaturbekannten Methoden. Die Chlorsulfonierung der Verbindungen (XXIV') mit Chlorsulfonsäure liefert die entsprechenden Sulfonylchloride und diese können in deren Disulfide mit literaturbekannten Methoden wie zum Beispiel Eisen in Salzsäure oder Iodid überführt werden. Die Umsetzung der Disulfide mit Haloalkylelektrophilen der Formel (XV) liefert die Sulfide (Ia).

Durch Oxidation der Thioether der allgemeinen Formel (Ia) nach literaturbekannten Methoden können die Sulfoxide der allgemeinen Formel (Ib) erhalten werden.

Hierbei stellen $LG^1$ und $LG^2$ typische Abgangsgruppen in nucleophilen Substitutionsreaktionen wie zum Beispiel Fluorid oder Chlorid dar, X und Y stellen in Verfahren V3 elektronenziehende Substituenten wie zum Beispiel Nitro, Chlorid, Fluorid, Cyano dar.

Verfahren V2 eignet sich insbesondere zur Darstellung der Ausführungsformen I-A bis I-D, bei denen X für Wasserstoff oder elektronenziehende Substituenten (insbesondere Nitro, Chlorid, Fluorid, Cyano) und Y für elektronenziehende Substituenten (insbesondere Nitro, Chlorid, Fluorid, Cyano) stehen.

__Verfahren V3 & Verfahren V3'__

Alternativ können die Verbindungen der allgemeinen Formel (Ia) nach **Verfahren V3 und V3'** hergestellt werden, wie im folgendem Schema dargestellt,

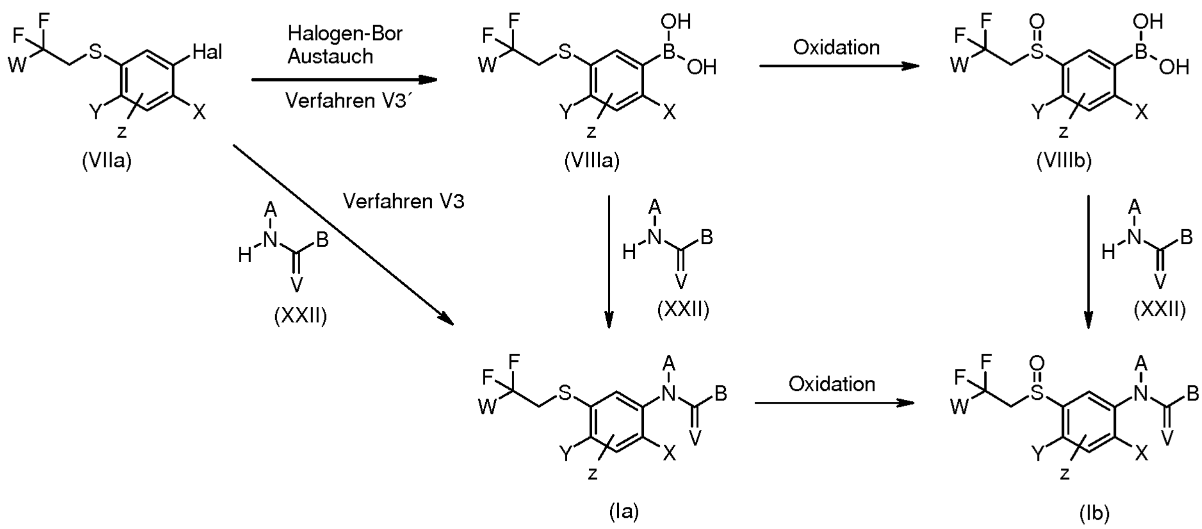


wobei X, Y, Z, W, A, B und V die oben angegebene Bedeutung haben und Hal steht für Halogen (bevorzugt Chlor, Brom Iod).

**Verfahren V3**

Laut Verfahren V3 können Verbindungen der allgemeinen Formel (Ia) nach literaturbekannten Methoden durch Umsetzung von Arylhalogeniden der allgemeinen Formel (VIIa) mit heterocyclischen Verbindungen der allgemeinen Formel (XXII) hergestellt werden. Bevorzugt findet die Umsetzung durch Übergangsmetall-Katalyse oder -Vermittlung

statt. Beispielhafte Reaktionsbedingungen sind in der Literatur zahlreich bekannt, zum Beispiel in WO 2006/117657 A1, in US 2010/99725 A1, in WO 2010/47956 A1, in Chem. Pharm. Bull. 1997, Vol. 45, No. 4, 719-721, in J. Amer. Chem. Soc. 2003, Vol. 125, No. 37, 11253-11258 oder auch in Bull. Korean Chem. Soc. 2010, Vol. 31, No. 8, 2143-2146. Bevorzugt werden Kupfer oder Kupfersalze, zum Beispiel Kupfer(I)-iodid, Kupfer(I)-oxid, Kupfer(I)-triflat oder Kupfer(II)-triflat, als Katalysator verwendet, häufig in Gegenwart eines Liganden, zum Beispiel Diamin-Liganden wie *N,N'*-Dimethylethylendiamin, *N,N*-Dimethylethylendiamin oder *trans-N,N'*-Dimethyl-1,2-cyclohexandiamin. Eine Übersicht findet sich zum Beispiel in Chem. Sei. 2010, Vol. 1, 13-31. Alternativ können 1,3-Diketone, wie z. B. 2,4-Pentandion, 2,2,6,6-Tetramethyl-3,5-heptandion oder Dibenzoylmethan, Aminosäuren, wie z.B. L-Prolin oder Glycin oder andere Verbindungen wie 8-Hydroxychinolin (Tetrahedron Lett. 2009, Vol. 50, 7293-7296), Dibenzylidenaceton, Bipyridin oder Phenanthrolin Verwendung finden. In der Regel wird die Umsetzung unter Anwesenheit einer Base, häufig Carbonat- oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid durchgeführt. Desweiteren können Additive wie z. B. Kaliumiodid, Cäsiumfluorid oder andere Salze Einsatz finden.

Alternativ lassen sich derartige Umsetzungen unter Palladiumkatalyse durchführen, etwa durch Einsatz von Katalysatoren wie zum Beispiel Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl, 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid.

Verfahren V3 ist insbesondere zur Darstellung der Ausführungsformen I-A-1, I-A-2, I-A-3, I-A-4 und I-C-1 geeignet.

**Verfahren V3'**

Verbindungen der allgemeinen Formel (Ia) können alternativ nach Verfahren V3' durch Umsetzung von Boronsäuren der allgemeinen Formel (VIIIa) mit heterocyclischen Verbindungen der allgemeinen Formel (XXII) hergestellt werden.

In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel mit Molekularsieb) statt. Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N,N*-Dimethylformamid, Tetrahydrofuran, Dioxan, Acetonitril, Essigester oder Toluol verwendet. In der Literatur werden zahlreiche Beispiele beschrieben, unter anderem in Bioorg. Med. Chem. Lett. 2010, Vol. 20, No. 13, 3920-3924, in Proc. Natl. Acad. Sei. USA 2011, Vol. 108, No. 17, 6781-6786, in Chem. Eur. J. 2009, Vol. 15, No. 29, 7044-7047, in Synlett 2004, Vol. 6, 1095-97, in J. Org. Chem. 2005, 70, 4, 1486-1489, in Tetrahedron Lett. 1998, Vol. 39, 2933-2936, in WO 2005/85226 A1 oder in WO 2008/62905 A2. Zusammenfassende Übersichten finden sich zum Beispiel in Synthesis 2011, No. 6, 829-856 oder in Tetrahedron 2012, Vol. 68, 7735-7754. Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Durch Oxidation der Thioether der allgemeinen Formel (Ia) nach literaturbekannten Methoden können die Sulfoxide der allgemeinen Formel (Ib) erhalten werden. Alternativ kann die oxidative übergangsmetallvermittelte Kohlenstoff-Stickstoff-Kupplung zu Arylsulfoxiden der allgemeinen Formel (Ib) ausgehend von Boronsäure-Sulfoxiden der allgemeinen Formel (VIIIb), welche durch Oxidation der Boronsäuren (VIIIa), zum Beispiel mit Natriumperiodat, oder analoger Derivate zugänglich sind, ermöglicht werden.

Verfahren V3' ist insbesondere zur Darstellung der Ausführungsformen I-B-1, I-B-4, I-C-1 und I-C-2 geeignet.

Verfahren Va1 - Va8 eignen sich zur Herstellung der Ausführungsform I-A der Verbindungen der Formel (I).

**Verfahren Va1 (zur Herstellung von I-A-1)**

Die Imidazolidinonen der allgemeinen Formel (I-A-1) können in (I-A-1a) (für n=0) und (I-A-1b) (für n=1) unterteilt werden. Sie können beispielsweise nach Verfahren Va1 hergestellt werden.

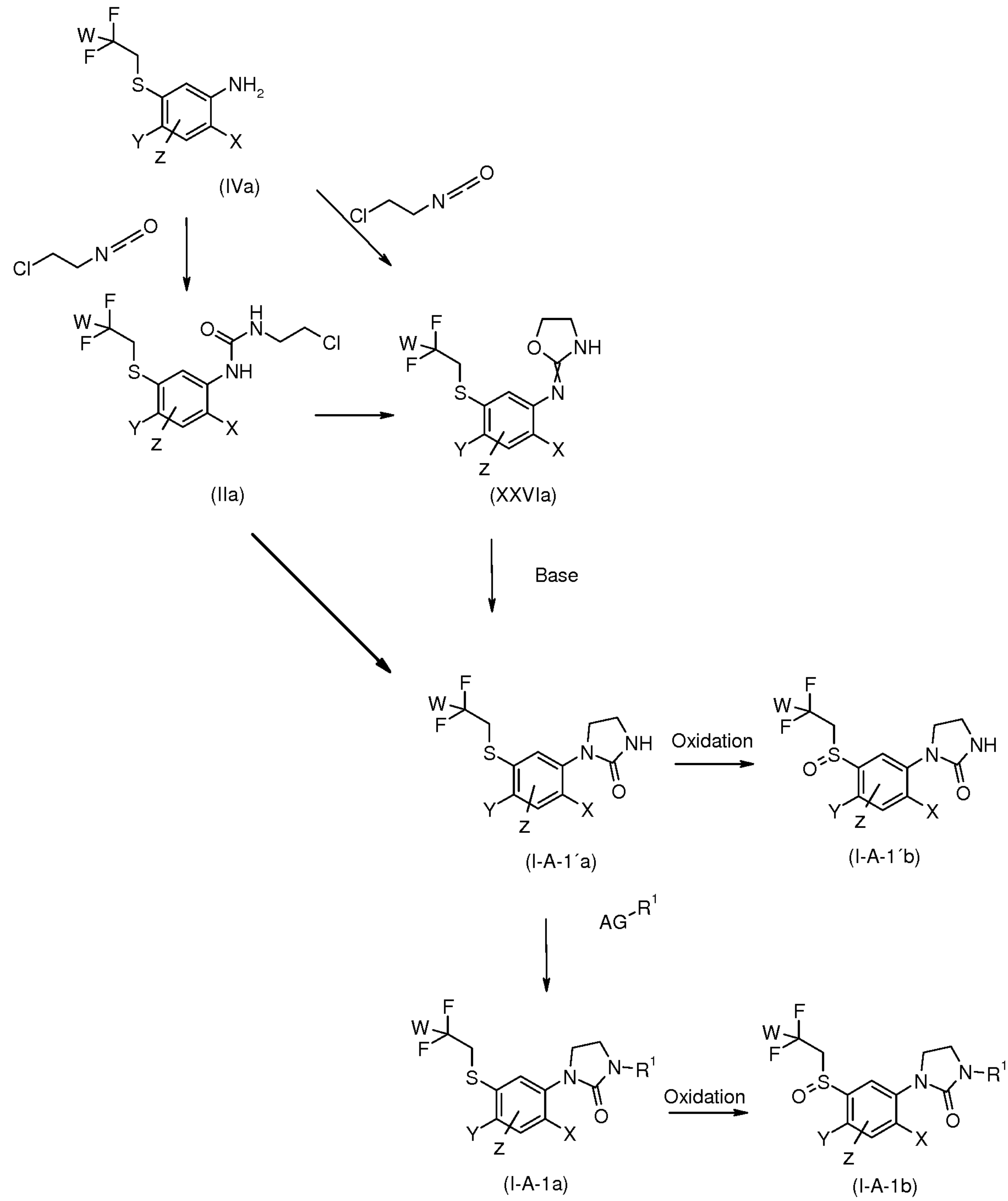


(IVa) (IIa) (XXVIa) (I-A-1´a) (I-A-1´b) (I-A-1a) (I-A-1b)

wobei W, X, Y, Z und $R^1$ die oben angegebene Bedeutung haben und AG für eine Abgangsgruppe wie z.B. Chlorid, Bromid, Iodid, Triflat und Mesylat steht.

Aniline der Formel (IVa) können durch die Umsetzung mit 2-Chlorethyl-isocyanat zu den cyclischen Amidinen der allgemeinen Formel (XXVIa) - ggf. unter Zwischenisolierung der Harnstoffe der Formel (IIa) - umgesetzt werden.

Die Herstellung von Harnstoffen ist literaturbekannt, z.B. aus JP 2011/042611.

1,3-Oxazolidin-2-yliden-aniline der allgemeinen Formel (XXVIa) werden in Gegenwart von Basen in die 1-Aryl-imidazolidin-2-one der allgemeinen Formel (I-A-1'a) umgewandelt.

Die Thioether (I-A-1'a) können dann nach literaturbekannten Methoden zu den Verbindungen der allgemeinen Formel (I-A-1a) umgesetzt werden. Geeignete Mittel zur Alkylierung sind zum Beispiel Alkylelektrophile der Formel $R^1$-AG, wobei AG z.B. für Chlorid, Bromid, Iodid, Triflat und Mesylat steht.

Weitere Herstellungsbeispiele und die Verwendung von 1-Aryl-imidazolidin-2-onen als Zwischenprodukte sind z.B. in den folgenden Patentschriften publiziert: WO 2012149413, WO 2011110575, WO 2011098776, WO 2010149755,

WO 2008077597, WO 2008057254, WO 2007038367, US 20070066588, WO 2005111038 oder WO 2005092890.

Die Herstellung von 1-Aryl-imidazolidin-2-onen durch die Reduktion von 1-Aryl-imidazolin-2-onen wird im J.Med.Chem. 1966, 9(6), 858-860 beschrieben.

N-Alkylierungen von 1-Aryl-imidazolidin-2-onen und die Herstellung von 1-Aryl-imidazolidin-2-onen durch die Umsetzung von N-Aryl-1,2-diaminen in Gegenwart von z.B. 1,1'-Carbonyldiimidazol werden in Can. J. Chem. 2004,82,1649 beschrieben.

**Verfahren Va2 (zur Herstellung von I-A-2)**

Die Oxazolidinone der allgemeinen Formel (I-A-2) können in (I-A-2a) (für n=0) und (I-A-2b) (für n=1) unterteilt werden. Sie können beispielsweise nach Verfahren Va2 hergestellt werden.

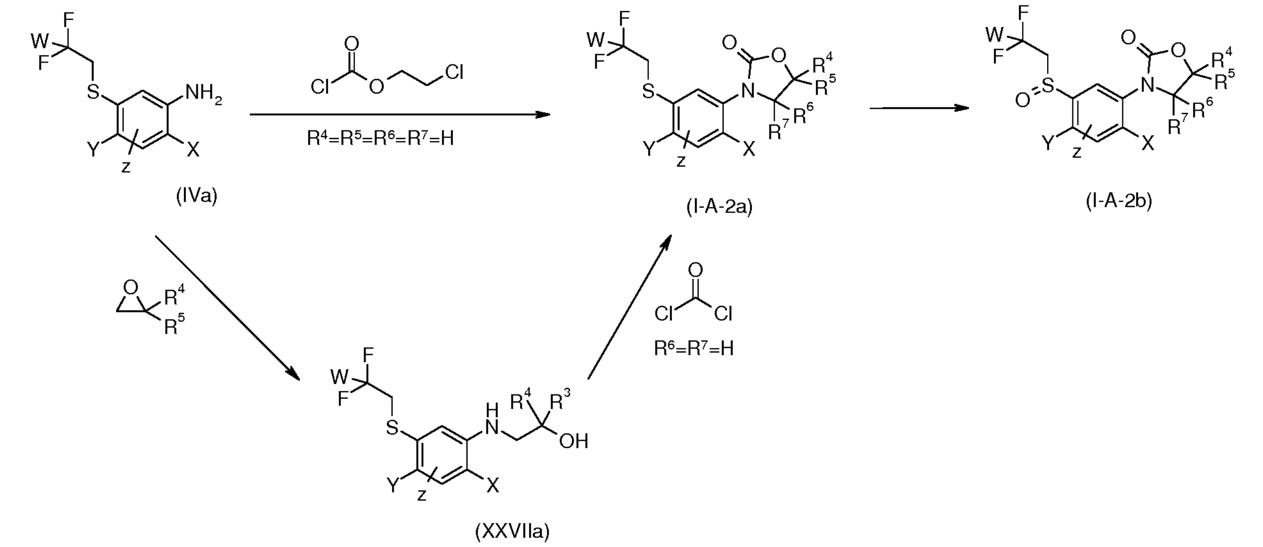


(IVa) (I-A-2a) (I-A-2b) (XXVIIa)

wobei W, X, Y, z, $R^3$, $R^4$, $R^5$ und $R^6$ die oben angegebene Bedeutung haben.

Aniline der Formel (IVa) können zu den Oxazolidinone der Formel (I-A-2a) nach literaturbekannten Methoden hergestellt werden, zum Beispiel durch die Umsetzung mit 2-Chlorethylchlorocarbonat, beispielsweise analog Molecules 2012, 17, 1233-1240.

Alternativ können Aniline der Formel (IVa) mit einem Epoxid zu Aminoalkohole der Formel (XXVIIa) reagieren, die mit Phosgen zu Oxazolidinone der Formel (I-A-2a) umgesetzt werden können, beispielsweise analog Organic Process Research and Development 2010, 14, 1457-1463 und Journal of Heterocyclic Chemistry 1986, 23, 1427-1429.

**Verfahren Va3 (zur Herstellung von I-A-2 und I-A-3)**

Die Oxazolidinonen der allgemeinen Formel (I-A-2) können in (I-A-2a) (für n=0) und (I-A-2b) (für n=1) unterteilt werden. Sie können beispielsweise nach Verfahren Va3 hergestellt werden.

Die Thiazolidinonen der allgemeinen Formel (I-A-3) können in (I-A-3a) (für n=0) und (I-A-3b) (für n=1) unterteilt werden. Sie können beispielsweise auch nach Verfahren Va3 hergestellt werden.

(XXVIII)

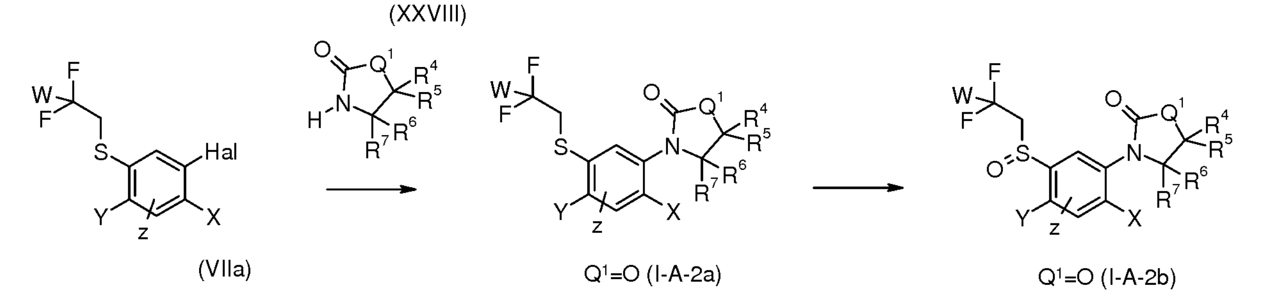

(VIIa)

$Q^1$=O (I-A-2a)
$Q^1$=S (I-A-3a)

$Q^1$=O (I-A-2b)
$Q^1$=S (I-A-3b)

wobei W, X, Y, z, $R^4$, $R^5$, $R^6$ und $R^7$ die oben angegebene Bedeutung haben, $Q^1$ für Sauerstoff oder Schwefel steht, und Hal für einen Halogen aus der Reihe Cl, Br oder Iod.

Halogenide der Formel (VIIa) können mit Oxazolidinone ($Q^1$=O) beziehungsweise Thiazolidinone ($Q^1$=S) der Formel (XXVIII) mit Hilfe eines Katalysators zu den Oxazolidinonen der Formel (I-A-2a) beziehungsweise Thiazolidinonen der Formel (I-A-3a) beispielsweise analog Chemical Biology & Drug Design 2007, 70, 100-112.

1,3-Oxazolidin-2-one (XXVIII, $Q^1$=O), die in der präparativen Chemie als Auxiliare (Evans-Auxiliar) bekannt sind, sind kommerziell erhältlich oder durch bekannte Methoden herstellbar.

1,3-Thiazolidin-2-one (XXVIII, $Q^1$=S) sind kommerziell erhältlich wie zum Beispiel 1,3-thiazolidin-2-one, 5-methyl-1,3-thiazolidin-2-one oder 5,5-dimethyl-1,3-thiazolidin-2-one, oder durch bekannte Methoden herstellbar.

**Verfahren Va4 (zur Herstellung von I-A-4)**

Die Pyrrolidin-2-one der allgemeinen Formel (I-A-4) können in (I-A-4a) (für n=0) und (I-A-4b) (für n=1) unterteilt werden. Sie können beispielsweise nach Verfahren Va4 hergestellt werden.

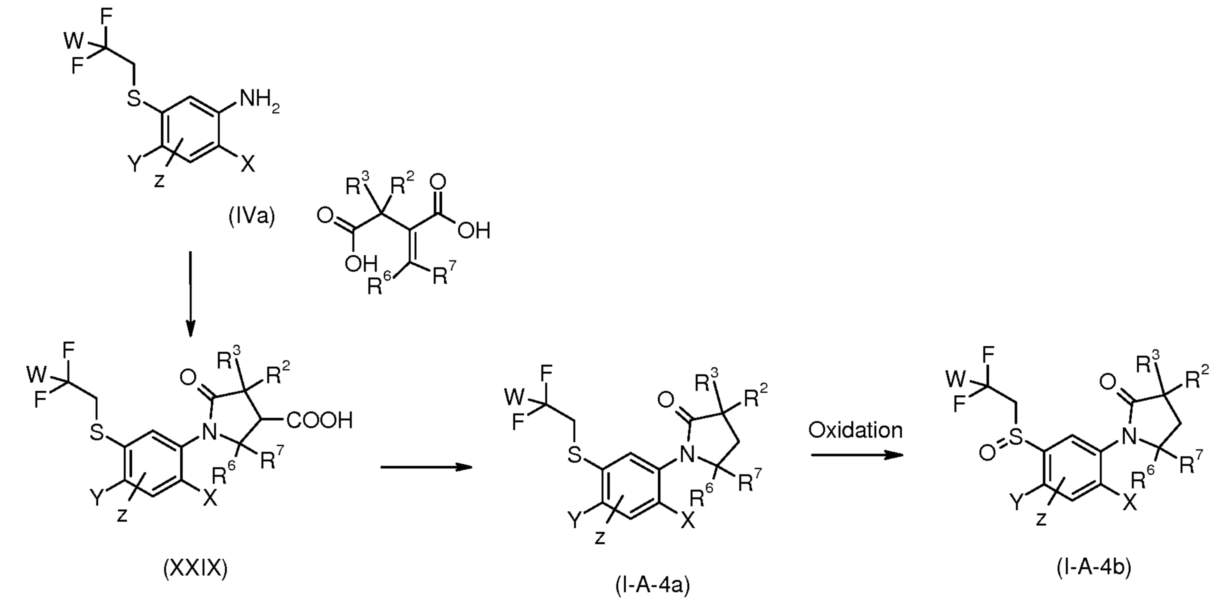


wobei W, X, Y, z, $R^2$, $R^3$, $R^5$ und $R^6$ die oben angegebene Bedeutung haben.

Die Herstellung der Verbindungen der allgemeinen Formel I-A-4 erfolgt z.B. nach dem in der DE 2650604 beschrieben Verfahren.

Aniline der allgemeinen Formel (IVa) werden durch die Umsetzung von ggf. substituierten Itaconsäuren zu den 5-Oxo-1-arylpyrrolidin-3-carbonsäuren der allgemeinen Formel (XXIX) umgesetzt. Deren Decarboxylierung liefert die 1-Arylpyrrolidine-2-one der allgemeinen Struktur (I-A-4a).

Ein weiteres allgemeines Herstellverfahren für die 1-Arylpyrrolidin-2-one besteht in der Umsetzung von Dihydrofuran-2(3H)-on mit Anilinen. Dieses Verfahren ist z.B. in der WO 2004/037787 oder in J. Het. Chem. 3, 311 (1966) beschrieben. Analoge Umsetzungen sind bekannt, z.B. aus Tetrahedron Lett., 1990, 31 (21) 2991.

Die Cyclisierung von 4-Chlor-N-Arylbutanamiden zu den 1-Arylpyrrolidin-2-onen ist in einem spanischen Patent, ES 1982-516898, beschrieben.

**Verfahren Va5 (zur Herstellung von I-A-5)**

Die 2-Imino-1,3-thiazolidine der allgemeinen Formel (I-A-5) können in (I-A-5a) (für n=0) und (I-A-5b) (für n=1) unterteilt werden. Sie können beispielsweise nach Verfahren Va5 hergestellt werden.

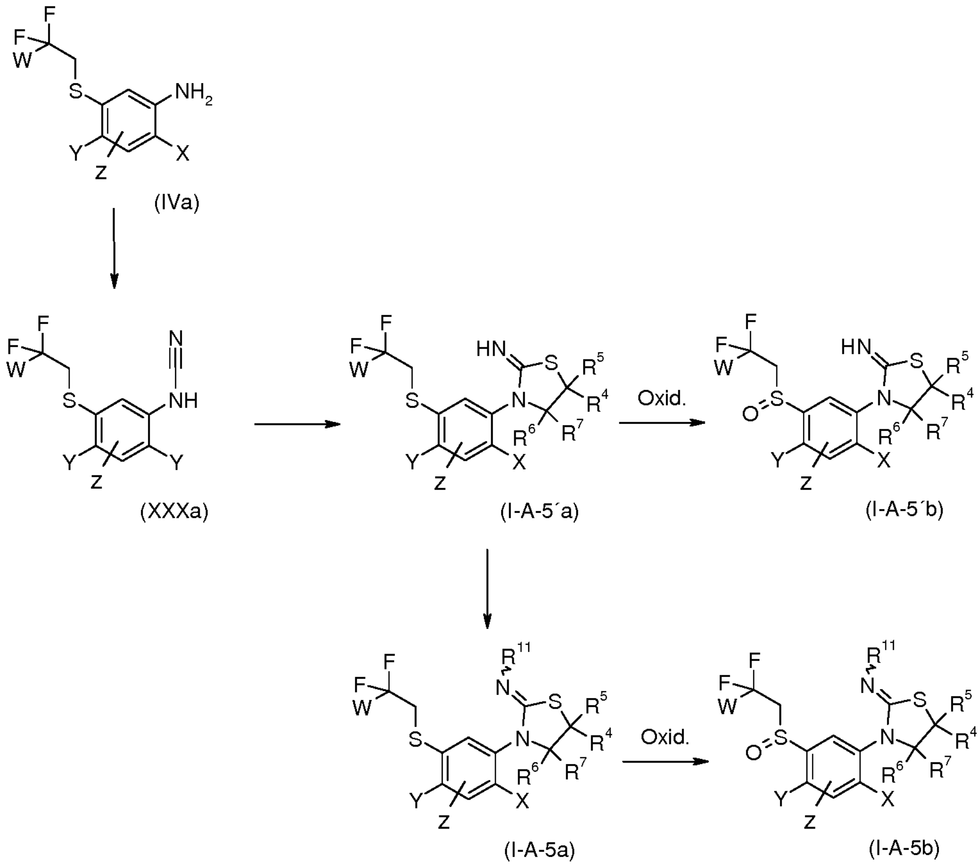


(IVa) (XXXa) (I-A-5´a) (I-A-5´b) (I-A-5a) (I-A-5b)

wobei W, X, Y, Z, $R^4$, $R^5$, $R^6$, $R^7$ und $R^{11}$ die oben angegebenen Bedeutungen haben.

Aniline der Formel (IVa) können zu Arylcyanamiden der Formel (XXXa) mit Hilfe von Halocyanverbindungen, wie z.B. mit Bromcyan nach der in US2007/27125 beschriebenen Methode, umgesetzt werden. Alternativ können die Aniline (IVa) erst nach literaturbekannten Methoden in deren Thioharnstoffen umgewandelt werden und diese werden dann zu Arylcyanamiden (XXXa) umgesetzt, zum Beispiel durch Übergangsmetall-katalysierte Reaktionen, wie von S. K. Sahoo in Adv. Synth. and Catal. 2010, 352, 2538-2548, beschrieben. Die Arylcyanamiden (XXXa) können zum Beispiel mit geeigneten Thiiranen reagieren, wie im Patent US5266701, um die unsubstituierten 2-Imino-1,3-thiazolidine (I-A-5') zu bilden. Verbindungen der Formel (I-A-5'a) lassen sich nach literaturbekannten Methoden am Stickstoffatom substituieren, zum Beispiel mit Alkyliodiden wie vom Y. V. Raschkes in J. Org. Chem. USSR (Engl.) 1981, 17, 529-536, beschrieben.

## <u>Verfahren Va6</u> (zur Herstellung von I-A-5)

Alternativ kann die Synthese der 2-Imino-1,3-thiazolidine (I-A-5) nach Verfahren Va6 erfolgen.

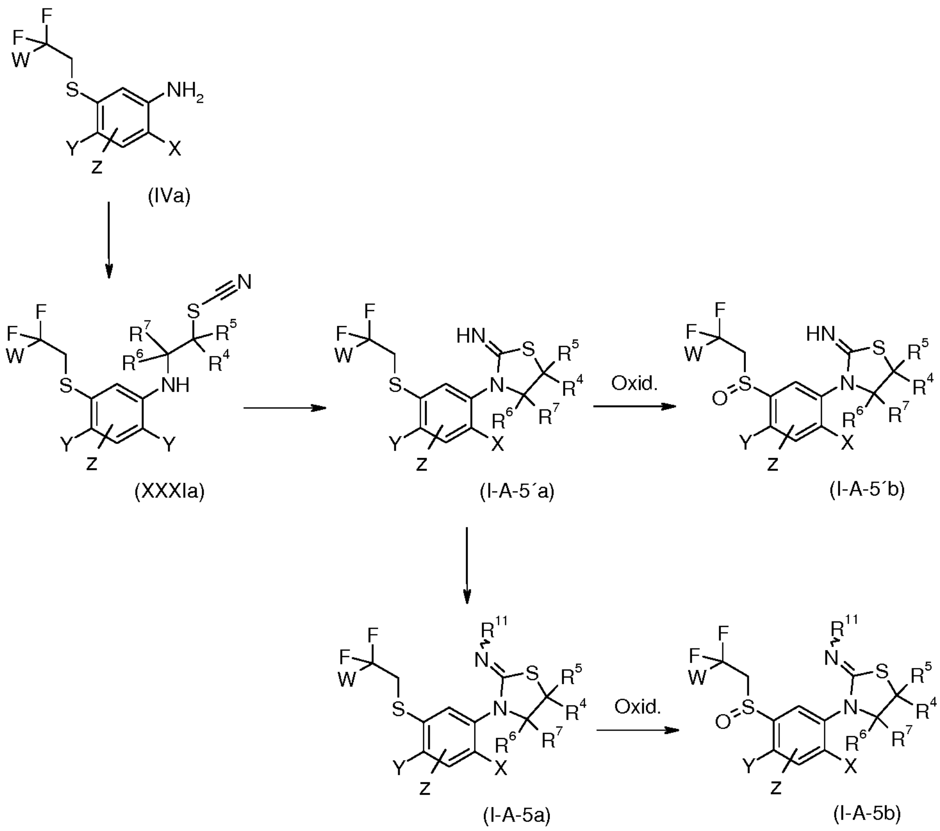


Aniline der Formel (IVa) können z.B. mit N-Alkylthiocyanaten zu den Produkten (XXXIa) umgesetzt werden, zum Beispiel in einem Alkohol als Lösungsmittel und mit Salzsäure als saures Medium, wie in US4665083 beschrieben.

**<u>Verfahren Va7</u> (zur Herstellung von I-A-5)**

Alternativ ist die Synthese der 2-Imino-1,3-thiazolidine (I-A-5) über Thioharnstoffe der Formel (II') nach Verfahren Va7.

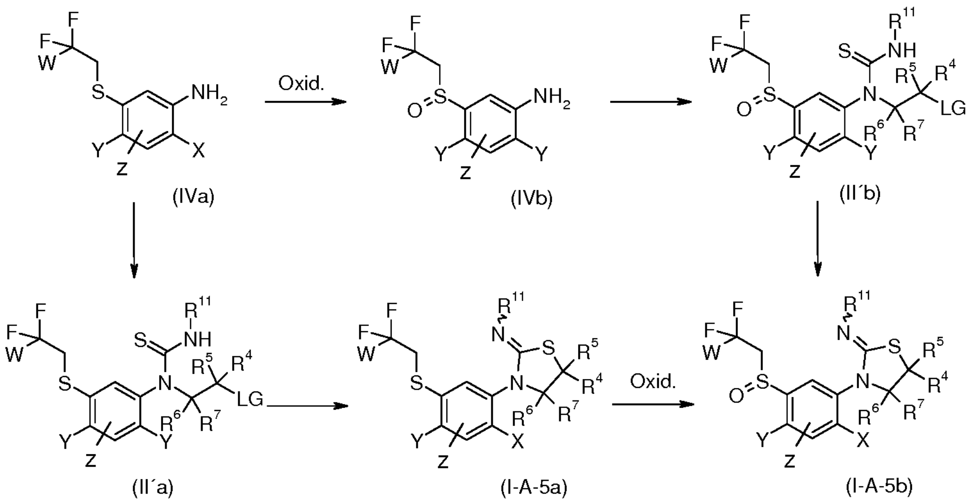


wobei W, X, Y, Z, $R^4$, $R^5$, $R^6$, $R^7$ und $R^{11}$ die oben angegebenen Bedeutungen haben und LG für eine Abgangsgruppe (bevorzugt Halogen und Alkoxy).

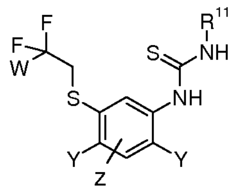

(II) $Q^1$=S

Thioharnstoffe der Formel (II') können aus Anilinen (IV) nach literaturbekannten Methoden hergestellt werden, zum Beispiel über Thioharnstoffen (II) und geeignete Alkylierungsmittel oder über alkylierte Aniline und Isothiocyanate, wie zum Beispiel im Patent US6353006. Die anschließende Cyclisierung zu den Thiazolidinen (I-A-5) kann spontan erfolgen oder gegebenenfalls mit Hilfe eines Aktivierungsreagenzien stattfinden. Hierfür können sich Mesylate, Tosylate oder Triflate als Intermediate bilden. Im Patent WO2003/97605 wird die Umsetzung eines Alkohols mit Mesylchlorid in Anwesenheit von Triethylamin und Dichlormethan als Lösungsmittel beschrieben.

Die Reihenfolge der Syntheseschritte ist variabel und so lassen sich z.B. mit 1,2-Dibromethan ($R^4$=$R^5$=$R^6$=$R^7$) nach H. G. Hoker in Synthesis 2009, 7, 1195-1203 die Cyclisierungen zu den Thiazolidinen (I-A-5) direkt aus den Harnstoffen (II) durchführen, ohne die Syntheseintermediaten (II') zu isolieren.

**Verfahren Va8 (zur Herstellung von I-A-6)**

Die N-Arylyl-pyrrolidin-2-yliden-ethanamine der allgemeinen Formel (I-A-6) können in (I-A-6a) (für n=0) und (I-A-6b) (für n=1) unterteilt werden. Sie können beispielsweise nach Verfahren Va8 hergestellt werden.

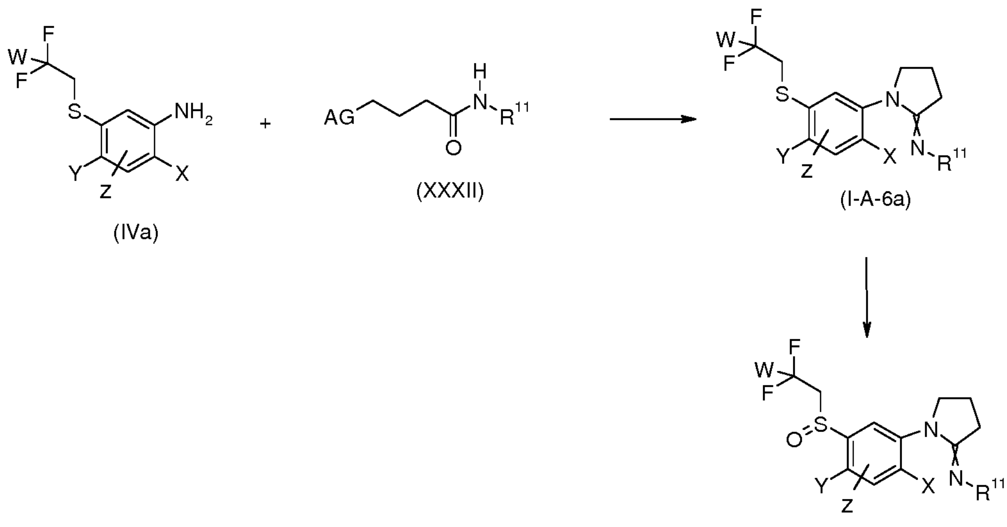

(IVa) + (XXXII) (I-A-6a)

(I-A-6b)

wobei W, X, Y, Z und $R^{11}$ die oben angegebene Bedeutung haben und AG für eine Abgangsgruppe wie Chlorid, Bromid, Iodid, Triflat oder Tosylat beispielsweise steht.

Die erfindungsgemäßen N-Arylyl-pyrrolidin-2-yliden-ethanamine der allgemeinen Formel (I-A-6a) werden durch die Umsetzung von Anilinen der Formel (IVa) mit Butanamiden der Formel (XXXII) in Gegenwart von Phosphorylchlorid erhalten. Butanamide der Formel (XXXII) sind teilweise kommerziell erhältlich oder können nach bekannten Verfahren hergestellt werden.

Verfahren Vb1 - Vb9 eignen sich zur Darstellung der Ausführungsform I-B der Verbindungen der Formel (I).

**Verfahren Vb1 und Vb2 (zur Herstellung von I-B-1)**

Die Imidazolidin-2,4-dionen der allgemeinen Formel (I-B-1) können in (I-B-1a) (für n=0) und (I-B-1b) (für n=1) unterteilt werden. Sie können beispielsweise nach Verfahren Vb1 und Verfahren Vb2 hergestellt werden.

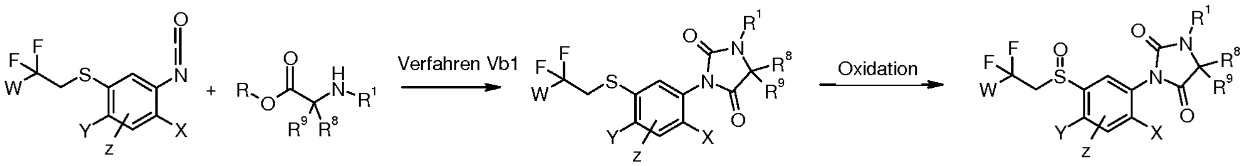


(IIIa) (I-B-1a) (I-B-1b)

wobei W, X, Y, z, $R^1$, $R^8$ und $R^9$ die oben angegebene Bedeutung haben.

Imidazolidin-2,4-dione der allgemeinen Formel (I-B-1a) können alternativ nach Verfahren Vb1 durch Kondensation von Isocyanaten Formel (IIIa) mit unterschiedlichen $\alpha$-Aminoestern oder deren Surrogaten z. B. nach J. Med. Chem. 2012, 55, 8225-8235, Synth. Commun. 2010, 40, 1377-1390 oder Tetrahedron Lett. 2012, 53, 5593-5596. Alternativ können $\alpha$-Aminoester auch in situ durch reduktive Aminierung, wie in J. Org. Chem. 1997, 62, 3230-3235 beschrieben, hergestellt und eingesetzt werden. Desweiteren können Isocyanate der Formel (IIIa) in situ durch Umsetzung mit Phosgen, Diphosgen oder Triphosgen dargestellt werden, wie zum Beispiel in J. Med. Chem. 2012, 55, 8236-8247, in US2011/53947 A1 oder in WO2005/103054 A2 beschrieben, und direkt eingesetzt werden.

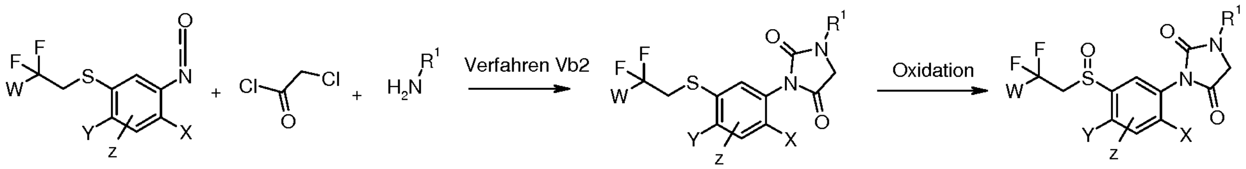


(IIIa) (I-B-1a) (I-B-1b)

Alternativ können die Imidazolidin-2,4-dione der allgemeinen Formel (I-B-1a) nach Verfahren Vb2 durch Kondensation von Isocyanaten der Formel (IIIa) mit Chloracetylchlorid und Aminen ($R^1$-$NH_2$) durch Einsatz von Polymerträgern und Mikrowellenbestrahlung nach Tetrahedron Lett. 2004, 45 ,437-440 hergestellt werden.

## Verfahren Vb3 (I-B-2)

Die Oxazolidindione der allgemeinen Formel (I-B-2) können in (I-B-2a) (für n=0) und (I-B-2b) (für n=1) unterteilt werden. Sie können beispielsweise nach Verfahren Vb3 hergestellt werden.

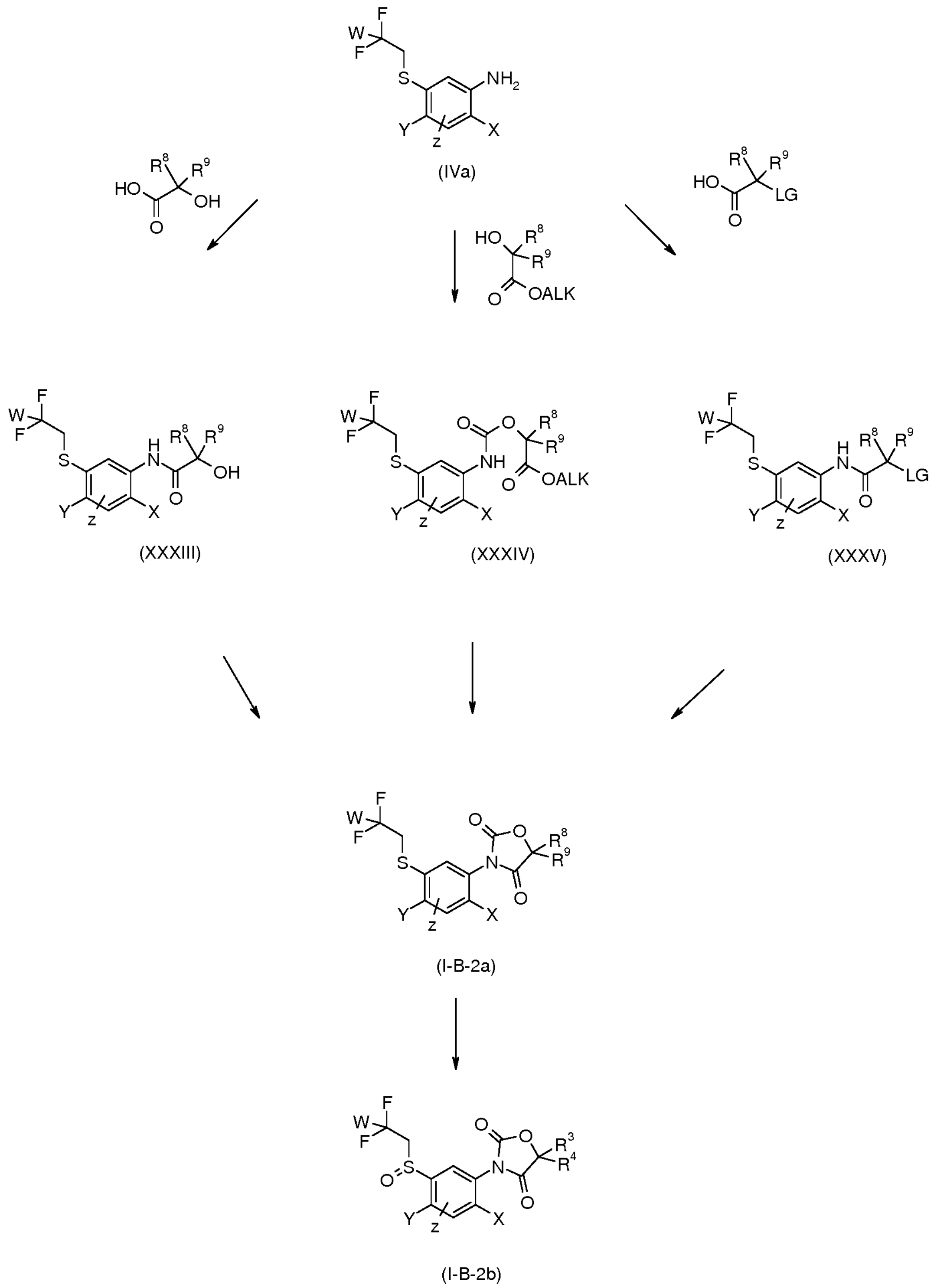

wobei X, Y, z, $R^8$ und $R^9$ die oben angegebene Bedeutung haben, LG für eine Abgangsgruppe wie Chlor, Brom, Iod, Tosylat, Mesylat, Tosylat oder Triflat beispielsweise steht und ALK für eine kleine Alkylgruppe wie Methyl oder Ethyl beispielsweise steht.

Aniline der Formel (IVa) können zu den α-Hydroxycarboxamiden der Formel (XXXIII) durch Umsetzung mit einer α-Hydroxycarbonsäure mit Hilfe eines Chlorierungsmittels wie Thionylchlorid beispielsweise und einer Base wie Triethylamin beispielsweise umgesetzt werden. Alternativ können die Aniline (IVa) aktiviert werden, zum Beispiel mit Trimethylaluminium, und dann mit α-Hydroxycarbonsäureestern zu den Carboxamiden der Formel (XXXIII) umgewandelt werden. α-Hydroxycarboxamide der Formel (XXXIII) können zu den Oxazolidindionen der Formel (I-B-2a) nach literaturbekannten Methoden hergestellt werden, zum Beispiel durch die Umsetzung mit Carbonyldiimidazol in Gegenwart

einer Base, beispielsweise analog Bioorg. & Med. Chem 2002, 3267-3276.

Aniline der Formel (IVa) können zu den Carbamaten der Formel (XXXIV) durch Umsetzung mit einen α-Hydroxyester mit Hilfe von Carbonyldiimidazol beispielsweise und einer Base wie Triethylamin beispielsweise umgesetzt werden. Carbamate der Formel (XXXIV) können zu den Oxazolidindionen der Formel (I-B-2a) nach literaturbekannten Methoden hergestellt werden, zum Beispiel durch die Umsetzung mit einer Base wie Natriummethoxid, beispielsweise analog Bioorg. & Med. Chem 2012, 3242-3254.

Aniline der Formel (IVa) können zu den Carboxamiden der Formel (XXXV), durch Umsetzung mit einem Kupplungsreagenz oder einem Chlorierungsmittel umgesetzt werden. Carboxamide der Formel (XXXV) können zu den Oxazolidindionen der Formel (I-B-2a) nach literaturbekannten Methoden hergestellt werden beispielsweise analog Tetrahedron 1999, 193-200, Tetrahedron Letters 2009, 50, 5123-5125 oder Pharmazie 1992,47,340.

Allgemeine Synthese von Oxazolidindione sind in JACS 1959, 6498-6503 beschrieben.

## Verfahren Vb4 (zur Herstellung von I-B-3)

Die Thiazolidindione der allgemeinen Formel (I-B-3) können in (I-B-3a) (für n=0) und (I-B-3b) (für n=1) unterteilt werden. Sie können beispielsweise nach Verfahren Vb4 hergestellt werden.

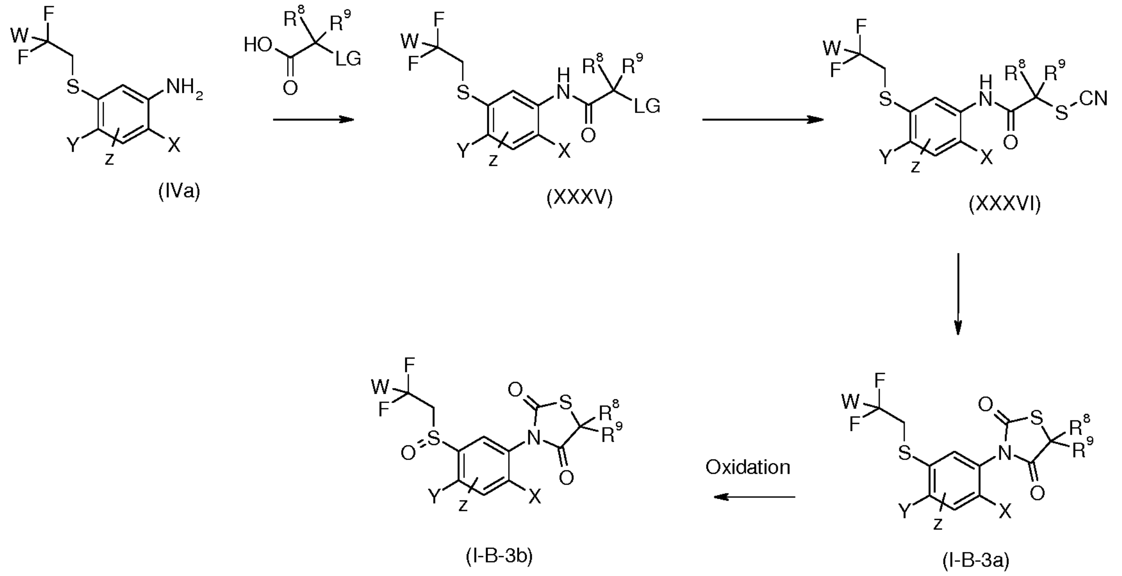

(IVa) (XXXV) (XXXVI) (I-B-3b) (I-B-3a)

wobei X, Y, z, $R^3$ und $R^4$ die oben angegebene Bedeutung haben, LG für eine Abgangsgruppe wie Chlor, Brom, Iod, Tosylat, Mesylat, Triflat beispielsweise steht.

Aniline der Formel (IV) können zu den Carboxamiden der Formel (XXXV) durch Umsetzung mit einem Kupplungsreagenz oder einem Chlorierungsmittel hergestellt werden. Carboxamide der Formel (XXXV) können zu den Thiocyanaten der Formel (XXXVI) durch Umsetzung mit Kaliumthiocyanat beispielsweise. Thiocyanate der Formel (XXXVI) können zu den Thiazolidindionen der Formel (I-B-3a) nach literaturbekannten Methoden hergestellt werden, beispielsweise analog Pharmazie 1992, 47, 340.

## Verfahren Vb5 (zur Herstellung von I-B-4)

Die N-Aryl-pyrrolidin-2,5-dione der allgemeinen Formel (I-B-4) können in (I-B-4a) (für n=0) und (I-B-4b) (für n=1) unterteilt werden. Sie können beispielsweise nach Verfahren Vb5 hergestellt werden.

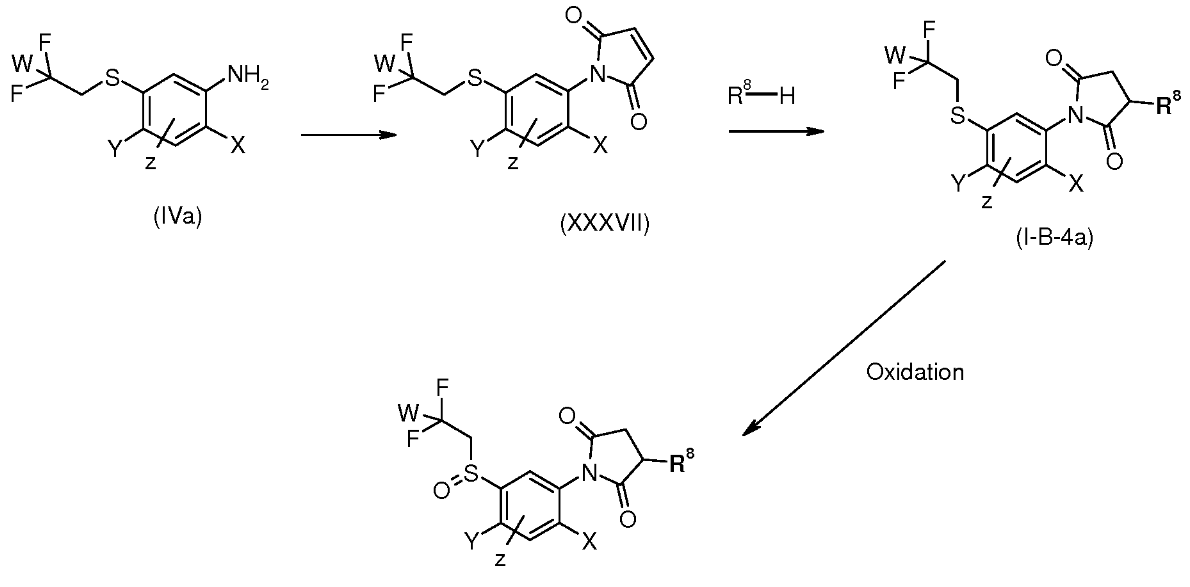


(IVa) (XXXVII) (I-B-4a) (I-B-4b)

wobei W, X, Y, z, R' und R" die oben angegebene Bedeutung haben und $R^8$ für eine ggf. substituierte Aminogruppe steht.

Die Herstellung der Verbindungen der allgemeinen Formel (I-B-4) erfolgt dadurch, dass man Aniline der Formel (IV) mit Furan-2,5-dionen gemäß Angewandte Makromolekulare Chemie (1988), 157 59-78 oder der US 3853912 in die N-Arylmaleinimide der allgemeinen Formel (I-B-4a) überführt.

Die erfindungsgemäßen N-Aryl-pyrrolidin-2,5-dione der allgemeinen Formel (I-B-4a) bzw. (I-B-4b) werden aus N-Arylmaleinimiden der allgemeinen Formel (XXXVII) durch die Umsetzung mit Aminen der Formel ($R^8$-H) erhalten, gegebenenfalls in Anwesenheit eines inerten, organischen Lösungsmittels.

Die Reaktion von Maleinimiden mit Aminen ist literaturbekannt, z.B. aus Biochemistry, 1966, 5 (9), 2963-2971 oder J. Org. Chem. (2007), 43(3), 393-396.

Weitere Synthesevarianten sind z.B. beschrieben in Tetrahedron (1999), 55(40),11859-11870 oder Acta Chem. Scand. (1999), 53(1), 48-56 oder Indian Drugs (1992), 29(7), 306-307 oder Bioorg. Med. Chem. Lett. (2006), 16(3), 525-528 oder Arzneimittel-Forsch. (2003), 53(4), 280-288.

## Verfahren Vb6 (zur Herstellung von I-B-4)

Alternativ können die N-Aryl-pyrrolidin-2,5-dione der allgemeinen Formel (I-B-4) nach Verfahren Vb6 hergestellt werden.

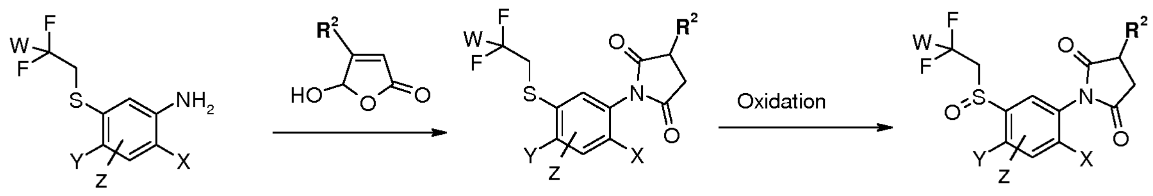


(IVa) (I-B-4a) (I-B-4b)

wobei W, X, Y und Z die oben angegebene Bedeutung haben.

Die erfindungsgemäßen N-Arylpyrrolidin-2,5-dione der allgemeinen Formel (I-B-4) werden durch die Umsetzung von Anilinen der allgemeinen Formel (IVa) mit 5-Hydroxyfuran-2(5H)-on in Gegenwart eines Lösungsmittels erhalten.

Weitere Synthesevarianten sind z.B. beschrieben in Bioorg. Med. Chem. Lett. (2012), 22(23), 7019-7023 oder in Tetrahedron Lett. (2010), 51, 2215-2217 oder in Revue Roumaine de Chimie (2005), 50(7-8), 655-661 oder J. Am. Chem. Soc. 1924,46, 2069-2078. (Für $R^2$=H)

**Verfahren Vb7 (zur Herstellung von I-B-5 und I-B-7)**

Die 3-Arylimino-1,3-thiazolidin-4-one ($Q^1$=S) der allgemeinen Formel (I-B-5) können in (I-B-5a) (für n=0) und (I-B-5b) (für n=1) unterteilt werden.

Die 3-Arylimino-1-3-oxazolidin-4-one ($Q^1$=O) der allgemeinen Formel (I-B-7) können in (I-B-7a) (für n=0) und (I-B-7b) (für n=1) unterteilt werden.

I-B-5 und I-B-7 können beispielsweise nach Verfahren Vb7 hergestellt werden.

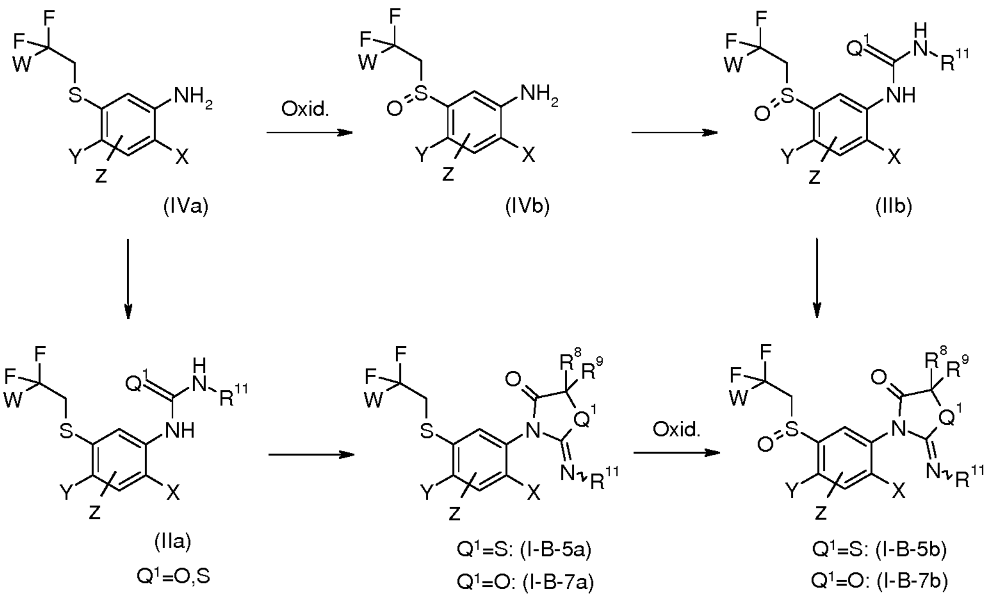


(IVa) (IVb) (IIb)

(IIa) $Q^1$=O,S

$Q^1$=S: (I-B-5a) $Q^1$=O: (I-B-7a)

$Q^1$=S: (I-B-5b) $Q^1$=O: (I-B-7b)

wobei W, X, Y, Z, A, $R^8$, $R^9$ und $R^{11}$ die oben angegebenen Bedeutungen haben.

Aniline der Formel (IV) können zu den Harnstoffen ($Q^1$=O) und Thioharnstoffen ($Q^1$=S) der Formel (II) nach literaturbekannten Methoden umgesetzt werden, zum Beispiel nach JP2011/042611, in dem sie mit Isocyanaten (für $Q^1$=O) und Isothiocyanaten (für $Q^1$=S) gegebenenfalls in Anwesenheit einer Base und gegebenfalls in Anwesenheit eines organischen Lösungsmittels versetzt werden oder in dem sie nach allgemein bekannten Methoden in deren Isocyanaten ($Q^1$=O) und Isothiocyanaten ($Q^1$=S) umgewandelt und diese mit Aminen zu den Harnstoffen oder Thioharnstoffen umgesetzt werden.

Aus den Harnstoffen ($Q^1$=O) und Thioharnstoffen ($Q^1$=S) der allgemeinen Formel (II) kann die Synthese der 3-Arylimino-1,3-oxazolidin-4-one ($Q^1$=O) bzw. -thiazolidin-4-one ($Q^1$=S) der allgemeinen Formel (I-B-7) bzw. (I-B-5) erfolgen, zum Beispiel durch Cycloacylierung mit einem adäquaten Halocarbonylderivaten in einem inerten Lösungsmittel, meistens bei Temperaturen höher als 100 °C. Geeignete Halocarbonylderivaten sind zum Beispiel Chloressigsäure und deren Säurechlorid, Bromessigsäure und deren Säurechlorid oder -bromid; wie von C. F. Howell in J. Org. Chem. 1962, 27, 1691 für Umsetzungen zu Oxazolidinonen und V.N.Britsun in Russ. J. Org. Chem. 2006, 41 (11), 1719-1729 für Thiazolidinonen beschrieben.

**Verfahren Vb8 und Vb9 (zur Herstellung von I-B-6)**

Die 4-Aryl-1,2,4-triazolidin-3,5-dionen der allgemeinen Formel (I-B-6) bzw. (I-B-6') können in (I-B-6a) bzw. (I-B-6'a) (für n=0) und (I-B-6b) bzw. (I-B-6'b) (für n=1) unterteilt werden. Sie können beispielsweise nach Verfahren Vb8, Vb9 und Vb10 hergestellt werden.

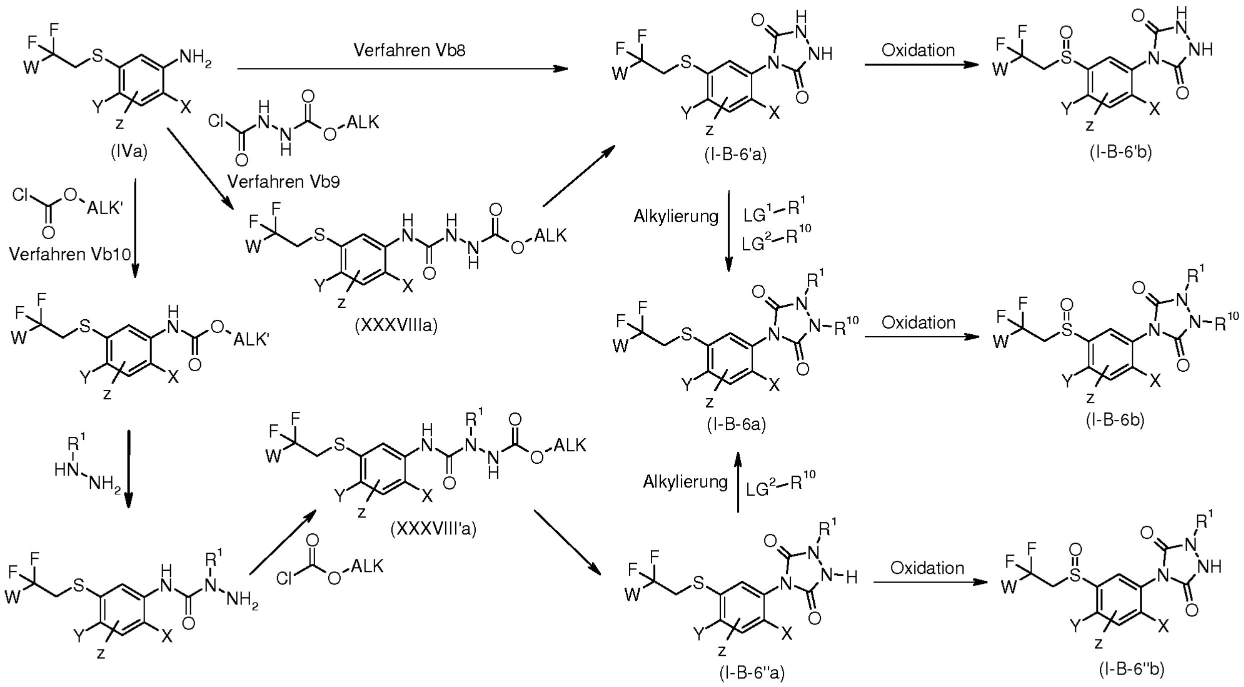


wobei W, X, Y, z, $R^1$ und $R^{10}$ die oben angegebene Bedeutung haben und ALK und ALK' für eine kleine Alkylgruppe wie Methyl oder Ethyl steht.

4-Aryl-1,2,4-triazolidin-3,5-dione der allgemeinen Formel (I-B-6'a) können alternativ nach Verfahren Vb8 durch Reaktion der Aniline (IVa) mit Chlorameisensäureestern und Semicarbaziden wie zum Beispiel in Synlett 2007, 8, 1255-1256 oder in Synth. Commun. 2007, 37, 1927-1934 beschrieben, hergestellt werden.

Alternativ können 4-Aryl-1,2,4-triazolidin-3,5-dione der allgemeinen Formel (I-B-6'a) durch Kondensation der Aniline (IVa) mit Diharnstoff nach Verfahren Vb8 hergestellt werden, wie zum Beispiel in Phamaceutical Bulletin 1954, 2, 403-411 beschrieben wird.

In Synthesis 1982, 2, 159-160 wird die Synthese von 4-Aryl-1,2,4-triazolidin-3,5-dione der allgemeinen Formel (I-B-6'a) durch Acylierung mit Alkyl-2-(chlorcarbonyl)hydrazincarboxylaten zu Verbindungen der allgemeinen Formel (XXXVIIIa) und anschließender Cyclisierung nach Verfahren Vb9 beschrieben.

Anschließende Alkylierung von (I-B-6'a) mit entsprechenden Alkylierungsmittel $R^1$-$LG^1$ bzw. $R^{10}$-$LG^2$ ergibt 4-Aryl-1,2,4-triazolidin-3,5-dione der allgemeinen Formel (I-B-6a), wobei $LG^1$ und $LG^2$ für klassische Abgangsgruppen wie Iodid, Bromid, Chlorid, Tosylat, Mesylat oder Triflat stehen.

Alternativ können 4-Aryl-1,2,4-triazolidin-3,5-dione der allgemeinen Formel (I-B-6"a) nach Verfahren Vb10 durch Cyclisierung von Verbindungen der allgemeinen Formel (XXXVIII'a) hergestellt werden, die wiederum durch Umsetzung der Aniline (IVa) mit Chlorameisensäureestern, substituierten Hydrazinen und erneuter Acylierung mit Chlorameisensäureestern zugänglich sind.

Anschließende Alkylierung von (I-B-6"a) mit entsprechenden Alkylierungsmittel $R^{10}$-$LG^2$ ergibt 4-Aryl-1,2,4-triazolidin-3,5-dione der allgemeinen Formel (I-B-6a), wobei $LG^2$ für klassische Abgangsgruppen wie Iodid, Bromid, Chlorid, Tosylat, Mesylat oder Triflat stehen.

Verfahren Vc1 - Vc11 eignen sich zur Darstellung der Ausführungsform I-C der Verbindungen der Formel (I).

**Verfahren Vc1 & Vc2 (zur Herstellung von I-C-1)**

Die 2-Aryl-1,2,4-triazolidin-3,5-dionen der allgemeinen Formel (I-C-1) können in (I-C-1a) (für n=0) und (I-C-1b) (für n=1) unterteilt werden.

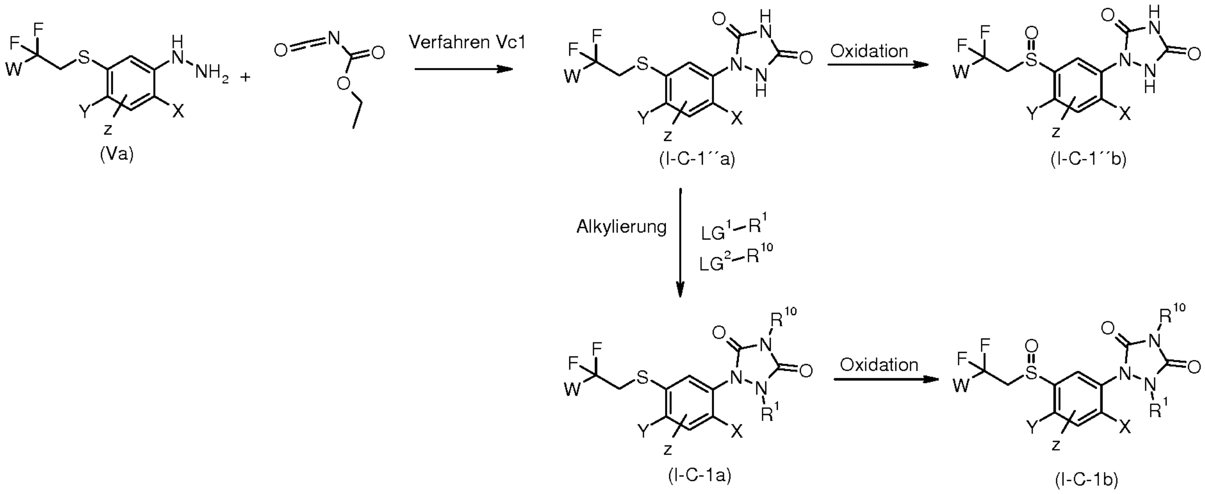


2-Aryl-1,2,4-triazolidin-3,5-dione der allgemeinen Formel (I-C-1"a) bzw. (I-C-1a) können alternativ nach Verfahren Vc1 durch Kondensation von Hydrazinen der Formel (Va) mit Ethylisocyanatocarbonat hergestellt werden, wie zum Beispiel in Monatsh. Chem. 1999, 130, 2, 327-332 beschrieben wird.

Verfahren Vc2

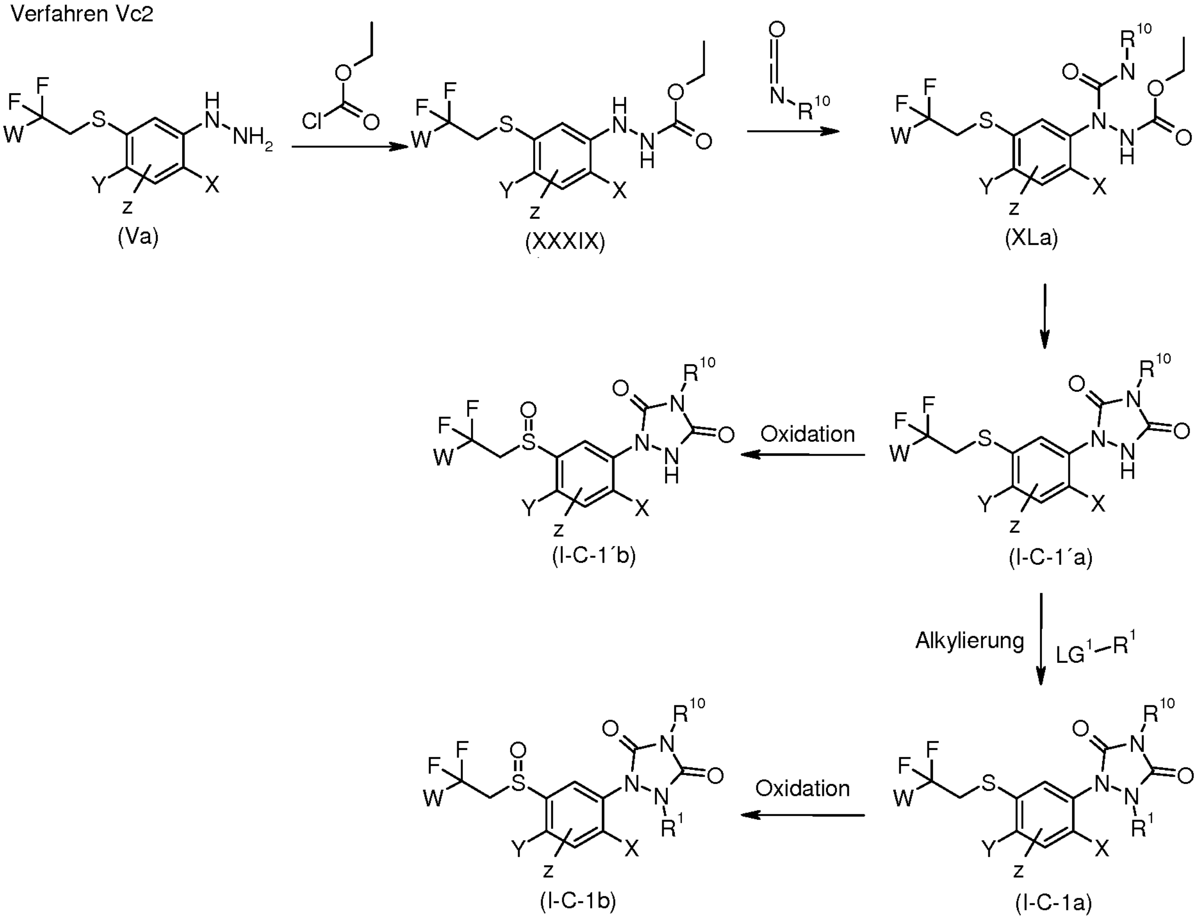


Nach J. Org. Chem. 1991, 56, 5643-5651 können 2-Aryl-1,2,4-triazolidin-3,5-dione der allgemeinen Formel (I-C-1a) alternativ nach Verfahren Vc2 hergestellt werden. Durch Kondensation von Hydrazinen der Formel (Va) mit Chlorameisensäureestern werden die entsprechenden Ethylphenylhydrazincarboxylaten (XXXIXa) erhalten, die anschließend mit Isocyanaten zu den entsprechenden substituierten Ethyl-2-(carbamoyl)-2-phenylhydrazincarboxylaten der allgemeinen Formel (XLa) reagieren und anschließend mit starken Basen wie zum Beispiel Kaliumhydroxid zu den 2-Aryl-1,2,4-triazolidin-3,5-dionen der allgemeinen Formel (I-C-1'a) cyclisiert werden können.

Anschließende Alkylierung mit entsprechendem Alkylierungsmittel $R^1$-$LG^1$ ergibt 2-Aryl-1,2,4-triazolidin-3,5-dione der allgemeinen Formel (I-C-1a), wobei $LG^1$ für eine klassische Abgangsgruppen wie Iodid, Bromid, Chlorid, Tosylat, Mesylat oder Triflat steht. Literaturvorschriften zur Alkylierung finden sich zum Beispiel in J. Org. Chem. 1991, 56, 5643-5651, in Amer. Chem. J. 1910, 43, 532-543 oder in Amer. Chem. J. 1910, 43, 380.

**Verfahren Vc3 & Vc4 (zur Herstellung von I-C-1)**

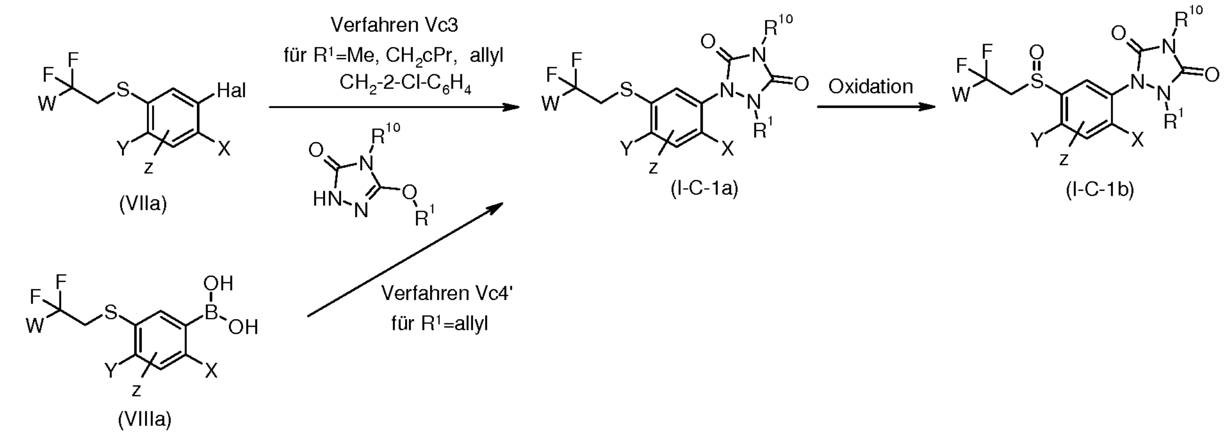

(VIIa) (I-C-1a) (I-C-1b) (VIIIa)

2-Aryl-1,2,4-triazolidin-3,5-dione der allgemeinen Formel (I-C-1a) können alternativ nach Verfahren Vc3 durch Umsetzung von Arylhalogeniden der Formel (VIIa) mit Triazolinonen hergestellt werden, wobei $R^1$ in diesem Fall für Methyl, cyclo-Alkyl-alkyl oder substituiertes Benzyl stehen kann. Bevorzugt findet die Umsetzung durch Übergangsmetall-Katalyse oder -Vermittlung statt, zum Beispiel durch Einsatz von Kupfersalzen wie zum Beispiel Kupfer(I)iodid in Anwesenheit von Liganden wie zum Beispiel N,N'-Dimethyl-1,2-cyclohexandiamin und einer Base wie Kaliumcarbonat in geeigneten Lösungsmitteln wie Dioxan oder Toluol statt.

Für $R^1$ = Allyl können 2-Aryl-1,2,4-triazolidin-3,5-dione der allgemeinen Formel (I-C-1a) alternativ nach Verfahren Vc4 durch Umsetzung von Boronsäuren der Formel (VIII) mit Triazolinonen hergestellt werden. Bevorzugt finden die Reaktionen unter Katalyse oder Vermittlung durch Kupfer(II)-salze wie zum Beispiel Kupfer(II)-acetat, unter Luft- oder Sauerstoffatmosphäre, unter wasserentziehenden Bedingungen (zum Beispiel mit Molekularsieb) statt. Als Basen werden zum Beispiel Triethylamin oder Pyridin in geeigneten Lösungmitteln wie zum Beispiel Dichlormethan verwendet.

**Verfahren Vc5 & Vc6 (zur Herstellung von I-C-2)**

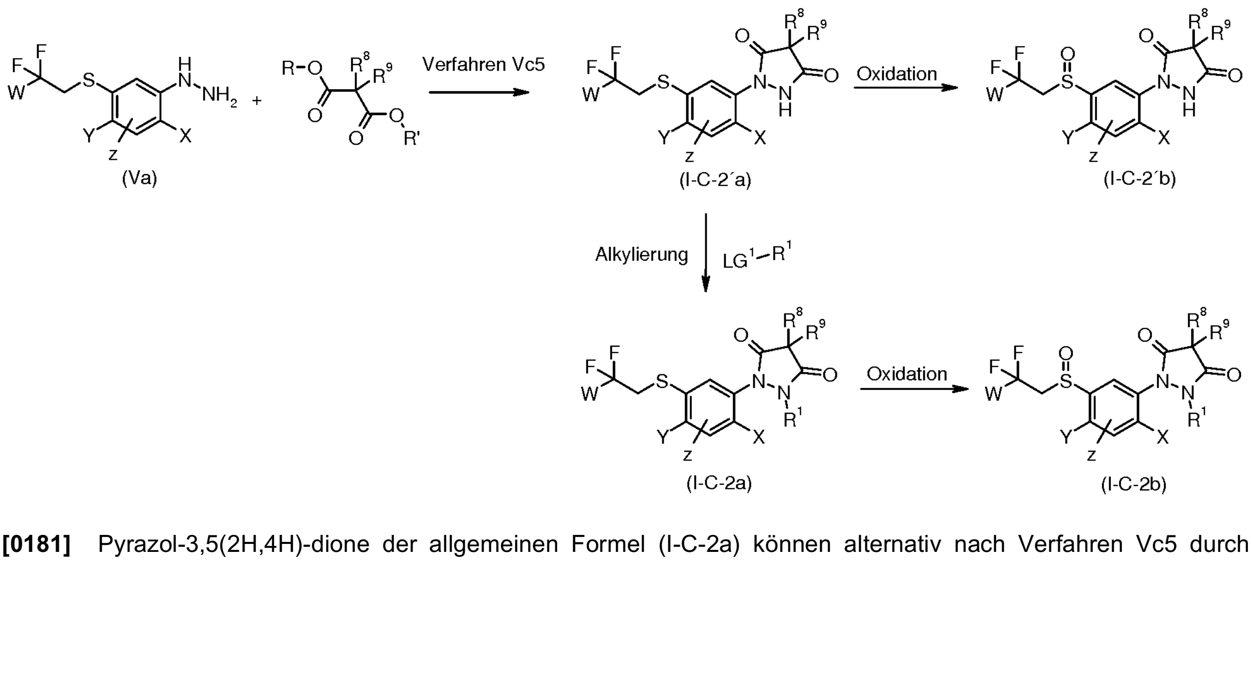

(Va) (I-C-2´a) (I-C-2´b) (I-C-2a) (I-C-2b)

Pyrazol-3,5(2H,4H)-dione der allgemeinen Formel (I-C-2a) können alternativ nach Verfahren Vc5 durch

Kondensation von Hydrazinen der Formel (Va) mit Malonsäuren oder Malonsäurediestern hergestellt werden wie zum Beispiel in Helv. Chim. Acta 1953, 36, 75-81, Can. J. of Research, Section B 1950, 28, 720-725 oder Tetrahedron Letters 2010, 51, 5328-5332 beschrieben wird.

Pyrazol-3,5(2H,4H)-dione der allgemeinen Formel (I-C-2a) können alternativ nach Verfahren Vc6 hergestellt werden wie zum Beispiel in Arch. Pharm. 1980, 313, 577-582 beschrieben wird. Hierbei werden durch Kondensation von Hydrazinen der Formel (Va) mit Cyanessigsäuren mit Dicyclohexylcarbodiimid zunächst die jeweiligen 2-Cyan-N'-Arylacetohydrazide erhalten, die unter sauren Bedingungen wie z. B. mit verdünnte Schwefelsäure zu den entsprechenden Iminopyrazolidinonen (XLIa) cyclisiert werden. Hydrolyse mit verdünnter Base wie z. B. Natriumhydroxid liefert Pyrazol-3,5(2H,4H)-dione der allgemeinen Formel (I-C-2'a).

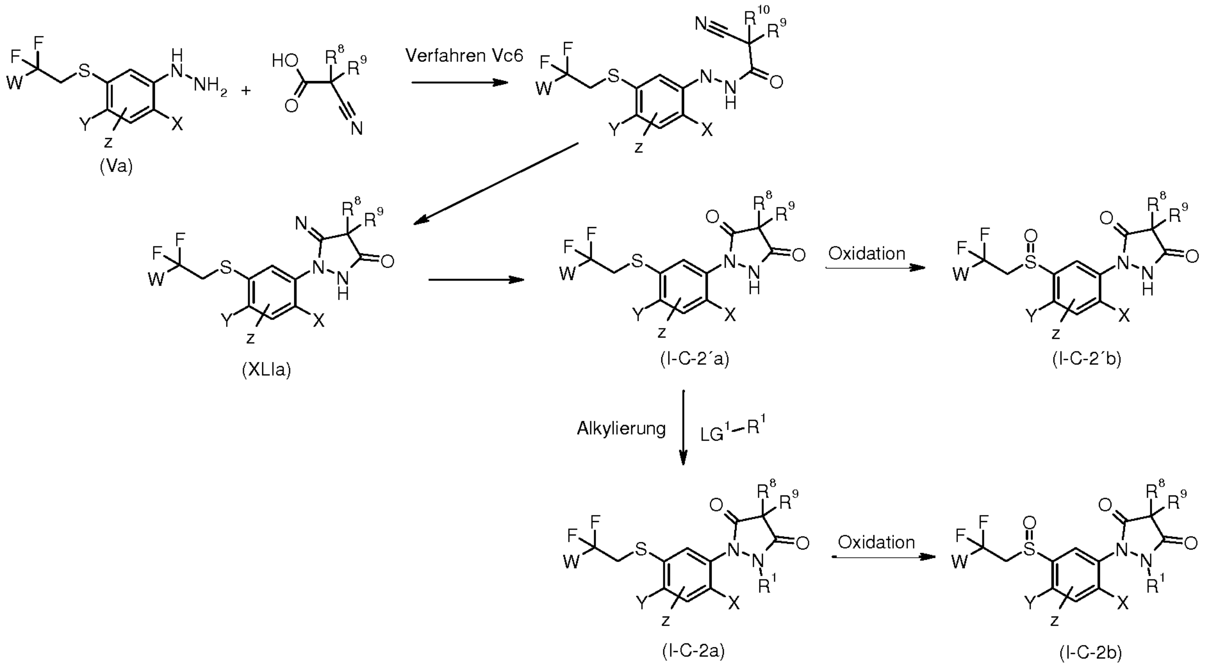


Anschließende Alkylierung mit entsprechendem Alkylierungsmittel $R^1$-$LG^1$ ergibt Pyrazol-3,5(2H,4H)-dione der allgemeinen Formel (I-C-2a), wobei $LG^1$ für eine klassische Abgangsgruppen wie Iodid, Bromid, Chlorid, Tosylat, Mesylat oder Triflat steht. Literaturvorschriften zur Alkylierung finden sich zum Beispiel in Arch. Pharm. 1958, 291, 404 oder in Chem. Ber. 1908, 41, 3872.

## Verfahren Vc7 und Vc8 (I-C-3)

Für die Herstellung der Imidazolidin-2,4-dione der allgemeinen Formel (I-C-3) können Verfahren Vc7 und Verfahren Vc8 beispielsweise verwendet werden.

Die Imidazolidin-2,4-dione der allgemeinen Formel (I-C-3) können in (I-C-3a) (für n=0) und (I-C-3b) (für n=1) unterteilt werden.

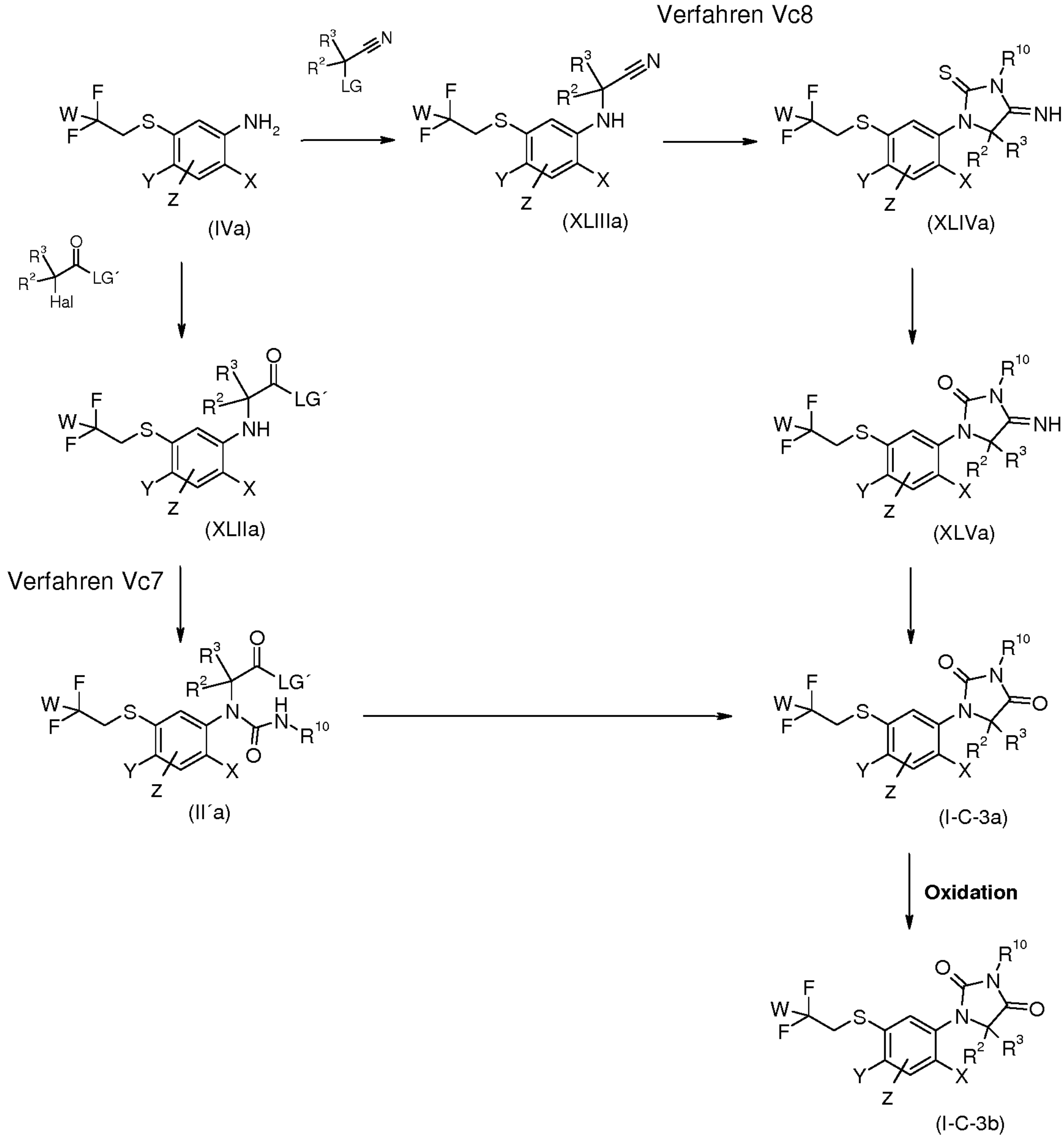


wobei W, X, Y, Z, $R^2$, $R^3$ und $R^{10}$ die oben angegebenen Bedeutungen haben, n kann für 0 oder 1 stehen, Hal für Halogen (bevorzugt Chlor und Brom) und LG und LG' für eine Abgansgruppe (z.B. Halogen, Hydroxy, Alkoxy) stehen.

**Verfahren Vc7**

Aniline der Formel (IVa) können mit adäquaten Halogencarbonylverbindungen nach literaturbekannten Methoden alkyliert werden. Zum Beispiel für $R^2$=$R^3$=Wasserstoff kann die Reaktion mit Bromessigsäureethylester nach US2012/277273 erfolgen. Die alkylierten Anilinen der Formel (XLIIa) können mit Isocyanaten oder 4-Nitrophenylalkylcarbamaten zu den Harnstoffen der Formel (II'a), gegebenenfalls mit Hilfe einer Base, umgesetzt werden, wie im US2012/277273. Anschließend erfolgt der Ringschluss zu den Imidazolidin-2,4-dionen (I), zum Beispiel nach US2012/277273, in dem die alkylierten Harnstoffe (II'a) mit einer Base in einem inerten organischen Lösungsmittel, gegebenenfalls bei erhöhter Temperatur, versetzt werden. Alternativ kann die Cyclisierung durch Versetzung der Harnstoffe (II'a) gegebenenfalls in einem Lösungsmittel mit neutralem Alox.

Alternativ können die Imidazolidin-2,4-dione der Formel (I) nach ***Verfahren*** Vc8 hergestellt werden, nach WO2006/124118.

Aniline der Formel (IV) können mit adäquaten Nitrilverbindungen zu den Verbindungen (XLIIIa) alkyliert werden. Zum Beispiel für $R^2$=$R^3$=Methyl kann die Reaktion mit 2-Hydroxy-2-methylpropanonitril erfolgen. Alternativ kann die Herstellung von den alkylierten Anilinen (XLIIIa) aus den Anilinen (IVa) durch Reaktion mit einer Cyanidquelle und einem Keton, z.B. mit Kaliumcyanid und Aceton in Essigsäure wie im Bull. Chem. Soc. Japan 1926, 1, 202. Die alkylierten

Anilinen der Formel (XLIIIa) können mit Isothiocyanaten zu den 3-Aryl-5-imino-imidazolidinthionen der Formel (XLIVa), gegebenenfalls mit Hilfe einer Base, in einem inerten organischen Lösungsmittel, umgesetzt werden, wie im US2012/277273. Die Imidazolidinthione (XLIVa) können zu den Imidazolidinonen (XLVa) führen, wenn sie mit einer Säure (beispielsweise Salzsäure) behandelt werden. Anschließend durch Reaktion mit einem Oxidationsmittel können die Imidazolidinonen (XLVa) in den Imidazolidindionen (I-C-3a) umgewandelt werden.

**Verfahren Vc9 (zur Herstellung von I-C-3)**

Imidazolidin-2,4-dione der Formel (I-C-3), wo $R^4$ für Wasserstoff steht, können auch nach Verfahren Vc9 hergestellt werden.

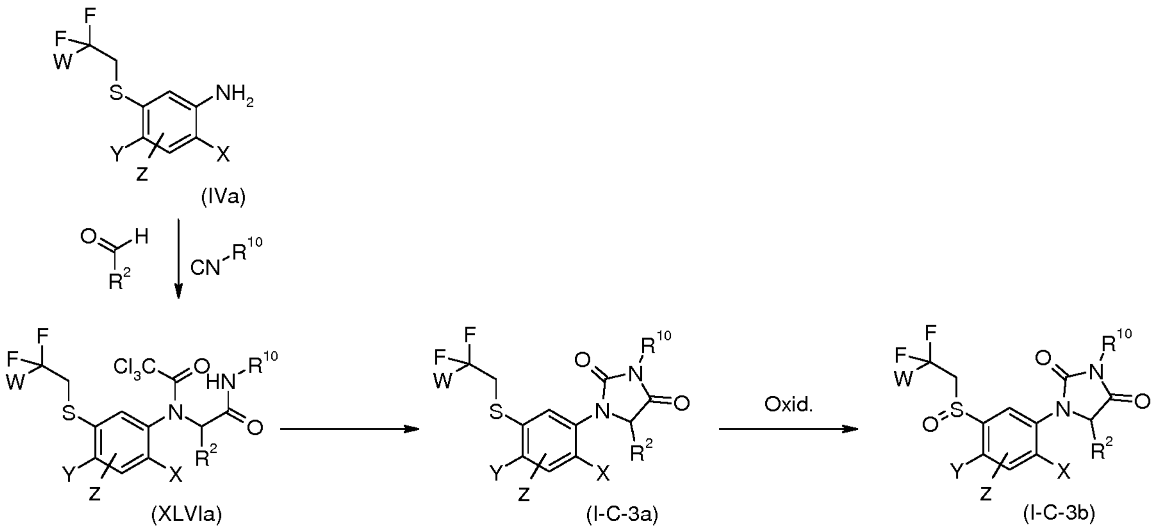


Aniline der Formel (IVa) können zu den 2-*N*-Trichloroacetylaminoacetamiden der Formel (XLVIa) nach literaturbekannten Methoden umgesetzt werden, zum Beispiel nach Tetrahedron 2012, 68, 2621-2629. Dafür wird das benötigte Aldehyd mit dem Anilin (IVa) versetzt, zum Beispiel in Methanol bei Raumtemperatur, und das resultierende Imin wird mit einem Isocyanid und Trichloressigsäure umgesetzt. Aus den 2-N-Trichloroacetylaminoacetamiden (XLVIa) lassen sich die Imidazolidin-2,4-dione der allgemeinen Formel (I-C-3a) herstellen, zum Beispiel durch Reaktion mit Natriumethanolat in Methanol, wie im oben genannten Artikel beschrieben.

**Verfahren Vc10 (zur Herstellung von I-C-4)**

Die 5-Thioxo-1,2,4-triazolidin-3-one der allgemeinen Formel (I-C-4) können in (I-C-4a) (für n=0) und (I-C-4b) (für n=1) unterteilt werden und können beispielsweise nach Verfahren Vc10 hergestellt werden.

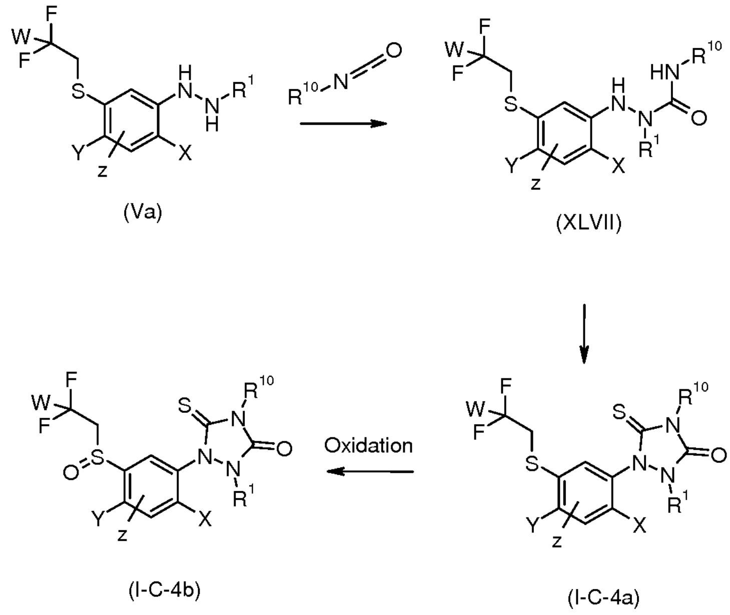


(Va) (XLVII)

(I-C-4b) (I-C-4a)

wobei W, X, Z, z, $R^1$ und $R^{10}$ die oben angegebene Bedeutung haben.

Durch die Umsetzung von Hydrazinen der allgemeinen Formel (Va) mit Isocyanaten ($R^{10}$-NCO) werden die Semicarbazide der Formel (XLVII) erhalten, die durch die anschließende Umsetzung mit z.B. 1,1'-Thiocarbonyldiimidazol in die 1-Aryl-5-thioxo-1,2,4-triazolidin-3-one der Formel (I-C-4a) umgewandelt werden.

Die Herstellung der Semicarbazide der Formel (XLVII) erfolgt nach Verfahren, die z.B. in Rec. Trav. Chim. 52, 979 (1933) oder in den Chem. Ber. 36, 1362 (1903) beschrieben werden.

Ein weiteres Herstellverfahren für 1-Aryl-5-thioxo-1,2,4-triazolidin-3-one besteht in der selektiven Umwandlung einer Carbonylgruppe entsprechender 1-Aryl-1,2,4-triazolin-3,5-dione in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Xylol oder Cumol. Dieses Verfahren ist z.B. in der DE 2554866 beschrieben.

Die Synthese von 1-Aryl-1,2,4-triazolin-3,5-dionen durch die Umsetzung von 1-Alkoxycarbonyl-Hydrazinen mit Isocyanaten ist ebenfalls in der DE 2554866 beschrieben. Die Verwendung von Isothiocyanaten liefert die entsprechenden 1-Aryl-5-thioxo-1,2,4-triazolidin-3-one.

In Berichte der Deutschen Chemischen Gesellschaft (1910),42, 4763-4769 und (1911),44, 560-583 werden 1-Aryl-5-thioxo-1,2,4-triazolidin-3-one durch die Umsetzung spezieller Thiosemicarbazide mit Phosgen hergestellt.

Die Herstellung und Verwendung von 1-Aryl-1,2,4-triazolin-3,5-dionen als Herbizide und Insektizide werden in der WO 9726248 beschrieben.

Die Herstellung weiterer 1-Aryl-1,2,4-triazolin-3,5-dion-Vorstufen wird auch beschrieben in der US 3663564 und der US 3621099.

**Verfahren Vc11 (zur Herstellung von I-C-5 und I-C-6)**

Die 1-Aryl-5-Thioxo-1,2,4-triazolidin-3-one der allgemeinen Formel (I-C-5) können in (I-C-5a) (für n=0) und (I-C-5b) (für n=1) unterteilt werden und können beispielsweise nach Verfahren Vc11 hergestellt werden.

1-Aryl-1,2,4-triazolidin-3,5-dithione der allgemeinen Formel (I-C-6) können in (I-C-6a) (für n=0) und (I-C-6b) (für n=1) unterteilt werden und können beispielsweise nach Verfahren Vc11 hergestellt werden.

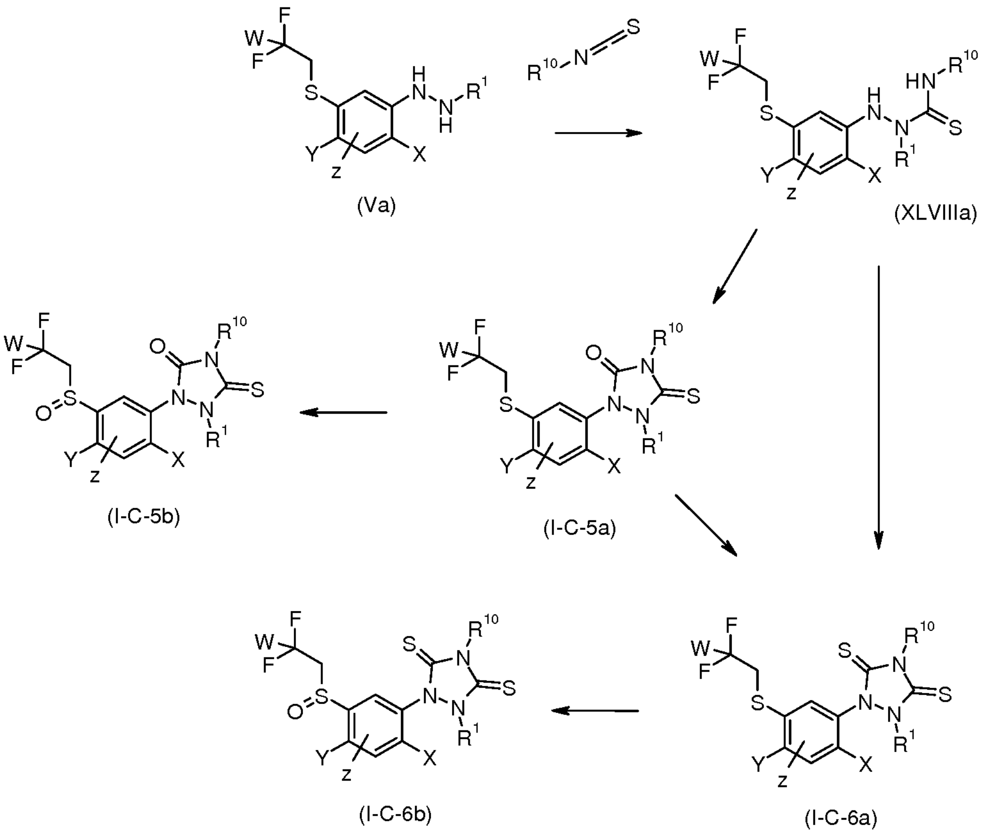
(Va)
(XLVIIIa)
(I-C-5b)
(I-C-5a)
(I-C-6b)
(I-C-6a)

wobei W, X, Z, z, $R^1$ und $R^{10}$ die oben angegebene Bedeutung haben.

Durch die Umsetzung von Hydrazinen der allgemeinen Formel (Va) mit Isothiocyanaten ($R^{10}$-NCS) werden die Thiosemicarbazide der allgemeinen Formel (XLVIIIa) erhalten, die durch die anschließende Umsetzung mit z.B. 1,1'-Carbonyldiimidazol in die 1-Aryl-5-Thioxo-1,2,4-triazolidin-3-one der Formel (I-C-5a) oder durch die anschließende Umsetzung mit z.B. 1,1'-Thiocarbonyldiimidazol in die 1-Aryl-1,2,4-triazolidin-3,5-dithione der Formel (I-C-6a) umgewandelt werden.

Die Herstellung der Thiosemicarbazide der Formel (XLVIII) erfolgt nach Verfahren, die z.B. in Rec. Trav. Chim. 55, 101 (1936), in J. Am. Chem. Soc. 1552 (1931) oder in den Chem. Ber. 34, 320 (1901) beschrieben werden.

Ein weiteres allgemeines Herstellverfahren für die 2-Aryl-5-thioxo-1,2,4-triazolidin-3-one und der 2-Aryl-1,2,4-triazolidin-3,5-dithione besteht in der selektiven Umwandlung einer Carbonylgruppe entsprechender 1-Aryl-1,2,4-triazolin-3,5-dione in die Thiocarbonylgruppe mit Hilfe geeigneter Schwefelungsreagenzien wie beispielsweise Phosphorpentasulfid oder Lawssons Reagenz in einem geeigneten Lösungsmittel, beispielsweise Xylol oder Cumol. Dieses Verfahren ist z.B. in der DE 2554866 beschrieben.

Die Synthese der entsprechenden 1-Aryl-1,2,4-triazolin-3,5-dione durch die Umsetzung von 1-Alkoxycarbonyl-Hydrazinen mit Isocyanaten ist ebenfalls in der DE 2554866 beschrieben. Die Verwendung von 1-Alkoxythiocarbonyl-Hydrazinen ergibt 1-Aryl-5-thioxo-1,2,4-triazolidin-3-one.

In Berichte der Deutschen Chemischen Gesellschaft (1910),42, 4763-4769 und (1911),44, 560-583 werden die 1-Aryl-5-thioxo-1,2,4-triazolidin-3-one durch die Umsetzung spezieller Thiosemicarbazide mit Phosgen hergestellt.

Durch Oxidation der Thioether der Formel (I-C-5a) bzw. (I-C-6a) nach literaturbekannten Methoden werden die Sulfoxide der Formel (I-C-5b) bzw. (I-C-6b) erhalten.

Verfahren Vd1 - Vd3 eignen sich zur Darstellung der Ausführungsform I-D der Verbindungen der Formel (I).

**Verfahren Vd1 (I-D-1)**

Die 3-Arylimidazolidin-2,4,5-trione der allgemeinen Formel (I-D-1) können in (I-D-1a) (für n=0) und (I-D-1b) (für n=1) unterteilt werden und können beispielsweise nach Verfahren Vd1 hergestellt werden.

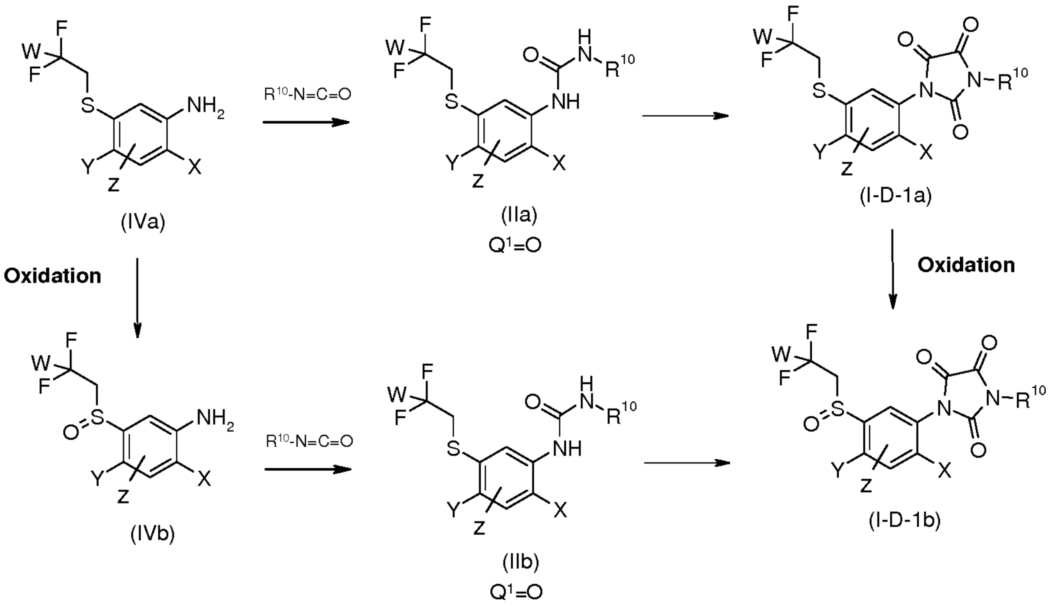


(IVa) (IIa) $Q^1$=O (I-D-1a)

(IVb) (IIb) $Q^1$=O (I-D-1b)

Die Herstellung von Phenylharnstoffen der Formel (II, $Q^1$=O) erfolgt nach literaturbekannten Methoden, beispielsweise beschrieben im Patent JP2011/042611.

Durch die Umsetzung von Phenylharnstoffen der Formel (II) mit Oxalylchlorid werden die 1-R-3-Arylimidazolidin-2,4,5-trione der Formel (I-D-1) erhalten, in Analogie zu den Vorschriften aus J. Korean Chem. Soc. (1999), 43(4), 491-493 oder JP 1976-92865 oder Tetrahedron Lett. 2012, 53, 4758-4762.

Weitere Synthesevarianten sind beschrieben in Synthesis (1977), (9), 641-2 oder Liebigs Ann. Chem. (1927), 458, 76-92 oder Recl. Trav. Chim. Pays-Bas (1915), 34 289-325 oder J. Fluorine Chem. 2011,132, 596-611 oder Bioorg. Med. Chem. Lett. (2009), 19(9), 2570-2573 oder Asian J. Chem. (2007), 19(2), 1455-1460 oder J. Het. Chem. 2003, 40(5), 885-893 oder US 20030119889 oder US 20030119890 oder J. Het. Chem. (1994), 31(6), 1535-9 oder J. Het. Chem. (1971), 8(4), 669-70 oder Pharmazie (1990), 45(10), 795-6 oder Zeitschrift f. Chemie (1989), 29(8), 281-3 oder J. Org. Chem., 1979, 44 (22), 3858-3861 oder Synthesis (1977), (9), 641-2 oder in der DE 1969-1916932 bzw. der DE 19660523.

Einige 3-Arylimidazolidin-2,4,5-trione sind auch kommerziell erhältlich, z.B. 1-[3 (Methylsulfanyl)phenyl]imidazolidin-2,4,5-trion [CAS-RN: 1152595-83-9] .

**Verfahren Vd2 (zur Herstellung von I-D-2)**

Die Pyrrolidin-2,3,5-trione der allgemeinen Formel (I-D-2) können in (I-D-2a) (für n=0) und (I-D-2b) (für n=1) unterteilt werden. Sie können beispielsweise nach Verfahren Vd2 hergestellt werden

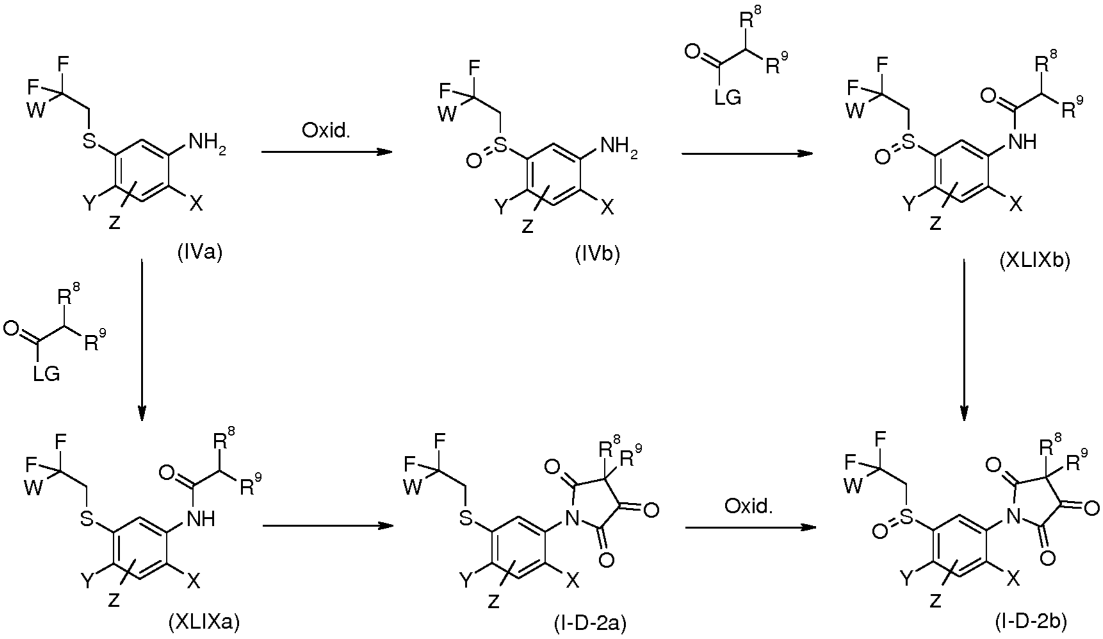


(IVa) (IVb) (XLIXb) (XLIXa) (I-D-2a) (I-D-2b)

wobei W, X, Y, Z, $R^8$ und $R^9$ die oben angegebenen Bedeutungen haben und LG für OH oder Halogen (insbesondere Chlor) steht.

Aniline der Formel (IV) können mit Carbonsäuren oder Carbonsäurederivaten (insbesondere Chloride) zu den Amiden der Formel (XLIX) umgesetzt werden, gegebenenfalls in Anwesenheit einer Base und gegebenfalls in Anwesenheit eines organischen Lösungsmittels, wie zum Beispiel von G. S. Skinner in J. Amer. Chem. Soc. 1950, 72, 5569-5573, beschrieben. Die Amiden (XLIX) können zum Beispiel mit Oxalylchlorid oder Oxalsäurediethylester versetzt werden, um die Pyrrolidin-2,3,5-trionen (I) zu liefern. Wie von G. S. Skinner beschrieben, kann die Umsetzung in einem inerten organischen Lösungsmittel wie Toluol bei 60 °C erfolgen. Alternativ, und je nach Natur der $R^8$ und $R^9$ Reste, können die Amiden (XLIX) mit Oxalsäurediethylester zu Oxazolidindionen führen, die dann in einem Alkohol zu den gewünschten Pyrrolidintrionen der Formel (I-D-2) umlagern. Dies wurde vom G. S. Skinner in J. Amer. Chem. Soc. 1956, 77, 4656-4659, veröffentlicht.

**Verfahren Vd3 (zur Herstellung von I-D-3)**

Die 2-Thioxoimidazolidin-4,5-dione der allgemeinen Formel (I-D-3) können in (I-D-3a) (für n=0) und (I-D-3b) (für n=1) unterteilt werden. Sie können beispielsweise nach Verfahren Vd3 hergestellt werden.

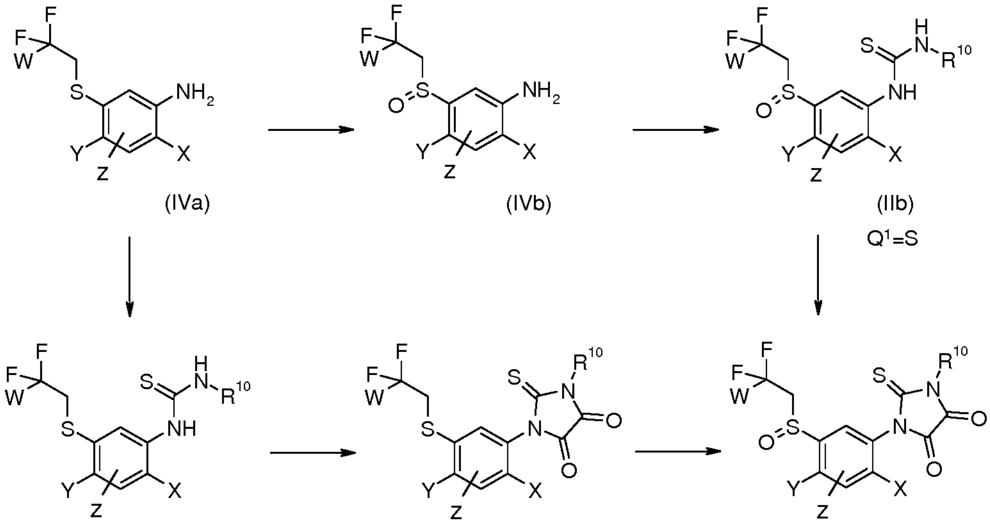

(IVa) (IVb) (IIb) $Q^1$=S

(IIa) $Q^1$=S (I-D-3a) (I-D-3b)

wobei W, X, Y, Z, A und $R^{10}$ die oben angegebenen Bedeutungen haben.

Aniline der Formel (IV) können zu den Thioharnstoffen der Formel (II) nach literaturbekannten Methoden umgesetzt werden, zum Beispiel nach JP2011/042611, in dem sie mit Isothiocyanaten, gegebenenfalls in Anwesenheit einer Base und gegebenfalls in Anwesenheit eines organischen Lösungsmittels, versetzt werden oder in dem sie nach allgemein bekannten Methoden in deren Isothiocyanaten umgewandelt und diese dann mit Aminen zu den Thioharnstoffen umgesetzt werden.

Aus den Thioharnstoffen der allgemeinen Formel (II) kann die Synthese der 2-Thioxoimidazolidin-4,5-dione der allgemeinen Formel (I-D-3a) bzw. (I-D-3b) erfolgen, zum Beispiel durch Umsetzung mit Oxalsäuredichlorid oder Oxalsäureethylesterchlorid in einem inerten Lösungsmittel, wie Chloroform, Dichlormethan oder Acetonitril, gegebenenfalls bei erhöhter Temperatur. Beispiele solcher Reaktionen finden sich unter anderem in J. Med. Chem. 2004, 47, 681-695 und US2003/119889.

**Reaktionen in der Mikrowelle**

Bei der Durchführung der erfindungsgemäßen Verfahren kann gegebenenfalls jede für diese Reaktionen geeignete, handelsübliche Mikrowellenapparatur verwendet werden (z.B. Anton Paar Monowave 300, CEM Discover S, Biotage Initiator 60).

**Thionierung**

Ein weiteres allgemeines Verfahren zur Herstellung der erfindungsgemäßen Verbindungen der allgemeinen Formel (Ia) oder (Ib), in denen V für Schwefel steht, besteht in der Umwandlung der Carbonylgruppe entsprechender Vorstufen in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Pyridin, Xylol oder Cumol. Diese Variante ist in zahlreichen Publikationen beschrieben, z.B. in J. Amer. Chem. Soc. 1956, 1938-1941, Chem. Pharm. Bull. 1962, 10, 647-652, US 3007927, DE 2554866 oder WO 2000026194.

**Allgemeine Herstellverfahren für die Oxidation von Thioethern zu Sulfoxiden**

Verbindungen der allgemeinen Formel (Ib) lassen sich durch Oxidation nach literaturbekannten Verfahren aus Verbindungen der allgemeinen Formel (Ia) herstellen, beispielsweise durch ein Oxidationsmittel in einem geeignetem Lösungs- und Verdünnungsmittel. Als Oxidationsmittel eignen sich zum Beispiel verdünnte Salpetersäure, Wasserstoffperoxid und Peroxycarbonsäuren, wie etwa meta-Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum Beispiel mit Titatium und Vanadium als meistbenutzten Katalysatorquellen, in Form von $Ti(O^iPr_4)$ und $VO(acac)_2$, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nichtmetall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC getrennt werden.

**Erläuterung der Ausgangsstoffe und Zwischenprodukte**

Aniline der Formel (IV), der Halogenide der Formel (VII), Boronsäuren der Formel (VIII), Hydrazine der Formel (V) und Isocyanate der Formel (III) sind zentrale Bausteine um die Verbindungen der Formel (I) herzustellen.

Die ***Aniline der allgemeinen Formel (IV)*** lassen sich in Verbindungen mit n=0 (IVa) und n=1 (IVb) unterteilen.

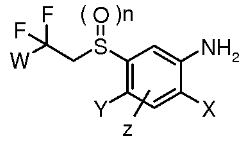

(IVa) n=0
(IVb) n=1

Aniline der Formel (IVa) sind teilweise literaturbekannt, z.B. aus JP 2007/284356, oder können anhand literaturbekannter Verfahren synthetisiert werden, insbesondere nach den in den Herstellbeispielen genannten Bedingungen.

Die Verbindungen der Formel (IVb) sind neu und lassen sich durch Oxidation, insbesondere nach den in den Herstellbeispielen genannten Bedingungen, herstellen.

Die Aniline der allgemeinen Formel (IVa) können beispielsweise wie im folgenden Schema hergestellt werden.

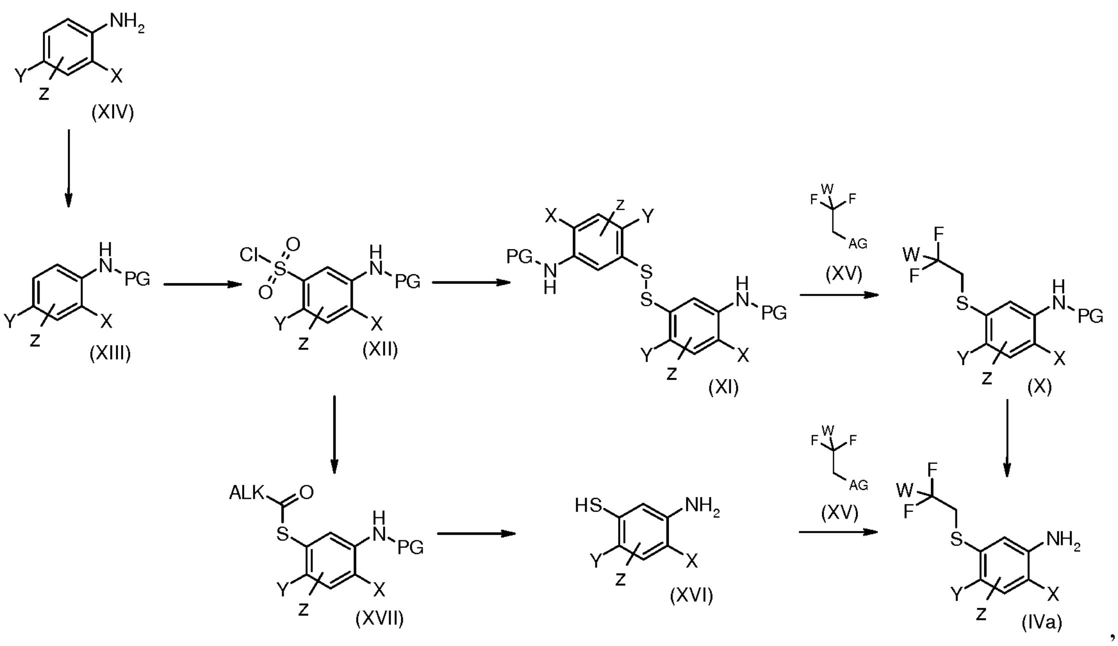

,

wobei X, Y, Z und W die oben angegebenen Bedeutungen haben, AG für eine Abgangsgruppe und PG für eine Schutzgruppe steht.

Aniline der Formel (XIV) sind entweder kommerziell erhältlich oder können durch bekannte Methoden hergestellt werden. Sie können mit einer geeigneten Schutzgruppe, wie z.B. einer Acetylgruppe, zu Verbindungen der Formel (XIII) geschützt werden. Beispielsweise in Gegenwart von Säuren, Säureanhydriden oder Säurechloriden können die Aniline (XIV) in die entsprechenden Anilide (XIII) überführt werden. Die Chlorsulfonierung der geschützen Aniline (XIII) mit Chlorsulfonsäure liefert die entsprechenden Sulfonylchloride (XII). Die Reduktion der Sulfonylchloride (XII) in die Disulfide (XI) ist mit literaturbekannten Methoden wie zum Beispiel Eisen in Salzsäure oder Iodid möglich. Die Umsetzung der Disulfide (XI) mit Haloalkylelektrophilen der Formel (XV), wobei AG für eine Abgangsgruppe wie zum Beispiel Chlor, Brom, Tosylat, Mesylat oder Triflat steht, liefert die Sulfide (X). Die Schutzgruppe kann durch geeignete literaturbekannte Methoden abgespalten werden, so dass man Aniline der Formel (IVa) erhält.

Anstatt der Reduktion zum Disulfid (XI), kann das Sulfonylchlorid (XII) mit einem geeigneten Reduktionsmittel wie zum Beispiel Iod/Phosphor zum Alkylthioat (XVII) reduziert und anschließend durch eine geeignete Methode, wie zum Beispiel die Umsetzung mit Kaliumhydroxidlösung, zu Thiolen der Formel (XVI) entschützt werden. Die Umsetzung der Thiole (XVI) mit Haloalkylelektrophilen der Formel (XV), wobei AG für eine Abgangsgruppe wie zum Beispiel Chlor, Brom, Tosylat, Mesylat oder Triflat steht, liefert die Sulfide (IVa).

Die Verbindungen der Formel (X), (XI), (XII), (XIII), (XVI) und (XVII) sind neu und lassen sich insbesondere nach den in den Herstellbeispielen genannten Bedingungen herstellen.

Ebenfalls bevorzugt können die Thioether der Formel (IVa) alternativ nach folgendem Schema hergestellt werden,

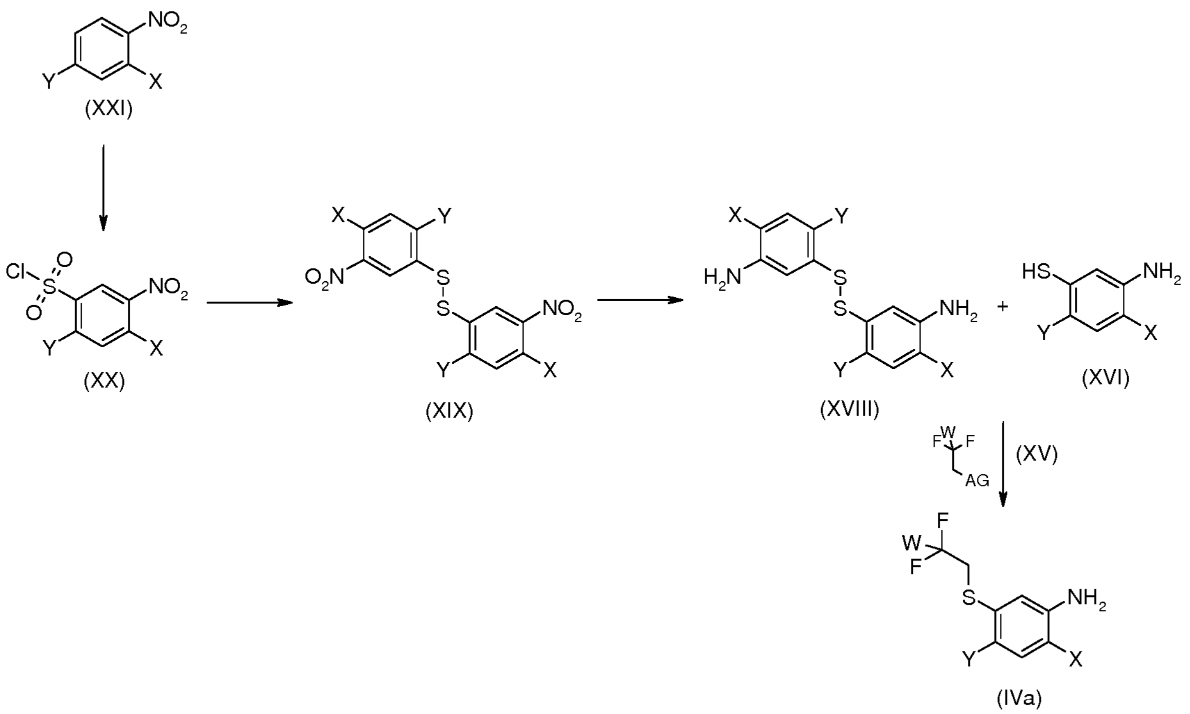
(XXI) (XX) (XIX) (XVIII) (XVI) (XV) (IVa)

wobei X, Y, Z und W die oben angegebenen Bedeutungen haben, AG für eine Abgangsgruppe und PG für eine Schutzgruppe steht.

Die Chlorsulfonierung der Nitroaromaten der Formel (XXI) mit Chlorsulfonsäure liefert die entsprechenden Sulfonylchloride (XX). Die Reduktion der Sulfonylchloride (XX) in die Bis(nitroaryl)disulfide (XIX) ist mit literaturbekannten Methoden, wie zum Beispiel Iodid, möglich. Die Reduktion der Disulfide (XXI) in die Disulfanediyldianiline (XIX), die teilweise als Gemisch mit den entsprechenden Aminoarylthiolen (XVI) entstehen, ist mit allgemein bekannten Reduktionsmittel, wie zum Beispiel Wasserstoff, gegebenenfalls mit Hilfe heterogener Katalysatoren, wie zum Beispiel Raney-Nickel, Platin auf Aktivkohle oder Palladium auf Aktivkohle, möglich. Die Umsetzung der Disulfide (XVIII) bzw. Thiophenole (XVI) mit Haloalkylelektrophilen der Formel (XV), wobei AG für eine Abgangsgruppe wie zum Beispiel Chlor, Brom, Iod, Tosylat, Mesylat oder Triflat steht, liefert die 3-[(2,2,2-Trifluorethyl)sulfanyl]aniline der Formel (IVa).

Die Verbindungen der Formel (XVI), (XVIII), (XIX) und (XX) sind neu und lassen sich insbesondere nach den in den Herstellbeispielen genannten Bedingungen herstellen.

**Halogenide der allgemeinen Formel (VIIa)**

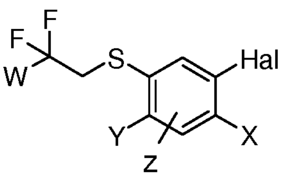
(VIIa)

in welcher X, Y, Z und W die oben angegebenen Bedeutungen haben und Hal für Chlor, Brom oder Iod steht, sind literaturbekannt aus WO 2007/034755, JP 2007/081019, JP 2007/284385, JP 2008/260706, JP 2008/308448, JP 2009/023910 oder WO 2012/176856 oder können anhand literaturbekannter Verfahren synthetisiert werden, die gegebenenfalls geringfügig modifiziert sein können, insbesondere wie in den konkreten Synthesebeipielen beschrieben.

Als Ausgangsstoffe für die Synthese der Iodide der allgemeinen Formel (VIIa) eignen sich Bromide derselben Formel, zum Beispiel in Halogenaustauschreaktionen nach literaturbekannten Methoden gegebenenfalls unter Metallkatalyse (s. H. Suzuki, Chem. Let. 1985, 3, 411-412; S. L. Buchwald, J. Amer. Chem. Soc. 2002, 124 (50), 14844-14845) . Ebenso ist die Synthese möglich ausgehend von Anilinen der Formel (IVa) unter Sandmeyers Reaktionsbedingungen, wie von E. B. Merkushev in Synthesis 1988, 12, 923-937, beschrieben.

**Boronsäuren der allgemeinen Formel (VIIIa) und (VIIIb)**

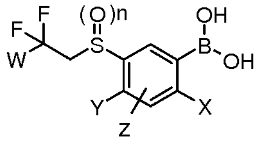

(VIIIa) n=0
(VIIIb) n=1

in welcher X, Y, Z und W die oben angegebenen Bedeutungen haben, sind literaturbekannt, z.B. aus WO2007/034755, JP2007/284385, JP2009/023910 und WO2012/176856 oder können anhand literaturbekannter Verfahren synthetisiert werden, insbesondere wie in den konkreten Synthesebeipielen beschrieben.

**Hydrazine der allgemeinen Formel (Va)**

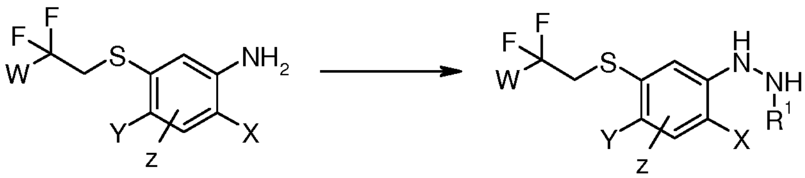

(IVa) (Va)

Hydrazine der allgemeinen Formel (Va) sind teilweise literaturbekannt, zum Beispiel aus EP 1803712 A1 und WO 2006043635, oder können anhand literaturbekannter Verfahren synthetisiert werden, wie zum Beispiel in J. Med. Chem. 2003, 46, 4405-4418 beschrieben.

**Isocyanate der Formel (IIIa)**

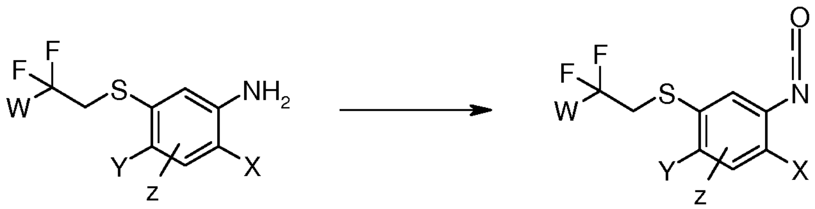

(IVa) (IIIa)

Isocyanate der allgemeinen Formel (IIIa) sind teilweise literaturbekannt, zum Beispiel aus JP 2011/042611 A, oder können anhand literaturbekannter Verfahren synthetisiert werden, wie zum Beispiel in EP 1183229 B1, in J. Amer. Chem. Soc. 1940, 62, 2965-2966, in Angew. Chem. 1995, 107, 2746-2749 oder in US2010/160388 A1 beschrieben, insbesondere wie in den konkreten Synthesebeipielen beschrieben.

**Heterocyclischen Verbindungen der Formel (XXII)**

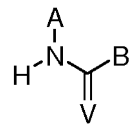

(XXII)

in welcher A und B die vorstehende Bedeutung aufweisen sind kommerziell erhältlich, literaturbekannt oder können anhand literaturbekannter Verfahren synthetisiert werden. Als Beispiele werden die unterschiedlichen heterocyclischen Verbindungen in ihre Subklassen unterteilt und dort genannt.

**Imidazolidin-2,4-dione der allgemeinen Formel (XXII-B-1)**

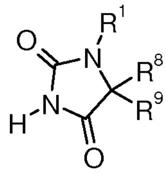

(XXII-B-1)

Imidazolidin-2,4-dione der allgemeinen Formel (XXII-B-1) sind kommerziell erhältlich, literaturbekannt oder können anhand literaturbekannter Verfahren synthetisiert werden. Als Beispiel sei genannt: 1,5,5-Trimethylimidazolidin-2,4-dion (kommerziell erhältlich)

**Pyrrolidin-2,5-dione der allgemeinen Formel (XXII-B-4)**

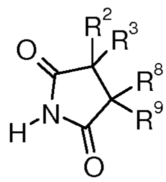

(XXII-B-4)

Pyrrolidin-2,5-dione der allgemeinen Formel (XXII-B-4) sind kommerziell erhältlich, literaturbekannt oder können anhand literaturbekannter Verfahren synthetisiert werden. Als Beispiel sei genannt: Pyrrolidin-2,5-dion (kommerziell erhältlich) **1,2,4-Triazolidin-3,5-dione der allgemeinen Formel (XXII-C-1)**

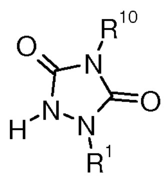

(XXII-C-1)

1,2,4-Triazolidin-3,5-dione der allgemeinen Formel (XXII-C-1) sind kommerziell erhältlich, literaturbekannt oder können anhand literaturbekannter Verfahren synthetisiert werden (siehe auch Synthesebeispiele). Als Beispiele seien genannt: 4-Methyl-1,2,4-triazolidin-3,5-dion, [16312-79-1] (kommerziell erhältlich), 4-Ethyl-1,2,4-triazolidin-3,5-dion (siehe zum Beispiel Bioorg. Med. Chem. 2010, 18, 1573-1582), 1,4-Diethyl-1,2,4-triazolidin-3,5-dion (siehe zum Beispiel J.

Org. Chem. 1986, 51, 1719-1723; J. Org. Chem. 1973, 38, 2442-2446; siehe Synthesebeispiele), 1-Ethyl-4-phenyl-1,2,4-triazolidin-3,5-dion (siehe zum Beispiel JP 53053655 A; WO 2012149335 A2; siehe Synthesebeispiele), 1-Benzyl-4-methyl-1,2,4-triazolidin-3,5-dion (siehe zum Beispiel WO 2012149335 A2), 1-Ethyl-4-methyl-1,2,4-triazolidin-3,5-dion (siehe zum Beispiel JP 53053655 A; WO 2012149335 A2),2,5-Difluor-4-(4-methyl-3,5-dioxo-1,2,4-triazolidin-1-yl)benzonitril (siehe zum Beispiel WO 9726248 A1), 1-(Cyclohex-2-en-1-yl)-4-methyl-1,2,4-triazolidin-3,5-dion (siehe zum Beispiel Org. Lett. 2000, 2, 1295-1297; J. Org. Chem. 1980, 45, 3472-3476), 1,4-Dimethyl-1,2,4-triazolidin-3,5-dion (siehe zum Beispiel Arch. Pharm. 1971, 304, 706-712; J. Org. Chem. 1991, 56, 5643-5651), 1-Allyl-4-methyl-1,2,4-triazolidin-3,5-dion (siehe zum Beispiel J. Org. Chem. 1981, 46, 614-9), 4-Cyclopropyl-1-isobutyl-1,2,4-triazolidin-3,5-dion, 4-tert-Butyl-1-methyl-1,2,4-triazolidin-3,5-dion, 4-tert-Butyl-1-ethyl-1 ,2,4-triazolidin-3,5-dion, 1-Cyclopentyl-4-methyl-1,2,4-triazolidin-3,5-dion, 1-Ethyl-4-isopropyl-1,2,4-triazolidin-3,5-dion, 1-Benzyl-4-cyclopropyl-1 ,2,4-triazolidin-3,5-dion, 4-Isopropyl-1-methyl-1,2,4-triazolidin-3,5-dion, 1-Methyl-4-[3-(trifluormethyl)phenyl]-1,2,4-triazolidin-3,5-dion, 4-Cyclopropyl-1-isopropyl-1,2,4-triazolidin-3,5-dion, 4-Cyclopropyl-1,2,4-triazolidin-3,5-dion, 1-Isopropyl-4-methyl-1,2,4-triazolidin-3,5-dion, 1-sec-Butyl-4-cyclopropyl-1,2,4-triazolidin-3,5-dion, 4-Ethyl-1-methyl-1,2,4-triazolidin-3,5-dion.

**2,4-Dihydro-3H-1,2,4-triazol-3-one**

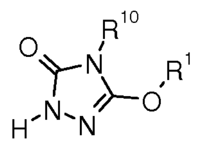

2,4-Dihydro-3H-1,2,4-triazol-3-one sind kommerziell erhältlich, literaturbekannt oder können anhand literaturbekannter Verfahren synthetisiert werden. Als Beispiele seien genannt: 4-Allyl-5-methoxy-2,4-dihydro-3H-1,2,4-triazol-3-on (siehe zum Beispiel US5541337 A1; EP 507171 A1), 4-Cyclopropyl-5-methoxy-2,4-dihydro-3H-1,2,4-triazol-3-on (siehe zum Beispiel US5541337 A1; EP 507171 A1; WO 2010063402 A1), 5-(Allyloxy)-4-cyclopropyl-2,4-dihydro-3H-1,2,4-triazol-3-on (siehe zum Beispiel US5541337 A1), 5-(Cyclopropylmethoxy)-4-methyl-2,4-dihydro-3H-1,2,4-triazol-3-on, 5-[(2-Chlorbenzyl)oxy]-4-methyl-2,4-dihydro-3H-1,2,4-triazol-3-on.

**Verwendung**

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp.,

Cryptorhynchus lapathi, Cylindrocopturus spp., Dermestes spp., Diabrotica spp., Dichocrocis spp., Diloboderus

spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp.

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp.

Aus der Ordnung der Hymenoptera z.B. Athalia spp., Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp.

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp.

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp.

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z.B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z.B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanolalkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z.B. Dioctylsulfosuccinat oder Hydroxypropyl-guar Polymere und/oder Humectants wie z.B. Glycerin und/oder Dünger wie beispielsweise Ammonium-, Kalium- oder Phosphorenthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die gegebenenfalls auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und

Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffe in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z.B. hohen oder niedrigen Temperaturen, gegen Trockenheit oder gegen erhöhten Wasser- bzw. Bodensalzgehalt. Auch lässt sich das Blüh- und Fruchtverhalten verbessern, die Keimfähigkeit und Bewurzelung optimieren die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die Folgenden:

**Insektizide/Akarizide/Nematizide**

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos,

Pyridaphenthion, Quinalphos, Sulfotep, Tebupirimfos, Temephos, Terbufos, Tetrachlorvinphos, Thiometon, Triazophos, Trichlorfon und Vamidothion.

(2) GABA-gesteuerte Chlorid-Kanal-Antagonisten, wie beispielsweise

Cyclodien-organochlorine, z.B. Chlordane und Endosulfan; oder

Phenylpyrazole (Fiprole), z.B. Ethiprole und Fipronil.

(3) Natrium-Kanal-Modulatoren / Spannungsabhängige Natrium-Kanal-Blocker, wie beispielsweise

Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder

DDT; oder Methoxychlor.

(4) Nikotinerge Acetylcholin-Rezeptor (nAChR) Agonisten, wie beispielsweise

Neonikotinoide, z.B. Acetamiprid, Clothianidin, Dinotefuran, Imidacloprid, Nitenpyram, Thiacloprid und Thiamethoxam; oder

Nikotin.

(5) Nikotinerge Acetylcholin-Rezeptor (nAChR) allosterische Aktivatoren, wie beispielsweise

Spinosine, z.B. Spinetoram und Spinosad.

(6) Chlorid-Kanal-Aktivatoren, wie beispielsweise

Avermectine/Milbemycine, z.B. Abamectin, Emamectin-benzoat, Lepimectin und Milbemectin.

(7) Juvenilhormon-Imitatoren, wie beispielsweise

Juvenilhormon-Analoge, z.B. Hydroprene, Kinoprene und Methoprene; oder

Fenoxycarb; oder Pyriproxyfen.

(8) Wirkstoffe mit unbekannten oder nicht spezifischen Wirkmechanismen, wie beispielsweise

Alkylhalide, z.B. Methylbromid und andere Alkylhalide; oder

Chloropicrin; oder Sulfurylfluorid; oder Borax; oder Brechweinstein.

(9) Selektive Fraßhemmer, z.B. Pymetrozine; oder Flonicamid.

(10) Milbenwachstumsinhibitoren, z.B. Clofentezine, Hexythiazox und Diflovidazin; oder

Etoxazole.

(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.

(12) Inhibitoren der oxidativen Phosphorylierung, ATP-Disruptoren, wie beispielsweise Diafenthiuron; oder

Organozinnverbindungen, z.B. Azocyclotin, Cyhexatin und Fenbutatin-oxid; oder

Propargite; oder Tetradifon.

(13) Entkoppler der oxidativen Phoshorylierung durch Unterbrechung des H-Protongradienten, wie beispielsweise Chlorfenapyr, DNOC und Sulfluramid.

(14) Nikotinerge Acetylcholin-Rezeptor-Antagonisten, wie beispielsweise Bensultap, Cartap-hydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsstörende Wirkstoffe, Dipteran, wie beispielsweise Cyromazine.

(18) Ecdyson-Rezeptor Agonisten, wie beispielsweise Chromafenozide, Halofenozide, Methoxyfenozide und Tebufenozide.

(19) Oktopaminerge Agonisten, wie beispielsweise Amitraz.

(20) Komplex-III-Elektronentransportinhibitoren, wie beispielsweise Hydramethylnon; oder Acequinocyl; oder Fluacrypyrim.

(21) Komplex-I-Elektronentransportinhibitoren, beispielsweise

METI-Akarizide, z.B. Fenazaquin, Fenpyroximate, Pyrimidifen, Pyridaben, Tebufenpyrad und Tolfenpyrad; oder

Rotenone (Derris).

(22) Spannungsabhängige Natriumkanal-Blocker, z.B. Indoxacarb; oder Metaflumizone.

(23) Inhibitoren der Acetyl-CoA-Carboxylase, wie beispielsweise

Tetron- und Tetramsäurederivate, z.B. Spirodiclofen, Spiromesifen und Spirotetramat.

(24) Komplex-IV-Elektronentransportinhibitoren, wie beispielsweise

Phosphine, z.B. Aluminiumphosphid, Calciumphosphid, Phosphin und Zinkphosphid; oder
Cyanid.
(25) Komplex-II-Elektronentransportinhibitoren, wie beispielsweise Cyenopyrafen.
(28) Ryanodinrezeptor-Effektoren, wie beispielsweise
Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)anüno}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-$\lambda^4$-sulfanyliden}cyanamid (bekannt aus WO2007/149134) und seine Diastereomere {[(1R)-1-(6-Chlorpyridin-3-yl)ethyl](methyl)oxido-$\lambda^4$-sulfanyliden}cyanamid (A) und {[(1S)-1-(6-Chlorpyridin-3-yl)ethyl](methyl)oxido-$\lambda^4$-sulfanyliden}cyanamid (B) (ebenfalls bekannt aus WO2007/149134) sowie Sulfoxaflor und seine Diastereomere [(R)-Methyl(oxido){(1R)-1-[6-(trifluormethyl)pyridin-3-yl]ethyl}-$\lambda^4$-sulfanyliden] cyanamid (A1) und [(S)-Methyl(oxido){(1S)-1-[6-(trifluormethyl)pyridin-3-yl]ethyl}-$\lambda^4$-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-$\lambda^4$-sulfanyliden]cyanamid (B1) und [(S)-Methyl(oxido){(1R)-1-[6-(trifluormethyl)pyridin-3-yl]ethyl}-$\lambda^4$-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3 -methylbenzoyl] -2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233).

**Fungizide**

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).

(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus WO2010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.

(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).

(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).

(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137 -30-4).

(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).

(7) Inhibitoren der Aminosäure- und Protein-Biosynthese, wie beispielsweise (7.1) Andoprim (23951-85-1), (7.2)

Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus WO2005070917).

(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).

(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).

(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).

(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus WO2005042474).

(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).

(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).

(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).

(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-l-(morpholin-4-yl)prop-2-en-l-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus WO2003035617),

(15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yllethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-l-yl]-1-{4-[4-(5-phenyl-4,5-dihydro-1,2-oxazol-3-yl)-1,3-thiazol-2-yllpiperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus WO2005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus WO2009094442), (15.75) 5-Fluor-2-[(4-methylbenzyl)oxy]pyrimidin-4-amin (1174376-25-0) (bekannt aus WO2009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4- [(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.

(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl] -5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N- [4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle,

Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp. Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.)

vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

## Beispiele

### Herstellungsbeispiele einschließlich der weiteren Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC-MS (Liquid Chromatography Mass Spectrometry) und/oder GC-MS (Gas Chromatography-Mass Spectrometry) charakterisiert.

Die Bestimmung der logP Werte erfolgte analog OECD Guideline 117 (EC Directive 92/69/EEC) durch HPLC (High Performance Liquid Chromatography) an reversed-phase Säulen (C 18), mit nachfolgenden Methoden:

[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. LogP[a] wird auch logP(HCOOH) genannt.

[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. LogP[b] wird auch logP(neutral) genannt.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker II Avance 400, ausgestattet mit einem 1,7 mm TCI-Probenkopf, gemessen. In Einzelfällen wurden die NMR Spektren mit einem Bruker Avance II 600 bestimmt.

Die NMR-Daten ausgewählter Beispiele werden in klassischer Form ($\delta$-Werte, Multiplettaufspaltung, Anzahl der H-Atome) aufgeführt. Die Aufstpaltung der Signale wurde wie folgt beschrieben: s (Singulett), d (Duplett), t (Triplett), q (Quartett), m (Multiplett), breit (für breite Signale). Als Lösungsmittel wurden $CD_3CN$, $CDCl_3$ oder D6-DMSO verwendet, wobei als Referenz Tetramethylsilan (0.00 ppm) eingesetzt wurde.

Die GC-MS-Spektren wurden mit einem Agilent 6890 GC, HP 5973 MSD an Dimethylsilikonphase bestimmt, mit einem Temperaturgradient von 50 °C bis 320 °C. GC-MS-Indices wurden als Kovats-Indices mit Lösung einer homologen Reihe von n-Alkanen (mit geradzahliger Anzahl von 8 bis 38 Kohlenstoffatomen) bestimmt.

Die Enantiomere wurden aus dem Racemat gewonnen, in dem sie präparativ mittels HPLC über einer chiralen Säule (ChiralCel OJ-H z.B. 5nm 250 x4.6 mm) mit Laufmittel Heptan / Methanol / Ethanol (95:2.5:2.5) getrennt wurden.

**Synthese von Anilinen der Formel (IVa), (IVb) und Zwischenstufen (X), (XI), (XII), (XIII), (XVI), (XVII), (XVIII), (XIX) und (XX)**

**2,2,2-Trifluor-*N*-(2-fluor-4-methylphenyl)acetamid (XIII-1)**

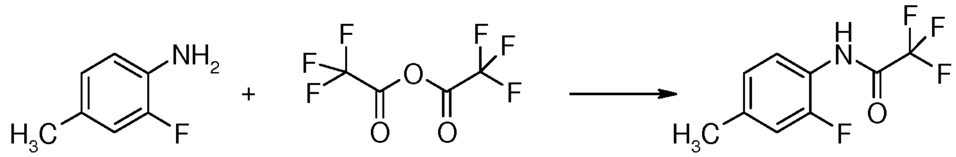

(XIV-1) (XIII-1)

27,5 g 2-Fluor-4-methylanilin werden bei 0 °C in 300 mL Dichlormethan vorgelegt, 26,7 g Triethylamin werden zugegeben und anschließend werden 50,8 g Trifluoressigsäureanhydrid zugetropft. Es wird 2 h bei 0 °C nachgerührt und anschließend einrotiert. Der Rückstand wird in Wasser aufgenommen und zweimal mit Ethylacetat extrahiert. Die vereinigten organischen Phasen werden über Natriumsulfat getrocknet, filtriert, und das Lösemittel wird im Vakuum entfernt. Es werden 49,0 g (100% d.Th.) der Titelverbindung erhalten. logP[a]: 2,40

Analog erhalten wurde:

***N*-(4-Chlor-2-fluorphenyl)-2,2,2-trifluoracetamid (XIII-2)**

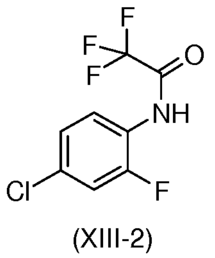

(XIII-2)

logP[a]: 2,53; logP[b]: 2,40; 1H-NMR (D6-DMSO,400MHz) δ ppm 11.29(s,1H), 7,62(dd,1H), 7,55(dd,1H), 7,37(dd,1H)

**4-Fluor-2-methyl-5-[(trifluoracetyl)amino]benzolsulfonylchlorid (XII-1)**

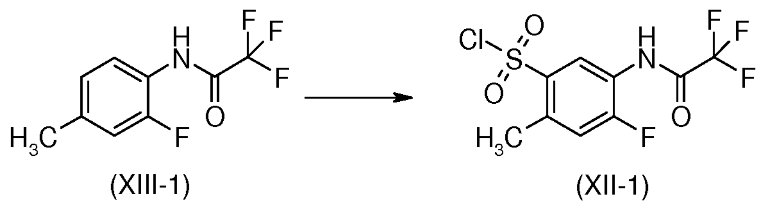

(XIII-1) (XII-1)

258 g Chlorsulfonsäure werden vorgelegt und bei Raumtemperatur werden 49 g 2,2,2-Trifluor-N-(2-fluor-4-methylphenyl)acetamid in Portionen zugesetzt. Bei Raumtemperatur wird noch 16 h nachgerührt. Die Mischung wird unter Rühren auf Eis gegeben und zweimal mit Ethylacetat extrahiert. Die vereinigten organischen Phasen werden über Natriumsulfat getrocknet, filtriert, und das Lösemittel wird im Vakuum entfernt. Es werden 70,8 g des Chlorsulfonyls (XII-1) erhalten. Das Rohprodukt wird sofort weiter umgesetzt.

***N,N'*-[Disulfanediylbis(6-fluor-4-methylbenzol-3,1-diyl)]bis(2,2,2-trifluoracetamid) (XI-1)**

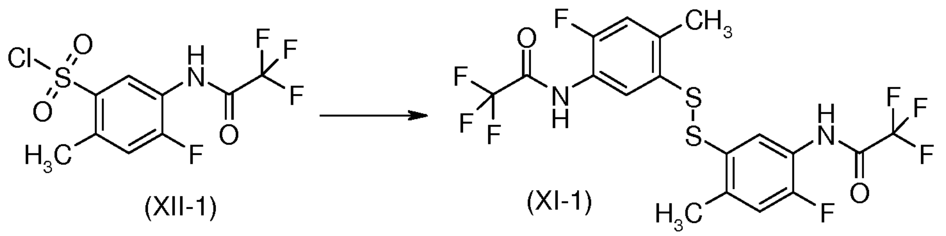

(XII-1) (XI-1)

298,8 g Natriumiodid werden in 1000 mL Trifluoressigsäure gelöst und 70,8 g 4-Fluor-2-methyl-5-[(trifluoracetyl)amino]benzolsulfonylchlorid werden bei Raumtemperatur zugegeben. Es wird 16 h bei Raumtemperatur nachgerührt und anschließend das Lösemittel im Vakuum entfernt. Der Rückstand wird mit Wasser verrührt und abgesaugt. Es werden 62,3 g (86% d.Th.) der Titelverbindung als Feststoff erhalten. logP[a]: 4,41

Analog erhalten wurde:

***N,N'*-[Disulfanediylbis(4-chlor-6-fluorbenzol-3,1-diyl)]bis(2,2,2-trifluoracetamid) (XI-2)**

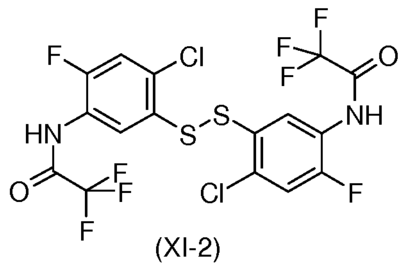

(XI-2)

logP[a]: 4,60; logP[b]: 3,82; 1H-NMR (D6-DMSO,400MHz) δ ppm 11,44(s,2H), 7,95(d,2H), 7,83(d,2H)

**2,2,2-Trifluor-*N*-{2-fluor-4-methyl-5-[(2,2,2-trifluorethyl)sulfanyl]phenyl}acetamid (X-1)**

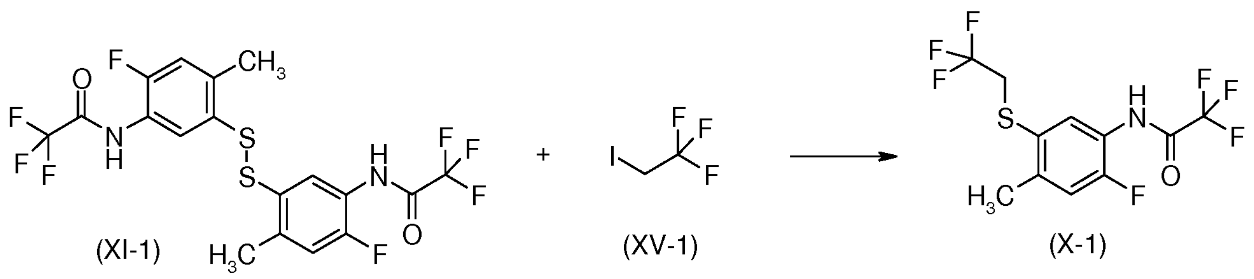

(XI-1) + (XV-1) (X-1)

3,4 g N,N'-[Disulfanediylbis(6-fluor-4-methylbenzol-3,1-diyl)]bis(2,2,2-trifluoracetamid) werden in 150 ml N,N-Dimethylformamid gelöst und mit 1,86 g Kaliumcarbonat, 3,11g 1,1,1-Trifluoriodethan, 2,39 g Rongalit und einigen Tropfen Wasser versetzt. Die Reaktionsmischung wird 16 h bei Raumtemperatur gerührt. Die Hauptmenge des *N,N*-Dimethylformamids wird im Vakuum abdestilliert. Der Rückstand wird in Wasser aufgenommen und zweimal mit Ethylacetat extrahiert. Die vereinigten organischen Phasen werden über Natriumsulfat getrocknet, filtriert, und anschließend wird das Lösemittel im Vakuum entfernt. Es werden 4,48 g (90% d.Th.) der Titelverbindung erhalten. logP[a]: 3,31

Analog erhalten wurde:

***N*-{4-Chlor-2-fluor-5-[(2,2,2-trifluorethyl)sulfanyl]phenyl}-2,2,2-trifluoracetamid (X-2)**

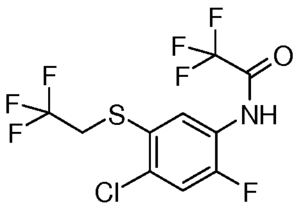

(X-2)

logP[a]: 3,34; logP[b]: 3,14; 1H-NMR (D6-DMSO,400MHz) δ ppm 11,47(bs,1H), 7,85(d,1H), 7,76(d,1H), 4,09(q,2H)

**S-(5-Acetamido-4-fluor-2-methylphenyl)ethanthioat (XVII-1)**

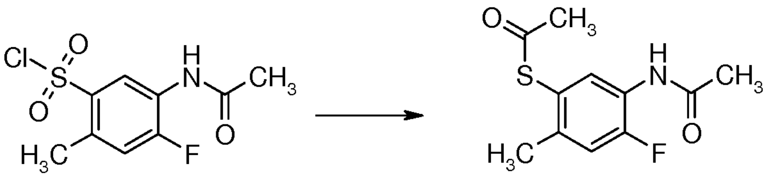

(XII-1) (XVII-1)

99,3 g 5-Acetamido-4-fluor-2-methylbenzolsulfonylchlorid werden in 700 mL Eisessig suspendiert, mit 0,9 g Iod und 38,7 g rotem Phosphor versetzt und 5 h bei Rückfluß nachgerührt. Nach Abkühlen wird der Feststoff abfiltriert und das Filtrat einrotiert. Der Rückstand wird mit Wasser verrührt und abgesaugt. 57,6 g (67% d.Th.) der Titelverbindung werden als Feststoff erhalten. logP[a]: 1,78

**5-Amino-4-fluor-2-methylbenzolthiol (XVI-1)**

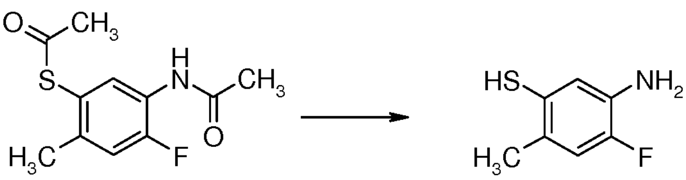

(XVII-1) (XVI-1)

57,4 g S-(5-Acetamido-4-fluor-2-methylphenyl)ethanthioat werden in 750 mL Wasser und 96,6 g Kaluimhydroxid gelöst. Die Reaktionsmischung wird 16 h bei Rückfluß gekocht. Nach Abkühlen wird die Lösung mit Salzsäure auf pH 2-3 gestellt und der ausgefallene Feststoff abgesaugt. 35,8 g (94% d.Th.) der Titelverbindung werden als Feststoff erhalten. logP[a]: 3,70

**2-Fluor-4-methyl-5-[(2,2,2-trifluorethyl)sulfinyl]anilin (IVb-1)**

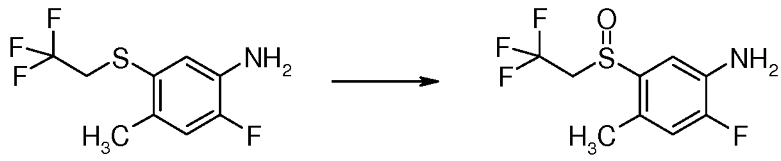

(IVa-1) (IVb-1)

5,00 g (0,21 mmol) 2-Fluor-4-methyl-5-[(2,2,2-trifluorethyl)sulfanyl]anilin werden bei 0-4 °C in 100 ml Dichlormethan vorgelegt, 6,18 g (0,25 mmol) *meta*-Chlorperbenzoesäure werden dazu gegeben und das Reaktionsgemisch wird 2 h bei Raumtemperatur nachgerührt. Anschließend mit einer 33%igen Natriumthiosulfatlösung versetzt (Peroxid-Test durchgeführt) und zweimal mit Dichlormethan ausgeschüttelt. Die vereinigten organischen Phasen werden mit einer gesättigten Natriumcarbonatlösung gewaschen, über Natriumsulfat getrocknet, filtriert, und das Lösemittel wird im Vakuum entfernt. Der Rückstand enthält 5,10 g (90% Reinheit, 86% d.Th.) der Titelverbindung als brauner Öl.
logP[a]: 1,77; logP[b]: 1,72; 1H-NMR (D6-DMSO, 400MHz) δ ppm 7,26(d,1H), 7,02(d,1H), 5,45(bs,2H), 4,08-3,95(m,1H), 3,88-3,75(m,1H), 2,19(s,3H)

**1,1'-Disulfanediylbis(2-chlor-5-nitrobenzol) (XIX-3)**

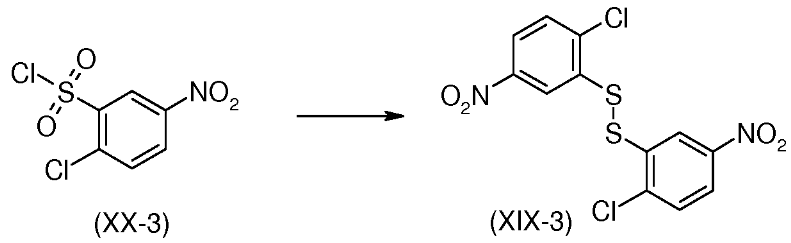

(XX-3) (XIX-3)

52,0 g (203,1 mmol) 2-Chlor-5-nitrobenzolsulfonylchlorid werden unter starkem Rühren mit 236,1 g (1,02 mol) Chlorsulfonsäure versetzt und über Nacht bei Raumtemperatur gerührt. Nach Zugabe von 40%iger wässriger Natriumhydrogensulfit-Lösung wird der gebildete Feststoff abgesaugt, mit Wasser gewaschen und über Nacht auf einer Tonplatte getrocknet. Es werden 36,1 g (100% Reinheit, 94% d.Th.) der Titelverbindung als graubrauner Feststoff erhalten.
logP[a]: 5,03; logP[b]: 5,01; $^{1}$H-NMR (D6-DMSO, 400MHz) δ ppm 8,40(d,2H), 8,18-8,16(m,2H), 7,91(d,2H); GC-MS: EI-Masse (m/z): 376 (2Cl) $[M]^+$

**1,1'-Disulfanediylbis(2,4-dichlor-5-nitrobenzol) (XIX-6)**

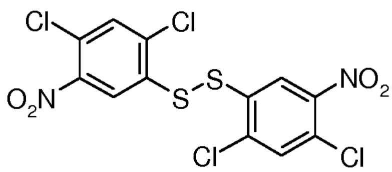

(XIX-6)

logP[a]: 5,69; logP[b]: 5,64; $^{1}$H-NMR (D6-DMSO,400MHz) δ ppm 8,33(s,2H), 8,21(s,2H)

**3,3'-Disulfanediylbis(4-chloranilin) (XVIII-3)**

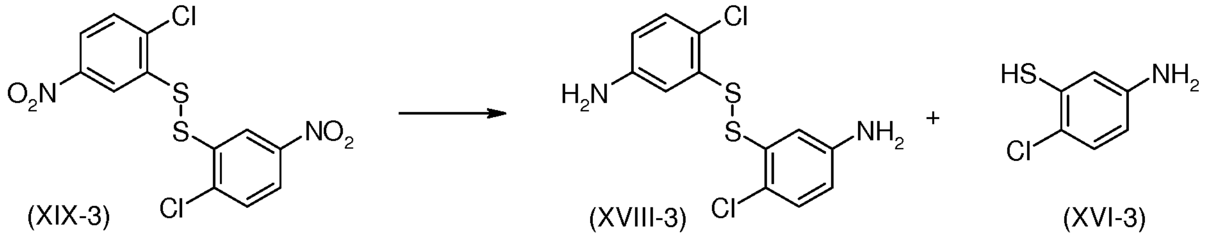

(XIX-3) (XVIII-3) (XVI-3)

8,00 g (21,2 mmol) 1,1'-Disulfanediylbis(2-chlor-5-nitrobenzol) werden in 150 mL THF gelöst, mit 1,6 g Raney-Nickel versetzt und 72 h bei 50 °C unter Wasserstoffatmosphäre (20 bar) gerührt. Die Reaktionsmischung wird mit THF über Kieselgur filtriert, das Filtrat wird unter vermindertem Druck vom Lösungsmittel berfreit. Es werden 6,64 g (90% Reinheit, 89% der Theorie) eines Gemisches aus 1,1'-Disulfanediylbis(2-chlor-5-nitrobenzol) und 5-Amino-2-chlorbenzolthiol erhalten, das ohne weitere Aufreinigung alkyliert wird.

1,1'-Disulfanediylbis(2-chlor-5-nitrobenzol) (XIX-3):

logP[a]: 3,31; logP[b]: 3,35; $^1$H-NMR (D6-DMSO, 400MHz) $\delta$ ppm 7,10(d,2H), 6,73(d,2H), 6,47-6,44(m,2H), 5,51(breit,4H); GC-MS: EI-Masse (m/z): 316 (2Cl) $[M]^+$

5-Amino-2-chlorbenzolthiol (XVI-3):

logP[a]: 1,64; logP[b]: nicht messbar; $^1$H-NMR (D6-DMSO,400MHz) $\delta$ ppm 7,01(d,1H), 6,54(d,1H), 6,35-6,32(m,1H), 5,28(breit,3H); GC-MS: EI-Masse (m/z): 159 (1Cl) $[M]^+$

Analog erhalten wurden:

**3,3'-Disulfanediylbis(4,6-dichloranilin) (XVIII-6)**

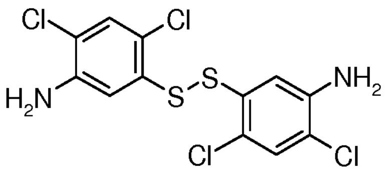

(XVIII-6)

logP[a]: 5,14; logP[b]: 4,95; $^1$H-NMR (D6-DMSO, 400MHz) $\delta$ ppm 7,41(s,2H), 6,95(s,2H), 5,78(breit,4H); GC-MS: EI-Masse (m/z): 386 (4Cl) $[M]^+$

**4-Chlor-3-[(2,2,2-trifluorethyl)sulfanyl]anilin (IVa-3)**

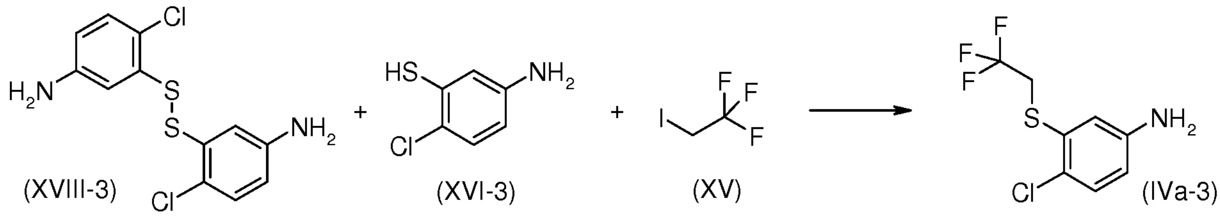

(XVIII-3) (XVI-3) (XV) (IVa-3)

6,40 g eines Gemisches aus Disulfanediylbis(2-chlor-5-nitrobenzol) und 5-Amino-2-chlorbenzolthiol (ca. 20 mmol) werden in 100 mL N,N-Dimethylformamid vorgelegt und mit 7,02 g (40,3 mmol) Natriumdithionit, 5,58 g (40,3 mmol) Kaliumcarbonat und 5,49 g (40,3 mmol) Rongalit versetzt und auf 0 °C abgekühlt. 9,32 g 1,1,1-Trifluor-2-iodethan werden bei 0 °C zugetropft. Die Reaktionsmischung wird über Nacht bei Raumtemperatur gerührt. Ein Großteil des Lösungsmittels wird unter vermindertem Druck entfernt, der Rückstand wird mit Wasser versetzt und mit Essigester extrahiert. Die vereinigten organischen Phasen werden sukzessive mit Wasser, gesättigter wässriger Natriumchlorid-Lösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 4,70 g (98% Reinheit, 47% der Theorie) der Titelverbindung als gelbe Flüssigkeit.
logP[a]: 2,64; logP[b]: 2,69; $^1$H-NMR (D6-DMSO, 400MHz) $\delta$ ppm 7,09(d,1H), 6,78(d,1H), 6,49-6,46(m,1H), 5,37(breit,2H), 3,90(q,2H) ; GC-MS: EI-Masse (m/z): 241 (1Cl) $[M]^+$

Analog erhalten wurden:

**4-Chlor-2-fluor-5-[(2,2,2-trifluorethyl)sulfanyl]anilin (IVa-2)**

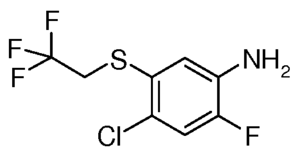

(IVa-2)

11,0 g (30,9 mmol) *N*-{4-Chlor-2-fluor-5-[(2,2,2-trifluorethyl)sulfanyl]phenyl}-2,2,2-trifluoracetamid in 150 ml Dioxan werden vorsichtig zu einer Lösung aus 10,3 ml (186 mmol) Schwefelsäure (96%ig) in 100 ml Wasser zu gegeben. Anschließend wird das Reaktionsgemisch über Nacht refluxiert. Nach Abkühlen wird die Lösung mit einer gesättigten Natriumhydrogencarbonatlösung und etwas Natriumcarbonat auf pH 7 gebracht und dreimal mit Essigester extrahiert. Die vereinten organischen Phasen werden mit einer gesättigten Natriumchloridlösung gewaschen, über Natriumsulfat getrocknet, filtriert und eingeengt. Der Rückstand enthält 8,27 g (96% Reinheit, 99% d.Th.) der Titelverbindung als schwarzes Öl/Feststoff-Gemisch.
logP[a]: 3,02; logP[b]: 3,00; 1H-NMR (D6-DMSO,400MHz) $\delta$ ppm 7,27(d,1H), 7,04(d,1H), 5,46(bs,2H), 3,85(q,2H)

**Synthese von Bromiden der Formel (VIIa)**

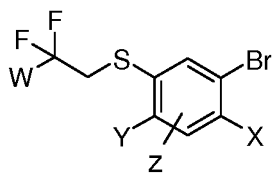

(VIIa)

**5-Brom-2-methylphenyl-2,2,2-trifluorethylsulfid (VIIa-7)**

**Stufe 1:** *5-Brom-2-methylbenzolsulfonylchlorid*

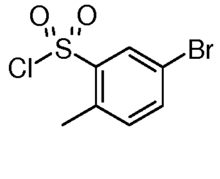

69,50 g (406,3 mmol) 4-Bromtoluol werden in 250 ml Dichlormethan vorgelegt und bei -5 - 5 °C tropfenweise mit 175,33 g (1,50 mol) Chlorsulfonsäure versetzt. Die Reaktionsmischung wird über Nacht unter Rühren auf Raumtemperatur gebracht, mit 1000 mL Eiswasser versetzt und mit Dichlormethan extrahiert. Die vereinigten organischen Phasen werden mit gesättigter wässriger Kochsalzlösung gewaschen, über Magnesiumsulfat getrocknet, filtriert und unter vermindertem Druck vom Lösungsmittel befreit. Man erhält 89,54 g (95% Reinheit, 78% der Theorie) der Titelverbindung als gelbe Flüssigkeit, die ohne weitere Aufreinigung weiter umgesetzt wird.
logP[a]: 3,73; logP[b]: 3,74; GC-MS: EI-Masse (m/z): 270 (1Cl,1Br) $[M]^+$; $^1$H-NMR ($CDCl_3$, 400 MHz): 8,19(d,1H), 7,73-7,72(m,1H), 7,31(d,1H), 2,73(s,3H); $^1$H-NMR ($CD_3CN$, 400 MHz): 8,18(d,1H), 7,88-7,85(m,1H), 7,46(d,1H), 2,71(s,3H)

**Stufe 2:** *1,1'-Disulfanediylbis(5-brom-2-methylbenzol)*

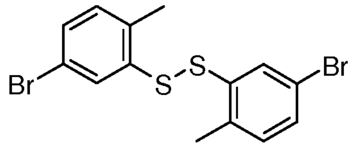

25,00 g (92,7 mmol) 5-Brom-2-methylbenzolsulfonylchlorid werden mit 145,7 g wässriger Iodwasserstoffsäure (57%ig, 649,2 mmol) unter kräftigem Rühren versetzt. Die Reaktionsmischung wird 3 d bei Raumtemperatur gerührt, dann mit 40%iger wässriger Natriumhydrogensulfitlösung versetzt. Der Feststoff wird abgesaugt, gründlich mit Wasser gewaschen und über Nacht auf einer Tonplatte getrocknet. Man erhält 19,50 g (95% Reinheit, 99% der Theorie) der Titelverbindung als gelben Feststoff, der ohne weitere Aufreinigung weiter umgesetzt wird.
logP[a]: >7,36; logP[b]: >7,36; GC-MS: EI-Masse (m/z): 404 (2Br) $[M]^+$;
$^1$H-NMR (D6-DMSO): 7,56(d,2H), 7,46-7,43(m,2H), 7,26(d,2H), 2,35(s,6H)

**Stufe 3:** *5-Brom-2-methylphenyl-2,2,2-trifluorethylsulfid* (VIIa-7)

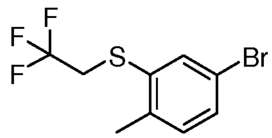

(VIIa-7)

27,70 g (68,5 mmol) 1,1'-Disulfanediylbis(5-brom-2-methylbenzol) werden in 350 mL *N*,*N*-Dimethylformamid vorgelegt, mit 23,86 g (137,1 mmol) Natriumdithionit, 18,66 g (137,1 mmol) Rongalit® sowie 18,94 g (137,1 mmol) Kaliumcarbonat versetzt und auf 0 °C gekühlt. 31,65 g (150,8 mmol) 1,1,1-Trifluor-2-iodethan in 20 mL *N*,*N*-Dimethylformamid werden bei 0 °C tropfenweise zugegeben. Die Reaktionsmischung wird über Nacht unter Rühren auf Raumtemperatur gebracht, mit 500 mL Wasser versetzt und mit *tert*-Butyl-methylether extrahiert. Die vereinigten organischen Phasen werden mit Wasser, gesättigter wässriger Natriumbicarbonatlösung und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 30,01 g (93% Reinheit, 71% der Theorie) der Titelverbindung als farblose Flüssigkeit.
logP[a]: 4,29; logP[b]: 4,26; GC-MS: EI-Masse (m/z): 286 (1Br) $[M]^+$;
$^1$H-NMR (D6-DMSO, 400MHz) $\delta$ ppm: 7,70(d,1H), 7,39-7,36(m,1H), 7,21(d,1H), 4,09(q,2H), 2,30(s,3H)

Analog erhalten wurden:

**4-Brom-1-methoxy-2-[(2,2,2-trifluorethyl)sulfanyl]benzol (VIIa-8)**

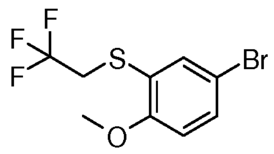

(VIIa-8)

logP[a]: 3,69; logP[b]: 3,81; GC-MS: EI-Masse (m/z): 302 (1Br) $[M]^+$;
$^1$H-NMR (D6-DMSO, 400MHz) δ ppm: 7,59(d,1H), 7,45-7,42(m,1H), 7,00(d,1H), 4,02(q,2H), 3,85(s,3H)

**4-Brom-1-chlor-2-[(2,2,2-trifluorethyl)sulfanyl]benzol (VIIa-3)**

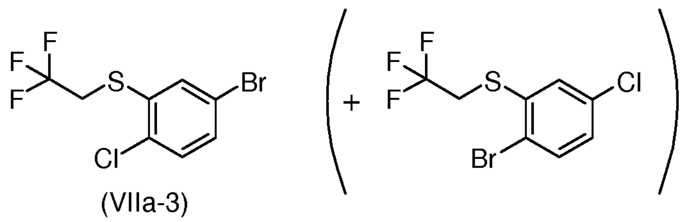

(VIIa-3)

Erhalten als Mischung aus 60% 4-Brom-1-chlor-2-[(2,2,2-trifluorethyl)sulfanyl]benzol und 40% 1-Brom-4-chlor-2-[(2,2,2-trifluorethyl)sulfanyl]benzol.
logP[a]: 4,20; logP[b]: 4,21; GC-MS: EI-Masse (m/z): 306 (1Br,1Cl) $[M]^+$;
$^1$H-NMR (D6-DMSO, 400MHz) δ ppm: 7,85(breit,1H), 7,70(d,1'H), 7,67(d,1'H), 7,50-7,44(m,2H), 7,27-7,24(m,1'H), 4,30-4,22(m,2H+2'H)

**1-Brom-2-fluor-4-methyl-5-[(2,2,2-trifluorethyl)sulfanyl]benzol (VIIa-1)**

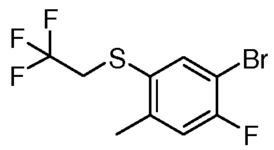

(VIIa-1)

logP[a]: 4,30; logP[b]: 4,34; GC-MS: EI-Masse (m/z): 304 (1Br) $[M]^+$;
$^1$H-NMR (D6-DMSO, 400MHz): 7,87(d,1H), 7,36(d,1H), 4,02(q,2H), 2,35(s,3H)

**1-Brom-2,4-dichlor-5-[(2,2,2-trifluorethyl)sulfanyl]benzol (VIIa-6)**

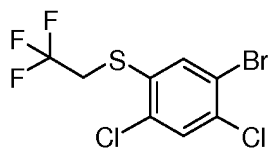

(VIIa-6)

logP[a]: 4,80; logP[b]: 4,77; GC-MS: EI-Masse (m/z): 340 (1Br,2Cl) $[M]^+$;

$^1$H-NMR (D6-DMSO, 400MHz): 8,04(s,1H), 7,91(s,1H), 4,28(q,2H)

**1-Brom-2,4-dimethyl-5-[(2,2,2-trifluorethyl)sulfanyl]benzol (VIIa-11)**

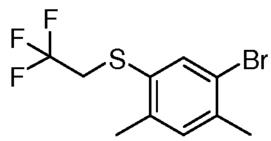

(VIIa-11)

logP[a]: 4,81; logP[b]: 4,77; GC-MS: EI-Masse (m/z): 340 (1Br,2Cl) $[M]^+$; $^1$H-NMR (D6-DMSO, 400MHz): 8,04(s,1H), 7,91(s,1H), 4,28(q,2H)

**1-Brom-4-chlor-2-fluor-5-[(2,2,2-trifluorethyl)sulfanyl]benzol (VIIa-2)**

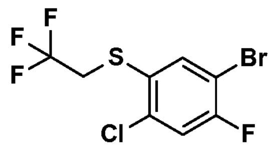

(VIIa-2)

logP[a]: 4,28; logP[b]: 4,29; GC-MS: EI-Masse (m/z): 324 (1Cl,1Br) $[M]^+$; $^1$H-NMR (D6-DMSO, 400MHz): 8,04(d,1H), 7,77(d,1H), 4,20(q,2H)

**1,4-Dibrom-2-[(2,2,2-trifluorethyl)sulfanyl]benzol (VIIa-13)**

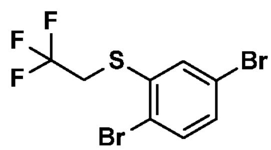

(VIIa-13)

logP[a]: 4,29; logP[b]: 4,25; GC-MS: EI-Masse (m/z): 350 (2Br) $[M]^+$; $^1$H-NMR (D6-DMSO, 400MHz): 7,81(d,1H), 7,60(d,1H), 7,39-7,37(m,1H), 4,26(q,2H)

**1,4-Dibrom-2-fluor-5-[(2,2,2-trifluorethyl)sulfanyl]benzol (VIIa-14)**

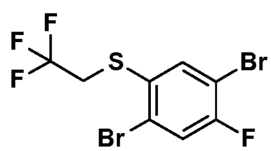

(VIIa-14)

logP[a]: 4,39; logP[b]: 4,36; GC-MS: EI-Masse (m/z): 368 (2Br) $[M]^+$; $^1H$-NMR (D6-DMSO, 400MHz): 8,00(d,1H), 7,87(d,1H), 4,21(q,2H)

**1-Brom-2,4-difluor-5-[(2,2,2-trifluorethyl)sulfanyl]benzol (VIIa-15)**

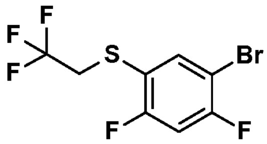

(VIIa-15)

logP[a]: 3,89; logP[b]: 3,86; GC-MS: EI-Masse (m/z): 308 $[M]^+$; $^1H$-NMR (D6-DMSO, 400MHz): 8,07(t,1H), 7,63(t,1H), 4,03(q,2H)

**4-Brom-2-[(2,2,2-trifluorethyl)sulfanyl]benzonitril (VIIa-9)**

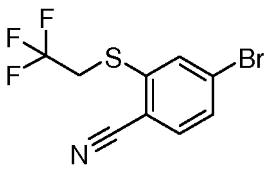

(VIIa-9)

24,0 g (86,4 mmol) Natriumhydrid (60%ig in Mineralöl) werden in 300 ml *N*,*N*-Dimethylformamid vorgelegt und bei 0 °C tropfenweise mit 10,0 g (86,4 mmol) 2,2,2-Trifluorethanthiol versetzt. Die Reaktionsmischung wird bei 0 °C tropfenweise zu einer Lösung aus 14,4 g (72,0 mmol) 4-Brom-2-fluorbenzonitril in 100 mL *N*,*N*-Dimethylformamid gegeben und über Nacht unter Rühren auf Raumtemperatur gebracht. Die Reaktionsmischung wird in Wasser gegossen, mit gesättigter wässriger Ammoniumchloridlösung neutralisiert und mit tert-Butyl-methylether extrahiert. Die vereinigten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Der Rückstand wird mit Petrolether verrührt, abfiltriert und aus Diethylether umkristallisiert. Man erhält 17,6 g (99% Reinheit, 82% der Theorie) der Titelverbindung als farblosen Feststoff.
logP[a]: 3,21; logP[b]: 3,16; $^1H$-NMR (D6-DMSO, 400MHz) $\delta$ ppm: 8,12(d,1H), 7,83(d,1H), 7,72-7,69(m,1H), 4,33(q,2H)

Analog erhalten wurde:

**4-Brom-2-[(2,2,2-trifluorethyl)sulfanyl]-1-(trifluormethyl)benzol (VIIa-10)**

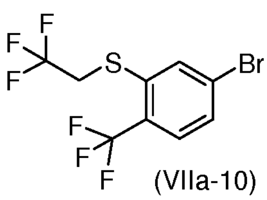

(VIIa-10)

logP[a]: 4,22; logP[b]: 4,20; $^1H$-NMR (D6-DMSO, 400MHz): 8,18(s,1H), 7,73-7,68(m,2H), 4,30(q,2H)

**Synthese von Iodiden der Formel (VIIa)**

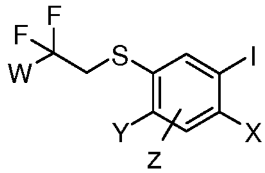

(VIIa)

**1-Fluor-2-iod-5-methyl-4-[(2,2,2-trifluorethyl)sulfanyl]benzol (VIIa-1-I)**

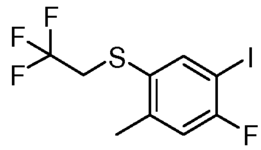

(VIIa-1-I)

10,0 g (33,0 mmol) 5-Brom-2-methylphenyl-2,2,2-trifluorethylsulfid, 9,89 g (66,0 mmol) Natriumiodid, 314 mg (1,65 mmol) Kupfer(I)-iodid und 469 mg (3,3 mmol) *trans-N,N'*-Dimethylcyclohexan-1,2-diamin (racemisch) wurden in 33 mL entgastem Dioxan über Nacht bei 110 °C gerührt. Erneut wurden 9,89 g (66,0 mmol) Natriumiodid, 314 mg (1,65 mmol) Kupfer(I)-iodid und 234 mg (1,65 mmol) *trans-N,N'*-Dimethylcyclohexan-1,2-diamin (racemisch) zugesetzt und über Nacht bei 110 °C gerührt. Erneut wurden 9,89 g (66,0 mmol) Natriumiodid und 314 mg (1,65 mmol) Kupfer(I)-iodid sowie 20 mL Dioxan zugesetzt und über Nacht bei 110 °C gerührt. Nach Abkühlen auf Raumtemperatur wurde mit Essigester verdünnt, über Kieselgur filtriert und konzentriert. Nach säulenchromatographischer Reinigung an Kieselgel mittels MPLC mit Cyclohexan/Essigester als Laufmittel erhält man 8,77 g (97% Reinheit, 74% der Theorie) der Titelverbindung als farbloses Öl.
logP[a]: 4,44; logP[b]: 4,44; GC-MS: EI-Masse (m/z): 350 $[M]^+$
$^1$H-NMR (D6-DMSO, 400MHz): 7,97(d,1H), 7,24(d,1H), 3,96(q,2H), 2,35(s,3H)

Analog erhalten wurde:

**4-Iod-1-methyl-2-[(2,2,2-trifluorethyl)sulfanyl]benzol (VIIa-7-I)**

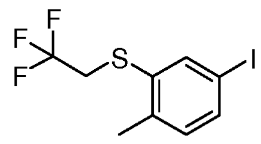

(VIIa-7-I)

logP[a]: 4,49; logP[b]: 4,48; GC-MS: EI-Masse (m/z): 332 $[M]^+$
$^1$H-NMR (D6-DMSO, 400 MHz): 7,83(d,1H), 7,55-7,53(m,1H), 7,05(d,1H), 4,05(q,2H), 2,30(s,3H)

**Synthese von Boronsäuren der Formel (VIII)**

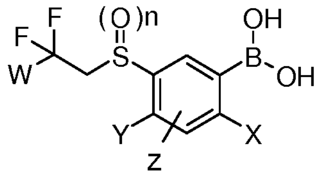

(VIII)

**{4-Methyl-3-[(2,2,2-trifluorethyl)sulfanyl]phenyl}boronsäure (VIIIa-7)**

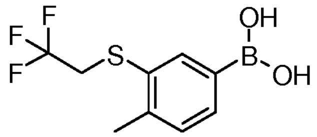

(VIIIa-7)

**Stufe 1:** *4,4,5,5-Tetramethyl-2-{4-methyl-3-[(2,2,2-trifluorethyl)sulfanyl]phenyl}-1,3,2-dioxaborolan*

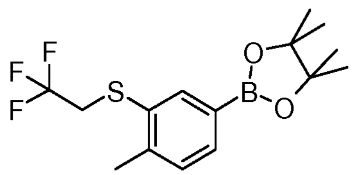

15,0 g (52,6 mmol) 5-Brom-2-methylphenyl-2,2,2-trifluorethylsulfid, 14,7 g (57,9 mmol) 4,4,4',4',5,5,5',5'-Octamethyl-2,2'-bi-1,2,3-dioxaborolan, 10,3 g (105,2 mmol) Kaliumacetat und 2,15 g (2,63 mmol) 1,1'-Bis(diphenylphosphino)ferrocen-palladium(II)dichlorid-Methylenchlorid-Addukt werden in 78 ml entgastem trockenen Dioxan vorgelegt und 40 min unter Mikrowellenbestrahlung (Anton Paar Multiwave) bei 160 °C gerührt. Die Reaktionsmischung wird mit Essigester über Kieselgel filtriert und unter vermindertem Druck vom Lösungsmittel befreit. Nach säulenchromatographischer Reinigung an Kieselgel mittels MPLC mit Cyclohexan/Essigester als Laufmittel erhält man 14,07 g (90% Reinheit, 72% der Theorie) der Titelverbindung als grünes Öl.
logP[a]: 3,73; logP[b]: 3,74; ESI-Masse (m/z): 333 $[M+1]^+$; GC-MS: EI-Masse (m/z): 332 $[M]^+$
$^1$H-NMR(D6-DMSO, 400MHz) $\delta$ ppm: 7,77(s,1H), 7,52(d,1H), 7,30(d,1H), 3,87(q,2H), 2,42(s,3H), 1,29(s,12H)

**Stufe 2:** *{4-Methyl-3-[(2,2,2-trifluorethyl)sulfanyl]phenyl}boronsäure (VIIIa-7) und {4-Methyl-3-[(2,2,2-trifluorethyl)sulfinyl]phenyl}boronsäure (VIIIb-7)*

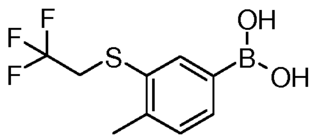

(VIIIa-7)

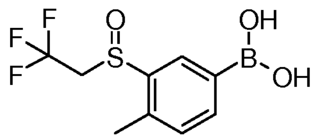

(VIIIb-7)

730 mg (2,2 mmol) 4,4,5,5-Tetramethyl-2-{4-methyl-3-[(2,2,2-trifluorethyl)sulfanyl]phenyl}-1,3,2-dioxaborolan werden in 20 ml Aceton und 20 mL Wasser vorgelegt und bei 0 °C mit 381 mg (4,9 mmol) Ammoniumacetat sowie 1,06 g (4,9 mmol) Natriumperiodat versetzt. Die Reaktionsmischung wird über Nacht bei Raumtemperatur gerührt und anschließend unter vermindertem Druck von Aceton befreit. Die saure wässrige Phase wird mit Essigester extrahiert, die vereinigten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 105 mg (96% Reinheit, 18% der Theorie) {4-Methyl-3-[(2,2,2-trifluorethyl)sulfanyl]phenyl}boronsäure sowie 138 mg (97% Reinheit, 23% der Theorie) {4-Methyl-3-[(2,2,2-trifluorethyl)sulfinyl]phenyl}boronsäure als farblose Feststoffe.

{4-Methyl-3-[(2,2,2-trifluorethyl)sulfanyl]phenyl}boronsäure

logP[a]: 2,30; logP[b]: 2,24; ESI-Masse (m/z): pos.[a]: 251 $[M+1]^+$, neg.[b]: 249 $[M-1]^-$
$^1$H-NMR (D6-DMSO, 400 MHz): 8,08(s,2H), 7,92(s,1H), 7,61-7,59(m,1H), 7,23(d,1H), 3,90(q,2H), 2,38(s,3H)

{4-Methyl-3-[(2,2,2-trifluorethyl)sulfinyl]phenyl}boronsäure

logP[a]: 1,41; logP[b]: 1,36; ESI-Masse (m/z): pos.[a]: 267 $[M+1]^+$;
$^1$H-NMR (D6-DMSO, 400 MHz): 8,31(s,1H), 8,24(s,2H), 7,89-7,87(m,1H), 7,31(d,1H), 4,12-4,02(m,1H), 3,94-3,82(m,1H), 2,39(s,3H)

Analog erhalten wurden:

**2-{2-Fluor-4-methyl-5-[(2,2,2-trifluorethyl)sulfanyl]phenyl}-4,4,5,5-tetramethyl-1,3,2-dioxaborolan**

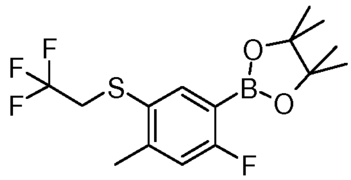

logP[a]: 4,91; logP[b]: 4,81; ESI-Masse (m/z): pos.[a]: 351 $[M+1]^+$; GC-MS: EI-Masse (m/z): 350 $[M]^+$; $^1$H-NMR (D6-DMSO, 400MHz): 7,77(d,1H), 7,17(d,1H), 3,79(q,2H), 2,46(s,3H), 1,30(s,12H)

**{2-Fluor-4-methyl-5-[(2,2,2-trifluorethyl)sulfanyl]phenyl}boronsäure (VIIIa-1)**

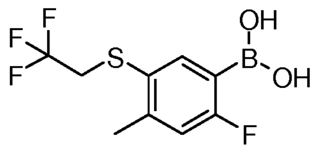

(VIIIa-1)

logP[a]: 2,34; logP[b]: 2,41; ESI-Masse (m/z): pos.[a]: 269 $[M+1]^+$, neg.[a]: 313 $[M+HCOO^-]^-$, neg.[b]: 267 $[M-1]^-$; $^1$H-NMR (D6-DMSO, 400MHz): 8,21(s,2H), 7,74(d,1H), 7,06(d,1H), 3,82(q,2H), 2,40(s,3H)

**2-{2,4-Dimethyl-5-[(2,2,2-trifluorethyl)sulfanyl]phenyl}-4,4,5,5-tetramethyl-1,3,2-dioxaborolan**

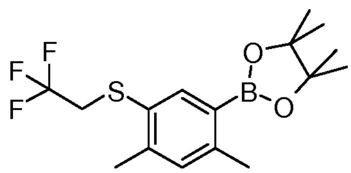

logP[a]: 5,75; logP[b]: 5,64; ESI-Masse (m/z): pos.[a]: 347 $[M+1]^+$; GC-MS: EI-Masse (m/z): 346 $[M]^+$; 1H-NMR (D6-DMSO, 400MHz): 7,75(s,1H), 7,13(s,1H), 3,74(q,2H), 2,41(s,3H), 2,40(s,3H), 1,30(s,12H)

**{2,4-Dimethyl-5-[(2,2,2-trifluorethyl)sulfanyl]phenyl}boronsäure (VIIIa-11)**

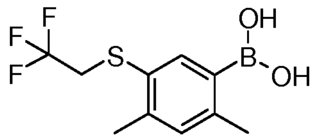

(VIIIa-11)

logP[a]: 2,58; logP[b]: 2,57; ESI-Masse (m/z): neg.[a]: 309 $[M+HCOO^-]^-$, $^1$H-NMR (D6-DMSO, 400MHz): 8,02(breit,2H), 7,61(s,1H), 7,03(s,1H), 3,81(q,2H), 2,36(s,3H),2,34(s,3H)

**2-{2,4-Dichlor-5-[(2,2,2-trifluorethyl)sulfanyl]phenyl}-4,4,5,5-tetramethyl-1,3,2-dioxaborolan**

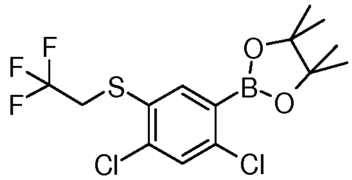

logP[a]: 5,68; logP[b]: 5,31; ESI-Masse (m/z): pos.[a]: 387 $[M+1]^+$; GC-MS: EI-Masse (m/z): 386$[M]^+$; $^1$H-NMR (D6-DMSO, 400MHz): 7,79(s,1H), 7,72(s,1H), 4,08(q,2H), 1,32(s,12H)

**{2,4-Dichlor-5-[(2,2,2-trifluorethyl)sulfanyl]phenyl}boronsäure (VIIIa-6)**

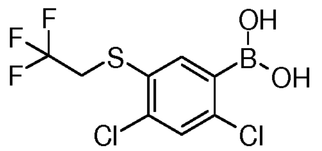

(VIIIa-6)

logP[a]: 2,61; logP[b]: 2,52; ESI-Masse (m/z): neg.[a]: 348 $[M+HCOO^-]^-$, $^1$H-NMR (D6-DMSO, 400MHz): 8,50(breit,2H), 7,68(s,1H), 7,60(s,1H), 4,11(q,2H)

**2-{4-Chlor-3-[(2,2,2-trifluorethyl)sulfanyl]phenyl}-4,4,5,5-tetramethyl-1,3,2-dioxaborolan**

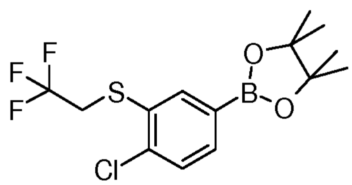

logP[a]: 5,27; logP[b]: 5,16; ESI-Masse (m/z): pos.[a]: 353 $[M+1]^+$; GC-MS: EI-Masse (m/z): 352$[M]^+$; $^1$H-NMR (D6-

DMSO, 400MHz): 7,87(d,1H), 7,62-7,60(m,1H), 7,50(d,1H), 3,71(q,2H), 1,33(s,12H)

**{4-Chlor-3-[(2,2,2-trifluorethyl)sulfanyl]phenyl}boronsäure (VIIIa-3)**

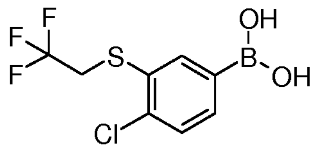

(VIIIa-3)

logP[a]: 2,40; logP[b]: 2,41; ESI-Masse (m/z): neg.[a]: 315 $[M+HCOO^-]^-$, $^1$H-NMR (D6-DMSO, 400MHz): 8,30(breit,2H), 8,00(d,1H), 7,67-7,64(m,1H), 7,49(d,1H), 4,07(q,2H)

**2-{4-Methoxy-3-[(2,2,2-trifluorethyl)sulfanyl]phenyl}-4,4,5,5-tetramethyl-1,3,2-dioxaborolan**

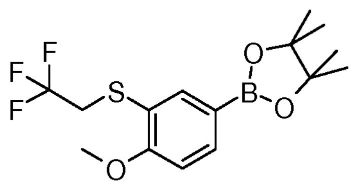

logP[a]: 4,54; logP[b]: 4,46; ESI-Masse (m/z): pos.[a]: 349 $[M+1]^+$; GC-MS: EI-Masse (m/z): 348$[M]^+$; $^1$H-NMR (D6-DMSO, 400MHz): 7,67(d,1H), 7,64-7,62(m,1H), 7,07(d,1H), 3,89(s,3H), 3,83(q,2H), 1,29(s,12H)

**{4-Methoxy-3-[(2,2,2-trifluorethyl)sulfanyl]phenyl}boronsäure (VIIIa-8)**

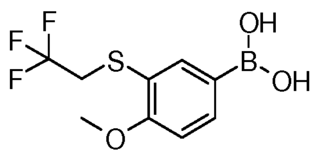

(VIIIa-8)

logP[a]: 1,94; logP[b]: 1,93; ESI-Masse (m/z): neg.[a]: 311 $[M+HCOO^-]^-$, $^1$H-NMR (D6-DMSO, 400MHz): 7,98(breit,2H), 7,86(d,1H), 7,74-7,71(m,1H), 7,02(d,1H), 3,87(s,3H), 3,84(q,2H)

**{2-Fluor-4-methyl-5-[(2,2,2-trifluorethyl)sulfinyl]phenyl}boronsäure (VIIIb-1)**

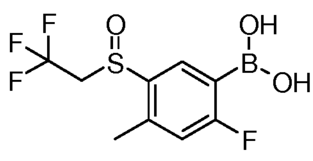

(VIIIb-1)

300 mg (1,12 mmol) {2-Fluor-4-methyl-5-[(2,2,2-trifluorethyl)sulfanyl]phenyl}boronsäure werden in Acetonitril gelöst, mit 396,4 g (1,12 mmol) Selectfluor® versetzt und über Nacht bei Raumtemperatur gerührt. Weitere 39,8 mg (0,12 mmol) Selectfluor® werden zugegeben und die Reaktionsmischung wird 2 h gerührt, dann mit Essigester verdünnt und mit IN Salzsäure gewaschen. Die organische Phase wird über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 102 mg (97% Reinheit, 31% der Theorie der Titelverbindung als farbloses Öl, das bei Lagerung langsam zu farblosem Feststoff kristallisiert.
logP[a]: 1,38; logP[b]: 1,11; ESI-Masse (m/z): pos.[a]: 285 $[M+1]^+$, neg.[a]: 329 $[M+HCOO^-]^-$
$^1$H-NMR (D6-DMSO, 400MHz): 8,36(s,2H), 8,08(d,1H), 7,15(d,1H), 4,14-3,92(m,2H), 2,39(s,3H)

Analog erhalten wurden:

**{2,4-Dimethyl-5-[(2,2,2-trifluorethyl)sulfinyl]phenyl}boronsäure (VIIIb-11)**

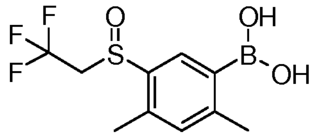

(VIIIb-11)

logP[a]: 1,61; logP[b]: 1,61; ESI-Masse (m/z): pos.[a]: 281 $[M+1]^+$; $^1$H-NMR (D6-DMSO, 400MHz): 8,15(s,2H), 7,96(s,1H), 7,09(s,1H), 4,05-3,85(m,2H), 2,44(s,3H), 2,33(s,3H)

**{2,4-Dichlor-5-[(2,2,2-trifluorethyl)sulfinyl]phenyl}boronsäure (VIIIb-6)**

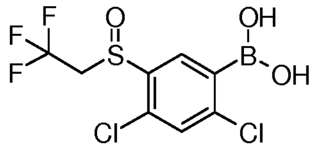

(VIIIb-6)

logP[a]: 1,86; logP[b]: 1,51; ESI-Masse (m/z): pos.[a]: 321 $[M+1]^+$; $^1$H-NMR (D6-DMSO, 400MHz): 8,64(breit,2H), 7,92(s,1H), 7,79(s,1H), 4,29-4,04(m,2H)

**{4-Chlor-3-[(2,2,2-trifluorethyl)sulfinyl]phenyl}boronsäure (VIIIb-3)**

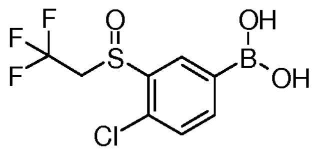

(VIIIb-3)

logP[a]: 1,74; logP[b]: 1,68; ESI-Masse (m/z): pos.[a]: 287 $[M+1]^+$; $^1$H-NMR (D6-DMSO, 400MHz): 8,45(breit,2H), 8,33(d,1H), 8,01-7,99(m,1H), 7,62(d,1H), 4,24-4,12(m,1H), 4,08-3,96(m,1H)

**{4-Methoxy-3-[(2,2,2-trifluorethyl)sulfanyl]phenyl}boronsäure (VIIIb-8)**

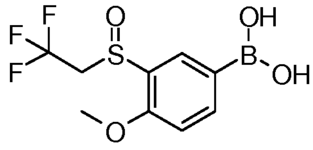

(VIIIb-8)

logP[a]: 1,36; logP[b]: 1,37; ESI-Masse (m/z): pos.[a]: 283 $[M+1]^+$; $^1$H-NMR (D6-DMSO, 400MHz): 8,15(s,1H), 8,13(s,2H), 8,02-8,00(m,1H), 7,17(d,1H), 4,08-3,99(m,1H), 3,90(s,3H), 3,87-3,78(m,1H)

***1,2,4-Triazolidin-3,5-dione der allgemeinen Formel (XXII-C-1)***

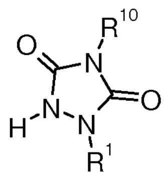

(XXII-C-1)

| R1 | R10 | logP [a] | logP [b] | [M+1]+ | NMR | LöMi | M+ (GCMS) | GCMS-Index |
|---|---|---|---|---|---|---|---|---|
| $CH_2CH(CH_3)_2$ | Cyclopropyl | 0,94 | n.d. | 198 | 10,36(s,1H), 3,17(d,2H), 2,60-2,56(m,1H), 1,96-1,89(m,1H), 0,85-0,78(m,10H) | D6-DMSO | 197 | 1651 |
| $CH_3$ | $C(CH_3)_3$ | 0,83 | n.d. | 172 | 10,15(s,1H), 2,94(s,3H), 1,52(s,9H) | D6-DMSO | 171 | 1380 |
| $CH_2CH_3$ | $C(CH_3)_3$ | 1,15 | n.d. | 186 | 10,11(s,1H), 3,36(q,2H), 1,52(s,9H), 1,07(t,3H) | D6-DMSO | 185 | 1436 |
| Cyclopentyl | $CH_3$ | 0,78 | n.d. | 184 | 10,25(s,1H), 4,38-4,30(m,1H), 2,87(s,3H), 1,81- 1,75(m,2H), 1,66-1,56(m,4H), 1,53-1,48(m,2H) | D6-DMSO | 183 | 1672 |
| $CH_2CH_3$ | $CH(CH_3)_2$ | 0,62 | n.d. | 172 | 10,27(s,1H), 4,14-4,07(m,1H), 3,39(q,2H), 1,32(d,6H), 1,08(t,3H) | D6-DMSO | 171 | 1392 |
| Benzyl | Cyclopropyl | 1,21 | n.d. | 232 | 10,39(s,1H), 7,38-7,28(m,3H), 7,25-7,23(m,2H), 4,56(s,2H), 2,63-2,59(m,1H), 0,84-0,81(m,4H) | D6-DMSO | 231 | 2054 |
| $CH_3$ | $CH(CH_3)_2$ | 0,27 | n.d. | 158 | 10,31(s,1H), 4,14-4,07(m,1H), 2,98(s,3H), 1,32(d,6H) | D6-DMSO | 157 | 1341 |
| $CH_3$ | 3-Trifluormethylpheny 1 | 1,53 | n.d. | 260 | 7,86(s,1H), 7,82-7,80(m,1H), 7,70-7,67(m,2H), 3,16(s,3H) | CD3CN | 259 | 1826 |
| $CH(CH_3)_2$ | Cyclopropyl | 0,50 | n.d. | 184 | 7,53(breit,1H) ,4,20-4,09(m,1H), 2,58-2,50(m,1H), 1,14(d,6H), 0,89-0,83(m,4H) | CD3CN | 183 | 1557 |
| H | Cyclopropyl | n.d. | n.d. | 142 | 9,90(s,2H), 2,61-2,52(m,1H), 0,86-0,76(m,4H) | D6-DMSO | 141 | 1563 |
| $CH(CH_3)_2$ | $CH_3$ | 0,12 | n.d. | 158 | 10,20(s,1H), 4,16-4,08(m,1H), 2,87(s,3H), 1,11(d,6H) | D6-DMSO | 157 | 1359 |
| $CH(CH_3)CH_2CH$ 3 | Cyclopropyl | 0,82 | n.d. | 198 | 7,53(breit,1H) , 3,96-3,88(m,1H), 2,59-2,51(m,1H), 1,61-1,46(m,2H), 1,11(d,3H), 0,91-0,86(m,7H) | CD3CN | 197 | 1634 |
| $CH_3$ | $CH_2CH_3$ | n.d. | n.d. | 144 | 10,40(s,1H), 3,40(q,2H), 3,00(s,3H), 1,10(t,3H) | D6-DMSO | 143 | 1306 |

*Analytik der 2,4-Dihydro-3H-1,2,4-triazol-3-one*

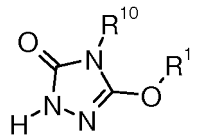

| $R^1$ | $R^{10}$ | logP [a] | logP [b] | NMR | LöMi | M+ (GCMS) | GCMS-Index |
|---|---|---|---|---|---|---|---|
| $CH_3$ | $CH_2CH=CH_2$ | 0,45 | 0,47 | 10,97(s,1H), 5,87-5,76(m,1H), 5,15-5,11(m,1H), 5,03-4,97(m,1H), 4,06-4,03(m,2H), 3,87(s,3H) | D6-DMSO | 155 | 1381 |
| $CH_3$ | Cyclopropyl | 0,27 | 0,31 | 10,79(s,1H), 3,86(s,3H), 2,68-2,61(m,1H), 0,85-0,80(m,1H) | D6-DMSO | 155 | 1448 |
| $CH_2CH=CH_2$ | Cyclopropyl | 1,00 | 1,03 | 8,67(s,1H), 6,10-6,00(m,1H), 5,47-5,39(m,1H), 5,33-5,28(m,1H), 4,72-4,69(m,2H), 2,65-2,58(m,1H), 0,93-0,84(m,4H) | CD3CN | 181 | 1588 |
| $CH_2$-Cyclopropyl | $CH_3$ | 0,84 | 0,88 | 10,85(s,1H), 4,04(d,2H), 2,98(s,3H), 1,28-1,22(m,1H), 0,60-0,52(m,2H), 0,39-0,30(m,2H) | D6-DMSO | 169 | 1576 |
| 2-Chlorbenzyl | $CH_3$ | 1,66 | 1,63 | 8,85(s,1H), 7,58-7,55(m,1H), 7,50-7,46(m,1H), 7,42-7,35(m,2H), 5,36(s,2H), 3,03(s,3H) | CD3CN | 239 | 2101 |

**Herstellbeispiel 1: 2,2,2-Trifluor-N-(1-{2-fluor-4-methyl-5-[(2,2,2-trifluorethyl)sulfinyl]phenyl}pyrrolidin-2-yliden)ethanamin (Bsp. Nr. 3)**

**Stufe 1:** *2,2,2-Trifluor-N-(1-{2-fluor-4-methyl-5-[(2,2,2-trifluorethyl)sulfanyl]phenyl}pyrrolidin-2-yliden)ethanamin (Bsp. Nr. 6)*

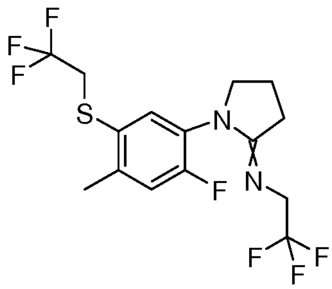

200 mg (0,836 mmol) 2-Fluor-4-methyl-5-[(2,2,2-trifluorethyl)sulfanyl]anilin und 190 mg (0,933 mmol) 4-Chlor-N-(2,2,2-trifluorethyl)butanamid [CAS-RN: 1017050-83-7] werden mit 3 g (19,565 mmol) Phosphorylchlorid 18 h bei 95°C gerührt. Nach Entfernen von überschüssigem Phosphorylchlorid unter vermindertem Druck wird der Rückstand mit Eiswasser versetzt, mit Kaliumcarbonat basisch gestellt und mehrmals mit Dichlormethan extrahiert. Die vereinigten organischen Phasen werden über Natriumsulfat getrocknet und filtriert. Nach Entfernen des Lösungsmittels unter vermindertem Druck werden 320 mg Produkt erhalten (98,5 % der Theorie, Reinheit 86,6 % nach LC/MS).
1H-NMR(D6-DMSO) δ ppm: 7,64-7,62(m,1H), 7,29-7,26(m,1H), 4,30-3,87(m,6H), 3,33(m,2H), 2,38(s,3H), 2,33(m,2H)
logP(HCOOH): 1,68 logP(neutral): 4,24

**Stufe 2:** *2,2,2-Trifluor-N-(1-{2-fluor-4-methyl-5-[(2,2,2-trifluorethyl)sulfinyl]phenyl}pyrrolidin-2-yliden)ethanamin (Bsp. Nr. 3)*

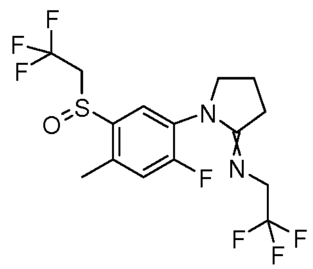

315 mg (0,81 mmol) 2,2,2-Trifluor-N-(1-{2-fluor-4-methyl-5-[(2,2,2-trifluorethyl)sulfanyl]phenyl}pyrrolidin-2-yliden)ethanamin werden bei 0-4 °C in 20 ml Trichlormethan vorgelegt. Nach der Zugabe von 300 mg Puffer-Lösung pH 7 ($KH_2PO_4$/$Na_2HPO_4$) und 55 mg Benzyltriethylammoniumchlorid werden bei 0-4 °C portionsweise 25,4 mg (77 %ig, 0,93 mmol) *meta*-Chlorperbenzoesäure dazugegeben und das Reaktionsgemisch wird 24 h bei Raumtemperatur gerührt. Nach der Zugabe einer 33% igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 54,3 mg Produkt als weissen Feststoff (16,5 % der Theorie, Reinheit 96,6 % nach LC/MS).
1H-NMR(D6-DMSO) δ ppm: 7,99-7,97(m,1H), 7,32-7,29(m,1H), 4,20-4,11(m,1H), 3,90-3,64(m,5H), 2,62(t,2H), 2,37(s,3H), 2,12-2,05(m,2H)
logP(HCOOH): 1,02 logP(neutral): 2,88

**Herstellbeispiel 2: 1-{2,4-Dimethyl-5-[(2,2,2-trifluorethyl)sulfinyl]phenyl}imidazolidin-2-on (Bsp. Nr. 4)**

**Stufe 1:** *1-(2-Chlorethyl)-3-{2,4-dimethyl-5-[(2,2,2-trifluorethyl)sulfanyl]phenyl}harnstoff*

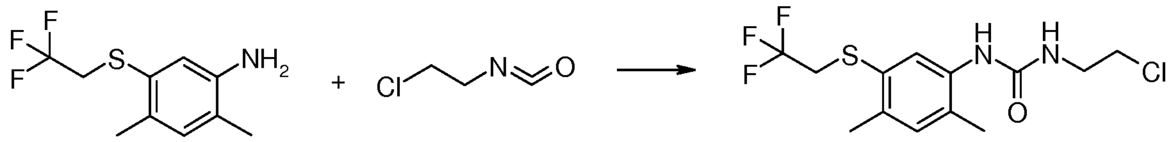

Zu einer Lösung von 0,7 g (6,63 mmol) 2-Chlorethylisothiocyanat in 50 ml tert.-Butylmethylether und einer katalytischen Menge 1,8-Diazabicyclo[5.4.0] undec-7-en (DBU) werden portionsweise 1,45 g (6,16 mmol) 2,4-Dimethyl-5-[(2,2,2-trifluorethyl)sulfanyl]anilin dazugegeben und anschließend noch 18 h bei Raumtemperatur gerührt. Das Gemisch wird fast vollständig im Vakuum vom Lösungsmittel befreit, der anfallende weiße Feststoff wird abgesaugt. Es verbleiben 2 g Produkt (88,5 % der Theorie, Reinheit 94,4 % nach LC/MS.
1H-NMR(D6-DMSO) δ ppm: 8,01(s,1H), 7,83(s,1H), 7,04(s,1H), 7,43-7,40(m,1H), 6,83(t,1H), 3,77-3,69(q,2H), 3,68-3,65(m,2H), 3,45-3,40(m,2H), 2,30(s,3H), 2,14(s,3H)

**Stufe 2:** *1-{2,4-Dimethyl-5-[(2,2,2-trifluorethyl)sulfanyl]phenyl}imidazolidin-2-on (Bsp. Nr. 1)*

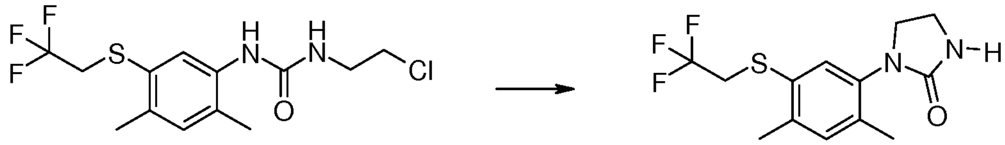

900 mg (2,64 mmol) 1-(2-Chlorethyl)-3-{2,4-Dimethyl-5-[(2,2,2-trifluorethyl)sulfanyl]phenyl}harnstoff werden in einem Gemisch aus 15 ml Wasser und 20 ml Propionitril mit 4 g Kaliumcarbonat 18 h unter Rückfluß erhitzt. Das Gemisch wird im Vakuum vom Lösungsmittel befreit und der verbleibende Feststoffbrei wird mit verdünnter Salzsäure angesäuert. Man lässt 18 h stehen und saugt anschließend den beigen Niederschlag ab. Man erhält 560 mg Rohprodukt. Nach säulenchromatographischer Aufreinigung mit Essigester / Cyclohexan 2:1 v/v als Laufmittel werden 118 mg Produkt erhalten (14,6 % der Theorie, Reinheit nach LC/MS 91,1 %).
1H-NMR(D6-DMSO) δ ppm: 7,37(s,1H), 7,14(s,1H), 6,64(s,1H), 3,92-3,87(q,2H), 3,73-3,70(m,2H), 3,42-3,39(m,2H), 2,33(s,3H), 2,15(s,3H)
13C-NMR(D6-DMSO) δ ppm: 160.3, 137.6, 136.7, 135.6, 132.6, 129.8, 128.9, 126.3, 47.6, 38.1, 35.1, 19.7, 17.6
logP(HCOOH): 2,43 logP(neutral): 2,39

**Stufe 3:** *1-{2,4-Dimethyl-5-[(2,2,2-trifluorethyl)sulfinyl]phenyl}imidazolidin-2-on (Bsp. Nr. 4)*

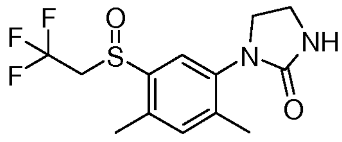

30 mg (0,099 mmol) 1-{2,4-Dimethyl-5-[(2,2,2-trifluorethyl)sulfanyl]phenyl}imidazolidin-2-on werden bei 0-4 °C in 10 ml Trichlormethan vorgelegt. Nach der Zugabe von 100 mg Puffer-Lösung pH 7 ($KH_2PO_4$/$Na_2HPO_4$) und 15 mg Benzyltriethylammoniumchlorid werden bei 0-4 °C portionsweise 25,4 mg (77 %ig, 0,113 mmol) meta-Chlorperbenzoesäure dazugegeben und das Reaktionsgemisch wird 24 h bei Raumtemperatur gerührt. Nach der Zugabe einer 33% igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 9,1 mg Produkt als beigen Feststoff (28,8 % der Theorie, Reinheit 88,4 % nach LC/MS).
13C-NMR(D6-DMSO) δ ppm: 159.9, 139.8, 138.2, 138.2, 132.8, 132.6, 121.0, 56.9,47.1, 37.7, 17.7, 16.7
1H-NMR(D6-DMSO) δ ppm: 7,67(s,1H), 7,24(s,1H), 6,77(breit,1H), 4,13-3,93(m,2H), 3,78-3,71(m,2H), 3,44(t,2H), 2,33(s,3H), 2,24(s,3H)
logP(HCOOH): 1,51 logP(neutral): 1,52

**Herstellbeispiel 3: 1-{2-Fluor-4-methyl-5-[(2,2,2-trifluorethyl)sulfanyl]phenyl}imidazolidin-2-on (Bsp. Nr. 9)**

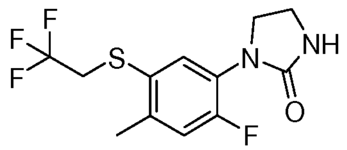

28 mg (0,09 mmol) 2-Fluor-4-methyl-N-(1,3-oxazolidin-2-yliden)-5-[(2,2,2-trifluorethyl)sulfanyl]anilin werden in 5 ml Methyl tert-Butylether nach der Zugabe von 20 mg Triethylamin 24 h bei Raumtemperatur gerührt. Nach Entfernen des Lösungsmittels unter vermindertem Druck verbleiben 23 mg Produkt als weisser Feststoff (82,1 % der Theorie, Reinheit 100 % nach 1H-NMR).
1H-NMR(D6-DMSO) δ ppm: 7,67-7,65(m,1H), 7,23-7,20(m,1H), 6,90(breit,1H), 3,91-3,83(q,2H), 3,80(t,2H), 3,41(t,2H), 2,37(s,3H)
logP(HCOOH): 2,36 logP(neutral): 2,31

**Herstellbeispiel 4: 3-{2-Fluor-4-methyl-5-[(2,2,2-trifluorethyl)sulfinyl]phenyl}-2-[(2,2,2-trifluorethyl)imino]-1,3-thiazolidin-4-on (Bsp. Nr. 13)**

**Stufe 1:** *1-{2-Fluor-4-methyl-5-[(2,2,2-trifluorethyl)sulfanyl]phenyl}-3-(2,2,2-trifluorethyl)thioharnstoff*

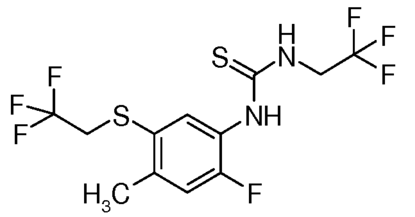

1,00 g (4,18 mmol) 2-Fluor-4-methyl-5-[(2,2,2-trifluorethyl)sulfanyl]anilin wird in 5 ml Dichlormethan vorgelegt und dazu werden 0,006 ml (0,042 mmol) Triethylamin gegeben. Nach der Zugabe von 0,59 g (4,18 mmol) 1,1,1-Trifluor-2-isothiocyanatoethan wird das Reaktionsgemisch über Nacht bei Raumtemperatur gerührt. Unter Vakuum wird das Lösungsmittel entfert, der Rückstand wird mit etwas Toluol verrührt und der unlösliche Teil wird abgesaugt und getrocknet. Es werden 0,31 g (100% Reinheit, 20% d.Th.) der Titelverbindung als weißer Feststoff erhalten. Das Filtrat wird unter Vakuum von Lösungsmittel befreit. Der Rückstand aus 1,30 g enthält die Titelverbindung mit einer Reinheit von 77%.
logP(HCOOH): 3,32; logP(neutral): 3,24; 1H-NMR (D6-DMSO, 400MHz) δ ppm 9,62(bs,1H), 8,34(bs,1H), 7,76(d,1H), 7,26(d,1H), 4,46-4,40(m,2H), 3,87(q,2H), 2,38(s,3H)

**Stufe 2:** *3-{2-Fluor-4-methyl-5-[(2,2,2-trifluorethyl)sulfanyl]phenyl}-2-[(2,2,2-trifluorethyl)imino]-1,3-thiazolidin-4-on (Bsp. Nr. 11)*

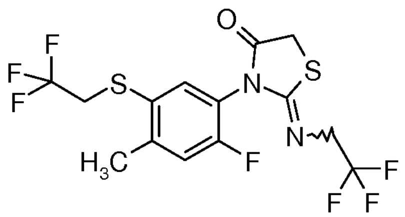

In 80 ml Toluol werden 3,00 g (7,89 mmol) 1-{2-Fluor-4-methyl-5-[(2,2,2-trifluorethyl)sulfanyl]phenyl}-3-(2,2,2-trifluorethyl)thioharnstoff und 1,10 g (7,89 mmol) Bromessigsäure vorgelegt und 6 h bei Rückfluss gerührt. Nach Abkühlen wird das Reaktionsgemisch mit einer gesättigten Natriumchloridlösung versetzt, die organische Phase wird getrennt,

über Natriumsulfat getrocknet und unter Vakuum vom Lösungsmittel befreit. Der Rückstand wird auf RP(C-18) Material aufgezogen und mittels MPLC über RP(C-18) mit Wasser/Acetonitril aufgereinigt. Es werden 0,30 g (95% Reinheit, 9% d. Th.) der Titelverbindung als weißer Feststoff isoliert.
logP(HCOOH): 3,60; logP(neutral): 3,50; 1H-NMR (D6-DMSO, 400MHz) δ ppm 7,62 (d,1H), 7,39(d,1H), 4,39(d,1H), 4,26(d,1H), 4,00-3,83(m,4H), 2,43(s,3H)

**Stufe 3:** *3-{2-Fluor-4-methyl-5-[(2,2,2-trifluorethyl)sulfinyl]phenyl}-2-[(2,2,2-trifluorethyl)imino]-1,3-thiazolidin-4-on (Bsp. Nr. 13)*

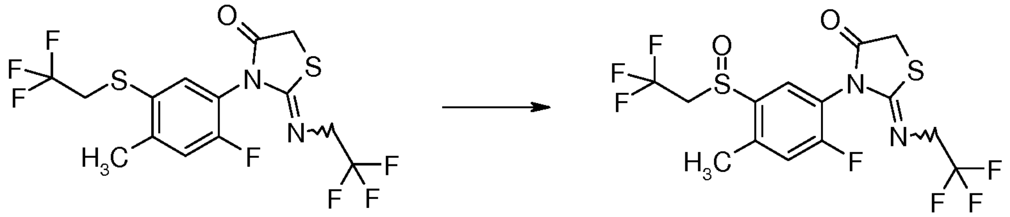

270 mg (0,64 mmol) 3-{2-Fluor-4-methyl-5-[(2,2,2-trifluorethyl)sulfanyl]phenyl}-2-[(2,2,2-trifluorethyl)imino]-1,3-thiazolidin-4-on werden bei 0-4 °C in 5 mL Dichlormethan vorgelegt, 166 mg (0,67 mmol) *meta*-Chlorperbenzoesäure (70%ig) werden dazu gegeben und das Reaktionsgemisch wird 2 h bei Raumtemperatur nachgerührt. Anschließend mit einer 33%igen Natriumthiosulfatlösung (Peroxid-Test durchgeführt) und einer gesättigten Natriumhydrogencarbonatlösung versetzt und zweimal mit Dichlormethan ausgeschüttelt. Die vereinten organischen Phasen werden mit einer gesättigten Natriumcarbonatlösung gewaschen, über Natriumsulfat getrocknet, filtriert, und das Lösemittel wird im Vakuum entfernt. Der Rückstand wird auf RP(C-18) Material aufgezogen und mittels MPLC über RP(C-18) mit Wasser/Acetonitril aufgereinigt. Es werden 195 mg (100% Reinheit, 70% d. Th.) der Titelverbindung als weißer Feststoff isoliert.
logP(HCOOH): 2,70; logP(neutral): 2,64; 1H-NMR (D6-DMSO, 400MHz) δ ppm 7,92(dd,1H), 7,52(dd,1H), 4,41-4,25(m,3H), 4,06-3,69(m,3H), 2,43(s,3H)

**Herstellbeispiel 5: 3-{2-Fluor-4-methyl-5-[(2,2,2-trifluorethyl)sulfanyl]phenyl}-1,5,5-trimethylimidazolidin-2,4-dion (Bsp. Nr. 18)**

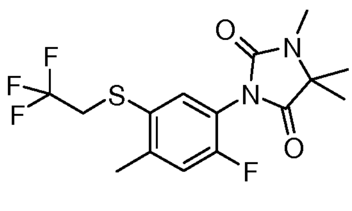

268,0 mg (1,0 mmol) {2-Fluor-4-methyl-5-[(2,2,2-trifluorethyl)sulfanyl]phenyl}boronsäure, 284 mg (2,0 mmol) 1,5,5-Trimethylimidazolidin-2,4-dion, 272 mg (1,5 mmol) Kupfer(II)-acetat, 158 mg (2,0 mmol) Pyridin sowie 0,5 g aktiviertes 3Å-Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 34 mg (99% Reinheit, 9% der Theorie) der Titelverbindung als farbloses Öl.
logP(HCOOH): 2,96; logP(neutral): 2,92; $^1$H-NMR (D6-DMSO, 400MHz) δ ppm 7,69(d,1H), 7,39(d,1H), 3,93(q,2H), 2,88(s,3H), 2,43(s,3H), 1,43(s,6H)

**Herstellbeispiel 6: 3-{2-Fluor-4-methyl-5-[(2,2,2-trifluorethyl)sulfinyl]phenyl}-1,5,5-trimethylimidazolidin-2,4-dion (Bsp. Nr. 17)**

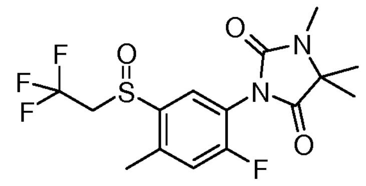

Zu einer Lösung aus 24,0 mg (0,07 mmol) 3-{2-Fluor-4-methyl-5-[(2,2,2-trifluorethyl)sulfanyl]phenyl}-1,5,5-trimethylimidazolidin-2,4-dion in 10 mL Dichlormethan werden bei 0 °C 14,7 mg (0,07 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 25 mg (97% Reinheit, 97% der Theorie) der Titelverbindung als farblosen Feststoff.
logP(HCOOH): 2,04; logP(neutral): 2,01; $^1$H-NMR (D6-DMSO, 400MHz) δ ppm 7,97(d,1H), 7,51(d,1H), 4,32-4,20(m,1H), 4,00-3,89(m,1H), 2,89(s,3H), 2,43(s,3H), 1,45(s,3H), 1,44(s,3H)

**Herstellbeispiel 7: 1-{2-Fluor-4-methyl-5-[(2,2,2-trifluorethyl)sulfanyl]phenyl}pyrrolidin-2,5-dion (Bsp. Nr. 19)**

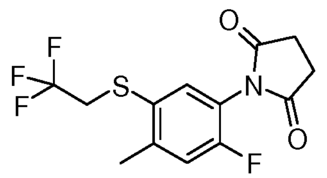

268 mg (1,0 mmol) {2-Fluor-4-methyl-5-[(2,2,2-trifluorethyl)sulfanyl]phenyl}boronsäure, 198 mg (2,0 mmol) Pyrrolidin-2,5-dion, 272 mg (1,5 mmol) Kupfer(II)-acetat, 158 mg (2,0 mmol) Pyridin sowie 0,5 g aktiviertes 3Å -Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 28 mg (100% Reinheit, 9% der Theorie) der Titelverbindung als farbloses Öl.
logP(HCOOH): 2,52; logP(neutral): 2,45; $^1$H-NMR (D6-DMSO, 400MHz) δ ppm 7,56(d,1H), 7,40(d,1H), 3,87(q,2H), 2,86(breit,4H), 2,44(s,3H)

**Herstellbeispiel 8: 1,4-Dimethyl-2-{4-methyl-3-[(2,2,2-trifluorethyl)sulfanyl]phenyl}-1,2,4-triazolidin-3,5-dion (Bsp. Nr. 20)**

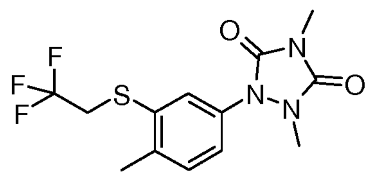

350 mg (1,2 mmol) 5-Brom-2-methylphenyl-2,2,2-trifluorethylsulfid, 190,2 mg (1,5 mmol) 5-Methoxy-4-methyl-2,4-dihydro-3H-1,2,4-triazol-3-on, 23,4 mg (0,12 mmol) Kupfer(I)-iodid, 34,9 mg (0,25 mmol) *trans-N,N'*-Dimethylcyclohexan-1,2-diamin (racemisch), 61 mg (0,37 mmol) Kaliumiodid sowie 508,9 mg (3,7 mmol) Kaliumcarbonat werden in 3 mL entgastem, trockenem Dioxan über Nacht bei 110 °C gerührt. Nach Abkühlen auf Raumtemperatur wird die Reaktionsmischung mit Essigester verdünnt und mit Essigester über Kieselgel filtriert. Nach Adsorption auf Kieselgur und säulenchromatographischer Reinigung an Kieselgel mittels MPLC mit Cyclohexan/Essigester als Laufmittel erhält man 123 mg (90% Reinheit, 27% der Theorie) der Titelverbindung als farbloses Öl.
logP(HCOOH): 2,59; logP(neutral): 2,58; $^1$H-NMR (D6-DMSO, 400MHz) δ ppm 7,49(d,1H), 7,38(d,1H), 7,23-7,21(m,1H), 4,03(q,2H), 3,00(s,3H), 2,99(s,3H), 2,38(s,3H)

**Herstellbeispiel 9: 1,4-Dimethyl-2-{4-methyl-3-[(2,2,2-trifluorethyl)sulfinyl]phenyl}-1,2,4-triazolidin-3,5-dion (Bsp. Nr. 21)**

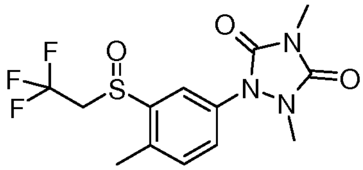

Zu einer Lösung aus 85,0 mg (0,25 mmol) 1,4-Dimethyl-2-{4-methyl-3-[(2,2,2-trifluorethyl)sulfanyl]phenyl}-1,2,4-triazolidin-3,5-dion in 10 mL Dichlormethan werden bei 0 °C 57,1 mg (0,25 mmol) meta-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach Adsorption auf Kieselgur und säulenchromatographischer Reinigung an Kieselgel mittels MPLC mit Cyclohexan/Essigester als Laufmittel erhält man 80,0 mg (87% Reinheit, 78% der Theorie) der Titelverbindung als farbloses Öl.
logP(HCOOH): 1,63; logP(neutral): 1,63; $^{1}$H-NMR (D6-DMSO, 400MHz) $\delta$ ppm 7,79(d,1H), 7,47-7,54(m,2H), 4,12-4,22(m,1H), 4,02-4,08(m,1H), 3,04(s,3H), 3,00(s,3H), 2,38(s,3H)

**Herstellbeispiel 10: 1-Ethyl-2-{2-fluor-4-methyl-5-[(2,2,2-trifluorethyl)sulfanyl]phenyl}-4-phenyl-1,2,4-triazolidin-3,5-dion (Bsp. Nr. 87)**

**Stufe 1:** *1-Ethyl-4-phenyl-1,2,4-triazolidin-3,5-dion*

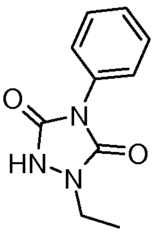

1,35 g (24,0 mmol) Kaliumhydroxid werden in 60 mL Ethanol bei 0 °C mit 3,54 g (20,0 mmol) 4-Phenylurazol versetzt und 30 min bei 0 °C gerührt. 2,50 g (16,0 mmol) Ethyliodid werden bei 0 °C zugesetzt und die Reaktionsmischung wird über Nacht bei Raumtemperatur gerührt. Erneut werden 2,50 g (16,0 mmol) Ethyliodid und 20 mL Ethanol zugesetzt und 3 d bei Raumtemperatur gerührt. Die Reaktionsmischung wird 3 h auf 60 °C erhitzt, dann abgekühlt, in Wasser gegossen, mit verdünnter Salzsäure auf pH = 2-3 gebracht und unter vermindertem Druck vom Lösungsmittel befreit. Nach säulenchromatographischer Reinigung an RP(C-18)-Kieselgel mittels MPLC mit Wasser/Acetonitril (1% Ameisensäure) als Laufmittel erhält man 1,54 g (100% Reinheit, 37% der Theorie) der Titelverbindung als farblosen Feststoff.
logP(HCOOH): 0,91; $^{1}$H-NMR (D6-DMSO, 400MHz) $\delta$ ppm 10,79(s,1H), 7,51-7,37(m,5H), 3,54(q,2H), 1,18(t,3H)

**Stufe 2:** *1-Ethyl-2-{2-fluor-4-methyl-5-[(2,2,2-trifluorethyl)sulfanyl]phenyl}-4-phenyl-1,2,4-triazolidin-3,5-dion (Bsp. Nr. 87)*

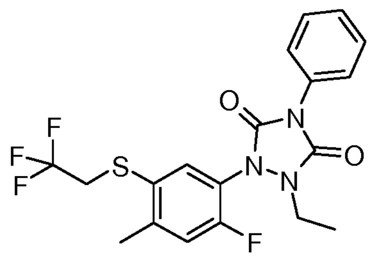

230,0 mg (0,86 mmol) {2-Fluor-4-methyl-5-[(2,2,2-trifluorethyl)sulfanyl]phenyl}boronsäure, 352 mg (1,72 mmol) 1-Ethyl-4-phenyl-1,2,4-triazolidin-3,5-dion, 233,8 mg (1,29 mmol) Kupfer(II)-acetat, 136 mg (1,72 mmol) Pyridin sowie 0,5 g aktiviertes 3Å -Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 160 mg (96% Reinheit, 42% der Theorie) der Titelverbindung als farbloses Öl. logP(HCOOH): 3,71; logP(neutral): 3,69; $^1$H-NMR (D6-DMSO, 400MHz) δ ppm 7,87(d,1H), 7,57-7,44(m,6H), 4,05(q,2H), 3,49(q,2H), 2,44(s,3H), 1,08(t,3H)

**Herstellbeispiel 11: 1-Ethyl-2-{2-fluor-4-methyl-5-[(2,2,2-trifluorethyl)sulfinyl]phenyl}-4-phenyl-1,2,4-triazolidin-3,5-dion (Bsp. Nr. 88)**

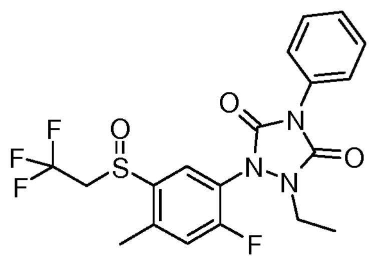

Zu einer Lösung aus 80,0 mg (0,19 mmol) 1-Ethyl-2-{2-fluor-4-methyl-5-[(2,2,2-trifluorethyl)sulfanyl]phenyl}-4-phenyl-1,2,4-triazolidin-3,5-dion in 10 mL Dichlormethan werden bei 0 °C 44,0 mg (0,20 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach Adsorption auf Kieselgur und säulenchromatographischer Reinigung an Kieselgel mittels MPLC mit Cyclohexan/Essigester als Laufmittel erhält man 74 mg (100% Reinheit, 89% der Theorie) der Titelverbindung als farblosen Feststoff.
logP(HCOOH): 2,71; logP(neutral): 2,66; $^1$H-NMR (D6-DMSO, 400MHz) δ ppm 8,10(d,1H), 7,59(d,1H), 7,55-7,51(m,4H), 7,48-7,44(m,1H), 4,31-4,09(m,2H), 3,57-3,51(m,2H), 2,44(s,3H), 1,09(t,3H)

**Herstellbeispiel 12: 1,4-Diethyl-2-{4-methyl-3-[(2,2,2-trifluorethyl)sulfanyl]phenyl}-1,2,4-triazolidin-3,5-dion (Bsp. Nr. 100)**

**Stufe 1:** *1,4-Diethyl-1,2,4-triazolidin-3,5-dion*

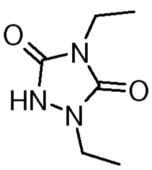

5,00 g (83,2 mmol) Ethylhydrazin werden mit 10,32 g (87,4 mmol) Diethylcarbonat versetzt und über Nacht zum Rückfluss erhitzt. Nach Abkühlen auf Raumtemperatur wird das Lösungsmittel unter vermindertem Druck entfernt.

Der Rückstand wird in 50 mL Toluol aufgenommen, mit 7,10 g (99,8 mmol) Ethylisocyanat versetzt, 30 min zum Rückfluss erhitzt und nach Abkühlen auf Raumtemperatur unter vermindertem Druck vom Lösungmittel befreit. Der Rückstand wird mit 83,2 mL einer 4N Kaliumhydroxid-Lösung (332,8 mmol) versetzt und 20 min zum Rückfluss erhitzt. Nach Abkühlen auf Raumtemperatur wird die Reaktionslösung unter Kühlung mit halbkonzentrierter Salzsäure sauer gestellt und mit Dichlormethan extrahiert. Die vereinten organischen Phasen werden über Magnesiumsulfat getrocknet, filtriert und unter vermindertem Druck vom Lösungsmittel befreit. Nach Gefriertrocknung erhält man 4,61 g (100% Reinheit, 35% der Theorie) der Titelverbindung als farblosen Feststoff.
logP(HCOOH): 0,14; $^1$H-NMR (D6-DMSO, 400MHz) $\delta$ ppm 10,35(s,1H), 3,42(q,2H), 3,38(q,2H), 1,10(t,3H), 1,09(t,3H)

**Stufe 2:** *1,4-Diethyl-2-{4-methyl-3-[(2,2,2-trifluorethyl)sulfanyl]phenyl}-1,2,4-triazolidin-3,5-dion (Bsp. Nr. 100)*

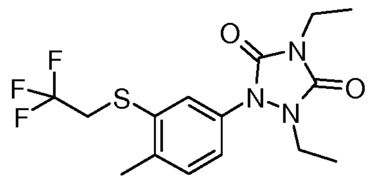

250,0 mg (1,0 mmol) {4-Methyl-3-[(2,2,2-trifluorethyl)sulfanyl]phenyl}boronsäure, 314,3 mg (2,0 mmol) 1,4-Diethyl-1,2,4-triazolidin-3,5-dion, 272,5 mg (1,5 mmol) Kupfer(II)-acetat, 158 mg (2,0 mmol) Pyridin sowie 0,5 g aktiviertes 3Å -Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel sowie säulenchromatographisch an RP(C-18)-Kieselgel mittels MPLC mit Wasser/Acetonitril als Laufmittel gereinigt. Man erhält man 177,0 mg (100% Reinheit, 49% der Theorie) der Titelverbindung als farbloses Öl.
logP(HCOOH): 3,32; logP(neutral): 3,29; $^1$H-NMR (D6-DMSO, 400MHz) $\delta$ ppm 7,52(d,1H), 7,38(d,1H), 7,25-7,22(m,1H), 4,05(q,2H), 3,52(q,2H), 3,46(q,2H), 2,37(s,3H), 1,17(t,3H), 0,96(t,3H)

**Herstellbeispiel 13: 1-(2,2-Difluorethyl)-3-{2-fluor-4-methyl-5-[(2,2,2-trifluorethyl)sulfinyl]phenyl}imidazolidin-2,4,5-trion (Bsp. Nr. 103)**

**Stufe 1:** *1-(2,2-Difluorethyl)-3-[2-fluor-4-methyl-5-[(2,2,2-trifluorethyl)sulfanyl]phenyl}harnstoff*

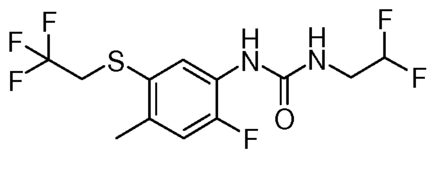

Zu einer Lösung von 600 mg (2,51 mmol) 2-Fluor-4-methyl-5-[(2,2,2-trifluorethyl)sulfanyl]anilin in 40 ml Methyl tert-Butylether und einer katalytischen Menge 1,8-Diazabicyclo[5.4.0] undec-7-en (DBU) werden portionsweise 300 mg (2,8 mmol) 1,1-Difluor-2-isocyanatoethan hinzugefügt und es wird anschliessend noch 18 h bei Raumtemperatur gerührt. Das Gemisch wird fast vollständig im Vakuum vom Lösungsmittel befreit, der anfallende weisse Feststoff wird abgesaugt. Es verbleiben 660 mg Produkt (76 % der Theorie, Reinheit 96,5 % nach LC/MS).
1H-NMR(D6-DMSO) $\delta$ ppm: 8,52(s,1H), 8,31-8,29(m,1H), 7,20-7,17(m,1H), 6,92(t,1H), 6,07(tt,1H), 3,79-3,71(q,2H), 3,60-3,48(m,2H), 2,34(s,3H)

**Stufe 2:** *1-(2,2-Difluorethyl)-3-{2-fluor-4-methyl-5-[(2,2,2-trifluorethyl)sulfanyl]phenyl]imidazolidin-2,4,5-trion (Bsp. Nr. 101)*

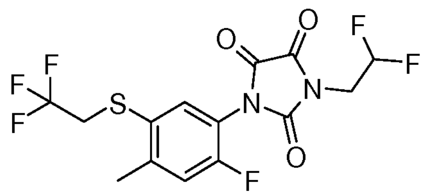

Zu 200 mg (0,578 mmol) 1-(2,2-Difluorethyl)-3-{2-fluor-4-methyl-5-[(2,2,2 trifluorethyl)sulfanyl]phenyl}harnstoff in 20 ml Acetonitril tropft man 160 mg (1,261 mmol) Oxalylchlorid (gelöst in 5 ml Acetonitril) und rührt noch 18 h unter Rückfluß. Der nach dem Einrotieren im Vakuum verbleibende Rückstand wird mit Wasser verrührt und abgesaugt. Als Rückstand verbleiben 195 mg Produkt als weisser Feststoff (84,4 % der Theorie, Reinheit 100 % nach 1H-NMR).
1H-NMR(D6-DMSO) δ ppm: 7,70-7,68(m,1H), 7,51-7,48(m,1H), 6,25(tt,1H), 4,10-4,01(dt,2H), 3,90-3,82(q,2H), 2,48(s,3H)
13C-NMR(D6-DMSO) δ ppm: 156.4, 156.3, 151.6, 144.1, 133.0, 128.4, 125.3, 118.3, 115.4, 112.8, 40.4, 35.0, 19.9

logP(HCOOH): 3,22 logP(neutral): 1,73

**Stufe 3:** *1-(2,2-Difluorethyl)-3-{2-fluor-4-methyl-5-[(2,2,2-trifluorethyl)sulfinyl]phenyl)imidazolidin-2,4,5-trion (Bsp. Nr. 103)*

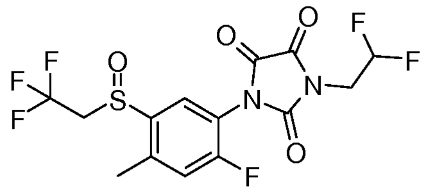

195 mg (0,487 mmol) 1-(2,2-Difluorethyl)-3-{2-fluor-4-methyl-5-[(2,2,2-trifluorethyl)sulfanyl]phenyl}imidazolidin-2,4,5-trion werden bei 0 °C in 10 ml Essigsäure vorgelegt. Nach der Zugabe katalytischer Mengen von Natriumwolframat werden bei 0-4 °C portionsweise 765 mg (0,675 mmol) 3 % ige wässrige Wasserstoffperoxidlösung dazugegeben und das Reaktionsgemisch wird 24 h bei RT gerührt. Nach der Zugabe einer 33% igen wäßrigen Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) aufgereinigt. Mit Wasser/Acetonitril erhält man 100 mg Produkt als weissen Feststoff (44,4 % der Theorie, Reinheit 90 % nach 1H-NMR).
1H-NMR(D6-DMSO) δ ppm: 8,04-8,03(m,1H), 7,61-7,58(m,1H), 6,24(tt,1H), 4,36-4,27(m,1H), 4,10-4,01(dt,2H), 3,92-3,83(m,1H), 2,43(s,3H)
logP(HCOOH): 2,32 logP(neutral): 0,87

**Herstellbeispiel 14: 3-[2-Fluor-4-methyl-5-(2,2,2-trifluorethylsulfinyl)phenyl]-oxazolidin-2-on (Bsp. Nr. 105)**

**Stufe 1:** *2-Chlorethyl-N-[2-fluor-4-methyl-5-(2,2,2-trifluorethylsuffanyl)phenyl]carbamat*

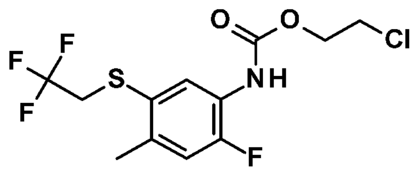

2-Fluor-4-methyl-5-[(2,2,2-trifluorethyl)sulfanyl]anilin (500 mg, 2,09 mmol) wird in 5 ml THF bei 0 °C vorgelegt. Dazu werden Triethylamin (435 μL, 2 mmol) und 2-Chlorethylchlorformiat (218 μL, 2,09 mmol) gegeben und die Reaktionsmischung wird 4 h unter Rückfluss gerührt. Die Lösung wird abgekühlt und mit Wasser versetzt. Nach der Extraktion mit Ethylacetat werden die vereinigten organischen Phasen mit Wasser und ges. NaCl-Lösung gewaschen und danach über $Na_2SO_4$ getrocknet. Das Lösungsmittel wird am Rotationsverdampfer entfernt. Durch säulenchromatographische Reinigung (25% EtOAc/Petrolether) des Rohprodukts werden 360 mg (1,04 mmol, 50% der Theorie) der Titelverbindung erhalten.
1H-NMR (D6-DMSO, 400MHz) δ ppm: 9,51 (s,1H), 7,78 (d,1H), 7,22 (d,1H), 4,34 (t,2H), 3,87-3,77 (m,4H), 2,37 (s,3H)
logP(HCOOH): 3,45

**Stufe 2:** *3-[2-Fluor-4-methyl-5-(2,2,2-trifluorethylsulfanyl)phenyl]oxazolidin-2-on (Bsp. Nr. 104)*

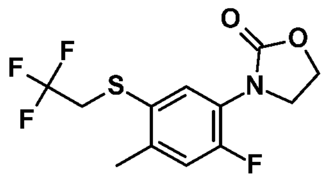

2-Chlorethyl-N-[2-fluor-4-methyl-5-(2,2,2-trifluorethylsulfanyl)phenyl]carbamat (400 mg, 1,16 mmol) wird in 5 mL DMF gelöst und mit Cäsiumcarbonat (565 mg, 1,74 mmol) versetzt. Die Mischung wird 8 h bei 80 °C gerührt. Die Lösung wird auf Raumtemperatur gekühlt, mit Wasser versetzt und es wird mit Ethylacetat extrahiert. Die vereinigten organischen Phasen werden mit Wasser und ges. NaCl-Lösung gewaschen und danach über $Na_2SO_4$ getrocknet. Das Lösungsmittel wird am Rotationsverdampfer entfernt. Durch säulenchromatographische Reinigung (35% EtOAc/Petrolether) des Rohprodukts werden 250 mg (0,81 mmol, 70% der Theorie) der Titelverbindung erhalten.
1H-NMR ($CDCl_3$, 400MHz) δ ppm: 7.71 (d,1H), 7.03 (d,1H), 4.54-4.49 (m,2H), 4.08-4.03 (m,2H), 3.37-3.31 (m,2H), 2.46 (s,3H)
logP(HCOOH): 2,66

**Stufe 3:** *3-[2-Fluor-4-methyl-5-(2,2,2-trifluorethylsulfinyl)phenyl]oxazolidin-2-on (Bsp. Nr. 105)*

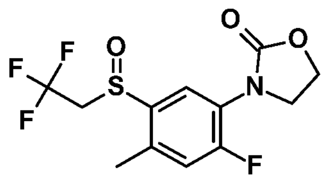

3-[2-Fluor-4-methyl-5-(2,2,2-trifluorethylsulfanyl)phenyl]oxazolidin-2-on (170 mg, 0,55 mmol) wird in 3 mL Aceton/Wasser (1:1) gelöst und mit Oxon (169 mg, 0,34 mmol) versetzt. Die Lösung wird 2 h bei Raumtemperatur gerührt. Die Mischung wird im Vakuum eingeengt und der Rückstand wird mit Wasser versetzt. Es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (40% EtOAc/Petrolether) des Rohprodukts werden 90 mg (0,23 mmol, 50% der Theorie) der Titelverbindung erhalten.
1H-NMR ($CDCl_3$,400MHz) δ ppm:8.11 (d,1H), 7.19 (d,1H), 4.58-4.49 (m,2H), 4.18-4.10 (m,1H), 4.04-3.96 (m,1H), 3.59-3.33 (m,2H), 2.41 (s,3H)
logP(HCOOH): 1,64

**Herstellbeispiel 15: 3-[2-Fluor-4-methyl-5-(2,2,2-trifluorethylsulfinyl)phenyl]-5-methyl-oxazolidin-2-on (Bsp. Nr. 109)**

**Stufe 1:** *1-[2-Fluor-4-methyl-5-(2,2,2-trifluorethylsulfanyl)anilin]propan-2-ol*

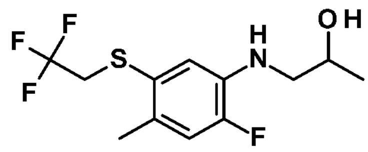

2-Fluor-4-methyl-5-[(2,2,2-trifluorethyl)sulfanyl]anilin (700 mg, 2,93 mmol) wird in 10 ml Dichlormethan vorgelegt. Dazu werden Propylenoxid (4,39 mmol) und LiBr (76,2 mg, 0,88 mmol) gegeben und die Reaktionsmischung wird 16 h bei Raumtemperatur gerührt. Die Lösung wird abgekühlt und mit Wasser versetzt. Nach der Extraktion mit Dichlormethan werden die vereinigten organischen Phasen mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (25% EtOAc/Petrolether) des Rohprodukts werden 200 mg (0,67 mmol, 23% der Theorie) der Titelverbindung erhalten.
1H-NMR ($CDCl_3$, 400MHz) δ ppm: 6.91 (d,1H), 6.86 (d,1H), 4.10-4.02 (m,2H), 3.32-3.20 (m,3H), 3.05-3.00 (m,1H), 2.37 (s,3H), 1.85-1.80 (m,1H), 1,30-1.29 (m,3H)
logP(HCOOH): 3,01

**Stufe 2:** *3-[2-Fluor-4-methyl-5-(2,2,2-trifluorethylsulfanyl)phenyl]-5-methyl-oxazolidin-2-on (Bsp. Nr. 106)*

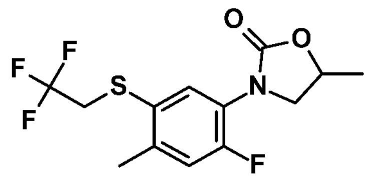

1-[2-Fluor-4-methyl-5-(2,2,2-trifluorethylsulfanyl)anilin]propan-2-ol (200 mg, 0,67 mmol) wird in 5 mL THF gelöst und mit Phosgen (1,01 mmol, als 20%ige Lösung in Toluol) und DIPEA (172 μL, 1,01 mmol) versetzt. Die Mischung wird 16 h bei Raumtemperatur gerührt. Die Lösung wird auf Raumtemperatur gekühlt, mit Wasser versetzt und es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (15% EtOAc/Petrolether) des Rohprodukts werden 120 mg (0,37 mmol, 55% der Theorie) der Titelverbindung erhalten.
1H-NMR (D6-DMSO,400MHz) δ ppm: 7.74 (d,1H), 7.31 (d,1H), 4.87-4.80 (m,1H), 4.10-4.04 (m,1H), 3.98-3.88 (m,2H), 3.64-3.59 (m,1H), 2.39 (s,3H), 1.43 (d,3H)
logP(HCOOH): 2.98

**Stufe 3:** *3-[2-Fluor-4-methyl-5-(2,2,2-trifluorethylsulfinyl)phenyl]-5-methyl-oxazolidin-2-on (Bsp. Nr. 109)*

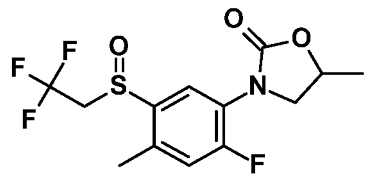

3-[2-Fluor-4-methyl-5-(2,2,2-trifluorethylsulfanyl)phenyl]-5-methyl-oxazolidin-2-on (120 mg, 0,37 mmol) wird in 3 mL Aceton/Wasser (1:1) gelöst und mit Oxon (184 mg, 0,37 mmol) versetzt. Die Lösung wird 2 h bei Raumtemperatur gerührt. Die Mischung wird im Vakuum eingeengt und der Rückstand wird mit Wasser versetzt. Es wird mit Ethylacetat extrahiert. Die vereinigten organischen Phasen werden mit Wasser und ges. NaCl-Lösung gewaschen und danach über $Na_2SO_4$ getrocknet. Das Lösungsmittel wird am Rotationsverdampfer entfernt. Durch säulenchromatographische Reinigung (40% EtOAc/Petrolether) des Rohprodukts werden 82 mg (0,24 mmol, 65% der Theorie) der Titelverbindung erhalten.
1H-NMR ($CDCl_3$, 400MHz) δ ppm 8.11 (d,1H), 7.08 (d,1H), 4.90-4.81 (m,1H), 4.20-4.01 (m,1H), 3.76-3.37 (m,3H), 2.40

(s,3H), 1.57-1.54 (m,3H)
logP(HCOOH): 1,93; logP (neutral): 1,89

**Herstellbeispiel 16: 3-[2-Fluor-4-methyl-5-(2,2,2-trifluorethylsulfinyl)phenyl]-4-methyl-oxazolidin-2-on (Bsp. Nr. 107)**

**Stufe 1:** *Allyl N-[2-fluor-4-methyl-5-(2,2,2-trifluorethylsulfanyl)phenyl]carbamat*

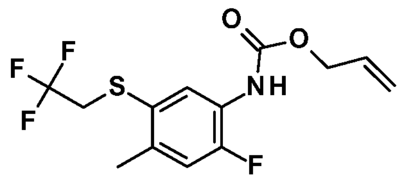

2-Fluor-4-methyl-5-[(2,2,2-trifluorethyl)sulfanyl]anilin (1.0 g, 4,18 mmol) wird in 10 ml THF vorgelegt. Dazu werden Allyloxycarbonylchlorid (530 μL, 5,02 mmol) und Cäsiumcarbonat (2,04 g, 6,27 mmol) gegeben und die Reaktionsmischung wird 16 h bei Raumtemperatur gerührt. Die Lösung wird abgekühlt und mit Wasser versetzt. Nach der Extraktion mit Ethylacetat werden die vereinigten organischen Phasen mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (10% EtOAc/Petrolether) des Rohprodukts werden 800 mg (2,47 mmol, 59% der Theorie) der Titelverbindung erhalten.
1H-NMR ($CDCl_3$, 400MHz) δ ppm 8.27 (s,1H), 6.98-6.79 (m,2H), 6.03-5.93 (m,1H), 5.41-5.28 (m,2H), 4.69 (d,2H), 3.38 (q,2H), 2.42 (s,3H)
logP(HCOOH): 3,59

**Stufe 2:** *3-[2-Fluor-4-methyl-5-(2,2,2-trifluorethylsulfanyl)phenyl]-4-methyl-oxazolidin-2-on (Bsp. Nr. 108)*

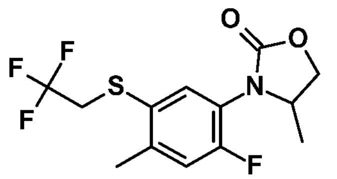

Allyl *N*-[2-fluor-4-methyl-5-(2,2,2-trifluorethylsulfanyl)phenyl]carbamat (800 mg, 2,5 mmol) wird in 10 mL THF/DMSO/Fluorbenzol (8:1:1) gelöst und mit IBX (12,3 mmol) versetzt. Die Mischung wird 48 h auf 120 °C in einem verschlossenen, druckstabilen Gefäß erhitzt. Die Lösung wird auf Raumtemperatur gekühlt, mit Wasser versetzt und es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (25% EtOAc/Petrolether) des Rohprodukts werden 171 mg (0,53 mmol, 21% der Theorie) der Titelverbindung erhalten.
1H-NMR ($CDCl_3$,400MHz) δ ppm 7.56 (d,1H), 7.06 (d,1H), 4.65-4.60 (m,1H), 4.45-4.38 (m,1H), 4.06-4.01 (m,1H), 3.35 (q, 2H), 2.48 (s,3H), 1.26-1.22 (m,3H)
logP(HCOOH): 2,92

**Stufe 3:** *3-[2-Fluor-4-methyl-5-(2,2,2-trifluorethylsulfinyl)phenyl]-4-methyl-oxazolidin-2-on (Bsp. Nr. 107)*

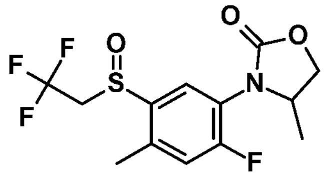

3-[2-Fluor-4-methyl-5-(2,2,2-trifluorethylsulfanyl)phenyl]-4-methyl-oxazolidin-2-on (103 mg, 0,32 mmol) wird in 3 mL Aceton/Wasser (1:1) gelöst und mit Oxon (184 mg, 0,37 mmol) versetzt. Die Lösung wird 2 h bei Raumtemperatur gerührt. Die Mischung wird im Vakuum eingeengt und der Rückstand wird mit Wasser versetzt. Es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (40% EtOAc/Petrolether) des Rohprodukts werden 67 mg (0,20 mmol, 62% der Theorie) der Titelverbindung erhalten.
1H-NMR ($CDCl_3$,400MHz) $\delta$ ppm 7.98 (dd, 1H), 7.26-7.07 (m,1H), 4.68-4.62 (m,1H), 4.51-4.42 (m,1H), 4.11-4.02 (m,1H), 3.53-3.39 (m,2H), 2.41 (s,3H), 1.26-1.23 (m,3H)
logP(HCOOH): 1,79

**Herstellbeispiel 17: 3-[2-fluor-4-methyl-5-(2,2,2-trifluorethylsulfinyl)phenyl]-5,5-dimethyl-oxazolidin-2,4-dion (Bsp. Nr. 110)**

**Stufe 1:** *N-[2-fluor-4-methyl-5-(2,2,2-trifluorethylsulfanyl)phenyl]-2-hydroxy-2-methyl-propanamid*

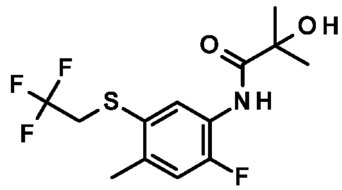

2-Fluor-4-methyl-5-[(2,2,2-trifluorethyl)sulfanyl]anilin (1,00 g, 4,18 mmol) wird in 10 ml Dichlormethan bei 0 °C vorgelegt. Dazu werden 2-Hydroxyisobuttersäureethylester (1,15 mL, 8,36 mmol) und $AlMe_3$ (10,5 mmol, als 2 M Lösung in Toluol) gegeben und die Reaktionsmischung wird 16 h bei 65 °C in einem verschlossenen, druckstabilen Gefäß gerührt. Die Lösung wird abgekühlt und mit Wasser versetzt. Nach der Extraktion mit Dichlormethan werden die vereinigten organischen Phasen mit 1 N HCl und gesättigter Natriumchlorid-Lösung gewaschen und danach über Natriumsulfat getrocknet. Das Lösungsmittel wird am Rotationsverdampfer entfernt. Durch säulenchromatographische Reinigung (15% EtOAc/Petrolether) des Rohprodukts werden 500 mg (1,53 mmol, 37% der Theorie) der Titelverbindung erhalten.
1H-NMR ($CDCl_3$, 400MHz) $\delta$ ppm 8.89 (s,1H), 8.57 (d,1H), 6.98 (d,1H), 3.40 (q,2H), 2.43 (s,3H), 2.15 (s,1H), 1.57 (s,6H)
logP(HCOOH): 2,81

**Stufe 2:** *3-[2-Fluor-4-methyl-5-(2,2,2-trifluorethylsulfanyl)phenyl]-5,5-dimethyl-oxazolidin-2,4-dion (Bsp. Nr. 111)*

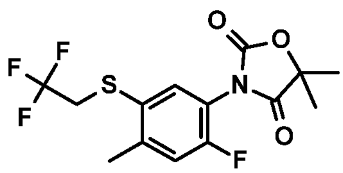

*N*-[2-fluor-4-methyl-5-(2,2,2-trifluorethylsulfanyl)phenyl]-2-hydroxy-2-methyl-propanamid (400 mg, 1,23 mmol) wird in 5 mL THF gelöst und mit Phosgen (1,84 mmol, als 20%ige Lösung in Toluol) sowie Diisopropylethylamin (418 µL, 2,36 mmoL) versetzt. Die Mischung wird 16 h bei Raumtemperatur gerührt. Die Lösung wird mit Wasser versetzt und es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (10% EtOAc/Petrolether) des Rohprodukts werden 200 mg (0,57 mmol, 46% der Theorie) der Titelverbindung erhalten.
1H-NMR ($CDCl_3$,400MHz) $\delta$ ppm 7.50 (d,1H), 7.16 (d,1H), 3.39-3.32 (m,2H), 2.53 (s,3H), 1.71 (s,6H)
logP(HCOOH): 3,35

**Stufe 3:** *3-[2-fluor-4-methyl-5-(2,2,2-trifluorethylsulfinyl)phenyl]-5,5-dimethyl-oxazolidin-2,4-dione (Bsp. Nr. 110)*

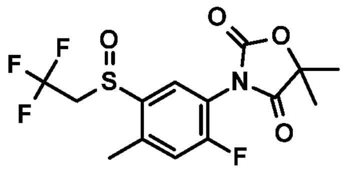

3-[2-Fluor-4-methyl-5-(2,2,2-trifluorethylsulfanyl)phenyl]-5,5-dimethyl-oxazolidin-2,4-dion (120 mg, 0,34 mmol) wird in 3 mL Aceton/Wasser (1:1) gelöst und mit Oxon (105 mg, 0,34 mmol) versetzt. Die Lösung wird 2 h bei Raumtemperatur gerührt. Die Mischung wird im Vakuum eingeengt und der Rückstand wird mit Wasser versetzt. Es wird mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (20% EtOAc/Petrolether) des Rohprodukts werden 85 mg (0,23 mmol, 68% der Theorie) der Titelverbindung erhalten.
1H-NMR ($CDCl_3$,400MHz) $\delta$ ppm 7.99 (d,1H), 7.24 (d,1H), 3.52-3.41 (m,2H), 2.45 (s,3H), 1.72 (s,6H)
logP(HCOOH): 2,38

**Herstellbeispiel 18: 4-{2-Fluor-4-methyl-5-[(2,2,2-trifluorethyl)sulfanyl]phenyl}-1,2-dimethyl-1,2,4-triazolidin-3,5-dion (Bsp. Nr. 116)**

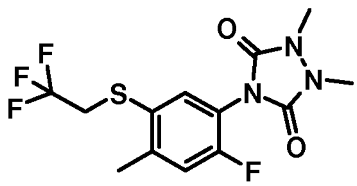

Eine Lösung von 1,0 g (4,18 mmol) 2-Fluor-4-methyl-5-[(2,2,2-trifluorethyl)sulfanyl]anilin in 10 mL Toluol wird mit 2,1 mL (4,18 mmol) einer Lösung von Trimethylaluminium in Toluol (2M) versetzt und 30 min unter Argon bei Raumtemperatur gerührt. 426,8 mg (2,09 mmol) Diethyl-1,2-dimethylhydrazin-1,2-dicarboxylat werden zugegeben. Die Reaktionsmischung wird 30 min bei Raumtemperatur gerührt und anschließend über Nacht zum Rückfluss erhitzt. Nach Abkühlen wird die Reaktionsmischung in eine Mischung aus Essigester und 1N Salzsäure gegeben, die Phasen werden getrennt, die organische Phase wird über Magnesiumsulfat getrocknet, filtriert und konzentriert. Nach säulenchromatographischer Reinigung an Kieselgel mittels MPLC mit Cyclohexan/Essigester als Laufmittel erhält man 486 mg (97% Reinheit, 64% der Theorie) der Titelverbindung als beigen Feststoff.
logP[a]: 2,40; logP[b]: 2,39; $^{1}H$-NMR ($D^6$-DMSO, 400MHz) ppm: 7,72(d,1H), 7,42(d,1H), 3,93(q,2H), 3,19(s,6H), 2,44(s,3H)

**Herstellbeispiel 19: 4-{2-Fluor-4-methyl-5-[(2,2,2-trifluorethyl)sulfinyl]phenyl}-1,2-dimethyl-1,2,4-triazolidin-3,5-dion (Bsp. Nr. 117)**

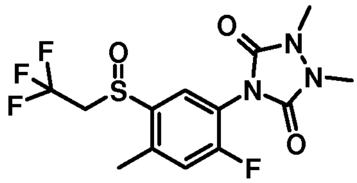

Zu einer Lösung von 243 mg (0,69 mmol) 4-{2-Fluor-4-methyl-5-[(2,2,2-trifluorethyl)sulfanyl]phenyl}-1,2-dimethyl-1,2,4-triazolidin-3,5-dion in 10 mL Dichlormethan werden bei 0 °C 155 mg (0,69 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 270 mg (100% Reinheit, 100% der Theorie) der Titelverbindung als farbloses Öl.
logP[a]: 1,54; logP[b]: 1,50; $^1$H-NMR (D$^6$-DMSO, 400MHz) δ ppm: 7,99(d,1H), 7,54(d,1H), 4,29-4,25(m,1H), 3,96-3,89(m,1H), 3,19(s,6H), 2,50(s,3H)

**<u>Herstellbeispiel 20:</u> 1,2-Diethyl-4-{2-fluor-4-methyl-5-[(2,2,2-trifluorethyl)sulfanyl]phenyl}-1,2,4-triazolidin-3,5-dion (Bsp. Nr. 118)**

**Stufe 1:** *Ethyl-2-({2-fluor-4-methyl-5-[(2,2,2-trifluorethyl)sulfanyl]phenyl}carbamoyl)hydrazin-carboxylat*

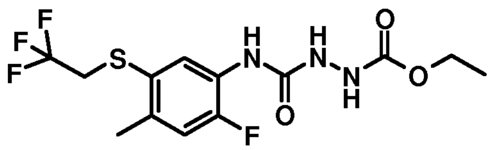

Zu einer Lösung von 1,10 g (3,53 mmol) Ethyl-{2-fluor-4-methyl-5-[(2,2,2-trifluorethyl)sulfanyl]phenyl}carbamat (siehe z. B. JP2011/042611) in Ethanol (15 mL) werden bei Raumtemperatur 404,3 mg (3,88 mmol) Ethyl-hydrazincarboxylat gegeben. Die Reaktionsmischung wird 4 h zum Rückfluss erhitzt, dann auf Raumtemperatur abgekühlt, mit Wasser verdünnt und mit Essigester extrahiert. Die vereinten organische Phasen werden mit Wasser und wässriger gesättigter Natriumchloridlösung gewaschen, über Natriumsulfat getrocknet, filtriert und unter vermindertem Druck konzentriert. Nach säulenchromatographischer Reinigung an Kieselgel mit Petrolether/Essigester (85/15) als Laufmittel erhält man 850 mg (100% Reinheit, 65% der Theorie) der Titelverbindung als beigen Feststoff.
logP[a]: 2,41; $^1$H-NMR (D$^6$-DMSO, 400MHz) δ ppm: 9,00(breit,1H), 8,49(breit,1H), 8,25(breit,1H), 8,17(breit,1H), 7,21(d,1H), 4,06(q,2H), 3,76(q,2H), 2,36(s,3H), 1,19(t,3H)

**Stufe 2:** *4-{2-Fluor-4-methyl-5-[(2,2,2-trifluorethyl)sulfanyl]phenyl}-1,2,4-triazolidin-3,5-dion*

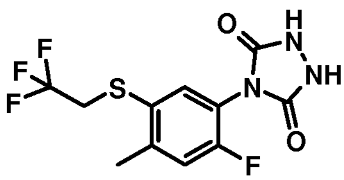

Eine Lösung von 500 mg (1,35 mmol) Ethyl-2-({2-fluor-4-methyl-5-[(2,2,2-trifluorethyl)sulfanyl]phenyl}carbamoyl)hydrazincarboxylat in Ethanol (8 mL) und 6N wässriger Kaliumhydroxidlösung (2 mL) wird 4 h zum Rückfluss erhitzt. Nach Abkühlen auf Raumtemperatur wird die Reaktionsmischung in kaltes Wasser gegossen. Der gebildete Feststoff wird filtriert, mit Wasser gewaschen und getrocknet. Man erhält 270 mg (90% Reinheit, 56% der Theorie) der Titelverbindung als beigen Feststoff.
logP[a]: 1,82; $^1$H-NMR (D$^6$-DMSO, 400MHz) δ ppm: 10,58(breit,2H), 7,70(d,1H), 7,39(d,1H), 3,96(q,2H), 2,43(s,3H)

**Stufe 3:** *1,2-Diethyl-4-{2-fluor-4-methyl-5-[(2,2,2-trifluorethyl)sulfanyl]phenyl}-1,2,4-triazolidin-3,5-dion (Bsp. Nr. 118)*

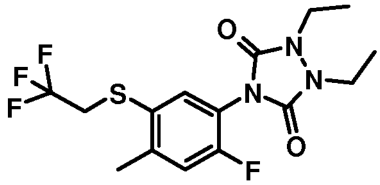

Zu einer Lösung von 500 mg (1,55 mmol) 4-{2-Fluor-4-methyl-5-[(2,2,2-trifluorethyl)sulfanyl]phenyl}-1,2,4-triazolidin-3,5-dion in *N,N*-Dimethylformamid (5 mL) werden bei 0 °C 322 mg (2,33 mmol) Kaliumcarbonat und 484 mg (3,10 mmol) Ethyliodid gegeben. Die Reaktionsmischung wird 6 h bei Raumtemperatur gerührt, dann in kaltes Wasser gegossen. Der gebildete Feststoff wird filtriert, mit Wasser gewaschen und getrocknet. Man erhält 170 mg (98% Reinheit, 28% der Theorie) der Titelverbindung als beigen Feststoff.
logP[a]: 3,03; $^1$H-NMR (D$^6$-DMSO, 400MHz) δ ppm: 7,72(d,1H), 7,40(d,1H), 3,96(q,2H), 3,61(q,4H), 2,42(s,3H), 1,10(t,6H)

**Herstellbeispiel 21: 1,2-Diethyl-4-{2-fluor-4-methyl-5-[(2,2,2-trifluorethyl)sulfinyl]phenyl}-1,2,4-triazolidin-3,5-dion (Bsp. Nr. 119)**

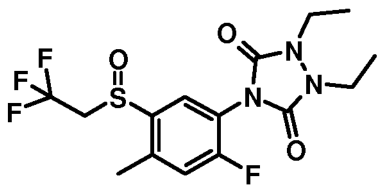

Zu einer Lösung von 120 mg (0,32 mmol) 1,2-Diethyl-4-{2-fluor-4-methyl-5-[(2,2,2-trifluorethyl)sulfanyl]phenyl}-1,2,4-triazolidin-3,5-dion in Aceton/Wasser (1:1) werden bei Raumtemperatur 98 mg (0,32 mmol) Oxon gegeben. Die Reaktionsmischung wird 2 h bei Raumtemperatur gerührt, dann unter vermindertem Druck konzentriert. Der Rückstand wird mit Wasser verdünnt und mit Essigester extrahiert. Die vereinten organische Phasen werden mit Wasser und wässriger gesättigter Natriumchloridlösung gewaschen, über Natriumsulfat getrocknet, filtriert und unter vermindertem Druck konzentriert. Nach säulenchromatographischer Reinigung an Kieselgel mit Petrolether/Essigester (75/25) als Laufmittel erhält man 50 mg (98% Reinheit, 39% der Theorie) der Titelverbindung als beigen Feststoff.
logP[a]: 2,07; $^1$H-NMR (D$^6$-DMSO, 400MHz) δ ppm: 7,99(d,1H), 7,55(d,1H), 4,38-4,18(m,1H), 4,15-3,92(m,1H), 3,80-3,58(m,4H), 2,44(s,3H), 1,13(t,6H)

**Herstellbeispiel 22: 1-Ethyl-4-{2-fluor-4-methyl-5-[(2,2,2-trifluorethyl)sulfanyl]phenyl}-2-methyl-1,2,4-triazolidin-3,5-dion (Bsp. Nr. 122)**

**Stufe 1:** *N-{2-Fluor-4-methyl-5-[(2,2,2-trifluorethyl)sulfanyl]phenyl}-1-methylhydrazincarboxamid*

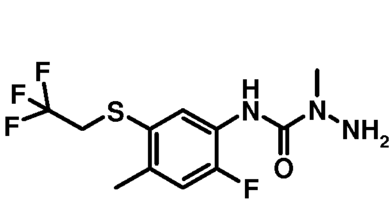

Zu einer Lösung von 2,5 g (8,0 mmol) Ethyl-{2-fluor-4-methyl-5-[(2,2,2-trifluorethyl)sulfanyl]phenyl}carbamat (siehe z. B. JP2011/042611) in Dichlormethan (25 mL) werden bei Raumtemperatur 2,1 g (16,1 mmol) Diisopropylethylamin und 407 mg (8,8 mmol) Methylhydrazin gegeben. Die Reaktionsmischung wird 6 h bei Raumtemperatur gerührt, dann mit Wasser verdünnt und mit Dichlormethan extrahiert. Die vereinten organische Phasen werden mit Wasser und

wässriger gesättigter Natriumchloridlösung gewaschen, über Natriumsulfat getrocknet, filtriert und unter vermindertem Druck konzentriert. Nach säulenchromatographischer Reinigung an Kieselgel mit Petrolether/Essigester (80/20) als Laufmittel erhält man 2,0 g (80% der Theorie) der Titelverbindung.

**Stufe 2:** *Ethyl-2-([2-fluor-4-methyl-5-[(2,2,2-trifluorethyl)sulfanyl]phenyl}carbamoyl)-2-methylhydrazincarboxylat*

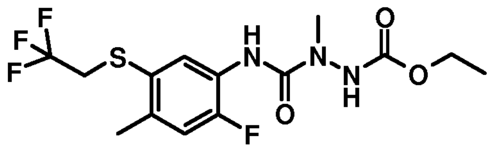

Zu einer Lösung von 2,0 g (6,4 mmol) N-{2-Fluor-4-methyl-5-[(2,2,2-trifluorethyl)sulfanyl]phenyl}-1-methylhydrazincarboxamid in Tetrahydrofuran (20 mL) werden bei Raumtemperatur 4,2 g (12,8 mmol) Caesiumcarbonat und 764 mg (7,0 mmol) Chlorameisensäureethylester gegeben. Die Reaktionsmischung wird 6 h bei Raumtemperatur gerührt, dann mit Wasser verdünnt und mit Essigester extrahiert. Die vereinten organische Phasen werden mit Wasser und wässriger gesättigter Natriumchloridlösung gewaschen, über Natriumsulfat getrocknet, filtriert und unter vermindertem Druck konzentriert. Nach säulenchromatographischer Reinigung an Kieselgel mit Petrolether/Essigester (90/10) als Laufmittel erhält man 2,0 g (81 % der Theorie) der Titelverbindung.

**Stufe 3:** *4-[2-Fluor-4-methyl-5-[(2,2,2-trifluorethyl)sulfanyl]phenyl]-1-methyl-1,2,4-triazolidin-3,5-dion*

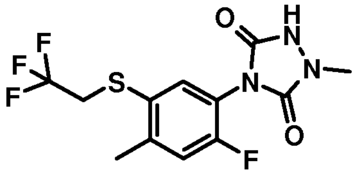

Eine Lösung von 2,0 g (5,2 mmol) Ethyl-2-({2-fluor-4-methyl-5-[(2,2,2-trifluorethyl)sulfanyl]phenyl}carbamoyl)-2-methylhydrazincarboxylat in Ethanol (16 mL) und 6N wässriger Kaliumhydroxidlösung (4 mL) wird 1 h zum Rückfluss erhitzt. Nach Abkühlen auf Raumtemperatur wird die Reaktionsmischung in kaltes Wasser gegossen. Der gebildete Feststoff wird filtriert, mit Wasser gewaschen und getrocknet. Man erhält 1,4 g (80% der Theorie) der Titelverbindung als beigen Feststoff.

logP[a]: 2,02; $^1$H-NMR (D$^6$-DMSO, 400MHz) $\delta$ ppm: 10,96(breit,1H), 7,70(d,1H), 7,40(d,1H), 3,95(q,2H), 3,12(s,3H), 2,43(s,3H)

**Stufe 4:** *1-Ethyl-4-[2-fluor-4-methyl-5-[(2,2,2-trifluorethyl)sulfanyl]phenyl}-2-methyl-1,2,4-triazolidin-3,5-dion(Bsp. Nr. 122)*

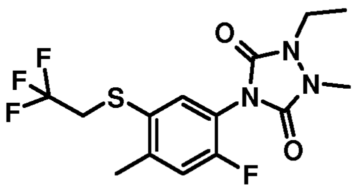

Zu einer Lösung von 300 mg (0,89 mmol) 4-{2-Fluor-4-methyl-5-[(2,2,2-trifluorethyl)sulfanyl]phenyl}-1-methyl-1,2,4-triazolidin-3,5-dion in N,N Dimethylformamid (5 mL) werden bei 0 °C 184 mg (1,33 mmol) Kaliumcarbonat und 207 mg (1,33 mmol) Ethyliodid gegeben. Die Reaktionsmischung wird 16 h bei Raumtemperatur gerührt, dann in kaltes Wasser gegossen. Der gebildete Feststoff wird filtriert, mit Wasser gewaschen und getrocknet. Man erhält 270 mg (98% Reinheit, 81% der Theorie) der Titelverbindung als beigen Feststoff.

logP[a]: 2,71; $^1$H-NMR ($D^6$-DMSO, 400MHz) δ ppm: 7,71(d,1H), 7,40(d,1H), 3,93(q,2H), 3,66(q,2H), 3,16(s,3H), 2,42(s,3H), 1,10(t,3H)

**Herstellbeispiel 23: 1-{2-Fluor-4-methyl-5-[(2,2,2-trifluorethyl)sulfanyl]phenyl}-2,4,4-trimethyl-pyrazolidin-3,5-dion (Bsp. Nr. 174)**

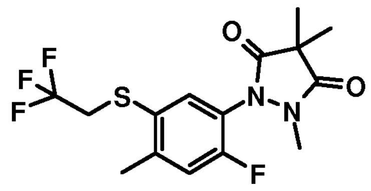

268,0 mg (1,0 mmol) {2-Fluor-4-methyl-5-[(2,2,2-trifluorethyl)sulfanyl]phenyl}boronsäure, 284 mg (2,00 mmol) 1,4,4-Trimethylpyrazolidin-3,5-dion, 272 mg (1,5 mmol) Kupfer(II)-acetat, 158 mg (2,0 mmol) Pyridin sowie 1,0 g aktiviertes 3Å-Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 109 mg (99% Reinheit, 30% der Theorie) der Titelverbindung als farbloses Öl.
logP[a]: 2,84; logP[b]: 2,79; $^1$H-NMR ($D^6$-DMSO, 400MHz) δ ppm: 7,76(d,1H), 7,45(d,1H), 4,04(q,2H), 2,93(s,3H), 2,43(s,3H), 1,30(s,6H)

**Herstellbeispiel 24: 1-{2-Fluor-4-methyl-5-[(2,2,2-trifluorethyl)sulfinyl]phenyl}-2,4,4-trimethyl-pyrazolidin-3,5-dion (Bsp. Nr. 177)**

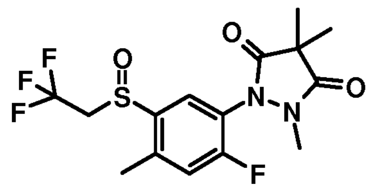

Zu einer Lösung von 55,0 mg (0,15 mmol) 1-{2-Fluor-4-methyl-5-[(2,2,2-trifluorethyl)sulfanyl]phenyl}-2,4,4-trimethylpyrazolidin-3,5-dion in 10 mL Dichlormethan werden bei 0 °C 35,5 mg (0,16 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 50 mg (97% Reinheit, 84% der Theorie) der Titelverbindung als farbloses Öl.
logP[a]: 1,89; logP[b]: 1,85; $^1$H-NMR ($D^6$-DMSO, 400MHz) δ ppm: 7,98(d,1H), 7,57(d,1H), 4,30-4,10(m,2H), 2,98(s,3H), 2,44(s,3H), 1,32(s,6H)

**Herstellbeispiel 25: 4-Cyclopropyl-2-{2-fluor-4-methyl-5-[(2,2,2-trifluorethyl)sulfanyl]phenyl}-1-methyl-5-thioxo-1,2,4-triazolidin-3-on (Bsp. Nr. 181) und 4-Cyclopropyl-1-{2-fluor-4-methyl-5-[(2,2,2-trifluorethyl)sulfanyl]phenyl}-2-methyl-1,2,4-triazolidin-3,5-dithion (Bsp. Nr. 182)**

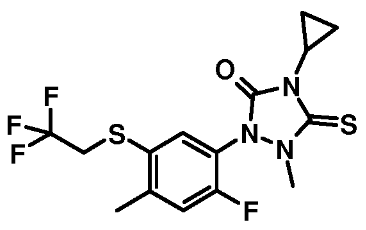
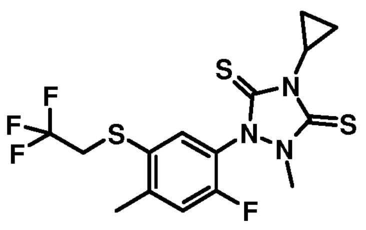
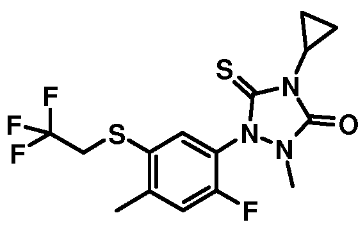

Zu einer Lösung von 223 mg (0,59 mmol) 4-Cyclopropyl-1-{2-fluor-4-methyl-5-[(2,2,2-trifluorethyl)sulfanyl]phenyl}-2-methyl-1,2,4-triazolidin-3,5-dion in 10 mL Toluol werden 239 mg (0,59 mmol) 2,4-Bis(4-methoxyphenyl)-1,3,2,4-dithiadiphosphetan-2,4-disulfid (Lawessons Reagenz) gegeben. Die Reaktionsmischung wird über Nacht unter Argon zum Rückfluss erhitzt, dann konzentriert. Nach mehrfacher säulenchromatographischer Reinigung mittels MPLC an Kieselgel mit Cyclohexan/Essigester als Laufmittel und an RP(C-18) mit Wasser/Acetonitril erhält man 13 mg (85% Reinheit, 5% der Theorie) 4-Cyclopropyl-2-{2-fluor-4-methyl-5-[(2,2,2-trifluorethyl)sulfanyl]phenyl}-1-methyl-5-thioxo-1,2,4-triazolidin-3-on, 26 mg (88% Reinheit, 9% der Thoerie) 4-Cyclopropyl-1-{2-fluor-4-methyl-5-[(2,2,2-trifluorethyl)sulfanyl]phenyl}-2-methyl-1,2,4-triazolidin-3,5-dithion sowie 7 mg 4-Cyclopropyl-1-{2-fluor-4-methyl-5-[(2,2,2-trifluorethyl)sulfanyl]phenyl}-2-methyl-5-thioxo-1,2,4-triazolidin-3-on jeweils als farblose Feststoffe.

4-Cyclopropyl-2-{2-fluor-4-methyl-5-[(2,2,2-trifluorethyl)sulfanyl]phenyl}-1-methyl-5-thioxo-1,2,4-triazolidin-3-on (Bsp. Nr. 181): logP[a]: 3,44; logP[b]: 3,39; $^1$H-NMR (D$^6$-DMSO, 400MHz) δ ppm: 7,85(d,1H), 7,50(d,1H), 4,03(q,2H), 3,31(s,3H), 2,92-2,87(m,1H), 2,44(s,3H), 1,04-0,97(m,4H)

4-Cyclopropyl-1-{2-fluor-4-methyl-5-[(2,2,2-trifluorethyl)sulfanyl]phenyl}-2-methyl-1,2,4-triazolidin-3,5-dithion (Bsp. Nr. 182): logP[a]: 3,83; logP[b]: 3,76; 1H-NMR (D$^6$-DMSO, 400MHz) δ ppm: 7,96(d,1H), 7,55(d,1H), 4,11-3,95(m,2H), 3,39(s,3H), 3,00-2,91(m,1H), 2,46(s,3H), 1,25-1,10(m,4H)

4-Cyclopropyl-1-{2-fluor-4-methyl-5-[(2,2,2-trifluorethyl)sulfanyl]phenyl}-2-methyl-5-thioxo-1,2,4-triazolidin-3-on: logP[a]: 3,24; logP[b]: 3,18; $^1$H-NMR (D$^6$-DMSO, 400MHz) δ ppm: 7,81(d,1H), 7,48(d,1H), 4,10-3,92(m,2H), 2,98(s,3H), 2,92-2,87(m,1H), 2,44(s,3H), 1,11-0,99(m,4H)

**Herstellbeispiel 26: 1-Cyclohexyl-3-{2-fluor-4-methyl-5-[(2,2,2-trifluorethyl)sulfanyl]-phenyl}-imidazolidin-2,4,5-trion (Bsp. Nr. 185)**

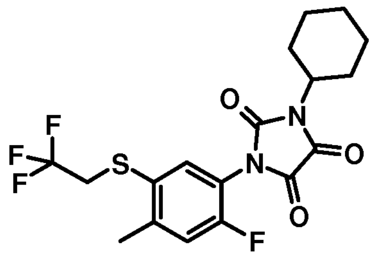

268,0 mg (1,0 mmol) {2-Fluor-4-methyl-5-[(2,2,2-trifluorethyl)sulfanyl]phenyl}boronsäure, 392 mg (2,00 mmol) 1-Cyclohexylimidazolidin-2,4,5-trion, 272 mg (1,5 mmol) Kupfer(II)-acetat, 158 mg (2,0 mmol) Pyridin sowie 0,5 g aktiviertes 3Å -Molsieb werden in 5 mL trockenem Dichlormethan 3 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 82 mg (96% Reinheit, 19% der Theorie) der Titelverbindung als farbloses Öl.
logP[a]: 4,32; logP[b]: 4,27; $^1$H-NMR (D$^6$-DMSO, 400MHz) δ ppm: 7,67(d,1H), 7,47(d,1H), 4,00-3,90(m,1H), 3,84(q,2H), 2,47(s,3H), 2,00-1,89(m,2H), 1,86-1,75(m,4H), 1,67-1,58(m,1H), 1,39-1,25(m,2H), 1,20-1,09(m,1H)

**Herstellbeispiel 27: 1-Cyclohexyl-3-{2-fluor-4-methyl-5-[(2,2,2-trifluorethyl)sulfinyl]-phenyl}-imidazolidin-2,4,5-trion (Bsp. Nr. 186)**

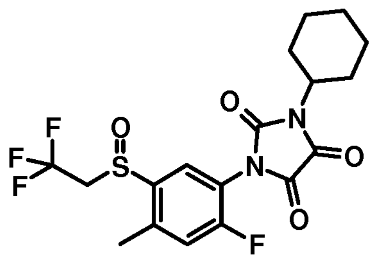

Zu einer Lösung von 41,0 mg (0,10 mmol) 1-Cyclohexyl-3-{2-fluor-4-methyl-5-[(2,2,2-trifluorethyl)sulfanyl]phenyl}imidazolidin-2,4,5-trion in 10 mL Dichlormethan werden bei 0 °C 23,1 mg (0,10 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 29 mg (92% Reinheit, 63% der Theorie) der Titelverbindung als farbloses Öl.

logP[a]: 3,36; logP[b]: 3,33; $^1$H-NMR (D$^6$-DMSO, 400MHz) δ ppm: 8,03(d,1H), 7,57(d,1H), 4,35-4,26(m,1H), 3,99-3,83(m,2H), 2,43(s,3H), 2,00-1,90(m,2H), 1,86-1,75(m,3H), 1,69-1,60(m,1H), 1,39-1,22(m,2H), 1,22-1,09(m,2H)

**Herstellbeispiel 28: 1-[2-Fluor-4-methyl-5-(2,2,2-trifluoroethylsulfinyl)phenyl]-3,5,5-trimethyl-imidazolidin-2,4-dion (Bsp. Nr. 216)**

**Stufe 1:** *Ethyl 2-[2-Fluor-4-methyl-5-(2,2,2-trifluoroethylsulfanyl)anilino]-2-methyl-propanoat*

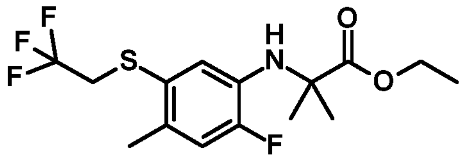

Zu einer Lösung von 5,00 g (20,8 mmol) 2-Fluor-4-methyl-5-(2,2,2-trifluoroethylsulfanyl)anilin in *N,N*-Dimethylacetamid (90 mL) wurden bei Raumtemperatur 4,47 g (41,8 mmol) 2,6-Lutidin und 4,60 g (25,1 mmol) 2-Bromisobuttersäureethylester gegeben. Die Reaktionsmischung wurde 6 h bei 90 °C gerührt. Nach Abkühlen wurde das Reaktionsgemisch mit Wasser verdünnt und mit Essigester extrahiert. Die vereinten organischen Phasen wurden mit gesättigter Natriumchlorid-Lösung gewaschen, über Natriumsulfat getrocknet, filtriert und unter Vakuum vom Lösungsmittel befreit. Durch säulenchromatographische Reinigung (20% EtOAc/Petrolether) des Rückstands wurden 1,50 g (20% der Theorie) der Titelverbindung erhalten.

**Stufe 2:** *4-Nitrophenyl-N-methylcarbamat*

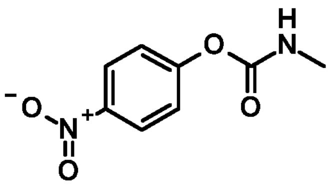

Zu einer Lösung von 1,00 g (4.97 mmol) Chlorameisensäure-4-nitrophenylester in 50 ml Tetrahydrofuran bei 0 °C wurden 3,00 ml (5,97 mmol) Methylamin. Das Reaktionsgemisch wurde auf Raumtemperatur gehen lassen und 4 h weiter gerührt. Anschließend wurde es eingeengt und der Rückstand wurde mit Wasser verdünnt und mit Essigester extrahiert. Die vereinten organischen Phasen wurden mit gesättigter Natriumchlorid-Lösung gewaschen, über Natriumsulfat getrocknet, filtriert und unter Vakuum vom Lösungsmittel befreit. Das Rohprodukt wurde durch Waschungen mit Diethylether aufgereinigt. So wurden 600 mg (61 % der Theorie) der Titelverbindung erhalten.

**Stufe 3:** *Ethyl 2-[2-Fluor-4-methyl-N-(methylcarbamoyl)-5-(2,2,2-trifluoroethylsulfanyl)anilino]-2-methyl-propanoat*

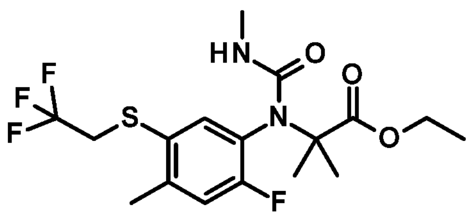

Zu einer Lösung von 1,00 g (2,83 mmol) Ethyl 2-[2-Fluor-4-methyl-5-(2,2,2-trifluoroethylsulfanyl)anilino]-2-methyl-propanoat in 40 ml Methylenchlorid bei Raumtemperatur wurden 665 mg (3,11 mmol) 4-Nitrophenyl-*N*-methylcarbamat und 379 mg (3,11 mmol) *N,N*-Dimethylaminopyridin gegeben. Das Reaktionsgemisch bei Raumtemperatur 36 h gerührt. Anschließend wurde das Reaktionsgemisch mit Wasser verdünnt und mit Methylenchlorid extrahiert. Die vereinten organischen Phasen wurden mit gesättigter Natriumchlorid-Lösung gewaschen, über Natriumsulfat getrocknet, filtriert und unter Vakuum vom Lösungsmittel befreit. 850 mg vom Rohprodukt wurden isoliert und weiter umgesetzt.

**Stufe 4:** *1-[2-Fluor-4-methyl-5-(2,2,2-trifluoroethylsulfanyl)phenyl]-3,5,5-trimethyl-imidazolidin-2,4-dion (Bsp. Nr. 217)*

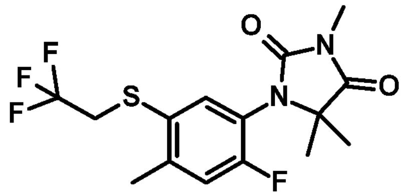

Zu einer Lösung von 650 mg (1,78 mmol) Ethyl 2-[2-Fluor-4-methyl-*N*-(methylcarbamoyl)-5-(2,2,2-trifluoroethylsulfanyl)anilino]-2-methyl-propanoat in 25 ml Essigester wurde 1 g neutrales Alox bei Raumtemperatur gegeben und 16 h gerührt. Anschließend wurde das Reaktionsgemisch filtriert und das Filtrat wurde eingeengt. Durch säulenchromatographische Reinigung (25% EtOAc/Petrolether) des Rückstands wurden 150 mg (26% der Theorie) der Titelverbindung erhalten.
logP(HCOOH): 3,11; $^1$H-NMR (CDCl3, 400MHz) δ ppm 7,41(d,1H), 7,14(d,1H), 3,32(q,2H), 3,12(s,3H), 1,41(s,3H), 1,40(s,3H)

**Stufe 5:** *1-[2-Fluor-4-methyl-5-(2,2,2-trifluoroethylsulfinyl)phenyl]-3,5,5-trimethyl-imidazolidin-2,4-dion (Bsp. Nr. 216)*

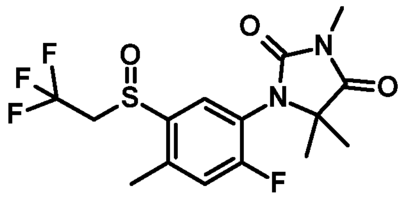

Zu einer Lösung aus 90,0 mg (0,21 mmol) 1-[2-Fluor-4-methyl-5-(2,2,2-trifluoroethylsulfanyl)phenyl]-3,5,5-trimethyl-imidazolidin-2,4-dion in 8 ml Aceton / Wasser (1:1) bei Raumtemperatur wurden 135 mg (0,21 mmol) Oxon gegeben. Die Lösung wurde 4 h bei Raumtemperatur gerührt und anschließend wurde das Lösungsmittel unter Vakuum entfernt. Der Rückstand wurde mit Wasser versetzt und mit Ethylacetat extrahiert. Die vereinigten organischen Phasen werden mit Wasser und gesättigter Natriumchlorid-Lösung gewaschen und danach über Natriumsulfat getrocknet. Das Lösungsmittel wird am Rotationsverdampfer entfernt. Durch säulenchromatographische Reinigung (40% EtOAc/Petrolether) des Rohprodukts wurden 55 mg (59% der Theorie) der Titelverbindung erhalten.
logP(HCOOH): 2,05; $^1$H-NMR (CDCl3, 400MHz) δ ppm 7,86(d,1H), 7,17(d,1H), 3,45(q,2H), 3,14(s,3H), 2,44(s,3H), 1,47(s,3H), 1,41(s,3H)

Gemäß den zuvor beschriebenen Verfahren wurden die folgenden Verbindungen der allgemeinen Formel (I) hergestellt, wobei Verbindungen, in denen der Rest X und/oder der Rest Y und/oder der Rest Z Cyano ist, nicht erfindungsgegenständlich sind:

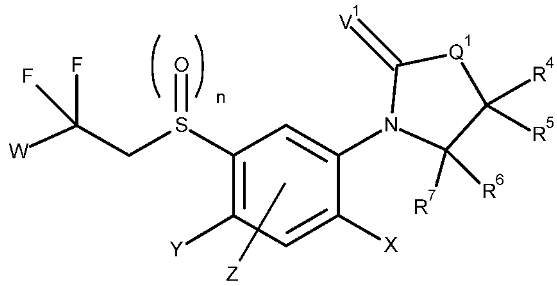

(I) mit (I-A)

(Verbindung der allgemeinen Formel (I) mit (I-A) mit Z = H)

| Beipsiel-Nr. | W | n | Y | X | $V^1$ | $Q^1$ | $R^4$ | $R^5$ | $R^6$ | $R^7$ |
|---|---|---|---|---|---|---|---|---|---|---|
| **1** | F | 0 | $CH_3$ | $CH_3$ | O | NH | H | H | H | H |
| **2** | F | 0 | $CH_3$ | $CH_3$ | $(NCH(CH_3)CF_3)HCl$ | $CH_2$ | H | H | H | H |
| **3** | F | 1 | $CH_3$ | F | $NCH_2CF_3$ | $CH_2$ | H | H | H | H |
| **4** | F | 1 | $CH_3$ | $CH_3$ | O | NH | H | H | H | H |
| **5** | F | 0 | $CH_3$ | $CH_3$ | $(NCH_2CF_3)HCl$ | $CH_2$ | H | H | H | H |
| **6** | F | 0 | $CH_3$ | F | $NCH_2CF_3$ | $CH_2$ | H | H | H | H |
| **7** | F | 1 | $CH_3$ | F | O | NH | H | H | H | H |
| **8** | F | 0 | $CH_3$ | F | $NCH(CH_3)CF_3$ | $CH_2$ | H | H | H | H |
| **9** | F | 0 | $CH_3$ | F | O | NH | H | H | H | H |
| **10** | F | 1 | $CH_3$ | F | $NCH(CH_3)CF_3$ | $CH_2$ | H | H | H | H |
| **104** | F | 0 | $CH_3$ | F | O | O | H | H | H | H |
| **105** | F | 1 | $CH_3$ | F | O | O | H | H | H | H |
| **106** | F | 0 | $CH_3$ | F | O | O | $CH_3$ | H | H | H |
| **107** | F | 1 | $CH_3$ | F | O | O | H | H | $CH_3$ | H |
| **108** | F | 0 | $CH_3$ | F | O | O | H | H | $CH_3$ | H |
| **109** | F | 1 | $CH_3$ | F | O | O | $CH_3$ | H | H | H |
| **198** | F | 0 | $CH_3$ | F | O | $NCH_3$ | H | H | H | H |
| **199** | F | 1 | $CH_3$ | F | O | $NCH_3$ | H | H | H | H |

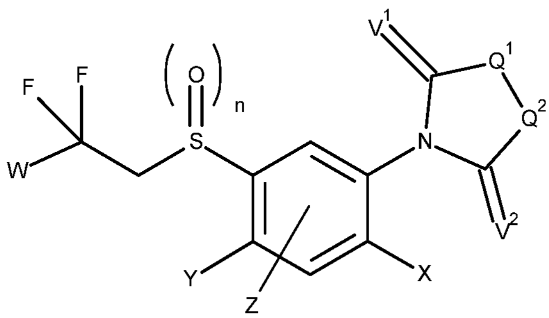

(I) mit (I-B)

(Verbindung der allgemeinen Formel (I) mit (I-B) mit Z = H)

| Beispiel-Nr. | W | n | Y | X | $V^2$ | $Q^2$ | $V^1$ | $Q^1$ |
|---|---|---|---|---|---|---|---|---|
| **11** | F | 0 | $CH_3$ | F | O | $CH_2$ | $NCH_2CF_3$ | s |
| **12** | F | 1 | $CH_3$ | F | O | $CH_2$ | N -Cyclopropyl | S |
| **13** | F | 1 | $CH_3$ | F | O | $CH_2$ | $NCH_2CF_3$ | s |
| **14** | F | 0 | $CH_3$ | $CH_3$ | O | $CH_2$ | N -Cyclopropyl | S |
| **15** | F | 1 | $CH_3$ | $CH_3$ | O | $CH_2$ | N -Cyclopropyl | S |
| **16** | F | 1 | $CH_3$ | H | O | $NCH_3$ | O | $C(CH_3)_2$ |
| **17** | F | 1 | $CH_3$ | F | O | $NCH_3$ | O | $C(CH_3)_2$ |
| **18** | F | 0 | $CH_3$ | F | O | $NCH_3$ | O | $C(CH_3)_2$ |
| **19** | F | 0 | $CH_3$ | F | O | $CH_2$ | O | $CH_2$ |
| **110** | F | 1 | $CH_3$ | F | O | $C(CH_3)_2$ | O | O |
| **111** | F | 0 | $CH_3$ | F | O | $C(CH_3)_2$ | O | O |
| **112** | F | 0 | $CH_3$ | $CH_3$ | O | $CH_2$ | N -Cyclopropyl | S |
| **113** | F | 0 | $CH_3$ | F | O | $CH_2$ | $N-CH_2C(CH_3)_3$ | s |
| **114** | F | 1 | $CH_3$ | F | O | $CH_2$ | $N-CH_2C(CH_3)_3$ | s |
| **115** | F | 0 | $CH_3$ | F | O | $CH_2$ | $N-CH_2CH(CH_3)_2$ | s |
| **116** | F | 0 | $CH_3$ | F | O | $NCH_3$ | O | $NCH_3$ |
| **117** | F | 1 | $CH_3$ | F | O | $NCH_3$ | O | $NCH_3$ |
| **118** | F | 0 | $CH_3$ | F | O | $NCH_2CH_3$ | O | $NCH_2CH_3$ |
| **119** | F | 1 | $CH_3$ | F | O | $NCH_2CH_3$ | O | $NCH_2CH_3$ |
| **120** | F | 1 | $CH_3$ | F | O | $NCH_2CH=CH_2$ | O | $NCH_2CH=CH_2$ |
| **121** | F | 0 | $CH_3$ | F | O | $NCH_2CCH$ | O | $NCH_3$ |
| **122** | F | 0 | $CH_3$ | F | O | $NCH_2CH_3$ | O | $NCH_3$ |
| **123** | F | 1 | $CH_3$ | F | O | $NCH_2CCH$ | O | $NCH_3$ |
| **124** | F | 1 | $CH_3$ | F | O | $NCH_2CH=CH_2$ | O | $NCH_3$ |
| **125** | F | 0 | $CH_3$ | F | O | $NCH_2CH=CH_2$ | O | $NCH_3$ |
| **126** | F | 0 | $CH_3$ | F | O | $NCH_2$-Cyclopropyl | O | $NCH_3$ |
| **200** | F | 0 | $CH_3$ | F | O | $CH_2$ | O | $NCH_3$ |
| **201** | F | 1 | $CH_3$ | F | O | $CH_2$ | O | $NCH_2CH=CH_2$ |
| **202** | F | 0 | $CH_3$ | F | O | $CH_2$ | O | N-nButyl |
| **203** | F | 1 | $CH_3$ | F | O | $CH_2$ | O | N-nButyl |
| **204** | F | 0 | $CH_3$ | F | O | $CH_2$ | O | N-Benzyl |
| **205** | F | 1 | $CH_3$ | F | O | $CH_2$ | O | N-Benzyl |
| **206** | F | 1 | $CH_3$ | F | O | $CH(CH_3)$ | O | O |

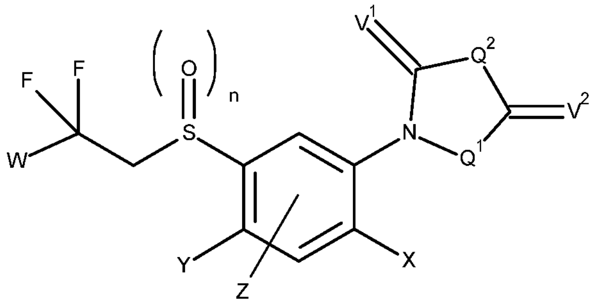

(I) mit (I-C)

(Verbindung der allgemeinen Formel (I) mit (I-C) mit Z = H, wobei Verbindungen, in denen $Q^1$ und/oder $Q^2$ NH ist, ebenfalls nicht erfindungsgegenständlich sind)

| Beipsiel-Nr. | W | n | Y | X | $V^1$ | $Q^1$ | $V^2$ | $Q^2$ |
|---|---|---|---|---|---|---|---|---|
| **20** | F | 0 | $CH_3$ | H | O | $NCH_3$ | O | $NCH_3$ |
| **21** | F | 1 | $CH_3$ | H | O | $NCH_3$ | O | $NCH_3$ |
| **22** | F | 0 | $CH_3$ | F | O | $NCH_3$ | O | $NCH_3$ |
| **23** | F | 1 | $CH_3$ | F | O | $NCH_3$ | O | $NCH_3$ |
| **24** | F | 1 | Cl | H | O | $NCH_3$ | O | $NCH_3$ |
| **25** | F | 0 | $CF_3$ | H | O | $NCH_3$ | O | $NCH_3$ |
| **26** | F | 1 | $CF_3$ | H | O | $NCH_3$ | O | $NCH_3$ |
| **27** | F | 1 | CN | H | O | $NCH_3$ | O | $NCH_3$ |
| **28** | F | 0 | CN | H | O | $NCH_3$ | O | $NCH_3$ |
| **29** | F | 1 | Cl | F | O | $NCH_3$ | O | $NCH_3$ |
| **30** | F | 0 | Cl | F | O | $NCH_3$ | O | $NCH_3$ |
| **31** | F | 0 | Cl | H | O | $NCH_3$ | O | $NCH_3$ |
| **32** | F | 1 | $OCH_3$ | H | O | $NCH_3$ | O | $NCH_3$ |
| **33** | F | 1 | $CH_3$ | $CH_3$ | O | $NCH_3$ | O | $NCH_3$ |
| **34** | F | 1 | $CH_3$ | F | O | $NCH_3$ | O | N-Cyclopropyl |
| **35** | F | 0 | $CH_3$ | F | O | $NCH_3$ | O | N-Cyclopropyl |
| **36** | F | 0 | $CH_3$ | H | O | $NCH_3$ | O | N-Cyclopropyl |
| **37** | F | 1 | $CH_3$ | H | O | $NCH_3$ | O | N-Cyclopropyl |
| **38** | F | 1 | Cl | F | O | $NCH_3$ | O | N-Cyclopropyl |
| **39** | F | 1 | Cl | H | O | $NCH_3$ | O | N-Cyclopropyl |
| **40** | F | 0 | Cl | H | O | $NCH_3$ | O | N-Cyclopropyl |
| **41** | F | 1 | $OCH_3$ | H | O | $NCH_3$ | O | N-Cyclopropyl |
| **42** | F | 0 | $OCH_3$ | H | O | $NCH_3$ | O | N-Cyclopropyl |
| **43** | F | 1 | CN | H | O | $NCH_3$ | O | N-Cyclopropyl |
| **44** | F | 0 | CN | H | O | $NCH_3$ | O | N-Cyclopropyl |
| **45** | F | 1 | Br | H | O | $NCH_3$ | O | N-Cyclopropyl |
| **46** | F | 0 | Br | H | O | $NCH_3$ | O | N-Cyclopropyl |

(fortgesetzt)

| Beipsiel-Nr. | W | n | Y | X | V1 | Q1 | V2 | Q2 |
|---|---|---|---|---|---|---|---|---|
| **47** | F | 1 | $CH_3$ | $CH_3$ | O | $NCH_3$ | O | N-Cyclopropyl |
| **48** | F | 0 | $CH_3$ | H | O | $NCH_2CH=CH_2$ | O | N-Cyclopropyl |
| **49** | F | 1 | $CH_3$ | H | O | $NCH_2CH=CH_2$ | O | N-Cyclopropyl |
| **50** | F | 1 | $CH_3$ | H | O | $NCH_2CH(CH_3)_2$ | O | N-Cyclopropyl |
| **51** | F | 0 | $CH_3$ | H | O | $NCH_2CH_3$ | O | $NCH_3$ |
| **52** | F | 0 | $CH_3$ | H | O | $NCH_2$-Cyclopropyl | O | $NCH_3$ |
| **53** | F | 0 | $CF_3$ | H | O | $NCH_3$ | O | N-Cyclopropyl |
| **54** | F | 1 | $CF_3$ | H | O | $NCH_3$ | O | N-Cyclopropyl |
| **55** | F | 0 | $CH_3$ | F | O | NH | O | $NCH_3$ |
| **56** | F | 0 | $CH_3$ | H | O | N-(3-(2,2,2-trifluoroethylsulfan yl)-4-methyl-phenyl) | O | $NCH_3$ |
| **57** | F | 0 | $CH_3$ | H | O | N-Benzyl | O | $NCH_3$ |
| **58** | F | 0 | $CH_3$ | H | O | $NCH_2CH=CH_2$ | O | $NCH_3$ |
| **59** | F | 1 | $CH_3$ | H | O | $NCH_2CH=CH_2$ | O | $NCH_3$ |
| **60** | F | 0 | $CH_3$ | H | O | N-((2-chlorophenyl) methyl) | O | $NCH_3$ |
| **61** | F | 0 | $CH_3$ | H | O | $NCH(CH_3)_2$ | O | $NCH_3$ |
| **62** | F | 0 | $CH_3$ | F | O | $NCH(CH_3)_2$ | O | $NCH_3$ |
| **63** | F | 0 | $CH_3$ | H | O | $NCH(CH_3)_2$ | O | N-Cyclopropyl |
| **64** | F | 1 | $CH_3$ | F | O | $NCH(CH_3)_2$ | O | N-Cyclopropyl |
| **65** | F | 0 | $CH_3$ | F | O | $NCH(CH_3)_2$ | O | N-Cyclopropyl |
| **66** | F | 0 | $CH_3$ | F | O | NH | O | N-Cyclopropyl |
| **67** | F | 1 | Br | H | O | $NCH_3$ | O | $NCH_3$ |
| **68** | F | 0 | Br | H | O | $NCH_3$ | O | $NCH_3$ |
| **69** | F | 1 | Br | F | O | $NCH_3$ | O | $NCH_3$ |
| **70** | F | 1 | F | F | O | $NCH_3$ | O | $NCH_3$ |
| **71** | F | 0 | F | F | O | $NCH_3$ | O | N-Cyclopropyl |
| **72** | F | 1 | Br | F | O | $NCH_3$ | O | N-Cyclopropyl |
| **73** | F | 0 | $CH_3$ | F | O | $NCH(CH_3)CH_2CH_3$ | O | N-Cyclopropyl |
| **74** | F | 1 | $CH_3$ | F | O | $NCH_3$ | O | $NCH_2CH_3$ |
| **75** | F | 1 | $CH_3$ | F | O | $NCH(CH_3)CH_2CH_3$ | O | N-Cyclopropyl |
| **76** | F | 0 | $CH_3$ | F | O | $NCH_3$ | O | N-((3-(trifluoromethyl)ph enyl) |
| **77** | F | 1 | $CH_3$ | F | O | $NCH_3$ | O | N-((3-(trifluoromethyl)ph enyl) |
| **78** | F | 0 | $CH_3$ | F | O | N-(4-cyano-2,5-difluoro-phenyl) | O | $NCH_3$ |
| **79** | F | 0 | $CH_3$ | H | O | N-(3-(2,2,2-trifluoroethylsulfan yl)-4-methylphenyl) | O | $NCH_2CH=CH_2$ |

(fortgesetzt)

| Beipsiel-Nr. | W | n | Y | X | $V^1$ | $Q^1$ | $V^2$ | $Q^2$ |
|---|---|---|---|---|---|---|---|---|
| **80** | F | 1 | $CH_3$ | F | O | $NCH_3$ | O | $NCH_2CH=CH_2$ |
| **81** | F | 0 | $CH_3$ | F | O | $NCH_3$ | O | $NCH_2CH=CH_2$ |
| **82** | F | 1 | $CH_3$ | H | O | N-(3-(2,2,2-trifluoroethylsulfin yl)-4-methylphenyl) | O | $NCH_3$ |
| **83** | F | 0 | $CH_3$ | F | O | $NCH_3$ | O | $NCH_2CH_3$ |
| **84** | F | 1 | $CH_3$ | H | O | N-(3-(2,2,2-trifluoroethylsulfan yl)-4-methylphenyl) | O | $NCH_3$ |
| **85** | F | 0 | $CH_3$ | F | O | NH | O | $NCH_2CH_3$ |
| **86** | F | 0 | $CH_3$ | F | O | N-Cyclopentyl | O | $NCH_3$ |
| **87** | F | 0 | $CH_3$ | F | O | $NCH_2CH_3$ | O | N-Phenyl |
| **88** | F | 1 | $CH_3$ | F | O | $NCH_2CH_3$ | O | N-Phenyl |
| **89** | F | 1 | $CH_3$ | H | O | $NCH_3$ | O | $NCH_2CH_3$ |
| **90** | F | 0 | $CH_3$ | H | O | N-Benzyl | O | N-Cyclopropyl |
| **91** | F | 0 | $CH_3$ | F | O | $NCH_3$ | O | $NCH(CH_3)_2$ |
| **92** | F | 1 | $CH_3$ | F | O | $NCH_3$ | O | $NCH(CH_3)_2$ |
| **93** | F | 1 | $CH_3$ | F | O | $NCH_2CH_3$ | O | $NCH(CH_3)_2$ |
| **94** | F | O | $CH_3$ | F | O | $NCH_3$ | O | $NC(CH_3)_3$ |
| **95** | F | 1 | $CH_3$ | F | O | $NCH_3$ | O | $NC(CH_3)_3$ |
| **96** | F | 1 | $CH_3$ | F | O | N-(Cyclohex-2-en-1-yl) | O | $NCH_3$ |
| **97** | F | 0 | $CH_3$ | F | O | $NCH_2CH_3$ | O | $NCH_3$ |
| **98** | F | 1 | $CH_3$ | F | O | $NCH_2CH_3$ | O | $NCH_3$ |
| **99** | F | 0 | $CH_3$ | F | O | $NCH_2CH_3$ | O | $NC(CH_3)_3$ |
| **100** | F | 0 | $CH_3$ | H | O | $NCH_2CH_3$ | O | $NCH_2CH_3$ |
| **127** | F | 0 | $CH_3$ | F | O | $NCH_2CH_3$ | O | N-Cyclopropyl |
| **128** | F | 0 | $CH_3$ | F | O | $NCH_3$ | O | N-Cyclopropyl |
| **129** | F | 0 | $CH_3$ | F | O | $NCH_2CF_3$ | O | N-Cyclopropyl |
| **130** | F | 0 | $CH_3$ | F | O | $NCH_2CH=CH_2$ | O | N-Cyclopropyl |
| **131** | F | 0 | $CH_3$ | H | O | $NCH_3$ | O | $NCH_2CH_3$ |
| **132** | F | 0 | $CH_3$ | H | O | $NCH_3$ | O | $NCH(CH_3)_2$ |
| **133** | F | 0 | $CH_3$ | H | O | $NCH_2CH_3$ | O | $NCH(CH_3)_2$ |
| **134** | F | 0 | $CH_3$ | H | O | $NCH_2CH_3$ | O | $NC(CH_3)_3$ |
| **135** | F | 0 | $CH_3$ | H | O | $NCH(CH_3)_2$ | O | $NC(CH_3)_3$ |
| **136** | F | 0 | $CH_3$ | F | O | $NCH_3$ | O | N-Cyclohexyl |
| **137** | F | 1 | $CH_3$ | F | O | $NCH_2CH_3$ | O | $NC(CH_3)_3$ |
| **138** | F | 1 | $CH_3$ | H | O | $NCH_2CH_3$ | O | $NCH_2CH_3$ |
| **139** | F | 1 | $CH_3$ | H | O | $NCH_3$ | O | $NC(CH_3)_3$ |

(fortgesetzt)

| Beispiel-Nr. | W | n | Y | X | $V^1$ | $Q^1$ | $V^2$ | $Q^2$ |
|---|---|---|---|---|---|---|---|---|
| **140** | F | 1 | $CH_3$ | H | O | $NCH(CH_3)_2$ | O | $NCH_2CH_3$ |
| **141** | F | 1 | $CH_3$ | H | O | $NCH_3$ | O | $NCH(CH_3)_2$ |
| **142** | F | 0 | $CH_3$ | F | O | $NC(CH_3)_3$ | O | $NCH_2CH_3$ |
| **143** | F | 0 | $CH_3$ | F | O | $NC(CH_3)_3$ | O | $NCH(CH_3)_2$ |
| **144** | F | 0 | $CH_3$ | F | O | $NC(CH_3)_3$ | O | N-Cyclopropyl |
| **145** | F | 0 | $CH_3$ | F | O | $NCH_2CF_3$ | O | $NCH_2CH_3$ |
| **146** | F | 0 | $CH_3$ | F | O | $NCH_2CF_3$ | O | $NCH(CH_3)_2$ |
| **147** | F | 0 | $CH_3$ | F | O | $NCH_2CF_3$ | O | $NC(CH_3)_3$ |
| **148** | F | 0 | $CH_3$ | F | O | $NCH_2CH(CH_3)_2$ | O | $NCH_2CH_3$ |
| **149** | F | 0 | $CH_3$ | F | O | $NCH_2CH(CH_3)_2$ | O | $NCH(CH_3)_2$ |
| **151** | F | 1 | $CH_3$ | F | O | $NCH_3$ | O | N-Cyclopropyl |
| **152** | F | 1 | $CH_3$ | F | O | $NC(CH_3)_3$ | O | $NCH(CH_3)_2$ |
| **153** | F | 1 | $CH_3$ | F | O | $NCH_2CF_3$ | O | $NCH(CH_3)_2$ |
| **154** | F | 0 | $CH_3$ | F | O | $NCH_2CF_3$ | O | $NCH_2CH=CH_2$ |
| **155** | F | 0 | $CH_3$ | F | O | $NCH_2CF_3$ | O | N-Phenyl |
| **156** | F | 0 | $CH_3$ | F | O | $NCH_2CF_3$ | O | $NCH_2CHF_2$ |
| **157** | F | 0 | $CH_3$ | F | O | NH | O | N-(3-Chlorphenyl) |
| **158** | F | 1 | $CH_3$ | F | O | NH | O | N-(3-Trifluoromethylphe nyl) |
| **159** | F | 0 | $CH_3$ | F | O | $NCH_3$ | O | $N(CH_2)_3OCH_3$ |
| **160** | F | 0 | $CH_3$ | F | O | $NCH_3$ | O | $NCH_2CH_2CH_3$ |
| **161** | F | 0 | $CH_3$ | F | O | $NCH_3$ | O | $NCH_2CH(CH_2)_2$ |
| **162** | F | 0 | $CH_3$ | F | O | $NCH_3$ | O | N-Phenyl |
| **163** | F | 1 | $CH_3$ | F | O | $NCH_3$ | O | N-Phenyl |
| **164** | F | 1 | $CH_3$ | F | O | $NCH_2CF_3$ | O | N-Cyclopropyl |
| **165** | F | 1 | $CH_3$ | F | O | $NCH_2CH=CH_2$ | O | N-Cyclopropyl |
| **166** | F | 1 | $CH_3$ | F | O | $NCH_2CF_3$ | O | $NCH_2CH=CH_2$ |
| **167** | F | 1 | $CH_3$ | F | O | $NCH_2CF_3$ | O | N-Phenyl |
| **168** | F | 1 | $CH_3$ | F | O | $NCH_2CF_3$ | O | $NCH_2CHF_2$ |
| **169** | F | 1 | $CH_3$ | F | O | $NCH_3$ | O | $NCH_2CH(CH_2)_2$ |
| **170** | F | 1 | $CH_3$ | F | O | $NCH_3$ | O | $N(CH_2)_3OCH_3$ |
| **171** | F | 1 | $CH_3$ | F | O | $NCH_2CH_3$ | O | N-Cyclopropyl |
| **172** | F | 1 | $CH_3$ | F | O | $NCH_2CH_2CH_3$ | O | N-Cyclopropyl |
| **173** | F | 0 | $CH_3$ | F | O | N-Phenyl | O | $C(CH_3)_2$ |
| **174** | F | 0 | $CH_3$ | F | O | $NCH_3$ | O | $C(CH_3)_2$ |
| **175** | F | 0 | $CH_3$ | F | O | $NCH_3$ | O | $C(CH_2CH_3)_2$ |
| **176** | F | 1 | $CH_3$ | F | O | $NCH_3$ | O | $C(CH_2CH_3)_2$ |
| **177** | F | 1 | $CH_3$ | F | O | $NCH_3$ | O | $C(CH_3)_2$ |

(fortgesetzt)

| Beipsiel-Nr. | W | n | Y | X | V¹ | Q¹ | V² | Q² |
|---|---|---|---|---|---|---|---|---|
| **178** | F | 0 | $CH_3$ | F | O | $NCH_2$Phenyl | O | $C(CH_3)_2$ |
| **179** | F | 0 | $CH_3$ | F | O | $NCH_3$ | O | $C(CH_2)_3$ |
| **180** | F | 1 | $CH_3$ | F | O | $NCH_3$ | O | $C(CH_2)_3$ |
| **181** | F | 0 | $CH_3$ | F | O | $NCH_3$ | s | N-Cyclopropyl |
| **182** | F | 0 | $CH_3$ | F | s | $NCH_3$ | s | N-Cyclopropyl |
| **197** | F | 1 | $CH_3$ | F | O | $NCH_3$ | O | N-Cyclopropyl |
| **207** | F | 0 | $CH_3$ | F | O | NH | O | N-Cyclopropyl |
| **208** | F | 1 | $CH_3$ | F | O | $CH_2$ | O | $N-CH_3$ |
| **209** | F | 1 | $CH_3$ | F | O | $CH_2$ | O | N-Cyclopropyl |
| **210** | F | 0 | $CH_3$ | F | O | $CH_2$ | O | N-Cyclopropyl |
| **211** | F | 0 | $CH_3$ | F | O | $CH_2$ | O | $N-CH_3$ |
| **212** | F | 0 | $CH_3$ | F | O | $CH(CH_3)$ | O | $N-CH_3$ |
| **213** | F | 1 | $CH_3$ | F | O | $CH(CH_3)$ | O | $N-CH_3$ |
| **214** | F | 0 | $CH_3$ | F | O | $CH(CH_3)$ | O | N-Cyclopropyl |
| **215** | F | 1 | $CH_3$ | F | O | $CH(CH_3)$ | O | N-Cyclopropyl |
| **216** | F | 1 | $CH_3$ | F | O | $C(CH_3)_2$ | O | $N-CH_3$ |
| **217** | F | 0 | $CH_3$ | F | O | $C(CH_3)_2$ | O | $N-CH_3$ |

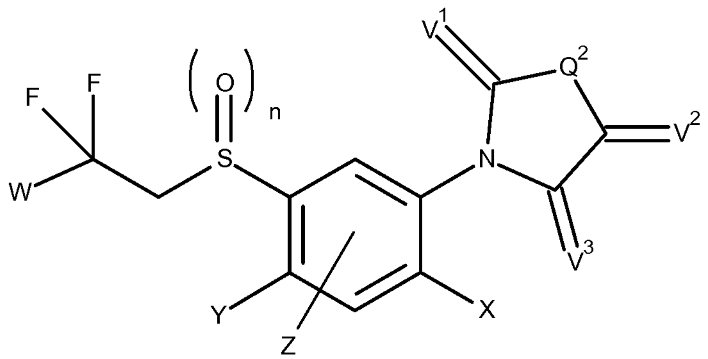

(I) mit (I-D)

(Verbindung der allgemeinen Formel (I) mit (I-D) mit Z = H)

| Beispiel-Nr. | W | n | Y | X | V¹ | V² | Q² | V³ |
|---|---|---|---|---|---|---|---|---|
| **101** | F | 0 | $CH_3$ | H | O | O | $NCH_2CHF_2$ | O |
| **102** | F | 0 | $CH_3$ | F | O | O | N-Cyclopropyl | O |
| **103** | F | 1 | $CH_3$ | F | O | O | $NCH_2CHF_2$ | O |
| **183** | F | 1 | $CH_3$ | F | O | O | N-Cyclopropyl | O |
| **184** | F | 1 | $CH_3$ | $CH_3$ | O | O | $NCH_2CHF_2$ | O |
| **185** | F | 0 | $CH_3$ | F | O | O | N-Cyclohexyl | O |

(fortgesetzt)

| Beispiel-Nr. | W | n | Y | X | $V^1$ | $V^2$ | $Q^2$ | $V^3$ |
|---|---|---|---|---|---|---|---|---|
| **186** | F | 1 | $CH_3$ | F | O | O | N-Cyclohexyl | O |
| **187** | F | 0 | $CH_3$ | $CH_3$ | S | O | $NCH_2CF_3$ | O |
| **188** | F | 1 | $CH_3$ | $CH_3$ | S | O | $NCH_2CF_3$ | O |
| **189** | F | 0 | $CH_3$ | F | S | O | $NCH_2CF_3$ | O |
| **190** | F | 1 | $CH_3$ | F | S | O | $NCH_2CF_3$ | O |
| **191** | F | 0 | $CH_3$ | F | S | O | $NCH_2CH(CH_3)_2$ | O |
| **192** | F | 0 | $CH_3$ | F | S | O | $NCH_2C(CH_3)_3$ | O |
| **193** | F | 0 | $CH_3$ | F | S | O | $NC(CH_3)_3$ | O |
| **194** | F | 1 | $CH_3$ | F | S | O | $NCH_2CH(CH_3)_2$ | O |
| **195** | F | 1 | $CH_3$ | F | S | O | $NCH_2C(CH_3)_3$ | O |
| **196** | F | 1 | $CH_3$ | F | S | O | $NC(CH_3)_3$ | O |

Die Bestimmung der logP Werte erfolgte analog OECD Guideline 117 (EC Directive 92/69/EEC) durch HPLC (High Performance Liquid Chromatography) an reversed-phase Säulen (C 18), mit nachfolgenden Methoden:

[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. LogP[a] wird auch logP(HCOOH) genannt.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. LogP[b] wird auch logP(neutral) genannt.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).
Die NMR-Spektren wurden mit einem Bruker II Avance 400, ausgestattet mit einem 1,7 mm TCI-Probenkopf, gemessen. In Einzelfällen wurden die NMR Spektren mit einem Bruker Avance II 600 bestimmt.
Die NMR-Daten ausgewählter Beispiele werden in klassischer Form (δ-Werte, Multiplettaufspaltung, Anzahl der H-Atome) aufgeführt. Die Aufstpaltung der Signale wurde wie folgt beschrieben: s (Singulett), d (Duplett), t (Triplett), q (Quartett), m (Multiplett), breit (für breite Signale). Als Lösungsmittel wurden $CD_3CN$, $CDCl_3$ oder D6-DMSO verwendet, wobei als Referenz Tetramethylsilan (0.00 ppm) eingesetzt wurde.
Die GC-MS-Spektren werden mit einem Agilent 6890 GC, HP 5973 MSD an Dimethylsilikonphase bestimmt, mit einem Temperaturgradient von 50 °C bis 320 °C. GC-MS-Indices werden als Kovats-Indices mit Lösung einer homologen Reihe von n-Alkanen (mit geradzahliger Anzahl von 8 bis 38 Kohlenstoffatomen) bestimmt.

| Bsp.-Nr. | logP (HCOOH) | logP (neutral) | NMR Daten | Lösungsmittel |
|---|---|---|---|---|
| 1 | 2,43 | 2,39 | 7,37(s,1H), 7,14(s,1H), 6,64(s,1H), 3,92-3,87(q,2H), 3,73-3,70(m,2H), 3,42-3,39(m,2H), 2,33(s,3H), 2,15(s,3H) | D6-DMSO |
| 2 | 2,42 | | 9,32(m,1H), 7,64(s,1H), 7,37(s,1H), 4,73(m,1H), 3,99(m,4H), 2,38(d,3H), 2,30(m,2H), 2,15(s,3H), 2,09(s,3H), 1,34(m,2H) | D6-DMSO |
| 3 | 1,02 | 2,88 | 7,99-7,97(m,1H), 7,32-7,29(m,1H), 4,20-4,11(m,1H), 3,90-3,64(m,5H), 2,62(t,2H), 2,37(s,3H), 2,12-2,05(m,2H) | D6-DMSO |
| 4 | 1,51 | 1,52 | 7,67(s,1H), 7,24(s,1H), 6,77(breit,1H), 4,13-3,93(m,2H), 3,78-3,71(m,2H), 3,44(t,2H), 2,33(s,3H), 2,24(s,3H) | D6-DMSO |

(fortgesetzt)

| Bsp.-Nr. | logP (HCOOH) | logP (neutral) | NMR Daten | Lösungsmittel |
|---|---|---|---|---|
| 5 | 1,50 | 4,38 | 9,46(t,1H), 7,64(s,1H), 7,37(s,1H), 4,30-4,19(m,2H), 4,05-3,95(m,4H), 3,30-3,26(m,2H), 2,38(s,3H), 2,34-2,27(m,2H), 2,12(s,3H) | D6-DMSO |
| 6 | 1,68 | 4,24 | 7,64-7,62(m,1H), 7,29-7,26(m,1H), 4,30-3,87(m,6H), 3,33(m,2H), 2,38(s,3H), 2,33(m,2H) | D6-DMSO |
| 7 | 1,47 | 1,43 | 7,99-7,97(m,1H), 7,34-7,32(m,1H), 7,03(breit,1H), 4,16-4,10(m,1H), 3,99-3,95(m,1H), 3,91-3,80(m,2H), 3,44(t,2H), 2,35(s,3H) | D6-DMSO |
| 8 | 1,82 | 4,63 | 7,80(breit,1H), 7,50(breit,1H), 4,75(breit,1H), 4,10-3,90(m,4H), 3,17(m,2H), 2,42(s,3H), 2,25(m,2H), 1,35(breit,3H) | D6-DMSO |
| 9 | 2,36 | 2,31 | 7,67-7,65(m,1H), 7,23-7,20(m,1H), 6,90(breit,1H), 3,91-3,83(q,2H), 3,80(t,2H), 3,41(t,2H), 2,37(s,3H) | D6-DMSO |
| 10 | 1,10 | 3,20 | 7,99-7,96(m,1H), 7,32-7,29(m,1H), 4,21-3,59(m,5H), 2,68-2,61(m,2H), 2,37(s,3H), 2,12-2,05(m,2H), 1,11-1,09(d,3H) | D6-DMSO |
| 11 | 3,60 | 3,50 | 7,62(d,1H), 7,39(d,1H), 4.39(d,1H), 4.26(d,1H), 4.00-3.83(m,4H), 2,43(s,3H) | D6-DMSO |
| 12 | 3,96 | 3,90 | 7,91(s,1H), 7,80(s,1H), 4,33(m,1H), 4,23-4.00(m,3H), 2,68-2,65(m,1H), 0,76-0,72(m,2H), 0,41-0,37(m,2H) | D6-DMSO |
| 13 | 2,70 | 2,64 | 7,92(dd,1H), 7,52(dd,1H), 4,41-4,25(m,3H), 4,06-3,69(m,3H), 2,43(s,3H) | D6-DMSO |
| 14 | 2,68 | 3,00 | 7,34(s,1H), 7,23(s,1H), 4,25(d,1H), 4,14(d,1H), 3.92-3.84(m,2H), 2,95(s,3H), 2,36(s,3H), 2,00(s,3H) | D6-DMSO |
| 15 | 2,48 | 2,48 | | D6-DMSO |
| 16 | 1,96 | 1,91 | 7,91(d,1H), 7,55-7,52(m,1H), 7,47(d,1H), 4,24-4,18(m,1H), 3,93-3,87(m,1H), 2,89(s,3H), 2,40(s,3H), 1,45(s,3H), 1,43(s,3H) | D6-DMSO |
| 17 | 2,04 | 2,01 | 7,97(d,1H), 7,51(d,1H), 4,32-4,20(m,1H), 4,00-3,89(m,1H), 2,89(s,3H), 2,43(s,3H), 1,45(s,3H), 1,44(s,3H) | D6-DMSO |
| 18 | 2,96 | 2,92 | 7,69(d,1H), 7,39(d,1H), 3,93(q,2H), 2,88(s,3H), 2,43(s,3H), 1,43(s,6H) | D6-DMSO |
| 19 | 2,52 | 2,45 | 7,56(d,1H), 7,40(d,1H), 3,87(q,2H), 2,86(breit,4H), 2,44(s,3H) | D6-DMSO |
| 20 | 2,59 | 2,58 | 7,49(d,1H), 7,38(d,1H), 7,23-7,21(m,1H), 4,03(q,2H), 3,00(s,3H), 2,99(s,3H), 2,38(s,3H) | D6-DMSO |
| 21 | 1,63 | 1,63 | 7,79(d,1H), 7,47-7,54(m,2H), 4,12-4,22(m,1H), 4,02-4,08(m,1H), 3,04(s,3H), 3,00(s,3H), 2,38(s,3H) | D6-DMSO |
| 22 | 2,61 | 2,56 | 7,70(d,1H), 7,43(d,1H), 4,01(q,2H), 3,01(s,3H), 2,95(s,3H), 2,42(s,3H) | D6-DMSO |
| 23 | 1,64 | 1,63 | 7,92(d,1H), 7,55(d,1H), 4,26-4,11(m,2H), 3,01(s,3H), 2,99(s,3H), 2,42(s,3H) | D6-DMSO |
| 24 | 1,97 | 1,95 | 7,81(d,1H), 7,78(d,1H), 7,67-7,64(m,1H), 4,31-4,14(m,2H), 3,07(s,3H), 3,00(s,3H) | D6-DMSO |

(fortgesetzt)

| Bsp.-Nr. | logP (HCOOH) | logP (neutral) | NMR Daten | Lösungsmittel |
|---|---|---|---|---|
| 25 | 3,04 | 2,97 | 7,89(d,1H), 7,81(d,1H), 7,51-7,48(m,1H), 4,21(q,2H), 3,07(s,3H), 3,01(s,3H) | D6-DMSO |
| 26 | 2,37 | 2,31 | 8,18(d,1H), 8,07(d,1H), 7,85-7,82(m,1H), 4,30-4,20(m,2H), 3,12(s,3H), 3,02(s,3H) | D6-DMSO |
| 27 | 1,67 | 1,64 | 8,19(d,1H), 7,98(d,1H), 7,80-7,77(m,1H), 4,42-4,32(m,2H), 3,11(s,3H), 3,01(s,3H) | D6-DMSO |
| 28 | 2,18 | 2,19 | 7,99(d,1H), 7,73(d,1H), 7,51-7,47(m,1H), 4,26(q,2H), 3,07(s,3H), 3,00(s,3H) | D6-DMSO |
| 29 | 1,93 | 1,91 | 8,03(d,1H), 7,94(d,1H), 4,34-4,19(m,2H), 3,01(s,6H) | D6-DMSO |
| 30 | 2,71 | 2,67 | 7,86(d,1H), 7,82(d,1H), 4,18(q,2H), 3,01(s,3H), 2,97(s,3H) | D6-DMSO |
| 31 | 2,72 | 2,69 | 8,14(d,1H), 7,78-7,75(m,1H), 7,59(d,1H), 4,05(s,3H), 4,04(q,2H), 3,09(s,3H) | D6-DMSO |
| 32 | 1,58 | 1,54 | 7,64-7,59(m,2H), 7,33(d,1H), 4,19-4,10(m,1H), 4,00-3,94(m,1H), 3,92(s,3H), 3,00(s,3H), 2,99(s,3H) | D6-DMSO |
| 33 | 1,75 | 1,70 | 7,77-7,74(m,1H), 7,39(s,1H), 4,19-4,00(m,2H), 3,01(s,3H), 2,97-2,90(m,3H), 2,37(s,3H)m 2,34(s,3H) | D6-DMSO |
| 34 | 1,91 | 1,88 | 7,90(d,1H), 7,53(d,1H), 4,25-4,10(m,2H), 2,95(s,3H), 2,73-2,67(m,1H), 2,42(s,3H), 0,93-0,88(m,4H) | D6-DMSO |
| 35 | 2,83 | 2,81 | 7,68(d,1H), 7,42(d,1H), 4,00(q,2H), 2,91(s,3H), 2,73-2,68(m,1H), 2,42(s,3H), 0,91-0,88(m,4H) | D6-DMSO |
| 36 | 2,89 | 2,87 | 7,46(d,1H), 7,37(d,1H), 7,21-7,19(m,1H), 4,02(q,2H), 2,96(s,3H), 2,70-2,65(m,1H), 2,37(s,3H), 0,91-0,87(m,4H) | D6-DMSO |
| 37 | 1,89 | 1,87 | 7,77(d,1H), 7,52-7,46(m,2H), 4,21-415(m,1H), 4,06-4,00(m,1H), 3,01(s,3H), 2,71-2,66(m,1H), 2,38(s,3H), 0,92-0,87(m,4H) | D6-DMSO |
| 38 | 2,18 | 2,16 | 8,02(d,1H), 7,92(d,1H), 4,34-4,18(m,2H), 2,97(s,3H), 2,71-2,67(m,1H), 0,93-0,88(m,4H) | D6-DMSO |
| 39 | 2,20 | 2,17 | 7,80-7,76(m,2H), 7,64-7,62(m,1H), 4,30-4,12(m,2H), 3,03(s,3H), 2,71-2,66(m,1H), 0,93-0,87(m,4H) | D6-DMSO |
| 40 | 3,04 | 2,98 | 7,63(d,1H), 7,55(d,1H), 7,30-7,27(m,1H), 4,18(q,2H), 2,98(s,3H), 2,70-2,67(m,1H), 0,92-0,87(m,4H) | D6-DMSO |
| 41 | 1,77 | 1,75 | 7,62-7,57(m,2H), 7,32(d,1H), 4,19-4,10(m,1H), 4,06-3,95(m,1H), 3,92(s,3H), 2,97(s,3H), 2,71-2,66(m,1H), 0,93-0,86(m,4H) | D6-DMSO |
| 42 | 2,51 | 2,46 | 7,42(d,1H), 7,29-7,27(m,1H), 7,14(d,1H), 3,97(q,2H), 3,89(s,3H), 2,91(s,3H), 2,70-2,65(m,1H), 0,91-0,86(m,4H) | D6-DMSO |
| 43 | 1,93 | 1,90 | 8,18(d,1H), 7,96(d,1H), 7,77-7,74(m,1H), 4,42-4,30(m,2H), 3,08(s,3H), 2,72-2,66(m,1H), 0,91-0,89(m,4H) | D6-DMSO |
| 44 | 2,53 | 2,45 | 7,98(d,1H), 7,70(d,1H), 7,48-7,45(m,1H), 4,25(q,2H), 3,03(s,3H), 2,71-2,65(m,1H), 0,91-0,88(m,4H) | D6-DMSO |
| 45 | 2,27 | 2,24 | 7,90(d,1H), 7,78(d,1H), 7,57-7,55(m,1H), 4,26-4,10(m,2H), 3,03(s,3H), 2,71-2,66(m,1H), 0,91-0,87(m,4H) | D6-DMSO |
| 46 | 3,11 | 3,05 | 7,77(d,1H), 7,51(d,1H), 7,21-7,18(m,1H), 4,19(q,2H), 2,98(s,3H), 2,70-2,66(m,1H), 0,92-0,87(m,4H) | D6-DMSO |

(fortgesetzt)

| Bsp.-Nr. | logP (HCOOH) | logP (neutral) | NMR Daten | Lösungsmittel |
|---|---|---|---|---|
| 47 | 1,98 | 1,93 | 7,76-7,72(m,1H), 7,38(s,1H), 4,19-3,97(m,2H), 2,93-2,86(m,3H), 2,73-2,67(m,1H), 2,49-2,32(m,6H), 0,92-0,87(m,4H) | D6-DMSO |
| 48 | 3,37 | 3,31 | 7,47(d,1H), 7,36(d,1H), 7,20-7,17(m,1H), 5,75-5,67(m,1H), 5,16-5,13(m,1H), 5,06-5,01(m,1H), 4,05-3,97(m,4H), 2,74-2,67(m,1H), 2,37(s,3H), 0,90-0,84(m,4H) | D6-DMSO |
| 49 | 2,28 | 2,24 | 7,78(d,1H), 7,51-7,46(m,2H), 5,76-5,69(m,1H), 5,18-5,15(m,1H), 5,08-5,04(m,1H), 4,22-4,16(m,1H), 4,07-3,99(m,3H), 2,74-2,70(m,1H), 2,38(s,3H), 0,90-0,88(m,4H) | D6-DMSO |
| 50 | 3,58 | 3,47 | 7,91(d,1H), 7,73(d,1H), 7,50-7,41(m,1H), 4,15-3,96(m,4H), 2,83-2,78(m,1H), 2,43(s,3H), 2,12-2,05(m,1H), 1,03-0,92(m,10H) | D6-DMSO |
| 51 | 2,82 | 2,83 | 7,53(s,1H), 7,26(d,1H), 7,19-7,17(m,1H), 4,32(q,2H), 3,95(q,2H), 3,09(s,3H), 2,36(s,3H), 1,36(q,3H) | D6-DMSO |
| 52 | 3,34 | 3,29 | 7,51(s,1H), 7,38(d,1H), 7,24-7,21(m,1H), 4,01(q,2H), 3,33(d,2H), 3,02(s,3H), 2,38(s,3H), 0,90-0,80(m,1H), 0,41-0,32(m,4H) | D6-DMSO |
| 53 | 3,33 | 3,23 | 7,88(d,1H), 7,78(d,1H), 7,48-7,46(m,1H), 4,20(q,2H), 3,03(s,3H), 2,70-2,66(m,1H), 0,92-0,88(m,4H) | D6-DMSO |
| 54 | 2,63 | 2,58 | 8,15(d,1H), 8,06(d,1H), 7,82-7,80(m,1H), 4,24-4,20(m,2H), 3,08(s,3H), 2,71-2,69(m,1H), 0,93-0,89(m,4H) | D6-DMSO |
| 55 | 2,09 | 0,60 | 10,98(s,1H), 7,72(d,1H), 7,39(d,1H), 3,95(q,2H), 2,97(s,3H), 2,41(s,3H) | D6-DMSO |
| 56 | 4,34 | 4,05 | 7,50(d,2H), 7,25(d,2H), 7,22-7,19(m,2H), 3,86(q,4H), 3,07(s,3H), 2,28(s,6H) | D6-DMSO |
| 57 | 3,62 | 3,56 | 7,44(d,1H), 7,37(d,1H), 7,30-7,28(m,3H), 7,14-7,12(m,1H), 7,04-7,02(m,2H), 4,61(s,2H), 3,97(q,2H), 2,98(s,3H), 2,39(s,3H) | D6-DMSO |
| 58 | 3,03 | 2,88 | 7,49(d,1H), 7,37(d,1H), 7,22-7,19(m,1H), 5,75-5,68(m,1H), 5,17-5,14(m,1H), 5,08-5,04(m,1H), 4,06-3,98(m,4H), 3,00(s,3H), 2,38(s,3H) | D6-DMSO |
| 59 | 2,03 | 2,00 | 7,80(d,1H), 7,53-7,47(m,2H), 5,77-5,71(m,1H), 5,19-5,16(m,1H), 5,11-5,06(m,1H), 4,22-4,13(m,1H), 4,11-4,01(m,3H), 3,01(s,3H), 2,39(s,3H) | D6-DMSO |
| 60 | 3,80 | 3,75 | 7,42-7,36(m,2H), 7,31-7,26(m,2H), 7,24-7,16(m,2H), 7,06-7,04(m,1H), 4,78(s,2H), 3,90(q,2H), 3,02(s,3H), 2,35(s,3H) | D6-DMSO |
| 61 | 3,33 | 3,27 | 7,55(d,1H), 7,37(d,1H), 7,25-7,23(m,1H), 4,04(q,2H), 3,79-3,71(m,1H), 3,97(s,3H), 2,37(s,3H), 1,17(d,6H) | D6-DMSO |
| 62 | 3,29 | 3,23 | 7,77(d,1H), 7,41(d,1H), 4,01(q,2H), 3,89-3,85(m,1H), 2,99(s,3H), 2,41(s,3H) | D6-DMSO |
| 63 | 3,65 | 3,57 | 7,53(d,1H), 7,36(d,1H), 7,24-7,21(m,1H), 4,03(q,2H), 3,75-3,68(m,1H), 2,71-2,66(m,1H), 2,37(s,3H), 1,15(d,6H); 0,89-0,86(m,4H) | D6-DMSO |

(fortgesetzt)

| Bsp.-Nr. | logP (HCOOH) | logP (neutral) | NMR Daten | Lösungsmittel |
|---|---|---|---|---|
| 64 | 2,51 | 2,45 | 8,01(d,1H), 7,52(d,1H), 4,27-4,18(m,1H), 4,12-4,02(m,1H), 3,92-3,85(m,1H), 2,75-2,69(m,1H), 2,41(s,3H), 1,12(d,3H), 1,08(d,3H), 0,90-0,88(m,4H) | D6-DMSO |
| 65 | 3,59 | 3,51 | 7,76(d,1H), 7,40(d,1H), 4,00(q,2H), 3,89-3,82(m,1H), 2,74-2,67(m,1H), 2,41(s,3H), 1,09(d,6H), 0,90-0,86(m,4H) | D6-DMSO |
| 66 | 2,30 | 0,69 | 10,86(s,1H), 7,70(d,1H), 7,37(d,1H), 3,94(q,2H), 2,71-2,65(m,1H), 2,41(s,3H), 0,94-0,84(m,4H) | D6-DMSO |
| 67 | 2,05 | 2,02 | 7,91(d,1H), 7,80(d,1H), 7,60-7,57(m,1H), 4,26-4,11(m,2H), 3,07(s,3H), 3,00(s,3H) | D6-DMSO |
| 68 | 2,83 | 2,78 | 7,77(d,1H), 7,54(d,1H), 7,23-7,20(m,1H), 4,19(q,2H), 3,02(s,3H), 2,99(s,3H) | D6-DMSO |
| 69 | 2,01 | 1,98 | 8,14(d,1H), 7,91(d,1H), 4,29-4,16(m,2H), 3,01(s,3H), 3,00(s,3H) | D6-DMSO |
| 70 | 1,64 | 1,59 | 7,97(t,1H), 7,88(d,1H), 4,38(q,2H), 3,01(s,3H) 3,00(s,3H) | D6-DMSO |
| 71 | 2,67 | 2,62 | 7,87(t,1H), 7,69(t,1H), 4,02(q,2H), 2,92(s,3H), 2,73-2,68(m,1H), 0,91-0,88(m,4H) | D6-DMSO |
| 72 | 2,24 | 2,18 | 8,12(d,1H), 7,90(d,1H), 4,29-4,14(m,2H), 2,97(s,3H), 2,72-2,67(m,1H), 0,91-0,88(m,4H) | D6-DMSO |
| 73 | 3,86 | 3,72 | 7,80(d,1H), 7,41(d,1H), 4,00(q,2H), 3,64-3,58(m,1H), 2,75-2,70(m,1H), 2,42(s,3H), 1,59-1,52(m,1H), 1,43-1,36(m,1H), 1,07(d,3H), 0,91-0,89(m,4H), 0,74(t,3H) | D6-DMSO |
| 74 | 1,93 | 1,91 | 7,92(d,1H), 7,55(d,1H), 4,26-4,13(m,2H), 3,54(q,2H), 2,99(s,3H), 2,42(s,3H), 1,19(t,3H) | D6-DMSO |
| 75 | 2,73 | 2,68 | 8,03-8,00(m,1H), 7,52(d,1H), 4,29-4,22(m,1H), 4,06-4,02(m,1H), 3,70-3,55(m,1H), 2,75-2,72(m,1H), 2,42(s,3H), 1,69-1,60(m,1H), 1,55-1,45(m,1H), 1,40(s,3H), 1,11-1,06(m,2H), 0,90(d,3H), 0,80-0,75(m,2H) | D6-DMSO |
| 76 | 4,09 | 3,94 | 7,96(s,1H), 7,90-7,78(m,4H), 7,49(d,1H), 4,02(q,2H), 3,06(s,3H), 2,45(s,3H) | D6-DMSO |
| 77 | 3,09 | 3,05 | 8,09(d,1H), 7,97(s,1H), 7,89(d,1H), 7,85-7,78(m,2H), 7,59(d,1H), 4,29-4,23(m,1H), 4,15-4,09(m,1H), 3,10(s,3H), 2,44(s,3H) | D6-DMSO |
| 78 | 3,40 | 3,29 | 8,20-8,16(m,1H), 7,85-7,81(m,2H), 7,39(d,1H), 3,93(q,2H), 3,11(s,3H), 2,34(s,3H) | D6-DMSO |
| 79 | 4,86 | 4,67 | 7,54(d,2H), 7,28-7,22(m,4H), 5,94-5,87(m,1H), 5,26-5,20(m,2H), 4,19(d,2H), 3,898q,4H), 2,26(s,6H) | D6-DMSO |
| 80 | 2,07 | 2,03 | 7,95(d,1H), 7,56(d,1H), 5,93-5,81(m,1H), 5,21-5,17(m,2H), 4,27-4,14(m,2H), 4,14-4,11(m,2H), 3,01(s,3H), 2,43(s,3H) | D6-DMSO |
| 81 | 3,05 | 3,01 | 7,73(d,1H), 7,44(d,1H), 5,90-5,82(m,1H), 5,21-5,15(m,2H), 4,13-4,11(m,2H) 4,03(q,2H) 2,97(s,3H) 2,43(s,3H) | D6-DMSO |
| 82 | 2,67 | 2,61 | 7,87(t,2H), 7,55-7,50(m,2H), 7,38(d,2H), 4,14-4,04(m,2H), 3,92-3,75(m,2H), 3,08(d,3H), 2,33-2,30(m,6H) | D6-DMSO |
| 83 | 2,92 | 2,88 | 7,72-7,70(m,1H), 7,43(d,1H), 4,06-3,98(m,2H), 3,53(q,2H), 2,95(s,3H), 2,43(s,3H) | D6-DMSO |

(fortgesetzt)

| Bsp.-Nr. | logP (HCOOH) | logP (neutral) | NMR Daten | Lösungsmittel |
|---|---|---|---|---|
| 84 | 3,46 | 3,38 | 7,88(d,1H), 7,53(d,1H), 7,48-7,46(m,1H), 7,39-7,33(m,1H), 7,29-7,23(m,2H), 4,13-4,07(m,1H), 3,91(q,2H), 3,77-3,70(m,1H), 3,07(s,3H), 2,30(s,3H), 2,28(s,3H) | D6-DMSO |
| 85 | 2,43 | 0,86 | 10,97(s,1H), 7,72(d,1H), 7,38(d,1H), 3,96(q,2H), 3,50(q,2H), 2,41(s,3H), 1,17(t,3H) | D6-DMSO |
| 86 | 3,75 | 3,67 | 7,77(d,1H), 7,41(d,1H), 4,07-3,97(m,3H), 2,99(s,3H), 2,41(s,3H), 1,75-1,69(m,2H), 1,67-1,60(m,2H), 1,56-1,48(m,2H), 1,46-1,40(m,2H) | D6-DMSO |
| 87 | 3,71 | 3,69 | 7,87(d,1H), 7,57-7,44(m,6H), 4,05(q,2H), 3,49(q,2H), 2,44(s,3H), 1,08(t,3H) | D6-DMSO |
| 88 | 2,71 | 2,66 | 8,10(d,1H), 7,59(d,1H), 7,55-7,51(m,4H), 7,48-7,44(m,1H), 4,31-4,09(m,2H), 3,57-3,51(m,2H), 2,44(s,3H), 1,09(t,3H) | D6-DMSO |
| 89 | 1,96 | 1,91 | 7,91(d,1H), 7,55-7,48(m,2H), 4,22-4,15(m,1H), 4,09-4,03(m,1H), 3,53(q,2H), 3,05(s,3H), 2,33(s,3H), 1,18(t,3H) | D6-DMSO |
| 90 | 3,81 | 3,71 | 7,58(d,1H), 7,33(d,1H), 7,28-7,24(m,3H), 7,05-7,01(m,2H), 4,56(s,2H), 3,92(q,2H), 2,77-2,72(m,1H), 2,39(s,3H), 0,94-0,85(m,4H) | D6-DMSO |
| 91 | 3,35 | 3,28 | 7,70(d,1H), 7,43(d,1H), 4,26-4,19(m,1H), 4,03(q,2H), 2,93(s,3H), 2,42(s,3H), 1,38(d,6H) | D6-DMSO |
| 92 | 2,27 | 2,22 | 7,91(d,1H), 7,55(d,1H), 4,27-4,15(m,3H), 2,97(s,3H), 2,42(s,3H), 1,39(d,6H) | D6-DMSO |
| 93 | 2,58 | 2,54 | 7,93(d,1H), 7,55(d,1H), 4,27-4,14(m,3H), 3,48-3,34(m,2H), 2,42(s,3H), 1,39(d,6H), 1,00(t,3H) | D6-DMSO |
| 94 | 3,85 | 3,82 | 7,68(d,1H), 7,42(d,1H), 4,02(q,2H), 2,90(s,3H), 2,42(s,3H), 1,58(s,9H) | D6-DMSO |
| 95 | 2,69 | 2,65 | 7,89(d,1H), 7,53(d,1H), 4,26-4,12(m,2H), 2,94(s,3H), 2,42(s,3H), 1,58(s,9H) | D6-DMSO |
| 96 | 2,66 | 2,61 | 8,05-7,99(m,1H), 7,49(d,1H), 5,67-5,63(m,1H), 5,39-5,28(m,1H), 4,55-4,50(m,1H), 4,27-4,16(m,1H), 4,12-3,95(m,1H), 3,01(s,3H), 2,41(s,3H), 1,87-1,63(m,5H), 1,54-1,43(m,1H) | D6-DMSO |
| 97 | 2,91 | 2,83 | 7,72(d,1H), 7,44(d,1H), 4,01(q,2H), 3,38(q,2H), 3,01(s,3H), 2,42(s,3H), 0,99(t,3H) | D6-DMSO |
| 98 | 1,93 | 1,91 | 7,94(d,1H), 7,55(d,1H), 4,26-4,09(m,2H), 3,50-3,38(m,2H), 3,02(s,3H), 2,42(s,3H), 1,00(t,3H) | D6-DMSO |
| 99 | 4,21 | 4,16 | 7,70(d,1H)m 7,42(d,1H), 4,03(q,2H), 3,34(q,2H), 2,42(s,3H), 1,58(s,9H), 0,97(t,3H) | D6-DMSO |
| 100 | 3,32 | 3,29 | 7,52(d,1H), 7,38(d,1H), 7,25-7,22(m,1H), 4,05(q,2H), 3,52(q,2H), 3,46(q,2H), 2,37(s,3H), 1,17(t,3H), 0,96(t,3H) | D6-DMSO |
| 101 | 3,22 | 1,73 | 7,70-7,68(m,1H), 7,51-7,48(m,1H), 6,25(tt,1H), 4,10-4,01(dt,2H), 3,90-3,82(q,2H), 2,48(s,3H) | D6-DMSO |
| 102 | 3,24 | | 7,64-7,62(m,1H), 7,48-7,46(m,1H), 3,88-3,81(q,2H), 2,73-2,68(m,1H), 2,47(s,3H), 0,95-0,88(m,4H) | D6-DMSO |

(fortgesetzt)

| Bsp.-Nr. | logP (HCOOH) | logP (neutral) | NMR Daten | Lösungsmittel |
|---|---|---|---|---|
| 103 | 2,32 | 0,87 | 8,04-8,03(m,1H), 7,61-7,58(m,1H), 6,24(tt,1H), 4,36-4,27(m,1H), 4,10-4,01(dt,2H), 3,92-3,83(m,1H), 2,43(s,3H) | D6-DMSO |
| 104 | 2,66 | | 7.71 (d,1H), 7.03 (d,1H), 4.54-4.49 (m,2H), 4.08-4.03 (m,2H), 3.37-3.31 (m,2H), 2.46 (s,3H) | D6-DMSO |
| 105 | 1,64 | | 8.11 (d,1H), 7.19 (d,1H), 4.58-4.49 (m,2H), 4.18-4.10 (m,1H), 4.04-3.96 (m,1H), 3.59-3.33 (m,2H), 2.41 (s,3H) | CDCl3 |
| 106 | 2,98 | | 7.74 (d,1H), 7.31 (d,1H), 4.87-4.80 (m,1H), 4.10-4.04 (m,1H), 3.98-3.88 (m,2H), 3.64-3.59 (m,1H), 2.39 (s,3H), 1.43 (d,3H) | D6-DMSO |
| 107 | 1,79 | | 7.98 (dd, 1H), 7.26-7.07 (m,1H), 4.68-4.62 (m,1H), 4.51-4.42 (m,1H), 4.11-4.02 (m,1H), 3.53-3.39 (m,2H), 2.41 (s,3H), 1.26-1.23 (m,3H). | CDCl3 |
| 108 | 2,92 | | 7.56 (d,1H), 7.06 (d,1H), 4.65-4.60 (m,1H), 4.45-4.38 (m,1H), 4.06-4.01 (m,1H), 3.35 (q, 2H), 2.48 (s,3H), 1.26-1.22 (m,3H) | CDCl3 |
| 109 | 1,93 | 1,89 | 8.11 (d,1H), 7.08 (d,1H), 4.90-4.81 (m,1H), 4.20-4.01 (m,1H), 3.76-3.37 (m,3H), 2.40 (s,3H), 1.57-1.54 (m,3H) | CDCl3 |
| 110 | 2,38 | | 7.99 (d,1H), 7.24 (d,1H), 3.52-3.41 (m,2H), 2.45 (s,3H), 1.72 (s,6H) | CDCl3 |
| 111 | 3,35 | | 7.50 (d,1H), 7.16 (d,1H), 3.39-3.32 (m,2H), 2.53 (s,3H), 1.71 (s,6H) | CDCl3 |
| 112 | 3,65 | 3,65 | 7,14(s,1H), 7,00(s,1H), 3,92(s,2H), 3,82(q,2H), 2,71(septet,1H), 2,33(s,3H), 2,06(s,3H), 1,00-0,90(m,4H) | D6-DMSO |
| 113 | 4,84 | 4,73 | 7,63(d,1H), 7,37(d,1H), 4,30-4,15(m,2H), 3,89-3,86(m,2H), 2,90(d,2H), 2,43(s,3H), 0,78(s,9H) | D6-DMSO |
| 114 | 3,66 | 3,63 | 7,91-7,88(m,1H), 7,49(d,1H), 4,33-3,66(m,4H), 2,97-2,87(m,2H), 2,43(d,3H), 0,81(d,9H) | D6-DMSO |
| 115 | 4,33 | 4,31 | 7,63(d,1H), 7,38(d,1H), 4,50-4,10(m,2H), 3,89(q,2H), 3,02-3,00(m,2H), 2,43(s,3H), 1,77-1,70(m,1H), 0,82-0,80(m,6H) | D6-DMSO |
| 116 | 2,40 | 2,39 | 7,72(d,1H), 7,42(d,1H), 3,93(q,2H), 3,19(s,6H), 2,44(s,3H) | D6-DMSO |
| 117 | 1,54 | 1,50 | 7,99(d,1H), 7,54(d,1H), 4,29-4,25(m,1H), 3,96-3,89(m,1H), 3,19(s,6H), 2,50(s,3H) | D6-DMSO |
| 118 | 3,03 | | 7,72(d,1H), 7,40(d,1H), 3,96(q,2H), 3,61(q,4H), 2,42(s,3H), 1,10(t,6H) | D6-DMSO |
| 119 | 2,07 | | 7,99(d,1H), 7,55(d,1H), 4,38-4,18(m,1H), 4,15-3,92(m,1H), 3,80-3,58(m,4H), 2,44(s,3H), 1,13(t,6H) | D6-DMSO |
| 120 | 2,46 | | 8,02(d,1H), 7,56(d,1H), 5,92-5,79(m,2H), 5,41-5,28(m,4H), 4,36-4,20(m,5H), 4,09-3,96(m,1H), 2,44(s,3H) | D6-DMSO |
| 121 | 2,81 | | 7,72(d,1H), 7,44(d,1H), 4,50(d,2H), 3,95(q,2H), 3,20(s,3H), 2,45-2,40(m,4H) | D6-DMSO |
| 122 | 2,71 | | 7,71(d,1H), 7,40(d,1H), 3,93(q,2H), 3,66(q,2H), 3,16(s,3H), 2,42(s,3H), 1,10(t,3H) | D6-DMSO |
| 123 | 1,96 | | 8,02(d,1H), 7,18(d,1H), 4,41(d,2H), 3,54-3,38(m,2H), 3,33(s,3H), 2,44(s,3H), 2,40(t,1H) | CDCl3 |

(fortgesetzt)

| Bsp.-Nr. | logP (HCOOH) | logP (neutral) | NMR Daten | Lösungsmittel |
|---|---|---|---|---|
| 124 | 2,00 | | 8,00(d,1H), 7,55(d,1H), 5,92-5,80(m,1H), 5,41-5,28(m,2H), 4,33-4,20(m,3H), 4,04-3,91(m,1H), 3,17(s,3H), 2,43(s,3H) | D6-DMSO |
| 125 | 2,91 | | 7,74(d,1H), 7,43(d,1H), 5,90-5,79(m,1H), 5,41-5,28(m,2H), 4,28(d,2H), 3,94(q,2H), 3,16(s,3H), 2,44(s,3H) | D6-DMSO |
| 126 | 3,15 | | 7,72(d,1H), 7,43(d,1H), 3,95(q,2H), 3,54(d,2H), 3,21(s,3H), 2,44(s,3H), 1,08-1,05(m,1H), 0,54-0,47(m,2H), 0,36-0,30(m,2H) | D6-DMSO |
| 127 | 3,19 | 3,17 | 7,71(d,1H), 7,43(d,1H), 4,01(q,2H), 3,35(q,2H), 2,75-2,70(m,1H), 2,42(s,3H), 0,97(t,3H), 0,91-0,87(m,4H) | D6-DMSO |
| 128 | 3,49 | 3,45 | 7,72(d,1H), 7,43(d,1H), 4,01(q,2H), 3,30(t,2H), 2,76-2,70(m,1H), 2,42(s,3H), 1,42-1,34(m,2H), 0,92-0,87(m,4H), 0,72(t,3H) | D6-DMSO |
| 129 | 3,48 | 3,43 | 7,79(d,1H), 7,42(d,1H), 4,35(q,2H), 4,00(q,2H), 2,81-2,73(m,1H), 2,42(s,3H), 0,98-0,88(m,4H) | D6-DMSO |
| 130 | 3,31 | 3,28 | 7,67(d,1H), 7,40(d,1H), 5,74-5,64(m,1H), 5,13-5,10(m,1H), 5,07-5,02(m,1H), 4,02-3,94(m,4H), 2,76-2,70(m,1H), 2,42(s,3H), 0,92-0,89(m,4H) | D6-DMSO |
| 131 | 3,00 | 2,97 | 7,49(d,1H), 7,38(d,1H), 7,23-7,21(m,1H), 4,04(q,2H), 3,51(q,2H), 3,00(s,3H), 2,37(s,3H), 1,17(t,3H) | D6-DMSO |
| 132 | 3,42 | 3,38 | 7,48(d,1H), 7,38(d,1H), 7,22-7,19(m,1H), 4,25-4,18(m,1H), 4,04(q,2H), 2,98(s,3H), 2,37(s,3H), 1,37(d,6H) | D6-DMSO |
| 133 | 3,77 | 3,73 | 7,52(d,1H), 7,38(d,1H), 7,24-7,21(m,1H), 4,26-4,19(m,1H), 4,05(q,2H), 3,44(q,2H), 2,37(s,3H), 1,38(d,6H), 0,95(t,3H) | D6-DMSO |
| 134 | 4,32 | 4,27 | 7,49(d,1H), 7,37(d,1H), 7,22-7,20(m,1H), 4,05(q,2H), 3,40(q,2H), 2,37(s,3H), 1,58(s,9H), 0,94(t,3H) | D6-DMSO |
| 135 | 4,77 | 4,70 | 7,52(d,1H), 7,35(d,1H), 7,23-7,21(m,1H), 4,04(q,2H), 3,74(m,1H), 2,37(s,3H), 1,56(s,9H), 1,14(d,6H) | D6-DMSO |
| 136 | 4,17 | 4,13 | 7,71(d,1H), 7,43(d,1H), 4,03(q,2H), 3,87-3,78(m,1H), 2,93(s,3H), 2,42(s,3H), 2,03-1,99(m,2H), 1,81-1,71(m,4H), 1,64-1,61(m,1H), 1,33-1,25(m,2H), 1,13(m,1H) | D6-DMSO |
| 137 | 3,02 | 2,96 | 7,90(d,1H), 7,54(d,1H), 4,22-4,18(m,2H), 3,40-3,37(m,2H), 2,42(s,3H), 1,58(s,9H), 0,98(t,3H) | D6-DMSO |
| 138 | 2,20 | 2,19 | 7,82(d,1H), 7,58-7,55(m,1H), 7,49(d,1H), 4,22-4,03(m,2H), 3,59-3,45(m,4H), 2,39(s,3H), 1,18(t,3H), 0,98(t,3H) | D6-DMSO |
| 139 | 2,71 | 2,70 | 7,77(d,1H), 7,50-7,48(m,2H), 4,18-4,06(m,2H), 3,00(s,3H), 2,38(s,3H), 1,58(s,9H) | D6-DMSO |
| 140 | 2,56 | 2,54 | 7,87(d,1H), 7,57-7,55(m,1H), 7,48(d,1H), 4,23-4,13(m,1H), 4,07-3,98(m,1H), 3,80-3,74(m,1H), 3,51(q,2H), 2,38(s,3H), 1,23(d,3H), 1,21-1,15(m,6H) | D6-DMSO |
| 141 | 2,27 | 2,24 | 7,78(d,1H), 7,53-7,47(m,2H), 4,26-4,15(m,2H), 4,09-4,02(m,1H), 3,03(s,3H), 2,38(s,3H), 1,38(d,6H) | D6-DMSO |
| 142 | 4,19 | 4,13 | 7,74(d,1H), 7,35(d,1H), 4,12-3,86(breit,2H), 3,52-3,41(breit,2H), 2,39(s,3H), 1,23(s,9H), 1,14(t,3H) | D6-DMSO |
| 143 | | 4,56 | 7,73(d,1H), 7,35(d,1H), 4,22-4,15(m,1H), 4,11-3,85(breit,2H), 2,39(s,3H), 1,35(d,6H), 1,22(s,9H) | D6-DMSO |

(fortgesetzt)

| Bsp.-Nr. | logP (HCOOH) | logP (neutral) | NMR Daten | Lösungsmittel |
|---|---|---|---|---|
| 144 | 4,08 | 4,07 | 7,73(d,1H), 7,34(d,1H), 4,10-3,85(breit,2H), 2,70-2,64(m,1H), 2,39(s,3H), 1,20(s,9H), 0,91-0,82(m,4H) | D6-DMSO |
| 145 | 3,54 | 3,49 | 7,81(d,1H), 7,43(d,1H), 4,41(q,2H), 4,02(q,2H), 3,58(q,2H), 2,43(s,3H), 1,20(t,3H) | D6-DMSO |
| 146 | 3,85 | 3,90 | 7,80(d,1H), 7,43(d,1H), 4,39(q,2H), 4,30-4,23(m,1H), 4,02(q,2H), 2,43(s,3H), 1,40(d,6H) | D6-DMSO |
| 147 | 4,34 | 4,28 | 7,78(d,1H), 7,42(d,1H), 4,35(q,2H), 4,02(q,2H), 2,42(s,3H), 1,59(s,9H) | D6-DMSO |
| 148 | 3,82 | 3,78 | 7,78(d,1H), 7,44(d,1H), 4,04(q,2H), 3,54(q,2H), 3,24(d,2H), 2,42(s,3H), 1,75-1,65(m,1H), 1,18(t,3H), 0,73(d,6H) | D6-DMSO |
| 149 | 4,27 | 4,21 | 7,76(d,1H), 7,43(d,1H), 4,30-4,20(m,1H), 4,04(q,2H), 3,21(d,2H), 2,42(s,3H), 1,76-1,65(m,1H), 1,38(d,6H), 0,73(d,6H) | D6-DMSO |
| 151 | 1,98 | 1,88 | 7,90(d,1H), 7,53(d,1H), 4,25-4,10(m,2H), 2,95(s,3H), 2,73-2,67(m,1H), 2,42(s,3H), 0,93-0,88(m,4H) | D6-DMSO |
| 152 | 3,40 | 3,35 | 8,10-7,85(breit,1H), 7,47(d,1H), 4,30-4,10(m,3H), 2,44(s,3H), 1,35(d,6H), 1,30-1,15(breit,9H) | D6-DMSO |
| 153 | 2,95 | 3,04 | 8,03(d,1H), 7,55(d,1H), 4,44(q,2H), 4,31-4,10(m,3H), 2,44(s,3H), 1,40(d,6H) | D6-DMSO |
| 154 | 3,62 | 3,64 | 7,82(d,1H), 7,44(d,1H), 5,94-5,82(m,1H), 5,24-5,15(m,2H), 4,44(q,2H), 4,18-4,15(m,2H), 4,03(q,2H), 2,43(s,3H) | D6-DMSO |
| 155 | 3,93 | 3,90 | 7,94(d,1H), 7,60-7,44(m,6H), 4,50(q,2H), 4,04(q,2H), 2,45(s,3H) | D6-DMSO |
| 156 | 3,44 | 3,51 | 7,83(d,1H), 7,45(d,1H), 6,46-6,14(m,1H), 4,46(q,2H), 4,07-3,96(m,4H), 2,43(s,3H) | D6-DMSO |
| 157 | 3,31 | 1,33 | 11,55(s,1H), 7,84(d,1H), 7,68(s,1H), 7,59-7,53(m,2H), 7,52-7,50(m,1H), 7,42(d,1H), 3,96(q,2H), 2,43(s,3H) | D6-DMSO |
| 158 | 2,69 | 1,10 | | |
| 159 | 2,96 | 2,95 | 7,70(d,1H), 7,49(d,1H), 4,02(q,2H), 3,56(t,2H), 3,36(t,2H), 3,21(s,3H), 2,95(s,3H), 2,43(s,3H), 1,86-1,79(m,2H) | D6-DMSO |
| 160 | 3,28 | 3,22 | 7,72(d,1H), 7,44(d,1H), 4,03(q,2H), 3,46(t,2H), 2,95(s,3H), 2,43(s,3H), 1,67-1,58(m,2H), 0,88(t,3H) | D6-DMSO |
| 161 | 3,39 | 3,34 | 7,73(d,1H), 7,44(d,1H), 4,03(q,2H), 3,38(d,2H), 2,97(s,3H), 2,43(s,3H), 1,16-1,08(m,1H), 0,52-0,46(m,2H), 0,35-0,29(m,2H) | D6-DMSO |
| 162 | 3,42 | 3,27 | 7,83(d,1H), 7,56-7,44(m,6H), 4,04(q,2H), 3,05(s,3H), 2,45(s,3H) | D6-DMSO |
| 163 | 2,44 | 2,38 | 8,07(d,1H), 7,58(d,1H), 7,55-7,51(m,4H), 7,48-7,41(m,1H), 4,31-4,10(m,2H), 3,09(s,3H), 2,44(s,3H) | D6-DMSO |
| 164 | 2,58 | 2,54 | 8,03(d,1H), 7,53(d,1H), 4,40(q,2H), 4,24-4,18(m,1H), 4,15-4,04(m,1H), 2,80-2,74(m,1H), 2,43(s,3H), 0,94-0,92(m,4H) | D6-DMSO |

(fortgesetzt)

| Bsp.-Nr. | logP (HCOOH) | logP (neutral) | NMR Daten | Lösungsmittel |
|---|---|---|---|---|
| 165 | 2,32 | 2,30 | 7,92(d,1H), 7,51(d,1H), 5,75-5,67(m,1H), 5,16-5,14(m,1H), 5,09-5,05(m,1H), 4,25-4,07(m,2H), 4,00(d,2H), 2,77-2,71(m,1H), 2,41(s,3H), 0,91-0,89(m,4H) | D6-DMSO |
| 166 | 2,72 | 2,89 | 8,05(d,1H), 7,56(d,1H), 5,94-5,84(m,1H), 5,24-5,17(m,2H), 4,48(q,2H), 4,25-4,10(m,4H), 2,44(s,3H) | D6-DMSO |
| 167 | 3,05 | 3,01 | 8,20(d,1H), 7,60-7,47(m,6H), 4,55(q,2H), 4,28-4,02(m,2H), 2,45(s,3H) | D6-DMSO |
| 168 | 2,60 | 2,58 | 8,06(d,1H), 7,57(d,1H), 6,45-6,16(m,1H), 4,51-4,46(m,2H), 4,26-3,96(m,4H), 2,44(s,3H) | D6-DMSO |
| 169 | 2,30 | 2,33 | 7,93(d,1H), 7,56(d,1H), 4,28-4,11(m,2H), 3,38(d,2H), 3,01(s,3H), 2,43(s,3H), 1,15-1,05(m,1H), 0,51-0,45(m,2H), 0,35-0,28(m,2H) | D6-DMSO |
| 170 | 1,97 | 1,98 | 7,92(d,1H), 7,55(d,1H), 4,23-4,15(m,2H), 3,56(t,2H), 3,36(t,2H), 3,21(s,3H), 2,99(s,3H), 2,42(s,3H), 1,87-1,80(m,2H) | D6-DMSO |
| 171 | 2,18 | 2,15 | 7,93(d,1H), 7,54(d,1H), 4,25-4,09(m,2H), 3,43-3,37(m,2H), 2,76-2,70(m,1H), 2,42(s,3H), 0,98(t,3H), 0,91-0,87(m,4H) | D6-DMSO |
| 172 | 2,46 | 2,42 | 7,93(d,1H), 7,54(d,1H), 4,27-4,08(m,2H), 3,40-3,35(m,2H), 2,76-2,71(m,1H), 2,42(s,3H), 1,45-1,36(m,2H), 0,91-0,88(m,4H), 0,75(t,3H) | D6-DMSO |
| 173 | 3,64 | 3,58 | 7,74(d,1H), 7,36-7,30(m,5H), 7,27-7,23(m,1H), 3,85(q,2H), 2,30(s,3H), 1,43(s,6H) | D6-DMSO |
| 174 | 2,84 | 2,79 | 7,76(d,1H), 7,45(d,1H), 4,04(q,2H), 2,93(s,3H), 2,43(s,3H), 1,30(s,6H) | D6-DMSO |
| 175 | 3,41 | 3,40 | 7,64(d,1H), 7,46(d,1H), 4,04(q,2H), 2,96(s,3H), 2,38(s,3H), 1,79-1,66(m,4H), 0,83(t,6H) | D6-DMSO |
| 176 | 2,37 | 2,31 | 7,87(d,1H), 7,59(d,1H), 4,27-4,18(m,2H), 3,00(s,3H), 2,44(s,3H), 1,80-1,70(m,4H), 0,84(q,6H) | D6-DMSO |
| 177 | 1,89 | 1,85 | 7,98(d,1H), 7,57(d,1H), 4,30-4,10(m,2H), 2,98(s,3H), 2,44(s,3H), 1,32(s,6H) | D6-DMSO |
| 178 | 3,80 | 3,76 | 7,61(d,1H), 7,31-7,23(m,4H), 6,97-6,89(m,2H), 4,65(breit,2H), 3,96(q,2H), 2,39(s,3H), 1,30(s,6H) | D6-DMSO |
| 179 | 3,00 | 2,96 | 7,71(d,1H), 7,43(d,1H), 4,01(q,2H), 2,89(s,3H), 2,43(s,3H), 2,46-2,33(m,4H), 2,16-2,09(m,2H) | D6-DMSO |
| 180 | 1,99 | 1,97 | 7,92(d,1H), 7,54(d,1H), 4,30-4,08(m,2H), 2,93(s,3H), 2,48-2,31(m,7H), 2,19-2,05(m,2H) | D6-DMSO |
| 181 | 3,44 | 3,39 | 7,85(d,1H), 7,50(d,1H), 4,03(q,2H), 3,31(s,3H), 2,92-2,87(m,1H), 2,44(s,3H), 1,04-0,97(m,4H) | D6-DMSO |
| 182 | 3,83 | 3,76 | 7,96(d,1H), 7,55(d,1H), 4,11-3,95(m,2H), 3,39(s,3H), 3,00-2,91(m,1H), 2,46(s,3H), 1,25-1,10(m,4H) | D6-DMSO |
| 183 | 2,31 | | 7,98-7,96(m,1H), 7,58-7,56(m,1H), 4,35-4,26(m,1H), 3,90-3,81(m,1H), 2,73-2,67(m,1H), 2,42(s,3H), 0,98-0,88(m,4H) | D6-DMSO |
| 184 | 2,44 | | 7,91-7,90(m,1H), 7,42(s,1H), 6,22(tt,1H), 4,30-4,22(m,1H), 4,08-3,99(m,2H), 3,83-3,73(m,1H), 2,38(s,3H), 2,25(s,3H) | D6-DMSO |

(fortgesetzt)

| Bsp.-Nr. | logP (HCOOH) | logP (neutral) | NMR Daten | Lösungsmittel |
|---|---|---|---|---|
| 185 | 4,32 | 4,27 | 7,67(d,1H), 7,47(d,1H), 4,00-3,90(m,1H), 3,84(q,2H), 2,47(s,3H), 2,00-1,89(m,2H), 1,86-1,75(m,4H), 1,67-1,58(m,1H),1,39-1,25(m,2H), 1,20-1,09(m,1 H) | D6-DMSO |
| 186 | 3,36 | 3,33 | 8,03(d,1H), 7,57(d,1H), 4,35-4,26(m,1H), 3,99-3,83(m,2H), 2,43(s,3H), 2,00-1,90(m,2H), 1,86-1,75(m,3H), 1,69-1,60(m,1H), 1,39-1,22(m,2H), 1,22-1,09(m,2H) | D6-DMSO |
| 187 | 4,21 | | 7,54(s,1H), 7,32(s,1H), 4,84-4,69(m,2H), 3,83-3,76(q,2H), 2,41(s,3H), 2,13(s,3H) | D6-DMSO |
| 188 | 3,18 | | 7,94(s,1H), 7,41(s,1H), 4,84-4,68(m,2H), 4,31-4,23(m,1H), 3,81-3,68(m,1H), 2,39(s,3H), 2,22(s,3H) | D6-DMSO |
| 189 | 3,95 | | 7,70(d,1H), 7,49(d,1H), 4,87-4,71(m,2H), 3,84(q,2H), 2,49(s,3H) | D6-DMSO |
| 190 | 3,03 | | 8,08(d,1H), 7,59(d,1H), 4,87-4,69(m,2H), 4,38-3,80(m,2H), 2,44(s,3H) | D6-DMSO |
| 191 | 4,62 | 4,54 | 7,70(d,1H), 7,47(d,1H), 3,83(q,2H), 3,75-3,66(m,2H), 2,48(s,3H), 2,18-2,11(m,1H), 0,96-0,91(m,6H) | D6-DMSO |
| 192 | 4,89 | 4,80 | 7,69(d,1H), 7,47(d,1H), 3,87-3,77(m,4H), 2,48(s,3H), 0,99(s,9H) | D6-DMSO |
| 193 | 4,50 | 4,42 | 7,64(d,1H), 7,44(d,1H), 3,82(q,2H), 2,47(s,3H), 1,79(s,9H) | D6-DMSO |
| 194 | 3,56 | 3,53 | 8,09-8,06(m,1H), 7,57(d,1H), 4,38-3,64(m,4H), 2,44(s,3H), 2,17-2,12(m,1H), 0,95(d,6H) | D6-DMSO |
| 195 | 3,85 | 3,79 | 8,08-8,05(m,1H), 7,57(d,1H), 4,36-4,30(m,1H), 3,89-3,71(m,3H), 2,44(s,3H), 1,00(d,9H) | D6-DMSO |
| 196 | 3,45 | 3,41 | 8,00(d,1H), 7,54(d,1H), 4,40-3,45(m,2H), 2,43(d,3H), 1,79(d,9H) | D6-DMSO |
| 197 | 1,97 | 1,89 | 7,90(d,1H), 7,53(d,1H), 4,25-4,10(m,2H), 2,95(s,3H), 2,73-2,67(m,1H), 2,42(s,3H), 0,93-0,88(m,4H) | D6-DMSO |
| 198 | 2,79 | 2,75 | 7,67-7,65(m,1H), 7,25-7,21(m,1H), 3,91-3,83(q,2H), 3,73(t,2H), 3,44(t,2H), 2,75(s,3H), 2,37(s,3H) | D6-DMSO |
| 199 | 1,76 | 1,74 | 7,99-7,97(m,1H), 7,35-7,32(m,1H), 4,18-4,09(m,1H), 3,98-3,92(m,1H), 3,83-3,81(d,1H), 3,76-3,74(d,1H), 3,48(t,2H), 2,77(s,3H), 2,35(s,3H) | D6-DMSO |
| 200 | 2,42 | 2,33 | 7,61(d,1H), 7,39(d,1H), 4,20(breit,2H), 3,90(q,2H), 2,93(s,3H), 2,43(s,3H) | D6-DMSO |
| 201 | 2,02 | 1,99 | 7,94(d,1H), 7,51(d,1H), 5,92-5,82(m,1H), 5,37-5,31(m,1H), 5,28-5,21(m,1H), 4,35-4,22(m,1H), 4,17(s,2H), 4,00(d,2H), 3,98-3,85(m,1H), 2,42(s,3H) | D6-DMSO |
| 202 | 3,39 | 3,35 | 7,62(d,1H), 7,39(d,1H), 4,21(breit,2H), 3,90(q,2H), 3,35(t,2H), 2,44(s,3H), 1,59-1,50(m,2H), 1,38-1,28(m,2H), 0,91(t,3H) | D6-DMSO |
| 203 | 2,47 | 2,43 | 8,91(d,1H), 7,50(d,1H), 4,38-4,20(m,1H), 4,22(s,2H), 3,98-3,88(m,1H), 3,36(t,2H), 2,42(s,3H), 1,60-1,50(m,2H), 1,39-1,30(m,2H), 0,92(t,3H) | D6-DMSO |
| 204 | 3,49 | 3,48 | 7,69(d,1H), 7,42-7,31(m,6H), 4,48(s,2H), 4,14(breit,2H), 3,91(q,2H), 2,45(s,3H) | D6-DMSO |

(fortgesetzt)

| Bsp.-Nr. | logP (HCOOH) | logP (neutral) | NMR Daten | Lösungsmittel |
|---|---|---|---|---|
| 205 | 2,60 | 2,52 | 7,99(d,1H), 7,52(d,1H), 7,42-7,31(m,5H), 4,59(s,2H), 4,38-4,21(m,1H), 4,15(s,2H), 3,99-3,85(m,1H), 2,43(s,3H) | D6-DMSO |
| 206 | 2,08 | | 7,99(dd,1H), 7,21(d,1H), 5,15-5,08(m,1H), 3,52-3,41(m,2H), 2,45(s,3H), 1,76-1,73(m,3H) | CDCl3 |
| 207 | 1,57 | -0,23 | 10,89(s,1H), 7,99(d,1H), 7,48(d,1H), 4,25-4,16(m,1H), 4,06-3,97(m, 1H), 2,73-2,66(m,1H), 2,40(s,3H), 0,91-0,87(m,4H) | D6-DMSO |
| 208 | 1,68 | | 8,18(d,1H), 7,11(d,1H), 4,44(d,1H), 4,27(d,1H), 3,54-3,38(m,2H), 3,14(s,3H), 2,42(s,3H) | CDCl3 |
| 209 | 1,92 | | 8,18(d,1H), 7,10(d,1H), 4,38(d,1H), 4,18(d,1H), 3,55-3,39(m,2H), 2,73-2,67(m,1H), 2,42(s,3H), 1,05-1,04(m,4H) | CDCl3 |
| 210 | 2,91 | | 7,76(d,1H), 7,05(d,1H), 4,26(s,2H), 3,35(q,2H), 2,72-2,66(m,1H), 2,48(s,3H), 1,06-1,01(m,4H) | CDCl3 |
| 211 | 2,66 | | 7,76(d,1H), 7,06(d,1H), 4,44(s,2H), 3,34(q,2H), 3,13(s,3H), 2,48(s,3H) | CDCl3 |
| 212 | 2,92 | | 7,60(d,1H), 7,08(d,1H), 4,51(q,1H), 3,34(q,2H), 3,12(s,3H), 2,50(s,3H), 1,37(d,3H) | CDCl3 |
| 213 | 1,91 | | 8,12-7,97(m,1H), 7,16-7,10(m,1H), 4,62-4,49(m,1H), 3,60-3,39(m,2H), 3,13(s,3H), 2,42(s,3H), 1,40-1,37(m,3H) -Isomerengemisch | CDCl3 |
| 214 | 3,12 | | 7,61(d,1H), 7,07(d,1H), 4,42(q,1H), 3,32(q,2H), 2,72-2,70(m,1H), 2,49(s,3H), 1,34(d,3H), 1,06-0,97(m,4H) | CDCl3 |
| 215 | 2,15 | | 8,13-7,95(m,1H), 7,15-7,09(m,1H), 4,55-4,40(m,1H), 3,57-3,40(m,2H), 2,72-2,69(m,1H), 2,42(s,3H), 1,37-1,26(m,3H), 1,05-1,01(m,4H) | CDCl3 |
| 216 | 2,05 | | 7,86(d,1H), 7,17(d,1H), 3,45(q,2H), 3,14(s,3H), 2,44(s,3H), 1,47(s,3H), 1,41(s,3H) | CDCl3 |
| 217 | 3,11 | | 7,41(d,1H), 7,14(d,1H), 3,32(q,2H), 3,12(s,3H), 1,41(s,3H), 1,40(s,3H) | CDCl3 |

Die Bestimmung der Drehwerte erfolgte an einem Perkin Elmer 341, Seriennummer 9123, bei einer Wellenlänge von 589 nm und einer Temperatur von 20 °C, nach der nachfolgender Formel:

$$(spezifischer Drehwert\,\alpha)_D^{°C} = \frac{Drehwinkel\,\alpha * Lösungsvolumen(ml)}{Küvettenlänge(dm) * Einwaage(g)}$$

Die unten stehenden spezifischen Drehwerte sind als Durchschnitt aus 5 unterschiedlichen Messungen zu verstehen:

| | |
|---|---|
| 151 | 88,7 in $CHCl_3$ (c=0,009) |
| 197 | -89,1 in $CHCl_3$ (c=0,009) |

**Anwendungsbeispiele**

**Amblyomma hebaraeum -Test (AMBYHE)**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100% bei einer Aufwandmenge von 100ppm: 91

**Boophilus microplus - Diptest (BOOPMI Dip)**

Testtiere: Rinderzecken *(Boophilus microplus)* Stamm Parkhurst,SP-resistent
Lösungsmittel: Dimethylsulfoxid

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: 91

Bei diesem Test zeigen z.B. die folgenden Verbindungen der Herstellungsbeispiele eine Wirkung von 90% bei einer Aufwandmenge von 100ppm: 141

**Boophilus microplus-Iniektionstest (BOOPMI Inj)**

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1μl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20μg/Tier: 13, 26, 34, 35, 37, 47, 54, 91, 104, 106, 115, 141, 208, 209, 210, 211

Bei diesem Test zeigen z.B. die folgenden Verbindungen der Herstellungsbeispiele eine Wirkung von 80% bei einer Aufwandmenge von 20μg/Tier: 16, 48

**Meloidogyne incognita-Test (MELGIN)**

Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.
Gefäße werden mit Sand, Wirkstofflösung, einer Ei-Larven-Suspension des südlichen Wurzelgallenälchens *(Meloidogyne incognita)* und Salatsamen gefüllt. Die Salatsamen keimen und die Pflänzchen entwickeln sich. An den Wurzeln entwickeln sich die Gallen.
Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.
Bei diesem Test zeigen z. B. die folgenden Verbindungen der Herstellungsbeispiele Wirkung von 100% bei einer Aufwandmenge von 20ppm: 35, 36, 37, 38, 52, 83
Bei diesem Test zeigen z. B. die folgenden Verbindungen der Herstellungsbeispiele Wirkung von 90% bei einer Aufwandmenge von 20ppm: 13, 20, 24, 27, 28, 29, 30, 33, 34, 41, 42, 44, 45, 54, 69, 70, 74, 80, 81, 127, 177, 179, 181, 182, 208, 209, 210 (27, 28 und 44 nur als Referenz).

**Myzus persicae - Sprühtest (MYZUPE)**

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt. Nach 6 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 100% bei einer Aufwandmenge von 500g/ha: 141, 206

**Phaedon cochleariae - Sprühtest (PHAECO)**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.
Nach 7 Tagen wird die Wirkung in % bestimmt. Dabei bedeutet 100 %, dass alle Käferlarven abgetötet wurden; 0 % bedeutet, dass keine Käferlarven abgetötet wurden.
Bei diesem Test zeigen z. B. die folgenden Verbindungen der Herstellungsbeispiele Wirkung von 100% bei einer Aufwandmenge von 500g/ha: 52, 57, 84, 185, 186, 190, 200
Bei diesem Test zeigen z. B. die folgenden Verbindungen der Herstellungsbeispiele Wirkung von 83% bei einer

Aufwandmenge von 500g/ha: 60, 198, 211

**Spodoptera frugiperda - Sprühtest (SPODFR)**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.
Nach 7 Tagen wird die Wirkung in % bestimmt. Dabei bedeutet 100 %, dass alle Raupen abgetötet wurden; 0 % bedeutet, dass keine Raupe abgetötet wurde.
Bei diesem Test zeigen z. B. die folgenden Verbindungen der Herstellungsbeispiele Wirkung von 100% bei einer Aufwandmenge von 500g/ha: 157, 200 (157 nur als Referenz)
Bei diesem Test zeigen z. B. die folgenden Verbindungen der Herstellungsbeispiele Wirkung von 83% bei einer Aufwandmenge von 500g/ha: 207

(nur als Referenz) **Tetranychus urticae - Sprühtest, OP-resistent (TETRUR)**

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt. Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.
Bei diesem Test zeigen z. B. die folgenden Verbindungen der Herstellungsbeispiele eine Wirkung von 100% bei einer Aufwandmenge von 500g/ha: 2, 5, 8, 14, 15, 17, 18, 19, 20, 21, 23, 26, 27, 30, 32, 34, 35, 36, 37, 38, 39, 40, 42, 46, 48, 52, 53, 54, 58, 59, 65, 66, 67, 68, 76, 80, 83, 89, 90, 91, 92, 97, 101, 104, 106, 109, 113, 115, 116, 118, 121, 123, 124, 125, 127, 129, 130, 132, 133, 134, 139, 142, 143, 145, 147, 154, 159, 161, 171, 174, 175, 176, 178, 179, 180, 183, 184, 186, 191, 193, 198, 199, 201, 202, 204, 208, 209, 210,211,213,214,215 (27 und 66 nur als Referenz)
Bei diesem Test zeigen z. B. die folgenden Verbindungen der Herstellungsbeispiele eine Wirkung von 90% bei einer Aufwandmenge von 500g/ha: 1, 3, 6, 9, 10, 11, 12, 13, 22, 24, 25, 29, 31, 33, 41, 43, 47, 49, 50, 51, 55, 56, 61, 62, 63, 64, 72, 82, 85, 87, 98, 99, 105, 108, 111, 114, 117, 120, 122, 126, 131, 135, 136, 137, 138, 140, 141, 150, 151, 155, 156, 163, 164, 165, 166, 167, 168, 170, 172, 173, 177, 181, 185, 187, 188, 189, 190, 192, 193, 194, 195, 196, 200, 203, 205, 207, 212, 217 (43, 55, 85 und 207 nur als Referenz)
Bei diesem Test zeigen z. B. die folgenden Verbindungen der Herstellungsbeispiele eine Wirkung von 100% bei einer Aufwandmenge von 100g/ha: 81, 110, 112, 160, 162
Bei diesem Test zeigen z. B. die folgenden Verbindungen der Herstellungsbeispiele eine Wirkung von 90% bei einer Aufwandmenge von 100g/ha: 74, 79, 94, 169

**Tetranychus urticae- Sprühtest; OP-resistent (TETRUR)**

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*), die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele Wirkung von 100% bei einer Aufwandmenge von 20ppm: 77

**Patentansprüche**

1. Verbindung der Formel (I)

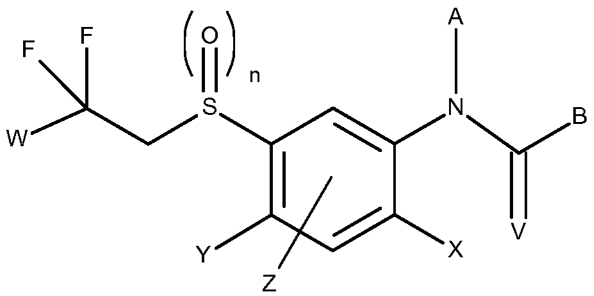

(I)

in welcher

A und B gemeinsam mit den Atomen, an die sie gebunden sind, für eine Substruktur stehen, die ausgewählt ist aus der Gruppe, bestehend aus

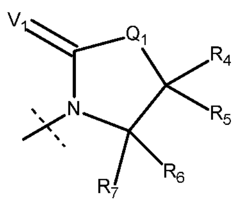

(I-A)

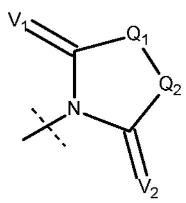

(I-B)

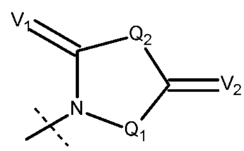

(I-C)

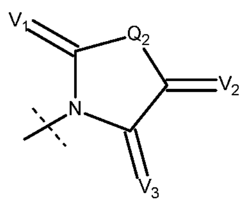

(I-D)

wobei
für den Fall, dass eine Substruktur gemäß Formel (I-A), (I-B) oder (I-D) vorliegt,

$V^1$, $V^2$ und $V^3$, jeweils unabhängig voneinander,
für Sauerstoff; Schwefel, einen gegebenenfalls substituierten Stickstoff oder Salze eines gegebenenfalls substituierten Stickstoffs stehen;

und für den Fall, dass eine Substruktur gemäß Formel (I-C) vorliegt,

$V^1$ und $V^2$, jeweils unabhängig voneinander, für Sauerstoff oder Schwefel stehen; für den Fall, dass eine Substruktur gemäß Formel (I-A), (I-B) oder (I-D) vorliegt, $Q^1$ und $Q^2$, jeweils unabhängig voneinander, für Sauerstoff, Schwefel oder einen gegebenenfalls substituierten Stickstoff stehen; oder für ein gegebenenfalls substituiertes Kohlenstoffatom stehen; mit der Maßgabe, dass $Q_1$ keinen Alkylcarbonylaminorest darstellt;

und für den Fall, dass eine Substruktur gemäß Formel (I-C) vorliegt,

$Q^1$ und $Q^2$, jeweils unabhängig voneinander, für Sauerstoff, Schwefel oder einen substituierten Stickstoff stehen; oder für ein gegebenenfalls substituiertes Kohlenstoffatom stehen; mit der Maßgabe, dass $Q_1$ keinen Alkylcarbonylaminorest darstellt;
$R^4$, $R^5$, $R^6$, und $R^7$, jeweils unabhängig voneinander, für Wasserstoff, Cyano, Halogen oder Nitro stehen; oder für Alkyl, Cycloalkylalkyl, Halogenalkyl, Cyanoalkyl, Hydroxyalkyl, Alkoxyalkyl, Aminoalkyl, Alkoxycarbonylalkyl, Alkylthioalkyl, Halogenalkylthioalkyl, Alkoxyalkylthioalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl oder Hetarylthioalkyl stehen, wobei die vorgenannten Reste jeweils gesättigt oder ungesättigt und/oder gegebenenfalls substituiert sein können; oder für gegebenenfalls substituiertes gesättigtes oder ungesättigtes Cycloalkyl, welches gegebenenfalls durch ein oder mehrere Heteroatome unterbrochen sein kann, stehen; oder für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder für gegebenenfalls substituiertes Phenyl oder gegebenenfalls substituiertes Hetaryl stehen; für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder
$R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können; oder
$R^6$ und $R^7$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können;
W für Wasserstoff oder Halogen steht;

X, Y und Z, jeweils unabhängig voneinander,
für Wasserstoff, Halogen, Hydroxy, Amino, Nitro, OCN, SCN, $SF_5$, stehen; oder
für Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl oder Dialkylaminothiocarbonyl stehen, wobei alle vorgenannten Reste gegebenenfalls substituiert sein können; oder
für Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy, Hetarylthio, Hetarylsulfinyl oder Hetarylsulfonyl stehen, wobei alle vorgenannten Reste gegebenenfalls substituiert sein können; oder
für Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl oder Cycloalkenyl stehen, wobei alle vorgenannten Reste jeweils gegebenenfalls substituiert sein können; oder
für NR'R" stehen,
wobei R' und R" unabhängig voneinander
für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl oder Alkoxycarbonyl stehen; oder
R' und R" gemeinsam mit dem N-Atom, an das sie gebunden sind, einen gegebenenfalls substituierten und gegebenenfalls durch ein oder mehrere Heteroatome, welche unabhängig ausgewählt werden aus der Gruppe, bestehend aus O, S und N, unterbrochenen gesättigten oder ungesättigten fünf- bis achtgliedrigen Ring bilden können; oder
für einen 3- bis 6-gliedrigen gesättigten, teilgesättigten oder aromatischen Ring, dergegebenenfalls ein bis drei Heteroatome, welche unabhängig ausgewählt werden aus der Gruppe, bestehend aus O, S und N, enthalten kann und der gegebenenfalls substituiert sein kann stehen;

und

n für die Zahl 0 oder 1 steht.

**2.** Verbindung nach Anspruch 1, in welcher

A und B gemeinsam mit den Atomen, an die sie gebunden sind, für eine Substruktur stehen, die ausgewählt ist aus der Gruppe, bestehend aus (I-A) bis (I-D),

wobei
für den Fall, dass eine Substruktur gemäß Formel (I-A), (I-B) oder (I-D) vorliegt,

$V^1$, $V^2$ und $V^3$, jeweils unabhängig voneinander,
für Sauerstoff; Schwefel, $NR^{11}$ oder ein Salz von $NR^{11}$ stehen;

und für den Fall, dass eine Substruktur gemäß Formel (I-C) vorliegt,

$V^1$ und $V^2$, jeweils unabhängig voneinander,
für Sauerstoff oder Schwefel stehen;
$Q^1$ für Sauerstoff, Schwefel, $NR^1$ oder $CR^2R^3$ steht;
$Q^2$ $NR^{10}$ oder $CR^8R^9$ steht;

für den Fall, dass eine Substruktur gemäß Formel (I-A), (I-B) oder (I-D) vorliegt,

$R^1$ für Wasserstoff, Cyano oder Nitro stehen; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl,

Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder

für gegebenenfalls substituiertes gesättigtes oder ungesättigtes Cycloalkyl, welches gegebenenfalls durch ein oder mehrere Heteroatome unterbrochen sein kann, steht; oder

für Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder

für gegebenenfalls substituiertes Phenyl oder gegebenenfalls substituiertes Hetaryl steht; oder

für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder

für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder

für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^{10}$ und $R^{11}$, jeweils unabhängig voneinander,

für Wasserstoff, Cyano oder Nitro stehen; oder

für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder

für gegebenenfalls substituiertes gesättigtes oder ungesättigtes Cycloalkyl, welches gegebenenfalls durch ein oder mehrere Heteroatome unterbrochen sein kann, steht; oder

für Alkylcarbonyl, Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder

für gegebenenfalls substituiertes Phenyl oder gegebenenfalls substituiertes Hetaryl steht; oder

für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder

für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder

für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht;

und für den Fall, dass eine Substruktur gemäß Formel (I-C) vorliegt,

$R^1$ für Cyano oder Nitro steht; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und
$R^{10}$ für Cyano oder Nitro steht; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl, Alkoxycarbonyl, Aryloxycarbonyl, Arylcarbamoyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Alkylcarbonyl, Halogenalkylcarbonyl, Hydroxyalkylcarbonyl, Alkoxyalkylcarbonyl, Phenylcarbonyl, Hetarylcarbonyl, Halogenalkoxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^2$, $R^3$, $R^4$, $R^5$, $R^6$, $R^7$, $R^8$ und $R^9$, jeweils unabhängig voneinander,

für Wasserstoff, Cyano, Halogen oder Nitro stehen; oder

für Alkyl, Cycloalkylalkyl, Halogenalkyl, Cyanoalkyl, Hydroxyalkyl, Alkoxyalkyl, Aminoalkyl, Alkoxycarbonylalkyl, Alkylthioalkyl, Halogenalkylthioalkyl, Alkoxyalkylthioalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl oder Hetarylthioalkyl stehen, wobei die vorgenannten Reste jeweils gesättigt oder ungesättigt und/oder gegebenenfalls substituiert sein können; oder

für gegebenenfalls substituiertes gesättigtes oder ungesättigtes Cycloalkyl, welches gegebenenfalls durch ein oder mehrere Heteroatome unterbrochen sein kann, stehen; oder

für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder

für gegebenenfalls substituiertes Phenyl oder gegebenenfalls substituiertes Hetaryl stehen;

für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder

für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder

für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder

$R^2$ und $R^3$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können; oder

$R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können; oder

$R^6$ und $R^7$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können; oder

$R^8$ und $R^9$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei-bis achtgliedrigen Ring bilden können;

W für Wasserstoff oder Halogen steht;

X, Y und Z die in Anspruch 1 genannten Bedeutungen haben; und

n für die Zahl 0 oder 1 steht.

**3.** Verbindung nach Anspruch 1 oder 2, in welcher

A und B gemeinsam mit den Atomen, an die sie gebunden sind, für eine Substruktur stehen, die ausgewählt ist aus der Gruppe, bestehend aus (I-A) bis (I-D),

wobei für den Fall, dass eine Substruktur gemäß Formel (I-A), (I-B) oder (I-D) vorliegt,

$V^1$, $V^2$ und $V^3$, jeweils unabhängig voneinander,
für Sauerstoff; Schwefel, $NR^{11}$ oder ein Salz von $NR^{11}$ stehen;

und für den Fall, dass eine Substruktur gemäß Formel (I-C) vorliegt,

$V^1$ und $V^2$, jeweils unabhängig voneinander,
für Sauerstoff oder Schwefel stehen;
$Q^1$ für Sauerstoff, Schwefel, $NR^1$ oder $CR^2R^3$ steht;
$Q^2$ $NR^{10}$ oder $CR^8R^9$ steht;

für den Fall, dass eine Substruktur gemäß Formel (I-A), (I-B) oder (I-D) vorliegt,

$R^1$ für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl

an Kohlenstoffatomen 2 beträgt, die Reste gesättigt oder ungesättigt sein können oder für Sulfanyl steht; und $R^{10}$ und $R^{11}$, jeweils unabhängig voneinander,

für Wasserstoff, Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht;

und für den Fall, dass eine Substruktur gemäß Formel (I-C) vorliegt,

$R^1$ für Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an

Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder

für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{10}$ für Cyano oder Nitro steht; oder

für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, ($C_1$-$C_6$)Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder

Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino,

($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, $R^5$, $R^6$, $R^7$, $R^8$ und $R^9$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylthio($C_1$-$C_6$)alkyl, Halogen($C_1$-$C_6$)alkylthio($C_1$-$C_6$)alkyl, ($C_1$-$C_6$)Alkoxy($C_1$-$C_6$)alkylthioalkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert

sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;-oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können oder

$R^6$ und $R^7$ gemeinsam mit dem Atom, an das sie gebunden sind-einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;-oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3\text{-}C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Halogen steht;

X, Y und Z die in den Ansprüchen 1 oder 2 genannten Bedeutungen haben; und

n für die Zahl 0 oder 1 steht.

**4.** Verbindung nach einem der Ansprüche 1 bis 3, in welcher

A und B gemeinsam mit den Atomen, an die sie gebunden sind, für eine Substruktur der Formel (I-A) stehen

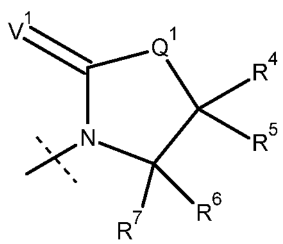

(I-A)

wobei

$V^1$ für Sauerstoff; Schwefel, $NR_{11}$ oder ein Salz von $NR^{11}$ steht;

$Q^1$ für Sauerstoff, Schwefel, $NR^1$ oder $CR^2R^3$ steht;

$R^1$ für Wasserstoff, Cyano oder Nitro steht; oder

für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, Hydroxy$(C_1\text{-}C_6)$alkyl, $(C_1\text{-}C_6)$Alkoxy$(C_1\text{-}C_6)$alkyl, Amino$(C_1\text{-}C_6)$alkyl, $(C_1\text{-}C_6)$Alkoxycarbonyl$(C_1\text{-}C_6)$alkyl, $(C_1\text{-}C_6)$Alkylsulfanyl$(C_1\text{-}C_6)$alkyl, $(C_1\text{-}C_6)$Alkylsulfinyl$(C_1\text{-}C_6)$alkyl, $(C_1\text{-}C_6)$Alkylsulfonyl$(C_1\text{-}C_6)$alkyl, Halogen$(C_1\text{-}C_6)$alkylsulfanyl$(C_1\text{-}C_6)$alkyl, Halogen$(C_1\text{-}C_6)$alkylsulfinyl$(C_1\text{-}C_6)$alkyl, Halogen$(C_1\text{-}C_6)$alkylsulfonyl$(C_1\text{-}C_6)$alkyl, $(C_1\text{-}C_6)$Alkoxy$(C_1\text{-}C_6)$alkylsulfanyl$(C_1\text{-}C_6)$alkyl, $(C_1\text{-}C_6)$Alkoxy$(C_1\text{-}C_6)$alkylsulfinyl$(C_1\text{-}C_6)$alkyl, $(C_1\text{-}C_6)$Alkoxy$(C_1\text{-}C_6)$alkylsulfonyl$(C_1\text{-}C_6)$alkyl, Phenyl$(C_1\text{-}C_6)$alkyl, Phenoxy$(C_1\text{-}C_6)$alkyl, Phenylsulfanyl$(C_1\text{-}C_6)$alkyl, Phenylsulfinyl$(C_1\text{-}C_6)$alkyl, Phenylsulfonyl$(C_1\text{-}C_6)$alkyl, Hetaryl$(C_1\text{-}C_6)$alkyl, Hetaryloxy$(C_1\text{-}C_6)$alkyl, Hetarylsulfanyl$(C_1\text{-}C_6)$alkyl, Hetarylsulfinyl$(C_1\text{-}C_6)$alkyl, Hetarylsulfonyl$(C_1\text{-}C_6)$alkyl, $(C_1\text{-}C_6)$Alkoxycarbonyl, Aryloxycarbo-

nyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^{11}$ für Wasserstoff, Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy

steht, wobei die vorgenannten Reste jeweils gegebenenfalls substiuiert sein können, oder für Hydroxy steht; oder für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, $R^5$, $R^6$ und $R^7$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylthio($C_1$-$C_6$)alkyl, Halogen($C_1$-$C_6$)alkylthio($C_1$-$C_6$)alkyl, ($C_1$-$C_6$)Alkoxy($C_1$-$C_6$)alkylthioalkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl,

Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^6$ und $R^7$ gemeinsam mit dem Atom, an das sie gebunden sind-einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

W für Wasserstoff, Fluor oder Chlor steht;

X, Y und Z unabhängig voneinander für Wasserstoff, Fluor, Chlor, Brom, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**5.** Verbindung nach Anspruch 4, in welcher die Substruktur der Formel (I-A) für eine Substruktur steht, welche ausgewählt wird aus der Gruppe, bestehend aus

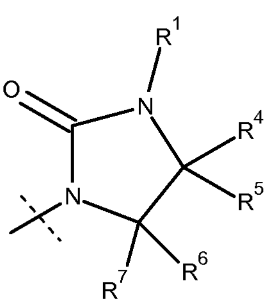
(I-A-1)

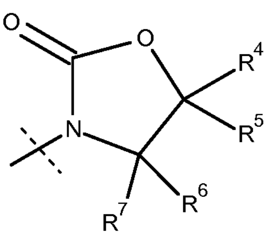
(I-A-2)

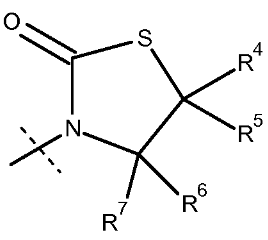
(I-A-3)

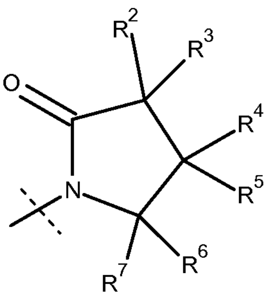

(I-A-4)

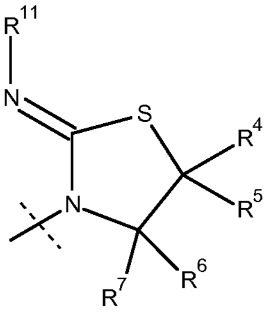

(I-A-5)

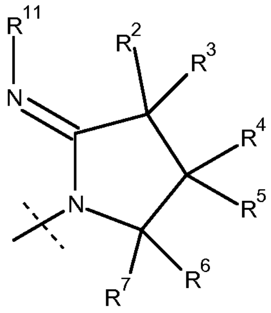

(I-A-6)

wobei

$R^1$ für Wasserstoff steht; oder

für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, $(C_1\text{-}C_6)$Alkoxy$(C_1\text{-}C_6)$alkyl, $(C_1\text{-}C_6)$Alkoxycarbonyl$(C_1\text{-}C_6)$alkyl, Phenyl$(C_1\text{-}C_6)$alkyl, Hetaryl$(C_1\text{-}C_6)$alkyl, $(C_2\text{-}C_6)$Alkenyl, Halogen$(C_2\text{-}C_6)$Alkenyl, $(C_3\text{-}C_6)$Cycloalkenyl$(C_1\text{-}C_6)$alkyl, $(C_2\text{-}C_6)$Alkinyl, wobei die vorgenannten Reste jeweils gegebenenfalls substituiert sein können steht; oder

für gegebenenfalls substituiertes $(C_3\text{-}C_6)$Cycloalkyl, $(C_3\text{-}C_6)$Cycloalkenyl, welches gegebenenfalls durch ein oder mehrere Heteroatome aus der Reihe O, S oder N unterbrochen sein kann, steht; oder

für Halogen$(C_1\text{-}C_6)$alkylcarbonyl, $(C_1\text{-}C_6)$Alkoxy$(C_1\text{-}C_6)$alkylcarbonyl, $(C_1\text{-}C_6)$Alkoxycarbonyl, Halogen$(C_1\text{-}C_6)$alkoxycarbonyl, Aminocarbonyl, $(C_1\text{-}C_6)$Alkylaminocarbonyl, Di$(C_1\text{-}C_6)$alkylaminocarbonyl, $(C_3\text{-}C_6)$Cycloalkylaminocarbonyl, $(C_1\text{-}C_6)$Alkylaminothiocarbonyl, Di$(C_1\text{-}C_6)$alkylaminothiocarbonyl, $(C_2\text{-}C_6)$Alkenylcarbonyl, $(C_3\text{-}C_6)$Cycloalkenyl$(C_1\text{-}C_6)$alkylcarbonyl, $(C_2\text{-}C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1\text{-}C_6)$Alkoxy, Halogen$(C_1\text{-}C_6)$alkoxy, $(C_3\text{-}C_6)$Cycloalkyloxy, oder Carbonyloxy steht, wobei die vorgenannten Reste jeweils gegebenenfalls substiuiert sein können, oder für Hydroxy steht; oder

für $(C_1\text{-}C_6)$Alkylamino, Halogen$(C_1\text{-}C_6)$alkylamino, Dihalogen$(C_1\text{-}C_6)$alkylamino, Di$(C_1\text{-}C_6)$alkylamino, $(C_3\text{-}C_6)$Cycloalkylamino, Di$(C_3\text{-}C_6)$cycloalkylamino, $(C_1\text{-}C_6)$Alkylcarbonylamino, $(C_1\text{-}C_6)$Alkoxycarbonylamino, $(C_1\text{-}C_6)$Alkylcarbamoylamino, $(C_1\text{-}C_6)$Alkylsulfonylamino, $(C_2\text{-}C_6)$Alkenylamino, $(C_3\text{-}C_6)$Cycloalkenyl$(C_1\text{-}C_6)$alkylamino, $(C_2\text{-}C_6)$Alkinylamino steht, wobei die vorgenannten Reste jeweils gegebenenfalls substituiert sein können oder Amino steht; oder

für $(C_1\text{-}C_6)$Alkylsulfanyl, $(C_1\text{-}C_6)$Alkylsulfinyl, $(C_1\text{-}C_6)$Alkylsulfonyl, Halogen$(C_1\text{-}C_6)$alkylsulfanyl, Halogen$(C_1\text{-}C_6)$alkylsulfinyl, Halogen$(C_1\text{-}C_6)$alkylsulfanyl, $(C_3\text{-}C_6)$Cycloalkyl$(C_1\text{-}C_6)$alkylsulfanyl, $(C_3\text{-}C_6)$Cycloalkyl$(C_1\text{-}C_6)$alkylsulfinyl, $(C_3\text{-}C_6)$Cycloalkyl$(C_1\text{-}C_6)$alkylsulfonyl, Aminosulfonyl, $(C_1\text{-}C_6)$Alkylaminosulfonyl, Di$(C_1\text{-}C_6)$alkylaminosulfonyl oder Arylaminosulfonyl steht, wobei die vorgenannten Reste jeweils gegebenenfalls substituiert sein können oder für Sulfanyl steht; und

$R^{11}$ für Wasserstoff steht; oder

für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, $(C_1\text{-}C_6)$Alkoxy$(C_1\text{-}C_6)$alkyl, $(C_1\text{-}C_6)$Alkoxycarbonyl$(C_1\text{-}C_6)$alkyl, Phenyl$(C_1\text{-}C_6)$alkyl, Hetaryl$(C_1\text{-}C_6)$alkyl, $(C_2\text{-}C_6)$Alkenyl, Halogen$(C_2\text{-}C_6)$Alkenyl, $(C_3\text{-}C_6)$Cycloalkenyl$(C_1\text{-}C_6)$alkyl, $(C_2\text{-}C_6)$Alkinyl, wobei die vorgenannten Reste jeweils gegebenenfalls substituiert sein können steht; oder

für gegebenenfalls substituiertes $(C_3\text{-}C_6)$Cycloalkyl, $(C_3\text{-}C_6)$Cycloalkenyl, welches gegebenenfalls durch ein oder mehrere Heteroatome aus der Reihe O, S oder N unterbrochen sein kann, steht; oder

für $(C_1\text{-}C_6)$Alkylcarbonyl, Halogen$(C_1\text{-}C_6)$alkylcarbonyl, $(C_1\text{-}C_6)$Alkoxy$(C_1\text{-}C_6)$alkylcarbonyl, $(C_1\text{-}C_6)$Alkoxycarbonyl, Halogen$(C_1\text{-}C_6)$alkoxycarbonyl, Aminocarbonyl, $(C_1\text{-}C_6)$Alkylaminocarbonyl, Di$(C_1\text{-}C_6)$alkylaminocarbonyl, $(C_3\text{-}C_6)$Cycloalkylaminocarbonyl, $(C_1\text{-}C_6)$Alkylaminothiocarbonyl, Di$(C_1\text{-}C_6)$alkylaminothiocarbonyl, $(C_2\text{-}C_6)$Alkenylcarbonyl, $(C_3\text{-}C_6)$Cycloalkenyl$(C_1\text{-}C_6)$alkylcarbonyl, $(C_2\text{-}C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1\text{-}C_6)$Alkoxy, Halogen$(C_1\text{-}C_6)$alkoxy, $(C_3\text{-}C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, , $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^2$, $R^3$, $R^4$, $R^5$, $R^6$ und $R^7$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Phenyl$(C_1-C_6)$alkyl, , Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können oder für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl stehen;

für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;oder

$R^4$ und $R^5$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;oder

$R^6$ und $R^7$ gemeinsam mit dem Atom, an das sie gebunden sindeinen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;oder

W für Wasserstoff oder Fluor steht;
X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;
Z für Wasserstoff steht; und
n für die Zahl 0 oder 1 steht.

**6.** Verbindung nach Anspruch 4 oder 5, in welcher die Substruktur der Formel (I-A) für eine Substruktur steht, welche ausgewählt wird aus der Gruppe, bestehend aus

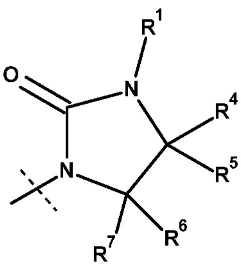
(I-A-1)

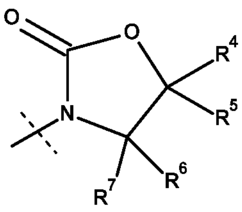
(I-A-2)

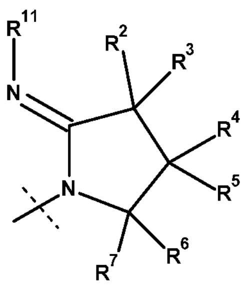
(I-A-6)

wobei

$R^1$ und $R^{11}$, jeweils unabhängig voneinander,

für Wasserstoff, Methyl, Ethyl, Propyl, Isopropyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$ stehen; oder

$R^2$, $R^3$, $R^4$, $R^5$, $R^6$ und $R^7$, jeweils unabhängig voneinander für Wasserstoff, Methyl, Trifluoromethyl oder Phenyl stehen;
W für Wasserstoff oder Fluor steht;
X für Wasserstoff, Chlor, Fluor oder Methyl steht;
Y für Chlor, Brom, Methyl, Trifluoromethyl oder Fluor steht;
insbesondere wobei X und Y für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht; und
n für die Zahl 0 oder 1 steht.

**7.** Verbindung nach einem der Ansprüche 1 bis 3, in welcher

A und B gemeinsam mit den Atomen, an die sie gebunden sind, für eine Substruktur der Formel (I-B) stehen

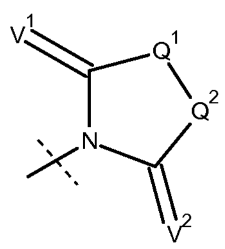
(I-B)

wobei

$V^1$ und $V^2$, jeweils unabhängig voneinander,
für Sauerstoff; Schwefel, $NR^{11}$ oder ein Salz von $NR^{11}$ stehen;
$Q^1$ für Sauerstoff, Schwefel, $NR^1$ oder $CR^2R^3$ steht;

$Q^2$ $NR^{10}$ oder $CR^8R^9$ steht;
$R^1$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$AlkylcarbonylaminoArylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und
$R^{10}$ und $R^{11}$, jeweils unabhängig voneinander,
für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)AlkylcarbonylaminoArylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^8$ und $R^9$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylthio($C_1$-$C_6$)alkyl, Halogen($C_1$-$C_6$)alkylthio($C_1$-$C_6$)alkyl, ($C_1$-$C_6$)Alkoxy($C_1$-$C_6$)alkylthioalkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino,

($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)AlkylcarbonylaminoArylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z unabhängig voneinander für Wasserstoff, Fluor, Chlor, Brom, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

8. Verbindung nach Anspruch 7, in welcher die Substruktur der Formel (I-B) für eine Substruktur steht, welche ausgewählt wird aus der Gruppe, bestehend aus

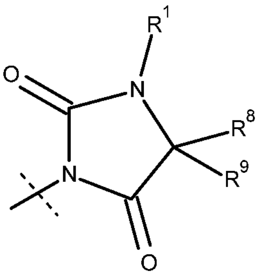

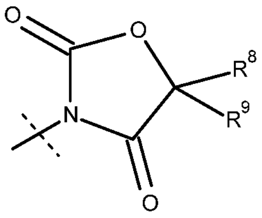

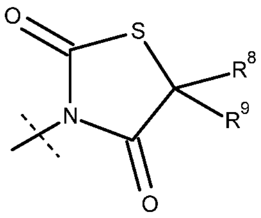

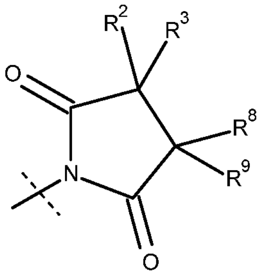

(I-B-1) (I-B-2) (I-B-3) (I-B-4)

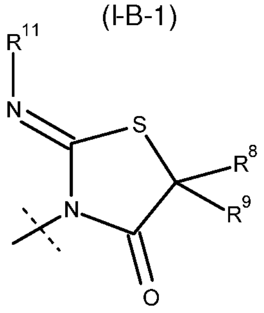

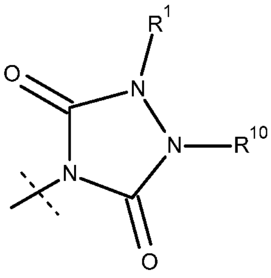

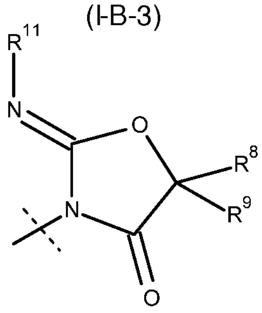

(I-B-5) (I-B-6) (I-B-7)

wobei

$R^1$ für Wasserstoff steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino$(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^{10}$ und $R^{11}$, jeweils unabhängig voneinander,

für Wasserstoff steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein

oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; und

$R^2$, $R^3$, $R^8$ und $R^9$, jeweils unabhängig voneinander,

für Wasserstoff, Cyano, Halogen oder Nitro steht; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können ; oder

für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy stehen; oder

für ($C_1$-$C_6$)Alkylamino, Halogen($C_1$-$C_6$)alkylamino, Dihalogen(Ci-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl,

Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;
W für Wasserstoff oder Fluor steht;
X und Y, unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;
Z für Wasserstoff steht; und
n für die Zahl 0 oder 1 steht.

**9.** Verbindung nach Anspruch 7 oder 8, in welcher die Substruktur der Formel (I-B) für eine Substruktur steht, welche ausgewählt wird aus der Gruppe, bestehend aus

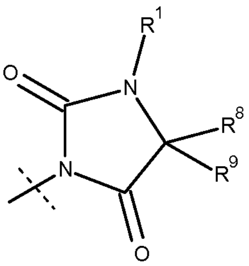
(I-B-1)

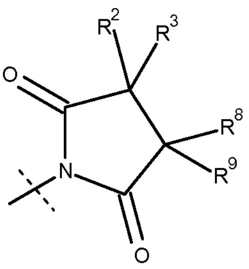
(I-B-4)

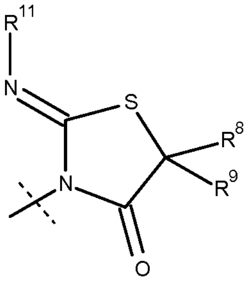
(I-B-5)

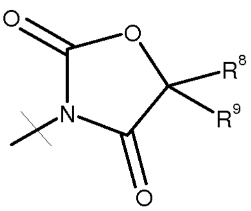
(I-B-2)

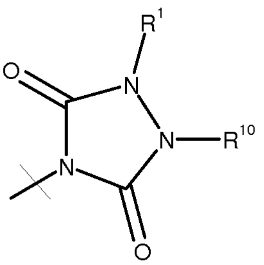
(I-B-6)

wobei

$R^1$, $R^{10}$ und $R^{11}$, jeweils unabhängig voneinander,
für Wasserstoff, Methyl, Ethyl, Propyl, Isopropyl, n-Butyl, Cyclopropyl, Cyclopropylmethyl, $CH_2CF_3$, $CH_2CHF_2$, $CH(CH_3)CF_3$, $CH_2CH_2CF_3$, $CH_2CH_2CHF_2$, $CH_2C(CH_3)_3$, $CH_2CH(CH_3)_2$, $CH_2CH{=}CH_2$ (= Allyl), $CH_2CCH$ oder Benzyl stehen;
$R^2$, $R^3$, $R^8$ und $R^9$, jeweils unabhängig voneinander,
für Wasserstoff, Methyl, Trifluoromethyl oder Phenyl stehen;
W für Wasserstoff oder Fluor steht;
X für Wasserstoff, Chlor, Fluor oder Methyl steht;
Y für Chlor, Brom, Methyl, Trifluoromethyl oder Fluor steht;
X und Y insbesondere für folgende Kombinationen (Y,X) stehen: (Me, F), (Me,H), (Me,Cl), (Me,Me), (Cl,Cl), (Cl,F), (Cl,H), (Br,H), (Br,F), (F,F), ($CF_3$,H);
Z für Wasserstoff steht; und
n für die Zahl 0 oder 1 steht.

**10.** Verbindung nach einem der Ansprüche 1 bis 3, in welcher

A und B gemeinsam mit den Atomen, an die sie gebunden sind, für eine Substruktur der Formel (I-C) stehen

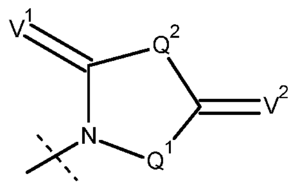

(I-C)

wobei

$V^1$ und $V^2$, jeweils unabhängig voneinander, für Sauerstoff oder Schwefel stehen;
$Q^1$ für Sauerstoff, Schwefel, $NR_1$ oder $CR_2R_3$ steht;
$Q^2$ $NR_{10}$ oder $CR_8R_9$ steht;
$R^1$ für Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, ($C_1$-$C_6$)Alkoxycarbonyl, Aryloxycarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)AlkylcarbonylaminoArylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl

an Kohlenstoffatomen 2 beträgt, die Reste gesättigt oder ungesättigt sein können oder für Sulfanyl steht; und $R^{10}$ für Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^8$ und $R^9$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylthio$(C_1-C_6)$alkyl, Halogen$(C_1-C_6)$alkylthio$(C_1-C_6)$alkyl, $(C_1-C_6)$Alkoxy$(C_1-C_6)$alkylthioalkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl oder Hetarylthio$(C_1-C_6)$alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$al-

kylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z, jeweils unabhängig voneinander,

für Wasserstoff, Fluor, Chlor, Brom, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**11.** Verbindung nach Anspruch 10, in welcher die Substruktur der Formel (I-C) für eine Substruktur steht, welche ausgewählt wird aus der Gruppe, bestehend aus

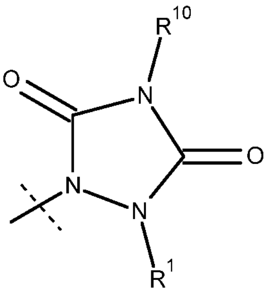

(I-C-1)

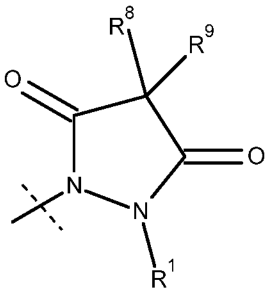

(I-C-2)

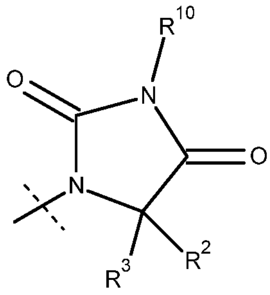

(I-C-3)

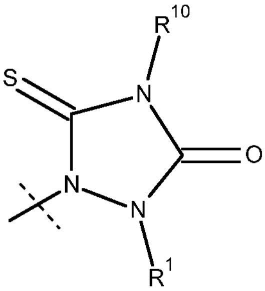

(I-C-4)

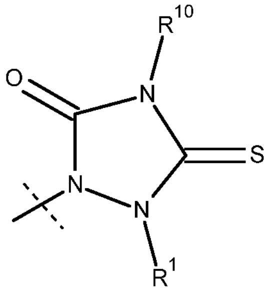

(I-C-5)

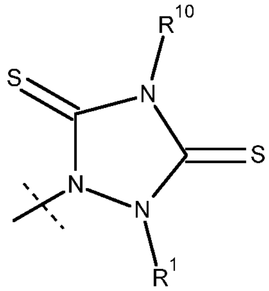

(I-C-6)

wobei

$R^1$ für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino$(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^{10}$ für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils

gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^2$, $R^3$, $R^8$ und $R^9$, jeweils unabhängig voneinander,

für Wasserstoff, Cyano, Halogen oder Nitro steht; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; oder

$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;
W für Wasserstoff oder Fluor steht;
X und Y, jeweils unabhängig voneinander,
für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;
Z für Wasserstoff steht; und
n für die Zahl 0 oder 1 steht.

**12.** Verbindung nach Anspruch 10 oder 11, in welcher die Substruktur der Formel (I-C) für eine Substruktur steht, welche ausgewählt wird aus der Gruppe, bestehend aus

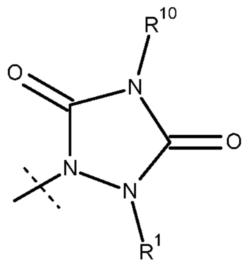
(I-C-1)

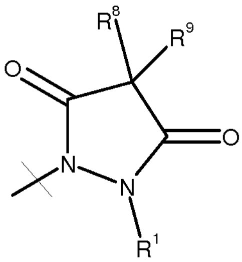
(I-C-2)

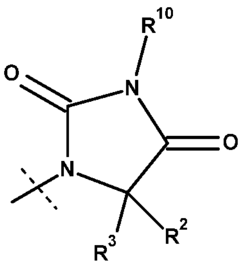
(I-C-3)

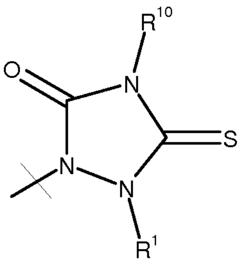
(I-C-5)

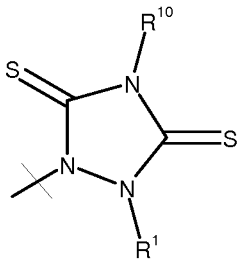
(I-C-6)

wobei

$R^1$ und $R^{10}$, jeweils unabhängig voneinander,
für $(C_1-C_4)$Alkyl, $(C_2-C_4)$Halogenalkyl, $(C_1-C_3)$Alkoxy$(C_1-C_4)$alkyl oder $(C_2-C_4)$Alkenyl stehen; oder
für gesättigtes oder ungesättigtes $(C_3-C_6)$Cycloalkyl, $(C_3-C_6)$Cycloalkyl$(C_1-C_2)$alkyl, Phenyl oder Phenyl$(C_1-C_2)$alkyl stehen, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können;
$R^2$, $R^3$, $R^8$ und $R^9$, jeweils unabhängig voneinander,
für Wasserstoff stehen; oder
für $(C_1-C_6)$Alkyl, $(C_2-C_6)$Alkenyl, $(C_2-C_6)$Alkinyl, Halogen$(C_1-C_6)$alkyl oder Phenyl$(C_1-C_3)$alkyl steht, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können; oder
für $(C_3-C_6)$Cycloalkyl oder Phenyl steht, wobei die vorgenannten Reste jeweils gegebenenfalls einfach oder mehrfach durch Halogen, Cyano, Nitro, Amino, Hydroxy,Methyl, Ethyl, Trifluoromethyl, Difluoromethyl, Trifluoroethyl, Difluoroethyl, Methoxy, Ethoxy, Trifluoromethoxy, Trifluoroethoxy, Trifluoroethylsulfanyl oder gegebenenfalls durch Methyl, Fluor, Chlor, Cyano substituiertes Cyclopropyl substituiert sein können; oder
$R^2$ und $R^3$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituiertes Cyclopropyl-, Cyclobutyl-, Cyclopentyl- oder Cyclohexylring bilden können; oder
$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes $(C_3-C_6)$Cycloalkyl, substituiertes Cyclopropyl-, Cyclobutyl-, Cyclopentyl- oder Cyclohexylring bilden können;
W Wasserstoff oder Fluor steht;
X für Wasserstoff, Chlor, Fluor oder Methyl steht;
Y für Chlor, Brom, Methyl, Trifluoromethyl oder Fluor steht; Insbesondere wobei X und Y für folgende Kombinationen (Y,X) stehen: (Me, F), (Me,H), (Me,Cl), (Me,Me), (Cl,Cl), (Cl,F), (Cl,H), (Me,H), (Br,H), (Br,F), (F,F), $(CF_3,H)$;
Z für Wasserstoff steht; und
n für die Zahl 0 oder 1 steht.

**13.** Verbindung nach einem der Ansprüche 1 bis 3, in welcher

A und B gemeinsam mit den Atomen, an die sie gebunden sind, für eine Substruktur der Formel (I-D) stehen

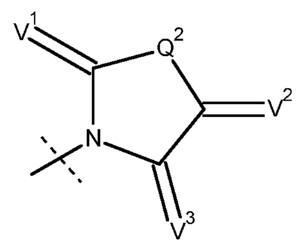

(I-D)

wobei

$V^1$, $V^2$ und $V^3$, jeweils unabhängig voneinander,
für Sauerstoff; Schwefel, $NR_{11}$ oder ein Salz von $NR_{11}$ stehen;
$Q^2$ $NR^{10}$ oder $CR^8R^9$ steht;
$R^{10}$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, Arylcarbonyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alky-

laminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht;

$R^{11}$ für Wasserstoff, Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, $(C_1-C_6)$Alkoxycarbonyl, Aryloxycarbonyl, $(C_1-C_6)$Alkylcarbonyl, Arylcarbamoyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halo-

gen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^8$ und $R^9$, jeweils unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylthio($C_1$-$C_6$)alkyl, Halogen($C_1$-$C_6$)alkylthio($C_1$-$C_6$)alkyl, ($C_1$-$C_6$)Alkoxy($C_1$-$C_6$)alkylthioalkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl oder Hetarylthio($C_1$-$C_6$)alkyl stehen, wobei die vorgenannten Reste jeweils gesättigt oder ungesättigt und/oder gegebenenfalls substituiert sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen;

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff, Fluor oder Chlor steht;

X, Y und Z, jeweils unabhängig voneinander,

für Wasserstoff, Fluor, Chlor, Brom, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**14.** Verbindung nach Anspruch 13, in welcher die Substruktur der Formel (I-D) für eine Substruktur steht, welche ausgewählt wird aus der Gruppe, bestehend aus

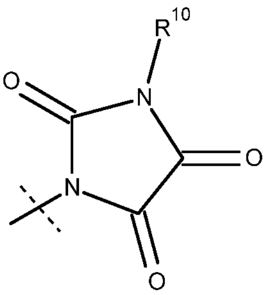
(I-D-1)

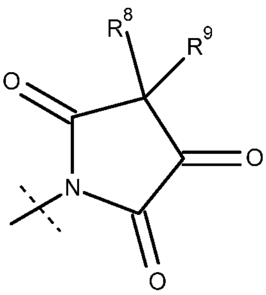
(I-D-2)

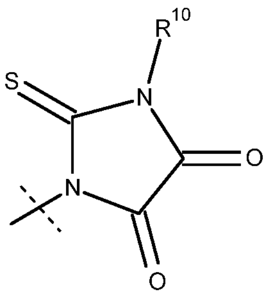
(I-D-3)

wobei

$R^{10}$ für Wasserstoff steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^8$ und $R^9$, jeweils unabhängig voneinander,

für Wasserstoff, Cyano, Halogen oder Nitro steht; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl, wobei die vorgenannten Reste jeweils gegebenenfalls substituiert sein können ; oder

für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy stehen; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; oder

$R^8$ und $R^9$ gemeinsam mit dem Atom, an das sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;

W für Wasserstoff oder Fluor steht;

X und Y, jeweils unabhängig voneinander, für Wasserstoff, Fluor, Chlor, Brom, Methyl, Ethyl, Trifluoromethyl, (2,2)-Difluoromethyl, Difluoromethoxy, Trifluoromethoxy, Cyclopropyl, Amino, Hydroxy oder Nitro stehen;

Z für Wasserstoff steht; und

n für die Zahl 0 oder 1 steht.

**15.** Verbindung nach Anspruch 13 oder 14, in welcher die Substruktur der Formel (I-D) für eine Substruktur steht, welche ausgewählt wird aus der Gruppe, bestehend aus

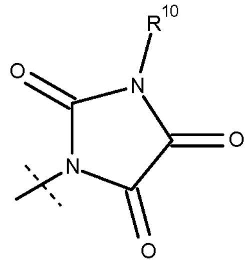

(I-D-1)

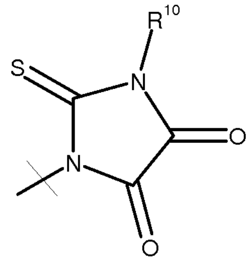

(I-D-3)

wobei

$R^{10}$ für Wasserstoff, Methyl, Ethyl, Propyl, Isopropyl, tert-Butyl, Cyclopropylmethyl, $CH_2CH(CH_3)_2$, $CH_2C(CH_3)_3$, $CH_2CF_3$, $CH_2CHF_2$, $CH(CH_3)CF_3$, $CH_2CH_2CF_3$, $CH_2CH_2CHF_2$ oder gegebenenfalls durch Methyl, Fluor, Chlor, Cyano substituiertes Cyclopropyl oder gegebenenfalls durch Methyl, Fluor, Chlor, Cyano substituiertes Cyclohexyl oder gegebenenfalls durch Methyl, Fluor, Chlor, Cyano substituiertes Cyclopropylmethyl steht;
W für Wasserstoff oder Fluor steht;
X für Wasserstoff, Chlor, Fluor oder Methyl steht;
Y für Chlor, Brom, Methyl, Trifluoromethyl oder Fluor steht;
Insbesondere wobei X und Y für folgende Kombinationen (Y,X) stehen: (Me, F), (Me,H), (Me,Cl), (Me,Me), (Cl,Cl), (Cl,F), (Cl,H), (Me,H), (Br,H), (Br,F), (F,F), ($CF_3$,H);
Z für Wasserstoff steht; und
n für die Zahl 0 oder 1 steht.

**16.** Wirkstoffzusammensetzung enthaltend mindestens eine Verbindung der allgemeinen Formel (I) gemäß einem der Ansprüche 1 bis 15 und mindestens einen weiteren insektiziden, akariziden oder nematiziden Wirkstoff ausgewählt aus der Gruppe bestehend aus

(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifosmethyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
Cyclodien-organochlorine, z.B. Chlordane und Endosulfan; oder
Phenylpyrazole (Fiprole), z.B. Ethiprole und Fipronil.
(3) Natrium-Kanal-Modulatoren / Spannungsabhängige Natrium-Kanal-Blocker, wie beispielsweise
Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder
DDT; oder Methoxychlor.
(4) Nikotinerge Acetylcholin-Rezeptor (nAChR) Agonisten, wie beispielsweise
Neonikotinoide, z.B. Acetamiprid, Clothianidin, Dinotefuran, Imidacloprid, Nitenpyram, Thiacloprid und Thiamethoxam; oder
Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
Avermectine/Milbemycine, z.B. Abamectin, Emamectin-benzoat, Lepimectin und Milbemectin.
(7) Juvenilhormon-Imitatoren, wie beispielsweise
Juvenilhormon-Analoge, z.B. Hydroprene, Kinoprene und Methoprene; oder
Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder Etoxazole.

(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, CrylFa, Cry2Ab, mCry3A, Cry3Ab, Cry3Bb, Cry34/35Abl.

(12) Inhibitoren der oxidativen Phosphorylierung, ATP-Disruptoren, wie beispielsweise Diafenthiuron; oder Organozinnverbindungen, z.B. Azocyclotin, Cyhexatin und Fenbutatin-oxid; oder
Propargite; oder Tetradifon.

(13) Entkoppler der oxidativen Phoshorylierung durch Unterbrechung des H-Protongradienten, wie beispielsweise Chlorfenapyr, DNOC und Sulfluramid.

(14) Nikotinerge Acetylcholin-Rezeptor-Antagonisten, wie beispielsweise Bensultap, Cartaphydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsstörende Wirkstoffe, Dipteran, wie beispielsweise Cyromazine.

(18) Ecdyson-Rezeptor Agonisten, wie beispielsweise Chromafenozide, Halofenozide, Methoxyfenozide und Tebufenozide.

(19) Oktopaminerge Agonisten, wie beispielsweise Amitraz.

(20) Komplex-III-Elektronentransportinhibitoren, wie beispielsweise Hydramethylnon; oder Acequinocyl; oder Fluacrypyrim.

(21) Komplex-I-Elektronentransportinhibitoren, beispielsweise
METI-Akarizide, z.B. Fenazaquin, Fenpyroximate, Pyrimidifen, Pyridaben, Tebufenpyrad und Tolfenpyrad; oder Rotenone (Derris).

(22) Spannungsabhängige Natriumkanal-Blocker, z.B. Indoxacarb; oder Metaflumizone.

(23) Inhibitoren der Acetyl-CoA-Carboxylase, wie beispielsweise
Tetron- und Tetramsäurederivate, z.B. Spirodiclofen, Spiromesifen und Spirotetramat.

(24) Komplex-IV-Elektronentransportinhibitoren, wie beispielsweise Phosphine, z.B. Aluminiumphosphid, Calciumphosphid, Phosphin und Zinkphosphid; oder Cyanid.

(25) Komplex-II-Elektronentransportinhibitoren, wie beispielsweise Cyenopyrafen.

(28) Ryanodinrezeptor-Effektoren, wie beispielsweise
Diamide, z.B. Chlorantraniliprole und Flubendiamide;

weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:

3-Brom-N-{2-brom-4-chlor-6-[(1-cyclopropylethyl)carbamoyl]phenyl}-1-(3-chlorpyridin-2-yl)-1H-pyrazol-5-carboxamid 4-{[(6-Brompyrid-3-yl)methyl](2-fluorethyl)amino}furan-2(5H)-on, 4-{[(6-Fluorpyrid-3-yl)methyl](2,2-difluorethyl)amino}furan-2(5H)-on, 4-{[(2-Chlor-1,3-thiazol-5-yl)methyl](2-fluorethyl)aminolfuran-2(5H)-on, 4-{[(6-Chlorpyrid-3-yl)methyl](2-fluorethyl)amino}furan-2(5H)-on, Flupyradifurone, 4-{[(6-Chlor-5-fluorpyrid-3-yl)methyl](methyl)amino }furan-2(5H)-on, 4-{[(5,6-Dichlorpyrid-3-yl)methyl](2-fluorethyl)amino}furan-2(5H)-on, 4-{[(6-Chlor-5-fluorpyrid-3-yl)methyl](cyclopropyl)amino}furan-2(5H)-on, 4-{ [(6-Chlorpyrid-3-yl)methyl](cyclopropyl)amino}furan-2(5H)-on, 4-{[(6-Chlorpyrid-3-yl)methyl](methyl)amino}furan-2(5H)-on, {[1-(6-Chlorpyridin-3-yl)ethyl](methyl)oxido-$\lambda^4$-sulfanyliden}cyanamid und seine Diastereomere {[(1R)-1-(6-Chlorpyridin-3-yl)ethyl](methyl)oxido-$\lambda^4$-sulfanyliden}cyanamid (A) und {[(1S)-1-(6-Chlorpyridin-3-yl)ethyl](methyl)oxido-$\lambda^4$-sulfanyliden}cyanamid (B) sowie Sulfoxaflor und seine Diastereomere [(R)-Methyl(oxido){(1R)-1-[6-(trifluormethyl)pyridin-3-yl]ethyl}-$\lambda^4$-sulfanyliden]cyanamid (A1) und [(S)-Methyl(oxido){(1S)-1-[6-(trifluormethyl)pyridin-3-yl]ethyl}-$\lambda^4$-sulfanyliden]cyanamid (A2), bezeichnet als Diastereomerengruppe A, [(R)-Methyl(oxido){(1S)-1-[6-(trifluormethyl)pyridin-3-yl]ethyl}-$\lambda^4$-sulfanyliden]cyanamid (B1) und [(S)-Methyl(oxido){(1R)-1-[6-(trifluormethyl)pyridin-3-yl]ethyl}-$\lambda^4$-sulfanyliden]cyanamid (B2), bezeichnet als Diastereomerengruppe B und 11-(4-Chlor-2,6-dimethylphenyl)-12-hydroxy-1,4-dioxa-9-azadispiro[4.2.4.2]tetradec-11-en-10-on, 3-(4'-Fluor-2,4-dimethylbiphenyl-3-yl)-4-hydroxy-8-oxa-1-azaspiro[4.5]dec-3-en-2-on, 1-{2-Fluor-4-methyl-5-[(2,2,2-trifluorethyl)sulfinyl]phenyl}-3-(trifluormethyl)-1H-1,2,4-triazol-5-amin, [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat, 2-Cyan-3-(difluormethoxy)-N,N-dimethylbenzolsulfonamid, 2-Cyan-3-(difluormethoxy)-N-methylbenzolsulfonamid, 2-Cyan-3-(difluormethoxy)-N-ethylbenzolsulfonamid, 4-(Difluormethoxy)-N-ethyl-N-methyl-l,2-benzothiazol-3-amin-1,1-dioxid, N-[1-(2,3-Dimethylphenyl)-2-(3,5-dimethylphenyl)ethyl]-4,5-dihydro-1,3-thiazol-2-amin, {1'-[(2E)-3-(4-Chlorphenyl)prop-2-en-1-yl]-5-fluorspiro[indol-3,4'-piperidin]-1(2H)-yl}(2-chlorpyridin-4-yl)methanon, 3-(2,5-Dimethylphenyl)-4-hydroxy-8-methoxy-1,8-diazaspiro[4.5]dec-3-en-2-on, 3-(2,5-Dimethylphenyl)-8-methoxy-2-oxo-1,8-diazaspiro[4.5]dec-3-en-4-yl-ethylcarbonat, 4-(But-2-in-1-yloxy)-6-(3,5-dimethylpiperidin-1-yl)-5-fluorpyrimidin, (2,2,3,3,4,4,5,5-Octafluorpentyl)(3,3,3-trifluorpropyl)malononitril, (2,2,3,3,4,4,5,5-Octafluorpentyl)(3,3,4,4,4-pentafluorbutyl)malononitril, 8-[2-(Cyclopropylmethoxy)-4-(trifluormethyl)phenoxy]-3-[6-(trifluormethyl)pyridazin-3-yl]-3-azabicyclo[3.2.1]octan, Flometoquin, PF1364 (CAS-Reg.Nr. 1204776-60-2), 5-[5-(3,5-Dichlorphenyl)-5-(trifluormethyl)-4,5-dihydro-1,2-oxazol-3-yl]-2-(1H-1,2,4-triazol-1-yl)benzonitril, 5-[5-(2-Chlorpyridin-4-yl)-5-(trifluormethyl)-4,5-dihydro-1,2-oxazol-3-yl]-2-(1H-1,2,4-triazol-1-yl)benzonitril, 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid, 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on, NNI-0711, 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid, Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat, Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat, Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat, Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat, Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat, (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin, 2-{6-[2-(5-Fluorpyridin-3-yl)-1,3-thiazol-5-yl]pyridin-2-yl}pyrimidin, 2-{6-[2-(Pyridin-3-yl)-l,3-thiazol-5-yl]pyridin-2-yl}pyrimidin, 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid, 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid, N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid, N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid, (1E)-N-[(6-Chlorpyridin-3-yl)methyl]-N'-cyan-N-(2,2-difluorethyl)ethanimidamid, N-[2-(5-Amino-1,3,4-thiadiazol-2-yl)-4-chlor-6-methylphenyl]-3-brom-1-(3-chlorpyridin-2-yl)-1H-pyrazol-5-carboxamid und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat,

und/oder mindestens einen weiteren fungiziden Wirkstoff ausgewählt aus der Gruppe bestehend aus

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazolp (83657-17-4), (1.58) Viniconazol

(77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).

(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.

(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2), (3.9) Famoxadon (131807-57-3), (3.10) Fenamidon (161326-34-7), (3.11) Fenoxystrobin (918162-02-4), (3.12) Fluoxastrobin (361377-29-9), (3.13) Kresoxim-Methyl (143390-89-0), (3.14) Metominostrobin (133408-50-1), (3.15) Orysastrobin (189892-69-1), (3.16) Picoxystrobin (117428-22-5), (3.17) Pyraclostrobin (175013-18-0), (3.18) Pyrametostrobin (915410-70-7), (3.19) Pyraoxystrobin (862588-11-2), (3.20) Pyribencarb (799247-52-2), (3.21) Triclopyricarb (902760-40-1), (3.22) Trifloxystrobin (141517-21-7), (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).

(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).

(5) Verbindungen mit Multisite-Aktivität, wie beispielsweise (5.1) Bordeauxmischung (8011-63-0), (5.2)

Captafol (2425-06-1), (5.3) Captan (133-06-2), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).

(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).

(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7).

(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).

(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).

(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).

(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6).

(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).

(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).

(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).

(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34)

Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4), (15.75) 5-Fluor-2-[(4-methylbenzyl)oxy]pyrimidin-4-amin (1174376-25-0), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.

(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid,

(16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid, (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbat-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl{6-[({[(Z)-(1-methyl-1H-tetrazol-5-yl)(phenyl)methylen]amino}oxy)methyl]pyridin-2-yl}carbamat.

**17.** Agrochemische Zusammensetzungen, **dadurch gekennzeichnet, dass** sie mindestens eine Verbindung der Formel (I) gemäß den Ansprüchen 1 bis 15 oder eine Zusamensetzung gemäß Anspruch 16, sowie Streckmittel und/oder oberflächenaktive Stoffe enthalten.

**18.** Verfahren zur Herstellung agrochemischer Zusammensetzungen, **dadurch gekennzeichnet, dass** man Verbindungen der Formel (I) gemäß den Ansprüchen 1 bis 15 oder eine Zusammensetzung gemäß Anspruch 16 mit Streckmitteln und/oder oberflächenaktiven Stoffen vermischt.

**19.** Verfahren zur Bekämpfung tierischer Schädlinge, **dadurch gekennzeichnet, dass** man Verbindungen der Formel (I) gemäß den Ansprüchen 1 bis 15 oder eine Zusammensetzung gemäß Anspruch 16 auf tierische Schädlinge und/oder deren Lebensraum einwirken lässt, wobei Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

**20.** Verwendung von Verbindungen der Formel (I) gemäß einem der Ansprüche 1 bis 15 oder einer Zusammensetzung gemäß Anspruch 16 zur Bekämpfung tierischer Schädlinge im Pflanzenschutz, im Materialschutz und/oder im veterinärmedizinischen Sektor, wobei Verwendungen in Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

## Claims

**1.** Compound of the formula (I)

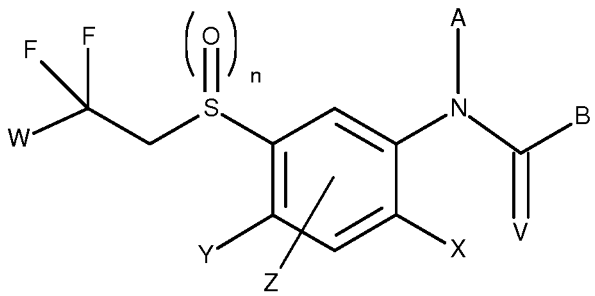

(I)

in which

A and B together with the atoms to which they are attached represent a substructure selected from the group consisting of

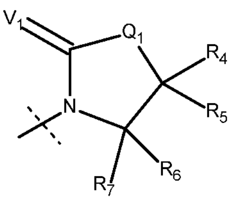
(I-A)

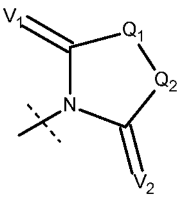
(I-B)

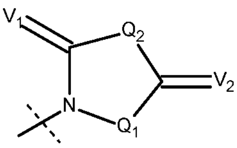
(I-C)

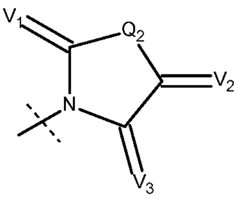
(I-D)

where
for the case that a substructure according to formula (I-A), (I-B) or (I-D) is present,
$V^1$, $V^2$ and $V^3$ each independently of one another
represent oxygen; sulfur, an optionally substituted nitrogen or salts of an optionally substituted nitrogen;
and for the case that a substructure according to formula (I-C) is present,
$V^1$, and $V^2$ each independently of one another represent oxygen or sulfur;
for the case that a substructure according to formula (I-A), (I-B) or (I-D) is present,

$Q^1$ and $Q^2$ each independently of one another
represent oxygen, sulfur or an optionally substituted nitrogen; or
represent an optionally substituted carbon atom; with the proviso, that $Q_1$ does not represent an alkylcarbonylamino radical;

and for the case that a substructure according to formula (I-C) is present,

$Q^1$ and $Q^2$ each independently of one another
represent oxygen, sulfur or a substituted nitrogen; or
represent an optionally substituted carbon atom; with the proviso that $Q_1$ does not represent an alkylcarbonylamino radical;

$R^4$, $R^5$, $R^6$ and $R^7$ each independently of one another

represent hydrogen, cyano, halogen or nitro; or
represent alkyl, cycloalkylalkyl, haloalkyl, cyanoalkyl, hydroxyalkyl, alkoxyalkyl, aminoalkyl, alkoxycarbonylalkyl, alkylthioalkyl, haloalkylthioalkyl, alkoxyalkylthioalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl or hetarylthioalkyl, where the aforementioned radicals may each be saturated or unsaturated and/or optionally substituted; or represent optionally substituted saturated or unsaturated cycloalkyl which may optionally be interrupted by one or more heteroatoms; or
represent alkylcarbonyl, haloalkylcarbonyl, hydroxyalkylcarbonyl, alkoxyalkylcarbonyl, phenylcarbonyl, hetarylcarbonyl, alkoxycarbonyl, haloalkoxycarbonyl, aryloxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represent carbonyl or carboxyl; or
represent optionally substituted phenyl or optionally substituted hetaryl;
represent alkoxy, haloalkoxy, alkoxyalkoxy, aryloxy, arylalkyloxy, cycloalkyloxy, cycloalkylalkyloxy or carbonyloxy, where the aforementioned radicals may be saturated or unsaturated and/or optionally substituted, or represent hydroxyl; or
represent alkylamino, dialkylamino, haloalkylamino, dihaloalkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represent amino; or
represent alkylsulfanyl, alkylsulfinyl, alkylsulfonyl, haloalkylsulfanyl, haloalkylsulfinyl, haloalkylsulfonyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may each be saturated or unsaturated and/or optionally substituted, or represent sulfanyl; or
$R^4$ and $R^5$ together with the atom to which they are attached may form an optionally substituted, saturated or unsaturated three- to eight-membered ring optionally interrupted by one or more heteroatoms which are selected independently from the group consisting of 0, S and N; or
$R^6$ and $R^7$ together with the atom to which they are attached may form an optionally substituted, saturated or unsaturated three- to eight-membered ring optionally interrupted by one or more heteroatoms which are selected independently from the group consisting of 0, S and N;
W represents hydrogen or halogen;
X, Y and Z each independently of one another
represent hydrogen, halogen, hydroxyl, amino, nitro, OCN, SCN, $SF_5$; or
represent trialkylsilyl, alkyl, haloalkyl, cyanoalkyl, hydroxyalkyl, alkoxycarbonylalkyl, alkoxyalkyl, alkenyl, haloalkenyl, cyanoalkenyl, alkynyl, haloalkynyl, cyanoalkynyl, alkoxy, haloalkoxy, cyanoalkoxy, hydroxycarbonylalkoxy, alkoxycarbonylalkoxy, alkoxyalkoxy, alkylhydroxyimino, alkoxyimino, alkylalkoxyimino, haloalkylalkoxyimino, alkylthio, haloalkylthio, alkoxyalkylthio, alkylthioalkyl, alkylsulfinyl, haloalkylsulfinyl, alkoxyalkylsulfinyl, alkylsulfinylalkyl, alkylsulfonyl, haloalkylsulfonyl, alkoxyalkylsulfonyl, alkylsulfonylalkyl, alkylsulfonyloxy, alkylcarbonyl, haloalkylcarbonyl, carboxyl, alkylcarbonyloxy, alkoxycarbonyl, haloalkoxycarbonyl, aminocarbonyl, alkylaminocarbonyl, dialkylaminocarbonyl, alkenylaminocarbonyl, dialkenylaminocarbonyl, cycloalkylaminocarbonyl, alkylsulfonylamino, aminosulfonyl, alkylaminosulfonyl, dialkylaminosulfonyl, alkylsulfoximino, aminothiocarbonyl, alkylaminothiocarbonyl or dialkylaminothiocarbonyl, where all the aforementioned radicals may optionally be substituted; or
represent phenylalkyl, phenoxy, phenylalkyloxy, phenoxyalkyl, phenylthio, phenylthioalkyl, phenylsulfinyl, phenylsulfonyl, hetarylalkyl, hetaryloxy, hetarylalkyloxy, hetarylthio, hetarylsulfinyl or hetarylsulfonyl, where all the aforementioned radicals may optionally be substituted; or
represent cycloalkylalkyl, cycloalkyloxy, cycloalkylalkoxy, cycloalkylthio, cycloalkylalkylthio, cycloalkylsulfinyl, cycloalkylalkylsulfinyl, cycloalkylsulfonyl, cycloalkylalkylsulfonyl or cycloalkenyl, where all the aforementioned radicals may each optionally be substituted; or
represent NR'R",
where R' and R'' each independently of one another
represent hydrogen, cyano, alkyl, haloalkyl, cyanoalkyl, hydroxyalkyl, alkoxyalkyl, alkylthioalkyl, alkenyl, haloalkenyl, cyanoalkenyl, alkynyl, haloalkynyl, cyanoalkynyl, acyl or alkoxycarbonyl; or
R' and R'' together with the nitrogen atom to which they are attached may form an optionally substituted, saturated or unsaturated five- to eight-membered ring optionally interrupted by one or more heteroatoms which are selected independently from the group consisting of 0, S and N; or represent a 3- to 6-membered saturated, partly saturated or aromatic ring which may optionally contain one to three heteroatoms which are selected independently from the group consisting of 0, S and N, and which may optionally be substituted;

and

n represents the number 0 or 1.

**2.** Compound according to Claim 1 in which

A and B together with the atoms to which they are attached represent a substructure selected from the group consisting of (I-A) to (I-D),

where
for the case that a substructure according to formula (I-A), (I-B), or (I-D) is present,
$V^1$, $V^2$ and $V^3$ each independently of one another
represent oxygen; sulfur, $NR^{11}$ or a salt of $NR^{11}$;
and for the case that a substructure according to formula (I-C) is present,
$V^1$, and $V^2$ each independently of one another
represent oxygen or sulfur;

$Q^1$ represents oxygen, sulfur, $NR^1$ or $CR^2R^3$;
$Q^2$ represents $NR^{10}$ or $CR^8R^9$;

for the case that a substructure according to formula (I-A), (I-B), or (I-D) is present,

$R^1$ represents hydrogen, cyano or nitro; or
represents alkyl, cycloalkylalkyl, haloalkyl, cyanoalkyl, hydroxyalkyl, alkoxyalkyl, aminoalkyl, alkoxycarbonylalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl, hetarylthioalkyl, alkoxycarbonyl, aryloxycarbonyl, arylcarbamoyl, where the aforementioned radicals may each be saturated or unsaturated and/or optionally substituted; or
represents optionally substituted saturated or unsaturated cycloalkyl which may optionally be interrupted by one or more heteroatoms; or
represents haloalkylcarbonyl, hydroxyalkylcarbonyl, alkoxyalkylcarbonyl, phenylcarbonyl, hetarylcarbonyl, haloalkoxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represents carbonyl or carboxyl; or
represents optionally substituted phenyl or optionally substituted hetaryl; or
represents alkoxy, haloalkoxy, cycloalkyloxy, aryloxy, arylalkyloxy or carbonyloxy, where the aforementioned radicals may optionally be substituted, or represents hydroxyl; or
represents alkylamino, haloalkylamino, dihaloalkylamino, dialkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represents amino; or
represents alkylsulfanyl, alkylsulfinyl, alkylsulfonyl, haloalkylsulfanyl, haloalkylsulfinyl, haloalkylsulfanyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may each be saturated or unsaturated and/or optionally substituted, or represents sulfanyl; and
$R^{10}$ and $R^{11}$ each independently of one another
represent hydrogen, cyano or nitro; or
represents alkyl, cycloalkylalkyl, haloalkyl, cyanoalkyl, hydroxyalkyl, alkoxyalkyl, aminoalkyl, alkoxycarbonylalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl, hetarylthioalkyl, alkoxycarbonyl, aryloxycarbonyl, arylcarbamoyl, where the aforementioned radicals may each be saturated or unsaturated and/or optionally substituted; or
represents optionally substituted saturated or unsaturated cycloalkyl which may optionally be interrupted by one or more heteroatoms; or
represents alkylcarbonyl, haloalkylcarbonyl, hydroxyalkylcarbonyl, alkoxyalkylcarbonyl, phenylcarbonyl, hetarylcarbonyl, haloalkoxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represents carbonyl or carboxyl; or
represents optionally substituted phenyl or optionally substituted hetaryl; or
represents alkoxy, haloalkoxy, cycloalkyloxy, aryloxy, arylalkyloxy or carbonyloxy, where the aforementioned radicals may optionally be substituted, or represents hydroxyl; or
represents alkylamino, haloalkylamino, dihaloalkylamino, dialkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represents amino; or
represents alkylsulfanyl, alkylsulfinyl, alkylsulfonyl, haloalkylsulfanyl, haloalkylsulfinyl, haloalkylsulfanyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may

each be saturated or unsaturated and/or optionally substituted, or represents sulfanyl;

and for the case that a substructure according to formula (I-C) is present,

$R^1$ represents cyano or nitro; or
represents alkyl, cycloalkylalkyl, haloalkyl, cyanoalkyl, hydroxyalkyl, alkoxyalkyl, aminoalkyl, alkoxycarbonylalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl, hetarylthioalkyl, alkoxycarbonyl, aryloxycarbonyl, arylcarbamoyl, where the aforementioned radicals may each be saturated or unsaturated and/or optionally substituted; or
represents optionally substituted saturated or unsaturated cycloalkyl which may optionally be interrupted by one or more heteroatoms; or
represents haloalkylcarbonyl, hydroxyalkylcarbonyl, alkoxyalkylcarbonyl, phenylcarbonyl, hetarylcarbonyl, haloalkoxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represents carbonyl or carboxyl; or
represents optionally substituted phenyl or optionally substituted hetaryl; or represents alkoxy, haloalkoxy, cycloalkyloxy, aryloxy, arylalkyloxy or carbonyloxy, where the aforementioned radicals may optionally be substituted, or represents hydroxyl; or
represents alkylamino, haloalkylamino, dihaloalkylamino, dialkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represents amino; or
represents alkylsulfanyl, alkylsulfinyl, alkylsulfonyl, haloalkylsulfanyl, haloalkylsulfinyl, haloalkylsulfanyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may each be saturated or unsaturated and/or optionally substituted, or represents sulfanyl; and
$R^{10}$ respresents cyano or nitro; or
represents alkyl, cycloalkylalkyl, haloalkyl, cyanoalkyl, hydroxyalkyl, alkoxyalkyl, aminoalkyl, alkoxycarbonylalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl, hetarylthioalkyl, alkoxycarbonyl, aryloxycarbonyl, arylcarbamoyl, where the aforementioned radicals may each be saturated or unsaturated and/or optionally substituted; or
represents optionally substituted saturated or unsaturated cycloalkyl which may optionally be interrupted by one or more heteroatoms; or
represents alkylcarbonyl, haloalkylcarbonyl, hydroxyalkylcarbonyl, alkoxyalkylcarbonyl, phenylcarbonyl, hetarylcarbonyl, haloalkoxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represents carbonyl or carboxyl; or
represents optionally substituted phenyl or optionally substituted hetaryl; or
represents alkoxy, haloalkoxy, cycloalkyloxy, aryloxy, arylalkyloxy or carbonyloxy, where the aforementioned radicals may optionally be substituted, or represents hydroxyl; or
represents alkylamino, haloalkylamino, dihaloalkylamino, dialkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represents amino; or
represents alkylsulfanyl, alkylsulfinyl, alkylsulfonyl, haloalkylsulfanyl, haloalkylsulfinyl, haloalkylsulfanyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may each be saturated or unsaturated and/or optionally substituted, or represents sulfanyl; and

$R^2$, $R^3$, $R^4$, $R^5$, $R^6$, $R^7$, $R^8$, and $R^9$ each independently of one another

represent hydrogen, cyano, halogen or nitro; or
represent alkyl, cycloalkylalkyl, haloalkyl, cyanoalkyl, hydroxyalkyl, alkoxyalkyl, aminoalkyl, alkoxycarbonylalkyl, alkylthioalkyl, haloalkylthioalkyl, alkoxyalkylthioalkyl, alkylsulfanylalkyl, alkylsulfinylalkyl, alkylsulfonylalkyl, haloalkylsulfanylalkyl, haloalkylsulfinylalkyl, haloalkylsulfonylalkyl, alkoxyalkylsulfanylalkyl, alkoxyalkylsulfinylalkyl, alkoxyalkylsulfonylalkyl, phenylalkyl, phenoxyalkyl, phenylsulfanylalkyl, phenylsulfinylalkyl, phenylsulfonylalkyl, hetarylalkyl, hetaryloxyalkyl or hetarylthioalkyl, where the aforementioned radicals may each be saturated or unsaturated and/or optionally substituted; or
represent optionally substituted saturated or unsaturated cycloalkyl which may optionally be interrupted by one or more heteroatoms; or
represent alkylcarbonyl, haloalkylcarbonyl, hydroxyalkylcarbonyl, alkoxyalkylcarbonyl, phenylcarbonyl, hetarylcarbonyl, alkoxycarbonyl, haloalkoxycarbonyl, aryloxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or represent carbonyl or carboxyl; or
represent optionally substituted phenyl or optionally substituted hetaryl;
represent alkoxy, haloalkoxy, alkoxyalkoxy, aryloxy, arylalkyloxy, cycloalkyloxy, cycloalkylalkyloxy or carbonyloxy, where the aforementioned radicals may be saturated or unsaturated and/or optionally substituted, or represent hydroxyl; or
represent alkylamino, dialkylamino, haloalkylamino, dihaloalkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulfonylamino, or arylsulfonylamino, where the aforementioned radicals may each be saturated or unsaturated and/or optionally substituted, or represent amino; or
represent alkylsulfanyl, alkylsulfinyl, alkylsulfonyl, haloalkylsulfanyl, haloalkylsulfinyl, haloalkylsulfonyl, cycloalkylsulfanyl, cycloalkylsulfinyl, cycloalkylsulfonyl, cycloalkylalkylsulfanyl, cycloalkylalkylsulfinyl, cycloalkylalkylsulfonyl, arylsulfanyl, arylsulfinyl, arylsulfonyl, arylalkylsulfanyl, arylalkylsulfinyl, arylalkylsulfonyl, aminosulfonyl, alkylaminosulfonyl, dialkylaminosulfonyl or arylaminosulfonyl, where the aforementioned radicals may each be saturated or unsaturated and/or optionally substituted, or represent sulfanyl; or
$R^2$ and $R^3$ together with the atom to which they are attached may form an optionally substituted, saturated or unsaturated three- to eight-membered ring optionally interrupted by one or more heteroatoms which are selected independently from the group consisting of 0, S and N; or
$R^4$ and $R^5$ together with the atom to which they are attached may form an optionally substituted, saturated or unsaturated three- to eight-membered ring optionally interrupted by one or more heteroatoms which are selected independently from the group consisting of 0, S and N; or
$R^6$ and $R^7$ together with the atom to which they are attached may form an optionally substituted, saturated or unsaturated three- to eight-membered ring optionally interrupted by one or more heteroatoms which are selected independently from the group consisting of 0, S and N; or
$R^8$ and $R^9$ together with the atom to which they are attached may form an optionally substituted, saturated or unsaturated three- to eight-membered ring optionally interrupted by one or more heteroatoms which are selected independently from the group consisting of 0, S and N;
W represents hydrogen or halogen;

X, Y and Z have the meanings mentioned in Claim 1; and

n represents the number 0 or 1.

**3.** Compound according to Claim 1 or 2 in which

A and B together with the atoms to which they are attached represent a substructure selected from the group consisting of (I-A) to (I-D),

where

for the case that a substructure according to formula (I-A), (I-B) or (I-D) is present,
$V^1$, $V^2$ and $V^3$ each independently of one another
represent oxygen; sulfur, $NR^{11}$ or a salt of $NR^{11}$;
and for the case that a substructure according to formula (I-C) is present

$V^1$ and $V^2$ each independently of one another represent oxygen or sulfur;
$Q^1$ represents oxygen, sulfur, $NR^1$ or $CR^2R^3$;
$Q^2$ represents $NR^{10}$ or $CR^8R^9$;

for the case that a substructure according to formula (I-A), (I-B) or (I-D) is present,

$R^1$ represents hydrogen, cyano or nitro; or
represents $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy $(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl $(C_1-C_6)$alkyl, halo$(C_1-C_6)$alkylsulfanyl $(C_1-C_6)$alkyl, halo $(C_1-C_6)$alkylsulfinyl$(C_1-C_6)$alkyl, halo$(C_1-C_6)$alkylsulfonyl$(C_1-C_6)$alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or
represents optionally substituted saturated or unsaturated $(C_3-C_6)$cycloalkyl which may optionally be interrupted by one or more heteroatoms; or
represents halo$(C_1-C_6)$alkylcarbonyl, hydroxy$(C_1-C_6)$alkylcarbonyl, $(C_1-C_6)$alkoxy $(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl) aminocarbonyl, arylaminocarbonyl, diarylaminocarbonyl, $(C_1-C_6)$alkyl (aryl) aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or
represents optionally substituted phenyl or optionally substituted hetaryl; or
represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or
represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or
represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl $(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl $(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and
$R^{10}$ and $R^{11}$ each independently of one another
represent hydrogen, cyano or nitro; or
represent $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulfanyl$(C_1-C_6)$alkyl, $(C_1-C_6)$alkylsulfinyl $(C_1-C_6)$alkyl, $(C_1-C_6)$alkylsulfonyl $(C_1-C_6)$alkyl, halo$(C_1-C_6)$alkylsulfanyl $(C_1-C_6)$alkyl, halo$(C_1-C_6)$alkylsulfinyl$(C_1-C_6)$alkyl, halo$(C_1-C_6)$alkylsulfonyl$(C_1-C_6)$alkyl,

$(C_1-C_6)$alkoxy$(C_1-C_6)$alkylsulfanyl$(C_1-C_6)$alkyl, $(C_1-C_6)$alkoxy $(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, $(C_1-C_6)$alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represent optionally substituted saturated or unsaturated $(C_3-C_6)$cycloalkyl which may optionally be interrupted by one or more heteroatoms; or

represent halo$(C_1-C_6)$alkylcarbonyl, hydroxy $(C_1-C_6)$alkylcarbonyl, $(C_1-C_6)$alkoxy$(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl) aminocarbonyl, arylaminocarbonyl, diarylaminocarbonyl, $(C_1-C_6)$alkyl (aryl) aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

represent optionally substituted phenyl or optionally substituted hetaryl; or

represent $(C_1-C_6)$alkoxy, halo$(C_1-C_6)$alkoxy, $(C_3-C_6)$ cycloalkyloxy, aryloxy, aryl$(C_1-C_6)$alkyloxy or carbonyloxy, where the aforementioned radicals may each optionally be substituted, or represents hydroxyl; or

represent $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl;

and for the case that a substructure according to formula (I-C) is present,

$R^1$ represents cyano or nitro; or

represents $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl $(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo$(C_1-C_6)$alkylsulfinyl$(C_1-C_6)$alkyl, halo$(C_1-C_6)$alkylsulfonyl $(C_1-C_6)$alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated $(C_3-C_6)$cycloalkyl which may optionally be interrupted by one or more heteroatoms; or

represents halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl) aminocarbonyl, arylaminocarbonyl, diarylaminocarbonyl, $(C_1-C_6)$alkyl(aryl)aminocarbonyl, $(C_3-C_6)$cycloalkyl(aryl)aminocarbonyl, $(C_1-C_6)$alkylaminothiocarbonyl, di$(Ci-C_6)$alkylaminothiocarbonyl or aminothiocarbonyl, where the aforementioned radicals may each optionally be substituted and/or, under the

condition that, in the case of an unsaturated radical, the defined minimum number of carbon atoms is 2, the radicals may be saturated or unsaturated, or is carbonyl or carboxyl; or

represents optionally substituted phenyl or optionally substituted hetaryl; or

represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^{10}$ represents cyano or nitro; or

represents $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo $(C_1-C_6)$alkylsulfinyl$(C_1-C_6)$alkyl, halo$(C_1-C_6)$alkylsulfonyl$(C_1-C_6)$alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, $(C_1-C_6)$alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated $(C_3-C_6)$cycloalkyl which may optionally be interrupted by one or more heteroatoms; or

represents halo$(C_1-C_6)$alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl) aminocarbonyl, arylaminocarbonyl, diarylaminocarbonyl, $(C_1-C_6)$alkyl(aryl)aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

represents optionally substituted phenyl or optionally substituted hetaryl; or

represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents $(C_1-C_6)$alkylsulfanyl, $(C_1-C_6)$alkylsulfinyl, $(C_1-C_6)$alkylsulfonyl, halo $(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl $(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl $(C_1-C_6)$alkylsulfi-

nyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^2$, $R^3$, $R^4$, $R^5$, $R^6$, $R^7$, $R^8$ and $R^9$, in each case independently of one another,

represent hydrogen, cyano, halogen or nitro; or
represent ($C_1$-$C_6$)alkyl, ($C_3$-$C_6$)cycloalkyl($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxy ($C_1$-$C_6$)alkyl, amino($C_1$-$C_6$)alkyl, ($C_1$-$C_6$)alkoxycarbonyl ($C_1$-$C_6$)alkyl, ($C_1$-$C_6$)alkylthio ($C_1$-$C_6$)alkyl, halo($C_1$-$C_6$)alkylthio($C_1$-$C_6$)alkyl, ($C_1$-$C_6$)alkoxy ($C_1$-$C_6$)alkylthioalkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl ($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl or hetarylthio($C_1$-$C_6$)alkyl, where the aforementioned radicals may each be saturated or unsaturated and/or optionally be substituted; or
represent optionally substituted saturated or unsaturated ($C_3$-$C_6$)cycloalkyl which may optionally be interrupted by one or more heteroatoms; or
represent ($C_1$-$C_6$)alkylcarbonyl, halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, ($C_1$-$C_6$)alkoxycarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl) aminocarbonyl, arylaminocarbonyl, diarylaminocarbonyl, ($C_1$-$C_6$)alkyl (aryl) aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represent carbonyl or carboxyl; or
represent optionally substituted phenyl or optionally substituted hetaryl;
represent ($C_1$-$C_6$)alkoxy, halo($C_1$-$C_6$)alkoxy, ($C_1$-$C_6$)alkoxy ($C_1$-$C_6$)alkoxy, aryloxy, aryl ($C_1$-$C_6$)alkyloxy, ($C_3$-$C_6$)cycloalkyloxy, ($C_3$-$C_6$) cycloalkyl($C_1$-$C_6$)alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or represent hydroxyl; or
represent ($C_1$-$C_6$)alkylamino, di($C_1$-$C_6$)alkylamino, halo ($C_1$-$C_6$)alkylamino, dihalo($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or
represent ($C_1$-$C_6$)alkylsulfanyl, ($C_1$-$C_6$)alkylsulfinyl, ($C_1$-$C_6$)alkylsulfonyl, halo($C_1$-$C_6$)alkylsulfanyl, halo($C_1$-$C_6$)alkylsulfinyl, halo($C_1$-$C_6$)alkylsulfonyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or are sulfanyl; or
$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or
$R^4$ and $R^5$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl,

methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or $R^6$ and $R^7$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or $R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N;
W represents hydrogen or halogen;

X, Y and Z have the meanings mentioned in Claims 1 or 2; and

n represents the number 0 or 1.

**4.** Compound according to any of Claims 1 to 3, in which

A and B together with the atoms to which they are bonded are a substructure of the formula (I-A)

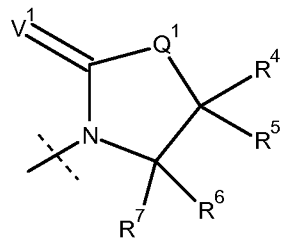

(I-A)

where
$V^1$ represents oxygen, sulfur, $NR_{11}$ or a salt of $NR^{11}$;

$Q^1$ represents oxygen, sulfur, $NR^1$ or $CR^2R^3$;
$R^1$ represents hydrogen, cyano or nitro; or
represents ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, hydroxy($C_1$-$C_6$)alkyl, ($C_1$-$C_6$)alkoxy ($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl ($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl, hetarylsulfanyl($C_1$-$C_6$)alkyl, hetarylsulfinyl($C_1$-$C_6$)alkyl, hetarylsulfonyl($C_1$-$C_6$)alkyl, ($C_1$-$C_6$)alkoxycarbonyl, aryloxycarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or
represents optionally substituted saturated or unsaturated ($C_3$-$C_6$)cycloalkyl which may optionally be interrupted by one or more heteroatoms; or
represents halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, halo($C_1$-$C_6$)alkoxycarbonyl, aminocarbonyl, ($C_1$-$C_6$)alkylaminocarbonyl, di(Ci-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl) aminocarbonyl, arylaminocarbonyl, diarylaminocarbonyl, ($C_1$-$C_6$)alkyl (aryl) aminocarbonyl, ($C_3$-$C_6$)cycloalkyl(aryl)aminocarbonyl, ($C_1$-$C_6$)alkylaminothiocarbonyl, di(Ci-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the

radicals may be saturated or unsaturated, or is carbonyl or carboxyl; or

represents optionally substituted phenyl or optionally substituted hetaryl; or

represents($C_1$-$C_6$)alkoxy, halo($C_1$-$C_6$)alkoxy, ($C_3$-$C_6$)cycloalkyloxy, aryloxy, aryl($C_1$-$C_6$)alkyloxy or carbonyloxy, where the aforementioned radicals may each optionally be substituted, or represents hydroxyl; or

represents ($C_1$-$C_6$)alkylamino, halo($C_1$-$C_6$)alkylamino, dihalo($C_1$-$C_6$)alkylamino, di ($C_1$-$C_6$)alkylamino, ($C_3$-$C_6$)cycloalkylamino, di($C_3$-$C_6$)cycloalkylamino, ($C_3$-$C_6$)cycloalkyl($C_1$-$C_6$)alkylamino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents ($C_1$-$C_6$)alkylsulfanyl, ($C_1$-$C_6$)alkylsulfinyl, ($C_1$-$C_6$)alkylsulfonyl, halo($C_1$-$C_6$)alkylsulfanyl, halo($C_1$-$C_6$)alkylsulfinyl, halo($C_1$-$C_6$)alkylsulfanyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl ($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and $R^{11}$ rep

resents hydrogen, cyano or nitro; or

represents ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, hydroxy($C_1$-$C_6$)alkyl, ($C_1$-$C_6$)alkoxy ($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl, hetarylsulfanyl($C_1$-$C_6$)alkyl, hetarylsulfinyl($C_1$-$C_6$)alkyl, hetarylsulfonyl($C_1$-$C_6$)alkyl, ($C_1$-$C_6$)alkoxycarbonyl, aryloxycarbonyl, ($C_1$-$C_6$)alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated ($C_3$-$C_6$)cycloalkyl which may optionally be interrupted by one or more heteroatoms; or

represents halo($C_1$-$C_6$)alkylcarbonyl, hydroxy ($C_1$-$C_6$)alkylcarbonyl, ($C_1$-$C_6$)alkoxy($C_1$-$C_6$)alkylcarbonyl, phenylcarbonyl, hetarylcarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

represents optionally substituted phenyl or optionally substituted hetaryl; or represents ($C_1$-$C_6$)alkoxy, halo($C_1$-$C_6$)alkoxy, ($C_3$-$C_6$)cycloalkyloxy, aryloxy, aryl($C_1$-$C_6$)alkyloxy or carbonyloxy, where the aforementioned radicals may each optionally be substituted, or represents hydroxyl; or

represents ($C_1$-$C_6$)alkylamino, halo($C_1$-$C_6$)alkylamino, dihalo($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl($C_1$-$C_6$)alkylamino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents ($C_1$-$C_6$)alkylsulfanyl, ($C_1$-$C_6$)alkylsulfinyl, ($C_1$-$C_6$)alkylsulfonyl, halo($C_1$-$C_6$)alkylsulfanyl, halo($C_1$-$C_6$)alkylsulfinyl, halo($C_1$-$C_6$)alkylsulfanyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylami-

nosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^2$, $R^3$, $R^4$, $R^5$, $R^6$ and $R^7$ each independently of one another

represent hydrogen, cyano, halogen or nitro; or
represent ($C_1$-$C_6$)alkyl, ($C_3$-$C_6$)cycloalkyl($C_1$-$C_6$)alkyl, halo($C_1$-$C_6$)alkyl, cyano($C_1$-$C_6$)alkyl, hydroxy ($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylthio($C_1$-$C_6$)alkyl, halo($C_1$-$C_6$)alkylthio($C_1$-$C_6$)alkyl, ($C_1$-$C_6$)alkoxy($C_1$-$C_6$)alkylthioalkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo ($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy($C_1$-$C_6$)alkylsulfonyl($C_1$-$C_6$)alkyl, phenyl ($C_1$-$C_6$)alkyl, phenoxy($C_1$-$C_6$)alkyl, phenylsulfanyl ($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl or hetarylthio($C_1$-$C_6$)alkyl, where the aforementioned radicals may each be saturated or unsaturated and/or optionally be substituted; or
represent optionally substituted saturated or unsaturated ($C_3$-$C_6$)cycloalkyl which may optionally be interrupted by one or more heteroatoms; or
represent ($C_1$-$C_6$)alkylcarbonyl, halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, ($C_1$-$C_6$) alkoxycarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represent carbonyl or carboxyl; or
represent optionally substituted phenyl or optionally substituted hetaryl;
represent ($C_1$-$C_6$)alkoxy, halo($C_1$-$C_6$)alkoxy, ($C_1$-$C_6$)alkoxy($C_1$-$C_6$)alkoxy, aryloxy, aryl($C_1$-$C_6$)alkyloxy, ($C_3$-$C_6$)cycloalkyloxy, ($C_3$-$C_6$)cycloalkyl($C_1$-$C_6$)alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or represent hydroxyl; or
represent ($C_1$-$C_6$)alkylamino, di($C_1$-$C_6$)alkylamino, halo($C_1$-$C_6$)alkylamino, dihalo($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or
represent ($C_1$-$C_6$)alkylsulfanyl, ($C_1$-$C_6$)alkylsulfinyl, ($C_1$-$C_6$)alkylsulfonyl, halo ($C_1$-$C_6$)alkylsulfanyl, halo($C_1$-$C_6$)alkylsulfinyl, halo($C_1$-$C_6$)alkylsulfonyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or are sulfanyl; or
$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or
$R^4$ and $R^5$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or

$R^6$ and $R^7$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or

W represents hydrogen, fluorine or chlorine;

X, Y and Z each independently of one another represent hydrogen, fluorine, chlorine, bromine, nitro, amino, hydroxyl, ($C_1$-$C_4$)alkyl, ($C_1$-$C_4$)haloalkyl, ($C_2$-$C_4$)alkenyl, ($C_2$-$C_4$)alkynyl, ($C_1$-$C_4$)alkoxy, ($C_1$-$C_4$)haloalkoxy or aminothiocarbonyl;

or represent a benzyl, phenoxy, phenylthio, cyclopropylmethyl, cyclopropyloxy or cyclopropylthio, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl;

or represent cyclopropyl, cyclobutyl, cyclopentyl, cyclohexyl or phenyl, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl; and

n represents the number 0 or 1.

**5.** Compound according to Claim 4, in which the substructure of the formula (I-A) represents a substructure which is selected from the group consisting of

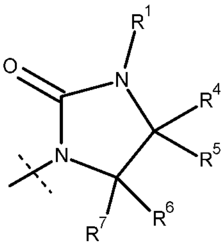
(I-A-1)

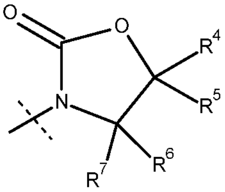
(I-A-2)

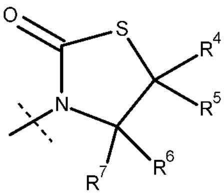
(I-A-3)

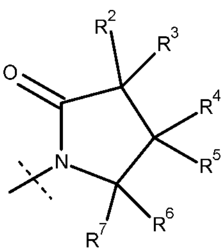
(I-A-4)

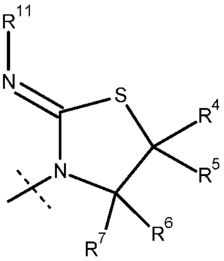
(I-A-5)

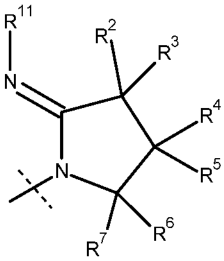
(I-A-6)

where

$R^1$ represents hydrogen; or

represents ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, ($C_1$-$C_6$)alkoxy($C_1$-$C_6$)alkyl, ($C_1$-$C_6$)alkoxycarbonyl($C_1$-$C_6$)alkyl, phenyl($C_1$-$C_6$)alkyl, hetaryl($C_1$-$C_6$)alkyl, ($C_2$-$C_6$)alkenyl, halo($C_2$-$C_6$)alkenyl, ($C_3$-$C_6$)cycloalkenyl($C_1$-$C_6$)alkyl, ($C_2$-$C_6$)alkynyl, where the aforementioned radicals may optionally be substituted; or

represents optionally substituted ($C_3$-$C_6$)cycloalkyl, ($C_3$-$C_6$)cycloalkenyl, which may optionally be interrupted by one or more heteroatoms from the group of 0, S and N; or

represents halo($C_1$-$C_6$)alkylcarbonyl, ($C_1$-$C_6$)alkoxy($C_1$-$C_6$)alkylcarbonyl, ($C_1$-$C_6$)alkoxycarbonyl, halo($C_1$-$C_6$)alkoxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, ($C_1$-$C_6$)alkylaminothiocarbonyl, di($C_1$-$C_6$)alkylaminothiocarbonyl, ($C_2$-$C_6$)alkenylcarbo-

nyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or are carbonyl or carboxyl; or
represents optionally substituted phenyl or optionally substituted hetaryl; or represents $(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or
represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or
represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and
$R^{11}$ represents hydrogen or
represents $(C_1-C_6)$alkyl, $(C_3-C_6)$cycloalkyl $(C_1-C_6)$alkyl, halo$(C_2-C_6)$alkyl, cyano $(C_1-C_6)$alkyl, $(C_1-C_6)$alkoxy$(C_1-C_6)$alkyl, $(C_1-C_6)$alkoxycarbonyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may optionally be substituted; or
represents optionally substituted $(C_3-C_6)$cycloalkyl, $(C_3-C_6)$cycloalkenyl, which may optionally be interrupted by one or more heteroatoms from the group of 0, S and N; or
represents $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or
represents optionally substituted phenyl or optionally substituted hetaryl; or
represents$(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or
represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or
represents$(C_1-C_6)$alkylsulfanyl, $(C_1-C_6)$alkylsulfinyl, $(C_1-C_6)$alkylsulfonyl, halo $(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and

$R^2$, $R^3$, $R^4$, $R^5$, $R^6$ and $R^7$ each independently of one another

represent hydrogen, cyano, halogen or nitro; or
represent $(C_1-C_6)$alkyl, $(C_3-C_6)$cycloalkyl$(C_1-C_6)$alkyl, halo$(C_1-C_6)$alkyl, cyano$(C_1-C_6)$alkyl, $(C_1-C_6)$alkoxy$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may each optionally be substituted, or represent optionally substituted $(C_3-C_6)$cycloalkyl, $(C_3-C_6)$cycloalkenyl, which may optionally be interrupted by one or more heteroatoms from the group consisting of O, S and N; or
represent $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or
represent optionally substituted phenyl or optionally substituted hetaryl;
represent $(C_1-C_6)$alkoxy, halo$(C_1-C_6)$alkoxy, $(C_1-C_6)$alkoxy $(C_1-C_6)$alkoxy, $(C_3-C_6)$cycloalkyloxy, $(C_3-C_6)$cycloalkyl$(C_1-C_6)$alkyloxy, $(C_2-C_6)$alkenyloxy, $(C_3-C_6)$cycloalkenyl $(C_1-C_6)$alkoxy, $(C_2-C_6)$alkynyloxy or carbony-

loxy, where the aforementioned radicals may each optionally be substituted, or represent hydroxyl; or
represent $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or
represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl $(C_1-C_6)$alkylsulfanyl, $(C_3-C_6)$cycloalkyl $(C_1-C_6)$alkylsulfinyl, $(C_3-C_6)$cycloalkyl $(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represent sulfanyl; or
$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of O, S and N; or
$R^4$ and $R^5$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of O, S and N; or
$R^6$ and $R^7$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of O, S and N; or
W represents hydrogen or fluorine;
X and Y independently of one another represent hydrogen, fluorine, chlorine, bromine, methyl, ethyl, trifluoromethyl, 2,2-difluoromethyl, difluoromethoxy, trifluoromethoxy, cyclopropyl, amino, hydroxyl or nitro;
Z represents hydrogen; and
n represents the number 0 or 1.

**6.** Compound according to Claim 4 or 5, in which the substructure of the formula (I-A) represents a substructure which is selected from the group consisting of

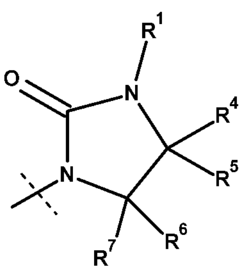

(I-A-1)

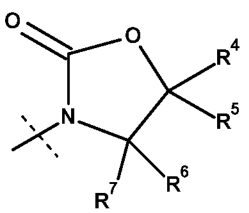

(I-A-2)

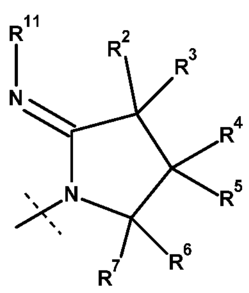

(I-A-6)

where

$R^1$ and $R^{11}$ each independently of one another
represent hydrogen, methyl, ethyl, propyl, isopropyl, cyclopropyl, cyclopropylmethyl, $CH_2CF_3$, $CH_2CHF_2$, $CH(CH_3)CF_3$, $CH_2CH_2CF_3$, $CH_2CH_2CHF_2$; or
$R^2$, $R^3$, $R^4$, $R^5$, $R^6$ and $R^7$ each independently of one another represent hydrogen, methyl, trifluoromethyl or phenyl;
W represents hydrogen or fluorine;
X represents hydrogen, chlorine, fluorine or methyl;
Y represents chlorine, bromine, methyl, trifluoromethyl or fluorine;
where X and Y represent in particular the following combinations (Y,X): (Me, F), (Me,H), (Me,Cl), (Me,Me),

(Cl,Cl), (Cl,F), (Cl,H), (Br,H), (Br,F), (F,F), ($CF_3$,H);
Z represents hydrogen; and
n represents the number 0 or 1.

**7.** Compound according to any of Claims 1 to 3, in which

A and B together with the atoms to which they are attached are a substructure of the formula (I-B)

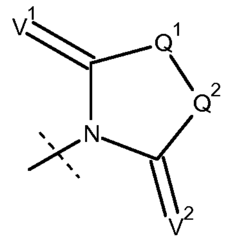

(I-B)

where

$V^1$ and $V^2$ each independently of one another

represent oxygen; sulfur, $NR^{11}$ or a salt of $NR^{11}$;

$Q^1$ represents oxygen, sulfur, $NR^1$ or $CR^2R^3$;
$Q^2$ represents $NR^{10}$ or $CR^8R^9$;
$R^1$ represents hydrogen, cyano or nitro; or
represents ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl ($C_1$-$C_6$)alkyl, ($C_1$-$C_6$)alkoxy($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl, hetarylsulfanyl($C_1$-$C_6$)alkyl, hetarylsulfinyl($C_1$-$C_6$)alkyl, hetarylsulfonyl($C_1$-$C_6$)alkyl, ($C_1$-$C_6$)alkoxycarbonyl, aryloxycarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or
represents optionally substituted saturated or unsaturated ($C_3$-$C_6$)cycloalkyl which may optionally be interrupted by one or more heteroatoms; or
represents halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, halo($C_1$-$C_6$)alkoxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or
represents optionally substituted phenyl or optionally substituted hetaryl; or
represents ($C_1$-$C_6$)alkoxy, halo($C_1$-$C_6$)alkoxy, ($C_3$-$C_6$)cycloalkyloxy, aryloxy, aryl($C_1$-$C_6$)alkyloxy or carbonyloxy, where the aforementioned radicals may each optionally be substituted, or represents hydroxyl; or
represents ($C_1$-$C_6$)alkylamino, halo($C_1$-$C_6$)alkylamino, dihalo($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl($C_1$-$C_6$)alkylamino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the

defined minimum number of carbon atoms is 2, the radicals may be saturated or unsaturated, or represent amino; or represents ($C_1$-$C_6$)alkylsulfanyl, ($C_1$-$C_6$)alkylsulfinyl, ($C_1$-$C_6$)alkylsulfonyl, halo($C_1$-$C_6$)alkylsulfanyl, halo($C_1$-$C_6$)alkylsulfinyl, halo($C_1$-$C_6$)alkylsulfanyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^{10}$ and $R^{11}$ each independently of one another

represent hydrogen, cyano or nitro; or

represent ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo ($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl ($C_1$-$C_6$)alkyl, ($C_1$-$C_6$)alkoxy($C_1$-$C_6$)alkylsulfonyl($C_1$-$C_6$)alkyl, phenyl ($C_1$-$C_6$)alkyl, phenoxy($C_1$-$C_6$)alkyl, phenylsulfanyl ($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl, hetarylsulfanyl($C_1$-$C_6$)alkyl, hetarylsulfinyl($C_1$-$C_6$)alkyl, hetarylsulfonyl($C_1$-$C_6$)alkyl, ($C_1$-$C_6$)alkoxycarbonyl, aryloxycarbonyl, ($C_1$-$C_6$)alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represent optionally substituted saturated or unsaturated ($C_3$-$C_6$)cycloalkyl which may optionally be interrupted by one or more heteroatoms; or

represent halo($C_1$-$C_6$)alkylcarbonyl, hydroxy ($C_1$-$C_6$)alkylcarbonyl, ($C_1$-$C_6$)alkoxy($C_1$-$C_6$)alkylcarbonyl, phenylcarbonyl, hetarylcarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

represent optionally substituted phenyl or optionally substituted hetaryl; or

represent ($C_1$-$C_6$)alkoxy, halo($C_1$-$C_6$)alkoxy, ($C_3$-$C_6$)cycloalkyloxy, aryloxy, aryl($C_1$-$C_6$)alkyloxy or carbonyloxy, where the aforementioned radicals may each optionally be substituted, or represents hydroxyl; or

represent ($C_1$-$C_6$)alkylamino, halo($C_1$-$C_6$)alkylamino, dihalo($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl($C_1$-$C_6$)alkylamino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represent ($C_1$-$C_6$)alkylsulfanyl, ($C_1$-$C_6$)alkylsulfinyl, ($C_1$-$C_6$)alkylsulfonyl, halo ($C_1$-$C_6$)alkylsulfanyl, halo($C_1$-$C_6$)alkylsulfinyl, halo($C_1$-$C_6$)alkylsulfanyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl($C_1$-$C_6$)alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^2$, $R^3$, $R^8$ and $R^9$ each independently of one another

represent hydrogen, cyano, halogen or nitro; or

represent ($C_1$-$C_6$)alkyl, ($C_3$-$C_6$)cycloalkyl($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylthio($C_1$-$C_6$)alkyl, halo($C_1$-$C_6$)alkylthio($C_1$-$C_6$)alkyl, ($C_1$-$C_6$)alkoxy($C_1$-$C_6$)alkylthioalkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, ha-

lo($C_1$-$C_6$)alkylsulfanyl($C_1$-$C_6$)alkyl, halo ($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy ($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl ($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl or hetarylthio($C_1$-$C_6$)alkyl, where the aforementioned radicals may each be saturated or unsaturated and/or optionally be substituted; or

represent optionally substituted saturated or unsaturated ($C_3$-$C_6$)cycloalkyl which may optionally be interrupted by one or more heteroatoms; or

represent ($C_1$-$C_6$)alkylcarbonyl, halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, ($C_1$-$C_6$)alkoxycarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represent carbonyl or carboxyl; or

represent optionally substituted phenyl or optionally substituted hetaryl;

represent ($C_1$-$C_6$)alkoxy, halo($C_1$-$C_6$)alkoxy, ($C_1$-$C_6$)alkoxy ($C_1$-$C_6$)alkoxy, aryloxy, aryl($C_1$-$C_6$)alkyloxy, (C3-$C_6$)cycloalkyloxy, ($C_3$-$C_6$)cycloalkyl($C_1$-$C_6$)alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or represent hydroxyl; or

represent ($C_1$-$C_6$)alkylamino, di(Ci-$C_6$)alkylamino, halo($C_1$-$C_6$)alkylamino, dihalo($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hetarylamino, dihetarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represent ($C_1$-$C_6$)alkylsulfanyl, ($C_1$-$C_6$)alkylsulfinyl, ($C_1$-$C_6$)alkylsulfonyl, halo ($C_1$-$C_6$) alkylsulfanyl, halo($C_1$-$C_6$)alkylsulfinyl, halo($C_1$-$C_6$)alkylsulfonyl, ($C_3$-$C_6$)cycloalkylsulfanyl, ($C_3$-$C_6$)cycloalkylsulfinyl, ($C_3$-$C_6$)cycloalkylsulfonyl, ($C_3$-$C_6$)cycloalkyl($C_1$-$C_6$)alkylsulfanyl, ($C_3$-$C_6$)cycloalkyl($C_1$-$C_6$)alkylsulfinyl, ($C_3$-$C_6$)cycloalkyl($C_1$-$C_6$)alkylsulfonyl, arylsulfanyl, arylsulfinyl, arylsulfonyl, aryl($C_1$-$C_6$)alkylsulfanyl, aryl ($C_1$-$C_6$) alkylsulfinyl, aryl($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or are sulfanyl; or

$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N; or

$R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N;

W represents hydrogen, fluorine or chlorine;

X, Y and Z independently of one another represent hydrogen, fluorine, chlorine, bromine, nitro, amino, hydroxyl, ($C_1$-$C_4$)alkyl,($C_1$-$C_4$) haloalkyl, ($C_2$-$C_4$)alkenyl, ($C_2$-$C_4$)alkynyl, ($C_1$-$C_4$)alkoxy, ($C_1$-$C_4$)haloalkoxy or aminothiocarbonyl;

or represent a benzyl, phenoxy, phenylthio, cyclopropylmethyl, cyclopropyloxy or cyclopropylthio, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl;

or represent cyclopropyl, cyclobutyl, cyclopentyl, cyclohexyl or phenyl, which is optionally mono- or polysubstituted by identical or different substituents from the group consisting of fluorine, chlorine, bromine, cyano, nitro,

hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl; and
n represents the number 0 or 1.

**8.** Compound according to Claim 7, in which the substructure of the formula (I-B) represents substructure which is selected from the group consisting of

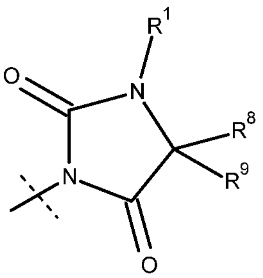
(I-B-1)

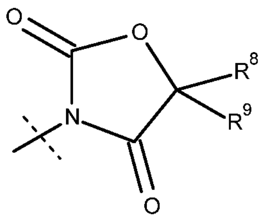
(I-B-2)

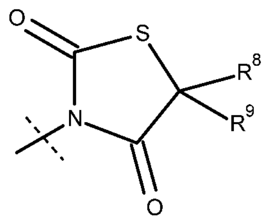
(I-B-3)

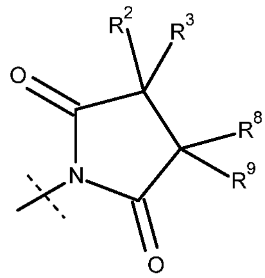
(I-B-4)

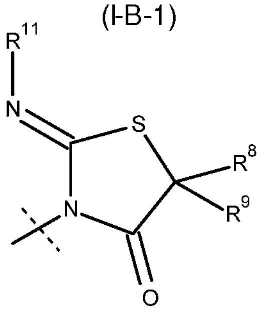
(I-B-5)

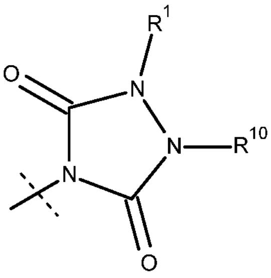
(I-B-6)

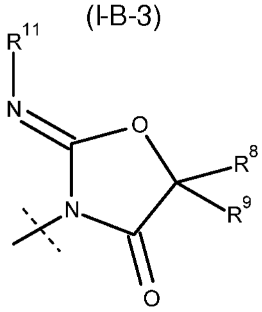
(I-B-7)

where

$R^1$ represents hydrogen or
represents ($C_1$-$C_6$)alkyl, ($C_3$-$C_6$)cycloalkyl($C_1$-$C_6$)alkyl, halo($C_2$-$C_6$)alkyl, cyano($C_1$-$C_6$)alkyl, ($C_1$-$C_6$)alkoxy($C_1$-$C_6$)alkyl, ($C_1$-$C_6$)alkoxycarbonyl($C_1$-$C_6$)alkyl, phenyl($C_1$-$C_6$)alkyl, hetaryl($C_1$-$C_6$)alkyl, ($C_2$-$C_6$)alkenyl, halo($C_2$-$C_6$)alkenyl, ($C_3$-$C_6$)cycloalkenyl($C_1$-$C_6$)alkyl, ($C_2$-$C_6$)alkynyl, where the aforementioned radicals may optionally be substituted; or
represents optionally substituted ($C_3$-$C_6$)cycloalkyl, ($C_3$-$C_6$)cycloalkenyl, which may optionally be interrupted by one or more heteroatoms from the group of 0, S and N; or
represents halo($C_1$-$C_6$)alkylcarbonyl, ($C_1$-$C_6$)alkoxy($C_1$-$C_6$)alkylcarbonyl, ($C_1$-$C_6$)alkoxycarbonyl, halo($C_1$-$C_6$)alkoxycarbonyl, aminocarbonyl, ($C_1$-$C_6$)alkylaminocarbonyl, di(Ci-$C_6$)alkylaminocarbonyl, ($C_3$-$C_6$)cycloalkylaminocarbonyl, ($C_1$-$C_6$)alkylaminothiocarbonyl, di($C_1$-$C_6$)alkylaminothiocarbonyl, ($C_2$-$C_6$)alkenylcarbonyl, ($C_3$-$C_6$)cycloalkenyl($C_1$-$C_6$)alkylcarbonyl, ($C_2$-$C_6$)alkynylcarbonyl or aminothiocarbonyl, where the aforementioned radicals may each optionally be substituted, or are carbonyl or carboxyl; or
represents optionally substituted phenyl or optionally substituted hetaryl; or represents ($C_1$-$C_6$)alkoxy, halo($C_1$-$C_6$)alkoxy, ($C_3$-$C_6$)cycloalkyloxy, or carbonyloxy, where the aforementioned radicals may each optionally be substituted, or represents hydroxyl; or
represents ($C_1$-$C_6$)alkylamino, halo($C_1$-$C_6$)alkylamino, dihalo($C_1$-$C_6$)alkylamino, di ($C_1$-$C_6$)alkylamino, ($C_3$-$C_6$)cycloalkylamino, di($C_3$-$C_6$)cycloalkylamino, ($C_1$-$C_6$)alkylcarbonylamino, ($C_1$-$C_6$)alkoxycarbonylamino, ($C_1$-$C_6$)alkylcarbamoylamino, ($C_1$-$C_6$)alkylsulfonylamino, ($C_2$-$C_6$)alkenylamino, ($C_3$-$C_6$)cycloalkenyl($C_1$-$C_6$)alkylamino, ($C_2$-$C_6$)alkynylamino, where the aforementioned radicals may each optionally be substituted, or represents amino; or
represents ($C_1$-$C_6$)alkylsulfanyl, ($C_1$-$C_6$)alkylsulfinyl, ($C_1$-$C_6$)alkylsulfonyl, halo ($C_1$-$C_6$) alkylsulfanyl, halo($C_1$-$C_6$)alkylsulfinyl, halo($C_1$-$C_6$)alkylsulfanyl, ($C_3$-$C_6$)cycloalkyl ($C_1$-$C_6$)alkylsulfanyl, ($C_3$-$C_6$)cycloalkyl ($C_1$-$C_6$)alkylsulfinyl, ($C_3$-$C_6$)cycloalkyl ($C_1$-$C_6$)alkylsulfonyl, aminosulfonyl, ($C_1$-$C_6$)alkylaminosulfonyl, di($C_1$-$C_6$)alkylaminosulfonyl or arylaminosulfonyl, where the aforementioned radicals may each optionally be substituted, or represents sulfanyl; and
$R^{10}$ and $R^{11}$ each independently of one another

represent hydrogen; or
represent $(C_1-C_6)$alkyl, $(C_3-C_6)$cycloalkyl$(C_1-C_6)$alkyl, halo$(C_2-C_6)$alkyl, cyano$(C_1-C_6)$alkyl, $(C_1-C_6)$alkoxy$(C_1-C_6)$alkyl, $(C_1-C_6)$alkoxycarbonyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may optionally be substituted; or
represent optionally substituted $(C_3-C_6)$cycloalkyl, $(C_3-C_6)$cycloalkenyl, which may optionally be interrupted by one or more heteroatoms from the group of 0, S and N; or
represent $(C_1-C_6)$alkylcarbonyl, halo$(C_1-C_6)$alkylcarbonyl, $(C_1-C_6)$alkoxy $(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di(Ci-$C_6$)alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or
represent optionally substituted phenyl or optionally substituted hetaryl; or
represent $(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or
represent $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or
represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl $(C_1-C_6)$alkylsulfanyl, $(C_3-C_6)$cycloalkyl $(C_1, C_6)$alkylsulfinyl, $(C_3-C_6)$cycloalkyl $(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and

$R^2$, $R^3$, $R^8$ and $R^9$ each independently of one another

represent hydrogen, cyano, halogen or nitro; or
represent $(C_1-C_6)$alkyl, $(C_3-C_6)$cycloalkyl $(C_1-C_6)$alkyl, halo$(C_1-C_6)$alkyl, cyano $(C_1-C_6)$alkyl, $(C_1-C_6)$alkoxy$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, phenyl$(C_1-C_6)$alkyl, hetaryl$(C_1-C_6)$alkyl, $(C_2-C_6)$alkenyl, halo $(C_2-C_6)$alkenyl, $(C_3-C_6)$cycloalkenyl $(C_1-C_6)$alkyl, $(C_2-C_6)$alkynyl, where the aforementioned radicals may each optionally be substituted; or
represent optionally substituted $(C_3-C_6)$cycloalkyl, $(C_3-C_6)$cycloalkenyl, which may optionally be interrupted by one or more heteroatoms from the group of 0, S and N; or
represent $(C_1-C_6)$alkylcarbonyl, halo$(C_1-C_6)$alkylcarbonyl, $(C_1-C_6)$alkoxy $(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or
represent optionally substituted phenyl or optionally substituted hetaryl; or represent $(C_1-C_6)$alkoxy, halo$(C_1-C_6)$alkoxy, $(C_1-C_6)$alkoxy$(C_1-C_6)$alkoxy, $(C_3-C_6)$cycloalkyloxy, $(C_3-C_6)$cycloalkyl$(C_1-C_6)$alkyloxy, $(C_2-C_6)$alkenyloxy, $(C_3-C_6)$cycloalkenyl $(C_1-C_6)$alkoxy, $(C_2-C_6)$alkynyloxy or carbonyloxy, where the aforementioned radicals may each optionally be substituted, or represent hydroxyl; or
represent $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represent amino; or
represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl $(C_1-C_6)$alkylsulfanyl, $(C_3-C_6)$cycloalkyl $(C_1-C_6)$alkylsulfinyl, $(C_3-C_6)$cycloalkyl $(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represent sulfanyl; or
$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted

($C_3$-$C_6$)cycloalkyl and which is optionally interrupted by heteroatoms from the group consisting of 0, S and N; or $R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl and which is optionally interrupted by heteroatoms from the group consisting of 0, S and N;
W represents hydrogen or fluorine;
X and Y independently of one another represent hydrogen, fluorine, chlorine, bromine, methyl, ethyl, trifluoromethyl, 2,2-difluoromethyl, difluoromethoxy, trifluoromethoxy, cyclopropyl, amino, hydroxyl or nitro;
Z represents hydrogen; and
n represents the number 0 or 1.

**9.** Compound according to Claim 7 or 8, in which the substructure of the formula (I-B) represents a substructure which is selected from the group consisting of

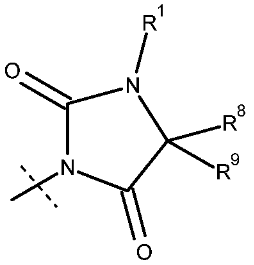

(I-B-1)

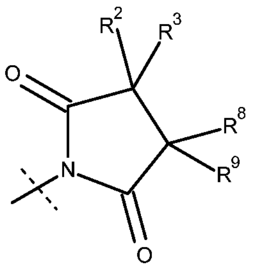

(I-B-4)

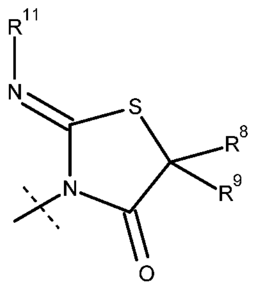

(I-B-5)

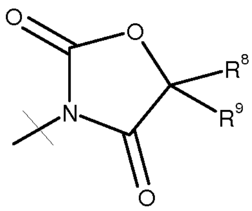

(I-B-2)

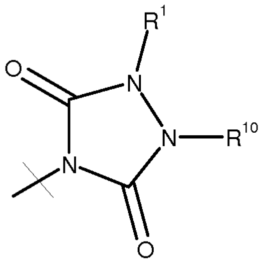

(I-B-6)

where
$R^1$, $R^{10}$ and $R^{11}$ each independently of one another
represent hydrogen, methyl, ethyl, propyl, isopropyl, n-butyl, cyclopropyl, cyclopropylmethyl, $CH_2CF_3$, $CH_2CHF_2$, $CH(CH_3)CF_3$, $CH_2CH_2CF_3$, $CH_2CH_2CHF_2$, $CH_2C(CH_3)_3$, $CH_2CH(CH_3)_2$, $CH_2CH=CH_2$ (= allyl), $CH_2CCH$ or benzyl;
$R^2$, $R^3$, $R^8$ and $R^9$ each independently of one another
represent hydrogen, methyl, trifluoromethyl or phenyl;

W represents hydrogen or fluorine;
X represents hydrogen, chlorine, fluorine or methyl;
Y represents chlorine, bromine, methyl, trifluoromethyl or fluorine;
X and Y represent in particular the following (Y,X) combinations: (Me, F), (Me,H), (Me,Cl), (Me,Me), (Cl,Cl), (Cl,F), (Cl, H), (Br, H), (Br, F), (F,F), ($CF_3$, H);
Z represents hydrogen; and
n represents the number 0 or 1.

**10.** Compound according to any of Claims 1 to 3, in which

A and B together with the atoms to which they are attached represent a substructure of the formula (I-C)

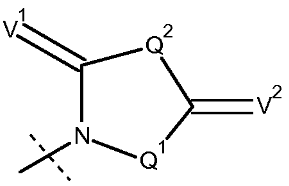

(I-C)

where

$V^1$ and $V^2$ each independently of one another represent oxygen or sulfur;

$Q^1$ represents oxygen, sulfur, $NR_1$ or $CR_2R_3$;

$Q^2$ represents $NR_{10}$ or $CR_8R_9$;

$R^1$ represents cyano or nitro; or

represents $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy $(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl $(C_1-C_6)$alkyl, halo$(C_1-C_6)$alkylsulfanyl$(C_1-C_6)$alkyl, halo $(C_1-C_6)$alkylsulfinyl$(C_1-C_6)$alkyl, halo$(C_1-C_6)$alkylsulfonyl$(C_1-C_6)$ alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$ alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl $(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated $(C_3-C_6)$cycloalkyl which may optionally be interrupted by one or more heteroatoms; or

represents halo$(C_1-C_6)$alkylcarbonyl, hydroxy$(C_1-C_6)$alkylcarbonyl, $(C_1-C_6)$alkoxy $(C_1-C_6)$ alkylcarbonyl, phenylcarbonyl, hetarylcarbonyl, halo$(C_1-C_6)$alkoxycarbonyl, aminocarbonyl, $(C_1-C_6)$alkylaminocarbonyl, di(Ci-$C_6$)alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$ alkyl) aminocarbonyl, arylaminocarbonyl, diarylaminocarbonyl, $(C_1-C_6)$alkyl(aryl) aminocarbonyl, $(C_3-C_6)$cycloalkyl(aryl)aminocarbonyl, $(C_1-C_6)$alkylaminothiocarbonyl, di(Ci-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represents carbonyl or carboxyl; or

represents optionally substituted phenyl or optionally substituted hetaryl; or

represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl $(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl $(C_1-C_6)$ alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^{10}$ represents, cyano or nitro; or

represents $(C_1-C_6)$alkyl, $(C_3-C_6)$cycloalkyl $(C_1-C_6)$alkyl, halo$(C_2-C_6)$alkyl, cyano $(C_1-C_6)$alkyl, hydroxy $(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulfanyl$(C_1-C_6)$ alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo$(C_1-C_6)$alkylsulfinyl$(C_1-C_6)$alkyl, halo$(C_1-C_6)$alkylsulfonyl$(C_1-C_6)$ alkyl, $(C_1-C_6)$alkoxy$(C_1-C_6)$alkylsulfanyl$(C_1-C_6)$alkyl, $(C_1-C_6)$alkoxy$(C_1-C_6)$ alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, $(C_1-C_6)$alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated $(C_3-C_6)$cycloalkyl which may optionally be interrupted by one or more heteroatoms; or

represents halo$(C_1-C_6)$alkylcarbonyl, hydroxy $(C_1-C_6)$alkylcarbonyl, $(C_1-C_6)$alkoxy$(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl) aminocarbonyl, arylaminocarbonyl, diarylaminocarbonyl, $(C_1-C_6)$alkyl (aryl) aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represents carbonyl or carboxyl; or

represents optionally substituted phenyl or optionally substituted hetaryl; or

represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represents $(C_1-C_6)$alkylsulfanyl, $(C_1-C_6)$alkylsulfinyl, $(C_1-C_6)$alkylsulfonyl, halo $(C_1-C_6)$ alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl $(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl $(C_1-C_6)$ alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; and

$R^2$, $R^3$, $R^8$ and $R^9$ each independently of one another

represent hydrogen, cyano, halogen or nitro; or

represent $(C_1-C_6)$alkyl, $(C_3-C_6)$cycloalkyl $(C_1-C_6)$alkyl, halo$(C_1-C_6)$alkyl, cyano $(C_1-C_6)$alkyl, hydroxy $(C_1-C_6)$alkyl, $(C_1-C_6)$alkoxy $(C_1-C_6)$alkyl, amino $(C_1-C_6)$alkyl, $(C_1-C_6)$alkoxycarbonyl$(C_1-C_6)$alkyl, $(C_1-C_6)$alkylthio $(C_1-C_6)$alkyl, halo$(C_1-C_6)$alkylthio$(C_1-C_6)$alkyl, $(C_1-C_6)$alkoxy $(C_1-C_6)$alkylthioalkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo$(C_1-C_6)$alkylsulfinyl$(C_1-C_6)$alkyl, halo$(C_1-C_6)$alkylsulfonyl$(C_1-C_6)$alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy $(C_1-C_6)$alkyl, phenylsulfanyl $(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy $(C_1-C_6)$alkyl or hetarylthio$(C_1-C_6)$alkyl, where the aforementioned radicals may each be saturated or unsaturated and/or optionally be substituted; or

represent optionally substituted saturated or unsaturated $(C_3-C_6)$cycloalkyl which may optionally be interrupted by one or more heteroatoms; or

represent $(C_1-C_6)$alkylcarbonyl, halo$(C_1-C_6)$alkylcarbonyl, hydroxy$(C_1-C_6)$alkylcarbonyl,

$(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represent carbonyl or carboxyl; or

represent optionally substituted phenyl or optionally substituted hetaryl;

represent $(C_1-C_6)$alkoxy, halo$(C_1-C_6)$alkoxy, $(C_1-C_6)$ alkoxy $(C_1-C_6)$ alkoxy, aryloxy, aryl $(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or represent hydroxyl; or

represent $(C_1-C_6)$alkylamino, di$(C_1-C_6)$alkylamino, halo $(C_1-C_6)$ alkylamino, dihalo$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl)amino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represent $(C_1-C_6)$alkylsulfanyl, $(C_1-C_6)$alkylsulfinyl, $(C_1-C_6)$alkylsulfonyl, halo $(C_1-C_6)$ alkylsulfanyl, halo$(C_1-C_6)$alkylsulfinyl, halo$(C_1-C_6)$alkylsulfonyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl $(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; or

$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of 0, S and N; or

$R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of 0, S and N;

W represents hydrogen, fluorine or chlorine;

X, Y and Z each independently of one another

represent hydrogen, fluorine, chlorine, bromine, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;

or represent a benzyl, phenoxy, phenylthio, cyclopropylmethyl, cyclopropyloxy or cyclopropylthio, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl;

or represent cyclopropyl, cyclobutyl, cyclopentyl, cyclohexyl or phenyl, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl; and

n represents the number 0 or 1.

**11.** Compound according to Claim 10, in which the substructure of the formula (I-C) represents a substructure which is selected from the group consisting of

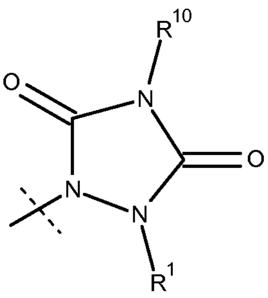

(I-C-1)

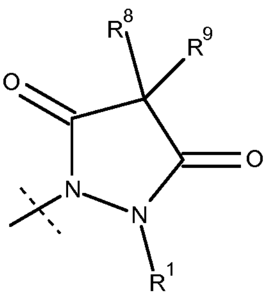

(I-C-2)

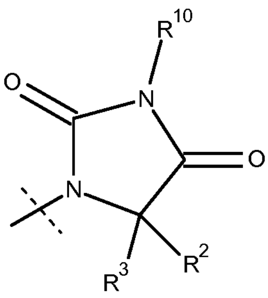

(I-C-3)

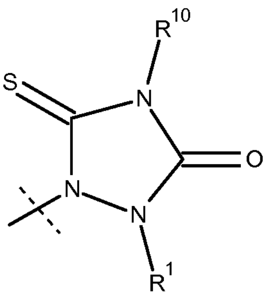

(I-C-4)

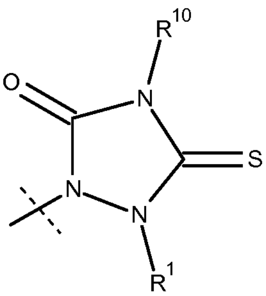

(I-C-5)

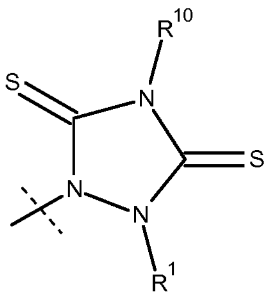

(I-C-6)

where

$R^1$ represents $(C_1-C_6)$ alkyl, $(C_3-C_6)$cycloalkyl$(C_1-C_6)$alkyl, halo$(C_2-C_6)$alkyl, cyano$(C_1-C_6)$alkyl, $(C_1-C_6)$alkoxy$(C_1-C_6)$alkyl, $(C_1-C_6)$alkoxycarbonyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl $(C_1-C_6)$alkyl, $(C_2-C_6)$alkynyl, where the aforementioned radicals may optionally be substituted; or

represents optionally substituted $(C_3-C_6)$cycloalkyl, $(C_3-C_6)$cycloalkenyl, which may optionally be interrupted by one or more heteroatoms from the group of 0, S and N; or

represents halo$(C_1-C_6)$alkylcarbonyl, $(C_1-C_6)$alkoxy $(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or are carbonyl or carboxyl; or

represents optionally substituted phenyl or optionally substituted hetaryl; or represents $(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or

represents $(C_1-C_6)$alkylsulfanyl, $(C_1-C_6)$alkylsulfinyl, $(C_1-C_6)$alkylsulfonyl, halo $(C_1-C_6)$ alkylsulfanyl, halo$(C_1-C_6)$alkylsulfinyl, halo$(C_1-C_6)$alkylsulfanyl, $(C_3-C_6)$cycloalkyl $(C_1-C_6)$alkylsulfanyl, $(C_3-C_6)$cycloalkyl $(C_1-C_6)$alkylsulfinyl, $(C_3-C_6)$cycloalkyl $(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and

$R^{10}$ represents $(C_1-C_6)$alkyl, $(C_3-C_6)$cycloalkyl $(C_1-C_6)$alkyl, halo$(C_2-C_6)$alkyl, cyano $(C_1-C_6)$alkyl, $(C_1-C_6)$alkoxy $(C_1-C_6)$alkyl, $(C_1-C_6)$alkoxycarbonyl$(C_1-C_6)$alkyl, phenyl$(C_1-C_6)$alkyl, hetaryl $(C_1-C_6)$alkyl, $(C_2-C_6)$alkenyl, halo $(C_2-C_6)$alkenyl, $(C_3-C_6)$cycloalkenyl $(C_1-C_6)$alkyl, $(C_2-C_6)$alkynyl, where the aforementioned radicals may optionally be substituted; or

represents optionally substituted $(C_3-C_6)$cycloalkyl, $(C_3-C_6)$cycloalkenyl, which may optionally be interrupted by one or more heteroatoms from the group of 0, S and N; or

represents $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or represents optionally substituted phenyl or optionally substituted hetaryl; or

represents $(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or

represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and

$R^2$, $R^3$, $R^8$ and $R^9$ each independently of one another

represent hydrogen, cyano, halogen or nitro; or

represent $(C_1-C_6)$alkyl, $(C_3-C_6)$cycloalkyl $(C_1-C_6)$alkyl, halo$(C_1-C_6)$alkyl, cyano$(C_1-C_6)$alkyl, $(C_1-C_6)$alkoxy$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may each optionally be substituted; or represent optionally substituted $(C_3-C_6)$cycloalkyl, $(C_3-C_6)$cycloalkenyl, which may optionally be interrupted by one or more heteroatoms from the group of 0, S and N; or

represent $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or represent optionally substituted phenyl or optionally substituted hetaryl; or

represent $(C_1-C_6)$alkoxy, halo$(C_1-C_6)$alkoxy, $(C_1-C_6)$alkoxy$(C_1-C_6)$alkoxy, $(C_3-C_6)$cycloalkyloxy, $(C_3-C_6)$cycloalkyl$(C_1-C_6)$alkyloxy, (C2-$C_6$)alkenyloxy, $(C_3-C_6)$cycloalkenyl$(C_1-C_6)$alkoxy, $(C_2-C_6)$alkynyloxy or carbonyloxy, where the aforementioned radicals may each optionally be substituted, or represent hydroxyl; or

represent $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or

represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represent sulfanyl; or

$R^2$ and $R^3$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of 0, S and N; or

$R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of 0, S and N;

W represents hydrogen or fluorine;

X and Y each independently of one another represent hydrogen, fluorine, chlorine, bromine, methyl, ethyl, trifluoromethyl, (2,2)-difluoromethyl, difluoromethoxy, trifluoromethoxy, cyclopropyl, amino, hydroxyl or nitro;
Z represents hydrogen; and
n represents the number 0 or 1.

**12.** Compound according to Claim 10 or 11, in which the substructure of the formula (I-C) represents a substructure which is selected from the group consisting of

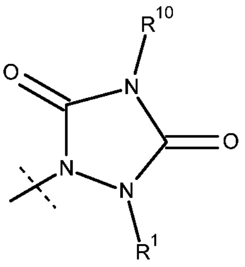
(I-C-1)

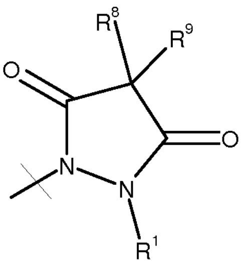
(I-C-2)

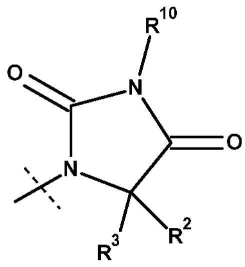
(I-C-3)

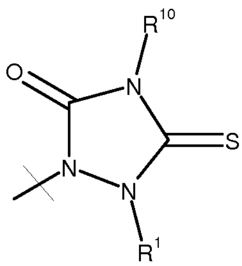
(I-C-5)

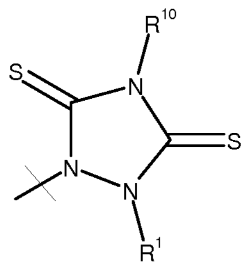
(I-C-6)

where

$R^1$ and $R^{10}$ each independently of one another
represent $(C_1\text{-}C_4)$alkyl, $(C_2\text{-}C_4)$haloalkyl, $(C_1\text{-}C_3)$alkoxy$(C_1\text{-}C_4)$alkyl or $(C_2\text{-}C_4)$alkenyl; or
represent saturated or unsaturated $(C_3\text{-}C_6)$cycloalkyl, $(C_3\text{-}C_6)$cycloalkyl $(C_1\text{-}C_2)$alkyl, phenyl or phenyl$(C_1\text{-}C_2)$alkyl, where the aforementioned radicals may each be mono- or polysubstituted by halogen, cyano, nitro, amino, hydroxy, methyl, ethyl, trifluoromethyl, difluoromethyl, trifluoroethyl, difluoroethyl, methoxy, ethoxy, trifluoromethoxy, trifluoroethoxy, trifluoroethylsulfanyl or optionally methyl-, fluorine-, chlorine-, cyano-substituted cyclopropyl;

$R^2$, $R^3$, $R^8$ and $R^9$ each independently of one another

represent hydrogen; or
represent $(C_1\text{-}C_6)$alkyl, $(C_2\text{-}C_6)$alkenyl, $(C_2\text{-}C_6)$alkynyl, halo$(C_1\text{-}C_6)$alkyl or phenyl$(C_1\text{-}C_3)$alkyl, where the aforementioned radicals may each be mono- or polysubstituted by halogen, cyano, nitro, amino, hydroxy, methyl, ethyl, trifluoromethyl, difluoromethyl, trifluoroethyl, difluoroethyl, methoxy, ethoxy, trifluoromethoxy, trifluoroethoxy, trifluoroethylsulfanyl or optionally methyl-, fluorine-, chlorine-, cyano-substituted cyclopropyl;
represent $(C_3\text{-}C_6)$cycloalkyl, or phenyl, where the aforementioned radicals may each be mono- or polysubstituted by halogen, cyano, nitro, amino, hydroxy, methyl, ethyl, trifluoromethyl, difluoromethyl, trifluoroethyl, difluoroethyl, methoxy, ethoxy, trifluoromethoxy, trifluoroethoxy, trifluoroethylsulfanyl or optionally methyl-, fluorine-, chlorine-, cyano-substituted cyclopropyl;
$R^2$ and $R^3$ together with the atom to which they are attached may form a cyclopropyl, cyclobutyl, cyclopentyl or cyclohexyl ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3\text{-}C_6)$cycloalkyl; or

$R^8$ and $R^9$ together with the atom to which they are attached may form a cyclopropyl, cyclobutyl, cyclopentyl or cyclohexyl ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted ($C_3$-$C_6$)cycloalkyl;
W represents hydrogen or fluorine;
X represents hydrogen, chlorine, fluorine or methyl;
Y represents chlorine, bromine, methyl, trifluoromethyl or fluorine;
where X and Y represent in particular the following combinations (Y,X): (Me, F), (Me,H), (Me,Cl), (Me,Me), (Cl,Cl), (Cl,F), (Cl,H), (Me,H), (Br,H), (Br,F), (F,F), ($CF_3$, H) ;
Z represents hydrogen; and
n represents the number 0 or 1.

**13.** Compound according to any of Claims 1 to 3, in which

A and B together with the atoms to which they are bonded are a substructure of the formula (I-D)

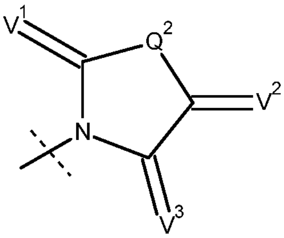

(I-D)

where
$V^1$, $V^2$ and $V^3$ each independently of one another represent oxygen; sulfur, $NR_{11}$ or a salt of $NR_{11}$;

$Q^2$ represents $NR^{10}$ or $CR^8R^9$;
$R^{10}$ represents hydrogen, cyano or nitro; or
represents ($C_1$-$C_6$)alkyl, (C3-$C_6$)cycloalkyl($C_1$-$C_6$)alkyl, halo($C_2$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulfanyl($C_1$-$C_6$)alkyl, ($C_1$-$C_6$)alkylsulfinyl($C_1$-$C_6$)alkyl, ($C_1$-$C_6$)alkylsulfonyl($C_1$-$C_6$)alkyl, halo($C_1$-$C_6$)alkylsulfanyl($C_1$-$C_6$)alkyl, halo($C_1$-$C_6$)alkylsulfinyl($C_1$-$C_6$)alkyl, halo($C_1$-$C_6$)alkylsulfonyl($C_1$-$C_6$)alkyl, ($C_1$-$C_6$)alkoxy($C_1$-$C_6$)alkylsulfanyl($C_1$-$C_6$)alkyl, ($C_1$-$C_6$)alkoxy($C_1$-$C_6$)alkylsulfinyl($C_1$-$C_6$)alkyl, ($C_1$-$C_6$)alkoxy($C_1$-$C_6$)alkylsulfonyl($C_1$-$C_6$)alkyl, phenyl($C_1$-$C_6$)alkyl, phenoxy($C_1$-$C_6$)alkyl, phenylsulfanyl($C_1$-$C_6$)alkyl, phenylsulfinyl($C_1$-$C_6$)alkyl, phenylsulfonyl($C_1$-$C_6$)alkyl, hetaryl($C_1$-$C_6$)alkyl, hetaryloxy($C_1$-$C_6$)alkyl, hetarylsulfanyl($C_1$-$C_6$)alkyl, hetarylsulfinyl($C_1$-$C_6$)alkyl, hetarylsulfonyl($C_1$-$C_6$)alkyl, arylcarbonyl ($C_1$-$C_6$)alkoxycarbonyl, aryloxycarbonyl, ($C_1$-$C_6$)alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or
represents optionally substituted saturated or unsaturated ($C_3$-$C_6$)cycloalkyl which may optionally be interrupted by one or more heteroatoms; or
represents halo($C_1$-$C_6$)alkylcarbonyl, hydroxy($C_1$-$C_6$)alkylcarbonyl, ($C_1$-$C_6$)alkoxy($C_1$-$C_6$)alkylcarbonyl, phenylcarbonyl, hetarylcarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represents carbonyl or carboxyl; or
represents optionally substituted phenyl or optionally substituted hetaryl; or represents ($C_1$-$C_6$)alkoxy, halo($C_1$-$C_6$)alkoxy, ($C_3$-$C_6$)cycloalkyloxy, aryloxy, aryl($C_1$-$C_6$)alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represents amino; or

represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represents sulfanyl;

$R^{11}$ represents hydrogen, cyano or nitro; or

represents $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo$(C_1-C_6)$alkylsulfinyl$(C_1-C_6)$alkyl, halo$(C_1-C_6)$alkylsulfonyl$(C_1-C_6)$alkyl, $(C_1-C_6)$alkoxy$(C_1-C_6)$alkylsulfanyl$(C_1-C_6)$alkyl, $(C_1-C_6)$alkoxy $(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy$(C_1-C_6)$alkyl, phenylsulfanyl$(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl, hetarylsulfanyl$(C_1-C_6)$alkyl, hetarylsulfinyl$(C_1-C_6)$alkyl, hetarylsulfonyl$(C_1-C_6)$alkyl, $(C_1-C_6)$alkoxycarbonyl, aryloxycarbonyl, $(C_1-C_6)$alkylcarbonyl, arylcarbamoyl, where the aforementioned radicals may each optionally be substituted and/or, under the condition that, in the case of an unsaturated radical, the defined minimum number of carbon atoms is 2, the radicals may be saturated or unsaturated; or

represents optionally substituted saturated or unsaturated $(C_3-C_6)$cycloalkyl which may optionally be interrupted by one or more heteroatoms; or

represents halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represents carbonyl or carboxyl; or

represents optionally substituted phenyl or optionally substituted hetaryl; or represents $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or represents hydroxyl; or

represents $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represents amino; or

represents $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfanyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represents sulfanyl; and

$R^8$ and $R^9$ each independently of one another

represent hydrogen, cyano, halogen or nitro; or

represent $(C_1-C_6)$alkyl, $(C_3-C_6)$cycloalkyl$(C_1-C_6)$alkyl, halo$(C_1-C_6)$alkyl, cyano $(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylthio$(C_1-C_6)$alkyl, halo$(C_1-C_6)$alkylthio$(C_1-C_6)$alkyl, $(C_1-C_6)$alkoxy$(C_1-C_6)$alkylthioalkyl, $(C_1-C_6)$alkylsulfanyl$(C_1-C_6)$alkyl, $(C_1-C_6)$alkylsulfinyl$(C_1-C_6)$alkyl, $(C_1-C_6)$alkylsulfonyl$(C_1-C_6)$alkyl, halo$(C_1-C_6)$alkylsulfanyl$(C_1-C_6)$alkyl, halo$(C_1-C_6)$alkylsulfinyl$(C_1-C_6)$alkyl, halo$(C_1-C_6)$alkylsulfonyl$(C_1-C_6)$alkyl, $(C_1-C_6)$alkoxy$(C_1-C_6)$alkylsulfanyl$(C_1-C_6)$alkyl, $(C_1-C_6)$alkoxy$(C_1-C_6)$alkylsulfinyl$(C_1-C_6)$alkyl, $(C_1-C_6)$alkoxy$(C_1-C_6)$alkylsulfonyl$(C_1-C_6)$alkyl, phenyl$(C_1-C_6)$alkyl, phenoxy $(C_1-C_6)$alkyl, phenylsulfanyl $(C_1-C_6)$alkyl, phenylsulfinyl$(C_1-C_6)$alkyl, phenylsulfonyl$(C_1-C_6)$alkyl, hetaryl$(C_1-C_6)$alkyl, hetaryloxy$(C_1-C_6)$alkyl or hetarylthio$(C_1-C_6)$alkyl, where the aforementioned radicals may each be saturated or unsaturated and/or optionally be substituted; or

represent optionally substituted saturated or unsaturated $(C_3-C_6)$cycloalkyl which may optionally be interrupted by one or more heteroatoms; or

represent $(C_1-C_6)$alkylcarbonyl, halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl(aryl)aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or represent carbonyl or carboxyl; or

represent optionally substituted phenyl or optionally substituted hetaryl;

represent $(C_1-C_6)$alkoxy, halo$(C_1-C_6)$alkoxy, $(C_1-C_6)$alkoxy $(C_1-C_6)$alkoxy, aryloxy, aryl$(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or represent hydroxyl; or

represent $(C_1-C_6)$alkylamino, di$(C_1-C_6)$alkylamino, halo$(C_1-C_6)$alkylamino, dihalo$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl)amino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulfonylamino, or arylsulfonylamino, where the aforementioned radicals may each optionally be substituted and/or, under the condition that, in the case of an unsaturated radical, the defined minimum number of carbon atoms is 2, the radicals may be saturated or unsaturated, or represent amino; or

represent $(C_1-C_6)$alkylsulfanyl, $(C_1-C_6)$alkylsulfinyl, $(C_1-C_6)$alkylsulfonyl, halo$(C_1-C_6)$alkylsulfanyl, halo$(C_1-C_6)$alkylsulfinyl, halo$(C_1-C_6)$alkylsulfonyl, $(C_3-C_6)$cycloalkylsulfanyl, $(C_3-C_6)$cycloalkylsulfinyl, $(C_3-C_6)$cycloalkylsulfonyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl,

arylsulfanyl, arylsulfinyl, arylsulfonyl, aryl$(C_1-C_6)$alkylsulfanyl, aryl$(C_1-C_6)$alkylsulfinyl, aryl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted and/or, under the condition that, in the case of an unsaturated radical, the defined minimum number of carbon atoms is 2, the radicals may be saturated or unsaturated, or represent sulfanyl; or

$R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of O, S and N;

W represents hydrogen, fluorine or chlorine;

X, Y and Z each independently of one another

represent hydrogen, fluorine, chlorine, bromine, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;

or represent a benzyl, phenoxy, phenylthio, cyclopropylmethyl, cyclopropyloxy or cyclopropylthio, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl;

or represent cyclopropyl, cyclobutyl, cyclopentyl, cyclohexyl or phenyl, which is optionally mono- or polysubstituted by identical or different substituents from the group consisting of fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl; and n represents the number 0 or 1.

**14.** Compound according to Claim 13, in which the substructure of the formula (I-D) represents a substructure which is selected from the group consisting of

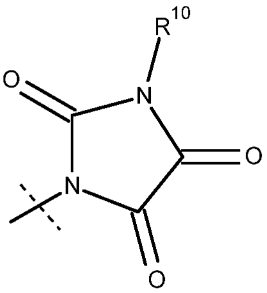
(I-D-1)

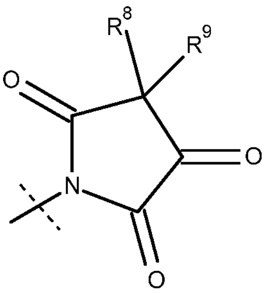
(I-D-2)

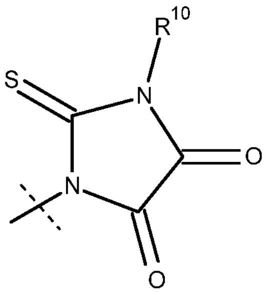
(I-D-3)

where

$R^{10}$ represents hydrogen or
represents $(C_1-C_6)$alkyl, $(C_3-C_6)$cycloalkyl$(C_1-C_6)$alkyl, halo$(C_2-C_6)$alkyl, cyano$(C_1-C_6)$alkyl, $(C_1-C_6)$alkoxy$(C_1-C_6)$alkyl, $(C_1-C_6)$alkoxycarbonyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may optionally be substituted; or
represents optionally substituted $(C_3-C_6)$cycloalkyl, $(C_3-C_6)$cycloalkenyl, which may optionally be interrupted by one or more heteroatoms from the group of O, S and N; or
represents $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represents carbonyl or carboxyl; or
represents optionally substituted phenyl or optionally substituted hetaryl; or
represents $(C_1-C_6)$alkoxy, halo$(C_1-C_6)$alkoxy, $(C_3-C_6)$cycloalkyloxy, or carbonyloxy, where the aforementioned radicals may each optionally be substituted, or represents hydroxyl; or
represents $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represents amino; or
represents $(C_1-C_6)$alkylsulfanyl, $(C_1-C_6)$alkylsulfinyl, $(C_1-C_6)$alkylsulfonyl, halo $(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represents sulfanyl; and
$R^8$ and $R^9$ each independently of one another
represent hydrogen, cyano, halogen or nitro; or
represent $(C_1-C_6)$alkyl, $(C_3-C_6)$cycloalkyl$(C_1-C_6)$alkyl, halo$(C_1-C_6)$alkyl, cyano $(C_1-C_6)$alkyl, $(C_1-C_6)$alkoxy$(C_1-C_6)$alkyl, $(C_1-C_6)$alkoxycarbonyl$(C_1-C_6)$alkyl, $(C_1-C_6)$alkylsulfanyl$(C_1-C_6)$alkyl, $(C_1-C_6)$alkylsulfinyl$(C_1-C_6)$alkyl, phenyl$(C_1-C_6)$alkyl, hetaryl$(C_1-C_6)$alkyl, $(C_2-C_6)$alkenyl, halo$(C_2-C_6)$alkenyl, $(C_3-C_6)$cycloalkenyl$(C_1-C_6)$alkyl, $(C_2-C_6)$alkynyl, where the aforementioned radicals may each optionally be substituted; or
represent optionally substituted $(C_3-C_6)$cycloalkyl, $(C_3-C_6)$cycloalkenyl, which may optionally be interrupted by one or more heteroatoms from the group of 0, S and N; or
represent $(C_1-C_6)$alkylcarbonyl, halo$(C_1-C_6)$alkylcarbonyl, $(C_1-C_6)$alkoxy$(C_1-C_6)$alkylcarbonyl, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbo-

nyl, $(C_3-C_6)$cycloalkylaminocarbonyl, $(C_1-C_6)$alkylaminothiocarbonyl, di$(C_1-C_6)$alkylaminothiocarbonyl, $(C_2-C_6)$alkenylcarbonyl, $(C_3-C_6)$cycloalkenyl$(C_1-C_6)$alkylcarbonyl, $(C_2-C_6)$alkynylcarbonyl or aminothiocarbonyl, where the aforementioned radicals may each optionally be substituted, or represent carbonyl or carboxyl; or represent optionally substituted phenyl or optionally substituted hetaryl; or

represent $(C_1-C_6)$alkoxy, halo$(C_1-C_6)$alkoxy, $(C_1-C_6)$alkoxy $(C_1-C_6)$alkoxy, $(C_3-C_6)$cycloalkyloxy, $(C_3-C_6)$cycloalkyl$(C_1-C_6)$alkyloxy, $(C_2-C_6)$alkenyloxy, $(C_3-C_6)$cycloalkenyl$(C_1-C_6)$alkoxy, $(C_2-C_6)$alkynyloxy or carbonyloxy, where the aforementioned radicals may each optionally be substituted, or represent hydroxyl; or

represent $(C_1-C_6)$alkylamino, halo$(C_1-C_6)$alkylamino, dihalo$(C_1-C_6)$alkylamino, di $(C_1-C_6)$alkylamino, $(C_3-C_6)$cycloalkylamino, di$(C_3-C_6)$cycloalkylamino, $(C_1-C_6)$alkylcarbonylamino, $(C_1-C_6)$alkoxycarbonylamino, $(C_1-C_6)$alkylcarbamoylamino, $(C_1-C_6)$alkylsulfonylamino, $(C_2-C_6)$alkenylamino, $(C_3-C_6)$cycloalkenyl$(C_1-C_6)$alkylamino, $(C_2-C_6)$alkynylamino, where the aforementioned radicals may each optionally be substituted, or represent amino; or

represent $(C_1-C_6)$alkylsulfanyl, $(C_1-C_6)$alkylsulfinyl, $(C_1-C_6)$alkylsulfonyl, halo $(C_1-C_6)$alkylsulfanyl, halo$(C_1-C_6)$alkylsulfinyl, halo$(C_1-C_6)$alkylsulfanyl, $(C_3-C_6)$cycloalkyl$(C_1-C_6)$alkylsulfanyl, $(C_3-C_6)$cycloalkyl$(C_1-C_6)$alkylsulfinyl, $(C_3-C_6)$cycloalkyl$(C_1-C_6)$alkylsulfonyl, aminosulfonyl, $(C_1-C_6)$alkylaminosulfonyl, di$(C_1-C_6)$alkylaminosulfonyl or arylaminosulfonyl, where the aforementioned radicals may each optionally be substituted, or represent sulfanyl; or

$R^8$ and $R^9$ together with the atom to which they are attached may form a saturated or unsaturated three- to six-membered ring which is optionally substituted by identical or different substituents from the group consisting of halogen, cyano, nitro, hydroxy, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted $(C_3-C_6)$cycloalkyl and which is optionally interrupted by heteroatoms from the group consisting of 0, S and N;

W represents hydrogen or fluorine;

X and Y each independently of one another represent hydrogen, fluorine, chlorine, bromine, methyl, ethyl, trifluoromethyl, 2,2-difluoromethyl, difluoromethoxy, trifluoromethoxy, cyclopropyl, amino, hydroxyl or nitro;

Z represents hydrogen; and

n represents the number 0 or 1.

**15.** Compound according to Claim 13 or 14, in which the substructure of the formula (I-D) represents a substructure which is selected from the group consisting of

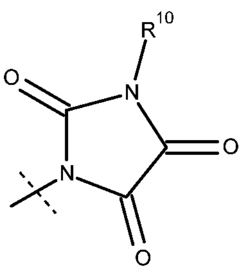
(I-D-1)

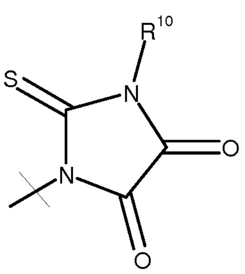
(I-D-3)

where

$R^{10}$ represents hydrogen, methyl, ethyl, propyl, isopropyl, tert-butyl, cyclopropylmethyl, $CH_2CH(CH_3)_2$, $CH_2C(CH_3)_3$, $CH_2CF_3$, $CH_2CHF_2$, $CH(CH_3)$ $CF_3$, $CH_2CH_2CF_3$, $CH_2CH_2CHF_2$ or optionally methyl-, fluorine-, chlorine-, cyano-substituted cyclopropyl or optionally methyl-, fluorine-, chlorine-, cyano-substituted cyclohexyl or optionally methyl-, fluorine-, chlorine-, cyano-substituted cyclopropylmethyl;

W represents hydrogen or fluorine;

X represents hydrogen, chlorine, fluorine or methyl;

Y represents chlorine, bromine, methyl, trifluoromethyl or fluorine;

where X and Y represent in particular the following combinations (Y,X): (Me, F), (Me,H), (Me,Cl), (Me,Me), (Cl,Cl), (Cl,F), (Cl,H), (Me,H), (Br,H), (Br,F), (F,F), $(CF_3,H)$ ;

Z represents hydrogen; and

n represents the number 0 or 1.

**16.** Active compound composition comprising at least one compound of the general formula (I) according to any of

Claims 1 to 15 and at least one further insecticidally, acaricidally or nematicidally active compound selected from the group consisting of

(1) Acetylcholinesterase (AChE) inhibitors, for example
carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or
organophosphates, for example acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon and vamidothion.
(2) GABA-gated chloride channel antagonists, for example
cyclodiene organochlorines, for example chlordane and endosulfan; or
phenylpyrazoles (fiproles), for example ethiprole and fipronil.
(3) Sodium channel modulators/voltage-dependent sodium channel blockers, for example
pyrethroids, for example acrinathrin, allethrin, d-cis-trans allethrin, d-trans allethrin, bifenthrin, bioallethrin, bioallethrin S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomers], deltamethrin, empenthrin [(EZ)-(1R) isomers], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrine (pyrethrum), resmethrin, silafluofen, tefluthrin, tetramethrin, tetramethrin [(1R) isomers], tralomethrin and transfluthrin; or
DDT; or methoxychlor.
(4) Nicotinergic acetylcholine receptor (nAChR) agonists, for example
neonicotinoids, for example acetamiprid, clothianidin, dinotefuran, imidacloprid, nitenpyram, thiacloprid and thiamethoxam; or
nicotine.
(5) Nicotinergic acetylcholine receptor (nAChR) allosteric activators, for example spinosyns, for example spinetoram and spinosad.
(6) Chloride channel activators, for example
avermectins/milbemycins, for example abamectin, emamectin benzoate, lepimectin and milbemectin.
(7) Juvenile hormone imitators, for example
juvenile hormone analogs, for example hydroprene, kinoprene and methoprene; or
fenoxycarb; or pyriproxyfen.
(8) Active compounds having unknown or nonspecific mechanisms of action, for example
alkyl halides, for example methyl bromide and other alkyl halides; or
chloropicrin; or sulfuryl fluoride; or borax; or tartar emetic.
(9) Selective antifeedants, for example pymetrozine; or flonicamid.
(10) Mite growth inhibitors, for example clofentezine, hexythiazox and diflovidazin; or etoxazole.
(11) Microbial disruptors of the insect gut membrane, e.g. Bacillus thuringiensis subspecies israelensis, Bacillus sphaericus, Bacillus thuringiensis subspecies aizawai, Bacillus thuringiensis subspecies kurstaki, Bacillus thuringiensis subspecies tenebrionis, and BT plant proteins: Cry1Ab, Cry1Ac, Cry1Fa, Cry2Ab, mCry3A, Cry3Ab, Cry3Bb, Cry34/35Ab1.
(12) Oxidative phosphorylation inhibitors, ATP disruptors, for example diafenthiuron; or
organotin compounds, e.g. azocyclotin, cyhexatin and fenbutatin oxide; or
propargite; or tetradifon.
(13) Oxidative phosphorylation decouplers that interrupt the H proton gradient, for example chlorfenapyr, DNOC and sulfluramid.
(14) Nicotinergic acetylcholine receptor antagonists, for example bensultap, cartap hydrochloride, thiocyclam, and thiosultap-sodium.
(15) Chitin biosynthesis inhibitors, type 0, for example bistrifluron, chlorfluazuron, diflubenzuron, flucycloxuron, flufenoxuron, hexaflumuron, lufenuron, novaluron, noviflumuron, teflubenzuron and triflumuron.

(16) Chitin biosynthesis inhibitors, type 1, for example buprofezin.
(17) Molting disruptors, dipteran, for example cyromazine.
(18) Ecdysone receptor agonists, for example chromafenozide, halofenozide, methoxyfenozide and tebufenozide.
(19) Octopaminergic agonists, for example amitraz.
(20) Complex-III electron transport inhibitors, for example hydramethylnon; or acequinocyl; or fluacrypyrim.
(21) Complex-I electron transport inhibitors, for example
METI acaricides, e.g. fenazaquin, fenpyroximate, pyrimidifen, pyridaben, tebufenpyrad and tolfenpyrad; or rotenone (Derris).
(22) Voltage-gated sodium channel blockers, for example indoxacarb; or metaflumizone.
(23) Inhibitors of acetyl-CoA carboxylase, for example
tetronic and tetramic acid derivatives, e.g. spirodiclofen, spiromesifen and spirotetramat.
(24) Complex-IV electron transport inhibitors, for example
phosphines, for example aluminum phosphide, calcium phosphide, phosphine and zinc phosphide; or cyanide.
(25) Complex-II electron transport inhibitors, for example cyenopyrafen.
(28) Ryanodine receptor effectors, for example diamides, for example chlorantraniliprole and flubendiamide;
further active compounds having an unknown mechanism of action, for example amidoflumet, azadirachtin, benclothiaz, benzoximate, bifenazate, bromopropylate, chinomethionat, cryolite, cyantraniliprole (Cyazypyr), cyflumetofen, dicofol, diflovidazin, fluensulfone, flufenerim, flufiprole, fluopyram, fufenozide, imidaclothiz, iprodione, meperfluthrin, pyridalyl, pyrifluquinazon, tetramethylfluthrin and iodomethane; and additionally preparations based on Bacillus firmus (particularly strain CNCM I-1582, for example VOTiVO™, BioNem), and the following known active compounds:

3-bromo-N-{2-bromo-4-chloro-6-[(1-cyclopropylethyl)carbamoyl]phenyl}-1-(3-chloropyridin-2-yl)-1H-pyrazole-5-carboxamide, 4-{[(6-bromopyrid-3-yl)methyl](2-fluoroethyl)amino}furan-2(5H)-one, 4-{[(6-fluoropyrid-3-yl)methyl](2,2-difluoroethyl)amino}furan-2(5H)-one, 4-{[(2-chloro-1,3-thiazol-5-yl)methyl](2-fluoroethyl)amino}furan-2(5H)-one, 4-{[(6-chloropyrid-3-yl)methyl](2-fluoroethyl)amino}furan-2(5H)-one, flupyradifurone, 4-{[(6-chloro-5-fluoropyrid-3-yl)methyl](methyl)amino}furan-2(5H)-one, 4-{[(5,6-dichloropyrid-3-yl)methyl](2-fluoroethyl)amino}furan-2(5H)-one, 4-{[(6-chloro-5-fluoropyrid-3-yl)methyl](cyclopropyl)amino}furan-2(5H)-one, 4-{[(6-chloropyrid-3-yl)methyl](cyclopropyl)amino}furan-2(5H)-one, 4-{[(6-chloropyrid-3-yl)methyl](methyl)amino}furan-2(5H)-one, {[1-(6-chloropyridin-3-yl)ethyl](methyl)oxido-$\lambda^4$-sulfanylidene}cyanamide and its diastereomers {[(1R)-1-(6-chloropyridin-3-yl)ethyl](methyl)oxido-$\lambda^4$-sulfanylidene}cyanamide (A) and {[(1S)-1-(6-chloropyridin-3-yl)ethyl](methyl)oxido-$\lambda^4$-sulfanylidene}cyanamide (B) and also sulfoxaflor and its diastereomers [(R)-methyl(oxido){(1R)-1-[6-(trifluoromethyl)pyridin-3-yl]ethyl}-$\lambda^4$-sulfanylidene]cyanamide (A1) and [(S)-methyl(oxido){(1S)-1-[6-(trifluoromethyl)pyridin-3-yl]ethyl}-$\lambda^4$-sulfanylidene]cyanamide (A2), identified as diastereomer group A, [(R)-methyl(oxido){(1S)-1-[6-(trifluoromethyl)pyridin-3-yl]ethyl}-$\lambda^4$-sulfanylidene]cyanamide (B1) and [(S)-methyl(oxido){(1R)-1-[6-(trifluoromethyl)pyridin-3-yl]ethyl}-$\lambda^4$-sulfanylidene]cyanamide (B2), identified as diastereomer group B and 11-(4-chloro-2,6-dimethylphenyl)-12-hydroxy-1,4-dioxa-9-azadispiro[4.2.4.2]tetradec-11-en-10-one, 3-(4'-fluoro-2,4-dimethylbiphenyl-3-yl)-4-hydroxy-8-oxa-1-azaspiro[4.5]dec-3-en-2-one 1-{2-fluoro-4-methyl-5-[(2,2,2-trifluoroethyl)sulfinyl]phenyl}-3-(trifluoromethyl)-1H-1,2,4-triazole-5-amine, [(3S,4aR,12R,12aS,12bS)-3-[(cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropanecarboxylate, 2-cyano-3-(difluoromethoxy)-N,N-dimethylbenzenesulfonamide, 2-cyano-3-(difluoromethoxy)-N-methylbenzenesulfonamide, 2-cyano-3-(difluoromethoxy)-N-ethylbenzenesulfonamide, 4-(difluoromethoxy)-N-ethyl-N-methyl-1,2-benzothiazole-3-amine 1,1-dioxide, N-[1-(2,3-dimethylphenyl)-2-(3,5-dimethylphenyl)ethyl]-4,5-dihydro-1,3-thiazole-2-amine, {1'-[(2E)-3-(4-chlorophenyl)prop-2-en-1-yl]-5-fluorospiro[indol-3,4'-piperidin]-1(2H)-yl}(2-chloropyridin-4-yl)methanone, 3-(2,5-dimethylphenyl)-4-hydroxy-8-methoxy-1,8-diazaspiro[4.5]dec-3-en-2-one, 3-(2,5-dimethylphenyl)-8-methoxy-2-oxo-1,8-diazaspiro[4.5]dec-3-en-4-yl ethyl carbonate, 4-(but-2-yn-1-yloxy)-6-(3,5-dimethylpiperidin-1-yl)-5-fluoropyrimidine, (2,2,3,3,4,4,5,5-octafluoropentyl)(3,3,3-trifluoropropyl)malononitrile, (2,2,3,3,4,4,5,5-octafluoropentyl)(3,3,4,4,4-pentafluorobutyl)malononitrile, 8-[2-(cyclopropylmethoxy)-4-(trifluoromethyl)phenoxy]-3-[6-(trifluoromethyl)pyridazin-3-yl]-3-azabicyclo[3.2.1]octane, flometoquin, PF1364 (CAS Reg. No. 1204776-60-2), 5-[5-(3,5-dichlorophenyl)-5-(trifluoromethyl)-4,5-dihydro-1,2-oxazol-3-yl]-2-(1H-1,2,4-triazol-1-yl)benzonitrile, 5-[5-(2-chloropyridin-4-yl)-5-(trifluoromethyl)-4,5-dihydro-1,2-oxazol-3-yl]-2-(1H-1,2,4-triazol-1-yl)benzonitrile, 4-[5-(3,5-dichlorophenyl)-5-(trifluoromethyl)-4,5-dihydro-1,2-oxazol-3-yl]-2-methyl-N-{2-oxo-2-[(2,2,2-trifluoroethyl)amino]ethyl}benzamide, 4-{[(6-chloropyridin-3-yl)methyl](cyclopropyl)amino}-1,3-oxazol-2(5H)-one, 4-{[(6-chloropyridin-3-yl)methyl](2,2-difluoroethyl)amino}-1,3-oxazol-2(5H)-one, 4-{[(6-chloropyridin-3-yl)methyl](ethyl)amino}-1,3-oxazol-2(5H)-one, 4-{[(6-chloropyridin-3-yl)methyl](methyl)amino}-1,3-oxazol-2(5H)-one, NNI-0711, 1-acetyl-N-[4-(1,1,1,3,3,3-hexafluoro-2-methoxypropan-2-yl)-3-isobutylphenyl]-N-isobutyryl-3,5-dimethyl-1H-pyrazole-4-carboxamide, methyl 2-[2-({[3-bromo-1-(3-chloropyridin-2-yl)-1H-pyrazol-5-yl]carbonyl}amino)-5-chloro-3-methylbenzoyl]-2-methylhydrazinecarboxylate, methyl 2-[2-({[3-bromo-1-(3-chloropyridin-2-yl)-1H-pyrazol-5-yl]carbonyl}amino)-5-cyano-3-methylbenzoyl]-2-ethylhydrazinecarboxylate, methyl 2-[2-({[3-bromo-1-(3-chloropyridin-2-yl)-1H-pyrazol-5-yl]carbonyl}amino)-5-cyano-3-methylbenzoyl]-2-methylhydrazinecarboxylate, methyl 2-[3,5-dibromo-2-({[3-bromo-1-(3-chloropyridin-2-yl)-1H-pyrazol-5-yl]carbonyl}amino)benzoyl]-1,2-diethylhydrazinecarboxylate, methyl 2-[3,5-dibromo-2-({[3-bromo-1-(3-chloropyridin-2-yl)-1H-pyrazol-5-yl]carbonyl}amino)benzoyl]-2-ethylhydrazinecarboxylate, (5RS,7RS;5RS,7SR)-1-(6-chloro-3-pyridylmethyl)-1,2,3,5,6,7-hexahydro-7-methyl-8-nitro-5-propoxyimidazo[1,2-a]pyridine, 2-{6-[2-(5-fluoropyridin-3-yl)-1,3-thiazol-5-yl]pyridin-2-yl}pyrimidine, 2-{6-[2-(pyridin-3-yl)-1,3-thiazol-5-yl]pyridin-2-yl}pyrimidine, 1-(3-chloropyridin-2-yl)-N-[4-cyano-2-methyl-6-(methylcarbamoyl)phenyl]-3-{ [5-(trifluoromethyl)-1H-tetrazol-1-yl]methyl}-1H-pyrazole-5-carboxamide, 1-(3-chloropyridin-2-yl)-N-[4-cyano-2-methyl-6-(methylcarbamoyl)phenyl]-3-{[5-(trifluoromethyl)-2H-tetrazol-2-yl]methyl}-1H-pyrazole-5-carboxamide, N-[2-(tert-butylcarbamoyl)-4-cyano-6-methylphenyl]-1-(3-chloropyridin-2-yl)-3-{[5-(trifluoromethyl)-1H-tetrazol-1-yl]methyl}-1H-pyrazole-5-carboxamide, N-[2-(tert-butylcarbamoyl)-4-cyano-6-methylphenyl]-1-(3-chloropyridin-2-yl)-3-{[5-(trifluoromethyl)-2H-tetrazol-2-yl]methyl}-1H-pyrazole-5-carboxamide, (1E)-N-[(6-chloropyridin-3-yl)methyl]-N'-cyano-N-(2,2-difluoroethyl)ethanimidamide, N-[2-(5-amino-1,3,4-thiadiazol-2-yl)-4-chloro-6-methylphenyl]-3-bromo-1-(3-chloropyridin-2-yl)-1H-pyrazole-5-carboxamide and methyl 2-[3,5-dibromo-2-({[3-bromo-1-(3-chloropyridin-2-yl)-1H-pyrazol-5-yl]carbonyl}amino)benzoyl]-2-ethyl-1-methylhydrazinecarboxylate,

and/or at least one further active fungicidal ingredient selected from the group consisting of

(1) Inhibitors of ergosterol biosynthesis such as, for example, (1.1) aldimorph (1704-28-5), (1.2) azaconazole (60207-31-0), (1.3) bitertanol (55179-31-2), (1.4) bromuconazole (116255-48-2), (1.5) cyproconazole (113096-99-4), (1.6) diclobutrazole (75736-33-3), (1.7) difenoconazole (119446-68-3), (1.8) diniconazole (83657-24-3), (1.9) diniconazole-M (83657-18-5), (1.10) dodemorph (1593-77-7), (1.11) dodemorph acetate (31717-87-0), (1.12) epoxiconazole (106325-08-0), (1.13) etaconazole (60207-93-4), (1.14) fenarimol (60168-88-9), (1.15) fenbuconazole (114369-43-6), (1.16) fenhexamid (126833-17-8), (1.17) fenpropidin (67306-00-7), (1.18) fenpropimorph (67306-03-0), (1.19) fluquinconazole (136426-54-5), (1.20) flurprimidol (56425-91-3), (1.21) flusilazole (85509-19-9), (1.22) flutriafol (76674-21-0), (1.23) furconazole (112839-33-5), (1.24) furconazole-cis (112839-32-4), (1.25) hexaconazole (79983-71-4), (1.26) imazalil (60534-80-7), (1.27) imazalil sulfate (58594-72-2), (1.28) imibenconazole (86598-92-7), (1.29) ipconazole (125225-28-7), (1.30) metconazole (125116-23-6), (1.31) myclobutanil (88671-89-0), (1.32) naftifin (65472-88-0), (1.33) nuarimol (63284-71-9), (1.34) oxpoconazole (174212-12-5), (1.35) paclobutrazole (76738-62-0), (1.36) pefurazoate (101903-30-4), (1.37) penconazole (66246-88-6), (1.38) piperalin (3478-94-2), (1.39) prochloraz (67747-09-5), (1.40) propiconazole (60207-90-1), (1.41) prothioconazole (178928-70-6), (1.42) pyributicarb (88678-67-5), (1.43) pyrifenox (88283-41-4), (1.44) quinconazole (103970-75-8), (1.45) simeconazole (149508-90-7), (1.46) spiroxamine (118134-30-8), (1.47) tebuconazole (107534-96-3), (1.48) terbinafin (91161-71-6), (1.49) tetraconazole (112281-77-3), (1.50) triadimefon (43121-43-3), (1.51) triadimenol (89482-17-7), (1.52) tridemorph (81412-43-3), (1.53) triflumizole (68694-11-1), (1.54) triforine (26644-46-2), (1.55) triticonazole (131983-72-7), (1.56) uniconazole (83657-22-1), (1.57) uniconazole-p (83657-17-4), (1.58) viniconazole (77174-66-4), (1.59) voriconazole (137234-62-9), (1.60) 1-(4-chlorophenyl)-2-(1H-1,2,4-triazol-1-yl)cycloheptanol (129586-32-9), (1.61) methyl 1-(2,2-dimethyl-2,3-dihydro-1H-inden-1-yl)-1H-imidazole-5-carboxylate (110323-95-0), (1.62) N'-{5-(difluoromethyl)-2-methyl-4-[3-(trimethylsilyl)propoxy]phenyl}-N-ethyl-N-methylimidoformamide, (1.63) N-ethyl-N-methyl-N'-{2-methyl-5-(trifluoromethyl)-4-[3-(trimethylsilyl)propoxy]phenyl}imidoformamide and (1.64) O-[1-(4-methoxyphenoxy)-3,3-dimethylbutan-2-yl] 1H-imidazole-1-carbothioate (111226-71-2).

(2) Respiration inhibitors (respiratory chain inhibitors) such as, for example, (2.1) bixafen (581809-46-3), (2.2) boscalid (188425-85-6), (2.3) carboxin (5234-68-4), (2.4) diflumetorim (130339-07-0), (2.5) fenfuram (24691-80-3), (2.6) fluopyram (658066-35-4), (2.7) flutolanil (66332-96-5), (2.8) fluxapyroxad (907204-31-3), (2.9) furametpyr (123572-88-3), (2.10) furmecyclox (60568-05-0), (2.11) isopyrazam mixture of the syn-epimeric racemate 1RS,4SR,9RS and the anti-epimeric racemate 1RS,4SR,9SR (881685-58-1), (2.12) isopyrazam (anti-epimeric racemate), (2.13) isopyrazam (anti-epimeric enantiomer 1R,4S,9S), (2.14) isopyrazam (anti-epimeric enantiomer 1S,4R,9R), (2.15) isopyrazam (syn-epimeric racemate 1RS,4SR,9RS), (2.16) isopyrazam (syn-epimeric enantiomer 1R,4S,9R), (2.17) isopyrazam (syn-epimeric enantiomer 1S,4R,9S), (2.18) mepronil (55814-41-0), (2.19) oxycarboxin (5259-88-1), (2.20) penflufen (494793-67-8), (2.21) penthiopyrad (183675-82-3), (2.22) sedaxane (874967-67-6), (2.23) thifluzamid (130000-40-7), (2.24) 1-methyl-N-[2-(1,1,2,2-tetrafluoroethoxy)phenyl]-3-(trifluoromethyl)-1H-pyrazole-4-carboxamide, (2.25) 3-(difluoromethyl)-1-methyl-N-[2-(1,1,2,2-tetrafluoroethoxy)phenyl]-1H-pyrazole-4-carboxamide, (2.26) 3-(difluoromethyl)-N-[4-fluoro-2-(1,1,2,3,3,3-hexafluoropropoxy)phenyl]-1-methyl-1H-pyrazole-4-carboxamide, (2.27) N-[1-(2,4-dichlorophenyl)-1-methoxypropan-2-yl]-3-(difluoromethyl)-1-methyl-1H-pyrazole-4-carboxamide (1092400-95-7), (2.28) 5,8-difluoro-N-[2-(2-fluoro-4-{[4-(trifluoromethyl)pyridin-2-yl]oxy}phenyl)ethyl]quinazoline-4-amine (1210070-84-0), (2.29) N-[9-(dichloromethylene)-1,2,3,4-tetrahydro-1,4-methanonaphthalen-5-yl]-3-(difluoromethyl)-1-methyl-1H-pyrazole-4-carboxamide, (2.30) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide and (2.31) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide.

(3) Respiration inhibitors (respiratory chain inhibitors) acting on complex III of the respiratory chain such as, for example, (3.1) ametoctradin (865318-97-4), (3.2) amisulbrom (348635-87-0), (3.3) azoxystrobin (131860-33-8), (3.4) cyazofamid (120116-88-3), (3.5) coumethoxystrobin (850881-30-0), (3.6) coumoxystrobin (850881-70-8), (3.5) dimoxystrobin (141600-52-4), (3.6) enestroburin (238410-11-2), (3.9) famoxadone (131807-57-3), (3.10) fenamidone (161326-34-7), (3.11) fenoxystrobin (918162-02-4), (3.12) fluoxastrobin (361377-29-9), (3.13) kresoxim-methyl (143390-89-0), (3.14) metominostrobin (133408-50-1), (3.15) orysastrobin (189892-69-1), (3.16) picoxystrobin (117428-22-5), (3.17) pyraclostrobin (175013-18-0), (3.18) pyrametostrobin (915410-70-7), (3.19) pyraoxystrobin (862588-11-2), (3.20) pyribencarb (799247-52-2), (3.21) triclopyricarb (902760-40-1), (3.22) trifloxystrobin (141517-21-7), (3.23) (2E)-2-(2-{[6-(3-chloro-2-methylphenoxy)-5-fluoropyrimidin-4-yl]oxy}phenyl)-2-(methoxyimino)-N-methylethanamide, (3.24) (2E)-2-(methoxyimino)-N-methyl-2-(2-{[({(1E)-1-[3-(trifluoromethyl)phenyl]ethylidene}amino)oxy]methy 1}phenyl)ethanamide, (3.25) (2E)-2-(methoxyimino)-N-methyl-2-{2-[(E)-({1-[3-(trifluoromethyl)phenyl]ethoxy}imino)methyl]phenyl }ethanamide (158169-73-4), (3.26) (2E)-2-{2-[({[(1E)-1-(3-{[(E)-1-fluoro-2-phenylethenyl]oxy}phenyl)ethylidene]amino}oxy)meth yl]phenyl}-2-(methoxyimino)-N-methylethanamide (326896-28-0), (3.27) (2E)-2-{2-[({[(2E,3E)-4-(2,6-dichlorophenyl)but-3-en-2-ylidene]amino}oxy)methyl]phenyl}-2-(methoxyimino)-N-methylethanamide, (3.28) 2-chloro-N-(1,1,3-trimethyl-2,3-dihydro-1H-inden-4-yl)pyridine-3-carboxamide (119899-14-8), (3.29) 5-methoxy-2-methyl-4-(2-{[({(1E)-1-[3-(trifluoromethyl)phenyl]ethylidene}amino)oxy]methy 1}phenyl)-2,4-dihydro-3H-1,2,4-triazol-3-one, (3.30) methyl (2E)-2-{2-[({cyclopropyl[(4-methoxyphenyl)imino]methyl}sulfanyl)methyl]phenyl} -3-methoxyprop-2-enoate (149601-03-6), (3.31) N-(3-ethyl-3,5,5-trimethylcyclohexyl)-3-(formylamino)-2-hydroxybenzamide (226551-21-9), (3.32) 2-{2-[(2,5-dimethylphenoxy)methyl]phenyl}-2-methoxy-N-methylacetamide (173662-97-0) and (3.33) (2R)-2-{2-[(2,5-dimethylphenoxy)methyl]phenyl}-2-methoxy-N-methylacetamide (394657-24-0).

(4) Inhibitors of mitosis and cell division, for example (4.1) benomyl (17804-35-2), (4.2) carbendazim (10605-21-7), (4.3) chlorfenazole (3574-96-7), (4.4) diethofencarb (87130-20-9), (4.5) ethaboxam (162650-77-3), (4.6) fluopicolid (239110-15-7), (4.7) fuberidazole (3878-19-1), (4.8) pencycuron (66063-05-6), (4.9) thiabendazole (148-79-8), (4.10) thiophanate-methyl (23564-05-8), (4.11) thiophanate (23564-06-9), (4.12) zoxamide (156052-68-5), (4.13) 5-chloro-7-(4-methylpiperidin-1-yl)-6-(2,4,6-trifluorophenyl)[1,2,4]triazolo[1,5-a]pyrimidine (214706-53-3) and (4.14) 3-chloro-5-(6-chloropyridin-3-yl)-6-methyl-4-(2,4,6-trifluorophenyl)pyridazine (1002756-87-7).

(5) Compounds having multisite activity, for example (5.1) Bordeaux mixture (8011-63-0), (5.2) captafol (2425-06-1), (5.3) captan (133-06-2), (5.4) chlorothalonil (1897-45-6), (5.5) copper preparations such as copper hydroxide (20427-59-2), (5.6) copper naphthenate (1338-02-9), (5.7) copper oxide (1317-39-1), (5.8) copper oxychloride (1332-40-7), (5.9) copper sulfate (7758-98-7), (5.10) dichlofluanid (1085-98-9), (5.11) dithianon (3347-22-6), (5.12) dodine (2439-10-3), (5.13) dodine free base, (5.14) ferbam (14484-64-1), (5.15) fluorofolpet (719-96-0), (5.16) folpet (133-07-3), (5.17) guazatine (108173-90-6), (5.18) guazatine acetate, (5.19) iminoctadine (13516-27-3), (5.20) iminoctadine albesilate (169202-06-6), (5.21) iminoctadine triacetate (57520-17-9), (5.22) mancopper (53988-93-5), (5.23) mancozeb (8018-01-7), (5.24) maneb (12427-38-2), (5.25) metiram (9006-42-2), (5.26) zinc metiram (9006-42-2), (5.27) copper-oxine (10380-28-6), (5.28) propamidine (104-32-5), (5.29) propineb (12071-83-9), (5.30) sulfur and sulfur preparations, for example calcium polysulfide (7704-34-9), (5.31) thiram (137-26-8), (5.32) tolylfluanid (731-27-1), (5.33) zineb (12122-67-7) and (5.34) ziram (137-30-4) .

(6) Resistance inductors, for example (6.1) acibenzolar-S-methyl (135158-54-2), (6.2) isotianil

(224049-04-1), (6.3) probenazole (27605-76-1) and (6.4) tiadinil (223580-51-6).

(7) Inhibitors of amino acid and protein biosynthesis such as, for example, (7.1) andoprim (23951-85-1), (7.2) blasticidin-S (2079-00-7), (7.3) cyprodinil (121552-61-2), (7.4) kasugamycin (6980-18-3), (7.5) kasugamycin hydrochloride hydrate (19408-46-9), (7.6) mepanipyrim (110235-47-7), (7.7) pyrimethanil (53112-28-0) and (7.8) 3-(5-fluoro-3,3,4,4-tetramethyl-3,4-dihydroisoquinolin-1-yl)quinoline (861647-32-7).

(8) ATP production inhibitors, for example (8.1) fentin acetate (900-95-8), (8.2) fentin chloride (639-58-7), (8.3) fentin hydroxide (76-87-9) and (8.4) silthiofam (175217-20-6).

(9) Inhibitors of cell wall synthesis, for example (9.1) benthiavalicarb (177406-68-7), (9.2) dimethomorph (110488-70-5), (9.3) flumorph (211867-47-9), (9.4) iprovalicarb (140923-17-7), (9.5) mandipropamid (374726-62-2), (9.6) polyoxins (11113-80-7), (9.7) polyoxorim (22976-86-9), (9.8) validamycin A (37248-47-8) and (9.9) valifenalate (283159-94-4; 283159-90-0).

(10) Inhibitors of lipid and membrane synthesis, for example (10.1) biphenyl (92-52-4), (10.2) chloroneb (2675-77-6), (10.3) dicloran (99-30-9), (10.4) edifenphos (17109-49-8), (10.5) etridiazole (2593-15-9), (10.6) iodocarb (55406-53-6), (10.7) iprobenfos (26087-47-8), (10.8) isoprothiolane (50512-35-1), (10.9) propamocarb (25606-41-1), (10.10) propamocarb hydrochloride (25606-41-1), (10.11) prothiocarb (19622-08-3), (10.12) pyrazophos (13457-18-6), (10.13) quintozene (82-68-8), (10.14) tecnazene (117-18-0) and (10.15) tolclofos-methyl (57018-04-9).

(11) Melanin biosynthesis inhibitors such as, for example, (11.1) carpropamid (104030-54-8), (11.2) diclocymet (139920-32-4), (11.3) fenoxanil (115852-48-7), (11.4) fthalide (27355-22-2), (11.5) pyroquilon (57369-32-1), (11.6) tricyclazole (41814-78-2) and (11.7) 2,2,2-trifluoroethyl{3-methyl-1-[(4-methylbenzoyl)amino]butan-2-yl}carbamate (851524-22-6).

(12) Inhibitors of nucleic acid synthesis, for example (12.1) benalaxyl (71626-11-4), (12.2) benalaxyl-M (kiralaxyl) (98243-83-5), (12.3) bupirimate (41483-43-6), (12.4) clozylacon (67932-85-8), (12.5) dimethirimol (5221-53-4), (12.6) ethirimol (23947-60-6), (12.7) furalaxyl (57646-30-7), (12.8) hymexazole (10004-44-1), (12.9) metalaxyl (57837-19-1), (12.10) metalaxyl-M (mefenoxam) (70630-17-0), (12.11) ofurace (58810-48-3), (12.12) oxadixyl (77732-09-3) and (12.13) oxolinic acid (14698-29-4).

(13) Signal transduction inhibitors, for example (13.1) chlozolinate (84332-86-5), (13.2) fenpiclonil (74738-17-3), (13.3) fludioxonil (131341-86-1), (13.4) iprodione (36734-19-7), (13.5) procymidone (32809-16-8), (13.6) quinoxyfen (124495-18-7) and (13.7) vinclozolin (50471-44-8).

(14) Decouplers, for example (14.1) binapacryl (485-31-4), (14.2) dinocap (131-72-6), (14.3) ferimzone (89269-64-7), (14.4) fluazinam (79622-59-6) and (14.5) meptyldinocap (131-72-6).

(15) Further compounds such as, for example, (15.1) benthiazole (21564-17-0), (15.2) bethoxazine (163269-30-5), (15.3) capsimycin (70694-08-5), (15.4) carvone (99-49-0), (15.5) chinomethionat (2439-01-2), (15.6) pyriofenone (chlazafenone) (688046-61-9), (15.7) cufraneb (11096-18-7), (15.8) cyflufenamid (180409-60-3), (15.9) cymoxanil (57966-95-7), (15.10) cyprosulfamide (221667-31-8), (15.11) dazomet (533-74-4), (15.12) debacarb (62732-91-6), (15.13) dichlorophen (97-23-4), (15.14) diclomezine (62865-36-5), (15.15) difenzoquat (49866-87-7), (15.16) difenzoquat methylsulfate (43222-48-6), (15.17) diphenylamine (122-39-4), (15.18) EcoMate, (15.19) fenpyrazamine (473798-59-3), (15.20) flumetover (154025-04-4), (15.21) fluoromid (41205-21-4), (15.22) flusulfamide (106917-52-6), (15.23) flutianil (304900-25-2), (15.24) fosetyl-aluminum (39148-24-8), (15.25) fosetyl-calcium, (15.26) fosetyl-sodium (39148-16-8), (15.27) hexachlorobenzene (118-74-1), (15.28) irumamycin (81604-73-1), (15.29) methasulfocarb (66952-49-6), (15.30) methyl isothiocyanate (556-61-6), (15.31) metrafenone (220899-03-6), (15.32) mildiomycin (67527-71-3), (15.33) natamycin (7681-93-8), (15.34) nickel dimethyldithiocarbamate (15521-65-0), (15.35) nitrothal-isopropyl (10552-74-6), (15.36) octhilinone (26530-20-1), (15.37) oxamocarb (917242-12-7), (15.38) oxyfenthiin (34407-87-9), (15.39) pentachlorophenol and its salts (87-86-5), (15.40) phenothrin, (15.41) phosphoric acid and its salts (13598-36-2), (15.42) propamocarb-fosetylate, (15.43) propanosine-sodium (88498-02-6), (15.44) proquinazid (189278-12-4), (15.45) pyrimorph (868390-90-3), (15.45e) (2E)-3-(4-tert-butylphenyl)-3-(2-chloropyridin-4-yl)-1-(morpholin-4-yl)prop-2-en-1-one (1231776-28-5), (15.45z) (2Z)-3-(4-tert-butylphenyl)-3-(2-chloropyridin-4-yl)-1-(morpholin-4-yl)prop-2-en-1-one (1231776-29-6), (15.46) pyrrolnitrin (1018-71-9), (15.47) tebufloquin (376645-78-2), (15.48) tecloftalam (76280-91-6), (15.49) tolnifanide (304911-98-6), (15.50) triazoxide (72459-58-6), (15.51) trichlamide (70193-21-4), (15.52) zarilamid (84527-51-5), (15.53) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate (517875-34-2), (15.54) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-79-6), (15.55) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-80-9), (15.56) 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003318-67-9), (15.57) 1-(4-methoxyphenoxy)-3,3-dimethylbutan-2-yl 1H-imidazole-1-carboxylate (111227-17-9), (15.58) 2,3,5,6-tetrachloro-4-(methylsulfonyl)pyridine (13108-52-6), (15.59) 2,3-dibutyl-6-chlorothieno[2,3-d]pyrimidin-4(3H)-one (221451-58-7), (15.60) 2,6-dimethyl-1H,5H-[1,4]dithiino[2,3-c:5,6-c']dipyrrole-1,3,5,7(2H,6H)-tetrone, (15.61) 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone (1003316-53-7), (15.62) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone (1003316-54-8), (15.63) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone (1003316-51-5), (15.64) 2-butoxy-6-iodo-3-propyl-4H-chromen-4-one, (15.65) 2-chloro-5-[2-chloro-1-(2,6-difluoro-4-methoxyphenyl)-4-methyl-1H-imidazol-5-yl]pyridine, (15.66) 2-phenylphenol and its salts (90-43-7), (15.67) 3-(4,4,5-trifluoro-3,3-dimethyl-3,4-dihydroisoquinolin-1-yl)quinoline (861647-85-0), (15.68) 3,4,5-trichloropyridine-2,6-dicarbonitrile (17824-85-0), (15.69) 3-[5-(4-chlorophenyl)-2,3-dimethyl-1,2-oxazolidin-3-yl]pyridine, (15.70) 3-chloro-5-(4-chlorophenyl)-4-(2,6-difluorophenyl)-6-methylpyridazine, (15.71) 4-(4-chlorophenyl)-5-(2,6-difluorophenyl)-3,6-dimethylpyridazine, (15.72) 5-amino-1,3,4-thiadiazole-2-thiole, (15.73) 5-chloro-N'-phenyl-N'-(prop-2-yn-1-yl)thiophene-2-sulfonohydrazide (134-31-6), (15.74) 5-fluoro-2-[(4-fluorobenzyl)oxy]pyrimidine-4-amine (1174376-11-4), (15.75) 5-fluoro-2-[(4-methylbenzyl)oxy]pyrimidine-4-amine (1174376-25-0), (15.76) 5-methyl-6-octyl[1,2,4]triazolo[1,5-a]pyrimidine-7-amine, (15.77) ethyl-(2Z)-3-amino-2-cyano-3-phenylprop-2-enoate, (15.78) N'-(4-{[3-(4-chlorobenzyl)-1,2,4-thiadiazol-5-yl]oxy}-2,5-dimethylphenyl)-N-ethyl-N-methylimidoformamide, (15.79) N-(4-chlorobenzyl)-3-[3-methoxy-4-(prop-2-yn-1-yloxy)phenyl]propanamide, (15.80) N-[(4-chlorophenyl)(cyano)methyl]-3-[3-methoxy-4-(prop-2-yn-1-yloxy)phenyl]propanamide, (15.81) N-[(5-bromo-3-chloropyridin-2-yl)methyl]-2,4-dichloropyridine-3-carboxamide, (15.82) N-[1-(5-bromo-3-chloropyridin-2-yl)ethyl]-2,4-dichloropyridine-3-carboxamide, (15.83) N-[1-(5-bromo-3-chloropyridin-2-yl)ethyl]-2-fluoro-4-iodopyridine-3-carboxamide, (15.84) N-{(E)-[(cyclopropylmethoxy)imino][6-(difluoromethoxy)-2,3-difluorophenyl]methyl}-2-phenylacetamide (221201-92-9), (15.85) N-{(Z)-[(cyclopropylmethoxy)imino][6-(difluoromethoxy)-2,3-difluorophenyl]methyl}-2-phenylacetamide (221201-92-9), (15.86) N'-{4-[(3-tert-butyl-4-cyano-1,2-thiazol-5-yl)oxy]-2-chloro-5-methylphenyl}-N-ethyl-N-methylimidoformamide, (15.87) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide (922514-49-6), (15.88) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide (922514-07-6), (15.89) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide (922514-48-5), (15.90) pentyl-{6-[({[(1-methyl-1H-tetrazol-5-yl)(phenyl)methylidene]amino}oxy)methyl]pyridin-2-yl}carbamate, (15.91) phenazine-1-carboxylic acid, (15.92) quinolin-8-ol (134-31-6), (15.93) quinolin-8-ol sulfate (2:1) (134-31-6) and (15.94) tert-butyl {6-[({[(1-methyl-1H-tetrazol-5-yl)(phenyl)methylene]amino}oxy)methyl]pyridin-2-yl}carbamate.

(16) Further compounds such as, for example, (16.1) 1-methyl-3-(trifluoromethyl)-N-[2'-(trifluoromethyl)biphenyl-2-yl]-1H-pyrazole-4-carboxamide, (16.2) N-(4'-chlorobiphenyl-2-yl)-3-(difluoromethyl)-1-methyl-1H-pyrazole-4-carboxamide, (16.3) N-(2',4'-dichlorobiphenyl-2-yl)-3-(difluoromethyl)-1-methyl-1H-pyrazole-4-carboxamide, (16.4) 3-(difluoromethyl)-1-methyl-N-[4'-(trifluoromethyl)biphenyl-2-yl]-1H-pyrazole-4-carboxamide, (16.5) N-(2',5'-difluorobiphenyl-2-yl)-1-methyl-3-(trifluoromethyl)-1H-pyrazole-4-carboxamide, (16.6) 3-(difluoromethyl)-1-methyl-N-[4'-(prop-1-yn-1-yl)biphenyl-2-yl]-1H-pyrazole-4-carboxamide, (16.7) 5-fluoro-1,3-dimethyl-N-[4'-(prop-1-yn-1-yl)biphenyl-2-yl]-1H-pyrazole-4-carboxamide, (16.8) 2-chloro-N-[4'-(prop-1-yn-1-yl)biphenyl-2-yl]pyridine-3-carboxamide, (16.9) 3-(difluoromethyl)-N-[4'-(3,3-dimethylbut-1-yn-1-yl)biphenyl-2-yl]-1-methyl-1H-pyrazole-4-carboxamide, (16.10) N-[4'-(3,3-dimethylbut-1-yn-1-yl)biphenyl-2-yl]-5-fluoro-1,3-dimethyl-1H-pyrazole-4-carboxamide, (16.11) 3-(difluoromethyl)-N-(4'-ethynylbiphenyl-2-yl)-1-methyl-1H-pyrazole-4-carboxamide, (16.12) N-(4'-ethynylbiphenyl-2-yl)-5-fluoro-1,3-dimethyl-1H-pyrazole-4-carboxamide, (16.13) 2-chloro-N-(4'-ethynylbiphenyl-2-yl)pyridine-3-carboxamide, (16.14) 2-chloro-N-[4'-(3,3-dimethylbut-1-yn-1-yl)biphenyl-2-yl]pyridine-3-carboxamide, (16.15) 4-(difluoromethyl)-2-methyl-N-[4'-(trifluoromethyl)biphenyl-2-yl]-1,3-thiazole-5-carboxamide, (16.16) 5-fluoro-N-[4'-(3-hydroxy-3-methylbut-1-yn-1-yl)biphenyl-2-yl]-1,3-dimethyl-1H-pyrazole-4-carboxamide, (16.17) 2-chloro-N-[4'-(3-hydroxy-3-methylbut-1-yn-1-yl)biphenyl-2-yl]pyridine-3-carboxamide, (16.18) 3-(difluoromethyl)-N-[4'-(3-methoxy-3-methylbut-1-yn-1-yl)biphenyl-2-yl]-1-methyl-1H-pyrazole-4-carboxamide,

(16.19) 5-fluoro-N-[4'-(3-methoxy-3-methylbut-1-yn-1-yl)biphenyl-2-yl]-1,3-dimethyl-1H-pyrazole-4-carboxamide, (16.20) 2-chloro-N-[4'-(3-methoxy-3-methylbut-1-yn-1-yl)biphenyl-2-yl]pyridine-3-carboxamide, (16.21) (5-bromo-2-methoxy-4-methylpyridin-3-yl)(2,3,4-trimethoxy-6-methylphenyl)methanone, (16.22) N-[2-(4-{ [3-(4-chlorophenyl)prop-2-yn-1-yl]oxy}-3-methoxyphenyl)ethyl]-N2-(methylsulfonyl)valinamide (220706-93-4), (16.23) 4-oxo-4-[(2-phenylethyl)amino]butanoic acid and (16.24) but-3-yn-1-yl {6-[({[(Z)-(1-methyl-1H-tetrazol-5-yl)(phenyl)methylene]amino}oxy)methyl]pyridin-2-yl}carbamate.

**17.** Agrochemical compositions, **characterized in that** they comprise at least one compound of the formula (I) according to any of Claims 1 to 15 or a composition according to Claim 16, and extenders and/or surfactants.

**18.** Process for producing agrochemical compositions, **characterized in that** compounds of the formula (I) according to any of Claims 1 to 15 or a composition according to Claim 16 are/is mixed with extenders and/or surfactants.

**19.** Method for controlling animal pests, **characterized in that** compounds of the formula (I) according to any of Claims 1 to 15 or a composition according to Claim 16 are/is allowed to act on animal pests and/or their habitat, where methods for the surgical or therapeutic treatment of the human or animal body and diagnostic methods carried out on the human or animal body are excluded.

**20.** Use of compounds of the formula (I) according to any of Claims 1 to 15 or of a composition according to Claim 16 for controlling animal pests in crop protection, in the protection of materials and/or in the veterinary sector, where uses in methods for the surgical or therapeutic treatment of the human or animal body and diagnostic methods carried out on the human or animal body are excluded.

## Revendications

**1.** Composé de formule (I)

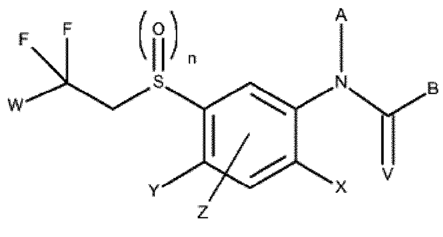

(I)

dans laquelle

A et B représentent ensemble, avec les atomes auxquels ils sont liés, une sous-structure qui est choisie dans le groupe constitué par

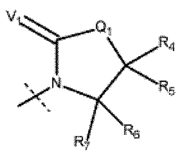

(I-A)

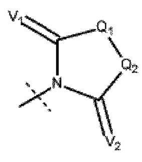

(I-B)

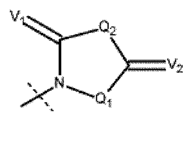

(I-C)

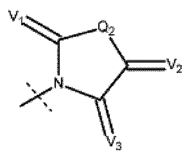

(I-D)

où
dans le cas où est présente une sous-structure selon la formule (I-A), (I-B) ou (I-D),

$V^1$, $V^2$ et $V^3$ représentent chacun indépendamment
un atome d'oxygène ; un atome de soufre, un atome d'azote éventuellement substitué ou des sels d'un atome d'azote éventuellement substitué ;

et dans le cas où est présente une sous-structure selon la formule (I-C),

$V^1$ et $V^2$ représentent chacun, indépendamment l'un de l'autre, un atome d'oxygène ou de soufre ;

dans le cas où est présente une sous-structure selon la formule (I-A), (I-B) ou (I-D),

$Q^1$ et $Q^2$ chacun indépendamment l'un de l'autre
représentent un atome d'oxygène, un atome de soufre ou un atome d'azote éventuellement substitué ; ou
représentent un atome de carbone éventuellement substitué ; étant entendu que $Q_1$ ne représente pas un radical alkylcarbonylamino ;

et dans le cas où est présente une sous-structure selon la formule (I-C),

$Q^1$ et $Q^2$ chacun indépendamment l'un de l'autre
représentent un atome d'oxygène, un atome de soufre ou un atome d'azote substitué ; ou
représentent un atome de carbone éventuellement substitué ; étant entendu que $Q_1$ ne représente pas un radical alkylcarbonylamino ;

$R^4$, $R^5$, $R^6$ et $R^7$, chacun indépendamment les uns des autres,

représentent un atome d'hydrogène ou d'halogène ou un groupe cyano ou nitro ; ou
représentent un groupe alkyle, cycloalkylalkyle, halogénoalkyle, cyanoalkyle, hydroxyalkyle, alcoxyalkyle, aminoalkyle, alcoxycarbonylalkyle, alkylthioalkyle, halogéno-alkylthioalkyle, alcoxyalkylthioalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle ou hétéroarylthioalkyle, les radicaux précités pouvant être chacun saturés ou insaturés et/ou éventuellement substitués ; ou
représentent un groupe cycloalkyle saturé ou insaturé, éventuellement substitué, qui peut éventuellement être interrompu par un ou plusieurs hétéroatomes ; ou
représentent un groupe alkylcarbonyle, halogénoalkylcarbonyle, hydroxyalkylcarbonyle, alcoxyalkylcarbonyle, phénylcarbonyle, hétéroarylcarbonyle, alcoxycarbonyle, halogénoalcoxycarbonyle, aryloxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représentent un groupe carbonyle ou carboxy ; ou
représentent un groupe phényle éventuellement substitué ou hétéroaryle éventuellement substitué ; ou
représentent un groupe alcoxy, halogénoalcoxy, alcoxyalcoxy, aryloxy, arylalkyloxy, cycloalkyloxy, cycloalkylalkyloxy ou carbonyloxy, les radicaux précités pouvant chacun être saturés ou insaturés et/ou éventuellement substitués, ou représentent un groupe hydroxy ; ou
représentent un groupe alkylamino, dialkylamino, halogénoalkylamino, dihalogéno-dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)-amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représentent un groupe amino ; ou
représentent un groupe alkylsulfanyle, alkylsulfinyle, alkylsulfonyle, halogénoalkylsulfanyle, halogénoalkylsulfinyle, halogénoalkylsulfonyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représentent un groupe sulfanyle ; ou
$R^4$ et $R^5$ peuvent former ensemble, avec l'atome auquel ils sont liés, un cycle à trois à huit chaînons saturé ou insaturé, éventuellement substitué et éventuellement interrompu par un ou plusieurs hétéroatomes qui sont choisis indépendamment dans le groupe constitué par les atomes d'oxygène, de soufre et d'azote ; ou
$R^6$ et $R^7$ peuvent former ensemble, avec l'atome auquel ils sont liés, un cycle à trois à huit chaînons saturé ou

insaturé, éventuellement substitué et éventuellement interrompu par un ou plusieurs hétéroatomes qui sont choisis indépendamment dans le groupe constitué par les atomes d'oxygène, de soufre et d'azote ;
W représente un atome d'hydrogène ou d'halogène ;
X, Y et Z, chacun indépendamment,
représentent un atome d'hydrogène ou d'halogène, un groupe hydroxy, amino, nitro, OCN, SCN, $SF_5$; ou représentent un groupe trialkylsilyle, alkyle, halogénoalkyle, cyanoalkyle, hydroxyalkyle, alcoxycarbonylalkyle, alcoxyalkyle, alcényle, halogénoalcényle, cyanoalcényle, alcynyle, halogénoalcynyle, cyano-alcynyle, alcoxy, halogénoalcoxy, cyano-alcoxy, hydroxycarbonylalcoxy, alcoxy-carbonylalcoxy, alcoxyalcoxy, alkylhydroxy-imino, alcoxyimino, alkylalcoxyimino, halogénoalkylalcoxyimino, alkylthio, halogénoalkylthio, alcoxyalkylthio, alkylthioalkyle, alkylsulfinyle, halogénoalkylsulfinyle, alcoxyalkylsulfinyle, alkylsulfinylalkyle, alkylsulfonyle, halogénoalkylsulfonyle, alcoxyalkylsulfonyle, alkylsulfonylalkyle, alkylsulfonyloxy, alkylcarbonyle, halogénoalkylcarbonyle, carboxy, alkylcarbonyloxy, alcoxycarbonyle, halogénoalcoxycarbonyle, aminocarbonyle, alkylaminocarbonyle, dialkylaminocarbonyle, alcényl-aminocarbonyle, dialcénylaminocarbonyle, cycloalkylaminocarbonyle, alkylsulfonylamino, aminosulfonyle, alkylaminosulfonyle, dialkylaminosulfonyle, alkylsulfoximino, aminothiocarbonyle, alkylaminothiocarbonyle ou dialkylaminothiocarbonyle, tous les radicaux précités pouvant éventuellement être substitués ; ou
représentent un groupe phénylalkyle, phénoxy, phénylalkyloxy, phénoxyalkyle, phénylthio, phénylthioalkyle, phénylsulfinyle, phényl-sulfonyle, hétéroarylalkyle, hétéroaryloxy, hétéroarylalkyloxy, hétéroarylthio, hétéroarylsulfinyle ou hétéroarylsulfonyle, tous les radicaux précités pouvant éventuellement être substitués ; ou représentent un groupe cycloalkylalkyle, cycloalkyloxy, cycloalkylalcoxy, cycloalkyl-thio, cycloalkylalkylthio, cycloalkylsulfinyle, cycloalkylalkylsulfinyle, cycloalkylsulfonyle, cycloalkylalkylsulfonyle ou cycloalcényle, tous les radicaux précités pouvant éventuellement être substitués ; ou
représentent NR'R",

R' et R", indépendamment l'un de l'autre,
représentant un atome d'hydrogène, un groupe cyano, alkyle, halogénoalkyle, cyanoalkyle, hydroxyalkyle, alcoxyalkyle, alkylthioalkyle, alcényle, halogénoalcényle, cyanoalcényle, alcynyle, halogénoalcynyle, cyano-alcynyle, acyle ou alcoxycarbonyle ; ou
R' et R", ensemble avec l'atome d'azote auquel ils sont liés, pouvant former un cycle à cinq à huit chaînons, saturé ou insaturé, éventuellement substitué et éventuellement interrompu par un ou plusieurs hétéroatomes qui sont choisis indépendamment dans le groupe constitué par les atomes d'oxygène, de soufre et d'azote ; ou

représentent un cycle saturé, partiellement saturé ou aromatique, à 3 à 6 chaînons, qui peut éventuellement contenir un à trois hétéroatomes, qui sont choisis indépendamment dans le groupe constitué par les atomes d'oxygène, de soufre et d'azote, et qui peut éventuellement être substitué ;
et
n représente le nombre 0 ou 1.

**2.** Composé selon la revendication 1, dans lequel

A et B représentent ensemble, avec les atomes auxquels ils sont liés, une sous-structure qui est choisie dans le groupe constitué par (I-A) à (I-D),

où
dans le cas où est présente une sous-structure selon la formule (I-A), (I-B) ou (I-D),

$V^1$, $V^2$ et $V^3$ représentent chacun indépendamment
un atome d'oxygène ; un atome de soufre, $NR^{11}$ ou un sel de $NR^{11}$ ;

et dans le cas où est présente une sous-structure selon la formule (I-C),

$V^1$ et $V^2$ représentent chacun, indépendamment l'un de l'autre
un atome d'oxygène ou de soufre ;
$Q^1$ représente un atome d'oxygène, un atome de soufre, $NR^1$ ou $CR^2R^3$ ;
$Q^2$ représente $NR^{10}$ ou $CR^8R^9$ ;

dans le cas où est présente une sous-structure selon la formule (I-A), (I-B) ou (I-D),

$R^1$ représente un atome d'hydrogène, un groupe cyano ou nitro ; ou

représente un groupe alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle, hétéroarylthioalkyle, alcoxycarbonyle, aryloxycarbonyle, arylcarbamoyle, les radicaux précités pouvant être chacun saturés ou insaturés et/ou éventuellement substitués ; ou

représente un groupe cycloalkyle saturé ou insaturé, éventuellement substitué, qui peut éventuellement être interrompu par un ou plusieurs hétéroatomes ; ou

représente un groupe halogénoalkylcarbonyle, hydroxyalkylcarbonyle, alcoxyalkylcarbonyle, phénylcarbonyle, hétéroarylcarbonyle, halogénoalcoxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)-aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représente un groupe carbonyle ou carboxy ; ou

représente un groupe phényle éventuellement substitué ou hétéroaryle éventuellement substitué ; ou

représente un groupe alcoxy, halogénoalcoxy, cycloalkyloxy, aryloxy, arylalkyloxy ou carbonyloxy, les radicaux précités pouvant éventuellement être substitués, ou représente un groupe hydroxy ; ou

représente un groupe alkylamino, halogénoalkylamino, dihalogénoalkylamino, dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)-amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représente un groupe amino ; ou

représente un groupe alkylsulfanyle, alkylsulfinyle, alkylsulfonyle, halogénoalkylsulfanyle, halogénoalkylsulfinyle, halogénoalkylsulfanyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représente un groupe sulfanyle ; et

$R^{10}$ et $R^{11}$ chacun indépendamment l'un de l'autre,

représentent un atome d'hydrogène, un groupe cyano ou nitro ; ou

représentent un groupe alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle, hétéroarylthioalkyle, alcoxycarbonyle, aryloxycarbonyle, arylcarbamoyle, les radicaux précités pouvant être chacun saturés ou insaturés et/ou éventuellement substitués ; ou

représentent un groupe cycloalkyle saturé ou insaturé, éventuellement substitué, qui peut éventuellement être interrompu par un ou plusieurs hétéroatomes ; ou

représentent un groupe alkylcarbonyle, halogénoalkylcarbonyle, hydroxyalkylcarbonyle, alcoxyalkylcarbonyle, phénylcarbonyle, hétéroarylcarbonyle, halogénoalcoxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)-aminocarbonyle, cycloalkyl(aryl)aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représentent un groupe carbonyle ou carboxy ; ou

représentent un groupe phényle éventuellement substitué ou hétéroaryle éventuellement substitué ; ou

représentent un groupe alcoxy, halogénoalcoxy, cycloalkyloxy, aryloxy, arylalkyloxy ou carbonyloxy, les radicaux précités pouvant éventuellement être substitués, ou représentent un groupe hydroxy ; ou

représentent un groupe alkylamino, halogénoalkylamino, dihalogénoalkylamino, dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)-amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représentent un groupe amino ; ou

représentent un groupe alkylsulfanyle, alkylsulfinyle, alkylsulfonyle, halogénoalkylsulfanyle, halogénoalkylsulfinyle, halogénoalkylsulfanyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représentent un groupe sulfanyle ;

et dans le cas où est présente une sous-structure selon la formule (I-C),

$R^1$ représente un groupe cyano ou nitro ; ou
représente un groupe alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle, hétéroarylthioalkyle, alcoxycarbonyle, aryloxycarbonyle, arylcarbamoyle, les radicaux précités pouvant être chacun saturés ou insaturés et/ou éventuellement substitués ; ou
représente un groupe cycloalkyle saturé ou insaturé, éventuellement substitué, qui peut éventuellement être interrompu par un ou plusieurs hétéroatomes ; ou
représente un groupe halogénoalkylcarbonyle, hydroxyalkylcarbonyle, alcoxyalkylcarbonyle, phénylcarbonyle, hétéroarylcarbonyle, halogénoalcoxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représente un groupe carbonyle ou carboxy ; ou
représente un groupe phényle éventuellement substitué ou hétéroaryle éventuellement substitué ; ou
représente un groupe alcoxy, halogénoalcoxy, cycloalkyloxy, aryloxy, arylalkyloxy ou carbonyloxy, les radicaux précités pouvant éventuellement être substitués, ou représente un groupe hydroxy ; ou
représente un groupe alkylamino, halogénoalkylamino, dihalogénoalkylamino, dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)-amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représente un groupe amino ; ou
représente un groupe alkylsulfanyle, alkylsulfinyle, alkylsulfonyle, halogénoalkylsulfanyle, halogénoalkylsulfinyle, halogénoalkylsulfanyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représente un groupe sulfanyle ; et
$R^{10}$ représente un groupe cyano ou nitro ; ou
représente un groupe alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle, hétéroarylthioalkyle, alcoxycarbonyle, aryloxycarbonyle, arylcarbamoyle, les radicaux précités pouvant être chacun saturés ou insaturés et/ou éventuellement substitués ; ou
représente un groupe cycloalkyle saturé ou insaturé, éventuellement substitué, qui peut éventuellement être interrompu par un ou plusieurs hétéroatomes ; ou
représente un groupe alkylcarbonyle, halogénoalkylcarbonyle, hydroxyalkylcarbonyle, alcoxyalkylcarbonyle, phénylcarbonyle, hétéroarylcarbonyle, halogénoalcoxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)-aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représente un groupe carbonyle ou carboxy ; ou
représente un groupe phényle éventuellement substitué ou hétéroaryle éventuellement substitué ; ou
représente un groupe alcoxy, halogénoalcoxy, cycloalkyloxy, aryloxy, arylalkyloxy ou carbonyloxy, les radicaux précités pouvant éventuellement être substitués, ou représente un groupe hydroxy ; ou

représente un groupe alkylamino, halogénoalkylamino, dihalogénoalkylamino, dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représente un groupe amino ; ou représente un groupe alkylsulfanyle, alkylsulfinyle, alkylsulfonyle, halogénoalkylsulfanyle, halogénoalkylsulfinyle, halogénoalkylsulfanyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représente un groupe sulfanyle ; et

$R^2$, $R^3$, $R^4$, $R^5$, $R^6$, $R^7$, $R^8$ et $R^9$ chacun indépendamment les uns des autres,

représentent un atome d'hydrogène ou d'halogène ou un groupe cyano ou nitro ; ou

représentent un groupe alkyle, cycloalkylalkyle, halogénoalkyle, cyanoalkyle, hydroxyalkyle, alcoxyalkyle, aminoalkyle, alcoxycarbonylalkyle, alkylthioalkyle, halogéno-alkylthioalkyle, alcoxyalkylthioalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétéroarylalkyle, hétéroaryloxyalkyle ou hétéroarylthioalkyle, les radicaux précités pouvant être chacun saturés ou insaturés et/ou éventuellement substitués ; ou

représentent un groupe cycloalkyle saturé ou insaturé, éventuellement substitué, qui peut éventuellement être interrompu par un ou plusieurs hétéroatomes ; ou

représentent un groupe alkylcarbonyle, halogénoalkylcarbonyle, hydroxyalkylcarbonyle, alcoxyalkylcarbonyle, phénylcarbonyle, hétéroarylcarbonyle, alcoxycarbonyle, halogénoalcoxycarbonyle, aryloxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)-aminocarbonyle, cycloalkyl(aryl)aminocarbonyle, alkylaminothiocarbonyle, dialkylaminothiocarbonyle ou aminothiocarbonyle, les radicaux précités pouvant chacun être saturés ou insaturés et/ou éventuellement substitués, ou représentent un groupe carbonyle ou carboxy ; ou

représentent un groupe phényle éventuellement substitué ou hétéroaryle éventuellement substitué ;

représentent un groupe alcoxy, halogénoalcoxy, alcoxyalcoxy, aryloxy, arylalkyloxy, cycloalkyloxy, cycloalkylalkyloxy ou carbonyloxy, les radicaux précités pouvant chacun être saturés ou insaturés et/ou éventuellement substitués, ou représentent un groupe hydroxy ; ou

représentent un groupe alkylamino, dialkylamino, halogénoalkylamino, dihalogéno-dialkylamino, cycloalkylamino, dicycloalkylamino, cycloalkyl(alkyl)amino, arylamino, diarylamino, hétéroarylamino, dihétéroarylamino, alkyl(aryl)amino, cycloalkyl(aryl)-amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, les radicaux précités pouvant chacun être saturés ou insaturés et/ou éventuellement substitués, ou représentent un groupe amino ; ou

représentent un groupe alkylsulfanyle, alkylsulfinyle, alkylsulfonyle, halogénoalkylsulfanyle, halogénoalkylsulfinyle, halogénoalkylsulfonyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, les radicaux précités pouvant chacun être saturés ou insaturés et/ou éventuellement substitués, ou représentent un groupe sulfanyle ; ou

$R^2$ et $R^3$ peuvent former ensemble, avec l'atome auquel ils sont liés, un cycle à trois à huit chaînons saturé ou insaturé, éventuellement substitué et éventuellement interrompu par un ou plusieurs hétéroatomes qui sont choisis indépendamment dans le groupe constitué par les atomes d'oxygène, de soufre et d'azote ; ou

$R^4$ et $R^5$ peuvent former ensemble, avec l'atome auquel ils sont liés, un cycle à trois à huit chaînons saturé ou insaturé, éventuellement substitué et éventuellement interrompu par un ou plusieurs hétéroatomes qui sont choisis indépendamment dans le groupe constitué par les atomes d'oxygène, de soufre et d'azote ; ou

$R^6$ et $R^7$ peuvent former ensemble, avec l'atome auquel ils sont liés, un cycle à trois à huit chaînons saturé ou insaturé, éventuellement substitué et éventuellement interrompu par un ou plusieurs hétéroatomes qui sont choisis indépendamment dans le groupe constitué par les atomes d'oxygène, de soufre et d'azote ; ou

$R^8$ et $R^9$ peuvent former ensemble, avec l'atome auquel ils sont liés, un cycle à trois à huit chaînons saturé ou insaturé, éventuellement substitué et éventuellement interrompu par un ou plusieurs hétéroatomes qui sont choisis indépendamment dans le groupe constitué par les atomes d'oxygène, de soufre et d'azote ;

W représente un atome d'hydrogène ou d'halogène ;

X, Y et Z ont les significations indiquées dans la revendication 1 ; et
n représente le nombre 0 ou 1.

**3.** Composé selon la revendication 1 ou 2, dans lequel

A et B représentent ensemble, avec les atomes auxquels ils sont liés, une sous-structure qui est choisie dans le groupe constitué par (I-A) à (I-D),

où
dans le cas où est présente une sous-structure selon la formule (I-A), (I-B) ou (I-D),

$V^1$, $V^2$ et $V^3$ représentent chacun indépendamment
un atome d'oxygène ; un atome de soufre, $NR^{11}$ ou un sel de $NR^{11}$ ;

et dans le cas où est présente une sous-structure selon la formule (I-C),

$V^1$ et $V^2$ représentent chacun, indépendamment l'un de l'autre,
un atome d'oxygène ou de soufre ;
$Q^1$ représente un atome d'oxygène, un atome de soufre, $NR^1$ ou $CR^2R^3$ ;
$Q^2$ représente $NR^{10}$ ou $CR^8R^9$ ;

dans le cas où est présente une sous-structure selon la formule (I-A), (I-B) ou (I-D),

$R^1$ représente un atome d'hydrogène, un groupe cyano ou nitro ; ou
représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanyl-alkyle($C_1$-$C_6$), alkyl($C_1$-$C_6$)sulfinyl-alkyle($C_1$-$C_6$), alkyl($C_1$-$C_6$)sulfonyl-alkyle($C_1$-$C_6$), halogénoalkyl($C_1$-$C_6$)-sulfanylalkyle($C_1$-$C_6$), halogénoalkyl($C_1$-$C_6$)-sulfinylalkyle($C_1$-$C_6$), halogénoalkyl($C_1$-$C_6$)-sulfonylalkyle($C_1$-$C_6$), alcoxy($C_1$-$C_6$)-alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), alcoxy($C_1$-$C_6$)carbonyle, aryloxycarbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou
représente un groupe cycloalkyle en $C_3$-$C_6$ saturé ou insaturé, éventuellement substitué, qui peut éventuellement être interrompu par un ou plusieurs hétéroatomes ; ou
représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phényl-carbonyle, hétéroarylcarbonyle, halogéno-alcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)amino-carbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou
représente un groupe phényle éventuellement substitué ou hétéroaryle éventuellement substitué ; ou
représente un groupe alcoxy en $C_1$-$C_6$, halogénoalcoxy($C_1$-$C_6$), cycloalkyloxy en $C_3$-$C_6$, aryloxy, arylalkyloxy($C_1$-$C_6$) ou carbonyloxy, les radicaux précités pouvant chacun éventuellement être substitués, ou représente un groupe hydroxy ; ou
représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl(C3-C6)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux

pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogéno-alkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)amino-sulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^{10}$ et $R^{11}$ chacun indépendamment l'un de l'autre,

représentent un atome d'hydrogène, un groupe cyano ou nitro ; ou

représentent un groupe alkyle en $C_1$-$C_6$, cycloalkyl($C_3$-$C_6$)alkyle($C_1$-$C_6$), halogénoalkyle($C_2$-$C_6$), cyanoalkyle($C_1$-$C_6$), hydroxyalkyle($C_1$-$C_6$), alcoxy($C_1$-$C_6$)-alkyle($C_1$-$C_6$), aminoalkyle($C_1$-$C_6$), alcoxy($C_1$-$C_6$)carbonylalkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogéno-alkyl($C_1$-$C_6$)sulfanylalkyle($C_1$-$C_6$), halogéno-alkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phényl-carbonyle, hétéroarylcarbonyle, halogéno-alcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)amino-carbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl) aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ;

représentent un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représentent un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl) amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogéno-alkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)amino-sulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ;

et dans le cas où est présente une sous-structure selon la formule (I-C),

$R^1$ représente un groupe cyano ou nitro ; ou

représente un groupe alkyle en $C_1$-$C_6$, cycloalkyl($C_3$-$C_6$)alkyle($C_1$-$C_6$), halogénoalkyle($C_2$-$C_6$), cyanoalkyle($C_1$-$C_6$), hydroxyalkyle($C_1$-$C_6$), alcoxy($C_1$-$C_6$)alkyle($C_1$-$C_6$), aminoalkyle($C_1$-$C_6$), alcoxy($C_1$-$C_6$)carbonylalkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)-sulfanylalkyle($C_1$-$C_6$), alcoxy($C_1$-$C_6$)-alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle($C_1$-$C_6$), hétéroarylsulfanylalkyle($C_1$-$C_6$), hétéroarylsulfinylalkyle($C_1$-$C_6$), hétéroarylsulfonylalkyle($C_1$-$C_6$), alcoxy($C_1$-$C_6$)carbonyle, aryloxycarbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant éventuellement être substitués chacun, ou représente un groupe hydroxy ; ou

représente un groupe alkyl($C_1$-$C_6$)amino, halogénoalkyl($C_1$-$C_6$)amino, dihalogénoalkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl ($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^{10}$ représente un groupe cyano ou nitro ; ou

représente un groupe alkyle en $C_1$-$C_6$, cycloalkyl($C_3$-$C_6$)alkyle($C_1$-$C_6$), halogénoalkyle($C_2$-$C_6$), cyanoalkyle($C_1$-$C_6$), hydroxyalkyle($C_1$-$C_6$), alcoxy($C_1$-$C_6$)alkyle($C_1$-$C_6$), aminoalkyle($C_1$-$C_6$), alcoxy($C_1$-$C_6$)carbonylalkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)-sulfanylalkyle($C_1$-$C_6$), alcoxy($C_1$-$C_6$)-alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle($C_1$-$C_6$), hétéroarylsulfanylalkyle($C_1$-$C_6$), hétéroarylsulfinylalkyle($C_1$-$C_6$), hétéroarylsulfonylalkyle($C_1$-$C_6$), alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogénoalcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl) aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant éventuellement être substitués, ou représente un groupe hydroxy ; ou

représente un groupe alkyl($C_1$-$C_6$)amino, halogénoalkyl($C_1$-$C_6$)amino, dihalogénoalkyl($C_1$-$C_6$)amino, dialkyl($C_1$-$C_6$)amino, cycloalkyl($C_3$-$C_6$) amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl) amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^2$, $R^3$, $R^4$, $R^5$, $R^6$, $R^7$, $R^8$ et $R^9$ chacun indépendamment les uns des autres,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogénoalkyle($C_1$-$C_6$), cyanoalkyle($C_1$-$C_6$), hydroxyalkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)thioalkyle($C_1$-$C_6$), halogénoalkyl($C_1$-$C_6$)thioalkyle($C_1$-$C_6$), alcoxy($C_1$-$C_6$)alkyl($C_1$-$C_6$)thioalkyle, alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle($C_1$-$C_6$)ou hétéroarylthioalkyle($C_1$-$C_6$), les radicaux précités pouvant être chacun saturés ou insaturés et/ou éventuellement substitués ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe alkyl($C_1$-$C_6$)carbonyle, halogénoalkyl($C_1$-$C_6$)carbonyle, hydroxyalkyl($C_1$-$C_6$)carbonyle, alcoxy($C_1$-$C_6$)-alkyl($C_1$-$C_6$)carbonyle, phénylcarbonyle, hétéroarylcarbonyle, alcoxy($C_1$-$C_6$)carbonyle, halogénoalcoxy($C_1$-$C_6$)carbonyle, aryloxycarbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), alcoxy($C_1$-$C_6$)-alcoxy($C_1$-$C_6$), aryloxy, arylalkyl($C_1$-$C_6$)oxy, cycloalkyloxy en $C_3$-$C_6$, cycloalkyl($C_3$-$C_6$)-alkyl($C_1$-$C_6$)oxy ou carbonyloxy, les radicaux précités

pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe hydroxy ; ou

représentent un groupe alkyl($C_1$-$C_6$)amino, dialkyl($C_1$-$C_6$)amino, halogénoalkyl($C_1$-$C_6$)amino, dihalogénoalkyl($C_1$-$C_6$)amino, cycloalkyl ($C_3$-$C_6$) amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl) amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfonyle, cycloalkyl($C_3$-$C_6$)sulfanyle, cycloalkyl($C_3$-$C_6$)sulfinyle, cycloalkyl($C_3$-$C_6$)sulfonyle, cycloalkyl($C_3$-$C_6$)alkyl($C_1$-$C_6$)sulfanyle, cycloalkyl($C_3$-$C_6$)alkyl($C_1$-$C_6$)sulfinyle, cycloalkyl($C_3$-$C_6$)alkyl($C_1$-$C_6$)sulfonyle, arylsulfanyle, arylsulfinyle, arylsulfonyle, arylalkyl($C_1$-$C_6$)sulfanyle, arylalkyl($C_1$-$C_6$)-sulfinyle, arylalkyl($C_1$-$C_6$)sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^4$ et $R^5$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^6$ et $R^7$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène ou d'halogène ;

X, Y et Z ont les significations indiquées dans la revendication 1 ou 2 ; et

n représente le nombre 0 ou 1.

**4.** Composé selon l'une quelconque des revendications 1 à 3, dans lequel

A et B représentent ensemble, avec les atomes auxquels ils sont liés, une sous-structure de formule (I-A),

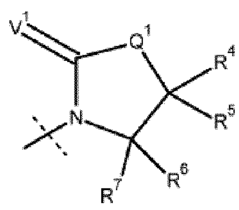

(I-A)

dans laquelle

$V^1$ représente un atome d'oxygène ; un atome de soufre, $NR^{11}$ ou un sel de $NR^{11}$ ;
$Q^1$ représente un atome d'oxygène, un atome de soufre, $NR^1$ ou $CR^2R^3$ ;
$R^1$ représente un atome d'hydrogène, un groupe cyano ou nitro ; ou
représente un groupe alkyle en $C_1$-$C_6$, cycloalkyl ($C_3$-$C_6$) alkyle ($C_1$-$C_6$), halogéno-alkyle ($C_2$-$C_6$), cyanoalkyle ($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy ($C_1$-$C_6$)carbonyl-alkyle ($C_1$-$C_6$), alkyl ($C_1$-$C_6$)sulfanyl-alkyle ($C_1$-$C_6$), alkyl ($C_1$-$C_6$)sulfinyl-alkyle ($C_1$-$C_6$), alkyl ($C_1$-$C_6$)sulfonyl-alkyle ($C_1$-$C_6$), halogénoalkyl ($C_1$-$C_6$)-sulfanylalkyle ($C_1$-$C_6$), halogénoalkyl ($C_1$-$C_6$)-sulfinylalkyle ($C_1$-$C_6$), halogénoalkyl ($C_1$-$C_6$)-sulfonylalkyle($C_1$-$C_6$), alcoxy($C_1$-$C_6$)-alkyl ($C_1$-$C_6$)sulfanylalkyle($C_1$-$C_6$), alcoxy ($C_1$-$C_6$)alkyl ($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle ($C_1$-$C_6$), phénoxyalkyle ($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanylalkyle($C_1$-$C_6$), hétéroarylsulfinylalkyle($C_1$-$C_6$), hétéroarylsulfonylalkyle($C_1$-$C_6$), alcoxy($C_1$-$C_6$)-carbonyle, aryloxycarbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou
représente un groupe cycloalkyle en $C_3$-$C_6$ saturé ou insaturé, éventuellement substitué, qui peut éventuellement être interrompu par un ou plusieurs hétéroatomes ; ou
représente un groupe halogénoalkyl($C_1$-$C_6$)-carbonyle, hydroxyalkyl($C_1$-$C_6$)carbonyle, alcoxy ($C_1$-$C_6$)alkyl ($C_1$-$C_6$)carbonyle, phénylcarbonyle, hétéroarylcarbonyle, halogénoalcoxy($C_1$-$C_6$)carbonyle, aminocarbonyle, alkyl ($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$) (alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$) (aryl) aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou
représente un groupe phényle éventuellement substitué ou hétéroaryle éventuellement substitué ; ou
représente un groupe alcoxy en $C_1$-$C_6$, halogénoalcoxy ($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représente un groupe hydroxy ; ou
représente un groupe alkyl($C_1$-$C_6$)amino, halogénoalkyl($C_1$-$C_6$)amino, dihalogéno-alkyl ($C_1$-$C_6$)amino, dialkyl($C_1$-$C_6$)amino, cycloalkyl($C_3$-$C_6$) amino, dicycloalkyl($C_3$-$C_6$)-amino, cycloalkyl($C_3$-$C_6$) (alkyl($C_1$-$C_6$))amino, arylamino, diarylamino, hétéroarylamino, dihétéroarylamino, alkyl ($C_1$-$C_6$)(aryl)amino, cycloalkyl ($C_3$-$C_6$) (aryl) amino, alkyl ($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou
représente un groupe alkyl($C_1$-$C_6$)sulfanyle, alkyl ($C_1$-$C_6$)sulfinyle, alkyl ($C_1$-$C_6$)sulfonyle, halogénoalkyl($C_1$-$C_6$)sulfanyle, halogéno-alkyl ($C_1$-$C_6$)sulfinyle, halogénoalkyl($C_1$-$C_6$)-sulfanyle, cycloalkyl($C_3$-$C_6$)sulfanyle, cycloalkyl ($C_3$-$C_6$) sulfinyle, cycloalkyl($C_3$-$C_6$) sulfonyle, cycloalkyl($C_3$-$C_6$) alkyl($C_1$-$C_6$)sulfanyle, cycloalkyl($C_3$-$C_6$) alkyl($C_1$-$C_6$)sulfinyle, cycloalkyl($C_3$-$C_6$) alkyl($C_1$-$C_6$)sulfonyle, arylsulfanyle, arylsulfinyle, arylsulfonyle, arylalkyl ($C_1$-$C_6$)sulfanyle, arylalkyl ($C_1$-$C_6$)-sulfinyle, arylalkyl ($C_1$-$C_6$)sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente

un groupe sulfanyle ; et

$R^{11}$ représente un atome d'hydrogène, un groupe cyano ou nitro ; ou

représente un groupe alkyle en $C_1$-$C_6$, cycloalkyl($C_3$-$C_6$) alkyle($C_1$-$C_6$), halogéno-alkyle ($C_2$-$C_6$) , cyanoalkyle ($C_1$-$C_6$), hydroxy-alkyle ($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle ($C_1$-$C_6$), alkyl($C_1$-$C_6$)sulfanylalkyle ($C_1$-$C_6$), alkyl ($C_1$-$C_6$)sulfinylalkyle ($C_1$-$C_6$), alkyl ($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle ($C_1$-$C_6$), halogénoalkyl ($C_1$-$C_6$)sulfonylalkyle($C_1$-$C_6$), alcoxy($C_1$-$C_6$)alkyl($C_1$-$C_6$)-sulfanylalkyle($C_1$-$C_6$), alcoxy ($C_1$-$C_6$)-alkyl ($C_1$-$C_6$)sulfinylalkyle($C_1$-$C_6$), alcoxy ($C_1$-$C_6$)alkyl($C_1$-$C_6$)sulfonylalkyle($C_1$-$C_6$), phénylalkyle ($C_1$-$C_6$), phénoxyalkyle ($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle($C_1$-$C_6$), hétéroarylsulfanylalkyle($C_1$-$C_6$), hétéroarylsulfinylalkyle($C_1$-$C_6$), hétéroarylsulfonylalkyle ($C_1$-$C_6$), alcoxy ($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl ($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogénoalcoxy($C_1$-$C_6$)carbonyle, aryloxycarbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$) aminocarbonyle, cycloalkyl($C_3$-$C_6$) (alkyl ($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$) (aryl) aminocarbonyle, alkyl ($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl ($C_1$-$C_6$)amino, dialkyl($C_1$-$C_6$)amino, cycloalkyl ($C_3$-$C_6$) amino, dicycloalkyl($C_3$-$C_6$)-amino, cycloalkyl($C_3$-$C_6$) (alkyl($C_1$-$C_6$))amino, arylamino, diarylamino, hétéroarylamino, dihétéroarylamino, alkyl ($C_1$-$C_6$)(aryl)amino, cycloalkyl ($C_3$-$C_6$) (aryl) amino, alkyl ($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl ($C_1$-$C_6$)sulfinyle, alkyl ($C_1$-$C_6$)sulfonyle, halogénoalkyl($C_1$-$C_6$)sulfanyle, halogénoalkyl ($C_1$-$C_6$)sulfinyle, halogénoalkyl($C_1$-$C_6$)-sulfanyle, cycloalkyl($C_3$-$C_6$)sulfanyle, cycloalkyl ($C_3$-$C_6$) sulfinyle, cycloalkyl ($C_3$-$C_6$) sulfonyle, cycloalkyl ($C_3$-$C_6$) alkyl ($C_1$-$C_6$)sulfanyle, cycloalkyl($C_3$-$C_6$)alkyl($C_1$-$C_6$)sulfinyle, cycloalkyl($C_3$-$C_6$)alkyl($C_1$-$C_6$)sulfonyle, arylsulfanyle, arylsulfinyle, arylsulfonyle, arylalkyl ($C_1$-$C_6$)sulfanyle, arylalkyl ($C_1$-$C_6$)sulfinyle, arylalkyl($C_1$-$C_6$)sulfonyle, aminosulfonyle, alkyl($C_1$-$C_6$)aminosulfonyle, dialkyl($C_1$-$C_6$)-aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^2$, $R^3$, $R^4$, $R^5$, $R^6$ et $R^7$, chacun indépendamment les uns des autres,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogénoalkyle($C_1$-$C_6$), cyanoalkyle($C_1$-$C_6$), hydroxy-alkyle ($C_1$-$C_6$), alcoxy ($C_1$-$C_6$)alkyle ($C_1$-$C_6$), aminoalkyle($C_1$-$C_6$), alcoxy($C_1$-$C_6$)carbonylalkyle($C_1$-$C_6$), alkyl($C_1$-$C_6$)thioalkyle($C_1$-$C_6$), halogénoalkyl($C_1$-$C_6$)thioalkyle($C_1$-$C_6$), alcoxy ($C_1$-$C_6$)alkyl ($C_1$-$C_6$)thioalkyle, alkyl ($C_1$-$C_6$)sulfanylalkyle($C_1$-$C_6$), alkyl ($C_1$-$C_6$)sulfinylalkyle($C_1$-$C_6$), alkyl ($C_1$-$C_6$)sulfonylalkyle($C_1$-$C_6$), halogénoalkyl ($C_1$-$C_6$)sulfanylalkyle($C_1$-$C_6$), halogénoalkyl ($C_1$-$C_6$)sulfinylalkyle($C_1$-$C_6$), halogénoalkyl ($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy ($C_1$-$C_6$)alkyl ($C_1$-$C_6$)sulfonylalkyle($C_1$-$C_6$), phénylalkyle($C_1$-$C_6$), phénoxyalkyle ($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle ($C_1$-$C_6$)ou hétéroarylthioalkyle($C_1$-$C_6$), les radicaux précités pouvant être chacun saturés ou insaturés et/ou éventuellement substitués ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe alkyl($C_1$-$C_6$)carbonyle, halogénoalkyl($C_1$-$C_6$)carbonyle, hydroxyalkyl ($C_1$-$C_6$)carbonyle, alcoxy ($C_1$-$C_6$)-alkyl($C_1$-$C_6$)carbonyle, phénylcarbonyle, hétéroarylcarbonyle, alcoxy ($C_1$-$C_6$)carbonyle, halogénoalcoxy ($C_1$-$C_6$)carbonyle, aryloxycarbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$) aminocarbonyle, cycloalkyl($C_3$-$C_6$) (alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$) (aryl) aminocarbonyle, alkyl ($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy ($C_1$-$C_6$), alcoxy ($C_1$-$C_6$)-alcoxy($C_1$-$C_6$), aryloxy, arylalkyl($C_1$-$C_6$)oxy, cycloalkyloxy en $C_3$-$C_6$, cycloalkyl($C_3$-$C_6$)-alkyl($C_1$-$C_6$)oxy ou carbonyloxy, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe hydroxy ; ou

représentent un groupe alkyl ($C_1$-$C_6$)amino, dialkyl($C_1$-$C_6$)amino, halogénoalkyl ($C_1$-$C_6$)amino, dihalogénoalkyl($C_1$-$C_6$)amino, cycloalkyl($C_3$-$C_6$) amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl ($C_1$-$C_6$)sulfinyle, alkyl ($C_1$-$C_6$)sulfonyle, halogénoalkyl($C_1$-$C_6$)sulfanyle, halogénoalkyl ($C_1$-$C_6$)sulfinyle, halogénoalkyl ($C_1$-$C_6$)-sulfonyle, cycloalkyl($C_3$-$C_6$)sulfanyle, cycloalkyl ($C_3$-$C_6$) sulfinyle, cycloalkyl ($C_3$-$C_6$) sulfonyle, cycloalkyl ($C_3$-$C_6$) alkyl ($C_1$-$C_6$)sulfanyle, cycloalkyl($C_3$-$C_6$) alkyl($C_1$-$C_6$)sulfinyle, cycloalkyl($C_3$-$C_6$) alkyl($C_1$-$C_6$)sulfonyle, arylsulfanyle, arylsulfinyle, arylsulfonyle, arylalkyl ($C_1$-$C_6$)sulfanyle, arylalkyl ($C_1$-$C_6$)-sulfinyle, arylalkyl($C_1$-$C_6$)sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^4$ et $R^5$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^6$ et $R^7$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

W représente un atome d'hydrogène, de fluor ou de chlore ;

X, Y et Z, chacun indépendamment, représentent un atome d'hydrogène, de fluor, chlore, brome, un groupe nitro, amino, hydroxy, alkyle en $C_1$-$C_4$, halogénoalkyle($C_1$-$C_4$), alcényle en $C_2$-$C_4$, alcynyle en $C_2$-$C_4$, alcoxy en $C_1$-$C_4$, halogénoalcoxy($C_1$-$C_4$) ou aminothiocarbonyle ;

ou représentent un groupe benzyle, phénoxy, phénylthio, cyclopropylméthyle, cyclopropyl-oxy ou cyclopropylthio, qui est éventuellement une ou plusieurs fois substitué par des substituants, identiques ou différents, fluoro,

chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ;

ou représentent un groupe cyclopropyle, cyclobutyle, cyclopentyle, cyclohexyle ou phényle, qui est éventuellement une ou plusieurs fois substitué par des substituants, identiques ou différents, fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ; et

n représente le nombre 0 ou 1.

**5.** Composé selon la revendication 4, dans lequel la sous-structure de formule (I-A) représente une sous-structure qui est choisie dans le groupe constitué par

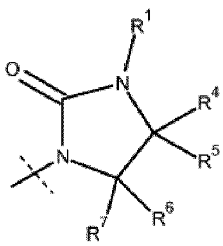
(I-A-1)

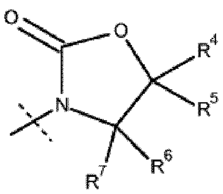
(I-A-2)

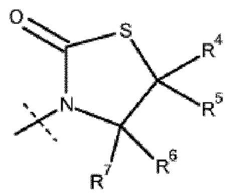
(I-A-3)

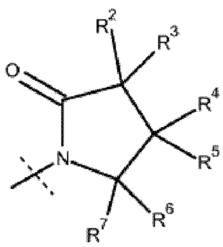
(I-A-4)

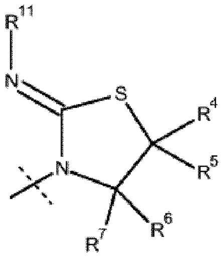
(I-A-5)

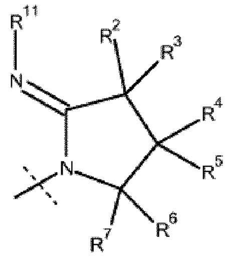
(I-A-6)

où

$R^1$ représente un atome d'hydrogène, ou

représente un groupe alkyle en $C_1$-$C_6$, cycloalkyl ($C_3$-$C_6$) alkyle ($C_1$-$C_6$), halogéno-alkyle ($C_2$-$C_6$) , cyanoalkyle($C_1$-$C_6$), alcoxy($C_1$-$C_6$)alkyle($C_1$-$C_6$), alcoxy ($C_1$-$C_6$)-carbonylalkyle ($C_1$-$C_6$), phénylalkyle($C_1$-$C_6$), hétéroarylalkyle ($C_1$-$C_6$), alcényle en $C_2$-$C_6$, halogénoalcényle ($C_2$-$C_6$), cycloalcényl ($C_3$-$C_6$)-alkyle ($C_1$-$C_6$), alcynyle en $C_2$-$C_6$, les radicaux précités pouvant éventuellement être substitués chacun ; ou

représente un groupe cycloalkyle en $C_3$-$C_6$, cycloalcényle en $C_3$-$C_6$, éventuellement substitué, qui peut éventuellement être interrompu par un ou plusieurs hétéroatomes de la série formée par les atomes d'oxygène, de soufre et d'azote ; ou

représente un groupe halogénoalkyl($C_1$-$C_6$)-carbonyle, alcoxy($C_1$-$C_6$)alkyl($C_1$-$C_6$)carbonyle, alcoxy ($C_1$-$C_6$)carbonyle, halogénoalcoxy($C_1$-$C_6$)-carbonyle, aminocarbonyle, alkyl($C_1$-$C_6$)aminocarbonyle, dialkyl($C_1$-$C_6$)aminocarbonyle, cycloalkyl ($C_3$-$C_6$) aminocarbonyle, alkyl ($C_1$-$C_6$)-aminothiocarbonyle, dialkyl($C_1$-$C_6$)aminothiocarbonyle, alcényl($C_2$-$C_6$)carbonyle, cycloalcényl ($C_3$-$C_6$) alkyl ($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy ($C_1$-$C_6$), cycloalkyloxy en $C_3$-$C_6$, ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)carbamoylamino, alkyl($C_1$-$C_6$)sulfonylamino, alcényl($C_2$-$C_6$)amino, cycloalcényl($C_3$-$C_6$)alkyl($C_1$-$C_6$)amino, alcynyl($C_2$-$C_6$)amino, les radicaux précités pouvant éventuellement être

substitués chacun, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl ($C_3$-$C_6$) alkyl ($C_1$-$C_6$)-sulfanyle, cycloalkyl ($C_3$-$C_6$) alkyl ($C_1$-$C_6$)-sulfinyle, cycloalkyl ($C_3$-$C_6$) alkyl ($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^{11}$ représente un atome d'hydrogène ; ou

représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou

représente un groupe cycloalkyle en $C_3$-$C_6$, cycloalcényle en $C_3$-$C_6$, qui peut éventuellement être interrompu par un ou plusieurs hétéroatomes de la série formée par les atomes d'oxygène, de soufre et d'azote ; ou

représente un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl ($C_3$-$C_6$) alkyl ($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$ ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^2$, $R^3$, $R^4$, $R^5$, $R^6$ et $R^7$, chacun indépendamment les uns des autres,

représentent un atome d'hydrogène ou d'halogène ou un groupe cyano ou nitro ; ou

représentent un groupe alkyle en $C_1$-$C_6$, cycloalkyl ($C_3$-$C_6$) alkyle ($C_1$-$C_6$), halogéno-alkyle($C_1$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), alkyl($C_1$-$C_6$)sulfanylalkyle($C_1$-$C_6$), alkyl($C_1$-$C_6$)sulfinyl-alkyle($C_1$-$C_6$), alkyl($C_1$-$C_6$)sulfonyl-alkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués, ou représentent un groupe cycloalkyle en $C_3$-$C_6$ ou cycloalcényle en $C_3$-$C_6$ éventuellement substitué, qui peut éventuellement être interrompu par un ou plusieurs hétéroatomes de la série formée par les atomes d'oxygène, de soufre et d'azote ; ou

représentent un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)aminothiocarbonyle, dialkyl($C_1$-$C_6$)aminothiocarbonyle, alcényl($C_2$-$C_6$)carbonyle, cycloalcényl ($C_3$-$C_6$) alkyl($C_1$-$C_6$)carbonyle, alcynyl($C_2$-$C_6$)carbonyle ou aminothiocarbonyle, les radicaux précités pouvant éventuellement être substitués chacun, ou représentent un groupe carbonyle ou carboxy ; ou

représentent un groupe phényle éventuellement substitué ou hétéroaryle éventuellement substitué ;

représentent un groupe alcoxy en $C_1$-$C_6$, halogénoalcoxy($C_1$-$C_6$), alcoxy($C_1$-$C_6$)alcoxy($C_1$-$C_6$), cycloalkyloxy en $C_3$-$C_6$, cycloalkyl($C_3$-$C_6$)alkyl($C_1$-$C_6$)oxy, alcényloxy en $C_2$-$C_6$, cycloalcényl($C_3$-$C_6$)alcoxy($C_1$-$C_6$), alcynyloxy en $C_2$-$C_6$ ou carbonyloxy, les radicaux précités pouvant éventuellement être substitués chacun, ou représentent un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl ($C_3$-$C_6$)-alkyl($C_1$-$C_6$)amino, alcynyl($C_2$-$C_6$)amino, les radicaux précités pouvant éventuellement être substitués chacun, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalk-

yl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^4$ et $R^5$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^6$ et $R^7$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

W représente un atome d'hydrogène ou de fluor ;

X et Y représentent chacun, indépendamment l'un de l'autre, un atome d'hydrogène, de fluor, chlore, brome, un groupe méthyle, éthyle, trifluorométhyle, (2,2)-difluorométhyle, difluorométhoxy, trifluorométhoxy, cyclopropyle, amino, hydroxy ou nitro ;

Z représente un atome d'hydrogène ; et

n représente le nombre 0 ou 1.

**6.** Composé selon la revendication 4 ou 5, dans lequel la sous-structure de formule (I-A) représente une sous-structure qui est choisie dans le groupe constitué par

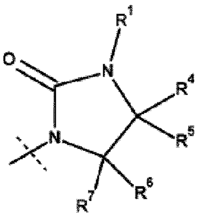
(I-A-1)

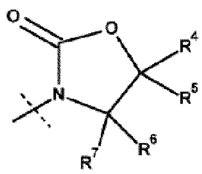
(I-A-2)

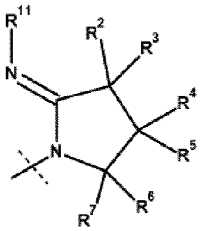
(I-A-6)

où

$R^1$ et $R^{11}$ chacun, indépendamment l'un de l'autre,

représentent un atome d'hydrogène, un groupe méthyle, éthyle, propyle, isopropyle, cyclopropyle, cyclopropylméthyle, $CH_2CF_3$, $CH_2CHF_2$, $CH(CH_3)CF_3$, $CH_2CH_2CF_3$, $CH_2CH_2CHF_2$ ; ou

$R^2$, $R^3$, $R^4$, $R^5$, $R^6$ et $R^7$, représentent chacun, indépendamment les uns des autres, un atome d'hydrogène, un groupe méthyle, trifluorométhyle ou phényle ;

W représente un atome d'hydrogène ou de fluor ;

X représente un atome d'hydrogène, de chlore ou de fluor ou un groupe méthyle ;

Y représente un atome de chlore ou de brome, un groupe méthyle, trifluorométhyle ou un atome de fluor ;

en particulier X et Y représentant les combinaisons (Y,X) suivantes : (Me, F), (Me, H), (Me, Cl), (Me, Me), (Cl, Cl), (Cl, F), (Cl, H), (Br, H), (Br, F), (F, F), ($CF_3$, H) ;

Z représente un atome d'hydrogène ; et

n représente le nombre 0 ou 1.

**7.** Composé selon l'une quelconque des revendications 1 à 3, dans lequel

A et B représentent ensemble, avec les atomes auxquels ils sont liés, une sous-structure de formule (I-B)

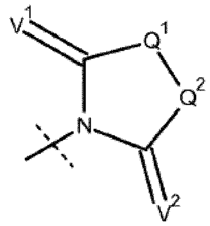

(I-B)

dans laquelle

$V^1$ et $V^2$ représentent chacun, indépendamment l'un de l'autre,
un atome d'oxygène ; un atome de soufre, $NR^{11}$ ou un sel de $NR^{11}$ ;
$Q^1$ représente un atome d'oxygène, un atome de soufre, $NR^1$ ou $CR^2R^3$ ;
$Q^2$ représente $NR^{10}$ ou $CR^8R^9$ ;
$R^1$ représente un atome d'hydrogène, un groupe cyano ou nitro ; ou
représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)-sulfanylalkyle($C_1$-$C_6$), halogénoalkyl($C_1$-$C_6$)-sulfinylalkyle($C_1$-$C_6$), halogénoalkyl($C_1$-$C_6$)-sulfonylalkyle($C_1$-$C_6$), alcoxy($C_1$-$C_6$)-alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), alcoxy($C_1$-$C_6$)carbonyle, aryloxycarbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou
représente un groupe cycloalkyle en $C_3$-$C_6$ saturé ou insaturé, éventuellement substitué, qui peut éventuellement être interrompu par un ou plusieurs hétéroatomes ; ou
représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogénoalcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl ($C_1$-$C_6$) (aryl) aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou
représente un groupe phényle éventuellement substitué ou hétéroaryle éventuellement substitué ; ou
représente un groupe alcoxy en $C_1$-$C_6$, halogénoalcoxy($C_1$-$C_6$), cycloalkyloxy en $C_3$-$C_6$, aryloxy, arylalkyloxy($C_1$-$C_6$) ou carbonyloxy, les radicaux précités pouvant chacun éventuellement être substitués, ou représente un groupe hydroxy ; ou
représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl ($C_3$-$C_6$) amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl ($C_1$-$C_6$) (aryl)amino, cycloalkyl ($C_3$-$C_6$) (aryl) amino, alkyl ($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou
représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl ($C_3$-$C_6$) sulfinyle, cycloalkyl ($C_3$-$C_6$) sulfonyle, cycloalkyl ($C_3$-$C_6$) alkyl ($C_1$-$C_6$) sulfanyle, cycloalkyl

($C_3$-$C_6$) alkyl ($C_1$-$C_6$) sulfinyle, cycloalkyl ($C_3$-$C_6$) alkyl ($C_1$-$C_6$) sulfonyle, arylsulfanyle, arylsulfinyle, arylsulfonyle, arylalkyl($C_1$-$C_6$)sulfanyle, arylalkyl($C_1$-$C_6$)-sulfinyle, arylalkyl ($C_1$-$C_6$) sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^{10}$ et $R^{11}$ chacun indépendamment l'un de l'autre,

représentent un atome d'hydrogène, un groupe cyano ou nitro ; ou

représentent un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy ($C_1$-$C_6$) alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)-sulfanylalkyle($C_1$-$C_6$), alcoxy($C_1$-$C_6$)-alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe halogénoalkyl($C_1$-$C_6$)-carbonyle, hydroxyalkyl($C_1$-$C_6$)carbonyle, alcoxy ($C_1$-$C_6$)alkyl ($C_1$-$C_6$)carbonyle, phénylcarbonyle, hétéroarylcarbonyle, halogénoalcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl) aminocarbonyle, cycloalkyl($C_3$-$C_6$) (aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ;

représentent un groupe alcoxy en $C_1$-$C_6$, halogénoalcoxy ($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représentent un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl ($C_3$-$C_6$) amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl ($C_1$-$C_6$) (aryl)amino, cycloalkyl ($C_3$-$C_6$) (aryl) amino, alkyl($C_1$-$C_6$)carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl ($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; et

$R^2$, $R^3$, $R^8$ et $R^9$ chacun, indépendamment les uns des autres,

représentent un atome d'hydrogène ou d'halogène, un groupe cyano ou nitro ; ou

représentent un groupe alkyle en $C_1$-$C_6$, cycloalkyl($C_3$-$C_6$)alkyle($C_1$-$C_6$), halogéno-alkyle($C_1$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)-carbonylalkyle($C_1$-$C_6$), alkyl($C_1$-$C_6$)thio-alkyle($C_1$-$C_6$), halogénoalkyl($C_1$-$C_6$)thio-alkyle($C_1$-$C_6$), alcoxy($C_1$-$C_6$)alkyl($C_1$-$C_6$)-thioalkyle, alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfony-

lalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle ($C_1$-$C_6$) ou hétéroarylthioalkyle($C_1$-$C_6$), les radicaux précités pouvant être chacun saturés ou insaturés et/ou éventuellement substitués ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe alkyl($C_1$-$C_6$)carbonyle, halogénoalkyl($C_1$-$C_6$)carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, alcoxy($C_1$-$C_6$)-carbonyle, halogénoalcoxy($C_1$-$C_6$)carbonyle, aryloxycarbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl ($C_1$-$C_6$) (aryl) aminocarbonyle, cycloalkyl($C_3$-$C_6$) (aryl) aminocarbonyle, alkyl ($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), alcoxy ($C_1$-$C_6$)-alcoxy($C_1$-$C_6$), aryloxy, arylalkyl($C_1$-$C_6$)oxy, cycloalkyloxy en $C_3$-$C_6$, cycloalkyl($C_3$-$C_6$)-alkyl($C_1$-$C_6$)oxy ou carbonyloxy, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe hydroxy ; ou

représentent un groupe alkyl($C_1$-$C_6$)amino, dialkyl($C_1$-$C_6$)amino, halogénoalkyl($C_1$-$C_6$)amino, dihalogénoalkyl($C_1$-$C_6$)amino,cycloalkyl($C_3$-$C_6$)-amino, dicycloalkyl($C_3$-$C_6$)amino, cycloalkyl ($C_3$-$C_6$) (alkyl ($C_1$-$C_6$))amino, arylamino, diarylamino, hétéroarylamino, dihétéroarylamino, alkyl ($C_1$-$C_6$) (aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfonyle, cycloalkyl($C_3$-$C_6$)sulfanyle, cycloalkyl($C_3$-$C_6$) sulfinyle, cycloalkyl ($C_3$-$C_6$) sulfonyle, cycloalkyl ($C_3$-$C_6$) alkyl ($C_1$-$C_6$) sulfanyle, cycloalkyl ($C_3$-$C_6$) alkyl ($C_1$-$C_6$) sulfinyle, cycloalkyl ($C_3$-$C_6$) alkyl ($C_1$-$C_6$) sulfonyle, arylsulfanyle, arylsulfinyle, arylsulfonyle, arylalkyl($C_1$-$C_6$)sulfanyle, arylalkyl($C_1$-$C_6$)-sulfinyle, arylalkyl($C_1$-$C_6$)sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène de fluor ou de chlore ;

X, Y et Z, chacun indépendamment,

représentent un atome d'hydrogène, de fluor, chlore, brome, un groupe nitro, amino, hydroxy, alkyle en $C_1$-$C_4$, halogéno-alkyle($C_1$-$C_4$), alcényle en $C_2$-$C_4$, alcynyle en $C_2$-$C_4$, alcoxy en $C_1$-$C_4$, halogénoalcoxy ($C_1$-$C_4$) ou aminothiocarbonyle ;

ou représentent un groupe benzyle, phénoxy, phénylthio, cyclopropylméthyle, cyclopropyl-oxy ou cyclopropylthio, qui est éventuellement une ou plusieurs fois substitué par des substituants, identiques ou différents, fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy, ou représentent un groupe cyclopropyle éventuellement substitué par méthyle, fluoro, chloro, cyano ;

ou représentent un groupe cyclopropyle, cyclobutyle, cyclopentyle, cyclohexyle ou phényle, qui est éventuellement une ou plusieurs fois substitué par des substituants, identiques ou différents, fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ; et

n représente le nombre 0 ou 1.

**8.** Composé selon la revendication 7, dans lequel la sous-structure de formule (I-B) représente une sous-structure qui est choisie dans le groupe constitué par

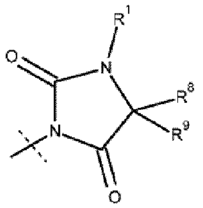

(I-B-1)

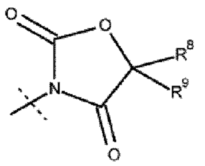

(I-B-2)

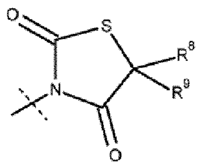

(I-B-3)

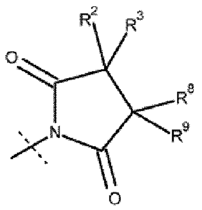

(I-B-4)

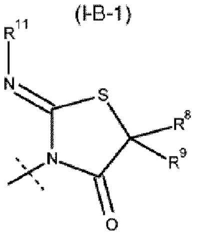

(I-B-5)

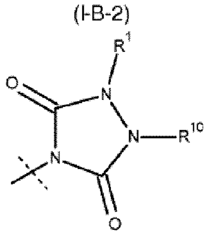

(I-B-6)

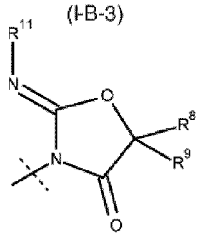

(I-B-7)

où

$R^1$ représente un atome d'hydrogène, ou

représente un groupe alkyle en $C_1$-$C_6$, cycloalkyl ($C_3$-$C_6$) alkyle ($C_1$-$C_6$), halogéno-alkyle($C_2$-$C_6$) , cyanoalkyle($C_1$-$C_6$) , alcoxy($C_1$-$C_6$) alkyle($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou

représente un groupe cycloalkyle en $C_3$-$C_6$, cycloalcényle en $C_3$-$C_6$, éventuellement substitué, qui peut éventuellement être interrompu par un ou plusieurs hétéroatomes de la série formée par les atomes d'oxygène, de soufre et d'azote ; ou

représente un groupe halogénoalkyl($C_1$-$C_6$)-carbonyle, alcoxy ($C_1$-$C_6$)alkyl ($C_1$-$C_6$) carbonyle, alcoxy ($C_1$-$C_6$) carbonyle, halogénoalcoxy($C_1$-$C_6$)-carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alk-

yl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^{10}$ et $R^{11}$ chacun, indépendamment l'un de l'autre,

représentent un atome d'hydrogène ; ou

représentent un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou

représentent un groupe cycloalkyle en $C_3$-$C_6$, cycloalcényle en $C_3$-$C_6$, qui peut éventuellement être interrompu par un ou plusieurs hétéroatomes de la série formée par les atomes d'oxygène, de soufre et d'azote ; ou

représentent un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)aminothiocarbonyle, dialkyl($C_1$-$C_6$)aminothiocarbonyle, alcényl($C_2$-$C_6$)carbonyle, cycloalcényl($C_3$-$C_6$)-alkyl($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), cycloalkyloxy en $C_3$-$C_6$ ou carbonyloxy, les radicaux précités pouvant chacun éventuellement être substitués, ou représente un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^2$, $R^3$, $R^8$ et $R^9$, chacun indépendamment les uns des autres,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogéno-alkyle($C_1$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), alkyl($C_1$-$C_6$)-sulfanylalkyle($C_1$-$C_6$), alkyl($C_1$-$C_6$)sulfinyl-alkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués, ou

représentent un groupe cycloalkyle en $C_3$-$C_6$ ou cycloalcényle en $C_3$-$C_6$ éventuellement substitué, qui peut éventuellement être interrompu par un ou plusieurs hétéroatomes de la série formée par les atomes d'oxygène, de soufre et d'azote ; ou

représentent un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl($C_1$-$C_6$)carbonyle, alcynyl($C_2$-$C_6$)carbonyle ou aminothiocarbonyle, les radicaux précités pouvant éventuellement être substitués chacun, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), alcoxy($C_1$-$C_6$)-alcoxy($C_1$-$C_6$), cycloalkyloxy en $C_3$-$C_6$, cycloalkyl($C_3$-$C_6$)alkyl($C_1$-$C_6$)oxy, alcényloxy en $C_2$-$C_6$, cycloalcényl($C_3$-$C_6$)alcoxy($C_1$-$C_6$), alcynyloxy en $C_2$-$C_6$ ou carbonyloxy, les radicaux précités pouvant éventuellement être substitués chacun, ou représentent un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alk-

yl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène ou de fluor ;

X et Y représentent indépendamment l'un de l'autre un atome d'hydrogène, de fluor, chlore, brome, un groupe méthyle, éthyle, trifluorométhyle, (2,2)-difluorométhyle, difluorométhoxy, trifluorométhoxy, cyclopropyle, amino, hydroxy ou nitro ;

Z représente un atome d'hydrogène ; et

n représente le nombre 0 ou 1.

**9.** Composé selon la revendication 7 ou 8, dans lequel la sous-structure de formule (I-B) représente une sous-structure qui est choisie dans le groupe constitué par

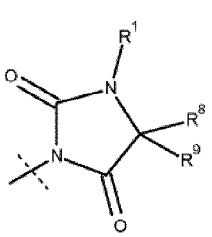

(I-B-1)

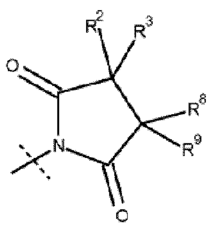

(I-B-4)

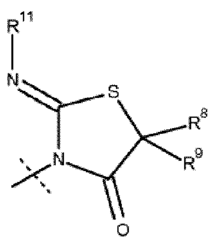

(I-B-5)

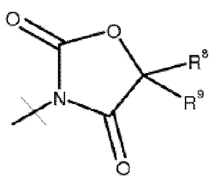

(I-B-2)

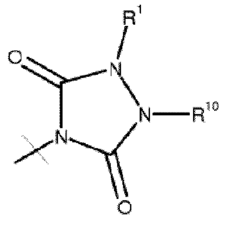

(I-B-6)

où

$R^1$, $R^{10}$ et $R^{11}$ représentent, chacun indépendamment,

un atome d'hydrogène, un groupe, méthyle, éthyle, propyle, isopropyle, n-butyle, cyclopropyle, cyclopropylméthyle, $CH_2CF_3$, $CH_2CHF_2$, $CH(CH_3)CF_3$, $CH_2CH_2CF_3$, $CH_2CH_2CHF_2$, $CH_2C(CH_3)_3$, $CH_2CH(CH_3)_2$, $CH_2CH{=}CH_2$ (= allyle), $CH_2CCH$ ou benzyle ;

$R^2$, $R^3$, $R^8$ et $R^9$ représentent, chacun indépendamment les uns des autres,

un atome d'hydrogène, un groupe méthyle, trifluorométhyle ou phényle ;

W représente un atome d'hydrogène ou de fluor ;

X représente un atome d'hydrogène, de chlore, de fluor ou un groupe méthyle ;

Y représente un atome de chlore, de brome, un groupe méthyle, trifluorométhyle ou un atome de fluor ;

X et Y représentent en particulier les combinaisons (Y, X) suivantes : (Me, F), (Me, H), (Me, Cl), (Me, Me), (Cl,

Cl), (Cl, F), (Cl, H), (Br, H), (Br, F), (F, F), ($CF_3$, H) ;
Z représente un atome d'hydrogène ; et
n représente le nombre 0 ou 1.

**10.** Composé selon l'une quelconque des revendications 1 à 3, dans lequel

A et B représentent ensemble, avec les atomes auxquels ils sont liés, une sous-structure de formule (I-C),

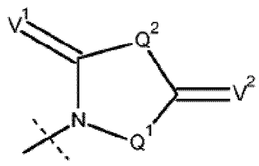

(I-C)

dans laquelle

$V^1$ et $V^2$ représentent chacun, indépendamment l'un de l'autre, un atome d'oxygène ou de soufre ;
$Q^1$ représente un atome d'oxygène, un atome de soufre, $NR^1$ ou $CR^2R^3$ ;
$Q^2$ représente $NR^{10}$ ou $CR^8R^9$ ;
$R^1$ représente un groupe cyano ou nitro ; ou
représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanyl-alkyle($C_1$-$C_6$), alkyl($C_1$-$C_6$)sulfinyl-alkyle($C_1$-$C_6$), alkyl($C_1$-$C_6$)sulfonyl-alkyle($C_1$-$C_6$), halogénoalkyl($C_1$-$C_6$)-sulfanylalkyle($C_1$-$C_6$), halogénoalkyl($C_1$-$C_6$)-sulfinylalkyle($C_1$-$C_6$), halogénoalkyl($C_1$-$C_6$)-sulfonylalkyle($C_1$-$C_6$), alcoxy($C_1$-$C_6$)-alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanylalkyle($C_1$-$C_6$), hétéroarylsulfinylalkyle($C_1$-$C_6$), hétéroaryl-sulfonylalkyle($C_1$-$C_6$), alcoxy($C_1$-$C_6$)carbonyle, aryloxycarbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou
représente un groupe cycloalkyle en $C_3$-$C_6$ saturé ou insaturé, éventuellement substitué, qui peut éventuellement être interrompu par un ou plusieurs hétéroatomes ; ou
représente un groupe halogénoalkyl($C_1$-$C_6$)-carbonyle, hydroxyalkyl($C_1$-$C_6$)carbonyle, alcoxy($C_1$-$C_6$)alkyl ($C_1$-$C_6$)carbonyle, phénylcarbonyle, hétéroarylcarbonyle, halogéno-alcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou
représente un groupe phényle éventuellement substitué ou hétéroaryle éventuellement substitué ; ou
représente un groupe alcoxy en $C_1$-$C_6$, halogénoalcoxy($C_1$-$C_6$), cycloalkyloxy en $C_3$-$C_6$, aryloxy, arylalkyloxy($C_1$-$C_6$) ou carbonyloxy, les radicaux précités pouvant chacun éventuellement être substitués, ou représente un groupe hydroxy ; ou
représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que,

dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^{10}$ représente un groupe cyano ou nitro ; ou

représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)-sulfanylalkyle($C_1$-$C_6$), alcoxy($C_1$-$C_6$)-alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxyalkyle($C_1$-$C_6$), hétéroarylsulfanylalkyle($C_1$-$C_6$), hétéroarylsulfinylalkyle($C_1$-$C_6$), hétéroarylsulfonylalkyle($C_1$-$C_6$), alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl ($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogéno-alcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl) aminocarbonyle, alkyl($C_1$-$C_6$)aminothiocarbonyle, dialkyl($C_1$-$C_6$)-aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représente un groupe phényle éventuellement substitué ou hétéroaryle éventuellement substitué ;

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl ($C_3$-$C_6$)sulfinyle, cycloalkyl ($C_3$-$C_6$)sulfonyle, cycloalkyl ($C_3$-$C_6$)alkyl ($C_1$-$C_6$)sulfanyle, cycloalkyl ($C_3$-$C_6$)alkyl ($C_1$-$C_6$)sulfinyle, cycloalkyl ($C_3$-$C_6$)alkyl ($C_1$-$C_6$)sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)sulfonyle, aminosulfonyle, alkyl($C_1$-$C_6$)aminosulfonyle, dialkyl($C_1$-$C_6$)-aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ;

$R^2$, $R^3$, $R^8$ et $R^9$ chacun, indépendamment les uns des autres,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogéno-alkyle($C_1$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)thioalkyle($C_1$-$C_6$), halogénoalkyl($C_1$-$C_6$)thioalkyle($C_1$-$C_6$), alcoxy($C_1$-$C_6$)alkyl($C_1$-$C_6$)thioalkyle, alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxyalkyle($C_1$-$C_6$)ou hétéroarylthioalkyle($C_1$-$C_6$), les radicaux précités pouvant être chacun saturés ou insaturés et/ou éventuellement substitués ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe alkyl($C_1$-$C_6$)carbonyle, halogénoalkyl($C_1$-$C_6$)carbonyle, hydroxy-alkyl($C_1$-$C_6$)carbonyle, alcoxy($C_1$-$C_6$)-alkyl($C_1$-$C_6$)carbonyle, phénylcarbonyle, hétéroarylcarbonyle, alcoxy($C_1$-$C_6$)carbonyle, halogénoalcoxy($C_1$-$C_6$)carbonyle, aryloxycarbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl) aminocarbonyle, cycloalkyl($C_3$-$C_6$)(aryl) aminocarbonyle, alkyl($C_1$-$C_6$)aminothiocarbonyle, dialkyl($C_1$-$C_6$)-aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), alcoxy($C_1$-$C_6$)-alcoxy($C_1$-$C_6$), aryloxy, arylalkyl($C_1$-$C_6$)oxy, cycloalkyloxy en $C_3$-$C_6$, cycloalkyl($C_3$-$C_6$)-alkyl($C_1$-$C_6$)oxy ou carbonyloxy, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe hydroxy ; ou

représentent un groupe alkyl ($C_1$-$C_6$)amino, dialkyl($C_1$-$C_6$)amino, halogénoalkyl($C_1$-$C_6$)amino, dihalogénoalkyl($C_1$-$C_6$)amino, cycloalkyl($C_3$-$C_6$)-amino, dicycloalkyl($C_3$-$C_6$)amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonyl-amino, alkyl($C_1$-$C_6$)carbamoylamino, aryl-carbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfonyle, cycloalkyl($C_3$-$C_6$)sulfanyle, cycloalkyl($C_3$-$C_6$)sulfinyle, cycloalkyl($C_3$-$C_6$)sulfonyle, cycloalkyl($C_3$-$C_6$)alkyl($C_1$-$C_6$)sulfanyle, cycloalkyl($C_3$-$C_6$)alkyl($C_1$-$C_6$)sulfinyle, cycloalkyl($C_3$-$C_6$)alkyl($C_1$-$C_6$)sulfonyle, arylsulfanyle, arylsulfinyle, arylsulfonyle, arylalkyl($C_1$-$C_6$)sulfanyle, arylalkyl($C_1$-$C_6$)-sulfinyle, arylalkyl($C_1$-$C_6$)sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène, de fluor ou de chlore ;

X, Y et Z, chacun indépendamment,

représentent un atome d'hydrogène, de fluor, chlore, brome, un groupe nitro, amino, hydroxy, alkyle en $C_1$-$C_4$, halogéno-alkyle($C_1$-$C_4$), alcényle en $C_2$-$C_4$, alcynyle en $C_2$-$C_4$, alcoxy en $C_1$-$C_4$, halogénoalcoxy($C_1$-$C_4$) ou aminothiocarbonyle ;

ou représentent un groupe benzyle, phénoxy, phénylthio, cyclopropylméthyle, cyclopropyloxy ou cyclopropylthio, qui est éventuellement une ou plusieurs fois substitué par des substituants identiques ou différents fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ;

ou représentent un groupe cyclopropyle, cyclobutyle, cyclopentyle, cyclohexyle ou phényle, qui est éventuellement une ou plusieurs fois substitué par des substituants identiques ou différents fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ; et

n représente le nombre 0 ou 1.

**11.** Composé selon la revendication 10, dans lequel la sous-structure de formule (I-C) représente une sous-structure qui est choisie dans le groupe constitué par

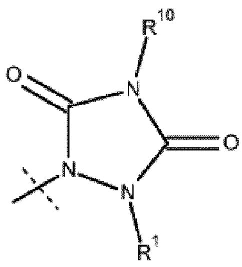
(I-C-1)

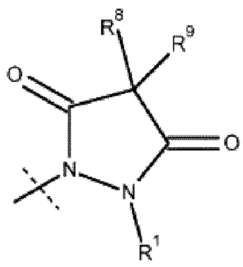
(I-C-2)

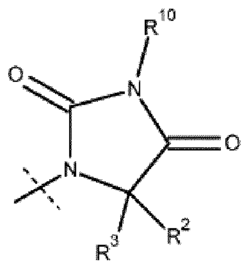
(I-C-3)

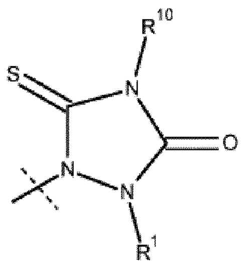
(I-C-4)

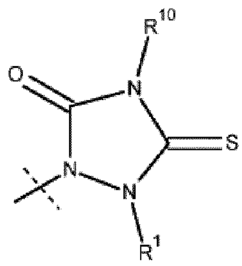
(I-C-5)

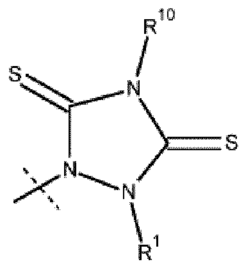
(I-C-6)

où

$R^1$ représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou

représente un groupe cycloalkyle en $C_3$-$C_6$, cycloalcényle en $C_3$-$C_6$, éventuellement substitué, qui peut éventuellement être interrompu par un ou plusieurs hétéroatomes de la série formée par les atomes d'oxygène, de soufre et d'azote ; ou

représente un groupe halogénoalkyl($C_1$-$C_6$)-carbonyle, alcoxy($C_1$-$C_6$)alkyl($C_1$-$C_6$)carbonyle, alcoxy($C_1$-$C_6$)carbonyle, halogénoalcoxy($C_1$-$C_6$)-carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^{10}$ représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou

représente un groupe cycloalkyle en $C_3$-$C_6$, cycloalcényle en $C_3$-$C_6$, qui peut éventuellement être interrompu par un ou plusieurs hétéroatomes de la série formée par les atomes d'oxygène, de soufre et d'azote ; ou

représente un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$ ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)carbamoylamino, alkyl($C_1$-$C_6$)sulfonylamino, alcényl($C_2$-$C_6$)amino, cycloalcényl($C_3$-$C_6$)alkyl($C_1$-$C_6$)amino, alcynyl($C_2$-$C_6$)amino, les radicaux précités pouvant éventuellement être substitués chacun, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et

$R^2$, $R^3$, $R^8$ et $R^9$, chacun indépendamment les uns des autres,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogéno-alkyle($C_1$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), alkyl($C_1$-$C_6$)sulfanyl-alkyle($C_1$-$C_6$), alkyl($C_1$-$C_6$)sulfinyl-alkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun, ou

représentent un groupe cycloalkyle en $C_3$-$C_6$ ou cycloalcényle en $C_3$-$C_6$ éventuellement substitué, qui peut éventuellement être interrompu par un ou plusieurs hétéroatomes de la série formée par les atomes d'oxygène, de soufre et d'azote ; ou

représentent un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl($C_1$-$C_6$)carbonyle, alcynyl($C_2$-$C_6$)carbonyle ou aminothiocarbonyle, les radicaux précités pouvant éventuellement être substitués chacun, ou représentent un groupe carbonyle ou carboxy ; ou

représentent un groupe phényle éventuellement substitué ou hétéroaryle éventuellement substitué ; ou

représentent un groupe alcoxy en $C_1$-$C_6$, halogénoalcoxy($C_1$-$C_6$), alcoxy($C_1$-$C_6$)-alcoxy($C_1$-$C_6$), cycloalkyloxy en $C_3$-$C_6$, cycloalkyl($C_3$-$C_6$)alkyl($C_1$-$C_6$)oxy, alcényloxy en $C_2$-$C_6$, cycloalcényl($C_3$-$C_6$)alcoxy($C_1$-$C_6$), alcynyloxy en $C_2$-$C_6$ ou carbonyloxy, les radicaux précités pouvant éventuellement être substitués chacun, ou représentent un groupe hydroxy ; ou

représentent un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représentent un groupe sulfanyle ; ou

$R^2$ et $R^3$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène ou de fluor ;

X et Y représentent indépendamment l'un de l'autre

un atome d'hydrogène, de fluor, chlore, brome, un groupe méthyle, éthyle, trifluorométhyle, (2,2)-difluorométhyle, difluorométhoxy, trifluorométhoxy, cyclopropyle, amino, hydroxy ou nitro ;

Z représente un atome d'hydrogène ; et

n représente le nombre 0 ou 1.

**12.** Composé selon la revendication 10 ou 11, dans lequel la sous-structure de formule (I-C) représente une sous-structure qui est choisie dans le groupe constitué par

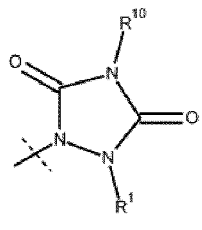
(I-C-1)

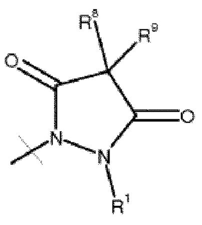
(I-C-2)

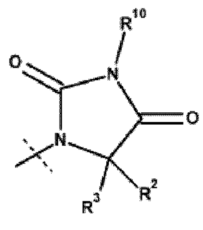
(I-C-3)

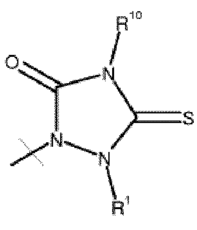
(I-C-5)

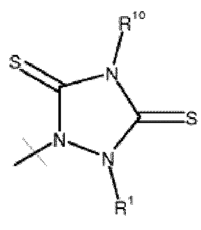
(I-C-6)

où

$R^1$ et $R^{10}$ chacun, indépendamment l'un de l'autre,

représentent un groupe alkyle en $C_1$-$C_4$, halogénoalkyle($C_2$-$C_4$), alcoxy($C_1$-$C_3$)alkyle($C_1$-$C_4$) ou alcényle en $C_2$-$C_4$ ; ou

un groupe cycloalkyle en $C_3$-$C_6$, cycloalkyl($C_3$-$C_6$)alkyle($C_1$-$C_2$), phényle ou phénylalkyle($C_1$-$C_2$), les radicaux

précités pouvant éventuellement être chacun une ou plusieurs fois substitués par halogéno, cyano, nitro, amino, hydroxy, méthyle, éthyle, trifluorométhyle, difluorométhyle, trifluoroéthyle, difluoroéthyle, méthoxy, éthoxy, trifluorométhoxy, trifluoroéthoxy, trifluoroéthylsulfanyle ou par un groupe cyclopropyle éventuellement substitué par méthyle, fluoro, chloro, cyano ; ou

$R^2$, $R^3$, $R^8$ et $R^9$, chacun indépendamment les uns des autres,

représentent un atome d'hydrogène ; ou

représentent un groupe alkyle en $C_1$-$C_6$, alcényle en $C_2$-$C_6$, alcynyle en $C_2$-$C_6$, halogénoalkyle($C_1$-$C_6$)ou phénylalkyle($C_1$-$C_3$), les radicaux précités pouvant éventuellement être chacun une ou plusieurs fois substitués par halogéno, cyano, nitro, amino, hydroxy, méthyle, éthyle, trifluorométhyle, difluorométhyle, trifluoroéthyle, difluoroéthyle, méthoxy, éthoxy, trifluorométhoxy, trifluoroéthoxy, trifluoroéthylsulfanyle ou par un groupe cyclopropyle éventuellement substitué par méthyle, fluoro, chloro, cyano ; ou

représentent un groupe cycloalkyle en $C_3$-$C_6$ ou phényle, les radicaux précités pouvant éventuellement être chacun une ou plusieurs fois substitués par halogéno, cyano, nitro, amino, hydroxy, méthyle, éthyle, trifluorométhyle, difluorométhyle, trifluoroéthyle, difluoroéthyle, méthoxy, éthoxy, trifluorométhoxy, trifluoroéthoxy, trifluoroéthylsulfanyle ou par un groupe cyclopropyle éventuellement substitué par méthyle, fluoro, chloro, cyano ; ou

$R^2$ et $R^3$ peuvent former ensemble, avec l'atome auquel ils sont liés, un cycle cyclopropyle, cyclobutyle, cyclopentyle ou cyclohexyle, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; ou

$R^8$ et $R^9$ peuvent former ensemble, avec l'atome auquel ils sont liés, un cycle cyclopropyle, cyclobutyle, cyclopentyle ou cyclohexyle, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ;

W représente un atome d'hydrogène ou de fluor ;

X représente un atome d'hydrogène, de chlore, de fluor ou un groupe méthyle ;

Y représente un atome de chlore, de brome, un groupe méthyle, trifluorométhyle ou un atome de fluor ;

en particulier X et Y représentant les combinaisons (Y, X) suivantes : (Me, F), (Me, H), (Me, Cl), (Me, Me), (Cl, Cl), (Cl, F), (Cl, H), (Me, H), (Br, H), (Br, F), (F, F), ($CF_3$, H) ;

Z représente un atome d'hydrogène ; et

n représente le nombre 0 ou 1.

**13.** Composé selon l'une quelconque des revendications 1 à 3, dans lequel

A et B représentent ensemble, avec les atomes auxquels ils sont liés, une sous-structure de formule (I-D),

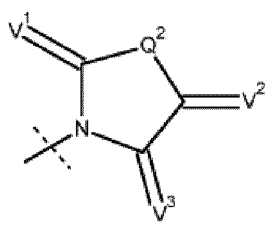

(I-D)

dans laquelle

$V^1$, $V^2$ et $V^3$ représentent chacun indépendamment,

un atome d'oxygène ; un atome de soufre, $NR^{11}$ ou un sel de $NR^{11}$ ;

$Q^2$ représente $NR^{10}$ ou $CR^8R^9$ ;

$R^{10}$ représente un atome d'hydrogène, un groupe cyano ou nitro ; ou

représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)-sulfanylalkyle($C_1$-$C_6$), halogénoalkyl($C_1$-$C_6$)sulfinylalkyle($C_1$-$C_6$), halogé-

noalkyl($C_1$-$C_6$)sulfonylalkyle($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), arylcarbonyle, alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogéno-alcoxy($C_1$-$C_6$)carbonyle, aryloxycarbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogéno-alkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl)amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ;

$R^{11}$ représente un atome d'hydrogène, un groupe cyano ou nitro ; ou

représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), hydroxy-alkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle($C_1$-$C_6$), alcoxy ($C_1$-$C_6$)alkyl($C_1$-$C_6$)-sulfanylalkyle ($C_1$-$C_6$), alcoxy($C_1$-$C_6$)-alkyl($C_1$-$C_6$)sulfinylalkyle($C_1$-$C_6$), alcoxy($C_1$-$C_6$)alkyl($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinylalkyle($C_1$-$C_6$), phénylsulfonylalkyle($C_1$-$C_6$), hétéroarylalkyle($C_1$-$C_6$), hétéroaryloxy-alkyle($C_1$-$C_6$), hétéroarylsulfanyl-alkyle($C_1$-$C_6$), hétéroarylsulfinyl-alkyle($C_1$-$C_6$), hétéroarylsulfonyl-alkyle($C_1$-$C_6$), alcoxy($C_1$-$C_6$)carbonyle, aryloxycarbonyle, alkyl($C_1$-$C_6$)carbonyle, arylcarbamoyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés ; ou

représente un groupe cycloalkyle en $C_3$-$C_6$ saturé ou insaturé, éventuellement substitué, qui peut éventuellement être interrompu par un ou plusieurs hétéroatomes ; ou

représente un groupe halogénoalkyl($C_1$-$C_6$)-carbonyle, hydroxyalkyl($C_1$-$C_6$)carbonyle, alcoxy($C_1$-$C_6$)alkyl($C_1$-$C_6$)carbonyle, phénylcarbonyle, hétéroarylcarbonyle, halogénoalcoxy($C_1$-$C_6$)carbonyle, aminocarbonyle, alkyl($C_1$-$C_6$)aminocarbonyle, dialkyl($C_1$-$C_6$)-aminocarbonyle, cycloalkyl($C_3$-$C_6$)aminocarbonyle, dicycloalk-

yl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl) aminocarbonyle, alkyl($C_1$-$C_6$)aminothiocarbonyle, dialkyl($C_1$-$C_6$)-aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe carbonyle ou carboxy ; ou

représente un groupe phényle éventuellement substitué ou hétéroaryle éventuellement substitué ; ou

représente un groupe alcoxy en $C_1$-$C_6$, halogénoalcoxy($C_1$-$C_6$), cycloalkyloxy en $C_3$-$C_6$, aryloxy, arylalkyl($C_1$-$C_6$)oxy ou carbonyloxy, les radicaux précités pouvant chacun éventuellement être substitués, ou représente un groupe hydroxy ; ou

représente un groupe alkyl($C_1$-$C_6$)amino, halogénoalkyl($C_1$-$C_6$)amino, dihalogénoalkyl($C_1$-$C_6$)amino, dialkyl($C_1$-$C_6$)amino, cycloalkyl($C_3$-$C_6$)amino, dicycloalkyl($C_3$-$C_6$)-amino, cycloalkyl($C_3$-$C_6$)(alkyl($C_1$-$C_6$))amino, arylamino, diarylamino, hétéroarylamino, dihétéroarylamino, alkyl($C_1$-$C_6$)(aryl)amino, cycloalkyl($C_3$-$C_6$)(aryl) amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe amino ; ou

représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représente un groupe sulfanyle ; et

$R^8$ et $R^9$, chacun indépendamment l'un de l'autre,

représentent un atome d'hydrogène ou d'halogène ou un groupe cyano ou nitro ; ou

représentent un groupe alkyle en $C_1$-$C_6$, cycloalkyl($C_3$-$C_6$)alkyle($C_1$-$C_6$), halogénoalkyle($C_1$-$C_6$), cyanoalkyle($C_1$-$C_6$), hydroxyalkyle($C_1$-$C_6$), alcoxy($C_1$-$C_6$)alkyle($C_1$-$C_6$), aminoalkyle($C_1$-$C_6$), alcoxy($C_1$-$C_6$)carbonyl-alkyle($C_1$-$C_6$), alkyl($C_1$-$C_6$)thioalkyle ($C_1$-$C_6$), halogénoalkyl($C_1$-$C_6$)thioalkyle($C_1$-$C_6$), alcoxy($C_1$-$C_6$)alkyl ($C_1$-$C_6$)thioalkyle, alkyl($C_1$-$C_6$)sulfanylalkyle($C_1$-$C_6$), alkyl($C_1$-$C_6$)sulfinylalkyle($C_1$-$C_6$), alkyl($C_1$-$C_6$)sulfonylalkyle ($C_1$-$C_6$), halogénoalkyl($C_1$-$C_6$)sulfanylalkyle($C_1$-$C_6$), halogénoalkyl($C_1$-$C_6$)sulfinylalkyle($C_1$-$C_6$), halogénoalkyl($C_1$-$C_6$)sulfonylalkyle ($C_1$-$C_6$), alcoxy($C_1$-$C_6$)alkyl($C_1$-$C_6$)sulfanylalkyle($C_1$-$C_6$), alcoxy($C_1$-$C_6$)alkyl($C_1$-$C_6$)sulfinylalkyle($C_1$-$C_6$), alcoxy($C_1$-$C_6$)alkyl ($C_1$-$C_6$)sulfonylalkyle($C_1$-$C_6$), phénylalkyle($C_1$-$C_6$), phénoxyalkyle($C_1$-$C_6$), phénylsulfanylalkyle($C_1$-$C_6$), phénylsulfinyl-alkyle($C_1$-$C_6$), phénylsulfonylalkyle($C_1$-$C_6$), hétéroarylalkyle($C_1$-$C_6$), hétéroaryloxy-alkyle($C_1$-$C_6$)ou hétéroarylthioalkyle($C_1$-$C_6$), les radicaux précités pouvant être chacun saturés ou insaturés et/ou éventuellement substitués ; ou

représentent un groupe cycloalkyle en $C_3$-$C_6$ saturé ou insaturé, éventuellement substitué, qui peut éventuellement être interrompu par un ou plusieurs hétéroatomes ; ou

représentent un groupe alkyl($C_1$-$C_6$)carbonyle, halogénoalkyl($C_1$-$C_6$)carbonyle, hydroxy-alkyl($C_1$-$C_6$)carbonyle, alcoxy($C_1$-$C_6$)-alkyl($C_1$-$C_6$)carbonyle, phénylcarbonyle, hétéroarylcarbonyle, alcoxy($C_1$-$C_6$)carbonyle, halogénoalcoxy($C_1$-$C_6$)carbonyle, aryloxy-carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, dicycloalkyl($C_3$-$C_6$)aminocarbonyle, cycloalkyl($C_3$-$C_6$)(alkyl($C_1$-$C_6$))aminocarbonyle, arylaminocarbonyle, diarylaminocarbonyle, alkyl($C_1$-$C_6$)(aryl)aminocarbonyle, cycloalkyl($C_3$-$C_6$)(aryl)aminocarbonyle, alkyl($C_1$-$C_6$)aminothiocarbonyle, dialkyl($C_1$-$C_6$)-aminothiocarbonyle ou aminothiocarbonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe carbonyle ou carboxy ; ou

représentent un groupe phényle éventuellement substitué ou hétéroaryle éventuellement substitué ;

représentent un groupe alcoxy en $C_1$-$C_6$, halogénoalcoxy($C_1$-$C_6$), alcoxy($C_1$-$C_6$)-alcoxy($C_1$-$C_6$), aryloxy, arylalkyl($C_1$-$C_6$)oxy, cycloalkyloxy en $C_3$-$C_6$, cycloalkyl($C_3$-$C_6$)-alkyl($C_1$-$C_6$)oxy ou carbonyloxy, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe hydroxy ; ou

représentent un groupe alkyl($C_1$-$C_6$)amino, dialkyl($C_1$-$C_6$)amino, halogénoalkyl($C_1$-$C_6$)amino, dihalogénoalkyl($C_1$-$C_6$)amino, cycloalkyl($C_3$-$C_6$)-amino, dicycloalkyl($C_3$-$C_6$)amino, cycloalkyl($C_3$-$C_6$)(alkyl($C_1$-$C_6$))amino,

arylamino, diarylamino, hétéroarylamino, dihétéroarylamino, alkyl($C_1$-$C_6$)(aryl)amino, cycloalkyl($C_3$-$C_6$)(aryl) amino, alkyl($C_1$-$C_6$)-carbonylamino, arylcarbonylamino, alcoxy($C_1$-$C_6$)carbonylamino, aryloxycarbonylamino, alkyl($C_1$-$C_6$)carbamoylamino, arylcarbamoylamino, alkyl($C_1$-$C_6$)sulfonylamino, ou arylsulfonylamino, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe amino ; ou

représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfonyle, cycloalkyl($C_3$-$C_6$)sulfanyle, cycloalkyl($C_3$-$C_6$)sulfinyle, cycloalkyl($C_3$-$C_6$)-sulfonyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, arylsulfanyle, arylsulfinyle, arylsulfonyle, arylalkyl($C_1$-$C_6$)sulfanyle, arylalkyl($C_1$-$C_6$)sulfinyle, arylalkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun et/ou avec la condition que, dans le cas d'un radical insaturé, le nombre minimum défini d'atomes de carbone soit égal à 2, les radicaux pouvant être saturés ou insaturés, ou représentent un groupe sulfanyle ; ou

$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ; et éventuellement interrompu par des hétéroatomes de la série formée par les atomes d'oxygène, de soufre et d'azote ;

W représente un atome d'hydrogène, de fluor ou de chlore ;

X, Y et Z chacun indépendamment

représentent un atome d'hydrogène, de fluor, chlore, brome, un groupe nitro, amino, hydroxy, alkyle en $C_1$-$C_4$, halogéno-alkyle($C_1$-$C_4$), alcényle en $C_2$-$C_4$, alcynyle en $C_2$-$C_4$, alcoxy en $C_1$-$C_4$, halogénoalcoxy($C_1$-$C_4$) ou aminothiocarbonyle ;

ou représentent un groupe benzyle, phénoxy, phénylthio, cyclopropylméthyle, cyclopropyloxy ou cyclopropylthio, qui est éventuellement une ou plusieurs fois substitué par des substituants, identiques ou différents, fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ;

ou représentent un groupe cyclopropyle, cyclobutyle, cyclopentyle, cyclohexyle ou phényle, qui est éventuellement une ou plusieurs fois substitué par des substituants, identiques ou différents, fluoro, chloro, bromo, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou représentent un groupe cyclopropyle éventuellement substitué par méthyle, fluoro, chloro, cyano ; et

n représente le nombre 0 ou 1.

**14.** Composé selon la revendication 13, dans lequel la sous-structure de formule (I-D) représente une sous-structure qui est choisie dans le groupe constitué par

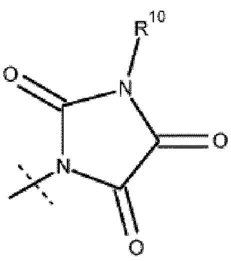
(I-D-1)

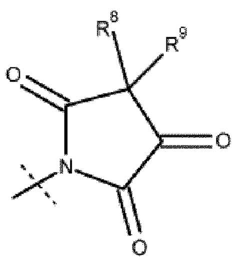
(I-D-2)

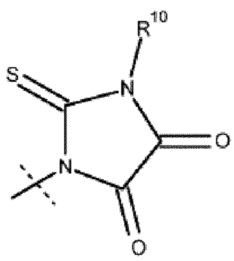
(I-D-3)

où

$R^{10}$ représente un atome d'hydrogène ; ou

représente un groupe alkyle en $C_1$-$C_6$, cycloalkyl($C_3$-$C_6$)alkyle($C_1$-$C_6$), halogéno-alkyle($C_2$-$C_6$), cyanoalkyle($C_1$-$C_6$), alcoxy ($C_1$-$C_6$)alkyle ($C_1$-$C_6$), alcoxy($C_1$-$C_6$)-carbonylalkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en

$C_2$-$C_6$, les radicaux précités pouvant éventuellement être substitués chacun ; ou
représente un groupe cycloalkyle en $C_3$-$C_6$, cycloalcényle en $C_3$-$C_6$, éventuellement substitué, qui peut éventuellement être interrompu par un ou plusieurs hétéroatomes de la série formée par les atomes d'oxygène, de soufre et d'azote ; ou
représente un groupe alkyl($C_1$-$C_6$)carbonyle, halogénoalkyl ($C_1$-$C_6$)carbonyle, alcoxy($C_1$-$C_6$)-alkyl($C_1$-$C_6$)carbonyle, alcoxy ($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl ($C_1$-$C_6$)carbonyle, alcynyl($C_2$-$C_6$)carbonyle ou aminothiocarbonyle, les radicaux précités pouvant éventuellement être substitués chacun, ou représente un groupe carbonyle ou carboxy ; ou
représente un groupe phényle éventuellement substitué ou hétéroaryle éventuellement substitué ; ou
représente un groupe alcoxy en $C_1$-$C_6$, halogénoalcoxy($C_1$-$C_6$), cycloalkyloxy en $C_3$-$C_6$ ou carbonyloxy, les radicaux précités pouvant chacun éventuellement être substitués, ou représente un groupe hydroxy ; ou
représente un groupe alkyl($C_1$-$C_6$)amino, halogénoalkyl($C_1$-$C_6$)amino, dihalogénoalkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)-carbamoylamino, alkyl($C_1$-$C_6$)sulfonylamino, alcényl($C_2$-$C_6$)amino, cycloalcényl($C_3$-$C_6$)-alkyl($C_1$-$C_6$)amino, alcynyl($C_2$-$C_6$)amino, les radicaux précités pouvant éventuellement être substitués chacun, ou représentent un groupe amino ; ou
représente un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl ($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représente un groupe sulfanyle ; et
$R^8$ et $R^9$, chacun indépendamment l'un de l'autre,
représentent un atome d'hydrogène ou d'halogène ou un groupe cyano ou nitro ; ou
représentent un groupe alkyle en $C_1$-$C_6$, cycloalkyl ($C_3$-$C_6$)alkyle ($C_1$-$C_6$), halogéno-alkyle($C_1$-$C_6$), cyanoalkyle($C_1$-$C_6$), alcoxy($C_1$-$C_6$)alkyle($C_1$-$C_6$), alcoxy($C_1$-$C_6$)-carbonylalkyle($C_1$-$C_6$), alkyl($C_1$-$C_6$)sulfanyl-alkyle($C_1$-$C_6$), alkyl($C_1$-$C_6$)sulfinyl-alkyle($C_1$-$C_6$), phénylalkyle($C_1$-$C_6$), hétéroarylalkyle($C_1$-$C_6$), alcényle en $C_2$-$C_6$, halogénoalcényle($C_2$-$C_6$), cycloalcényl($C_3$-$C_6$)-alkyle($C_1$-$C_6$), alcynyle en $C_2$-$C_6$, les radicaux précités pouvant éventuellement être substitués chacun ; ou
représentent un groupe cycloalkyle en $C_3$-$C_6$, cycloalcényle en $C_3$-$C_6$, éventuellement substitué, qui peut éventuellement être interrompu par un ou plusieurs hétéroatomes de la série formée par les atomes d'oxygène, de soufre et d'azote ; ou
représentent un groupe alkyl($C_1$-$C_6$)carbonyle, halogénoalkyl($C_1$-$C_6$)carbonyle, alcoxy($C_1$-$C_6$)-alkyl($C_1$-$C_6$)carbonyle, alcoxy($C_1$-$C_6$)carbonyle, halogénoalcoxy($C_1$-$C_6$)carbonyle, aminocarbonyle, alkyl($C_1$-$C_6$)aminocarbonyle, dialkyl($C_1$-$C_6$)aminocarbonyle, cycloalkyl($C_3$-$C_6$)aminocarbonyle, alkyl($C_1$-$C_6$)-aminothiocarbonyle, dialkyl($C_1$-$C_6$)aminothiocarbonyle, alcényl($C_2$-$C_6$)carbonyle, cycloalcényl($C_3$-$C_6$)alkyl ($C_1$-$C_6$)carbonyle, alcynyl($C_2$-$C_6$)carbonyle ou aminothiocarbonyle, les radicaux précités pouvant éventuellement être substitués chacun, ou représentent un groupe carbonyle ou carboxy ; ou
représentent un groupe phényle éventuellement substitué ou hétéroaryle éventuellement substitué ; ou
représentent un groupe alcoxy en $C_1$-$C_6$, halogénoalcoxy ($C_1$-$C_6$), alcoxy($C_1$-$C_6$)-alcoxy($C_1$-$C_6$), cycloalkyloxy en $C_3$-$C_6$, cycloalkyl($C_3$-$C_6$)alkyl ($C_1$-$C_6$)oxy, alcényloxy en $C_2$-$C_6$, cycloalcényl($C_3$-$C_6$)alcoxy($C_1$-$C_6$), alcynyloxy en $C_2$-$C_6$ ou carbonyloxy, les radicaux précités pouvant éventuellement être substitués chacun, ou représentent un groupe hydroxy ; ou
représentent un groupe alkyl($C_1$-$C_6$)amino, halogénoalkyl($C_1$-$C_6$)amino, dihalogénoalkyl($C_1$-$C_6$)amino, dialkyl($C_1$-$C_6$)amino, cycloalkyl($C_3$-$C_6$)amino, dicycloalkyl($C_3$-$C_6$)-amino, alkyl($C_1$-$C_6$)carbonylamino, alcoxy($C_1$-$C_6$)carbonylamino, alkyl($C_1$-$C_6$)carbamoylamino, alkyl($C_1$-$C_6$)-sulfonylamino, alcényl($C_2$-$C_6$)amino, cycloalcényl($C_3$-$C_6$)alkyl($C_1$-$C_6$)amino, alcynyl($C_2$-$C_6$)amino, les radicaux précités pouvant éventuellement être substitués chacun, ou représentent un groupe amino ; ou
représentent un groupe alkyl($C_1$-$C_6$)sulfanyle, alkyl($C_1$-$C_6$)sulfinyle, alkyl($C_1$-$C_6$)sulfonyle, halogénoalkyl($C_1$-$C_6$)sulfanyle, halogénoalkyl($C_1$-$C_6$)sulfinyle, halogénoalkyl($C_1$-$C_6$)-sulfanyle, cycloalkyl($C_3$-$C_6$)alkyl($C_1$-$C_6$)-sulfanyle, cycloalkyl($C_3$-$C_6$)alkyl($C_1$-$C_6$)-sulfinyle, cycloalkyl($C_3$-$C_6$)alkyl($C_1$-$C_6$)-sulfonyle, aminosulfonyle, alkyl($C_1$-$C_6$)aminosulfonyle, dialkyl($C_1$-$C_6$)aminosulfonyle ou arylaminosulfonyle, les radicaux précités pouvant éventuellement être substitués chacun, ou représentent un groupe sulfanyle ; ou
$R^8$ et $R^9$ peuvent former ensemble, avec l'atome auquel ils sont liés, un cycle à trois à six chaînons saturé ou insaturé, éventuellement substitué par des substituants identiques ou différents halogéno, cyano, nitro, hydroxy, amino, méthyle, éthyle, trifluorométhyle, difluorométhyle, trifluoroéthyle, difluoroéthyle, méthoxy, éthoxy, trifluorométhoxy, trifluoroéthoxy ou cycloalkyle en $C_3$-$C_6$ éventuellement substitué par méthyle, fluoro, chloro, cyano ;

et éventuellement interrompu par des hétéroatomes de la série formée par les atomes d'oxygène, de soufre et d'azote ;
W représente un atome d'hydrogène ou de fluor ;
X et Y représentent chacun, indépendamment l'un de l'autre, un atome d'hydrogène, de fluor, chlore, brome, un groupe méthyle, éthyle, trifluorométhyle, (2,2)-difluorométhyle, difluorométhoxy, trifluorométhoxy, cyclopropyle, amino, hydroxy ou nitro ;
Z représente un atome d'hydrogène ; et
n représente le nombre 0 ou 1.

**15.** Composé selon la revendication 13 ou 14, dans lequel la sous-structure de formule (I-D) représente une sous-structure qui est choisie dans le groupe constitué par

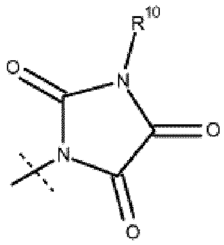
(I-D-1)

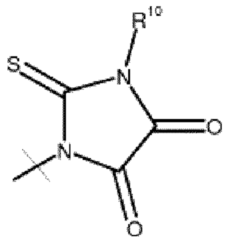
(I-D-3)

où

$R^{10}$ représente un atome d'hydrogène, un groupe méthyle, éthyle, propyle, isopropyle, tert-butyle, cyclopropylméthyle, $CH_2CH(CH_3)_2$, $CH_2C(CH_3)_3$, $CH_2CF_3$, $CH_2CHF_2$, $CH(CH_3)CF_3$, $CH_2CH_2CF_3$, $CH_2CH_2CHF_2$ ou un groupe cyclopropyle éventuellement substitué par méthyle, fluoro, chloro, cyano ou un groupe cyclohexyle éventuellement substitué par méthyle, fluoro, chloro, cyano ou un groupe cyclopropylméthyle éventuellement substitué par méthyle, fluoro, chloro, cyano ;
W représente un atome d'hydrogène ou de fluor ;
X représente un atome d'hydrogène, de chlore ou de fluor ou un groupe méthyle ;
Y représente un atome de chlore ou de brome, un groupe méthyle, trifluorométhyle ou un atome de fluor ;
en particulier X et Y représentant les combinaisons (Y, X) suivantes : (Me, F), (Me, H), (Me, Cl), (Me, Me), (Cl, Cl), (Cl, F), (Cl, H), (Br, H), (Br, F), (F, F), ($CF_3$, H) ;
Z représente un atome d'hydrogène ; et
n représente le nombre 0 ou 1.

**16.** Composition de substances actives, contenant au moins un composé de formule générale (I) selon l'une quelconque des revendications 1 à 15 et au moins une autre substance active insecticide, acaricide ou nématicide choisie dans le groupe constitué par

(1) Les inhibiteurs d'acétylcholinestérase (AChE), comme par exemple
carbamates, par exemple alanycarbe, aldicarbe, bendiocarbe, benfuracarbe, butocarboxime, butoxycarboxime, carbaryl, carbofuran, carbosulfan, éthiofencarbe, fénobucarbe, formétanate, furathiocarbe, isoprocarbe, méthiocarbe, méthomyl, métolcarbe, oxamyl, pirimicarbe, propoxur, thiodicarb, thiofanox, triazamate, trimétacarb, XMC et xylylcarb ; ou
organophosphates, par exemple acéphate, azaméthiphos, azinphos-éthyle, azinphos-méthyle, cadusafos, chloréthoxyfos, chlorfenvinphos, chlorméphos, chlorpyrifos, chlorpyrifos-méthyle, coumaphos, cyanophos, déméton-S-méthyle, diazinon, dichlorvos/DDVP, dicrotophos, diméthoate, diméthylvinphos, disulfoton, EPN, éthion, éthoprophos, famphur, fénamiphos, fénitrothion, fenthion, fosthiazate, hepténophos, imicyafos, isofenphos, 0-(méthoxyaminothio-phosphoryl)salicylate d'isopropyle, isoxathion, malathion, mécarbame, méthamidophos, méthidathion, mévinphos, monocrotophos, naled, ométhoate, oxydéméton-méthyle, parathion, parathion-méthyle, phenthoate, phorate, phosalone, phosmet, phosphamidon, phoxime, pirimiphos-méthyle, profénofos, propétamphos, prothiofos, pyraclofos, pyridaphenthion, quinalphos, sulfotep, tébupirimphos, téméphos, terbufos, tétrachlorvinphos, thiométon, triazophos, trichlorphon et vamidothion.
(2) Les antagonistes de canaux chlorure commandés par GABA, comme par exemple
cyclodiène-organochlores, par exemple chlordane et endosulfan ; ou

phénylpyrazoles (fiproles), par exemple éthiprol et fipronil.

(3) Les modulateurs de canaux sodiques / agents bloquant les canaux sodiques en fonction de la tension, comme par exemple

pyréthroïdes, par exemple acrinathrine, alléthrine, alléthrine d-cis-trans, alléthrine d-trans, bifenthrine, bioalléthrine, isomère S-cyclopenténylе de bioalléthrine, bioresméthrine, cycloprothrine, cyfluthrine, bêta-cyfluthrine, cyhalothrine, lambda-cyhalothrine, gamma-cyhalothrine, cyperméthrine, alpha-cyperméthrine, bêta-cyperméthrine, thêta-cyperméthrine, zêta-cyperméthrine, cyphénothrine [isomères (1R)-trans], deltaméthrine, empenthrine [isomères (EZ)-(1R)], esfenvalérate, étofenprox, fenpropathrine, fenvalérate, flucythrinate, fluméthrine, tau-fluvalinate, halfenprox, imiprothrine, kadéthrine, perméthrine, phénothrine [isomère (1R)-trans], pralléthrine, pyréthrine (pyréthrum), resméthrine, silafluofène, téfluthrine, tetraméthrine, tetraméthrine [isomères (1R)], tralométhrine et transfluthrine ; ou

DDT ; ou méthoxychlore.

(4) Les agonistes de récepteurs nicotinergiques d'acétylcholine (nAChR), comme par exemple

néonicotinoïdes, par exemple acétamipride, clothianidine, dinotéfuran, imidaclopride, nitenpyram, thiaclopride et thiaméthoxame ; ou

nicotine.

(5) Les activateurs allostériques des récepteurs nicotinergiques d'acétylcholine (nAChR), comme par exemple spinosines, par exemple spinétorame et spinosad.

(6) Les activateurs de canaux chlorure, comme par exemple

avermectines/milbémycines, par exemple abamectine, émamectine-benzoate, lépimectine et milbémectine.

(7) Les mimétiques d'hormone juvénile, comme par exemple

analogues d'hormone juvénile, par exemple hydroprène, kinoprène et méthoprène ; ou

fénoxycarb ; ou pyriproxyfène.

(8) Les substances actives à mécanisme d'action inconnu ou non spécifique comme par exemple

halogénures d'alkyle, par exemple bromure de méthyle et autres halogénures d'alkyle ; ou

chloropicrine ; ou fluorure de sulfuryle ; ou borax ; ou tartrate d'antimoine et de potassium.

(9) Les inhibiteurs d'alimentation sélectifs, par exemple pymétrozine ; ou flonicamide.

(10) Les inhibiteurs de la croissance d'acariens, par exemple chlofentézine, hexythiazox et diflovidazine ; ou étoxazole.

(11) Les perturbateurs microbiens de la membrane intestinale d'insectes, par exemple *Bacillus thuringiensis* sous-espèce *israelensis, Bacillus sphaericus, Bacillus thuringiensis* sous-espèce *aizawai, Bacillus thuringiensis* sous-espèce *kurstaki, Bacillus thuringiensis* sous-espèce *tenebrionis* et protéines végétales de BT : Cry1Ab, Cry1Ac, CrylFa, Cry2Ab, mCry3A, Cry3Ab, Cry3Bb, Cry34/35Ab1.

(12) Les inhibiteurs de la phosphorylation oxydante, perturbateurs d'ATP, comme par exemple diafenthiuron ; ou composés organostanniques, par exemple azocyclotine, cyhexatine et fenbutatine-oxyde ; ou

propargite ; ou tétradifon.

(13) Les découpleurs de la phosphorylation oxydante par interruption du gradient de protons H, comme par exemple chlorfénapyr, DNOC et sulfluramide.

(14) Les antagonistes des récepteurs nicotinergiques d'acétylcholine, comme par exemple bensultap, cartap-chlorhydrate, thiocyclame et thiosultap-sodium.

(15) Les inhibiteurs de la biosynthèse de la chitine, type 0, comme par exemple bistrifluron, chlorfluazuron, diflubenzuron, flucycloxuron, flufénoxuron, hexaflumuron, leufénuron, novaluron, noviflumuron, téflubenzuron et triflumuron.

(16) Les inhibiteurs de la biosynthèse de la chitine, type 1, comme par exemple buprofézine.

(17) Les substances actives perturbatrices de mue, diptéran, comme par exemple cyromazine.

(18) Les agonistes des récepteurs d'ecdysone, comme par exemple chromfénozide, halofénozide, méthoxyfénozide et tébufénozide.

(19) Les agonistes octopaminergiques, comme par exemple amitraz.

(20) Les inhibiteurs du transport d'électrons de complexe III, comme par exemple hydraméthylnone ; ou acéquinocyl ; ou fluacrypyrim.

(21) Inhibiteurs du transport d'électrons de complexe I, par exemple

les acaricides METI, par exemple fénazaquine, fenpyroximate, pyrimidifène, pyridabène, tébufenpyrad et tolfenpyrad ; ou

roténone (derris).

(22) Les agents bloquant les canaux sodiques en fonction de la tension, par exemple indoxacarbe ; ou métaflumizone.

(23) Les inhibiteurs de l'acétyl-CoA-carboxylase, comme par exemple

les dérivés d'acide tétronique et d'acide tétramique, par exemple spirodiclofène, spiromésifène et spirotétramate.

(24) Les inhibiteurs du transport d'électrons de complexe IV, comme par exemple
des phosphines, par exemple phosphure d'aluminium, phosphure de calcium, phosphine et phosphure de zinc ; ou
cyanure.
(25) Les inhibiteurs du transport d'électrons de complexe II, comme par exemple cyénopyrafène.
(28) Les effecteurs des récepteurs de ryanodine, comme par exemple
des diamides, par exemple chlorantraniliprole et flubendiamide ;
d'autres substances actives à mécanisme d'action inconnu, comme par exemple amidoflumet, azadirachtine, benclothiaz, benzoximate, bifénazate, bromopropylate, chinométhionate, cryolite, cyantraniliprole (cyazypyr), cyflumétofène, dicofol, diflovidazine, fluensulfone, flufénérim, flufiprole, fluopyram, fufénozide, imidaclothiz, iprodione, méperfluthrine, pyridalyl, pyrifluquinazone, tétraméthylfluthrine et iodométhane ; en outre des préparations à base de *Bacillus firmus* (en particulier souche CNCM I-1582, par exemple VOTiVO™ BioNem) ainsi que les composés actifs connus suivants :

3-Bromo-N-{2-bromo-4-chloro-6-[(1-cyclopropyléthyl)-carbamoyl]phényl}-1-(3-chloropyridin-2-yl)-1H-pyrazole-5-carboxamide 4-{[(6-bromopyrid-3-yl)méthyl](2-fluoro-éthyl)amino}furan-2(5H)-one, 4-{[(6-fluoropyrid-3-yl)-méthyl](2,2-difluoroéthyl)amino}furan-2(5H)-one, 4-{[(2-chloro-1,3-thiazol-5-yl)méthyl](2-fluoroéthyl)-amino}furan-2(5H)-one, 4-{[(6-chloropyrid-3-yl)méthyl]-(2-fluoroéthyl)amino}furan-2(5H)-one, flupyradifurone, 4-{[(6-chloro-5-fluoropyrid-3-yl)méthyl](méthyl)amino}furan-2(5H)-one, 4-{[(5,6-dichloropyrid-3-yl)méthyl](2-fluoroéthyl)amino}furan-2(5 H)-one, 4-{[(6-chloro-5-fluoropyrid-3-yl)méthyl](cyclopropyl)amino}furan-2(5H)-one, 4-{[(6-chloropyrid-3-yl)méthyl](cyclopropyl)-amino}furan-2(5H)-one, 4-{[(6-chloropyrid-3-yl)méthyl]-(méthyl)amino}furan-2(5H)-one, {[1-(6-chloropyridin-3-yl)éthyl](méthyl)oxydo-$\lambda^4$-sulfanylidène}cyanamide et ses diastéréoisomères {[(1R)-1-(6-chloropyridin-3-yl)-éthyl](méthyl)oxydo-$\lambda^4$-sulfanylidène}cyanamide (A) et {[(1S)-1-(6-chloropyridin-3-yl)éthyl](méthyl)oxydo-$\lambda^4$-sulfanylidène}cyanamide (B) ainsi que sulfoxaflor et ses diastéréoisomères [(R)-méthyl(oxydo){(1R)-1-[6-(trifluorométhyl)pyridin-3-yl]éthyl}-$\lambda^4$-sulfanylidène]-cyanamide (A1) et [(S)-méthyl(oxydo){(1S)-1-[6-(trifluorométhyl)pyridin-3-yl]éthyl}-$\lambda^4$-sulfanylidène]-cyanamide (A2), désignés par groupe de diastéréoisomères A, [(R)-méthyl(oxydo){(1S)-1-[6-(trifluorométhyl)pyridin-3-yl]éthyl}-$\lambda^4$-sulfanylidène]-cyanamide (B1) et [(S)-méthyl(oxo){(1R)-1-[6-(trifluorométhyl)pyridin-3-yl]éthyl}-$\lambda^4$-sulfanylidène]-cyanamide (B2), désignés par groupe de diastéréoisomères B et 11-(4-chloro-2,6-diméthylphényl)-12-hydroxy-1,4-dioxa-9-azadispiro-[4.2.4.2]tétradéc-11-én-10-one, 3-(4'-fluoro-2,4-diméthylbiphényl-3-yl)-4-hydroxy-8-oxa-1-azaspiro[4.5]-déc-3-én-2-one, 1-{2-fluoro-4-méthyl-5-[(2,2,2-trifluoroéthyl)sulfinyl]phényl}-3-(trifluorométhyl)-1H-1,2,4-triazol-5-amine, [(3S,4aR,12R,12aS,12bS)-3-[(cyclopropylcarbonyl)oxy]-6,12-dihydroxy-4,12b-diméthyl-11-oxo-9-(pyridin-3-yl)-1,3,4,4a,5,6,6a,12,12a,12b-décahydro-2H,11H-benzo[f]-pyrano[4,3-b]chromn-4-yl]méthylcyclopropanecarboxylate, 2-cyano-3-(difluorométhoxy)-N,N-diméthylbenzène-sulfonamide, 2-cyano-3-(difluorométhoxy)-N-méthyl-benzènesulfonamide, 2-cyano-3-(difluorométhoxy)-N-éthylbenzènesulfonamide, 4-(difluorométhoxy)-N-éthyl-N-méthyl-1,2-benzothiazol-3-amine-1,1-dioxyde, N-[1-(2,3-diméthylphényl)-2-(3,5-diméthylphényl)éthyl]-4,5-dihydro-1,3-thiazol-2-amine, {1'-[(2E)-3-(4-chloro-phényl)prop-2-én-1-yl]-5-fluorospiro[indole-3,4'-pipéridin]-1(2H)-yl}(2-chloropyridin-4-yl)méthanone, 3-(2,5-diméthylphényl)-4-hydroxy-8-méthoxy-1,8-diazaspiro[4.5]déc-3-én-2-one, carbonate de 3-(2,5-diméthylphényl)-8-méthoxy-2-oxo-1,8-diazaspiro[4.5]déc-3-én-4-yle et d'éthyle, 4-(but-2-yn-1-yloxy)-6-(3,5-diméthylpipéridin-1-yl)-5-fluoropyrimidine, (2,2,3,3,4,4,5,5-octafluoropentyl) (3,3,3-trifluoro-propyl)malononitrile, (2,2,3,3,4,4,5,5-octafluoropentyl) (3,3,4,4,4-pentafluorobutyl)malononitrile, 8-[2-(cyclopropylméthoxy)-4-(trifluorométhyl)phénoxy]-3-[6-(trifluorométhyl)pyridazin-3-yl]-3-azabicyclo[3.2.1]-octane, flométoquine, PF1364 (n° d'enregistrement CAS 1204776-60-2), 5-[5-(3,5-dichlorophényl)-5-(trifluorométhyl)-4,5-dihydro-1,2-oxazol-3-yl]-2-(1H-1,2,4-triazol-1-yl)benzonitrile, 5-[5-(2-chloropyridin-4-yl)-5-(trifluorméthyl)-4,5-dihydro-1,2-oxazol-3-yl]-2-(1H-1,2,4-triazol-1-yl)benzonitrile, 4-[5-(3,5-dichloro-phényl)-5-(trifluorméthyl)-4,5-dihydro-1,2-oxazol-3-yl]-2-méthyl-N-{2-oxo-2-[(2,2,2-trifluoroéthyl)amino]-éthyl}benzamide, 4-{[(6-chloropyridin-3-yl)méthyl]-(cyclopropyl)amino}-1,3-oxazol-2(5H)-one, 4-{[(6-chlorpyridin-3-yl)méthyl] (2,2-difluoréthyl)amino}-1,3-oxazol-2(5H)-one, 4-{[(6-chloropyridin-3-yl)méthyl]-(éthyl)amino}-1,3-oxazol-2(5H)-one, 4-{[(6-chloro-pyridin-3-yl)méthyl] (méthyl)amino}-1,3-oxazol-2(5H)-one, NNI-0711, 1-acétyl-N-[4-(1,1,1,3,3,3-hexafluoro-2-méthoxypropan-2-yl)-3-isobutylphényl]-N-isobutyryl-3,5-diméthyl-1H-pyrazole-4-carboxamide, 2-[2-({[3-bromo-1-(3-chloropyridin-2-yl)-1H-pyrazol-5-yl]carbonyl}amino)-5-chloro-3-méthylbenzoyl]-2-méthylhydrazincarboxylate de méthyle, 2-[2-({[3-bromo-1-(3-chloropyridin-2-yl)-1H-pyrazol-5-yl]carbonyl}amino)-5-cyano-3-méthyl-benzoyl]-2-éthylhydrazinecarboxylate de méthyle, 2-[2-({[3-bromo-1-(3-chloropyridin-2-yl)-1H-pyrazol-5-yl]carbonyl}amino)-5-cyano-3-méthylbenzoyl]-2-méthyl-hydrazinecarboxylate de méthyle, 2-[3,5-dibromo-2-({[3-bromo-1-(3-chloropyridin-2-yl)-1H-pyrazol-5-yl]-carbonyl}amino)benzoyl]-1,2-diéthylhydrazinecarboxylate de méthyle, 2-[3,5-dibromo-2-({[3-bromo-1-(3-chloropyridin-2-yl)-1H-pyrazol-5-yl]carbonyl}amino)-benzoyl]-2-éthylhydrazinecarboxylate de méthyle, (5RS,7RS;5RS,7SR)-1-(6-chloro-3-pyridylméthyl)-1,2,3,5,6,7-hexahydro-7-méthyl-8-nitro-5-propoxy-imidazo[1,2-a]pyridine, 2-{6-[2-(5-fluoropyridin-3-yl)-1,3-thiazol-5-yl]pyridin-2-yl}pyrimidine, 2-{6-[2-(pyridin-3-yl)-1,3-thiazol-5-yl]pyridin-2-yl}-pyrimidine, 1-(3-chloropyridin-2-yl)-N-[4-cyano-2-méthyl-6-(méthylcarbamoyl)phényl]-3-{[5-(trifluoro-méthyl)-1H-tétrazol-1-yl]méthyl}-1H-pyrazole-5-carboxamide, 1-(3-chlorpyridin-2-yl)-N-[4-cyano-2-méthyl-6-(méthylcarbamoyl)phényl]-3-{[5-(trifluoro-méthyl)-2H-tétrazol-2-yl]méthyl}-1H-pyrazole-5-carboxamide, N-[2-(tert-butylcarbamoyl)-4-cyano-6-méthylphényl]-1-(3-chloropyridin-2-yl)-3-{[5-(trifluorométhyl)-1H-tétrazol-1-yl]méthyl}-1H-pyrazole-5-carboxamide, N-[2-(tert-butylcarbamoyl)-4-cyano-6-méthylphényl]-1-(3-chloropyridin-2-yl)-3-{[5-(trifluorométhyl)-2H-tétrazol-2-yl]méthyl}-1H-pyrazole-5-carboxamide, (1E)-N-[(6-chloropyridin-3-yl)méthyl]-N'-cyano-N-(2,2-difluoroéthyl)éthanimidamide, N-[2-(5-amino-1,3,4-thiadiazol-2-yl)-4-chloro-6-méthylphényl]-3-bromo-1-(3-chloropyridin-2-yl)-1H-pyrazole-5-carboxamide et 2-[3,5-dibromo-2-({[3-bromo-1-(3-chloropyridin-2-yl)-1H-pyrazol-5-yl]carbonyl}amino)-benzoyl]-2-éthyl-1-méthylhydrazinecarboxylate de méthyle,
et/ou au moins une autres substance active fongicide choisie dans le groupe constitué par

(1) Les inhibiteurs de la biosynthèse de l'ergostérol, comme par exemple (1.1) aldimorphe (1704-28-5), (1.2) azaconazole (60207-31-0), (1.3) bitertanol (55179-31-2), (1.4) bromuconazole (116255-48-2), (1.5) cyproconazole (113096-99-4), (1.6) diclobutrazole (75736-33-3), (1.7) difénoconazole (119446-68-3), (1.8) diniconazole (83657-24-3), (1.9) diniconazole-M (83657-18-5), (1.10) dodémorphe (1593-77-7), (1.11) dodémorphe acétate (31717-87-0), (1.12) époxiconazole (106325-08-0), (1.13) étaconazole (60207-93-4), (1.14) fénarimol (60168-88-9), (1.15) fenbuconazole (114369-43-6), (1.16) fenhexamide (126833-17-8), (1.17) fenpropidine (67306-00-7), (1.18) fenpropimorphe (67306-03-0), (1.19) fluquinconazole (136426-54-5), (1.20) flurprimidol (56425-91-3), (1.21) flusilazole (85509-19-9), (1.22) flutriafol (76674-21-0), (1.23) furconazole (112839-33-5), (1.24) furconazole-cis (112839-32-4), (1.25) hexaconazole (79983-71-4), (1.26) imazalil (60534-80-7), (1.27) imazalil sulfate (58594-72-2), (1.28) imibenconazole (86598-92-7), (1.29) ipconazole (125225-28-7), (1.30) metconazole (125116-23-6), (1.31) myclobutanil (88671-89-0), (1.32) naftifine (65472-88-0), (1.33) nuarimol (63284-71-9), (1.34) oxpoconazole (174212-12-5), (1.35) paclobutrazole (76738-62-0), (1.36) pefurazoate (101903-30-4), (1.37) penconazole (66246-88-6), (1.38) pipéraline (3478-94-2), (1.39) prochloraz (67747-09-5), (1.40) propiconazole (60207-90-1), (1.41) prothioconazole (178928-70-6), (1.42) pyributicarbe (88678-67-5), (1.43) pyrifénox (88283-41-4), (1.44) quinconazole (103970-75-8), (1.45) siméconazole (149508-90-7), (1.46) spiroxamine (118134-30-8), (1.47) tébuconazole (107534-96-3), (1.48) terbinafine (91161-71-6), (1.49) tétraconazole (112281-77-3), (1.50) triadiméfon (43121-43-3), (1.51) triadiménol (89482-17-7), (1.52) tridémorphe (81412-43-3), (1.53) triflumizole (68694-11-1), (1.54) triforine (26644-46-2), (1.55) triticonazole (131983-72-7), (1.56) uniconazole (83657-22-1), (1.57) uniconazole-p (83657-17-4), (1.58) viniconazole (77174-66-4), (1.59) voriconazole (137234-62-9), (1.60) 1-(4-chlorophényl)-2-(1H-1,2,4-triazol-1-yl)cycloheptanol (129586-32-9), (1.61) 1-(2,2-diméthyl-2,3-dihydro-1H-indén-1-yl)-1H-imidazole-5-carboxylate de méthyle(110323-95-0), (1.62) N'-{5-(difluorométhyl)-2-méthyl-4-[3-(triméthyl-silyl)propoxy]phényl}-N-éthyl-N-méthylimidoformamide, (1.63) N-éthyl-N-méthyl-N'-{2-méthyl-5-(trifluoro-méthyl)-4-[3-(triméthylsilyl)propoxy]phényl}-imidoformamide et (1.64) O-[1-(4-méthoxyphénoxy)-3,3-diméthylbutan-2-yl]-1H-imidazole-1-carbothioate (111226-71-2).

(2) Les inhibiteurs de la respiration (inhibiteurs de la chaîne respiratoire), comme par exemple (2.1) bixafène (581809-46-3), (2.2) boscalide (188425-85-6), (2.3) carboxine (5234-68-4), (2.4) diflumétorime (130339-07-0), (2.5) fenfurame (24691-80-3), (2.6) fluopyram (658066-35-4), (2.7) flutolanil (66332-96-5), (2.8) fluxapyroxad (907204-31-3), (2.9) furametpyr (123572-88-3), (2.10) furmécyclox (60568-05-0), (2.11) isopyrazam, mélange du racémate épimère syn 1RS,4SR,9RS et du racémate épimère anti 1RS,4SR,9SR (881685-58-1), (2.12) isopyrazam (racémate épimère anti), (2.13) isopyrazam (énantiomère épimère anti 1R,4S,9S), (2.14) isopyrazam (énantiomère épimère anti 1S,4R,9R), (2.15) isopyrazam (racémate épimère syn 1RS,4SR,9RS), (2.16) isopyrazam (énantiomère épimère syn 1R,4S,9R), (2.17) Isopyrazam (énantiomère épimère syn 1S,4R,9S), (2.18) mépronil (55814-41-0), (2.19) oxycarboxine (5259-88-1), (2.20) penflufène (494793-67-8), (2.21) penthiopyrad (183675-82-3), (2.22) sédaxane (874967-67-6), (2.23) thifluzamide (130000-40-7), (2.24) 1-méthyl-N-[2-(1,1,2,2-tétrafluoroéthoxy)phényl]-3-(trifluorométhyl)-1H-pyrazole-4-carboxamide, (2.25) 3-(difluorométhyl)-1-méthyl-N-[2-(1,1,2,2-tétrafluoroéthoxy)phényl]-1H-pyrazole-4-carboxamide, (2.26) 3-(difluorométhyl)-N-[4-fluoro-2-(1,1,2,3,3,3-hexafluoropropoxy)phényl]-1-méthyl-1H-pyrazole-4-carboxamide, (2.27) N-[1-(2,4-dichlorophényl)-1-méthoxypropan-2-yl]-3-(difluorométhyl)-1-méthyl-1H-pyrazole-4-carboxamide (1092400-95-7), (2.28) 5,8-difluoro-N-[2-(2-fluoro-4-{[4-(trifluorométhyl)pyridin-2-yl]oxy}phényl)éthyl]quinazolin-4-amine (1210070-84-0), (2.29) N-[9-(dichlorométhylène)-1,2,3,4-tétrahydro-1,4-méthanonaphthalén-5-yl]-3-(difluorométhyl)-1-méthyl-1H-pyrazole-4-carboxamide, (2.30) N-[(1S,4R)-9-(dichlorométhylène)-1,2,3,4-tétrahydro-1,4-méthanonaphthalén-5-yl]-3-(difluorométhyl)-1-méthyl-1 H-pyrazole-4-carboxamide et (2.31) N-[(1R,4S)-9-(dichlorométhylène)-1,2,3,4-tétrahydro-1,4-méthanonaphtalén-5-yl]-3-(difluorométhyl)-1-méthyl-1H-pyrazole-4-carboxamide.

(3) Les inhibiteurs de la respiration (inhibiteurs de la chaîne respiratoire) au niveau du complexe III de la chaîne respiratoire, comme par exemple (3.1) amétoctradine (865318-97-4), (3.2) amisulbrom (348635-87-0), (3.3) azoxystrobine (131860-33-8), (3.4) cyazofamide (120116-88-3), (3.5) couméthoxystrobine (850881-30-0), (3.6) coumoxystrobine (850881-70-8), (3.5) dimoxystrobine (141600-52-4), (3.6) énestroburine (238410-11-2), (3.9) famoxadone (131807-57-3), (3.10) fénamidone (161326-34-7), (3.11) fénoxystrobine (918162-02-4), (3.12) fluoxastrobine (361377-29-9), (3.13) krésoxime-méthyle (143390-89-0), (3.14) métominostrobine (133408-50-1), (3.15) orysastrobine (189892-69-1), (3.16) picoxystrobine (117428-22-5), (3.17) pyraclostrobine (175013-18-0), (3.18) pyramétostrobine (915410-70-7), (3.19) pyraoxystrobine (862588-11-2), (3.20) pyribencarbe (799247-52-2), (3.21) triclopyricarbe (902760-40-1), (3.22) trifloxystrobine (141517-21-7), (3.23) (2E)-2-(2-{[6-(3-chloro-2-méthylphénoxy)-5-fluoropyrimidin-4-yl]oxy}-phényl)-2-(méthoxyimino)-N-méthyléthanamide, (3.24) (2E)-2-(méthoxyimino)-N-méthyl-2-(2-{[({(1E)-1-[3-(trifluorométhyl)phényl]éthylidène}amino)oxy]méthyl}-phényl)éthanamide, (3.25) (2E)-2-(méthoxyimino)-N-méthyl-2-{2-[(E)-({1-[3-(trifluorométhyl)phényl]-éthoxy}imino)méthyl]phényl}éthanamide (158169-73-4), (3.26) (2E)-2-{2-[({[(1E)-1-(3-{[(E)-1-fluoro-2-phényl-éthényl]oxy}phényl)éthylidène]amino}oxy)méthyl]phényl}-2-(méthoxyimino)-N-méthyléthanamide (326896-28-0), (3.27) (2E)-2-{2-[({[(2E,3E)-4-(2,6-dichlorophényl)but-3-én-2-ylidène]amino}oxy)méthyl]phényl}-2-(méthoxy-imino)-N-méthyléthanamide, (3.28) 2-chloro-N-(1,1,3-triméthyl-2,3-dihydro-1H-indén-4-yl)pyridine-3-carboxamide (119899-14-8),(3.29) 5-méthoxy-2-méthyl-4-(2-{[({(1E)-1-[3-(trifluorométhyl)phényl]éthylidène} amino)oxy]méthyl}phényl)-2,4-dihydro-3H-1,2,4-triazol-3-one, (3.30) (2E)-2-{2-[(cyclopropyl[(4-méthoxyphényl)imino]méthyl}sulfanyl)méthyl]phényl}-3-méthoxy-prop-2-énoate de méthyle (149601-03-6), (3.31) N-(3-éthyl-3,5,5-triméthylcyclohexyl)-3-(formylamino)-2-hydroxybenzamide (226551-21-9), (3.32) 2-{2-[(2,5-diméthylphénoxy)méthyl]phényl}-2-méthoxy-N-méthyl-acétamide (173662-97-0) et (3.33) (2R)-2-{2-[(2,5-diméthylphénoxy)méthyl]phényl}-2-méthoxy-N-méthyl-acétamide (394657-24-0).

(4) Les inhibiteurs de la mitose et de la division cellulaire, comme par exemple (4.1) bénomyl (17804-35-2), (4.2) carbendazime (10605-21-7), (4.3) chlorfénazole (3574-96-7), (4.4) diéthofencarb (87130-20-9), (4.5) éthaboxame (162650-77-3), (4.6) fluopicolide (239110-15-7), (4.7) fubéridazole(3878-19-1), (4.8) pencycuron (66063-05-6), (4.9) thiabendazole (148-79-8), (4.10) thiophanate-méthyle (23564-05-8), (4.11) thiophanate (23564-06-9), (4.12) zoxamide (156052-68-5), (4.13) 5-chloro-7-(4-méthylpipéridin-1-yl)-6-(2,4,6-trifluorophényl)[1,2,4]triazolo[1,5-a]-pyrimidine (214706-53-3) et (4.14) 3-chloro-5-(6-chloropyridin-3-yl)-6-méthyl-4-(2,4,6trifluorophényl)-pyridazine (1002756-87-7).

(5) Des composés à activité multisite, comme par exemple (5.1) bouillie bordelaise (8011-63-0), (5.2) captafol (2425-06-1), (5.3) captane (133-06-2), (5.4) chlorothalonil (1897-45-6), (5.5) des préparations à base de cuivre, telles que l'hydroxyde de cuivre (20427-59-2), (5.6) le naphténate de cuivre (1338-02-9), (5.7) l'oxyde de cuivre (1317-39-1), (5.8) l'oxychlorure de cuivre (1332-40-7), (5.9) le sulfate de cuivre (7758-98-7), (5.10) dichlofluanide (1085-98-9), (5.11) dithianone (3347-22-6), (5.12) dodine (2439-10-3), (5.13) dodine base libre, (5.14) ferbame (14484-64-1), (5.15) fluorofolpet (719-96-0), (5.16) folpet (133-07-3), (5.17) guazatine (108173-90-6), (5.18) guazatine acétate, (5.19) iminoctadine (13516-27-3), (5.20) iminoctadine albésilate (169202-06-6), (5.21) iminoctadine triacétate (57520-17-9), (5.22) mancopper (53988-93-5), (5.23) mancozèbe (8018-01-7), (5.24) manèbe (12427-38-2), (5.25) métirame (9006-42-2), (5.26) métirame zinc (9006-42-2), (5.27) oxine-cuivre (10380-28-6), (5.28) propamidine (104-32-5), (5.29) propinèbe (12071-83-9), (5.30) soufre et composés soufrés comme par exemple polysulfure de calcium (7704-34-9), (5.31) thirame (137-26-8), (5.32) tolylfluanid (731-27-1), (5.33) zinèbe (12122-67-7) et (5.34) zirame (137-30-4) .

(6) Les inducteurs de résistance, comme par exemple (6.1) acibenzolar-S-méthyle (135158-54-2), (6.2) isotianil (224049-04-1), (6.3) probénazole (27605-76-1) et (6.4) tiadinil (223580-51-6).

(7) Les inhibiteurs de la biosynthèse des acides aminés et des protéines, comme par exemple (7.1) andoprim (23951-85-1), (7.2) blasticidine-S (2079-00-7), (7.3) cyprodinil (121552-61-2), (7.4) kasugamycine (6980-18-3), (7.5) kasugamycine chlorhydrate hydrate (19408-46-9), (7.6) mépanipyrim (110235-47-7), (7.7) pyriméthanil (53112-28-0) et (7.8) 3-(5-fluoro-3,3,4,4-tetraméthyl-3,4-dihydroisoquinolin-1-yl)quinoléine (861647-32-7).

(8) Les inhibiteurs de la production de l'ATP, comme par exemple (8.1) fentine acétate (900-95-8),

(8.2) fentine chlorure (639-58-7), (8.3) fentine hydroxyde (76-87-9) et (8.4) silthiofame (175217-20-6).

(9) Les inhibiteurs de la synthèse de la paroi cellulaire, comme par exemple (9.1) benthiavalicarbe (177406-68-7), (9.2) diméthomorphe (110488-70-5), (9.3) flumorphe (211867-47-9), (9.4) iprovalicarbe (140923-17-7), (9.5) mandipropamide (374726-62-2), (9.6) polyoxines (11113-80-7), (9.7) polyoxorime (22976-86-9), (9.8) validamycine A (37248-47-8) et (9.9) valifénalate (283159-94-4 ; 283159-90-0).

(10) Les inhibiteurs de la synthèse des lipides et de la membrane, comme par exemple (10.1) biphényle (92-52-4), (10.2) chloronèbe (2675-77-6), (10.3) dichloran (99-30-9), (10.4) édifenphos (17109-49-8), (10.5) étridiazole (2593-15-9), (10.6) iodocarbe (55406-53-6), (10.7) iprobenfos (26087-47-8), (10.8) isoprothiolane (50512-35-1), (10.9) propamocarbe (25606-41-1), (10.10) propamocarbe chlorhydrate (25606-41-1), (10.11) prothiocarbe (19622-08-3), (10.12) pyrazophos (13457-18-6), (10.13) quintozène (82-68-8), (10.14) tecnazène (117-18-0) et (10.15) tolclofos-méthyle (57018-04-9).

(11) Les inhibiteurs de la biosynthèse de la mélanine, comme par exemple (11.1) capropamide (104030-54-8), (11.2) diclocymet (139920-32-4), (11.3) fénoxanil (115852-48-7), (11.4) fthalide (27355-22-2), (11.5) pyroquilone (57369-32-1), (11.6) tricyclazole (41814-78-2) et (11.7) {3-méthyl-1-[(4-méthylbenzoyl)amino]-butan-2-yl}carbamate de 2,2,2-trifluoroéthyle (851524-22-6).

(12) Les inhibiteurs de la synthèse des acides nucléiques, comme par exemple (12.1) bénalaxyl (71626-11-4), (12.2) bénalaxyl-M (kiralaxyl) (98243-83-5), (12.3) bupirimate (41483-43-6), (12.4) clozylacon (67932-85-8), (12.5) diméthirimol (5221-53-4), (12.6) éthirimol (23947-60-6), (12.7) furalaxyl (57646-30-7), (12.8) hyméxazole (10004-44-1), (12.9) métalaxyl (57837-19-1), (12.10) métalaxyl-M (méfénoxame) (70630-17-0), (12.11) ofurace (58810-48-3), (12.12) oxadixyl (77732-09-3) et (12.13) acide oxolinique (14698-29-4).

(13) Les inhibiteurs de la transduction de signaux, comme par exemple (13.1) chlozolinate (84332-86-5), (13.2) fenpiclonil (74738-17-3), (13.3) fludioxonil (131341-86-1), (13.4) iprodione (36734-19-7), (13.5) procymidone (32809-16-8), (13.6) quinoxyfène (124495-18-7) et (13.7) vinclozoline (50471-44-8).

(14) Les découpleurs, comme par exemple (14.1) binapacryl (485-31-4), (14.2) dinocap (131-72-6), (14.3) férimzone (89269-64-7), (14.4) fluazinam (79622-59-6) et (14.5) meptyldinocap (131-72-6).

(15) D'autres composés, comme par exemple (15.1) benthiazole (21564-17-0), (15.2) bethoxazine (163269-30-5), (15.3) capsimycine (70694-08-5), (15.4) carvone (99-49-0), (15.5) chinométhionate (2439-01-2), (15.6) pyriofénone (chlazafénone) (688046-61-9), (15.7) cufranèbe (11096-18-7), (15.8) cyflufénamide (180409-60-3), (15.9) cymoxanil (57966-95-7), (15.10) cyprosulfamide (221667-31-8), (15.11) dazomet (533-74-4), (15.12) débacarbe (62732-91-6), (15.13) dichlorophène (97-23-4), (15.14) diclomézine (62865-36-5), (15.15) difenzoquat (49866-87-7), (15.16) difenzoquat méthylsulfate (43222-48-6), (15.17) diphénylamine (122-39-4), (15.18) écomate, (15.19) fenpyrazamine (473798-59-3), (15.20) flumétover (154025-04-4), (15.21) fluoroimide (41205-21-4), (15.22) flusulfamide (106917-52-6), (15.23) flutianil (304900-25-2), (15.24) fosétyl-aluminium (39148-24-8), (15.25) fosétyl-calcium, (15.26) fosétyl-sodium (39148-16-8), (15.27) hexachlorobenzène (118-74-1), (15.28) irumamycine (81604-73-1), (15.29) méthasulfocarbe (66952-49-6), (15.30) isothiocyanate de méthyle (556-61-6), (15.31) métrafénone (220899-03-6), (15.32) mildiomycine (67527-71-3), (15.33) natamycine (7681-93-8), (15.34) diméthyldithiocarbamate de nickel (15521-65-0), (15.35) nitrothal-isopropyle (10552-74-6), (15.36) octhilinone (26530-20-1), (15.37) oxamocarbe (917242-12-7), (15.38) oxyfenthiine (34407-87-9), (15.39) pentachlorophénol et ses sels (87-86-5), (15.40) phénothrine, (15.41) acide phosphorique et ses sels (13598-36-2), (15.42) propamocarbe-fosétylate, (15.43) propanosine-sodium (88498-02-6), (15.44) proquinazide (189278-12-4), (15.45) pyrimorphe (868390-90-3), (15.45e) (2E)-3-(4-tert-butylphényl)-3-(2-chloropyridin-4-yl)-1-(morpholin-4-yl)prop-2-én-1-one (1231776-28-5), (15.45z) (2Z)-3-(4-tert-butylphényl)-3-(2-chloropyridin-4-yl)-1-(morpholin-4-yl)prop-2-én-1-one (1231776-29-6), (15.46) pyrrolnitrine (1018-71-9), (15.47) tébufloquine (376645-78-2), (15.48) técloftalame (76280-91-6), (15.49) tolnifanide (304911-98-6), (15.50) triazoxide (72459-58-6), (15.51) trichlamide (70193-21-4), (15.52) zarilamide (84527-51-5), (15.53) 2-méthylpropionate de(3S,6S,7R,8R)-8-benzyl-3-[({3-[(isobutyryloxy)méthoxy]-4-méthoxypyridin-2-yl}carbonyl)amino]-6-méthyl-4,9-dioxo-1,5-dioxonan-7-yle (517875-34-2), (15.54) 1-(4-{4-[(5R)-5-(2,6-difluorophényl)-4,5-dihydro-1,2-oxazol-3-yl]-1,3-thiazol-2-yl}pipéridin-1-yl)-2-[5-méthyl-3-(trifluoro-méthyl)-1H-pyrazol-1-yl]éthanone (1003319-79-6), (15.55) 1-(4-{4-[(5S)-5-(2,6-difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]-éthanone (1003319-80-9), (15.56) 1-(4-{4-[5-(2,6-difluorophényl)-4,5-dihydro-1,2-oxazol-3-yl]-1,3-thiazol-2-yl}pipéridin-1-yl)-2-[5-méthyl-3-(trifluoro-méthyl)-1H-pyrazol-1-yl]éthanone (1003318-67-9), (15.57) 1-(4-méthoxyphénoxy)-3,3-diméthylbutan-2-yl-1H-imidazol-1-carboxylate (111227-17-9), (15.58) 2,3,5,6-tétrachloro-4-(méthylsulfonyl)pyridine (13108-52-6), (15.59) 2,3-dibutyl-6-chlorothiéno[2,3-d]-pyrimidin-4(3H)-one (221451-58-7), (15.60) 2,6-diméthyl-1H,5H-[1,4]dithiino[2,3-c:5,6-c']dipyrrole-1,3,5,7(2H,6H)-tétrone, (15.61) 2-[5-méthyl-3-(trifluorméthyl)-1H-pyrazol-1-yl]-1-(4-{4-[(5R)-5-phényl-4,5-dihydro-1,2-oxazol-3-yl]-1,3-thiazol-2-yl}-pipéridin-1-yl)éthanone (1003316-53-7), (15.62) 2-[5-méthyl-3-(trifluorméthyl)-1H-pyrazol-1-yl]-1-(4-{4-[(5S)-5-phényl-4,5-dihydro-1,2-oxazol-3-yl]-1,3-thiazol-2-yl}pipéridin-1-yl)éthanone (1003316-54-8), (15.63) 2-[5-méthyl-3-(trifluorméthyl)-1H-pyrazol-1-yl]-1-{4-[4-(5-phényl-4,5-dihydro-1,2-oxazol-3-yl)-1,3-thiazol-2-yl]pipéridin-1-yl}éthanone (1003316-51-5), (15.64) 2-butoxy-6-iodo-3-propyl-4H-chromén-4-one, (15.65) 2-chloro-5-[2-chloro-1-(2,6-difluoro-4-méthoxy-phényl)-4-méthyl-1H-imidazol-5-yl]pyridine, (15.66) 2-phénylphénol et ses sels (90-43-7), (15.67) 3-(4,4,5-trifluoro-3,3-diméthyl-3,4-dihydroisoquinolin-1-yl)-quinoléine (861647-85-0), (15.68) 3,4,5-trichloro-pyridine-2,6-dicarbonitrile (17824-85-0), (15.69) 3-[5-(4-chloro-phényl)-2,3-diméthyl-1,2-oxazolidin-3-yl]-pyridine, (15.70) 3-chloro-5-(4-chlorophényl)-4-(2,6-difluorophényl)-6-méthylpyridazine,(15.71) 4-(4-chlorophényl)-5-(2,6-difluorophényl)-3,6-diméthyl-pyridazine, (15.72) 5-amino-1,3,4-thiadiazol-2-thiol, (15.73) 5-chloro-N'-phényl-N'-(prop-2-yn-1-yl)thiophén-2-sulfonohydrazide (134-31-6), (15.74) 5-fluoro-2-[(4-fluorobenzyl)oxy]pyrimidin-4-amine (1174376-11-4), (15.75) 5-fluoro-2-[(4-méthylbenzyl)oxy]pyrimidin-4-amine (1174376-25-0), (15.76) 5-méthyl-6-octyl[1,2,4]-triazolo[1,5-a]pyrimidin-7-amine, (15.77) (2Z)-3-amino-2-cyano-3-phénylprop-2-énoate d'éthyle, (15.78) N'-(4-{[3-(4-chlorobenzyl)-1,2,4-thiadiazol-5-yl]oxy}-2,5-diméthylphényl)-N-éthyl-N-méthylimidoformamide, (15.79) N-(4-chlorobenzyl)-3-[3-méthoxy-4-(prop-2-yn-1-yloxy)-phényl]propanamide, (15.80) N-[(4-chlorophényl)-(cyano)méthyl]-3-[3-méthoxy-4-(prop-2-yn-1-yloxy)-phényl]propanamide, (15.81) N-[(5-bromo-3-chloro-pyridin-2-yl)méthyl]-2,4-dichloropyridine-3-carboxamide, (15.82) N-[1-(5-bromo-3-chloropyridin-2-yl)éthyl]-2,4-dichloropyridine-3-carboxamide, (15.83) N-[1-(5-bromo-3-chloropyridin-2-yl)éthyl]-2-fluoro-4-iodopyridine-3-carboxamide, (15.84) N-{(E)-[(cyclopropylméthoxy)imino][6-(difluorométhoxy)-2,3-difluorophényl]méthyl}-2-phénylacétamide (221201-92-9), (15.85) N-{(Z)-[(cyclopropylméthoxy)imino][6-(difluorométhoxy)-2,3-difluorophényl]méthyl}-2-phénylacétamide (221201-92-9), (15.86) N'-{4-[(3-tert-butyl-4-cyano-1,2-thiazol-5-yl)oxy]-2-chloro-5-méthylphényl}-N-éthyl-N-méthylimidoformamid, (15.87) N-méthyl-2-(1-{[5-méthyl-3-(trifluorométhyl)-1H-pyrazol-1-yl]acetyl}pipéridin-4-yl)-N-(1,2,3,4-tétrahydronaphtalén-1-yl)-1,3-thiazol-4-carboxamide (922514-49-6), (15.88) N-méthyl-2-(1-{[5-méthyl-3-(trifluorométhyl)-1H-pyrazol-1-yl]acétyl}pipéridin-4-yl)-N-[(1R)-1,2,3,4-tétrahydronaphtalén-1-yl]-1,3-thiazol-4-carboxamide (922514-07-6), (15.89) N-méthyl-2-(1-{[5-méthyl-3-(trifluorométhyl)-1H-pyrazol-1-yl]acétyl}pipéridin-4-yl)-N-[(1S)-1,2,3,4-tétrahydronaphtalén-1-yl]-1,3-thiazole-4-carboxamide (922514-48-5), (15.90) {6-[({[(1-méthyl-1H-tetrazol-5-yl)(phényl)méthylidène]amino}oxy)méthyl]pyridin-2-yl}-carbamate de pentyle, (15.91) acide phénazine-l-carboxylique, (15.92) quinolin-8-ol (134-31-6), (15.93) sulfate de quinolin-8-ol (2:1) (134-31-6) et (15.94) {6-[({[(1-méthyl-1H-tétrazol-5-yl)(phényl)méthylène]-amino}oxy)méthyl]pyridin-2-yl}carbamate de tert-butyle.

(16) D'autres composés, comme par exemple (16.1) 1-méthyl-3-(trifluorométhyl)-N-[2'-(trifluorométhyl)-biphényl-2-yl]-1H-pyrazole-4-carboxamide, (16.2) N-(4'-chlorobiphényl-2-yl)-3-(difluorométhyl)-1-méthyl-1H-pyrazole-4-carboxamide, (16.3) N-(2',4'-dichloro-biphényl-2-yl)-3-(difluorométhyl)-1-méthyl-1H-pyrazole-4-carboxamide, (16.4) 3-(difluorométhyl)-1-méthyl-N-[4'-(trifluorométhyl)biphényl-2-yl]-1H-pyrazole-4-carboxamide, (16.5) N-(2',5'-difluorobiphényl-2-yl)-1-méthyl-3-(trifluorométhyl)-1H-pyrazole-4-carboxamide, (16.6) 3-(difluorométhyl)-1-méthyl-N-[4'-(prop-1-yn-1-yl)biphényl-2-yl]-1H-pyrazole-4-carboxamide, (16.7) 5-fluoro-1,3-diméthyl-N-[4'-(prop-1-yn-1-yl)biphényl-2-yl]-1H-pyrazole-4-carboxamide, (16.8) 2-chloro-N-[4'-(prop-1-yn-1-yl)biphényl-2-yl]pyridine-3-carboxamide, (16.9) 3-(difluorométhyl)-N-[4'-(3,3-diméthylbut-1-yn-1-yl)biphényl-2-yl]-1-méthyl-1H-pyrazole-4-carboxamide, (16.10) N-[4'-(3,3-diméthylbut-1-yn-1-yl)biphényl-2-yl]-5-fluoro-1,3-diméthyl-1H-pyrazole-4-carboxamide, (16.11) 3-(difluorométhyl)-N-(4'-éthynylbiphényl-2-yl)-1-méthyl-1H-pyrazole-4-carboxamide, (16.12) N-(4'-éthynylbiphényl-2-yl)-5-fluoro-1,3-diméthyl-1H-pyrazole-4-carboxamide, (16.13) 2-chloro-N-(4'-éthynylbiphényl-2-yl)pyridine-3-carboxamide, (16.14) 2-chloro-N-[4'-(3,3-diméthylbut-1-yn-1-yl)biphényl-2-yl]pyridine-3-carboxamide, (16.15) 4-(difluorométhyl)-2-méthyl-N-[4'-(tfluorométhyl)biphényl-2-yl]-1,3-thiazol-5-carboxamide, (16.16) 5-fluoro-N-[4'-(3-hydroxy-3-méthylbut-1-yn-1-yl)biphényl-2-yl]-1,3-diméthyl-1H-pyrazole-4-carboxamide, (16.17) 2-chloro-N-[4'-(3-hydroxy-3-méthylbut-1-yn-1-yl)biphényl-2-yl]pyridine-3-carboxamide, (16.18) 3-(difluorométhyl)-N-[4'-(3-méthoxy-3-méthylbut-1-yn-1-yl)biphényl-2-yl]-1-méthyl-1H-pyrazole-4-carboxamide, (16.19) 5-fluoro-N-[4'-(3-méthoxy-3-méthylbut-1-yn-1-yl)biphényl-2-yl]-1,3-diméthyl-1H-pyrazole-4-carboxamide, (16.20) 2-chloro-N-[4'-(3-méthoxy-3-méthylbut-1-yn-1-yl)biphényl-2-yl]pyridine-3-carboxamide, (16.21) (5-bromo-2-méthoxy-4-méthylpyridin-3-yl)(2,3,4-triméthoxy-6-méthylphényl)méthanone, (16.22) N-[2-(4-{[3-(4-chlorophényl)prop-2-in-1-yl]oxy}-3-méthoxyphényl)éthyl]-N2-(méthylsulfonyl)valinamide (220706-93-4), (16.23) acide 4-oxo-4-[(2-phényléthyl)amino]butanoïque et (16.24) {6-[({[(Z)-(1-méthyl-1H-tétrazol-5-yl)(phényl)-méthylène]amino}oxy)méthyl]pyridin-2-yl}carbamate de but-3-yn-1-yle.

**17.** Compositions agrochimiques, **caractérisées en ce qu'**elles contiennent au moins un composé de formule (I) selon les revendications 1 à 15 ou une composition selon la revendication 16, ainsi que des excipients et/ou des substances tensioactives.

**18.** Procédé pour la préparation de compositions agrochimiques, **caractérisé en ce qu'**on mélange des composés de formule (I) selon les revendications 1 à 15 ou une composition selon la revendication 16, avec des excipients et/ou des substances tensioactives.

**19.** Procédé pour la lutte contre des ravageurs animaux, **caractérisé en ce qu'**on fait agir sur des ravageurs animaux et/ou leur habitat des composés de formule (I) selon les revendications 1 à 15 ou une composition selon la revendication 16, à l'exclusion des procédés pour le traitement thérapeutique ou chirurgical du corps humain ou animal et des procédés de diagnostic qui sont effectués sur le corps humain ou animal.

**20.** Utilisation des composés de formule (I) selon les revendications 1 à 15 ou d'une composition selon la revendication 16 pour la lutte contre des ravageurs animaux dans la protection des plantes, dans la protection des matériaux et/ou dans le secteur de la médecine vétérinaire, à l'exclusion des utilisations dans des procédés pour le traitement thérapeutique ou chirurgical du corps humain ou animal et des procédés de diagnostic qui sont effectués sur le corps humain ou animal.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2007131680 A
- WO 2010100189 A
- JP 2011042611 A
- EP 2202226 A1
- JP 2007284386 A
- EP 1803712 A1
- WO 9726248 A1
- US 6906006 B
- DE 19500439
- WO 20100119194 A
- DE 4431218
- WO 2009012275 A
- WO 2008155034 A
- DE 19528305
- WO 2008086226 A
- WO 2006117657 A1
- US 201099725 A1
- WO 201047956 A1
- WO 200585226 A1
- WO 200862905 A2
- WO 2012149413 A
- WO 2011110575 A
- WO 2011098776 A
- WO 2010149755 A
- WO 2008077597 A
- WO 2008057254 A
- WO 2007038367 A
- US 20070066588 A
- WO 2005111038 A
- WO 2005092890 A
- DE 2650604
- WO 2004037787 A
- ES 1982516898
- US 200727125 B
- US 5266701 A
- US 4665083 A
- US 6353006 B
- WO 200397605 A
- US 201153947 A1
- WO 2005103054 A2
- US 3853912 A
- US 2012277273 A
- WO 2006124118 A
- DE 2554866
- WO 9726248 A
- US 3663564 A
- US 3621099 A
- JP 51092865 A
- US 20030119889 A
- US 20030119890 A
- DE 19691916932
- DE 19660523
- US 2003119889 A
- US 3007927 A
- WO 2000026194 A
- JP 2007284356 A
- WO 2007034755 A
- JP 2007081019 A
- JP 2007284385 A
- JP 2008260706 A
- JP 2008308448 A
- JP 2009023910 A
- WO 2012176856 A
- WO 2006043635 A
- EP 1183229 B1
- US 2010160388 A1
- JP 53053655 A
- WO 2012149335 A2
- US 5541337 A1
- EP 507171 A1
- WO 2010063402 A1
- WO 2005077934 A
- WO 2007115644 A
- WO 2007115643 A
- WO 2007115646 A
- EP 0539588 A
- WO 2007149134 A
- WO 2010074747 A
- WO 2010074751 A
- WO 2006089633 A
- WO 2008067911 A
- WO 2008066153 A
- WO 2006056433 A
- WO 2006100288 A
- WO 2005035486 A
- WO 2007057407 A
- WO 2008104503 A
- WO 2003106457 A
- WO 2009049851 A
- WO 2004099160 A
- WO 2005063094 A
- WO 2007040280 A
- JP 2010018586 A
- WO 2007075459 A
- WO 2005085216 A
- WO 2010005692 A
- WO 2002096882 A

- WO 2007101369 A
- WO 2010006713 A
- WO 2010069502 A
- WO 2008009360 A
- CN 102057925
- WO 2011049233 A
- WO 2010025451 A
- WO 2004058723 A
- WO 161326347 A
- WO 918162024 A
- WO 361377299 A
- WO 143390890 A
- WO 133408501 A
- WO 189892691 A
- WO 117428225 A
- WO 175013180 A
- WO 915410707 A
- WO 862588112 A
- WO 0212172 A
- WO 2005070917 A
- WO 2005042474 A
- EP 1559320 A
- WO 2003035617 A
- WO 2009094442 A

**In der Beschreibung aufgeführte Nicht-Patentliteratur**

- *Chem. Pharm. Bull.,* 1997, vol. 45 (4), 719-721
- *J. Amer. Chem. Soc.,* 2003, vol. 125 (37), 11253-11258
- *Bull. Korean Chem. Soc.,* 2010, vol. 31 (8), 2143-2146
- *Chem. Sei.,* 2010, vol. 1, 13-31
- *Tetrahedron Lett.,* 2009, vol. 50, 7293-7296
- *Bioorg. Med. Chem. Lett.,* 2010, vol. 20 (13), 3920-3924
- *Proc. Natl. Acad. Sei. USA,* 2011, vol. 108 (17), 6781-6786
- *Chem. Eur. J.,* 2009, vol. 15 (29), 7044-7047
- *Synlett,* 2004, vol. 6, 1095-97
- *J. Org. Chem.,* 2005, vol. 70 (4), 1486-1489
- *Tetrahedron Lett.,* 1998, vol. 39, 2933-2936
- *Synthesis,* 2011, 829-856
- *Tetrahedron,* 2012, vol. 68, 7735-7754
- *J.Med.Chem.,* 1966, vol. 9 (6), 858-860
- *Can. J. Chem.,* 2004, vol. 82, 1649
- *Molecules,* 2012, vol. 17, 1233-1240
- *Organic Process Research and Development,* 2010, vol. 14, 1457-1463
- *Journal of Heterocyclic Chemistry,* 1986, vol. 23, 1427-1429
- *Chemical Biology & Drug Design,* 2007, vol. 70, 100-112
- *J. Het. Chem.,* 1966, vol. 3, 311
- *Tetrahedron Lett.,* 1990, vol. 31 (21), 2991
- **S. K. SAHOO.** *Adv. Synth. and Catal.,* 2010, vol. 352, 2538-2548
- **Y. V. RASCHKES.** *J. Org. Chem. USSR (Engl.),* 1981, vol. 17, 529-536
- **H. G. HOKER.** *Synthesis,* 2009, vol. 7, 1195-1203
- *J. Med. Chem.,* 2012, vol. 55, 8225-8235
- *Synth. Commun.,* 2010, vol. 40, 1377-1390
- *Tetrahedron Lett.,* 2012, vol. 53, 5593-5596
- *J. Org. Chem.,* 1997, vol. 62, 3230-3235
- *J. Med. Chem.,* 2012, vol. 55, 8236-8247
- *Tetrahedron Lett.,* 2004, vol. 45, 437-440
- *Bioorg. & Med. Chem,* 2002, 3267-3276
- *Bioorg. & Med. Chem,* 2012, 3242-3254
- *Tetrahedron,* 1999, 193-200
- *Tetrahedron Letters,* 2009, vol. 50, 5123-5125
- *Pharmazie,* 1992, vol. 47, 340
- *JACS,* 1959, 6498-6503
- *Makromolekulare Chemie,* 1988, vol. 157, 59-78
- *Biochemistry,* 1966, vol. 5 (9), 2963-2971
- *J. Org. Chem.,* 2007, vol. 43 (3), 393-396
- *Tetrahedron,* 1999, vol. 55 (40), 11859-11870
- *Acta Chem. Scand.,* 1999, vol. 53 (1), 48-56
- *Indian Drugs,* 1992, vol. 29 (7), 306-307
- *Bioorg. Med. Chem. Lett.,* 2006, vol. 16 (3), 525-528
- *Arzneimittel-Forsch,* 2003, vol. 53 (4), 280-288
- *Bioorg. Med. Chem. Lett.,* 2012, vol. 22 (23), 7019-7023
- *Tetrahedron Lett.,* 2010, vol. 51, 2215-2217
- *Revue Roumaine de Chimie,* 2005, vol. 50 (7-8), 655-661
- *J. Am. Chem. Soc.,* 1924, vol. 46, 2069-2078
- **C. F. HOWELL.** *J. Org. Chem.,* 1962, vol. 27, 1691
- **V.N.BRITSUN.** *Russ. J. Org. Chem.,* 2006, vol. 41 (11), 1719-1729
- *Synlett,* 2007, vol. 8, 1255-1256
- *Synth. Commun.,* 2007, vol. 37, 1927-1934
- *Phamaceutical Bulletin,* 1954, vol. 2, 403-411
- *Synthesis,* 1982, vol. 2, 159-160
- *Monatsh. Chem.,* 1999, vol. 130 (2), 327-332
- *J. Org. Chem.,* 1991, vol. 56, 5643-5651
- **AMER.** *Chem. J.,* 1910, vol. 43, 532-543
- *Amer. Chem. J.,* 1910, vol. 43, 380
- *Helv. Chim. Acta,* 1953, vol. 36, 75-81
- *Can. J. of Research,* 1950, vol. 28, 720-725
- *Tetrahedron Letters,* 2010, vol. 51, 5328-5332
- *Arch. Pharm.,* 1980, vol. 313, 577-582
- *Arch. Pharm.,* 1958, vol. 291, 404
- *Chem. Ber.,* 1908, vol. 41, 3872
- *Bull. Chem. Soc. Japan,* 1926, vol. 1, 202
- *Tetrahedron,* 2012, vol. 68, 2621-2629

- *Rec. Trav. Chim.,* 1933, vol. 52, 979
- *Chem. Ber.,* 1903, vol. 36, 1362
- *Deutschen Chemischen Gesellschaft,* 1910, vol. 42, 4763-4769
- *DEUTSCHEN CHEMISCHEN GESELLSCHAFT,* 1911, vol. 44, 560-583
- *Rec. Trav. Chim.,* 1936, vol. 55, 101
- *J. Am. Chem. Soc.,* 1931, vol. 1552
- *Chem. Ber.,* 1901, vol. 34, 320
- *J. Korean Chem. Soc.,* 1999, vol. 43 (4), 491-493
- *Tetrahedron Lett.,* 2012, vol. 53, 4758-4762
- *Synthesis,* 1977, vol. 9, 641-2
- *Ann. Chem.,* 1927, vol. 458, 76-92
- *Recl. Trav. Chim. Pays-Bas,* 1915, vol. 34, 289-325
- *J. Fluorine Chem.,* 2011, vol. 132, 596-611
- *Bioorg. Med. Chem. Lett.,* 2009, vol. 19 (9), 2570-2573
- *Asian J. Chem.,* 2007, vol. 19 (2), 1455-1460
- *J. Het. Chem.,* 2003, vol. 40 (5), 885-893
- *J. Het. Chem.,* 1994, vol. 31 (6), 1535-9
- *J. Het. Chem.,* 1971, vol. 8 (4), 669-70
- *Pharmazie,* 1990, vol. 45 (10), 795-6
- *Chemie,* 1989, vol. 29 (8), 281-3
- *J. Org. Chem.,* 1979, vol. 44 (22), 3858-3861
- *CHEMICAL ABSTRACTS,* 1152595-83-9
- **G. S. SKINNER.** *J. Amer. Chem. Soc.,* 1950, vol. 72, 5569-5573
- **G. S. SKINNER.** *J. Amer. Chem. Soc.,* 1956, vol. 77, 4656-4659
- *J. Med. Chem.,* 2004, vol. 47, 681-695
- *J. Amer. Chem. Soc.,* 1956, 1938-1941
- *Chem. Pharm. Bull.,* 1962, vol. 10, 647-652
- **A.R. MAGUIRE.** *ARKIVOC,* 2011, 1-110
- **H. SUZUKI.** *Chem. Let.,* 1985, vol. 3, 411-412
- **S. L. BUCHWALD.** *J. Amer. Chem. Soc.,* 2002, vol. 124 (50), 14844-14845
- **E. B. MERKUSHEV.** *Synthesis,* 1988, vol. 12, 923-937
- *J. Med. Chem.,* 2003, vol. 46, 4405-4418
- *J. Amer. Chem. Soc.,* 1940, vol. 62, 2965-2966
- *Angew. Chem.,* 1995, vol. 107, 2746-2749
- *Beispiel Bioorg. Med. Chem.,* 2010, vol. 18, 1573-1582
- *J. Org. Chem.,* 1986, vol. 51, 1719-1723
- *J. Org. Chem.,* 1973, vol. 38, 2442-2446
- *Org. Lett.,* 2000, vol. 2, 1295-1297
- *J. Org. Chem.,* 1980, vol. 45, 3472-3476
- *Arch. Pharm.,* 1971, vol. 304, 706-712
- *J. Org. Chem.,* 1981, vol. 46, 614-9
- FAO Plant Production and Protection Papers - 173, prepared by the FAO/WHO Joint Meeting on Pesticide Specifications. Pesticide Specifications, Manual on development and use of FAO and WHO specifications for pesticides. Crop Life International, 2004
- **BAUR et al.** *Pesticide Science,* 1997, vol. 51, 131-152
- The Pesticide Manual. Crop Protection Council, 2006
- *CHEMICAL ABSTRACTS,* 1204776-60-2
- *CHEMICAL ABSTRACTS,* 1017050-83-7